# EUROPEAN PATENT SPECIFICATION

(11) **EP 2 755 987 B1**
(45) Date of publication and mention of the grant of the patent: **06.06.2018**
(21) Application number: 12831166.9
(22) Date of filing: 13.09.2012
(51) Int. Cl.: C12N 15/82

(54) **METHODS AND COMPOSITIONS FOR WEED CONTROL**
VERFAHREN UND ZUSAMMENSETZUNGEN ZUR UNKRAUTBEKÄMPFUNG
PROCÉDÉS ET COMPOSITIONS DE LUTTE CONTRE LES MAUVAISES HERBES

(30) Priority: 13.09.2011 US 201161534097 P
(43) Date of publication of application: 23.07.2014
(73) Proprietor: Monsanto Technology LLC, St. Louis, MO 63167 (US)
(72) Inventor: ADER, Daniel, St. Louis, MO 63167 (US); FINNESSY, John, J., St. Louis, MO 63167 (US); LI, Zhaolong, St. Louis, MO 63167 (US); SHAH, Ronak, Hasmukh, St. Louis, MO 63167 (US); TAO, Nengbing, St. Louis, MO 63167 (US); TAYLOR, Christina, Marie, St. Louis, MO 63167 (US); TAYLOR, Jennifer, Chou, St. Louis, MO 63167 (US)
(74) Representative: Uexküll & Stolberg
(86) International application number: PCT/US2012/054980
(87) International publication number: WO 2013/040117

(56) References cited:
- EP-A1- 2 545 182
- EP-A2- 2 305 813
- WO-A2-02/066660
- WO-A2-03/077648
- US-A1- 2009 011 934
- US-A1- 2011 126 311
- US-B1- 6 642 435
- US-B2- 7 525 013
- "Agronomy Facts 37: Adjuvants for enhancing herbicide performance", , 26 January 2000 (2000-01-26), pages 1-8, XP055097433, U.S.A. Retrieved from the Internet: URL:http://pubs.cas.psu.edu/freepubs/pdfs/ uc106.pdf [retrieved on 2014-01-21]
- XIUREN ZHANG ET AL: "Agrobacterium-mediated transformation of Arabidopsis thaliana using the floral dip method", NATURE PROTOCOLS, vol. 1, no. 2, 29 June 2006 (2006-06-29), pages 1-6, XP055146744, ISSN: 1754-2189, DOI: 10.1038/nprot.2006.97
- RAINER HOFGEN ' ET AL: "Repression of Acetolactate Synthase Activity through Antisense lnhibition Molecular and Biochemical Analysis of Transgenic Potato (Solanum fuberosum L. cv DCsirCe) Plants", PLANT PHYSIOL, vol. 107, no. 2, 1 February 1995 (1995-02-01), pages 469-477, XP055146743,
- DATABASE GENBANK [Online] 30 December 2010 'Solanum lycopersicum cultivar Ailsa Craig dihydropterin pyrophosphokinase-dihydropteroate synthase (HPPK-DHPS) gene, complete cds', XP003032582 Database accession no. FJ972198
- DATABASE GENBANK [Online] 13 March 2006 'PU2_plate27_F03 PU2 Prunus persica cDNA similar to expressed mRNA inferred from Prunus persica hypothetical domain/motif containing IPR011005:Dihydropteroate synthase-like, mRNA sequence', XP003032583 Database accession no. DY640489
- DATABASE EMBL [Online] 18 August 2010 'Sequence 192160 from Patent EP2213738.' Retrieved from EBI, accession no. EM_PAT:HD315444 Database accession no. HD315444
- CLOUGH S J ET AL: "Floral dip: a simplified method for Agrobacterium-mediated transformation of Arabidopsis thaliana", THE PLANT JOURNAL, BLACKWELL SCIENTIFIC PUBLICATIONS, OXFORD, GB, vol. 16, no. 6, 1 December 1998 (1998-12-01), pages 735-743, XP002132452, ISSN: 0960-7412, DOI: 10.1046/J.1365-313X.1998.00343.X

<110> Ader, Daniel
   Finnessy, John J.
   Li, Zhaolong
   Shah, Ronak Hasmukh
   Tao, Nengbing
   Taylor, Christina Marie
   Taylor, Jennifer Chou
<120> Methods and Compositions for Weed Control
<130> 40-21(58641)
<150> 61/534097
   <151> 2011-09-13
<160> 1222
<210> 1
   <211> 672
   <212> DNA
   <213> Amaranthus hybridus
<400> 1
   agatttgtga gcgccctgta gcagctgata gggatcctgc taccgttgct tcaataactg    60
   ctggagtttt aggtggtgca aacattgtaa gagtacataa tgtgagggat aaccttgatg   120
   ctgtcaagtt atgtgatgcc atactcggaa aaactgatta actgcttatt tgtaccacct   180
   tgtgaatgac gtctagtgga aagttcgatt gggatattga tgaagtccgt tagtttgctc   240
   gaaagtagag ctttagcccg taaggttata gttcttacag atgtagaatc attggctatt   300
   tgcctattcc tgcttagaag atcatagcat tgatccagac tttggattcc atgtagaatt   360
   atgttgcgac tcatggtcga attggccctt tgaggaattg ttgaagttcc ttgatctatg   420
   gttcaatcgg ctcggttcga tgaaggttgg actgtgtcga gacaagatgg gtcaaagaga   480
   ccaatggaca agaagtcgac cttgggaaag cttgatcaag ttacttggac gagcagatag   540
   cagcaggact ctggcgagca ttagacatac atatcatttg ttttggagtc agatctgcaa   600
   tcaatcagtt gcaatggaca aaccatatac ggttgttagt tgttactgtc actgcttgag   660
   ttgaattaga tc                                                       672
<210> 2
   <211> 458
   <212> DNA
   <213> Amaranthus lividus
<400> 2
   aagggaataa ggtatggtcc taggccaatt gacttggaca tactatttta tgggaaattt   60
   agggtgagct ctgagagcct cactatcccc catgaaagga tatgggaaag accatttgtg  120
   atggcaccat tgattgattg tattgggtct gatgtagaaa atgacactat ttgtacatgg  180
   cattcattat caaatttttt gggtggaatc tttgaagcat ggggtaaact cggtggaagt  240
   tccctaatcg ggaaggatgg aatgaaaagg gttttgcccg ttggaaatcg cttatgggat  300
   tggtctcata aaacctctgt aatgggagtc ttgaacttga ctcctgatag ctttagtgat  360
   ggcggaagtt ttcaatctgt cgatactgcg gttgctaagg ttcgtcagat gatctcagat  420
   ggggcagata taattgacat cggggcacaa tcaaccag                          458
<210> 3
   <211> 5590
   <212> DNA
   <213> Amaranthus palmeri
<400> 3
   ggtgtttatt tgggcatggc cttatgtgtg atgttggtaa attttgtggc ttcgtccact   60
   aatctgatgt atcggatctg ttaaagggag ccctctcatc ctatgcttat caatgacttt  120
   ttcatatgcc tatcaaacag atcagattgg atcgggttcg agttcaggtc atatataaac  180
   atgtcagcaa acccttgacc catacccaac ccatttagtt aattggcttg aaaatcacaa  240
   ccttaattcg accagcgaca gatcaggttg acctgatttc gacccactta acccatttat  300
   aatttttttt tcgatttaag ttttttaaca ttgattaaat ctaatttttt aggctttaat  360
   tttaatttta tgtttttttg tttaatttgt atttactatg aaaaataaga aactattata  420
   tgaactgagt ttagcttaca cttattaatt taactcgaaa ttaacacatt taattaaatg  480
   gttatatagg gttttttagt tttaaaattt caacctgaac ctaatctatt taataaaaag  540
   atcagatctg atcaacccat ataattaatt gggtcaaaat ctcaactcaa acccatttat  600
   ttcgaattaa attcagatca aattagcagg tcggatcaac tagcgctaat ttttcctctc  660
   aagttttttc atatgatgcc ccttgttttc tttgaataaa attcaccaat ttaaaatccc  720
   tgcccttgat ccgttggtat tggtatttgg taaagcacat atggtaaatt ctactccatt  780
   gttatctgta gttttggttc taaaccccaa acaaatccta ctcctagcac cactaatcct  840
   cattactttc tgtttgctgt ctttctctac attcctacta aattaagacc tagatgcagt  900
   gttattctgt tcgaggttgc cgaaaacggt acaatagttg cctaatttgc aattcatgtt  960
   tgttcttgcg ttttctacat tgtttactct attggaacaa aggttgaact ttcagccttt 1020
   cagggagtat tttgattttt ctgtcaaatt accttgccag atttatttgg ctattttgat 1080
   tgtgtgtatt tctttgtccc ttgtttccat ggggctaaac tccttttggt taatcttaaa 1140
   catttcttct aattttgaat tgcagtggta tgaagtgttg tttaatatac aatcaatttt 1200
   tctgaactgg gttaatgaag agaagcattg tagacatgag gaatgtgttc aagcatctca 1260
   aaacaaccaa attaatccca aatgggatct tcaactgtaa tagaggtaaa tcacatatat 1320
   tctcatttaa tggttattgt tttattttaa ggatgttatt atgttgttat caatgataga 1380
   aataagagaa gcttgagcat tcaaatgtat ctatcactca ctggttgtgg acatctaaaa 1440
   tatggaccaa gagttttctt catttcttgt accagaattt gcaaatgtat gattaaagtt 1500
   taggttggag ttgcaataaa atttcattac cccgacgcta ttaagtggct cacactgagg 1560
   cggggtttga gggtcggatg gtgcaacctt caggagaggt tctaagcatt ttcaacatgt 1620
   tcaaccgcag agggtcccgt aaaattggag gcttcaatat taaattatgt agtatgtgtt 1680
   aagtgtttaa agatataaaa atgttaatga atacataatt tttaaaattg cacagggctt 1740
   tcaaagaaat ttttgatttt gctccgccca tggcaacctt tggcaaatct ggatagaatt 1800
   agagaaattt acttttatgt atgtataatc tgatgaatga atgaatgaac aatgaagttc 1860
   atttctgagt caatgaccta tatactatcg aggtttcgtt aaataggata tataaataaa 1920
   attagagaca tcttttatcc ctaatgttgt ctgttttctt ttctgggcga gactgttttt 1980
   ggagaaaaac gttatgttga atgcttgatt tactcagaac tgatgcaaac agatggataa 2040
   aaaatggtgt tttcctagtg tttatgtttc ataaaaggat ttcaaattcc aatcgtatga 2100
   agtttgaaat actgcttttt gtttttgtag cttcgtgttt tgctttcctt cactcttcac 2160
   cagggaacgt tgttgaagtt tgttccaaga agcaagaagt cgtaattgca atcgggagca 2220
   atgttggtga cagattagaa aatttcaacc aagctctgca acaaatgaag aaattaggca 2280
   tagacatcac aaggcatggt tgtttatatg agacggaacc tgcatacgtg actgatcaac 2340
   cgaagtttct taactctgct ttaagaggct ttacaagact tggacctcat gaattattag 2400
   gggtattgaa gaaaattgag aaggatatgg gtagaaccaa gggaataagg tatggtccta 2460
   ggccaattga cttggacata ctattttatg ggaagtttag ggtgagctct gagagcctca 2520
   ctatccccca tgaaaggata tgggaaagac catttgtgat ggcaccattg attgattgta 2580
   ttgggtctga tgtagaaaat gacactattt gtacgtggca ttcattatca aatttttcgg 2640
   gtggaatctt tgaagcatgg ggtaaactcg gtggaagctc cctaatcggg aaggatggaa 2700
   tgaaaagggt tttgcccgtt ggaaatctct tatgggattg gtctcataaa acctctgtta 2760
   tgggagtctt gaacttgact cctgatagct ttagtgatgg cggaagtttt caatctgtca 2820
   atactgcggt tgctaaggtt cgtcagatga tctcagatgg ggcagatata attgacatcg 2880
   gggcacaatc aaccagaccc atggcaacta gaatttcggc tgaagaagag ctagcaagag 2940
   tagtacctgt attagaagct gtcaaggatt tgatcgagga agaaggaaga atcttgtcag 3000
   tggatacgtt ttattctaaa gttgctttgg aggccgtcaa gaagggggca cacattgtga 3060
   atgatgtctc tagtggtaaa ctcgattccg agatgtttaa tgttgttgcg gaccttaaag 3120
   ttccttatat agcaatgcac atgcgaggag atccgacttc aatgcaaaac tctgagaact 3180
   tgacctacaa tgatgtttgt aagcaagtgg cttcggagtt gagttctagg gtcatagatg 3240
   cagaattatc gggaattcct gcttggagga tagttattga tcccggcatc ggattttcta 3300
   agaatacgaa tcaaaatttg gaaattctta gtggtttaca aaagatacgg gaagagatag 3360
   ctaagaagag tttggcggtg gctcattgcc ccttgctaat tggaccttca agaaagaggt 3420
   ttctgggcga gatttgtgag cgccctgtag cagctgacag ggatcctgct accattgctt 3480
   ctataactgc tggagtttta ggtggtgcaa acattgtaag agtacataat gttagggata 3540
   accttgatgc tgtcaagtta tgtgatgcca tactcggaaa aactgattaa ctgcttgttt 3600
   gtaccacctt gtgaatgatg tctagtggaa ggttcgattg ggattatgat gaagtccgtt 3660
   agtttgctcg aaagaagagc tttagcccgt aaggttatag ttcttacaga tgtagaatca 3720
   ttggctattt gcctatttct gcttagaaga tcatagcatt gatccagact ttggattcca 3780
   tgtagaatta tgttgcgact catggtcgaa ttggcccttt gaggaattgt tgaagttcct 3840
   tgagtctatg gttcaatcgg ctcggttcga tgaaggttgg actgtgtcga gacaagatgg 3900
   gtcaaagaga ccaatggaca agaagtcgac cttgggcaag cttgatcaag ttacttggac 3960
   gagcagataa cagcaggctc tggcgagcat tagacataca tatcatttgt tttttgaagt 4020
   cagatctgca atcaatcagt tgcaatggac agaccatata cggttgttag ttgttcctgt 4080
   cactctttga gttgaattag atcatgcaaa cactgtaaga gtcttgattt tgtgaagcta 4140
   tgtgatactg tacttctcaa aatgaaatct ttgatatgat gttagcttca agagttgttt 4200
   ttccccccct cacattctgt tctatgttgt attgttgtgt gctttttgga attgtattcc 4260
   tgtaattaga attattcctg caaaatggta ttcctttata tcatcaaagg agtttttgta 4320
   cttctaatca ttgatcttta gatgcaaaac tgaattaata caagtgttct tgatgaaggc 4380
   ttattgaaga actcactttt tatttatgtc taaacttgtt aaagatgttc tgcttccgcc 4440
   cttgccacgg gatgtcggga ttgactaagg gtggtaatcg agggattcct atgcttgaat 4500
   tgactcgttt aagattttgc gttgatttta agtcaaatgt tttgctaagc ataaactatt 4560
   ctagctcgac ttgaataatg aagttttcga gtcaagtccg agctgttctg taagtttatg 4620
   tatcttgaag cttcttagtt cttacttctt atccttattt gtttgacaca ttgaaccgcg 4680
   ttgatgcttg cttgactcaa cccgttaaag tcttgaattc atcacgaatt cgagtcgagc 4740
   aagtttaaac ccaacattaa ctaagctctc gaggcttaat gaggcagggg tagctcaagt 4800
   ttgttattca agcaacttac catagtattc ttttaaaagg catatagtca gttagttatt 4860
   tgttaatttc agtagaacga aagaagtaca ttattaaact tggccattga tgcttgagct 4920
   agagagcttc ggttgtgatc gaaacgaatc ctgatttcgc tcttgatctt tgatgtgaag 4980
   tgacaaggcc ttcataaaac tcctttattc ttcagcagtg catacaaaaa tcatgtgtgc 5040
   ccagctccta gctcacatac cgcgtttttc ccctaatcta gtgtggtcaa ataaaaacag 5100
   caagatgtgt tgatgtcatg tgcaatagta attatcaggt gaagtaatgc aatatgtagc 5160
   cagtcacctc atatcaaaaa aatgttggga ggaaaacgta ttcgttcatc actaaacctg 5220
   tgtttataca tctaactgat cgatcagggt ttcaaaatcc aaattaatac aatggcattt 5280
   gacgaagata tccagaacac atcccccttg tttctgctag ataatcaact attctgattc 5340
   tgctgtaata cctcagcttc ggccctagcc atgggatcgt cctctttttc tccttcacgc 5400
   tcctcggcca gtttctttct gtgcagttct tcagcagctt ctattgaagc tttgttctcg 5460
   gcctgaagac gctgcatttc ctcttccatt tgctgcctct caaaccatgt atcagcatcc 5520
   gcccaaactg cagacaagta tacaaaagtt aaataacgaa agtcttcaaa actgcagatc 5580
   cagtgaacaa 5590
<210> 4
   <211> 5515
   <212> DNA
   <213> Amaranthus palmeri
<400> 4
   cggatctgtt aaagggagcc ctctcatcct atgcttatca atgacttttt catatgccta 60
   tcaaacagat cagattggat cgggttcgag ttcaggtcat atataaacat gtcagcaaac 120
   ccttgaccca tacccaaccc atttagttaa ttggcttgaa aatcacaacc ttaattcgac 180
   cagcgacaga tcaggttgac ctgatttcga cccacttaac ccatttataa tttttttttc 240
   gatttaagtt ttttaacatt gattaaatct aattttttag gctttaattt taatttatgt 300
   ttttttgttt aatttgtatt tactatgaaa aataagaaac tattatatga actgagttta 360
   gcttacactt attgatttaa ctcgaaatta acacatttaa ttaaatggtt atatagggtt 420
   ttttagtttt aaaatttcaa cctgaaccta atctatttaa taaaaagatc agatctgatc 480
   aacccatata attaattggg tcaaaatctc aactcaaacc catttatttc gaattaaatt 540
   cagatcaaat tagcaggtcg gatcaactag cgctaatttt tcctctcaag ttttttcata 600
   tgatgcccct tgttttcttt gaataaaatt caccaattta aaatccctgc ccttgatccg 660
   ttggtattgg tatttggtaa agcacatatg gtaaattcta ctccattgtt atctgtagtt 720
   ttggttctaa accccaaaca aatcctactc ctagcaccac taatcctcat tactttctgt 780
   ttgctgtctt tctctacatt cctactaaat taagacctag atgcagtgtt attctgttcg 840
   aggttgccga aaacggtaca atagttgcct aatttgcaat tcatgtttgt tcttgcgttt 900
   tctacattgt ttactctatt ggaacaaagg ttgaactttc agcctttcag ggagtatttt 960
   gatttttctg tcaaattacc ttgccagatt tatttggcta ttttgattgt gtgtatttct 1020
   ttgtcccttg tttccatggg gctaaactcc ttttggttaa tcttaaacat ttcttctaat 1080
   tttgaattgc agtggtatga agtgttgttt aatatacaat caatttttct gaactgggtt 1140
   aatgaagaga agcattgtag acatgaggaa tgtgttcaag catctcaaaa caaccaaatt 1200
   aatcccaaat gggatcttca actgtaatag aggtaaatca catatattct catttaatgg 1260
   ttattgtttt attttaagga tgttattatg ttgttatcaa tgatagaaat aagagaagct 1320
   tgagcattca aatttatcta tcactcactg gttgtggaca tctaaaatat ggaccaagag 1380
   ttttcttcat tttttgtacc agaagctgca aatgtatgat taaagtttag gttggagttg 1440
   caataaaatt ttattacccc gacgctatta agtggctcac actgaggcgg ggtttgaggg 1500
   tcagatggtg caaccttcag aagaggttct aagcattttc aacatgttca accgcatagg 1560
   gtcccgtaaa attggaggct tcaatattaa attatgtagt atgtgttatg tgtttaaaga 1620
   tataaatatg ttaatgaata cataattttt aaattgcaca gggccttcaa agaaattttc 1680
   gattttgctc tgcccctggc aacctttggc aaatttggat agaattagag aagtttactt 1740
   ttatgtgtgt ataatatgat gaatgaatga gtgaacaatg aagttcattt ctgagtcaat 1800
   gacctatata ctatcgaggt ttcgttaaaa aggatatata aataaaatta gagacatctt 1860
   ttatccctaa tgttgtctgt tttcttttct gggcgagact gtttttggag aaaaacgtta 1920
   tgttgaatgc ttgatttact cagaactgat gcaaacagat ggataaaaat ggtgttttcc 1980
   tattgtttat gtttcgataa caggtttcaa attccactcg tatgaagttt gatatactgc 2040
   tatgtgtttt tgtagcttcg tgttttgctt tccttcactc atcaccagtg aatgatgttg 2100
   aagtttgttc caagaagcaa gaagtcgtaa ttgcaatcgg gagcaatgtt ggtgacagat 2160
   tagaaaattt caaccaagct ctgcaacaaa tgaagaaatt aggcatagac atcacaaggc 2220
   atgggtgttt atatgagacg gaacctgcat acgtgactga tcaaccgaag tttcttaact 2280
   ctgctttaag aggctttaca aaacttgggc ctcatgaatt attaggggta ttaaagaaaa 2340
   ttgagaagga tatgggtaga accaagggaa taaggtatgg tcctaggcca attgacttgg 2400
   acatactatt ttatgggaag tttagggtga gctctgagag cctcactatc ccccatgaaa 2460
   ggatatggga aagaccattt gtgatggcac cattgattga ttgtattggg tctgatgtag 2520
   aaaatgacac tatttgtacg tggcattcat tatcaaattt ttcgggtgga atctttgaag 2580
   catggggtaa actcggtgga agttccctaa tcgggaagga tggaatgaaa agggttttgc 2640
   ccgttggaaa tcgcttatgg gattggtctc ataaaacctc tgttatggga gtcttgaact 2700
   tgactcctga tagctttagt gatggcggaa gttttcaatc tgtcgatact gcggttgcta 2760
   aggttcgtca gatgatctca gatggggcag atataattga catcggggca caatcaacca 2820
   gacccatggc aactagaatt tcggctgaag aagagctaga aagagtagta cctgtattgg 2880
   aagctgtcaa ggatttgatc gaggaagaag gaagaatctt gtcagtggat acgttttatt 2940
   ctaaagttgc ttcggaggcc gtcaagaagg gggcacacat tgtgaatgat gtctctagtg 3000
   ggaaactcga ttccgagatg tttaatgttg ttgcggacct taaagttcct tatatagcaa 3060
   tgcacatgcg aggagatccg acttcaatgc aaaactctga gaacttgacc tacaatgatg 3120
   tttgtaagca agtggctttg gagttgagtt ctagggtcat agatgcagaa ttatcgggaa 3180
   ttcctgcttg gaggatagtt attgatcccg gcatcggatt ttctaagaat acgaatcaaa 3240
   atttggaaat tcttagtggt ttacaaaaga tacgggaaga gatagctaag aagagtttgg 3300
   cggtggctca ttgccccttg ctaattggac cttcaagaaa gaggtttctg ggcgagattt 3360
   gtgagcgccc tgtagcagct gacagggatc ctgctaccat tgcttctata actgctggag 3420
   ttttaggtgg tgcaaacatt gtaagagtac ataatgtgag ggataacctt gatgctgtca 3480
   agttatgtga tgccatactc ggaaaaactg attaactgct tgtttgtacc accttgtgaa 3540
   tgatgtctag tggaaggttc gattgggatt atgatgaagt ccgttagttt gctcgaaaga 3600
   agagctttag cccgtaaggt tatagttctt acagatgtag aatcattggc tatttgccta 3660
   tttctgctta gaagatcata gcattgatcc agactttgga ttccatgtag aattatgttg 3720
   cgactcatgg tcgaattggc cctttgagga attgttgaag ttccttgagt ctatggttca 3780
   atcggctcgg ttcgatgaag gttggactgt gtcgagacaa gatgggtcaa agagaccaat 3840
   ggacaagaag tcgaccttgg gcaagcttga tcaagttact tggacaagca gataacagca 3900
   ggctctggcg agcattagac atatcatttg ttttttggag tcagatctgc aatcaatcag 3960
   ttgcaatgga caaaccatat acggttgtta gttgttactg tcactgtttg agttgaatta 4020
   gatcatgcaa acactgtagg agtcttgatt atgtgaagct atgtgacact gtacttctca 4080
   aaatgaaatc tttgatatga tgttagtttc aagagttgta attcccccct cacatttctg 4140
   ttctatgttg tattgttgtg tgctttttgg aattgtattc ctgcaataga attattcccg 4200
   caaaatggta ttcctttata tcatcaaagg agtttttgta cttctaatca ttgatcttta 4260
   gatgcaaaac tgaattaata caagtattct tgatgaaggc ttattgaaga actcactttt 4320
   tatttatgtc taaacttgtt aaagatgttc tgcttccgcc cttgccacgg gatgtcggga 4380
   ttgactaggg gtggtaatcg agggattcct atgcttgaat tgactcgttt aagattttgc 4440
   gttgatttta agtcaaacgt tttgctaagc ataaactatt ctagctcgac ttgaataatg 4500
   aagttttcga gtcaagtccg agctgttcta taagtttacg tatcttgaag cttcttagtt 4560
   cttacttctt atcctttatt ttttgacaca ttgaaccgca ttgatgcttg cttgactcaa 4620
   ctcgttaaag tcttgagttc atcacgaatt cgagtcgagc aagtttaaac ccaacattaa 4680
   ctaagctctc gaggcttaat gaggcagggg tagctcaagt ttgttattca agcaacttac 4740
   catagtattc ttttaaaagg catatagtca gttagttatt tgttaatttc agtagaacga 4800
   aagaagtaca ttattaaact tggccattga tgcttgagct agagagtttc ggttgtgatc 4860
   gaaacgaatc ctgatttcgc tcttgatctt tgatgtgaag tgacaaggcc ttcataaaac 4920
   tcctttattc ttcagcagtg catacaaaaa tcatgtgtgc ccagctccta gctcacatac 4980
   cgcgtttttc ccctaatcta gtgtggttaa ataaaaacag caagatgtgt tgatgtcatg 5040
   tgcaatagta attatcaggt gaagtaattc aatatgtagc cagtcacctc atatcaaaaa 5100
   aatgttggga ggaaaacgta ttcgttcatc actaaacctg tttttataca tctaactgat 5160
   cgatcagggt ttcaaaatcc aaattaatac aatggcattt gacgaagata tccagaacac 5220
   atcccccttg tttctgctag ataatcaact attctgattc tgctgtaata cctcagcttc 5280
   ggccctagcc atgggatcgt cctctttttc tccttcacgc tcctcggcca gtttctttct 5340
   gtgcagttct tcagcagctt ctattgaagc tttgttctcg gcctgaagac gctgcatttc 5400
   ctcttccatt tgctgcctct caaaccatgt atcagcatcc gcccaaactg cagacaagta 5460
   tacaaaagtt aaataacgaa agtcttcaaa actgcagatc cagtgaacaa agtca      5515
<210> 5
   <211> 1431
   <212> DNA
   <213> Amaranthus palmeri
<400> 5
   gctttccttc actcatcacc agtgaatgat gttgaagttt gttccaagaa gcaagaagtc   60
   gtaattgcaa tcgggagcaa tgttggtgac agattagaaa atttcaacca agctctgcaa  120
   caaatgaaga aattaggcat agacatcaca aggcatgggt gtttatatga gacggaacct  180
   gcatacgtga ctgatcaacc gaagtttctt aactctgctt taagaggctt tacaaaactt  240
   gggcctcatg aattattagg ggtattaaag aaaattgaga aggatatggg tagaaccaag  300
   ggaataaggt atggtcctag gccaattgac ttggacatac tattttatgg gaagtttagg  360
   gtgagctctg agagcctcac tatcccccat gaaaggatat gggaaagacc atttgtgatg  420
   gcaccattga ttgattgtat tgggtctgat gtagaaaatg acactatttg tacgtggcat  480
   tcattatcaa atttttcggg tggaatcttt gaagcatggg gtaaactcgg tggaagttcc  540
   ctaatcggga aggatggaat gaaaagggtt ttgcccgttg gaaatcgctt atgggattgg  600
   tctcataaaa cctctgttat gggagtcttg aacttgactc ctgatagctt tagtgatggc  660
   ggaagttttc aatctgtcga tactgcggtt gctaaggttc gtcagatgat ctcagatggg  720
   gcagatataa ttgacatcgg ggcacaatca accagaccca tggcaactag aatttcggct  780
   gaagaagagc tagaaagagt agtacctgta ttggaagctg tcaaggattt gatcgaggaa  840
   gaaggaagaa tcttgtcagt ggatacgttt tattctaaag ttgcttcgga ggccgtcaag  900
   aagggggcac acattgtgaa tgatgtctct agtgggaaac tcgattccga gatgtttaat  960
   gttgttgcgg accttaaagt tccttatata gcaatgcaca tgcgaggaga tccgacttca 1020
   atgcaaaact ctgagaactt gacctacaat gatgtttgta agcaagtggc tttggagttg 1080
   agttctaggg tcatagatgc agaattatcg ggaattcctg cttggaggat agttattgat 1140
   cccggcatcg gattttctaa gaatacgaat caaaatttgg aaattcttag tggtttacaa 1200
   aagatacggg aagagatagc taagaagagt ttggcggtgg ctcattgccc cttgctaatt 1260
   ggaccttcaa gaaagaggtt tctgggcgag atttgtgagc gccctgtagc agctgacagg 1320
   gatcctgcta ccattgcttc tataactgct ggagttttag gtggtgcaaa cattgtaaga 1380
   gtacataatg tgagggataa ccttgatgct gtcaagttat gtgatgccat a         1431
<210> 6
   <211> 1431
   <212> DNA
   <213> Amaranthus palmeri
<400> 6
   gctttccttc actcttcacc agggaacgtt gttgaagttt gttccaagaa gcaagaagtc   60
   gtaattgcaa tcgggagcaa tgttggtgac agattagaaa atttcaacca agctctgcaa  120
   caaatgaaga aattaggcat agacatcaca aggcatggtt gtttatatga gacggaacct  180
   gcatacgtga ctgatcaacc gaagtttctt aactctgctt taagaggctt tacaagactt  240
   ggacctcatg aattattagg ggtattgaag aaaattgaga aggatatggg tagaaccaag  300
   ggaataaggt atggtcctag gccaattgac ttggacatac tattttatgg gaagtttagg  360
   gtgagctctg agagcctcac tatcccccat gaaaggatat gggaaagacc atttgtgatg  420
   gcaccattga ttgattgtat tgggtctgat gtagaaaatg acactatttg tacgtggcat  480
   tcattatcaa atttttcggg tggaatcttt gaagcatggg gtaaactcgg tggaagctcc  540
   ctaatcggga aggatggaat gaaaagggtt ttgcccgttg gaaatctctt atgggattgg  600
   tctcataaaa cctctgttat gggagtcttg aacttgactc ctgatagctt tagtgatggc  660
   ggaagttttc aatctgtcaa tactgcggtt gctaaggttc gtcagatgat ctcagatggg  720
   gcagatataa ttgacatcgg ggcacaatca accagaccca tggcaactag aatttcggct  780
   gaagaagagc tagcaagagt agtacctgta ttagaagctg tcaaggattt gatcgaggaa  840
   gaaggaagaa tcttgtcagt ggatacgttt tattctaaag ttgctttgga ggccgtcaag  900
   aagggggcac acattgtgaa tgatgtctct agtggtaaac tcgattccga gatgtttaat  960
   gttgttgcgg accttaaagt tccttatata gcaatgcaca tgcgaggaga tccgacttca 1020
   atgcaaaact ctgagaactt gacctacaat gatgtttgta agcaagtggc ttcggagttg 1080
   agttctaggg tcatagatgc agaattatcg ggaattcctg cttggaggat agttattgat 1140
   cccggcatcg gattttctaa gaatacgaat caaaatttgg aaattcttag tggtttacaa 1200
   aagatacggg aagagatagc taagaagagt ttggcggtgg ctcattgccc cttgctaatt 1260
   ggaccttcaa gaaagaggtt tctgggcgag atttgtgagc gccctgtagc agctgacagg 1320
   gatcctgcta ccattgcttc tataactgct ggagttttag gtggtgcaaa cattgtaaga 1380
   gtacataatg ttagggataa ccttgatgct gtcaagttat gtgatgccat a          1431
<210> 7
   <211> 1163
   <212> DNA
   <213> Amaranthus palmeri
<400> 7
   gaagctgtca aggatttgat cgaggaagaa ggaagaatct tgtcagtgga tacgttttat   60
   tctaaagttg cttcggaggc cgtcaagaag ggggcacaca ttgtgaatga tgtctctagt  120
   gggaaactcg attccgagat gtttaatgtt gttgcggacc ttaaagttcc ttatatagca  180
   atgcacatgc gaggagatcc gacttcaatg caaaactctg agaacttgac ctacaatgat  240
   gtttgtaagc aagtggcttt ggagttgagt tctagggtca tagatgcaga attatcggga  300
   attcctgctt ggaggatagt tattgatccc ggcatcggat tttctaagaa tacgaatcaa  360
   aatttggaaa ttcttagtgg tttacaaaag atacgggaag agatagctaa gaagagtttg  420
   gcggtggctc attgcccctt gctaattgga ccttcaagaa agaggtttct gggcgagatt  480
   tgtgagcgcc ctgtagcagc tgacagggat cctgctacca ttgcttctat aactgctgga  540
   gttttaggtg gtgcaaacat tgtaagagta cataatgtta gggataacct tgatgctgtc  600
   aagttatgtg atgccatact cggaaaaact gattaactgc ttgtttgtac caccttgtga  660
   atgatgtcta gtggaaggtt cgattgggat tatgatgaag tccgttagtt tgctcgaaag  720
   tagagcttta gcccgtaagg ctatagttct tacagatgta gaatcattgg ctatttgcct  780
   atttctgctt agaagatcat agcattgatc cagactttgg attccatgta gaattatgtt  840
   gcgactcatg gtcgaattgg ccctttgagg aattgttgaa gttccttgag tctatggttc  900
   aatcggctcg gttcgatgaa ggttggactg tgtcgagaca agatgggtca aagagaccaa  960
   tggacaagaa gtcgaccttg ggcaagcttg atcaagttac ttggacaagc agataacagc 1020
   aggctctggc gagcattaga catatcattt gttttttgga gtcagatctg caatcaatca 1080
   gttgcaatgg acaaaccata tacggttgtt agttgttact gtcactgttt gagttgaatt 1140
   agatcatgca aacactgtag gag                                         1163
<210> 8
   <211> 1431
   <212> DNA
   <213> Amaranthus rudis
<400> 8
   gcgtaccttc actcatcacc agtggacgat gttgaacttt gttctaagaa gcaagaagtc   60
   ataattgcaa tcgggagcaa tgttggtgac agattagaaa attttaacca agctctgcaa  120
   caaatgaaga aattaggcat agacatcaca aggcatggtt gtttatatga gacagaaccc  180
   gcatacgtga ctgatcaacc gaagtttctt aactctgctt taagaggctt tacaagactt  240
   gggcctcatg aattattagg ggtattgaag aaaattgaga agaatatggg tagaaccaag  300
   ggaataaggt atggtcctag gccaattgac ttggacatac tattttatgg gaagtttagg  360
   gtgagctctg agagcctcac tattccccat gaaaggatat gggaaagacc atttgtgatg  420
   gcaccattaa ttgattgtat tgggtctggt gtagaaaatg acactatttg tacatggcat  480
   tcgttatcaa atttttcggg tggaatcttt gaagcatggg ataaactcgg tggaagttcc  540
   ctaatcggga aggatggaat gaaaagggtt ttgcccgttg ggaaccgctt atgggattgg  600
   tctcataaaa cctctgtaat gggagtcttg aacttgactc ctgatagctt tagtgatgga  660
   ggaagttttc aatctgtcga tactgcggtt gctaaggttc gtcagatgat atcagatggg  720
   gccgacataa ttgacattgg ggcacagtca acaagaccca tggcaactag gatttcggct  780
   gaagaagagc tagaaagagt tgtacctgta ttggaagctg ttaaggatgt gatcgaggaa  840
   gaaggaagaa tcttatcggt ggatactttt tattcaaaag ttgcttctga ggccgtcaag  900
   aagggggcac acattgtgaa tgatgtctct agtgggaaat tcgatcctga gatgttcaat  960
   gttgttgcgg accttaaagt tccttatata gcaatgcaca tgcgaggaga tccgacttca 1020
   atgcaaaatt ctgagaactt gacgtacagt gatgtttgta agcaagtggc ttcggagttg 1080
   agctctaggg tcatagatgc agaattatcg ggaattcctg cttggagaat agttattgat 1140
   cccggcattg gattttctaa gaatacgaag caaaatttgg aaattcttac tggtttacaa 1200
   aagatacggc tagagatagc taagaagagt ttggcggtgg ctcattgccc cttgctaatt 1260
   ggaccttcac gaaagaggtt tctgggtgag atttgtaatc gccctgtagc agctgatagg 1320
   gatcctgcta ccgtcgcttc tatcactgct ggagttttag gtggtgcaaa cattgtaaga 1380
   gtacataatg tgagggataa ccttgatgca gtcaagttat gtgatgccat a          1431
<210> 9
   <211> 1059
   <212> DNA
   <213> Amaranthus rudis
<400> 9
   tgtttcgata aaaggatttc aaatcccaat cgtatgaagt ttgaaataca actatgtgtt   60
   tttgcagctt tgttttttgc gtaccttcac tcatcaccag tggacgatgt tgaactttgt  120
   tctaagaagc aagaagtcat aattgcaatc gggagcaatg ttggtgacag attagaaaat  180
   tttaaccaag ctctgcaaca aatgaagaaa ttaggcatag acatcacaag gcatggttgt  240
   ttatatgaga cagaacccgc atacgtgact gatcaaccga agtttcttaa ctctgcttta  300
   agaggcttta caagacttgg gcctcatgaa ttattagggg tattgaagaa aattgagaag  360
   aatatgggta gaaccaaggg aataaggtat ggtcctaggc caattgactt ggacatacta  420
   ttttatggga agtttagggt gagctctgag agcctcacta ttccccatga aaggatatgg  480
   gaaagaccat ttgtgatggc accattaatt gattgtattg ggtctggtgt agaaaatgac  540
   actatttgta catggcattc gttatcaaat ttttcgggtg gaatctttga agcatgggat  600
   aaactcggtg gaagttccct aatcgggaag gatggaatga aaagggtttt gcccgttggg  660
   aaccgcttat gggattggtc tcataaaacc tctgtaatgg gagtcttgaa cttgactcct  720
   gatagcttta gtgatggagg aagttttcaa tctgtcgata ctgcggttgc taaggttcgt  780
   cagatgatat cagatggggc cgacataatt gacattgggg cacagtcaac aagacccatg  840
   gcaactagga tttcggctga agaagagcta gaaagagttg tacctgtatt ggaagctgtt  900
   aaggatgtga tcgaggaaga aggaagaatc ttatcggtgg atacttttta ttcaaaagtt  960
   gcttctgagg ccgtcaagaa gggggcacac attgtgaatg atgtctctag tgggaaattc 1020
   gatcctgaga tgttcaatgt tgttgcggac cttaaagtt                        1059
<210> 10
   <211> 792
   <212> DNA
   <213> Amaranthus rudis
<400> 10
   aatgggagtc ttgaacttga ctcctgatag ctttagtgat ggcggaaggt ttcaatctgt   60
   cgatactgcg gttgctaagg ttcgtcagat gatctctgac ggggcggaca taattgacat  120
   tggggcgcag tcaacaagac ccatggcaac taggatttcg gctgaagaag agctagaaag  180
   agtcgtacct gtattagaag ctgtcaagga tttgatcgag gaagaaggaa gaatcttgtc  240
   agtggatacg ttttattcta aagttgcttc ggaggccgtc aagaaggggg cacacattgt  300
   gaatgatgtc tctagtggga aactcgatcc cgagatgttt aatgttgttg cggaccttaa  360
   agttccttat atagcaatgc acatgcgagg agatccgact tcaatgcaaa attctgagaa  420
   cttgacgtac aatgatgtgt gtaagcaagt ggcttcggag ttgagctcta gggtcgtaga  480
   tgcagaatta tcgggaattc ctgcttggag gatggttatt gatcccggca ttggattttc  540
   taagaatacg aagcaaaatt tggaaattct tagtggttta caaaagatac ggcaagagat  600
   agctaagaag agtttggcgg tggctcattg ccccttgcta attggacctt caagaaagag  660
   gtttctgggc gagatttgca atcgccctgt agcagctgat agggatcctg ctaccgttgc  720
   ttctatcact gctggagttt taggtggtgc aaacattgta agagtacata atgtgaggga  780
   taaccttgat gc                                                      792
<210> 11
   <211> 764
   <212> DNA
   <213> Amaranthus rudis
<400> 11
   tggcaactag gatttcggct gaagaagagc tagaaagagt cgtacctgta ttagaagctg   60
   tcaaggattt gatcgaggaa gaaggaagaa tcttgtcagt ggatacgttt tattctaaag  120
   ttgcttcgga ggccgtcaag aagggggcac acattgtgaa tgatgtttct agtgggaaac  180
   tcgatcccga gatgtttaat gttgttgcgg accttaaagt tccttatata gcaatgcaca  240
   tgcgaggaga tccgacttca atgcaaaatt ctgagaactt gacgtacaat gatgtgtgta  300
   agcaagtggc ttcggagttg agctctaggg tcgtagatgc agaattatcg ggaattcctg  360
   cttggaggat ggttattgat cccggcattg gattttctaa gaatacgaag caaaatttgg  420
   aaattcttag tggtttacaa aagatacggc aagagatagc taagaagagt ttggcggtgg  480
   ctcattgccc cttgctaatt ggaccttcaa gaaagaggtt tctgggcgag atttgcaatc  540
   gccctgtagc agctgatagg gatcctgcta ccgttgcttc tatcactgct ggagttttag  600
   gtggtgcaaa cattgtaaga gtacataatg tgagggataa ccttgatgca gtcaagttat  660
   gtgatgccat actcagaaaa acggattagc cgattttttt ggattccaga gccgatgttc  720
   cagattttcc tttgctttca tgtgccgaag attcttagtt tagg                   764
<210> 12
   <211> 755
   <212> DNA
   <213> Amaranthus rudis
<400> 12
   gtaatgggag tcttgaactt gactcctgat agctttagtg atggcggaag gtttcaatct   60
   gtcgatactg cggttgctaa ggttcgtcag atgatctctg acggggcgga cataattgac  120
   attggggcgc agtcaacaag acccttggca actaggattt cggctgaaga agagctagaa  180
   agagtcgtac ctgtattaga agctgtcaag gatttgatcg aggaagaagg aagaatcttg  240
   tcagtggata cgttttattc taaagttgct tcggaggccg tcaagaaggg ggcacacatt  300
   gtgaatgatg tttctagtgg gaaactcgat cccgagatgt ttaatgttgt tgcggacctt  360
   aaagttcctt atatagcaat gcacatgcga ggagatccga cttcaatgca aaattctgag  420
   aacttgacgt acagtgatgt ttgtaagcaa gtggcttcgg agttgagctc tagggtcata  480
   gatgcagaat tatcgggaat tcctgcttgg agaatagtta ttgatcccgg cattggattt  540
   tctaagaata cgaagcaaaa tttggaaatt cttactggtt tacaaaagat acggctagag  600
   atagctaaga agagtttggc ggtggctcat tgccccttgc taattggacc ttcacgaaag  660
   aggtttctgg gtgagatttg taatcgccct gtagcagctg atagggatcc tgctaccgtc  720
   gcttctatca ctgctggagt tttaggtggt gcaaa                              755
<210> 13
   <211> 602
   <212> DNA
   <213> Amaranthus rudis
<400> 13
   aggatttcgg ctgaagaaga gctagaaaga gttgtacctg tattggaagc tgttaaggat   60
   gtgatcgagg aagaaggaag aatcttatcg gtggatacgt tttattctaa agttgcttcg  120
   gaggccgtca agaagggggc acacattgtg aatgatgttt ctagtgggaa actcgatccc  180
   gagatgttta atgttgttgc ggaccttaaa gttccttata tagcaatgca catgcgagga  240
   gatccgactt caatgcaaaa ttctgagaac ttgacgtaca atgatgtgtg taagcaagtg  300
   gcttcggagt tgagctctag ggtcgtagat gcagaattat cgggaattcc tgcttggagg  360
   atggttattg atcccggcat tggattttct aagaatacga agcaaaattt ggaaattctt  420
   agtggtttac aaaagatacg gcaagagata gctaagaaga gtttggcggt ggctcattgc  480
   cccttgctaa ttggaccttc aagaaagaag gtttctgggc gagatttgca atcgccctgt  540
   agcagctgat agggatcctg ctaccgttgc ttctatcact gctggagttt taggtggtgc  600
   aa                                                                 602
<210> 14
   <211> 584
   <212> DNA
   <213> Amaranthus rudis
<400> 14
   attagaggca tcttttatcc ctaatgctgt ccgttttctt ttctgggtga gactattttg   60
   gaagaaaaaa atttatgttc aatgtttaat ttactcagaa ttgatgcaaa cagatggata  120
   aaaatggtgt tttcttatgg tttatgtttc aataaaagga tttcaaattc taatcatatg  180
   aagtttgaaa tactatgtgt ttttgtagct tcttgtattg ctttccttca ctcatcacca  240
   gtgaacgatg ttgaagtttg ttccaagaag caagaagtcg taattgcaat cgggagcaat  300
   gttggtgaca gattagaaaa tttcaacgaa gctctgcaac aaatgaagaa attaggcata  360
   gacatcacaa ggcatggttg tttatacgag acggaacctg cttacgtgac tgatcagccg  420
   aagtttctta actctgcttt aagaggcttt acaagacttg ggcctcatga attattaggg  480
   gtattgaaga gaatcgagaa ggatatgggc agaaccaagg gaataaggta tggtcctagg  540
   ccaattgact tggacatact attttatggg aagtttaggg tgag                   584
<210> 15
   <211> 570
   <212> DNA
   <213> Amaranthus rudis
<400> 15
   tgatttactc agaattgatg caaacaaatg gataaaaatg gtgttttcct atggtttatg 60
   tttcgataaa aggatttcaa atcccaatcg tatgaagttt gaaatacaac tatgtgtttt 120
   tgtagcttct tgtattgctt tccttcactc atcaccagtg aacgatgttg aagtttgttc 180
   caagaagcaa gaagtcgtaa ttgcaatcgg gagcaatgtt ggtgacagat tagaaaattt 240
   caacgaagct ctgcaacaaa tgaagaaatt aggcatagac atcacaaggc atggttgttt 300
   atacgagacg gaacctgctt acgtgactga tcagccgaag tttcttaact ctgctttaag 360
   aggctttaca agacttgggc ctcatgaatt attaggggta ttgaagagaa tcgagaagga 420
   tatgggcaga accaagggaa taaggtatgg tcctaggcca attgacttgg acatactatt 480
   ttatgggaag tttagggtga gctctgagag cctcactatt ccccatgaaa ggatatggga 540
   aagaccattt gtgatggcac cattaattga 570
<210> 16
   <211> 411
   <212> DNA
   <213> Amaranthus rudis
<400> 16
   ctgagagcct cactattccc catgaaagga tatgggaaag accatttgtg atggcaccat 60
   taattgattg tattgggtct gatgtagaaa atgacactat ttgtacatgg cattcgttat 120
   caaatttttc gggtggaata tttgaagcat gggataaact cggaggaagt tccttaatcg 180
   ggaaggatgg aatgaaaagg gttttgcccg ttgggaaccg tttatgggat tggtctcata 240
   aaacctcagt aatgggagtc ttgaacttga ctcctgatag ctttagtgat ggcggaaggt 300
   ttcaatctgt cgatactgcg gttgctaagg ttcgtcagat gatctctgac ggggcggaca 360
   taattgacat tggggcgcag tcaacaagac ccatggcaac taggatttcg g 411
<210> 17
   <211> 260
   <212> DNA
   <213> Amaranthus rudis
<400> 17
   cgctactgct gcagctgtca ccgctgcaat tttgaatggt gctaacatag taagggtaca 60
   taatgtagga tatagttcag atgctgcaaa gttttgtgat gcacctatac tcattttacc 120
   catctcgctc ctaatggacc ccagcccctt aataacgtca atgttgtgcc cacatttctt 180
   ggagaaccca atgccaggat caagaactat cctccacaat ggaatcccag atgggtctgc 240
   ttctcacctt tgcatatagc 260
<210> 18
   <211> 1504
   <212> DNA
   <213> Amaranthus viridis
<400> 18
   tctgtataac ctaaagattg aaacttttga agatattttt gaagaagtac tctttggctt   60
   tttgatccag aaaatcatat tggtatgaag tgttgtttaa tatacaatca atttttctga  120
   actgggttat taaagagaag cattgtagac atgaggaatg tgttcaagca tctcaaaaca  180
   accaaattaa tcccaaatgg gatcttcaac tgtaatagag cttcgtgttt tgctttcctt  240
   cactcatcac cggtgaacga tgttgaagtt tgttccaaga agcaagaagt cgtaattgca  300
   atcgggagca atgttggtga cagattagaa aatttcaacc aagctctgca acaaatgaag  360
   aaattaggca tagacatcac aaggcatggg tgtttatatg agacggaacc tgcatacgtg  420
   actgatcaac cgaagtttct taactctgct ttaagaggct ttacaaaact tgggcctcat  480
   gaattattag gggttttaaa gaaaattgag aaggatatgg gtagaaccaa gggaataagg  540
   tatggtccta ggccaattga cttggacata ctattttatg ggaaatttag ggtgagctct  600
   gagagcctca ctatccccca tgaaaggata tgggaaagac catttgtgat ggcaccattg  660
   attgattgta ttgggtctga tgtagaaaat gacactattt gtacatggca ttcattatca  720
   aattttttgg gtggaatctt tgaagcatgg ggtaaactcg gtggaagttc cctaatcggg  780
   aaggatggaa tgaaaagggt tttgcccgtt ggaaatcgct tatgggattg gtctcataaa  840
   acctctgtaa tgggagtctt gaacttgact cctgatagct ttagtgatgg cggaagtttt  900
   caatctgtcg atactgcggt tgctaaggtt cgtcagatga tctcagatgg ggcagatata  960
   attgacatcg gggcacaatc aaccagaccc atggcaacta ggatttcggc tgaagaagag 1020
   ctagaaagag tagtacctgt attggaagct gtcaaggatt tgatcgagga agaaggaaga 1080
   atcttgtcag tggatacgtt ttattctaaa gttgcttcgg aggccgtcaa gaagggggca 1140
   cacattgtga atgatgtctc tagtgggaaa ctcgattccg agatgtttaa tgttgttgcg 1200
   gaccttaaag ttccttatat agcaatgcac atgcgaggag atccgacttc aatgcaaaat 1260
   tctgagaact tgacgtacaa tgatgtttgt aagcaagtgg ctacggagtt gagctctagg 1320
   gtcatagatg cagaattatc gggaattcct gcttggagga tagttattga tcccggcatt 1380
   ggattttcta agaatacgaa tcaaaatttg gaaattcttc gtggtttaca aaagatacgg 1440
   gaagagatag ctaagaagag tttggcagtg gctcattgcc ccttgctaat tggaccttca 1500
   agaa                                                              1504
<210> 19
   <211> 655
   <212> DNA
   <213> Amaranthus viridis
<400> 19
   gcagctgata gggatcctgc taccgttgct tctataactg ctggagtttt aggtggtgca   60
   aacattgtaa gagtacataa tgtgagggat aaccttgatg ctgtcaagtt atgtgatgcc  120
   atactcggaa aaactgatta actgcttatt tgtaccacct tgtgaatgac gtctagtgga  180
   aagttcgatt gggatatgat gaagtccgtt agtttgctcg aaagtagagc tttagcccgt  240
   aaggttatag ttcttacaga tgtagaatca ttggctattt gcctattttt gcttagaaga  300
   tcatagcatt gatccagact ttggattcca tctagaatta tgttgcgact catggtcgaa  360
   ttggcccttt gaggaattgt tgaagttcct tgatctatgg ttcaatcggc ttggttcgat  420
   gaaggttgga ctgtgtcgag acaagatggg tcaaagagac caatggacaa gaagtcgacc  480
   ttgggaaagc ttgatcaagt tacttggacg agcagatagc agcaggactc tggcgagcat  540
   tagacataca tatcatttgt ttttggagtc agatctgcaa tcaatcagtt gcaatggaca  600
   aaccatatac ggttgttagt tgttactgtc actgcttgag ttgaattaga tcatg        655
<210> 20
   <211> 1413
   <212> DNA
   <213> Ambrosia trifida
<400> 20
   tcgttctttc atttctcttc ggatgcttcg atacaagtcc attctcaaga acaagaagta   60
   gtaattgctt taggtagcaa tgtgggtgat aggcttaata actttaatga agccttaagt  120
   caaatgaaaa aatcaggcat agaaataaca agacatgcat gtttatacga aaccgagcca  180
   gcttacgtga ctgatcaacc tcttttcctc aattctgcca tcagaggcgt tacaaagctg  240
   ggcccacatg agctactatc ggccctcaag aaaattgaaa aagaaatggg ccgaaccaaa  300
   ggtattagat acggcccgcg acccattgac ttagatatac tgttttatgg taaacacaga  360
   gttaactcgg aaattctcac tgttccacat gaaagaatct tcgagaggcc atttgttatg  420
   gctccgttag ttgacttatt ggggtcggaa attgacaatg atacggttct atgctggcat  480
   tctttctcaa aaagaggact ttttgaatct tgggaaaaat taggtggcga atctttgata  540
   ggcaaagatg ggttaagaag agttttacca gtcaataatc ggttatggga ttggtcaaag  600
   aaaacctctg tcatgggaat tttgaacttg actcccgata gttttagcga cggaggaaag  660
   tttgacactg tggggtccgc tatatctcgt gttagggcca tgatatctga aggggcagac  720
   ataattgatc tgggagctca atcgacacgt ccaatggcaa ccaagatttc agtccaagaa  780
   gaactagata ggttaatccc tgttctcgaa aagattcttg aattacccga aattgaagga  840
   aaactgctct ctatcgacac gttttactca gaagttgctt tagaagcgat taagaaaggg  900
   gctcatataa tcaatgatgt atcgggtgga aatctagatt ctgatatgtt tcgggtagtt  960
   gctaatcttg atgttccata tattgctatg cacatgagag gggacccatc cacaatgcaa 1020
   aacagtgaga atttgaagta tgatgatgtt tgtaaagaag ttggtgatga gttgtatgag 1080
   cgtgtaagag ctgcagagtt atgcggtgtg cctgcatgga ggatcattct tgacccaggg 1140
   atcgggtttt caaagaaaac cgaagataat ttggatattt tgatgggatt aaagagaatt 1200
   aggagtgaga ttgcacggaa gagcttaggg gtgtcacgtg cacctttgtt aatcggtcct 1260
   tcaagaaaga gatttttggg ggagatttgt ggtcgggctt ctgctgtcga gagagatcca 1320
   gcgactgttg ctgctgttac atgtgcggtt ttgggtggtg ctaatgttgt tcgcgttcat 1380
   aacgttggag ataatgcgga tgctgtaaag ctt                              1413
<210> 21
   <211> 1328
   <212> DNA
   <213> Ambrosia trifida
<400> 21
   aatgaagcct taagtcaaat gaaaaaatca ggcatagaaa taacaagaca tgcatgttta   60
   tacgaaaccg agccagctta cgtgactgat caacctcttt tcctcaattc tgccatcaga  120
   ggcgttacaa agctgggccc acatgagcta ctatcggccc tcaagaaaat tgaaaaagaa  180
   atgggccgaa ccaaaggtat tagatacggc ccgcgaccca ttgacttaga tatactgttt  240
   tatggtaaac acagagttaa ctcggaaatt ctcactgttc cacatgaaag aatcttcgag  300
   aggccatttg ttatggctcc gttagttgac ttattggggt cggaaattga caatgatacg  360
   gttctatgct ggcattcttt ctcaaaaaga ggactttttg aatcttggga aaaattaggt  420
   ggcgaatctt tgataggcaa agatgggtta agaagagttt taccagtcaa taatcggtta  480
   tgggattggt caaagaaaac ctctgtcatg ggaattttga acttgactcc cgatagtttt  540
   agcgacggag gaaagtttga cactgtgggg tccgctatat ctcgtgttag ggccatgata  600
   tctgaagggg cagacataat tgatctggga gctcaatcga cacgtccaat ggcaaccaag  660
   atttcagtcc aagaagaact agataggtta atccctgttc tcgaaaagat tcttgaatta  720
   cccgaaattg aaggaaaact gctctctatc gacacgtttt actcagaagt tgctttagaa  780
   gcgattaaga aaggggctca tataatcaat gatgtatcgg gtggaaatct agattctgat  840
   atgtttcggg tagttgctaa tcttgatgtt ccatatattg ctatgcacat gagaggggac  900
   ccatccacaa tgcaaaacag tgagaatttg aagtatgatg atgtttgtaa agaagttggt  960
   gatgagttgt atgagcgtgt aagagctgca gagttatgcg gtgtgcctgc atggaggatc 1020
   attcttgacc cagggatcgg gttttcaaag aaaaccgaag ataatttgga tattttgatg 1080
   ggattaaaga gaattaggag tgagattgca cggaagagct taggggtgtc acgtgcacct 1140
   ttgttaatcg gtccttcaag aaagagattt ttgggggaga tttgtggtcg ggcttctgct 1200
   gtcgagagag atccagcgac tgttgctgct gttacatgtg cggttttggg tggtgctaat 1260
   gttgttcgcg ttcataacgt tggagataat gcggatgctg taaagctttg tgattcaatg 1320
   ttgaaccg                                                          1328
<210> 22
   <211> 486
   <212> DNA
   <213> Ambrosia trifida
<400> 22
   gccttaagtc aaatgaagaa atcaggcata gagataacaa gacatgcatg tttatacaaa   60
   accgagccag cttatgtaac tgatcaacct ctttttctta attctgccat cagaggcgtt  120
   acaaagctgg gcccacatga actactatcg gccctcaaga aaatcgaaaa agaattgggc  180
   cgaaccaaag gtattagata cggcccgcga cccattgact tagatatact gttttatggt  240
   aaacacagaa ttaactcgga aattctcact gttccacatg aaagaatctt cgagaggcca  300
   tttgttatgg ctccgttagt tgacttattg gggtcggata ttgacaatga tacggttcta  360
   tgctggcatt ctttctcaaa aagaggactt tttgaatctt gggaaaaatt aggtggcgaa  420
   tctttgatag gcaaagatgg gttaagaaga gttttaccgg tcaataatcg gttatgggat  480
   tggtca                                                             486
<210> 23
   <211> 486
   <212> DNA
   <213> Ambrosia trifida
<400> 23
   gccttaagtc aaatgaagaa atcaggcata gagataacaa gacatgcatg tttatacaaa   60
   accgagccag cttatgtaac tgatcaacct ctttttctta attctgccat cagaggcgtt  120
   acaaagctgg gcccacatga actactatcg gccctcaaga aaatcgaaaa agaattgggc  180
   cgaaccaaag gtattagata cggcccgcga cccattgact tagatatact gttttatggt  240
   aaacacagaa ttaactcgga aattctcact gttccacatg aaagaatctt cgagaggcca  300
   tttgttatgg ctccgttagt tgacttattg gggtcggata ttgacaatga tacggttcta  360
   tgctggcatt ctttctcaaa aagaggactt tttgaatctt gggaaaaatt aggtggcgaa  420
   tctttgatag gcaaagatgg gttaagaaga gttttaccgg tcaataatcg gttatgggat  480
   tggtca                                                             486
<210> 24
   <211> 410
   <212> DNA
   <213> Ambrosia trifida
<400> 24
   gacccatcca caatgcaaaa cagtgagaat ttgaagtata atgatgtttg taaagaagtt   60
   ggtgatgagt tgtatgagcg tgtaagagct gcagagttat gcggtgtgcc tgcatggagg  120
   atcattcttg acccagggat cgggttttca aagaaaaccg aagataattt ggatattttg  180
   atgggattaa agagaattag gagtgagatt gcccggaaga gcttaggggt gtcacgtgca  240
   cctttgttaa tcggtccttc aagaaagaga tttttgggag agatttgtgg tcgggcttct  300
   gctggtgaga gagatccaac aactgttgct gctgttacat gtgcggtttt gggtggtgct  360
   aatgttgttc gcgttcataa tgttggagat gatgctgatg ctgtaaagct             410
<210> 25
   <211> 381
   <212> DNA
   <213> Ambrosia trifida
<400> 25
   ttcgtgtatc tcatgatctc tgtataatgt taaacttatt atagttcaat ataagtcact   60
   gtgtgatcta atttcgtata caattacttt atttgtgttg attctgatcc taaatattgc  120
   taatagtttg tgcttaacta tatatcccgt taaacatcac atttacttta tccctagaag  180
   atttgggcaa gtttttacaa atttatggcg acgttattgt aattttttaa atcattttcg  240
   tgcagcatct ttttcgttct ttcatttctc ttcggatgct tcgatacaag tccattctca  300
   agaacaagaa gtagtaattg ctttaggtag caatgtgggt gataggctta ataactttaa  360
   tgaagcctta agtcaaatga a                                            381
<210> 26
   <211> 304
   <212> DNA
   <213> Ambrosia trifida
<400> 26
   gacccatcca caatgcaaaa cagtgagaat ttgaagtata atgatgtttg taaagaagtt   60
   ggtgatgagt tgtatgagcg tgtaagagct gcagagttat gcggtgtgcc tgcatggagg  120
   atcattcttg acccagggat cgggttttca aagaaaaccg aagataattt ggatattttg  180
   atgggattaa agagaattag gagtgagatt gcccggaaga gcttaggggt gtcacgtgca  240
   cctttgttaa tcggtccttc aagaaagaga tttttgggag agatttgtgg tcgggcttct  300
   gctg                                                               304
<210> 27
   <211> 255
   <212> DNA
   <213> Ambrosia trifida
<400> 27
   gatttgtggt cgggcttctg ctggtgagag agatccaaca actgttgctg ctgttacatg   60
   tgcggttttg ggtggtgcta atgttgttcg cgttcataat gttggagatg atgctgatgc  120
   tgtaaagctt tgtgattcaa tgttgaaccg ggttggaaga acgtaacagc tttttgaaat  180
   ttgattgtgg gttttggtcc gggtggtggt gctcgggtgt ggtttgggtt ttggttcgag  240
   tgggtggttg ctggt                                                   255
<210> 28
   <211> 201
   <212> DNA
   <213> Ambrosia trifida
<400> 28
   acctcatcac ctccacaaat caccaccacc catctccacc ctccaccacc acccacttca   60
   ttctccccac ccaccgtatc tcatcaccat catacctggt ggattgcagt ttgactctgt  120
   ggggtccgct atatctcgtg ttaggaccat gatatctgaa ggggcggaca taattgatct  180
   gggagctcaa tcgacacgtc c                                            201
<210> 29
   <211> 101
   <212> DNA
   <213> Ambrosia trifida
<400> 29
   ggattgcagt ttgactctgt ggggtccgct atatctcgtg ttaggaccat gatatctgaa   60
   ggggcggaca taattgatct gggagctcaa tcgacacgtc c                      101
<210> 30
   <211> 5446
   <212> DNA
   <213> Conyza canadensis
<400> 30
   tttcttcttc tagttggagc tcttttacgt ggtgtcttct tttccgagtc atcttcttcc   60
   ccatcagatg gattttctgc agcctttttt ctacgaactg ctgtaatagc aaatgtacta 120
   cgcaatctaa catctgggaa ttgtacaaag tttattgaag atagtgaagg cagattcgac 180
   tgccacctac acatataaga aaccattaac ttctatactt aactcacaat ataaaacatc 240
   atttgcatgt tcgtcaaaaa caataccatt tcttatcttt gctcaatgga tatgaaatct 300
   gccaatacaa gattattcat ttgacctcaa tctagcatct gatgtctatt caaggtatcg 360
   tttataatga agattattgt acttcaaaat actttcttgt gtagtggtat caaattttga 420
   gttaaagtta ctccatttga cctcaaaagc caaataatga gcaacaaaca acacaatttc 480
   acctccctta taccctgcga ctatatttta tcttcaaatc aatccatttc catgtttatc 540
   ctctccatac agctacataa taagttttag ggaaaaaaca aaactaaact aaactaagtt 600
   tacagaattc ccatttctta tcctaaaatt tacaagcaat tcaacatata ttctttattt 660
   acaactataa atagcatatg ggtatgttgg tatatagata tagatataga tatacatatg 720
   tagatgtgta ttatatgtgt gtaaatgggg agaaaaaagg aaacatatat tacctgtgta 780
   aagaacaaaa ttgagtaaaa gaaggcaaag aagccatttc taaccactaa aatcttgggc 840
   tttcaagaat cccacttcaa agctaccatt tcacatacca aacccccctt tctatgggtt 900
   taaagatgag ggctttacat ttatctcttt tacaatccaa attttccaaa accgaaataa 960
   tgtacgacat tcggtgcaca aggtacaaac agaaaaaact acttttgtat tttagatctc 1020
   aagaaaggag tggccttttt ggtaataaca tactttattc aattgccact taccttcaag 1080
   aaattaacaa aattacccac aattaaaaaa gggtttccca gtttcactca cacttattca 1140
   agattgatgc aaaataaacc cataaataag aaggttaaaa tagtgttaaa gccttaaagg 1200
   tgcttgcttt ttgtgttttt tgtgatgcct ataaaaagat tcttattgtt ttcaaatttt 1260
   tttatgtcaa gttttcgttt ttaacagttt atatagttat ataatttgca gaaagccata 1320
   aagctctgaa cttgaatgtt ttatagcagc tctttgcagt aatatcttgt agaggtaaac 1380
   ctgttaaccc atatttatta atttgtacat aaaattgttg aattgaatag agctaccact 1440
   gggacttgtg tagtcaaact gtagagcatc cctgtaatgc tggaagcttt atgacctaaa 1500
   aggatttgta gacttgttat ctgtggattg gtataaagat tttcaagttg ttattttgaa 1560
   actttgaata gtatattatg ctcattactg aatgatacaa tagggagtcg cgaatgttag 1620
   aattgataag aattaggatg aagtttgtgt ttgaacgttc cttctgttta taatcttcat 1680
   gtcacttcca tagtaccata accctagggc aacacgagcc tttagattat gaatgggcac 1740
   acccaattgt ttagaccttg tataaatgaa gtgaatatca ttgttataag cattctggtg 1800
   gtggttttgc agatttacct ttctctatct aatttgaagg tttgttgatt aaggtgatac 1860
   aaatgagtgt tttcaagcag ctaatagctt caagattagt acgcactcat gtctgtggat 1920
   cttatagagg tacacttcct cgaacattca tatagcgatg ttattgttaa gttttgaatc 1980
   tgtagcaaca ttatatataa ttttttttgc attgttttcc tgcagcatcc ttttcactct 2040
   ttcattcgtc ctcggatact tcaatacaag tgcattctcc agaacaagaa gtagtaattg 2100
   ctttaggtag caatgtgggt aatagactta ataactttaa tgaagcctta tcccagatga 2160
   agaaatcggg gatagaaata acaaggcatg cttgtctata cgaaactgaa ccagcttatg 2220
   tgacggacca gcctcttttt ctcaactctg ccgttagagc cactacaaag cttggccctc 2280
   atgagctact gtccattctc aagaaaattg aaaaggaaat gggtcgaacc aaagggctta 2340
   ggtacggccc acgacccatt gacctagata tattgttcta tggtaaatgc aaagttaact 2400
   ctgatattct aactgttcct catgaaagaa tctttgagag gccattcgtt atggctccat 2460
   tagttgactt gttaggatca gatgtagaca atgatacggt tctatgctgg cattcttttt 2520
   caaaaaatgg gctttttgga tcttgggaaa cattaggtgg tgaatcttcc ataggaaaag 2580
   atggtttaag aagggtttta cctgtcaatg atcgtttatg ggattggtca aagaaaactt 2640
   ccgtcatggg tattttgaat ataactcctg atagttttag tgatggaggg aagtttgatt 2700
   ccatggggtc cgctttatct cgtgttcaga ccatgatatc tgaaggggtt gacataattg 2760
   atctaggagc tcaatccaca cgcccaatgg cgaccaagat ctcagtcgaa gaggaactag 2820
   ataggctaat acccgttctt gaaaagattc ttgaattacc tgaaattgaa ggaaagttgt 2880
   tgtctgtgga cacattttac tcggaagttg cttcagaggc aatcaagaaa ggggctcata 2940
   tggtcaatga tgtatcgggt ggaatgttag attctgatat gcttcatgtt gtggctgatc 3000
   taaatgttcc atatatcact atgcacatga gaggggaccc atccacaatg caaaacagtg 3060
   agaatttgaa gtatgatgat gttgtcaaag aagttgggga ggaattgtat gaacgtgtaa 3120
   ggaatgcaga gttatgtggt gttcccgcat ggaggatggt tcttgatcca gggatcgggt 3180
   tttcaaagaa aaccgaagat aatttggaga tattgatggg actaaagagg tttagaagtg 3240
   agattggaca aaagagctta ggggtgtctc gtgcaccttt gttaatcgga ccttcaagaa 3300
   aaaggttttt gggtgagatt tgtggtcggc cttctgctgt tgagagagat ccagggactg 3360
   tagctgctgt taccagtgcg attttgggtg gtgccaatat cgttagggtt cataacattg 3420
   gacataatgt ggatgctgtt aagctttgtg attcaatgtt ggatcgagct ggtagatctt 3480
   agtagcttaa agttatttga catcttgttt tttattcaat tttctggaca ttactttttg 3540
   tatttctgtc tggcaaatga ttttacaggg atatcctaga ggctaaattg tatattacat 3600
   tcttgatttt ctcacaaagt tttacttttt cagtttttct tgttcagttt tgtttttgaa 3660
   aatttgctag atcagattaa atcgttgctt tatgatcttc gtgtcttttt tgggacgctg 3720
   cgagtttcat ttagtggtat tgtaaactcc ttttaaagtt tgtacacact tggtatttat 3780
   gcttctagtt ttcgtctatg tctgtcctat attgtgtata gtatgtttaa agttcataaa 3840
   aagaggataa acgaatagca cgtaaatctt ataattcgat tccacagagt gcaagttatg 3900
   ctttcctaaa tgaaagcaag agtctagaca gtttggtcaa cgttaacatg agactattgt 3960
   agaatacaac accacaagaa accaaaactg tcttgtgcat ttctctcttt tccaggttta 4020
   aaactcaata ccaagacttg atatcaacta ttattctgct tcaatgattc agcttcagct 4080
   atagccatag gatcatcttt ggtgtcaggt tcatccttct caccatctcg ttcctctgcc 4140
   agtttttttc tgtgcaattc ctctgcaact tgctttgatg cttggtcttc tttttgaagg 4200
   cgttgcatct cttcttcttg ttgctgtcgt tcaaaccatg tatccgcatc ggcccaaact 4260
   gtaatttaga gccaaaacaa catgattaga atgcagattc cagaagccaa attcaaactg 4320
   taagaaaaaa aatgtagtga tgatttaact caaaacaact cacacagctc caaaatggat 4380
   gggggttcaa attagaggat agcctaatag atagaagctt ctaattatgg ggaaaaaaaa 4440
   acgatcatgg caatgtatta cacaatctaa agcaaccact atgaataata aagataaatc 4500
   tttccgagag tccacatgcc ttgatatttc ataagatagg ctttttcaga aatcagaatc 4560
   ttcttggcaa atataacact aaaatcagtc taatgcatgt tattaagtaa atctttattt 4620
   gtccatagta taatgccata gattatcgtc aattatccac atcgtcatta aatatcaagt 4680
   aaccacatta tgaattgact tgtttaatca aagttcatct tacttcttca tagatgaaaa 4740
   agagctatgc aaatacagaa cacatcagtt gttctaacat ataattactg ctagctgtag 4800
   gaagaaacca agatgaaact tatctaggat tcaaattcat ggggatctgt tatttagatg 4860
   aatttagaga aataagaaaa taagttgagt gaagtcagtt tcgaggttcc aaatttgtct 4920
   ttattagttg agctaacaat attaacctct gaagcatgta tagtgtactt gtatgaacca 4980
   cttaaggata taaatgaagc caaatcagtt tcattcgcta attgagcaaa tgcacgttct 5040
   gtttgttagt attgaaattt ctttacagga tttctttcaa ctaaaagcat cacatgtccg 5100
   actctcggtt tgatatttgt gacaagtcta attggttcat cagatgtact tatggctaaa 5160
   tttcgaaact tgaaacaata agagcttagc actcttaacc ataaagggtt gctgttaatc 5220
   ttacttgttc ttgaaagcaa ttccacagtg aagcatttgt taattgggaa gttttgcaat 5280
   gtttctaaag cttcgatgat attttatatt ttcaaacatt tttcatcaca tgtgttaaca 5340
   aaagccataa gaataccggg gtaatcaact aaagtctatt taaagttgca tgctcgagag 5400
   caagaaaaaa ccatcaaatt tacataaact gaagggcaaa tgtacg 5446
<210> 31
   <211> 5446
   <212> DNA
   <213> Conyza canadensis
<400> 31
   ttttcttctt ctagttggag ctcttttacg tggtgtcttc ttttccgagt catcttcttc 60
   cccatcagat ggattttctg cagccttttt tctacgaact gctgtaatag caaatgtact 120
   acgcaatcta acatctggga attgtacaaa gtttattgaa gatagtgaag gcagattcga 180
   ctgccaccta cacatataag aaaccattaa cttctatact taactcacaa tataaaacat 240
   catttgcatg ttcgtcaaaa acaataccat ttcttatctt tgctcaatgg atatgaaatc 300
   tgccaataca agattattca tttgacctca atctagcatc tgatgtctat tcaaggtatc 360
   gtttataatg aagattattg tacttcaaaa tactttcttg tgtagtggta tcaaattttg 420
   agttaaagtt actccatttg acctcaaaag ccaaataatg agcaacaaac aacacaattt 480
   cacctccctt ataccctgcg actatatttt atcttcaaat caatccattt ccatgtttat 540
   cctctccata cagctacata ataagtttta gggaaaaaac aaaactaaac taaactaagt 600
   ttacagaatt cccatttctt atcctaaaat ttacaagcaa ttcaacatat attctttatt 660
   tacaactata aatagcatat gggtatgttg gtatatagat atagatatag atatacatat 720
   gtagatgtgt attatatgtg tgtaaatggg gagaaaaaag gaaacatata ttacctgtgt 780
   aaagaacaaa attgagtaaa agaaggcaaa gaagccattt ctaaccacta aaatcttggg 840
   ctttcaagaa tcccacttca aagctaccat ttcacatacc aaacccccct ttctatgggt 900
   ttaaagatga gggctttaca tttatctctt ttacaatcca aattttccaa aaccgaaata 960
   atgtacgaca ttcggtgcac aaggtacaaa cagaaaaaac tacttttgta ttttagatct 1020
   caagaaagga gtggcctttt tggtaataac atactttatt caattgccac ttaccttcaa 1080
   gaaattaaca aaattaccca caattaaaaa agggtttccc agtttcactc acacttattc 1140
   aagattgatg caaaataaac ccataaataa gaaggttaaa atagtgttaa agccttaaag 1200
   gtgcttgctt tttgtgtttt ttgtgatgcc tataaaaaga ttcttattgt tttcaaattt 1260
   ttttatgtca agttttcgtt tttaacagtt tatatagtta tataatttgc agaaagccat 1320
   aaagctctga acttgaatgt tttatagcag ctctttgcag taatatcttg tagaggtaaa 1380
   cctgttaacc catatttatt aatttgtaca taaaattgtt gaattgaata gagctaccac 1440
   tgggacttgt gtagtcaaac tgtagagcat ccctgtaatg ctggaagctt tatgacctaa 1500
   aaggatttgt agacttgtta tctgtggatt ggtataaaga ttttcaagtt gttattttga 1560
   aactttgaat agtatattat gctcattact gaatgataca atagggagtc gcgaatgtta 1620
   gaattgataa gaattaggat gaagtttgtg tttgaacgtt ccttctgttt ataatcttca 1680
   tgtcacttcc atagtaccat aaccctaggg caacacgagc ctttagatta tgaatgggca 1740
   cacccaattg tttagacctt gtataaatga agtgaatatc attgttataa gcattctggt 1800
   ggtggttttg cagatttacc tttctctatc taatttgaag gtttgttgat taaggtgata 1860
   caaatgagtg ttttcaagca gctaatagct tcaagattag tacgcactca tgtctgtgga 1920
   tcttatagag gtacacttcc tcgaacatta tatagcgatg ttattgttaa gttttgaatc 1980
   tgtagcaaca ttatatataa ttttttttgc attgttttcc tgcagcatcc ttttcactct 2040
   ttcattcgtc ctcggatact tcaatacaag tgcattctcc agaacaagaa gtagtaattg 2100
   ctttaggtag caatgtgggt aatagactta ataactttaa tgaagcctta tcccagatga 2160
   agaaatcggg gatagaaata acaaggcatg cttgtctata cgaaactgaa ccagcttatg 2220
   tgacggacca gcctcttttt ctcaactctg ccgttagagc cactacaaag cttggccctc 2280
   atgagctact gtccattctc aagaaaattg aaaaggaaat gggtcgaacc aaagggctta 2340
   ggtacggccc acgacccatt gacctagata tattgttcta tggtaaatgc aaagttaact 2400
   ctgatattct aactgttcct catgaaagaa tctttgagag gccattcgtt atggctccat 2460
   tagttgactt gttaggatca gatgtagaca atgatacggt tctatgctgg cattcttttt 2520
   caaaaaatgg gctttttgga tcttgggaaa cattaggtgg tgaatcttcc ataggaaaag 2580
   atggtttaag aagggtttta cctgtcaatg atcgtttatg ggattggtca aagaaaactt 2640
   ccgtcatggg tattttgaat ataactcctg atagttttag tgatggaggg aagtttgatt 2700
   ccatggggtc cgctttatct cgtgttcaga ccatgatatc tgaaggggtt gacataattg 2760
   atctaggagc tcaatccaca cgcccaatgg cgaccaagat ctcagtcgaa gaggaactag 2820
   ataggctaat acccgttctt gaaaagattc ttgaattacc tgaaattgaa ggaaagttgt 2880
   tgtctgtgga cacattttac tcggaagttg cttcagaggc aatcaagaaa ggggctcata 2940
   tggtcaatga tgtatcgggt ggaatgttag attctgatat gcttcatgtt gtggctgatc 3000
   taaatgttcc atatatcact atgcacatga gaggggaccc atccacaatg caaaacagtg 3060
   agaatttgaa gtatgatgat gttgtcaaag aagttgggga ggaattgtat gaacgtgtaa 3120
   ggaatgcaga gttatgtggt gttcccgcat ggaggatggt tcttgatcca gggatcgggt 3180
   tttcaaagaa aaccgaagat aatttggaga tattgatggg actaaagagg tttagaagtg 3240
   agattggaca aaagagctta ggggtgtctc gtgcaccttt gttaatcgga ccttcaagaa 3300
   aaaggttttt gggtgagatt tgtggtcggc cttctgctgt tgagagagat ccagggactg 3360
   tagctgctgt taccagtgcg attttgggtg gtgccaatat cgttagggtt cataacattg 3420
   gacataatgt ggatgctgtt aagctttgtg attcaatgtt ggatcgagct ggtagatctt 3480
   agtagcttaa agttatttga catcttgttt tttattcaat tttctggaca ttactttttg 3540
   tatttctgtc tggcaaatga ttttacaggg atatcctaga ggctaaattg tatattacat 3600
   tcttgatttt ctcacaaagt tttacttttt cagtttttct tgttcagttt tgtttttgaa 3660
   aatttgctag atcagattaa atcgttgctt tatgatcttc gtgtcttttt tgggacgctg 3720
   cgagtttcat ttagtggtat tgtaaactcc ttttaaagtt tgtacacact tggtatttat 3780
   gcttctagtt ttcgtctatg tctgtcctat attgtgtata gtatgtttaa agttcataaa 3840
   aagaggataa acgaatagca cgtaaatctt ataattcgat tccacagagt gcaagttatg 3900
   ctttcctaaa tgaaagcaag agtctagaca gtttggtcaa cgttaacatg agactattgt 3960
   agaatacaac accacaagaa accaaaactg tcttgtgcat ttctctcttt tccaggttta 4020
   aaactcaata ccaagacttg atatcaacta ttattctgct tcaatgattc agcttcagct 4080
   atagccatag gatcatcttt ggtgtcaggt tcatccttct caccatctcg ttcctctgcc 4140
   agtttttttc tgtgcaattc ctctgcaact tgctttgatg cttggtcttc tttttgaagg 4200
   cgttgcatct cttcttcttg ttgctgtcgt tcaaaccatg tatccgcatc ggcccaaact 4260
   gtaatttaga gccaaaacaa catgattaga atgcagattc cagaagccaa attcaaactg 4320
   taagaaaaaa aatgtagtga tgatttaact caaaacaact cacacagctc caaaatggat 4380
   gggggttcaa attagaggat agcctaatag atagaagctt ctaattatgg ggaaaaaaaa 4440
   acgatcatgg caatgtatta cacaatctaa agcaaccact atgaataata aagataaatc 4500
   tttccgagag tccacatgcc ttgatatttc ataagatagg ctttttcaga aatcagaatc 4560
   ttcttggcaa atataacact aaaatcagtc taatgcatgt tattaagtaa atctttattt 4620
   gtccatagta taatgccata gattatcgtc aattatccac atcgtcatta aatatcaagt 4680
   aaccacatta tgaattgact tgtttaatca aagttcatct tacttcttca tagatgaaaa 4740
   agagctatgc aaatacagaa cacatcagtt gttctaacat ataattactg ctagctgtag 4800
   gaagaaacca agatgaaact tatctaggat tcaaattcat ggggatctgt tatttagatg 4860
   aatttagaga aataagaaaa taagttgagt gaagtcagtt tcgaggttcc aaatttgtct 4920
   ttattagttg agctaacaat attaacctct gaagcatgta tagtgtactt gtatgaacca 4980
   cttaaggata taaatgaagc caaatcagtt tcattcgcta attgagcaaa tgcacgttct 5040
   gtttgttagt attgaaattt ctttacagga tttctttcaa ctaaaagcat cacatgtccg 5100
   actctcggtt tgatatttgt gacaagtcta attggttcat cagatgtact tatggctaaa 5160
   tttcgaaact tgaaacaata agagcttagc actcttaacc ataaagggtt gctgttaatc 5220
   ttacttgttc ttgaaagcaa ttccacagtg aagcatttgt taattgggaa gttttgcaat 5280
   gtttctaaag cttcgatgat attttatatt ttcaaacatt tttcatcaca tgtgttaaca 5340
   aaagccataa gaataccggg gtaatcaact aaagtctatt taaagttgca tgctcgagat 5400
   caagaaaaaa ccatcaaatt tacataaact gaagggcaaa tgtacg 5446
<210> 32
   <211> 1401
   <212> DNA
   <213> Conyza canadensis
<400> 32
   tcgtcctcgg atacttcaat acaagtgcat tctccagaac aagaagtagt aattgcttta   60
   ggtagcaatg tgggtaatag acttaataac tttaatgaag ccttatccca gatgaagaaa  120
   tcggggatag aaataacaag gcatgcttgt ctatacgaaa ctgaaccagc ttatgtgacg  180
   gaccagcctc tttttctcaa ctctgccgtt agagccacta caaagcttgg ccctcatgag  240
   ctactgtcca ttctcaagaa aattgaaaag gaaatgggtc gaaccaaagg gcttaggtac  300
   ggcccacgac ccattgacct agatatattg ttctatggta aatgcaaagt taactctgat  360
   attctaactg ttcctcatga aagaatcttt gagaggccat tcgttatggc tccattagtt  420
   gacttgttag gatcagatgt agacaatgat acggttctat gctggcattc tttttcaaaa  480
   aatgggcttt ttggatcttg ggaaacatta ggtggtgaat cttccatagg aaaagatggt  540
   ttaagaaggg ttttacctgt caatgatcgt ttatgggatt ggtcaaagaa aacttccgtc  600
   atgggtattt tgaatataac tcctgatagt tttagtgatg gagggaagtt tgattccatg  660
   gggtccgctt tatctcgtgt tcagaccatg atatctgaag ggtttgacat aattgatcta  720
   ggagctcaat ccacacgccc aatggcgacc aagatctcag tcgaagagga actagatagg  780
   ctaatacccg ttcttgaaaa gattcttgaa ttacctgaaa ttgaaggaaa gttgttgtct  840
   gtggacacat tttactcgga agttgcttca gaggcaatca agaaaggggc tcatatggtc  900
   aatgatgtat cgggtggaat gttagattct gatatgcttc atgttgtggc tgatctaaat  960
   gttccatata tcactatgca catgagaggg gacccatcca caatgcaaaa cagtgagaat 1020
   ttgaagtatg atgatgttgt caaagaagtt ggggaggaat tgtatgaacg tgtaaggaat 1080
   gcagagttat gtggtgttcc cgcatggagg atggttcttg atccagggat cgggttttca 1140
   aagaaaaccg aagataattt ggagatattg atgggactaa agaggtttag aagtgagatt 1200
   ggacaaaaga gcttaggggt gtctcgtgca cctttgttaa tcggaccttc aagaaaaagg 1260
   tttttgggtg agatttgtgg tcggccttct gctgttgaga gagatccagg gactgtagct 1320
   gctgttacca gtgcgatttt gggtggtgcc aatatcgtta gggttcataa cattggacat 1380
   aatgtggatg ctgttaagct t                                            1401
<210> 33
   <211> 1262
   <212> DNA
   <213> Conyza canadensis
<400> 33
   gaaataacaa ggcatgcttg tctatacgaa actgaaccag cttatgtgac ggaccagcct   60
   ctttttctca actctgccgt tagagccact acaaagcttg gccctcatga gctactgtcc  120
   attctcaaga aaattgaaaa ggaaatgggt cgaaccaaag ggcttaggta cggcccacga  180
   cccattgacc tagatatatt gttctatggt aaatgcaaag ttaactctga tattctaact  240
   gttcctcatg aaagaatctt tgagaggcca ttcgttatgg ctccattagt tgacttgtta  300
   ggatcagatg tagacaatga tacggttcta tgctggcatt ctttttcaaa aaatgggctt  360
   tttggatctt gggaaacatt aggtggtgaa tcttccatag gaaaagatgg tttaagaagg  420
   gttttacctg tcaatgatcg tttatgggat tggtcaaaga aaacttccgt catgggtatt  480
   ttgaatataa ctcctgatag ttttagtgat ggagggaagt ttgattccat ggggtccgct  540
   ttatctcgtg ttcagaccat gatatctgaa ggggttgaca taattgatct aggagctcaa  600
   tccacacgcc caatggcgac caagatctca gtcgaagagg aactagatag gctaataccc  660
   gttcttgaaa agattcttga attacctgaa attgaaggaa agttgttgtc tgtggacaca  720
   ttttactcgg aagttgcttc agaggcaatc aagaaagggg ctcatatggt caatgatgta  780
   tcgggtggaa tgttagattc tgatatgctt catgttgtgg ctgatctaaa tgttccatat  840
   atcactatgc acatgagagg ggacccatcc acaatgcaaa acagtgagaa tttgaagtat  900
   gatgatgttg tcaaagaagt tggggaggaa ttgtatgaac gtgtaaggaa tgcagagtta  960
   tgtggtgttc ccgcatggag gatggttctt gatccaggga tcgggttttc aaagaaaacc 1020
   gaagataatt tggagatatt gatgggacta aagaggttta gaagtgagat tggacaaaag 1080
   agcttagggg tgtctcgtgc acctttgtta atcggacctt caagaaaaag gtttttgggt 1140
   gagatttgtg gtcggccttc tgctgttgag agagatccag ggactgtagc tgctgttacc 1200
   agtgcgattt tgggtggtgc caatatcgtt agggttcata acattggaca taatgtggat 1260
   gc                                                                1262
<210> 34
   <211> 4643
   <212> DNA
   <213> Euphorbia heterophylla
<400> 34
<210> 35
   <211> 4556
   <212> DNA
   <213> Euphorbia heterophylla
<400> 35
   aaatataaat ataaatataa aaataaaaat aaattataat aatataaaat ataaaaatta   60
   atttaaacca tgttttaaaa atagtgttat gaaaaactcc gcttgggcgc cgtctagata  120
   gtttacttag gctgattttt aaaacaaaac cccccgctta acccgcctaa atcggtcaat  180
   tgtttatatc cttatgacta cttaaaatcg cagcggtgtt atggtgaatt atcatgtttt  240
   aatgagctta tgaaatgcaa taataaaaaa ataaccatta ataatttgat atacatttat  300
   cgggaaaaat gaaataataa aaaataaaaa aatatcaaaa aaatctataa aatatttgta  360
   cctttttcaa ttagtaccca ttttgtatat taaaacaata aaaatgatcc agattaataa  420
   atagaaaaag tattaataac ccgaaaatat caaatatcaa aatataatga aacatgaaca  480
   cgaaaacaca ataaagaagt tggtatcaca aacacaaaga cacccttaaa gctcctatgt  540
   ttgcggagag ttgtaagtcg actaatttca gattgaaaaa gttaaaaatg gccaactttt  600
   ttagctatga ggttagatta gatacgagaa tgcctttgtg aaaagctggt cactatttct  660
   tcattcgcgt ggcaattaac ccggcataat cgatagccaa ttcacggaca aggtcaaagg  720
   tatcagcttt actcaaccct tcgctttgat actaaattgc tgattcttat tctgattatg  780
   ctttatttcg ttacttcatt ttattgtcta tgcacataag ctgttcgaca aaaagcctga  840
   atgaaactgt gagtgctggt acgcggttac aatactcctg attatgtcca atgattattt  900
   gggtacttta ataccctaat ttgtgaagtt cactagcgaa agggtagttt cttccccggt  960
   tatttatcat tttatgaatc cccaagtctt tgaagtacag ctgattaaaa tgcaagtact 1020
   cataagttgt cattgtatgt ttgaaatcct tacgttgttg gtattttaca gaatttgtac 1080
   tgaagaagca taaattcata cctagttagg cttccttgtt cctaatatgc ttctcctcaa 1140
   gcggctggtg tccactaaac aagggttcaa taatgccatc aaccgttttg gaggtaatat 1200
   agttatgcct atgatgcttt aagtcgcttt tcatcgattt gttacattac caggctaaga 1260
   ttctgcaatt gaagttagaa atcgacgagt cttatcttga aaagtatgga ttttgttttg 1320
   tcaccctggg gccagttcaa gattgctttc ccaggttgct gtctcggcac cacatcctaa 1380
   tccaaaaccc taaggtgatg ggttaatggg tcccttcgca gttatatttc attcacttaa 1440
   ctcattagct tccatgtgga taatcaactc cacacttgcc ccaaaactgt ataaacgtag 1500
   attttgacct ttggaaatca ttagctggac aaatcctaac taattagtat caagagccaa 1560
   aatcttttga ccaagaccaa atatgttgtt tcattgttgc agcattgtca tcatcctttc 1620
   tccataccga accaaatagt tcggtggaag ttcattctca agagcaagaa gtagtaattg 1680
   cattaggaag caatgtggga gatagagttc ataatttcaa ccaagcattg cgattaatga 1740
   aaagttccgg cattaacgta actagacatg gttgtttgta ccagacagca cctgcttatg 1800
   tcactgacca acctcacttt ctcaactcag cagttcgagc tttcacgaaa ctcggacccc 1860
   acgagttatt aggagtttta aagcagatcg aaaaggactt gggccgtacc aaagggatta 1920
   ggtacggacc aaggccaatc gatttggata tacttttcta tggaaagttc cggattaatt 1980
   ccgatacact tattgttcct catgagagaa tatgggagag accctttgtg atggccccat 2040
   tggtggattt actcggctcg gaaatcgaga atgacacggt cgccagctgg cattccttgt 2100
   ccggcggtct ttttgaatca tgggaaaagt tgggcggcga aagcctgatc gggaaggacg 2160
   gaatgaaaag agttaccccg atcggaaacc atttatggga ttggtcggaa aagacttctg 2220
   taatgggaat tatcaattta acccccgata gtttcagcga tgggggcaag ttgacaacta 2280
   ttgattctat agtttctaag gttcgctcga tgatttccga aggggcggat attgtcgatt 2340
   ttggtgctca atcgacacgc cctatggcta aaaggatatc cccgcaagag gaaatggata 2400
   ggctaatccc tgtattggaa gcggttgtga aaatacctga gatgaccgga aagctcatat 2460
   cggtcgacac attttactcg gaagtcgcct tagaagcggt ccaaagagga gcaaatctcg 2520
   taaatgacgt atctgggggg caattagatc caaaaatgac ggaaaatgtc gctaatctcg 2580
   aggtaccata tatcttaatg cacatgaggg gggaccccac cacaatgcag aacactcaga 2640
   atctaaaata cgataatgtt tgtaaagagg ttgcctttga gttgttttcg agggttaaag 2700
   aagccgagat gtccggaatt ccagcctggc gaatgattat tgaccctgga attgggtttt 2760
   cgaagaacac gaatcagaat ctggaaatcc tgatgggaat tccaaggatc cgggccgaga 2820
   ttgggaggga aagcgtgggt atttctcgtg ggccgattct tattgggcct tcgagaaaga 2880
   ggtttttggg cgaaatttgc gaacgcccta aagcaactga aagagacccg gcaactgttg 2940
   cttctgttac tactgggatt ctgggagggg caaatattgt tagagttcat aatgttaggg 3000
   ataatgtgga tgctgtgaag ctatgcgact cgatgatgaa actaaagagg tctacttaaa 3060
   aagtatcggg tatagttcga tttctttctt tccgaatctt ttgtttgact ttcagattgt 3120
   ctttgattgg caaaacagtt tcggctgcgg ttactgttgt tgtttgttgt tgcagtttag 3180
   tgttgttgtt tctaattgtg attttaagtt attgttgtta gctgatttgt acttattgtt 3240
   tggtgaaaat ataattcacg gttgcggttg gaggtataaa ttactaaaat agacatattt 3300
   taaataaatt ataagttata ttgaagggta tttaggataa ataaaaatcc aaaccacaaa 3360
   aaaaaacccg taaaactatt gatttggtga ggtttggaaa aggagataaa tcccttataa 3420
   aaaaaacaac ttcatatatg ttttgaaact taaccaaaca cttgtatacc tccttttcct 3480
   tttcaaacct caaaagacct ttttatagta agctcttttg aagtctatac gctattgttt 3540
   ccgaatcatt ttggactttt catcgaaatc aacaatactg ctagctagtc atatttcaag 3600
   atatttggga tgtaaagatc aaggtagttg ttgttgtatt ctgttctcat cagcaaaatt 3660
   tgttataact ccaccaagat ttaatttttt ccaatctgaa aatgtcttat gttatagtaa 3720
   gaagtgagat gagtttatgt ctttgtgaaa agattttgcc caattaatgc aaaaattttg 3780
   tgacaagaca agaggggtga aagagacaag caacaagaga aagcagtgaa aggggcaaat 3840
   gttaggaaac tgttagctag aaaagggtag agggtggcct actttaccaa tttgagcatt 3900
   aaaaaagcac atggctatgt attttagacc aaagtcccta ttgttggcag atgaactaag 3960
   caaccatatg gttcattaac taactaacta gttattgcaa atttgactgt tccaattcat 4020
   caaaggactc tccccataga ccattcaaca cttgggttct agccctccct ctcaccctca 4080
   gacaaccctt gtgaacctca ttttcatcat tggtcccctt ttttgttagg actacacttg 4140
   tctctataaa atctcttcta gccatgtgtt atgtgtagac tttttcggtt tgaggattgg 4200
   gtcgggttcg ggctgggtct cgtcggactt tttactgcaa acatcaaact agggttctaa 4260
   acccatactt cagtactctg gttcggattg aacttgcgag atcattctaa attcaattta 4320
   cggctcatag attatatttg agagttttga gttttgggtg gattagattg aaaacgacta 4380
   ttttttgttg tataaatata tctaaaaagt tatataatta cattctttat tggaattcat 4440
   caatatatat aagaatttga ctttgttgta gccattttga aagttcgata agctactccc 4500
   ttcgtctagt ttcaatagtt catttaaaag tttcattaat tctgttttca attgtc 4556
<210> 36
   <211> 1425
   <212> DNA
   <213> Euphorbia heterophylla
<400> 36
   gcagcattgt catcatcctt tctccatacc gaaccaaata gttcggtgga agttcattct   60
   caagagcaag aagtagtaat tgcattagga agcaatgtgg gagatagagt tcataatttc  120
   aaccaagcat tgcgattaat gaaaagttcc ggcattaacg taactagaca tggttgtttg  180
   taccagacag cacctgctta tgtcactgac caacctcact ttctcaactc agcagttcga  240
   gctttcacga aactcggacc ccacgagtta ttaggagttt taaagcagat cgaaaaggac  300
   ttgggccgta ccaaagggat taggtacgga ccaaggccaa tcgatttgga tatacttttc  360
   tatggaaagt tccggattaa ttccgataca cttattgttc ctcatgagag aatatgggag  420
   agaccctttg tgatggcccc attggtggat ttactcggct cggaaatcga gaatgacacg  480
   gtcgccagct ggcattcctt gtccggcggt ctttttgaat catgggaaaa gttgggcggc  540
   gaaagcctga tcgggaagga cggaatgaaa agagttaccc cgatcggaaa ccatttatgg  600
   gattggtcgg aaaagacttc tgtaatggga attatcaatt taacccccga tagtttcagc  660
   gatgggggca agttgacaac tattgattct atagtttcta aggttcgctc gatgatttcc  720
   gaaggggcgg atattgtcga ttttggtgct caatcgacac gccctatggc taaaaggata  780
   tccccgcaag aggaaatgga taggctaatc cctgtattgg aagcggttgt gaaaatacct  840
   gagatgaccg gaaagctcat atcggtcgac acattttact cggaagtcgc cttagaagcg  900
   gtccaaagag gagcaaatct cgtaaatgac gtatctgggg ggcaattaga tccaaaaatg  960
   acggaaaatg tcgctaatct cgaggtacca tatatcttaa tgcacatgag gggggacccc 1020
   accacaatgc agaacactca gaatctaaaa tacgataatg tttgtaaaga ggttgccttt 1080
   gagttgtttt cgagggttaa agaagccgag atgtccggaa ttccagcctg gcgaatgatt 1140
   attgaccctg gaattgggtt ttcgaagaac acgaatcaga atctggaaat cctgatggga 1200
   attccaagga tccgggccga gattgggagg gaaagcgtgg gtatttctcg tgggccgatt 1260
   cttattgggc cttcgagaaa gaggtttttg ggcgaaattt gcgaacgccc taaagcaact 1320
   gaaagagacc cggcaactgt tgcttctgtt actactggga ttctgggagg ggcaaatatt 1380
   gttagagttc ataatgttag ggataatgtg gatgctgtga agcta                 1425
<210> 37
   <211> 1425
   <212> DNA
   <213> Euphorbia heterophylla
<400> 37
   gcagcattgt catcatcctt tctccatacc gaaccaaata gttcggtgga agttcattct   60
   caagagcaag aagtagtaat tgcattagga agcaatgtgg gagatagagt tcataatttc  120
   aaccaagcat tgcaattaat gaaaagttcc ggcattaaca taactagaca tggttgtttg  180
   taccagacag cacctgctta tgtcactgac caacctcact ttctcaactc agcagttcga  240
   gctttcacga aactcggacc ccacgagtta ttaggagttt taaagcagat cgaaaaggac  300
   ttgggccgta ccaaagggat taggtacgga ccaaggccaa tcgatttgga tatacttttc  360
   tatggaaagt tccggattaa ttccgataca cttattgttc ctcatgagag aatatgggag  420
   agaccctttg tgatggcccc attggtggat ttactcggct cggaaatcga gaatgacacg  480
   gtcgccagct ggcattcctt gtccggcggt ctttttgaat catgggaaaa gttgggcggc  540
   gaaagcctga tcgggaagga cggaatgaaa agagttaccc cgatcggaaa ccatttatgg  600
   gattggtcgg aaaagacttc tgtaatggga attatcaatt taacccccga tagtttcagc  660
   gatgggggca agttgacaac tattgattct atagtttcta aggttcgctc gatgatttcc  720
   gaaggggcgg atattgtcga ttttggtgct caatcgacac gccctatggc taaaaggata  780
   tccccgcaag aggaaatgga taggctaatc cctgtattgg aagcggttgt gaaaataccc  840
   gagatgaccg aaaagctcat atcggtcgac acattttact cggaagtcgc cttagaagcg  900
   gtccaaagag gagcaaatct cgtaaatgac gtatctgggg ggcaattaga tccaaaaatg  960
   acggaaaatg tcgctaatct cgaggtacca tatatcttaa tgcacatgag gggggacccc 1020
   accacaatgc agaacactca gaatctaaaa tacgataatg tttgtaaaga ggttgccttt 1080
   gagttgtttt cgagggttaa agaagccgag atgtccggaa ttccagcctg gcgaatgttt 1140
   attgaccctg gaattgggtt ttcgaagaac acgaatcaga atctggaaat cctgatggga 1200
   attccaagga tccgggccga gattgggagg aaaagcgtgg gtatttctcg tgggccgatt 1260
   cttattgggc cttcgagaaa gaggtttttg ggcgaaattt gcgaacgccc tgaagcaact 1320
   gaaagagacc cggcaactgt tgcttctgtt actactggga ttctgggagg ggcaaatatt 1380
   gttagagttc ataatgttag ggataatgtg gatgctgtga agcta                 1425
<210> 38
   <211> 681
   <212> DNA
   <213> Euphorbia heterophylla
<400> 38
   ttaaagcaga tcgaaaagga cttgggccgt accaaaggga ttaggtacgg accaaggcca   60
   atcgatttgg atatactttt tctatggaaa gttccggatt aattccgata cacttattgt  120
   tcctcatgag agaatatggg agagaccctt tgtgatggcc ccattggtgg atttactcgg  180
   ctcggaaatc gagaatgaca cggtcgccag ctggcattcc ttgtccggcg gtctttttga  240
   atcatgggaa aagttgggcg gcgaaagcct gatcgggaag gacggaatga aaagagttac  300
   cccgatcgga aaccatttat gggattggtc ggaaaagact tctgtaatgg gaattatcaa  360
   tttaaccccc gatagtttca gcgatggggg caagttgaca actattgatt ctatagtttc  420
   taaggttcgc tcgatgattt ccgaaggggc ggatattgtc gattttggtg ctcaatcgac  480
   acgccctatg gctaaaagga tatccccgca agaggaaatg gataggctaa tccctgtatt  540
   ggaagcggtt gtgaaaatac ccgagatgac cgaaaagctc atatcggtcg acacatttta  600
   ctcggaagtc gccttagaag cggtccaaag aggagcgaat ctcgtaaatg acgtatctgg  660
   ggggcaatta gatccaaaaa t                                            681
<210> 39
   <211> 444
   <212> DNA
   <213> Euphorbia heterophylla
<400> 39
   tctggggagt gatggttgga atagatgctt attcctggca gagatcgaaa tgctccattt   60
   tagtttcggg ttttgttaaa atggggattt gttaaattgt ttcaatggat aattcactgt  120
   tgtattagac ttgatattgt tagcagacac tgatttttat gtattcgtga actgaaaagg  180
   ttgattttct tccatgtttt gtctgattta tgatggaact tcatttctta gtggttaatg  240
   ttggtatttc cttctgttgt ttgatttgta tctgtaattg gtaccatgtt atatgcaatc  300
   ttttaaaaaa tgttttaaaa agcgctatac gtggcagctg agactgttga gacttgaaaa  360
   cgcacgggca tgtgcggggt ttaggcggat ttgtattttt tattttttaa aatatttttt  420
   aatatataaa aatataaata taaa                                         444
<210> 40
   <211> 379
   <212> DNA
   <213> Euphorbia heterophylla
<400> 40
   gttatgatga aagtataagt tgctaatcaa gaagaaagag attttaatgt aattatttaa   60
   ataataaatt cctaccattt taattgtgtg acaattggtt ataatgattc cgttgcatcg  120
   acaactaaaa ctagatagaa ggatcaaaat ataaaatttt aacataatta atacatataa  180
   tatataaatg tataatagat acaattatta ttcctcgaat taaaattggc taattatata  240
   ataatataat ccaacctata aaaacttttg agcttttaag cttgagatga gctttgtaaa  300
   aatgattgta caagcccgac catgaaagag agccaagtag ggtcaaatgc ataccctggc  360
   ttgaatataa attcaatat                                                 379
<210> 41
   <211> 4031
   <212> DNA
   <213> Digitaria sanguinalis
<400> 41
   aaatcgtaaa atttataact aacatgttcc ttcatgaatc caaaagccaa gtaaacaggt   60
   ccatgagtgc ctttgtgaga ttgcttcgaa tagaatatac ctacggctct taatttcaga  120
   ttctcttagg tagtgtttag gtagcgtttg gaagtagata tttaggcttg gaattaggaa  180
   ttggcattga gatctacgaa tacctgccgt ttggatgtct aagaatttgg aattggaatt  240
   gcagcccaat accaaccggc attccttggc ctctcatctg cctgccctca gtatagatag 300
   agcttatctc cttcatcctc gcggagtggt ggcaagcggc ggcgagatcc ggaccacatg 360
   gggtgtggga gcgcggtggc agagtggtgg caagcgatgg cgacggcacc gtgccgccgt 420
   gccacattgg cacggcgacg gacgagtttg gatggtgtca gtacagacca ctgcccaatt 480
   ctatcctttt gtgcatccaa acataaaatc tgaattagga tgctcttttg attctacaag 540
   gcaatgtaat gaccatccaa acaacagatt ctgaattgga gtcaattcaa tgttacgatt 600
   gtattggcat cggacctaat tcaatgtcat gccctctagt atctacattc aaacggagcg 660
   gaaaaatcta cctccgtccc aagattccgg cccaagatcc cgactcttaa tacttttttc 720
   atctttcgct gaaatttcag cccaagatcc cggcccgcct ttttgcctcc acgctgggcc 780
   tggtgggacg cggccccttc acgagagtcg agaggggaaa gcggaaaggg aaacaaaaaa 840
   actcaacgaa tcgccgccgc cgccttgccc tgcagcgaac aattaagggc cccgtacctc 900
   tctctgcccg ttcttccgct gcactccgca tctgagcgga cgggtgggga caccgccgac 960
   ggcgccgggg tggcgcctcc gtctgtgctc caaccaaggc agtgggtgct ggttgctgcc 1020
   aatcgaccgg ccgggcatgc tctgaccatc gagaaaatct tcgggtaaca caacgttgtt 1080
   catttcgttt gtatcgtggc atagttgatt tccctaactt ttaggatgcg tttcgattca 1140
   ttgtatctgt ataccaaaac agagttcgat aacttagatt gaacggtagg cttcagcatc 1200
   taaggcagca ggtgcaggtc tattctatca ctctgaaaat tagttttaac tgtctgatga 1260
   taggaggatt agctaatgtt gttatatgag gaggtttctt gctgactaga gagctatttg 1320
   accacctgat agattgcctc gttagcaact tagcattatg acgacgatgg cagcgagcca 1380
   tttcatgtag aaaacgaaca tgagctgaga agcttaatcg tataagcttc aattggcttc 1440
   ctggctgggg aggtttgcta gagaacttct gtattttgtt cattcattta atctgatgta 1500
   tgttggcagc tctcctgtca ggtacagaaa tttagacggc cctgaaacca agcagggcaa 1560
   cctcaccaat aggggggcag tcgcaccaca aaggttgctt gcctccagtg gggcatctcc 1620
   tctccttaca ttgagaggtt tatttgacgt ctaagatatc tgaactcctg cctccagtta 1680
   gagaaaaggc ccttgttcat acctcaaatt tgtctttgct cttctattga ccaggtgtga 1740
   tggagtgatg cttgcgtgaa atatcctaca aggctacaat ttatttgaag catttgcttt 1800
   cttaattatc atatgttcaa cgatgctcct ccatgctaag gagtccctta agaagatgta 1860
   tcctgttgct atgaactact ttggagggct cctcccatcg cattcgtttt caggtactgt 1920
   tcaaatcttt tcttttcaaa tcccctatca tgcgatcaag agcagctcta agcttctaac 1980
   tcaccccttt gcatttctaa acagtgtctg atcttgttca tccgctgagg ttcccaaaca 2040
   gagctcctag acatgctatt ccattcaggg ctcgttcgtt tacgcgatgt tcgcttgagg 2100
   gatgttcaac tgaccaagag attgtgattg ctatgggaag caacgtgggt gatggagtca 2160
   gtacatttga cagggcatta caaatgatga aaagctcagg cgtgaacatc actaggcatg 2220
   cctgtctata tgagactgcc cctgcttatg tgaccgatca gccacggttc ctgaactctg 2280
   ccattcgagg tacgactagg ctgggacctc acgagttgct taagaaacta aaggaaattg 2340
   agaaggatat aggacgcact ggtggaataa ggtatggccc aagaccaatc gatctagata 2400
   tacttctgta cggcaattcc cagattgata ctgaggctct aattgtgcca catgaacgca 2460
   tccaggagag gccatttgtt ctagcacctc ttgttgacct tctaggtgca tctggcaatg 2520
   atagtgtcga aacaagctgg cactctcttt caaagtgcag tggtggtttc tttgaattat 2580
   ggaataaact tgggggtgaa tctataattg gaacagaaag tattaaaagg gtattacctg 2640
   ttgggaatca tttgtttgat tggtgtgaga gaaccctcgt catgggggtc cttaatctaa 2700
   caccagacag ctttagtgat ggaggtaagt ttcaacaagt ggaagctgct atttctcagg 2760
   ctaaattatt aatttcagaa ggtgcagata tcatagatat tggtgctcaa tctaccaggc 2820
   cttttgcaaa gagattatct ccaaacgaag aaattgaaag attggttcct gttctggatg 2880
   tgattacgaa aattcctgaa atggagggaa agttgctctc agtggataca ttctatgcag 2940
   aagttgcatg tgaagctgtg aaaagaggag ttcacatgat caatgatgta tctggtggac 3000
   agcttgaccc caaaattctt aaagttgctg ctgaactcaa agttccgtat gttgcaatgc 3060
   acatgagggg agatccatca acaatgcaaa gtgaacaaaa tttacagtat gacgatgtct 3120
   gcaaggaagt tgcttcagag ctatatgcac aggtgagaga agcagagtta tctgggattc 3180
   cattgtggag gatagttcta gatccaggca ttgggttctc caagaaatcc aaacataacc 3240
   ttgaagtaat tatgggattg gaatccatta ggagggagat gagtaaaatg agtattggtg 3300
   cttcacatgt gccaatatta ctgggaccct ctaggaaaag atttttgggt gaaatatgca 3360
   atcgtgaaaa tccagttgag agagatgttg ctactgttgc agctgtgacg gcgggtattt 3420
   tgaatggtgc taacatagta agggtacata atgcaggata cagttcagat gctgcaaagt 3480
   tttgtgatgc attgaataag agaagaagaa tggaagactg aaccagctga tcaaaaagat 3540
   accgagctct gattttattc gagaaaatgg tgatgcagga tagttactct gctgctcaat 3600
   gggattctca tattacatca tttgtggagt attgtttttg taataaataa accagggatg 3660
   acgtttttcc tgtgtcatct cctatctagt ttctagatac ttcaatgaag catggttcaa 3720
   ctcagatcac ttggaaagag tagtcaagga ataaggttac agaatcagtt agtaattaca 3780
   gattcttacc tcttgtggtc ctattttaga tgattttcca gaacattttc tttccaattt 3840
   aataatatca gaagggccta atttgtgtgt atcttttcta gtccagacat atttgataag 3900
   ttggggtttt acctgcttct ccctttacct ttgtattttg cactttgttt gagctgtgca 3960
   tcattgtatt agaggaagtg tattttgtac gttggtatac ccaacgagga aacctcatgt 4020
   tttgcaagtg c 4031
<210> 42
   <211> 1428
   <212> DNA
   <213> Digitaria sanguinalis
<400> 42
   gctcgttcgt ttacgcgatg ttcgcttgag ggatgttcaa ctgaccaaga gattgtgatt 60
   gctatgggaa gcaacgtggg tgatggagtc agtacatttg acagggcatt acaaatgatg 120
   aaaagctcag gcgtgaacat cactaggcat gcctgtctat atgagactgc ccctgcttat 180
   gtgaccgatc agccacggtt cctgaactct gccattcgag gtacgactag gctgggacct 240
   cacgagttgc ttaagaaact aaaggaaatt gagaaggata taggacgcac tggtggaata 300
   aggtatggcc caagaccaat cgatctagat atacttctgt acggcaattc ccagattgat 360
   actgaggctc taattgtgcc acatgaacgc atccaggaga ggccatttgt tctagcacct 420
   cttgttgacc ttctaggtgc atctggcaat gatagtgtcg aaacaagctg gcactctctt 480
   tcaaagtgca gtggtggttt ctttgaatta tggaataaac ttgggggtga atctataatt 540
   ggaacagaaa gtattaaaag ggtattacct gttgggaatc atttgtttga ttggtgtgag 600
   agaaccctcg tcatgggggt ccttaatcta acaccagaca gctttagtga tggaggtaag 660
   tttcaacaag tggaagctgc tatttctcag gctaaattat taatttcaga aggtgcagat 720
   atcatagata ttggtgctca atctaccagg ccttttgcaa agagattatc tccaaacgaa 780
   gaaattgaaa gattggttcc tgttctggat gtgattacga aaattcctga aatggaggga 840
   aagttgctct cagtggatac attctatgca gaagttgcat gtgaagctgt gaaaagagga 900
   gttcacatga tcaatgatgt atctggtgga cagcttgacc ccaaaattct taaagttgct 960
   gctgaactca aagttccgta tgttgcaatg cacatgaggg gagatccatc aacaatgcaa 1020
   agtgaacaaa atttacagta tgacgatgtc tgcaaggaag ttgcttcaga gctatatgca 1080
   caggtgagag aagcagagtt atctgggatt ccattgtgga ggatagttct agatccaggc 1140
   attgggttct ccaagaaatc caaacataac cttgaagtaa ttatgggatt ggaatccatt 1200
   aggagggaga tgagtaaaat gagtattggt gcttcacatg tgccaatatt actgggaccc 1260
   tctaggaaaa gatttttggg tgaaatatgc aatcgtgaaa atccagttga gagagatgtt 1320
   gctactgttg cagctgtgac ggcgggtatt ttgaatggtg ctaacatagt aagggtacat 1380
   aatgcaggat acagttcaga tgctgcaaag ttttgtgatg cattgaat 1428
<210> 43
   <211> 4007
   <212> DNA
   <213> Kochia scoparia
<400> 43
   gaatgtgctt aggcggctgc aatcagccag tttgatttgg aagggaattt ccaagtatga   60
   tagaggtaaa gcacagttgt ttcatttttt gactatatat ttaatttggg aatctgtact  120
   gtttcatttt gtaactttat tttgatcctg agttattgaa tctgtcatgt ctcattttga  180
   gacggtattt tgaatctgag tctggatatg aaattatgaa taggccatag gtatttataa  240
   atatagggtt taagttgatg atctggatgc tatcagagct ctattatgca ggattacaaa  300
   tcataatttc attgtgaaat ttacttcttt atgtgtattc acttgaagat tcataatttt  360
   gatgtattca taaatttgat gtgccctaaa cagaaagagt cagcatgtgc tgaaagtata  420
   agggggaaaa ccaagatgta taaatctttt ccttgatgag ttgcttatat gattgatgtc  480
   atgattctaa aatgccttta cctaattcaa atggatttta aagattacgt atgtactttt  540
   gcagcttcaa gtcttgcttt tttgcactca tcaccagaaa ctactattga ggtttgttct  600
   aaagagcatg aagttgtaat tgctctaggg agcaatgtag gagacagatt agataacttt  660
   aaccaagctc tgcagctaat gaagaaatta ggagtaaata tcacaaggca tggctgctta  720
   tacgagacag atcctgccta tgtgactgat caaccaaagt ttcttaactc tgcagtgagg  780
   ggcttcacaa aacttgggcc tctagaacta ttagggatgc tgaagaaaat tgagaaggat  840
   atgggtcgaa ctaatggaat aagatatggc cctaggccaa ttgacttaga cattcttttt  900
   tatgggaagt ttagggtaag ctctgagaaa cttacagttc cgcatgaaag gatatgggaa  960
   agaccctttg tgatggcacc actaattgat ataattggat cggatgtaga aaatgacact 1020
   gttgctgcat ggcattcatt atcaaaattt tctggtggac tatttgaagc gtgggataaa 1080
   cttggtggag attcactcat tgggaaggac ggaatgagta gggttttgcc agttgggaac 1140
   cacttgtggg attggtcgtg caaaacctct gtaatgggag tcttgaattt gactccggac 1200
   agctttagtg atggaggaaa gtttctacct gtagaaaatg cagtttctca ggttcgtcaa 1260
   atgatctcag aaggtgctga cataattgat attggagcgc aatcaacaag gcccatggca 1320
   actaggattt ctgctgagga agagctggaa agactagtcc ctgttttaga aggtgtcaag 1380
   gatgttatcg aggaagaagg aagaatgtta tcagtggata cattttactc aaaagttgct 1440
   tctgaagctg tcaataaggg agcacatatg gtgaatgatg tttcgagtgg aaagctagat 1500
   cctgagatgt tcaatgttgt tgcagggctt aaagtgcctt atatagcaat gcacatgcga 1560
   ggtgatcctt cttcaatgca aaatgctgat aatttgacat acaatgatgt ttgtaaggag 1620
   gtggctttag agttgagctc taggattaca gatgcagaat tatcaggtat tcctgcttgg 1680
   aggatagtta ttgatcctgg cattggattt tccaagaata caaagcaaaa tttggaaatt 1740
   ctcacagggt tgaaaagaat tcggcaagaa atagcaaaaa agagtttggc tgtggctcat 1800
   ggtcccttac tgattggacc ttcaagaaag aggtttttgg gtgagatctg caatcgtcct 1860
   gtagcatctg atagagatcc ggcaactgtt gcttctatca ccgctggagt tttgggtggt 1920
   gcaaacattg taagagtaca taatgttcgg gatagccttg atggtgtcaa gctatgtgat 1980
   gcaatgcttg aatatcagta taactgaaga ttcttagatt cttagattct tagattctaa 2040
   gagttgattt tatagattat tgtctttgtt gtgtgccttc ggcagatatg aataatatga 2100
   ttgtaatttt gttgcttatg attttcttcc tacctttctt ttatcataga atttagatta 2160
   aaaaatgtac ttgaatttca taaatggtga aataaagttt gtcggtgctt catgcaatac 2220
   gacttttcta gtttgcattg ctctatcata ggggctgtaa gcagattagc ccagtcaatt 2280
   cacattagat ttagacggcc ggtgcaaaaa cagcataatg ttgctaacgc tgtttataca 2340
   tttaaactat atgatctcta agagcaaaat acaaaataat acaatgtgtt tcatgaagat 2400
   gtccaatcat ttctccctca aaaaaagatc gtgatgaaca atcaggtact ttgattttgt 2460
   tgtagaacct cagcttcagc cctagccatg ggatcatctt tttctccttc tcgctcctca 2520
   gccaatttct ttctgtgcat ctcctctgca gcttctattg aagctttgtg ctcagcctgg 2580
   agacgctgca tttcctcttc catctgctgt ctttcgaacc atgtatcagc atccgcccaa 2640
   actgcagaaa taatttaaac aagttaatca tacggagtgt gcagcatggc aaattggcaa 2700
   taagatggtg caagtaacaa gctcaaatga tccaccatag ccaaattcct aaccattgga 2760
   ctgtgtcata tactcatatc agctagtaga aacattctca ccataaccct aactattcga 2820
   ggataagtga taccattcaa caatagtaaa gatcaaatca ggtattacta gacctatcaa 2880
   agtaatacgg agtagttgtt attgttacag agttaggaag aatcctattt tcccatttct 2940
   cctatcaatc ctaaactttt gtgactgaac tatcgacata tagtgtgaag aacatgaatg 3000
   gcagaagtca caactctgat tcctgagtcc tgactataaa ctattggatg actgtgatac 3060
   atcattagtc ctttaagcat tgagttactc tattctacat agttaaatta tatagattcc 3120
   aagatccgaa tccatgaatt atcaattacc ataaattgat cttcttcctt tgagttttgt 3180
   caacagttcc tactccgaaa tatgtcttaa atctagaagc aatagacact tatcaatgat 3240
   acagcaatag acgaattcat ctacccaagg cagcatcaac aacagctgcc gtttgcgata 3300
   atcgaaacta tcatagtgtc cttttcacta ttagtgcaga tgttatctag ggtctaaacc 3360
   tctatgtttc tacattcctg gaaaatctta ttctgtactg cataatcctc tcacaaaaaa 3420
   ttcgccaatg aatgacaaat tagctatcca gaattgcaga ttgattgaat caaattgatc 3480
   atggcagcag cacaattgca gcagttatca attgaagcac aatattacct ctcaatcata 3540
   acaatcaaag cacagaaatt ccaatcaata actccacaat atagtatcaa tctctaactc 3600
   agaaactaaa cagaagcaaa aacccattca atcaaagcaa cagtaaaatt agggtttgaa 3660
   ttagaaagaa agagagggaa gagaaacata ctgggttgcg cagcccatcc ctgttcacca 3720
   cccttaatct tctcgggaag agcgtaattg acagtgagaa cacgaccgta aagctcagca 3780
   ccatccatgt tatccatggc agaagaagcg tcgtctctct ccaagaaagt gacgaaaccg 3840
   aaagaacgat gcttgttggt ggccaaatca agaggggttt tgacgtcttt gatttctcca 3900
   aaagggatga aggcagcatg aaggattgat tcgttgactt cttctgctaa tcctcctacg 3960
   tataatgtgt tcttctgcat tccttgttga ttcgtcgcca tttttcg 4007
<210> 44
   <211> 3505
   <212> DNA
   <213> Kochia scoparia
<400> 44
   taattcaaat ggattttaaa gattacgtat gtacttttgc agcttcaagt cttgcttttt 60
   tgcactcatc accagaaact actattgagg tttgttctaa agagcatgaa gttgtaattg 120
   ctctagggag caatgtagga gacagattag ataactttaa ccaagctctg cagctaatga 180
   agaaattagg agtaaatatc acaaggcatg gctgcttata cgagacagat cctgcctatg 240
   tgactgatca accaaagttt cttaactctg cagtgagggg cttcacaaaa cttgggcctc 300
   tagaactatt agggatgctg aagaaaattg agaaggatat gggtcgaact aatggaataa 360
   gatatggccc taggccaatt gacttagaca ttctttttta tgggaagttt agggtaagct 420
   ctgagaaact tacagttccg catgaaagga tatgggaaag accctttgtg atggcaccac 480
   taattgatat aattggatcg gatgtagaaa atgacactgt tgctgcatgg cattcattat 540
   caaaattttc tggtggacta tttgaagcgt gggataaact tggtggagat tcactcattg 600
   ggaaggacgg aatgagtagg gttttgccag ttgggaacca cttgtgggat tggtcgtgca 660
   aaacctctgt aatgggagtc ttgaatttga ctccggacag ctttagtgat ggaggaaagt 720
   ttctacctgt agaaaatgca gtttctcagg ttcgtcaaat gatctcagaa ggtgctgaca 780
   taattgatat tggagcgcaa tcaacaaggc ccatggcaac taggatttct gctgaggaag 840
   agctggaaag actagtccct gttttagaag gtgtcaagga tgttatcgag gaagaaggaa 900
   gaatgttatc agtggataca ttttactcaa aagttgcttc tgaagctgtc aataagggag 960
   cacatatggt gaatgatgtt tcgagtggaa agctagatcc tgagatgttc aatgttgttg 1020
   cagggcttaa agtgccttat atagcaatgc acatgcgagg tgatccttct tcaatgcaaa 1080
   atgctgataa tttgacatac aatgatgttt gtaaggaggt ggctttagag ttgagctcta 1140
   ggattacaga tgcagaatta tcaggtattc ctgcttggag gatagttatt gatcctggca 1200
   ttggattttc caagaataca aagcaaaatt tggaaattct cacagggttg aaaagaattc 1260
   ggcaagaaat agcaaaaaag agtttggctg tggctcatgg tcccttactg attggacctt 1320
   caagaaagag gtttttgggt gagatctgca atcgtcctgt agcatctgat agagatccgg 1380
   caactgttgc ttctatcacc gctggagttt tgggtggtgc aaacattgta agagtacata 1440
   atgttcggga tagccttgat ggtgtcaagc tatgtgatgc aatgcttgaa tatcagtata 1500
   actgaagatt cttagattct tagattctta gattctaaga gttgatttta tagattattg 1560
   tctttgttgt gtgccttcgg cagatatgaa taatatgatt gtaattttgt tgcttatgat 1620
   tttcttccta cctttctttt atcatagaat ttagattaaa aaatgtactt gaatttcata 1680
   aatggtgaaa taaagtttgt cggtgcttca tgcaatacga cttttctagt ttgcattgct 1740
   ctatcatagg ggctgtaagc agattagccc agtcaattca cattagattt agacggccgg 1800
   tgcaaaaaca gcataatgtt gctaacgctg tttatacatt taaactatat gatctctaag 1860
   agcaaaatac aaaataatac aatgtgtttc atgaagatgt ccaatcattt ctccctcaaa 1920
   aaaagatcgt gatgaacaat caggtacttt gattttgttg tagaacctca gcttcagccc 1980
   tagccatggg atcatctttt tctccttctc gctcctcagc caatttcttt ctgtgcatct 2040
   cctctgcagc ttctattgaa gctttgtgct cagcctggag acgctgcatt tcctcttcca 2100
   tctgctgtct ttcgaaccat gtatcagcat ccgcccaaac tgcagaaata atttaaacaa 2160
   gttaatcata cggagtgtgc agcatggcaa attggcaata agatggtgca agtaacaagc 2220
   tcaaatgatc caccatagcc aaattcctaa ccattggact gtgtcatata ctcatatcag 2280
   ctagtagaaa cattctcacc ataaccctaa ctattcgagg ataagtgata ccattcaaca 2340
   atagtaaaga tcaaatcagg tattactaga cctatcaaag taatacggag tagttgttat 2400
   tgttacagag ttaggaagaa tcctattttc ccatttctcc tatcaatcct aaacttttgt 2460
   gactgaacta tcgacatata gtgtgaagaa catgaatggc agaagtcaca actctgattc 2520
   ctgagtcctg actataaact attggatgac tgtgatacat cattagtcct ttaagcattg 2580
   agttactcta ttctacatag ttaaattata tagattccaa gatccgaatc catgaattat 2640
   caattaccat aaattgatct tcttcctttg agttttgtca acagttccta ctccgaaata 2700
   tgtcttaaat ctagaagcaa tagacactta tcaatgatac agcaatagac gaattcatct 2760
   acccaaggca gcatcaacaa cagctgccgt ttgcgataat cgaaactatc atagtgtcct 2820
   tttcactatt agtgcagatg ttatctaggg tctaaacctc tatgtttcta cattcctgga 2880
   aaatcttatt ctgtactgca taatcctctc acaaaaaatt cgccaatgaa tgacaaatta 2940
   gctatccaga attgcagatt gattgaatca aattgatcat ggcagcagca caattgcagc 3000
   agttatcaat tgaagcacaa tattacctct caatcataac aatcaaagca cagaaattcc 3060
   aatcaataac tccacaatat agtatcaatc tctaactcag aaactaaaca gaagcaaaaa 3120
   cccattcaat caaagcaaca gtaaaattag ggtttgaatt agaaagaaag agagggaaga 3180
   gaaacatact gggttgcgca gcccatccct gttcaccacc cttaatcttc tcgggaagag 3240
   cgtaattgac agtgagaaca cgaccgtaaa gctcagcacc atccatgtta tccatggcag 3300
   aagaagcgtc gtctctctcc aagaaagtga cgaaaccgaa agaacgatgc ttgttggtgg 3360
   ccaaatcaag aggggttttg acgtctttga tttctccaaa agggatgaag gcagcatgaa 3420
   ggattgattc gttgacttct tctgctaatc ctcctacgta taatgtgttc ttctgcattc 3480
   cttgttgatt cgtcgccatt tttcg 3505
<210> 45
   <211> 1446
   <212> DNA
   <213> Kochia scoparia
<400> 45
   gcagcttcaa gtcttgcttt tttgcactca tcaccagaaa ctactattga ggtttgttct 60
   aaagagcatg aagttgtaat tgctctaggg agcaatgtag gagacagatt agataacttt 120
   aaccaagctc tgcagctaat gaagaaatta ggagtaaata tcacaaggca tggctgctta 180
   tacgagacag atcctgccta tgtgactgat caaccaaagt ttcttaactc tgcagtgagg 240
   ggcttcacaa aacttgggcc tctagaacta ttagggatgc tgaagaaaat tgagaaggat 300
   atgggtcgaa ctaatggaat aagatatggc cctaggccaa ttgacttaga cattcttttt 360
   tatgggaagt ttagggtaag ctctgagaaa cttacagttc cgcatgaaag gatatgggaa 420
   agaccctttg tgatggcacc actaattgat ataattggat cggatgtaga aaatgacact 480
   gttgctgcat ggcattcatt atcaaaattt tctggtggac tatttgaagc gtgggataaa 540
   cttggtggag attcactcat tgggaaggac ggaatgagta gggttttgcc agttgggaac 600
   cacttgtggg attggtcgtg caaaacctct gtaatgggag tcttgaattt gactccggac 660
   agctttagtg atggaggaaa gtttctacct gtagaaaatg cagtttctca ggttcgtcaa 720
   atgatctcag aaggtgctga cataattgat attggagcgc aatcaacaag gcccatggca 780
   actaggattt ctgctgagga agagctggaa agactagtcc ctgttttaga aggtgtcaag 840
   gatgttatcg aggaagaagg aagaatgtta tcagtggata cattttactc aaaagttgct 900
   tctgaagctg tcaataaggg agcacatatg gtgaatgatg tttcgagtgg aaagctagat 960
   cctgagatgt tcaatgttgt tgcagggctt aaagtgcctt atatagcaat gcacatgcga 1020
   ggtgatcctt cttcaatgca aaatgctgat aatttgacat acaatgatgt ttgtaaggag 1080
   gtggctttag agttgagctc taggattaca gatgcagaat tatcaggtat tcctgcttgg 1140
   aggatagtta ttgatcctgg cattggattt tccaagaata caaagcaaaa tttggaaatt 1200
   ctcacagggt tgaaaagaat tcggcaagaa atagcaaaaa agagtttggc tgtggctcat 1260
   ggtcccttac tgattggacc ttcaagaaag aggtttttgg gtgagatctg caatcgtcct 1320
   gtagcatctg atagagatcc ggcaactgtt gcttctatca ccgctggagt tttgggtggt 1380
   gcaaacattg taagagtaca taatgttcgg gatagccttg atggtgtcaa gctatgtgat 1440
   gcaatg                                                            1446
<210> 46
   <211> 1446
   <212> DNA
   <213> Kochia scoparia
<400> 46
   gcagcttcaa gtcttgcttt tttgcactca tcaccagaaa ctactattga ggtttgttct   60
   aaagagcatg aagttgtaat tgctctaggg agcaatgtag gagacagatt agataacttt  120
   aaccaagctc tgcagctaat gaagaaatta ggagtaaata tcacaaggca tggctgctta  180
   tacgagacag atcctgccta tgtgactgat caaccaaagt ttcttaactc tgcagtgagg  240
   ggcttcacaa aacttgggcc tctagaacta ttagggatgc tgaagaaaat tgagaaggat  300
   atgggtcgaa ctaatggaat aagatatggc cctaggccaa ttgacttaga cattcttttt  360
   tatgggaagt ttagggtaag ctctgagaaa cttacagttc cgcatgaaag gatatgggaa  420
   agaccctttg tgatggcacc actaattgat ataattggat cggatgtaga aaatgacact  480
   gttgctgcat ggcattcatt atcaaaattt tctggtggac tatttgaagc gtgggataaa  540
   cttggtggag attcactcat tgggaaggac ggaatgagta gggttttgcc agttgggaac  600
   cacttgtggg attggtcgtg caaaacctct gtaatgggag tcttgaattt gactccggac  660
   agctttagtg atggaggaaa gtttctacct gtagaaaatg cagtttctca ggttcgtcaa  720
   atgatctcag aaggtgctga cataattgat attggagcgc aatcaacaag gcccatggca  780
   actaggattt ctgctgagga agagctggaa agactagtcc ctgttttaga aggtgtcaag  840
   gatgttatcg aggaagaagg aagaatgtta tcagtggata cattttactc aaaagttgct  900
   tctgaagctg tcaataaggg agcacatatg gtgaatgatg tttcgagtgg aaagctagat  960
   cctgagatgt tcaatgttgt tgcagggctt aaagtgcctt atatagcaat gcacatgcga 1020
   ggtgatcctt cttcaatgca aaatgctgat aatttgacat acaatgatgt ttgtaaggag 1080
   gtggctttag agttgagctc taggattaca gatgcagaat tatcaggtat tcctgcttgg 1140
   aggatagtta ttgatcctgg cattggattt tccaagaata caaagcaaaa tttggaaatt 1200
   ctcacagggt tgaaaagaat tcggcaagaa atagcaaaaa agagtttggc tgtggctcat 1260
   ggtcccttac tgattggacc ttcaagaaag aggtttttgg gtgagatctg caatcgtcct 1320
   gtagcatctg atagagatcc ggcaactgtt gcttctatca ccgctggagt tttgggtggt 1380
   gcaaacattg taagagtaca taatgttcgg gatagccttg atggtgtcaa gctatgtgat 1440
   gcaatg                                                            1446
<210> 47
   <211> 3920
   <212> DNA
   <213> Lolium multiflorum
<400> 47
   acgaaagctt tgaactctca agtggtgcaa catagataga ttatatatat ttcgaatata   60
   attcgaatga acataaaaat tacatataaa aactttaaaa caaacttaaa ctacttcttc  120
   ttcctcgatg gccccgcctc atcgtcgggg cggcgacgct tccggctcgt cacctcctcg  180
   tcggaggaag ttgggtcctc ctcctcgtca gtgtcctcag cctcttcgtc gtcctcctcc  240
   tcttcctcgg cctcttcgtc ggcctgctcc ttggcctctt cgtcctcctc ctcgtcgtca  300
   tcccatgcgt cctcctcctc gtcgtcatcc cattcgtcct tgctggccgg cgggggggaa  360
   tcgtcgctgc tggacgatgc agctttatcc caccaatggc gccatccagg cggcttcccc  420
   tcgctgtcgg tgtccgatgg ccaagagaga tcgctcatgg ttgacggagg atggtgagga  480
   gagaacagat gaatggcgtc ggccgtcgta aaccgtatat gtaggtctct cgacgaagag  540
   agcggcggtt gctcttccgc ggagttcgtg ctccattaca gcggttctcg catcgaggcc  600
   acttcggccg ttcccgacga gtcgtttggg ctctccagac cactttgcga cggttcaatg  660
   cgtcgagggc actccgacga ttgcccttcc cggtgactgc gccgtcgcta tgcacgcggt  720
   gtgtgcgcaa ccatgggctc gcggctggga aaatgggcct ccccaggcca caaaatacat  780
   ccatccggcg ataactaacg ccggatttcg gcctgggagc cccaacggct gggatgctct  840
   tagcccgtgg aacacattcc cgaatcggta ccgacggtct catctctctc gccctgatct  900
   atctctcttc cgcgatctgg aagggccgac ggcgtggcgg cgcaaccacg ggcgctgatg  960
   ggcggcggcg cgcagtctgt taagcaggac gcctgcttct cgctccgtcc tccctctcca 1020
   accgagcggc acgaggtaga cccggagacc ccaactccgt cctagcccag tggcgcccgt 1080
   gaggcgtcat ctccggcgcc ctctccatct ccgtctgctt cgcacttgta tgcacatact 1140
   cttcctcctt accccagttt cttggtcttg gcttgtttga ttacgatttc tagggttctt 1200
   ggcatgctcg gtagttgcag atttgtcttc tgttgggtgt ttgtttccat ggccttggct 1260
   gcatccatcg tttcgtatta tgtagttgtt tgtttccatg ccattgcttg tatccatcaa 1320
   ttggaaaggg aagaaagggc tacagggata ttaggaatta gatcagtcct tcctagctga 1380
   ttttctctga caggttgtta gtctcgcgca tgtggtggac ttcttgtgct tttggctagc 1440
   tccaagtctg atgtgtagct gctttctgag attttccaca taaacgttgc tatggctagt 1500
   aagttgtggc taactaaatc cttgggcaca agctagtaat ctgaatggca tatccgcccc 1560
   catgccttta attgttgcct aacgatgggc taggttctct tatttattca tttttgttaa 1620
   aagaagaact tgggcatgct aattcagcaa aatagaaaaa atatgtgttg tttctgtata 1680
   tagaggttct tggttttccc ttgccgattc tgaccgattt gcgggttttt tttttttttg 1740
   tctttcctca tcgtcaggca agaagagcaa gatgtgacag aggatttaag cagcagcaga 1800
   actagacctg gctcctatgc tctaagatgc ttcgtgccaa ggacacattc aggaagatgt 1860
   actcagctgc caagggctac tactgcggag ggctagcgtc tcctaatgcc ttctcaggta 1920
   gcttgcccaa agttccccaa taataactag ggaacatctc ttttccacct ctttcacatt 1980
   ccaaccaaag acagtcctaa ctcgtttccc ctctgaacat accagcattg gatcttcggc 2040
   atcctctcag ctcctcaaga aaaccaccag gccacgctaa cctgttcagg gtccgttcat 2100
   acacgcaatg cgccctcacc aacgattccg ccgaccagga ggtcgtgatt gccctgggca 2160
   gcaacgtggg agacagggtc agcatgttcg acagggcgct gcggctgatg aggagctcgg 2220
   gcatcaaggt caccaggcac gcctgcctgt acgagaccgc cccagcctac gtgaccgacc 2280
   agccgcggtt cctcaactct gccgttaggg gcacgacgaa gctggggcct cacgagttgc 2340
   tcaggaagct caaggagatc gagaaggata taggacgtac cgccggggta aggtacggcc 2400
   cgaggccgat cgatctggac attcttctgt atggggactc ccggatcaag accgagtctc 2460
   tgattgtgcc gcatgaacgc atccatgaga gaccgttcgt cttggcgcct cttgttgacc 2520
   tcctgggttc gtcggctgag gacggtatgg agaaaagctg gcactctctc tcgaagtgca 2580
   gtggcgggtt ctttgatttg tggaacaagc tcggcggtga atctgttgtt ggaacagaag 2640
   gcattaaaag ggtcatgtct gttggaaata cgctgttgga ctggcgtcag aggacccttg 2700
   ttatgggggt gcttaacctc acgccagaca gcttcagcga cggaggtaag tttcaagaag 2760
   tggaagctgc catttcccag gccaggttgt tgatatcaga aggtgctgac ataattgata 2820
   ttggtgccca gtccaccagg ccctttgcaa ggaggctatc cgcagaagaa gagcttgaga 2880
   ggctggtccc tgttctggat gctatcatga aactcccaga gatggaaggg aagttgcttt 2940
   cggtagacac gttctacgcg caagtcgctg ctgaagctgt aaaaagagga gccaccatga 3000
   tcaatgatgt atctggtggg cagcttgacc caagtattct tcaagttgtt gctgaactgg 3060
   gagttccgta tgttaccatg cacatgagag gcgatccatc aactatgcag aatgaacaga 3120
   atctacagta tgatgatgtc tgcaaagaag ttgcttctga gttatacgcc cggttgagag 3180
   cagcagagct ttctggaatt cctttgtgga ggattattct tgaccctggc attgggtttt 3240
   ctaagaaatc cacacagaat attgaagtaa tcgcgggttt ggaatccatt agagaagaga 3300
   tgggtaaaat gagcctaggt gcttcacatg tgccaatatt acttggaccc tcgaggaaaa 3360
   gtttcctagg tgaaatatgt agtcgcgccg atccagttca gagagatgct gctactgctt 3420
   ctgccgttac aattgcgatc ttgaatggcg ctaatatagt aagggtccat aatgttagat 3480
   acaatgtgga tgctgcaaag gtctctgatg cattactcaa gtacagaaga aaataataga 3540
   aagtatacag cccggataga agccatacca gttgccagtt ttgtgcaagg gaatgctgat 3600
   gtggagctca aaccatatgg ggtgtatcat cacttaataa gatcaaagca tgcgtgaata 3660
   actattacag tgaacaagag atatacagtt ttttcgtgcg cctcctcctt cgtagaatct 3720
   tcaaagaagc aaaggtaaca cagacctagg cctctggggt tggtatatga tgtaaccagt 3780
   tttgtgaggg tttaataatt atctaatagt gagacctaag agtaagacct ttataatata 3840
   ctttgcttgg aatttcctac ttgagagcat catttggggc tacagagttg attttcgtct 3900
   cacgcagttt tctgagggtt 3920
<210> 48
   <211> 1467
   <212> DNA
   <213> Lolium multiflorum
<400> 48
   agctcctcaa gaaaaccacc aggccacgct aacctgttca gggtccgttc atacacgcaa 60
   tgcgccctca ccaacgattc cgccgaccag gaggtcgtga ttgccctggg cagcaacgtg 120
   ggagacaggg tcagcatgtt cgacagggcg ctgcggctga tgaggagctc gggcatcaag 180
   gtcaccaggc acgcctgcct gtacgagacc gccccagcct acgtgaccga ccagccgcgg 240
   ttcctcaact ctgccgttag gggcacgacg aagctggggc ctcacgagtt gctcaggaag 300
   ctcaaggaga tcgagaagga tataggacgt accgccgggg taaggtacgg cccgaggccg 360
   atcgatctgg acattcttct gtatggggac tcccggatca agaccgagtc tctgattgtg 420
   ccgcatgaac gcatccatga gagaccgttc gtcttggcgc ctcttgttga cctcctgggt 480
   tcgtcggctg aggacggtat ggagaaaagc tggcactctc tctcgaagtg cagtggcggg 540
   ttctttgatt tgtggaacaa gctcggcggt gaatctgttg ttggaacaga aggcattaaa 600
   agggtcatgt ctgttggaaa tacgctgttg gactggcgtc agaggaccct tgttatgggg 660
   gtgcttaacc tcacgccaga cagcttcagc gacggaggta agtttcaaga agtggaagct 720
   gccatttccc aggccaggtt gttgatatca gaaggtgctg acataattga tattggtgcc 780
   cagtccacca ggccctttgc aaggaggcta tccgcagaag aagagcttga gaggctggtc 840
   cctgttctgg atgctatcat gaaactccca gagatggaag ggaagttgct ttcggtagac 900
   acgttctacg cgcaagtcgc tgctgaagct gtaaaaagag gagccaccat gatcaatgat 960
   gtatctggtg ggcagcttga cccaagtatt cttcaagttg ttgctgaact gggagttccg 1020
   tatgttacca tgcacatgag aggcgatcca tcaactatgc agaatgaaca gaatctacag 1080
   tatgatgatg tctgcaaaga agttgcttct gagttatacg cccggttgag agcagcagag 1140
   ctttctggaa ttcctttgtg gaggattatt cttgaccctg gcattgggtt ttctaagaaa 1200
   tccacacaga atattgaagt aatcgcgggt ttggaatcca ttagagaaga gatgggtaaa 1260
   atgagcctag gtgcttcaca tgtgccaata ttacttggac cctcgaggaa aagtttccta 1320
   ggtgaaatat gtagtcgcgc cgatccagtt cagagagatg ctgctactgc ttctgccgtt 1380
   acaattgcga tcttgaatgg cgctaatata gtaagggtcc ataatgttag atacaatgtg 1440
   gatgctgcaa aggtctctga tgcatta 1467
<210> 49
   <211> 834
   <212> DNA
   <213> Lolium multiflorum
<400> 49
   agggtcatgt ctgttggaaa tacgctgttg gattggcggg agaggaccct ggttatgggg 60
   gtgcttaacc tcacgccaga cagctttagc gatggaggta agtttcaaga ggtggaagct 120
   gccatttctc aggccaggct gttaatttca gaaggtgcag gcataattga tattggcgct 180
   cagtccacca ggccctttgc aaggtggcta tctgcagaag aagagcttga gaggctggtc 240
   cctgttctta atgctatcat gaaactcccc gagatggaag ggaagttgct ttcagtagat 300
   acgttctacg cgcaagtcgc ggctgaagcc gtgaaaagag gggccaccat gatcaatgat 360
   gtatctggtg ggcagcttga cccagatatt cttcaagttg ttgctgaatt gggagttccg 420
   tatgttgcca tgcacatgag aggggatcca tcaactatgc agaatgagca gaatttacag 480
   tatgatgatg tctgcaaaga agttgcttct gagttatact ctcggttgag agcagcagag 540
   ttgtctggaa ttcctttgtg gaggattatt cttgaccctg gcattgggtt ttccaagaaa 600
   tccacacaga atattgaagt aatcgcgggc ttggaatcca ttagagaaga gatgggtaaa 660
   atgagtctag gtgcttcaca tgtgccaata ttacttggac cctccaggaa acgtttctta 720
   gggcagatat gcaatcgcgc tgatccagtt gagagagatg cagctactgc tgcttctgtt 780
   acagttggga tattgaatgg tgctaatatt gtaagggtcc ataatgttag atac 834
<210> 50
   <211> 834
   <212> DNA
   <213> Lolium multiflorum
<400> 50
   agggtcatgt ctgttggaaa tacgctgttg gattggcggg agaggaccct ggttatgggg 60
   gtgcttaacc tcacgccaga cagctttagc gatggaggta agtttcaaga ggtggaagct 120
   gccatttctc aggccaggct gttaatttca gaaggtgcag gcataattga tattggcgct 180
   cagtccacca ggccctttgc aaggtggcta tctgcagaag aagagcttga gaggctggtc 240
   cctgttctta atgctatcat gaaactcccc gagatggaag ggaagttgct ttcagtagat 300
   acgttctacg cgcaagtcgc ggctgaagcc gtgaaaagag gggccaccat gatcaatgat 360
   gtatctggtg ggcagcttga cccagatatt cttcaagttg ttgctgaatt gggagttccg 420
   tatgttgcca tgcacatgag aggggatcca tcaactatgc agaatgagca gaatttacag 480
   tatgatgatg tctgcaaaga agttgcttct gagttatact ctcggttgag agcagcagag 540
   ttgtctggaa ttcctttgtg gaggattatt cttgaccctg gcattgggtt ttccaagaaa 600
   tccacacaga atattgaagt aatcgcgggc ttggaatcca ttagagaaga gatgggtaaa 660
   atgagtctag gtgcttcaca tgtgccaata ttacttggac cctccaggaa acgtttctta 720
   gggcagatat gcaatcgcgc tgatccagtt gagagagatg cagctactgc tgcttctgtt 780
   acagttggga tattgaatgg tgctaatatt gtaagggtcc ataatgttag atac 834
<210> 51
   <211> 212
   <212> DNA
   <213> Lolium multiflorum
<400> 51
   aggatatagg gcgcaccgcc gggataaggt acggtccgag gccgatcgat ctggacattc 60
   ttctgtatgg ggattcccgg atcaagaccg agtctctgac tgtgccgcat gaacgcatcc 120
   atgagagacc gttcgtcttg gcgcctcttg ttgacctcct gggttcatcg ggtgaggatg 180
   gtatggagag aagatggcac tctctctcga ag 212
<210> 52
   <211> 210
   <212> DNA
   <213> Lolium multiflorum
<400> 52
   gatatagggc gcaccgccgg gataaggtac ggtccgaggc cgatcgatct ggacattctt 60
   ctgtatgggg attcccggat caagaccgag tctctgactg tgccgcatga acgcatccat 120
   gagagaccgt tcgtcttggc gcctcttgtt gacctcctgg gttcatcggg tgaggatggt 180
   atggagagaa gatggcactc tctctcgaag 210
<210> 53
   <211> 201
   <212> DNA
   <213> Lolium multiflorum
<400> 53
   cgctaacctg ttcagggtcc gttcgtacat gcaatgcgcg gtcaccaacg attccgccga 60
   ccaagagatc gtgattgccc tgggcagcaa cgtgggagac agggtcagca cgttcgacag 120
   ggcgctgcgg ctgatgagga gctcgggcat caggatcacc aggcacgcct gcctgtacga 180
   gaccgcccct gcttacgtga c 201
<210> 54
   <211> 150
   <212> DNA
   <213> Lolium multiflorum
<400> 54
   gattccgccg accaagagat cgtgattgcc ctgggcagca acgtgggaga cagggtcagc 60
   acgttcgaca gggcgctgcg gctgatgagg agctcgggca tcaggatcac caggcacgcc 120
   tgcctgtacg agaccgcccc tgcttacgtg 150
<210> 55
   <211> 200
   <212> DNA
   <213> Amaranthus hybridus
<400> 55
   agatttgtga gcgccctgta gcagctgata gggatcctgc taccgttgct tcaataactg 60
   ctggagtttt aggtggtgca aacattgtaa gagtacataa tgtgagggat aaccttgatg 120
   ctgtcaagtt atgtgatgcc atactcggaa aaactgatta actgcttatt tgtaccacct 180
   tgtgaatgac gtctagtgga 200
<210> 56
   <211> 200
   <212> DNA
   <213> Amaranthus hybridus
<400> 56
   tccactagac gtcattcaca aggtggtaca aataagcagt taatcagttt ttccgagtat 60
   ggcatcacat aacttgacag catcaaggtt atccctcaca ttatgtactc ttacaatgtt 120
   tgcaccacct aaaactccag cagttattga agcaacggta gcaggatccc tatcagctgc 180
   tacagggcgc tcacaaatct 200
<210> 57
   <211> 200
   <212> DNA
   <213> Amaranthus hybridus
<400> 57
   caccttgtga atgacgtcta gtggaaagtt cgattgggat attgatgaag tccgttagtt 60
   tgctcgaaag tagagcttta gcccgtaagg ttatagttct tacagatgta gaatcattgg 120
   ctatttgcct attcctgctt agaagatcat agcattgatc cagactttgg attccatgta 180
   gaattatgtt gcgactcatg 200
<210> 58
   <211> 200
   <212> DNA
   <213> Amaranthus hybridus
<400> 58
   catgagtcgc aacataattc tacatggaat ccaaagtctg gatcaatgct atgatcttct 60
   aagcaggaat aggcaaatag ccaatgattc tacatctgta agaactataa ccttacgggc 120
   taaagctcta ctttcgagca aactaacgga cttcatcaat atcccaatcg aactttccac 180
   tagacgtcat tcacaaggtg 200
<210> 59
   <211> 200
   <212> DNA
   <213> Amaranthus hybridus
<400> 59
   atgtagaatt atgttgcgac tcatggtcga attggccctt tgaggaattg ttgaagttcc 60
   ttgatctatg gttcaatcgg ctcggttcga tgaaggttgg actgtgtcga gacaagatgg 120
   gtcaaagaga ccaatggaca agaagtcgac cttgggaaag cttgatcaag ttacttggac 180
   gagcagatag cagcaggact 200
<210> 60
   <211> 200
   <212> DNA
   <213> Amaranthus hybridus
<400> 60
   agtcctgctg ctatctgctc gtccaagtaa cttgatcaag ctttcccaag gtcgacttct 60
   tgtccattgg tctctttgac ccatcttgtc tcgacacagt ccaaccttca tcgaaccgag 120
   ccgattgaac catagatcaa ggaacttcaa caattcctca aagggccaat tcgaccatga 180
   gtcgcaacat aattctacat 200
<210> 61
   <211> 200
   <212> DNA
   <213> Amaranthus lividus
<400> 61
   aagggaataa ggtatggtcc taggccaatt gacttggaca tactatttta tgggaaattt 60
   agggtgagct ctgagagcct cactatcccc catgaaagga tatgggaaag accatttgtg 120
   atggcaccat tgattgattg tattgggtct gatgtagaaa atgacactat ttgtacatgg 180
   cattcattat caaatttttt 200
<210> 62
   <211> 200
   <212> DNA
   <213> Amaranthus lividus
<400> 62
   aaaaaatttg ataatgaatg ccatgtacaa atagtgtcat tttctacatc agacccaata 60
   caatcaatca atggtgccat cacaaatggt ctttcccata tcctttcatg ggggatagtg 120
   aggctctcag agctcaccct aaatttccca taaaatagta tgtccaagtc aattggccta 180
   ggaccatacc ttattccctt 200
<210> 63
   <211> 200
   <212> DNA
   <213> Amaranthus lividus
<400> 63
   catggcattc attatcaaat tttttgggtg gaatctttga agcatggggt aaactcggtg 60
   gaagttccct aatcgggaag gatggaatga aaagggtttt gcccgttgga aatcgcttat 120
   gggattggtc tcataaaacc tctgtaatgg gagtcttgaa cttgactcct gatagcttta 180
   gtgatggcgg aagttttcaa 200
<210> 64
   <211> 200
   <212> DNA
   <213> Amaranthus lividus
<400> 64
   ttgaaaactt ccgccatcac taaagctatc aggagtcaag ttcaagactc ccattacaga   60
   ggttttatga gaccaatccc ataagcgatt tccaacgggc aaaacccttt tcattccatc  120
   cttcccgatt agggaacttc caccgagttt accccatgct tcaaagattc cacccaaaaa  180
   atttgataat gaatgccatg                                              200
<210> 65
   <211> 200
   <212> DNA
   <213> Amaranthus palmeri
<400> 65
   cggatctgtt aaagggagcc ctctcatcct atgcttatca atgacttttt catatgccta   60
   tcaaacagat cagattggat cgggttcgag ttcaggtcat atataaacat gtcagcaaac  120
   ccttgaccca tacccaaccc atttagttaa ttggcttgaa aatcacaacc ttaattcgac  180
   cagcgacaga tcaggttgac                                              200
<210> 66
   <211> 200
   <212> DNA
   <213> Amaranthus palmeri
<400> 66
   gtcaacctga tctgtcgctg gtcgaattaa ggttgtgatt ttcaagccaa ttaactaaat   60
   gggttgggta tgggtcaagg gtttgctgac atgtttatat atgacctgaa ctcgaacccg  120
   atccaatctg atctgtttga taggcatatg aaaaagtcat tgataagcat aggatgagag  180
   ggctcccttt aacagatccg                                              200
<210> 67
   <211> 200
   <212> DNA
   <213> Amaranthus palmeri
<400> 67
   tcgaccagcg acagatcagg ttgacctgat ttcgacccac ttaacccatt tataattttt 60
   ttttcgattt aagtttttta acattgatta aatctaattt tttaggcttt aattttaatt 120
   tatgtttttt tgtttaattt gtatttacta tgaaaaataa gaaactatta tatgaactga 180
   gtttagctta cacttattga 200
<210> 68
   <211> 200
   <212> DNA
   <213> Amaranthus palmeri
<400> 68
   tcaataagtg taagctaaac tcagttcata taatagtttc ttatttttca tagtaaatac 60
   aaattaaaca aaaaaacata aattaaaatt aaagcctaaa aaattagatt taatcaatgt 120
   taaaaaactt aaatcgaaaa aaaaattata aatgggttaa gtgggtcgaa atcaggtcaa 180
   cctgatctgt cgctggtcga 200
<210> 69
   <211> 200
   <212> DNA
   <213> Amaranthus palmeri
<400> 69
   actgagttta gcttacactt attgatttaa ctcgaaatta acacatttaa ttaaatggtt 60
   atatagggtt ttttagtttt aaaatttcaa cctgaaccta atctatttaa taaaaagatc 120
   agatctgatc aacccatata attaattggg tcaaaatctc aactcaaacc catttatttc 180
   gaattaaatt cagatcaaat 200
<210> 70
   <211> 200
   <212> DNA
   <213> Amaranthus palmeri
<400> 70
   atttgatctg aatttaattc gaaataaatg ggtttgagtt gagattttga cccaattaat 60
   tatatgggtt gatcagatct gatcttttta ttaaatagat taggttcagg ttgaaatttt 120
   aaaactaaaa aaccctatat aaccatttaa ttaaatgtgt taatttcgag ttaaatcaat 180
   aagtgtaagc taaactcagt 200
<210> 71
   <211> 200
   <212> DNA
   <213> Amaranthus palmeri
<400> 71
   atttcgaatt aaattcagat caaattagca ggtcggatca actagcgcta atttttcctc   60
   tcaagttttt tcatatgatg ccccttgttt tctttgaata aaattcacca atttaaaatc  120
   cctgcccttg atccgttggt attggtattt ggtaaagcac atatggtaaa ttctactcca  180
   ttgttatctg tagttttggt                                              200
<210> 72
   <211> 200
   <212> DNA
   <213> Amaranthus palmeri
<400> 72
   accaaaacta cagataacaa tggagtagaa tttaccatat gtgctttacc aaataccaat   60
   accaacggat caagggcagg gattttaaat tggtgaattt tattcaaaga aaacaagggg  120
   catcatatga aaaaacttga gaggaaaaat tagcgctagt tgatccgacc tgctaatttg  180
   atctgaattt aattcgaaat                                              200
<210> 73
   <211> 200
   <212> DNA
   <213> Amaranthus palmeri
<400> 73
   ctccattgtt atctgtagtt ttggttctaa accccaaaca aatcctactc ctagcaccac   60
   taatcctcat tactttctgt ttgctgtctt tctctacatt cctactaaat taagacctag  120
   atgcagtgtt attctgttcg aggttgccga aaacggtaca atagttgcct aatttgcaat  180
   tcatgtttgt tcttgcgttt                                              200
<210> 74
   <211> 200
   <212> DNA
   <213> Amaranthus palmeri
<400> 74
   aaacgcaaga acaaacatga attgcaaatt aggcaactat tgtaccgttt tcggcaacct   60
   cgaacagaat aacactgcat ctaggtctta atttagtagg aatgtagaga aagacagcaa  120
   acagaaagta atgaggatta gtggtgctag gagtaggatt tgtttggggt ttagaaccaa  180
   aactacagat aacaatggag                                              200
<210> 75
   <211> 200
   <212> DNA
   <213> Amaranthus palmeri
<400> 75
   gcaattcatg tttgttcttg cgttttctac attgtttact ctattggaac aaaggttgaa 60
   ctttcagcct ttcagggagt attttgattt ttctgtcaaa ttaccttgcc agatttattt 120
   ggctattttg attgtgtgta tttctttgtc ccttgtttcc atggggctaa actccttttg 180
   gttaatctta aacatttctt 200
<210> 76
   <211> 200
   <212> DNA
   <213> Amaranthus palmeri
<400> 76
   aagaaatgtt taagattaac caaaaggagt ttagccccat ggaaacaagg gacaaagaaa 60
   tacacacaat caaaatagcc aaataaatct ggcaaggtaa tttgacagaa aaatcaaaat 120
   actccctgaa aggctgaaag ttcaaccttt gttccaatag agtaaacaat gtagaaaacg 180
   caagaacaaa catgaattgc 200
<210> 77
   <211> 200
   <212> DNA
   <213> Amaranthus palmeri
<400> 77
   ttttggttaa tcttaaacat ttcttctaat tttgaattgc agtggtatga agtgttgttt 60
   aatatacaat caatttttct gaactgggtt aatgaagaga agcattgtag acatgaggaa 120
   tgtgttcaag catctcaaaa caaccaaatt aatcccaaat gggatcttca actgtaatag 180
   aggtaaatca catatattct 200
<210> 78
   <211> 200
   <212> DNA
   <213> Amaranthus palmeri
<400> 78
   agaatatatg tgatttacct ctattacagt tgaagatccc atttgggatt aatttggttg 60
   ttttgagatg cttgaacaca ttcctcatgt ctacaatgct tctcttcatt aacccagttc 120
   agaaaaattg attgtatatt aaacaacact tcataccact gcaattcaaa attagaagaa 180
   atgtttaaga ttaaccaaaa 200
<210> 79
   <211> 200
   <212> DNA
   <213> Amaranthus palmeri
<400> 79
   aatagaggta aatcacatat attctcattt aatggttatt gttttatttt aaggatgtta   60
   ttatgttgtt atcaatgata gaaataagag aagcttgagc attcaaattt atctatcact  120
   cactggttgt ggacatctaa aatatggacc aagagttttc ttcatttttt gtaccagaag  180
   ctgcaaatgt atgattaaag                                              200
<210> 80
   <211> 200
   <212> DNA
   <213> Amaranthus palmeri
<400> 80
   ctttaatcat acatttgcag cttctggtac aaaaaatgaa gaaaactctt ggtccatatt   60
   ttagatgtcc acaaccagtg agtgatagat aaatttgaat gctcaagctt ctcttatttc  120
   tatcattgat aacaacataa taacatcctt aaaataaaac aataaccatt aaatgagaat  180
   atatgtgatt tacctctatt                                              200
<210> 81
   <211> 200
   <212> DNA
   <213> Amaranthus palmeri
<400> 81
   agaagctgca aatgtatgat taaagtttag gttggagttg caataaaatt ttattacccc   60
   gacgctatta agtggctcac actgaggcgg ggtttgaggg tcagatggtg caaccttcag  120
   aagaggttct aagcattttc aacatgttca accgcatagg gtcccgtaaa attggaggct  180
   tcaatattaa attatgtagt                                              200
<210> 82
   <211> 200
   <212> DNA
   <213> Amaranthus palmeri
<400> 82
   actacataat ttaatattga agcctccaat tttacgggac cctatgcggt tgaacatgtt 60
   gaaaatgctt agaacctctt ctgaaggttg caccatctga ccctcaaacc ccgcctcagt 120
   gtgagccact taatagcgtc ggggtaataa aattttattg caactccaac ctaaacttta 180
   atcatacatt tgcagcttct 200
<210> 83
   <211> 200
   <212> DNA
   <213> Amaranthus palmeri
<400> 83
   aggcttcaat attaaattat gtagtatgtg ttatgtgttt aaagatataa atatgttaat 60
   gaatacataa tttttaaatt gcacagggcc ttcaaagaaa ttttcgattt tgctctgccc 120
   ctggcaacct ttggcaaatt tggatagaat tagagaagtt tacttttatg tgtgtataat 180
   atgatgaatg aatgagtgaa 200
<210> 84
   <211> 200
   <212> DNA
   <213> Amaranthus palmeri
<400> 84
   ttcactcatt cattcatcat attatacaca cataaaagta aacttctcta attctatcca 60
   aatttgccaa aggttgccag gggcagagca aaatcgaaaa tttctttgaa ggccctgtgc 120
   aatttaaaaa ttatgtattc attaacatat ttatatcttt aaacacataa cacatactac 180
   ataatttaat attgaagcct 200
<210> 85
   <211> 200
   <212> DNA
   <213> Amaranthus palmeri
<400> 85
   ataatatgat gaatgaatga gtgaacaatg aagttcattt ctgagtcaat gacctatata 60
   ctatcgaggt ttcgttaaaa aggatatata aataaaatta gagacatctt ttatccctaa 120
   tgttgtctgt tttcttttct gggcgagact gtttttggag aaaaacgtta tgttgaatgc 180
   ttgatttact cagaactgat 200
<210> 86
   <211> 200
   <212> DNA
   <213> Amaranthus palmeri
<400> 86
   atcagttctg agtaaatcaa gcattcaaca taacgttttt ctccaaaaac agtctcgccc 60
   agaaaagaaa acagacaaca ttagggataa aagatgtctc taattttatt tatatatcct 120
   ttttaacgaa acctcgatag tatataggtc attgactcag aaatgaactt cattgttcac 180
   tcattcattc atcatattat 200
<210> 87
   <211> 200
   <212> DNA
   <213> Amaranthus palmeri
<400> 87
   aatgcttgat ttactcagaa ctgatgcaaa cagatggata aaaatggtgt tttcctattg 60
   tttatgtttc gataacaggt ttcaaattcc actcgtatga agtttgatat actgctatgt 120
   gtttttgtag cttcgtgttt tgctttcctt cactcatcac cagtgaatga tgttgaagtt 180
   tgttccaaga agcaagaagt 200
<210> 88
   <211> 200
   <212> DNA
   <213> Amaranthus palmeri
<400> 88
   acttcttgct tcttggaaca aacttcaaca tcattcactg gtgatgagtg aaggaaagca 60
   aaacacgaag ctacaaaaac acatagcagt atatcaaact tcatacgagt ggaatttgaa 120
   acctgttatc gaaacataaa caataggaaa acaccatttt tatccatctg tttgcatcag 180
   ttctgagtaa atcaagcatt 200
<210> 89
   <211> 200
   <212> DNA
   <213> Amaranthus palmeri
<400> 89
   aagtttgttc caagaagcaa gaagtcgtaa ttgcaatcgg gagcaatgtt ggtgacagat 60
   tagaaaattt caaccaagct ctgcaacaaa tgaagaaatt aggcatagac atcacaaggc 120
   atgggtgttt atatgagacg gaacctgcat acgtgactga tcaaccgaag tttcttaact 180
   ctgctttaag aggctttaca 200
<210> 90
   <211> 200
   <212> DNA
   <213> Amaranthus palmeri
<400> 90
   tgtaaagcct cttaaagcag agttaagaaa cttcggttga tcagtcacgt atgcaggttc 60
   cgtctcatat aaacacccat gccttgtgat gtctatgcct aatttcttca tttgttgcag 120
   agcttggttg aaattttcta atctgtcacc aacattgctc ccgattgcaa ttacgacttc 180
   ttgcttcttg gaacaaactt 200
<210> 91
   <211> 200
   <212> DNA
   <213> Amaranthus palmeri
<400> 91
   taactctgct ttaagaggct ttacaaaact tgggcctcat gaattattag gggtattaaa 60
   gaaaattgag aaggatatgg gtagaaccaa gggaataagg tatggtccta ggccaattga 120
   cttggacata ctattttatg ggaagtttag ggtgagctct gagagcctca ctatccccca 180
   tgaaaggata tgggaaagac 200
<210> 92
   <211> 200
   <212> DNA
   <213> Amaranthus palmeri
<400> 92
   gtctttccca tatcctttca tgggggatag tgaggctctc agagctcacc ctaaacttcc 60
   cataaaatag tatgtccaag tcaattggcc taggaccata ccttattccc ttggttctac 120
   ccatatcctt ctcaattttc tttaataccc ctaataattc atgaggccca agttttgtaa 180
   agcctcttaa agcagagtta 200
<210> 93
   <211> 200
   <212> DNA
   <213> Amaranthus palmeri
<400> 93
   ccccatgaaa ggatatggga aagaccattt gtgatggcac cattgattga ttgtattggg 60
   tctgatgtag aaaatgacac tatttgtacg tggcattcat tatcaaattt ttcgggtgga 120
   atctttgaag catggggtaa actcggtgga agttccctaa tcgggaagga tggaatgaaa 180
   agggttttgc ccgttggaaa 200
<210> 94
   <211> 200
   <212> DNA
   <213> Amaranthus palmeri
<400> 94
   tttccaacgg gcaaaaccct tttcattcca tccttcccga ttagggaact tccaccgagt   60
   ttaccccatg cttcaaagat tccacccgaa aaatttgata atgaatgcca cgtacaaata  120
   gtgtcatttt ctacatcaga cccaatacaa tcaatcaatg gtgccatcac aaatggtctt  180
   tcccatatcc tttcatgggg                                              200
<210> 95
   <211> 200
   <212> DNA
   <213> Amaranthus palmeri
<400> 95
   tgaaaagggt tttgcccgtt ggaaatcgct tatgggattg gtctcataaa acctctgtta   60
   tgggagtctt gaacttgact cctgatagct ttagtgatgg cggaagtttt caatctgtcg  120
   atactgcggt tgctaaggtt cgtcagatga tctcagatgg ggcagatata attgacatcg  180
   gggcacaatc aaccagaccc                                              200
<210> 96
   <211> 200
   <212> DNA
   <213> Amaranthus palmeri
<400> 96
   gggtctggtt gattgtgccc cgatgtcaat tatatctgcc ccatctgaga tcatctgacg   60
   aaccttagca accgcagtat cgacagattg aaaacttccg ccatcactaa agctatcagg  120
   agtcaagttc aagactccca taacagaggt tttatgagac caatcccata agcgatttcc  180
   aacgggcaaa acccttttca                                              200
<210> 97
   <211> 200
   <212> DNA
   <213> Amaranthus palmeri
<400> 97
   catcggggca caatcaacca gacccatggc aactagaatt tcggctgaag aagagctaga   60
   aagagtagta cctgtattgg aagctgtcaa ggatttgatc gaggaagaag gaagaatctt  120
   gtcagtggat acgttttatt ctaaagttgc ttcggaggcc gtcaagaagg gggcacacat  180
   tgtgaatgat gtctctagtg                                              200
<210> 98
   <211> 200
   <212> DNA
   <213> Amaranthus palmeri
<400> 98
   cactagagac atcattcaca atgtgtgccc ccttcttgac ggcctccgaa gcaactttag   60
   aataaaacgt atccactgac aagattcttc cttcttcctc gatcaaatcc ttgacagctt  120
   ccaatacagg tactactctt tctagctctt cttcagccga aattctagtt gccatgggtc  180
   tggttgattg tgccccgatg                                              200
<210> 99
   <211> 200
   <212> DNA
   <213> Amaranthus palmeri
<400> 99
   cacattgtga atgatgtctc tagtgggaaa ctcgattccg agatgtttaa tgttgttgcg   60
   gaccttaaag ttccttatat agcaatgcac atgcgaggag atccgacttc aatgcaaaac  120
   tctgagaact tgacctacaa tgatgtttgt aagcaagtgg ctttggagtt gagttctagg  180
   gtcatagatg cagaattatc                                              200
<210> 100
   <211> 200
   <212> DNA
   <213> Amaranthus palmeri
<400> 100
   gataattctg catctatgac cctagaactc aactccaaag ccacttgctt acaaacatca   60
   ttgtaggtca agttctcaga gttttgcatt gaagtcggat ctcctcgcat gtgcattgct  120
   atataaggaa ctttaaggtc cgcaacaaca ttaaacatct cggaatcgag tttcccacta  180
   gagacatcat tcacaatgtg                                              200
<210> 101
   <211> 200
   <212> DNA
   <213> Amaranthus palmeri
<400> 101
   ctagggtcat agatgcagaa ttatcgggaa ttcctgcttg gaggatagtt attgatcccg 60
   gcatcggatt ttctaagaat acgaatcaaa atttggaaat tcttagtggt ttacaaaaga 120
   tacgggaaga gatagctaag aagagtttgg cggtggctca ttgccccttg ctaattggac 180
   cttcaagaaa gaggtttctg 200
<210> 102
   <211> 200
   <212> DNA
   <213> Amaranthus palmeri
<400> 102
   cagaaacctc tttcttgaag gtccaattag caaggggcaa tgagccaccg ccaaactctt 60
   cttagctatc tcttcccgta tcttttgtaa accactaaga atttccaaat tttgattcgt 120
   attcttagaa aatccgatgc cgggatcaat aactatcctc caagcaggaa ttcccgataa 180
   ttctgcatct atgaccctag 200
<210> 103
   <211> 200
   <212> DNA
   <213> Amaranthus palmeri
<400> 103
   tggaccttca agaaagaggt ttctgggcga gatttgtgag cgccctgtag cagctgacag 60
   ggatcctgct accattgctt ctataactgc tggagtttta ggtggtgcaa acattgtaag 120
   agtacataat gtgagggata accttgatgc tgtcaagtta tgtgatgcca tactcggaaa 180
   aactgattaa ctgcttgttt 200
<210> 104
   <211> 200
   <212> DNA
   <213> Amaranthus palmeri
<400> 104
   aaacaagcag ttaatcagtt tttccgagta tggcatcaca taacttgaca gcatcaaggt 60
   tatccctcac attatgtact cttacaatgt ttgcaccacc taaaactcca gcagttatag 120
   aagcaatggt agcaggatcc ctgtcagctg ctacagggcg ctcacaaatc tcgcccagaa 180
   acctctttct tgaaggtcca 200
<210> 105
   <211> 200
   <212> DNA
   <213> Amaranthus palmeri
<400> 105
   ggaaaaactg attaactgct tgtttgtacc accttgtgaa tgatgtctag tggaaggttc    60
   gattgggatt atgatgaagt ccgttagttt gctcgaaaga agagctttag cccgtaaggt   120
   tatagttctt acagatgtag aatcattggc tatttgccta tttctgctta gaagatcata   180
   gcattgatcc agactttgga                                               200
<210> 106
   <211> 200
   <212> DNA
   <213> Amaranthus palmeri
<400> 106
   tccaaagtct ggatcaatgc tatgatcttc taagcagaaa taggcaaata gccaatgatt    60
   ctacatctgt aagaactata accttacggg ctaaagctct tctttcgagc aaactaacgg   120
   acttcatcat aatcccaatc gaaccttcca ctagacatca ttcacaaggt ggtacaaaca   180
   agcagttaat cagtttttcc                                               200
<210> 107
   <211> 200
   <212> DNA
   <213> Amaranthus palmeri
<400> 107
   tcatagcatt gatccagact ttggattcca tgtagaatta tgttgcgact catggtcgaa    60
   ttggcccttt gaggaattgt tgaagttcct tgagtctatg gttcaatcgg ctcggttcga   120
   tgaaggttgg actgtgtcga gacaagatgg gtcaaagaga ccaatggaca agaagtcgac   180
   cttgggcaag cttgatcaag                                               200
<210> 108
   <211> 200
   <212> DNA
   <213> Amaranthus palmeri
<400> 108
   cttgatcaag cttgcccaag gtcgacttct tgtccattgg tctctttgac ccatcttgtc    60
   tcgacacagt ccaaccttca tcgaaccgag ccgattgaac catagactca aggaacttca   120
   acaattcctc aaagggccaa ttcgaccatg agtcgcaaca taattctaca tggaatccaa   180
   agtctggatc aatgctatga                                               200
<210> 109
   <211> 200
   <212> DNA
   <213> Amaranthus palmeri
<400> 109
   tcgaccttgg gcaagcttga tcaagttact tggacaagca gataacagca ggctctggcg    60
   agcattagac atatcatttg ttttttggag tcagatctgc aatcaatcag ttgcaatgga   120
   caaaccatat acggttgtta gttgttactg tcactgtttg agttgaatta gatcatgcaa   180
   acactgtagg agtcttgatt                                               200
<210> 110
   <211> 200
   <212> DNA
   <213> Amaranthus palmeri
<400> 110
   aatcaagact cctacagtgt ttgcatgatc taattcaact caaacagtga cagtaacaac    60
   taacaaccgt atatggtttg tccattgcaa ctgattgatt gcagatctga ctccaaaaaa   120
   caaatgatat gtctaatgct cgccagagcc tgctgttatc tgcttgtcca agtaacttga   180
   tcaagcttgc ccaaggtcga                                               200
<210> 111
   <211> 200
   <212> DNA
   <213> Amaranthus palmeri
<400> 111
   tgcaaacact gtaggagtct tgattatgtg aagctatgtg acactgtact tctcaaaatg    60
   aaatctttga tatgatgtta gtttcaagag ttgtaattcc cccctcacat ttctgttcta   120
   tgttgtattg ttgtgtgctt tttggaattg tattcctgca atagaattat tcccgcaaaa   180
   tggtattcct ttatatcatc                                               200
<210> 112
   <211> 200
   <212> DNA
   <213> Amaranthus palmeri
<400> 112
   gatgatataa aggaatacca ttttgcggga ataattctat tgcaggaata caattccaaa    60
   aagcacacaa caatacaaca tagaacagaa atgtgagggg ggaattacaa ctcttgaaac   120
   taacatcata tcaaagattt cattttgaga agtacagtgt cacatagctt cacataatca   180
   agactcctac agtgtttgca                                               200
<210> 113
   <211> 200
   <212> DNA
   <213> Amaranthus palmeri
<400> 113
   caaaatggta ttcctttata tcatcaaagg agtttttgta cttctaatca ttgatcttta   60
   gatgcaaaac tgaattaata caagtattct tgatgaaggc ttattgaaga actcactttt  120
   tatttatgtc taaacttgtt aaagatgttc tgcttccgcc cttgccacgg gatgtcggga  180
   ttgactaggg gtggtaatcg                                              200
<210> 114
   <211> 200
   <212> DNA
   <213> Amaranthus palmeri
<400> 114
   cgattaccac ccctagtcaa tcccgacatc ccgtggcaag ggcggaagca gaacatcttt   60
   aacaagttta gacataaata aaaagtgagt tcttcaataa gccttcatca agaatacttg  120
   tattaattca gttttgcatc taaagatcaa tgattagaag tacaaaaact cctttgatga  180
   tataaaggaa taccattttg                                              200
<210> 115
   <211> 200
   <212> DNA
   <213> Amaranthus palmeri
<400> 115
   cgggattgac taggggtggt aatcgaggga ttcctatgct tgaattgact cgtttaagat   60
   tttgcgttga ttttaagtca aacgttttgc taagcataaa ctattctagc tcgacttgaa  120
   taatgaagtt ttcgagtcaa gtccgagctg ttctataagt ttacgtatct tgaagcttct  180
   tagttcttac ttcttatcct                                              200
<210> 116
   <211> 200
   <212> DNA
   <213> Amaranthus palmeri
<400> 116
   aggataagaa gtaagaacta agaagcttca agatacgtaa acttatagaa cagctcggac   60
   ttgactcgaa aacttcatta ttcaagtcga gctagaatag tttatgctta gcaaaacgtt  120
   tgacttaaaa tcaacgcaaa atcttaaacg agtcaattca agcataggaa tccctcgatt  180
   accaccccta gtcaatcccg 200
<210> 117
   <211> 200
   <212> DNA
   <213> Amaranthus palmeri
<400> 117
   cttcttagtt cttacttctt atcctttatt ttttgacaca ttgaaccgca ttgatgcttg 60
   cttgactcaa ctcgttaaag tcttgagttc atcacgaatt cgagtcgagc aagtttaaac 120
   ccaacattaa ctaagctctc gaggcttaat gaggcagggg tagctcaagt ttgttattca 180
   agcaacttac catagtattc 200
<210> 118
   <211> 200
   <212> DNA
   <213> Amaranthus palmeri
<400> 118
   gaatactatg gtaagttgct tgaataacaa acttgagcta cccctgcctc attaagcctc 60
   gagagcttag ttaatgttgg gtttaaactt gctcgactcg aattcgtgat gaactcaaga 120
   ctttaacgag ttgagtcaag caagcatcaa tgcggttcaa tgtgtcaaaa aataaaggat 180
   aagaagtaag aactaagaag 200
<210> 119
   <211> 200
   <212> DNA
   <213> Amaranthus palmeri
<400> 119
   attcaagcaa cttaccatag tattctttta aaaggcatat agtcagttag ttatttgtta 60
   atttcagtag aacgaaagaa gtacattatt aaacttggcc attgatgctt gagctagaga 120
   gtttcggttg tgatcgaaac gaatcctgat ttcgctcttg atctttgatg tgaagtgaca 180
   aggccttcat aaaactcctt 200
<210> 120
   <211> 200
   <212> DNA
   <213> Amaranthus palmeri
<400> 120
   aaggagtttt atgaaggcct tgtcacttca catcaaagat caagagcgaa atcaggattc 60
   gtttcgatca caaccgaaac tctctagctc aagcatcaat ggccaagttt aataatgtac 120
   ttctttcgtt ctactgaaat taacaaataa ctaactgact atatgccttt taaaagaata 180
   ctatggtaag ttgcttgaat 200
<210> 121
   <211> 200
   <212> DNA
   <213> Amaranthus palmeri
<400> 121
   tgacaaggcc ttcataaaac tcctttattc ttcagcagtg catacaaaaa tcatgtgtgc 60
   ccagctccta gctcacatac cgcgtttttc ccctaatcta gtgtggttaa ataaaaacag 120
   caagatgtgt tgatgtcatg tgcaatagta attatcaggt gaagtaattc aatatgtagc 180
   cagtcacctc atatcaaaaa 200
<210> 122
   <211> 200
   <212> DNA
   <213> Amaranthus palmeri
<400> 122
   tttttgatat gaggtgactg gctacatatt gaattacttc acctgataat tactattgca 60
   catgacatca acacatcttg ctgtttttat ttaaccacac tagattaggg gaaaaacgcg 120
   gtatgtgagc taggagctgg gcacacatga tttttgtatg cactgctgaa gaataaagga 180
   gttttatgaa ggccttgtca 200
<210> 123
   <211> 200
   <212> DNA
   <213> Amaranthus palmeri
<400> 123
   gtagccagtc acctcatatc aaaaaaatgt tgggaggaaa acgtattcgt tcatcactaa 60
   acctgttttt atacatctaa ctgatcgatc agggtttcaa aatccaaatt aatacaatgg 120
   catttgacga agatatccag aacacatccc ccttgtttct gctagataat caactattct 180
   gattctgctg taatacctca 200
<210> 124
   <211> 200
   <212> DNA
   <213> Amaranthus palmeri
<400> 124
   tgaggtatta cagcagaatc agaatagttg attatctagc agaaacaagg gggatgtgtt 60
   ctggatatct tcgtcaaatg ccattgtatt aatttggatt ttgaaaccct gatcgatcag 120
   ttagatgtat aaaaacaggt ttagtgatga acgaatacgt tttcctccca acattttttt 180
   gatatgaggt gactggctac 200
<210> 125
   <211> 200
   <212> DNA
   <213> Amaranthus palmeri
<400> 125
   attctgattc tgctgtaata cctcagcttc ggccctagcc atgggatcgt cctctttttc 60
   tccttcacgc tcctcggcca gtttctttct gtgcagttct tcagcagctt ctattgaagc 120
   tttgttctcg gcctgaagac gctgcatttc ctcttccatt tgctgcctct caaaccatgt 180
   atcagcatcc gcccaaactg 200
<210> 126
   <211> 200
   <212> DNA
   <213> Amaranthus palmeri
<400> 126
   cagtttgggc ggatgctgat acatggtttg agaggcagca aatggaagag gaaatgcagc 60
   gtcttcaggc cgagaacaaa gcttcaatag aagctgctga agaactgcac agaaagaaac 120
   tggccgagga gcgtgaagga gaaaaagagg acgatcccat ggctagggcc gaagctgagg 180
   tattacagca gaatcagaat 200
<210> 127
   <211> 200
   <212> DNA
   <213> Amaranthus palmeri
<400> 127
   gaagctgtca aggatttgat cgaggaagaa ggaagaatct tgtcagtgga tacgttttat 60
   tctaaagttg cttcggaggc cgtcaagaag ggggcacaca ttgtgaatga tgtctctagt 120
   gggaaactcg attccgagat gtttaatgtt gttgcggacc ttaaagttcc ttatatagca 180
   atgcacatgc gaggagatcc 200
<210> 128
   <211> 200
   <212> DNA
   <213> Amaranthus palmeri
<400> 128
   ggatctcctc gcatgtgcat tgctatataa ggaactttaa ggtccgcaac aacattaaac 60
   atctcggaat cgagtttccc actagagaca tcattcacaa tgtgtgcccc cttcttgacg 120
   gcctccgaag caactttaga ataaaacgta tccactgaca agattcttcc ttcttcctcg 180
   atcaaatcct tgacagcttc 200
<210> 129
   <211> 200
   <212> DNA
   <213> Amaranthus palmeri
<400> 129
   tagcaatgca catgcgagga gatccgactt caatgcaaaa ctctgagaac ttgacctaca 60
   atgatgtttg taagcaagtg gctttggagt tgagttctag ggtcatagat gcagaattat 120
   cgggaattcc tgcttggagg atagttattg atcccggcat cggattttct aagaatacga 180
   atcaaaattt ggaaattctt 200
<210> 130
   <211> 200
   <212> DNA
   <213> Amaranthus palmeri
<400> 130
   aagaatttcc aaattttgat tcgtattctt agaaaatccg atgccgggat caataactat 60
   cctccaagca ggaattcccg ataattctgc atctatgacc ctagaactca actccaaagc 120
   cacttgctta caaacatcat tgtaggtcaa gttctcagag ttttgcattg aagtcggatc 180
   tcctcgcatg tgcattgcta 200
<210> 131
   <211> 200
   <212> DNA
   <213> Amaranthus palmeri
<400> 131
   tacgaatcaa aatttggaaa ttcttagtgg tttacaaaag atacgggaag agatagctaa 60
   gaagagtttg gcggtggctc attgcccctt gctaattgga ccttcaagaa agaggtttct 120
   gggcgagatt tgtgagcgcc ctgtagcagc tgacagggat cctgctacca ttgcttctat 180
   aactgctgga gttttaggtg 200
<210> 132
   <211> 200
   <212> DNA
   <213> Amaranthus palmeri
<400> 132
   cacctaaaac tccagcagtt atagaagcaa tggtagcagg atccctgtca gctgctacag 60
   ggcgctcaca aatctcgccc agaaacctct ttcttgaagg tccaattagc aaggggcaat 120
   gagccaccgc caaactcttc ttagctatct cttcccgtat cttttgtaaa ccactaagaa 180
   tttccaaatt ttgattcgta 200
<210> 133
   <211> 200
   <212> DNA
   <213> Amaranthus palmeri
<400> 133
   tctataactg ctggagtttt aggtggtgca aacattgtaa gagtacataa tgttagggat 60
   aaccttgatg ctgtcaagtt atgtgatgcc atactcggaa aaactgatta actgcttgtt 120
   tgtaccacct tgtgaatgat gtctagtgga aggttcgatt gggattatga tgaagtccgt 180
   tagtttgctc gaaagtagag 200
<210> 134
   <211> 200
   <212> DNA
   <213> Amaranthus palmeri
<400> 134
   ctctactttc gagcaaacta acggacttca tcataatccc aatcgaacct tccactagac 60
   atcattcaca aggtggtaca aacaagcagt taatcagttt ttccgagtat ggcatcacat 120
   aacttgacag catcaaggtt atccctaaca ttatgtactc ttacaatgtt tgcaccacct 180
   aaaactccag cagttataga 200
<210> 135
   <211> 200
   <212> DNA
   <213> Amaranthus palmeri
<400> 135
   tccgttagtt tgctcgaaag tagagcttta gcccgtaagg ctatagttct tacagatgta 60
   gaatcattgg ctatttgcct atttctgctt agaagatcat agcattgatc cagactttgg 120
   attccatgta gaattatgtt gcgactcatg gtcgaattgg ccctttgagg aattgttgaa 180
   gttccttgag tctatggttc 200
<210> 136
   <211> 200
   <212> DNA
   <213> Amaranthus palmeri
<400> 136
   gaaccataga ctcaaggaac ttcaacaatt cctcaaaggg ccaattcgac catgagtcgc 60
   aacataattc tacatggaat ccaaagtctg gatcaatgct atgatcttct aagcagaaat 120
   aggcaaatag ccaatgattc tacatctgta agaactatag ccttacgggc taaagctcta 180
   ctttcgagca aactaacgga 200
<210> 137
   <211> 200
   <212> DNA
   <213> Amaranthus palmeri
<400> 137
   ttgaagttcc ttgagtctat ggttcaatcg gctcggttcg atgaaggttg gactgtgtcg 60
   agacaagatg ggtcaaagag accaatggac aagaagtcga ccttgggcaa gcttgatcaa 120
   gttacttgga caagcagata acagcaggct ctggcgagca ttagacatat catttgtttt 180
   ttggagtcag atctgcaatc 200
<210> 138
   <211> 200
   <212> DNA
   <213> Amaranthus palmeri
<400> 138
   gattgcagat ctgactccaa aaaacaaatg atatgtctaa tgctcgccag agcctgctgt 60
   tatctgcttg tccaagtaac ttgatcaagc ttgcccaagg tcgacttctt gtccattggt 120
   ctctttgacc catcttgtct cgacacagtc caaccttcat cgaaccgagc cgattgaacc 180
   atagactcaa ggaacttcaa 200
<210> 139
   <211> 200
   <212> DNA
   <213> Amaranthus palmeri
<400> 139
   gctttccttc actcatcacc agtgaatgat gttgaagttt gttccaagaa gcaagaagtc 60
   gtaattgcaa tcgggagcaa tgttggtgac agattagaaa atttcaacca agctctgcaa 120
   caaatgaaga aattaggcat agacatcaca aggcatgggt gtttatatga gacggaacct 180
   gcatacgtga ctgatcaacc 200
<210> 140
   <211> 200
   <212> DNA
   <213> Amaranthus palmeri
<400> 140
   ggttgatcag tcacgtatgc aggttccgtc tcatataaac acccatgcct tgtgatgtct 60
   atgcctaatt tcttcatttg ttgcagagct tggttgaaat tttctaatct gtcaccaaca 120
   ttgctcccga ttgcaattac gacttcttgc ttcttggaac aaacttcaac atcattcact 180
   ggtgatgagt gaaggaaagc 200
<210> 141
   <211> 200
   <212> DNA
   <213> Amaranthus palmeri
<400> 141
   aacctgcata cgtgactgat caaccgaagt ttcttaactc tgctttaaga ggctttacaa 60
   aacttgggcc tcatgaatta ttaggggtat taaagaaaat tgagaaggat atgggtagaa 120
   ccaagggaat aaggtatggt cctaggccaa ttgacttgga catactattt tatgggaagt 180
   ttagggtgag ctctgagagc 200
<210> 142
   <211> 200
   <212> DNA
   <213> Amaranthus palmeri
<400> 142
   gctctcagag ctcaccctaa acttcccata aaatagtatg tccaagtcaa ttggcctagg 60
   accatacctt attcccttgg ttctacccat atccttctca attttcttta atacccctaa 120
   taattcatga ggcccaagtt ttgtaaagcc tcttaaagca gagttaagaa acttcggttg 180
   atcagtcacg tatgcaggtt 200
<210> 143
   <211> 200
   <212> DNA
   <213> Amaranthus palmeri
<400> 143
   gaagtttagg gtgagctctg agagcctcac tatcccccat gaaaggatat gggaaagacc   60
   atttgtgatg gcaccattga ttgattgtat tgggtctgat gtagaaaatg acactatttg  120
   tacgtggcat tcattatcaa atttttcggg tggaatcttt gaagcatggg gtaaactcgg  180
   tggaagttcc ctaatcggga                                              200
<210> 144
   <211> 200
   <212> DNA
   <213> Amaranthus palmeri
<400> 144
   tcccgattag ggaacttcca ccgagtttac cccatgcttc aaagattcca cccgaaaaat   60
   ttgataatga atgccacgta caaatagtgt cattttctac atcagaccca atacaatcaa  120
   tcaatggtgc catcacaaat ggtctttccc atatcctttc atgggggata gtgaggctct  180
   cagagctcac cctaaacttc                                              200
<210> 145
   <211> 200
   <212> DNA
   <213> Amaranthus palmeri
<400> 145
   ctcggtggaa gttccctaat cgggaaggat ggaatgaaaa gggttttgcc cgttggaaat   60
   cgcttatggg attggtctca taaaacctct gttatgggag tcttgaactt gactcctgat  120
   agctttagtg atggcggaag ttttcaatct gtcgatactg cggttgctaa ggttcgtcag  180
   atgatctcag atggggcaga                                              200
<210> 146
   <211> 200
   <212> DNA
   <213> Amaranthus palmeri
<400> 146
   tctgccccat ctgagatcat ctgacgaacc ttagcaaccg cagtatcgac agattgaaaa 60
   cttccgccat cactaaagct atcaggagtc aagttcaaga ctcccataac agaggtttta 120
   tgagaccaat cccataagcg atttccaacg ggcaaaaccc ttttcattcc atccttcccg 180
   attagggaac ttccaccgag 200
<210> 147
   <211> 200
   <212> DNA
   <213> Amaranthus palmeri
<400> 147
   gtcagatgat ctcagatggg gcagatataa ttgacatcgg ggcacaatca accagaccca 60
   tggcaactag aatttcggct gaagaagagc tagaaagagt agtacctgta ttggaagctg 120
   tcaaggattt gatcgaggaa gaaggaagaa tcttgtcagt ggatacgttt tattctaaag 180
   ttgcttcgga ggccgtcaag 200
<210> 148
   <211> 200
   <212> DNA
   <213> Amaranthus palmeri
<400> 148
   cttgacggcc tccgaagcaa ctttagaata aaacgtatcc actgacaaga ttcttccttc 60
   ttcctcgatc aaatccttga cagcttccaa tacaggtact actctttcta gctcttcttc 120
   agccgaaatt ctagttgcca tgggtctggt tgattgtgcc ccgatgtcaa ttatatctgc 180
   cccatctgag atcatctgac 200
<210> 149
   <211> 200
   <212> DNA
   <213> Amaranthus palmeri
<400> 149
   taaagttgct tcggaggccg tcaagaaggg ggcacacatt gtgaatgatg tctctagtgg 60
   gaaactcgat tccgagatgt ttaatgttgt tgcggacctt aaagttcctt atatagcaat 120
   gcacatgcga ggagatccga cttcaatgca aaactctgag aacttgacct acaatgatgt 180
   ttgtaagcaa gtggctttgg 200
<210> 150
   <211> 200
   <212> DNA
   <213> Amaranthus palmeri
<400> 150
   ccaaagccac ttgcttacaa acatcattgt aggtcaagtt ctcagagttt tgcattgaag 60
   tcggatctcc tcgcatgtgc attgctatat aaggaacttt aaggtccgca acaacattaa 120
   acatctcgga atcgagtttc ccactagaga catcattcac aatgtgtgcc cccttcttga 180
   cggcctccga agcaacttta 200
<210> 151
   <211> 200
   <212> DNA
   <213> Amaranthus palmeri
<400> 151
   gatgtttgta agcaagtggc tttggagttg agttctaggg tcatagatgc agaattatcg 60
   ggaattcctg cttggaggat agttattgat cccggcatcg gattttctaa gaatacgaat 120
   caaaatttgg aaattcttag tggtttacaa aagatacggg aagagatagc taagaagagt 180
   ttggcggtgg ctcattgccc 200
<210> 152
   <211> 200
   <212> DNA
   <213> Amaranthus palmeri
<400> 152
   gggcaatgag ccaccgccaa actcttctta gctatctctt cccgtatctt ttgtaaacca 60
   ctaagaattt ccaaattttg attcgtattc ttagaaaatc cgatgccggg atcaataact 120
   atcctccaag caggaattcc cgataattct gcatctatga ccctagaact caactccaaa 180
   gccacttgct tacaaacatc 200
<210> 153
   <211> 200
   <212> DNA
   <213> Amaranthus palmeri
<400> 153
   agagtttggc ggtggctcat tgccccttgc taattggacc ttcaagaaag aggtttctgg 60
   gcgagatttg tgagcgccct gtagcagctg acagggatcc tgctaccatt gcttctataa 120
   ctgctggagt tttaggtggt gcaaacattg taagagtaca taatgtgagg gataaccttg 180
   atgctgtcaa gttatgtgat 200
<210> 154
   <211> 200
   <212> DNA
   <213> Amaranthus palmeri
<400> 154
   atcacataac ttgacagcat caaggttatc cctcacatta tgtactctta caatgtttgc    60
   accacctaaa actccagcag ttatagaagc aatggtagca ggatccctgt cagctgctac   120
   agggcgctca caaatctcgc ccagaaacct ctttcttgaa ggtccaatta gcaaggggca   180
   atgagccacc gccaaactct                                               200
<210> 155
   <211> 200
   <212> DNA
   <213> Amaranthus palmeri
<400> 155
   gctttccttc actcttcacc agggaacgtt gttgaagttt gttccaagaa gcaagaagtc    60
   gtaattgcaa tcgggagcaa tgttggtgac agattagaaa atttcaacca agctctgcaa   120
   caaatgaaga aattaggcat agacatcaca aggcatggtt gtttatatga gacggaacct   180
   gcatacgtga ctgatcaacc                                               200
<210> 156
   <211> 200
   <212> DNA
   <213> Amaranthus palmeri
<400> 156
   ggttgatcag tcacgtatgc aggttccgtc tcatataaac aaccatgcct tgtgatgtct    60
   atgcctaatt tcttcatttg ttgcagagct tggttgaaat tttctaatct gtcaccaaca   120
   ttgctcccga ttgcaattac gacttcttgc ttcttggaac aaacttcaac aacgttccct   180
   ggtgaagagt gaaggaaagc                                               200
<210> 157
   <211> 200
   <212> DNA
   <213> Amaranthus palmeri
<400> 157
   aacctgcata cgtgactgat caaccgaagt ttcttaactc tgctttaaga ggctttacaa    60
   gacttggacc tcatgaatta ttaggggtat tgaagaaaat tgagaaggat atgggtagaa   120
   ccaagggaat aaggtatggt cctaggccaa ttgacttgga catactattt tatgggaagt   180
   ttagggtgag ctctgagagc                                               200
<210> 158
   <211> 200
   <212> DNA
   <213> Amaranthus palmeri
<400> 158
   gctctcagag ctcaccctaa acttcccata aaatagtatg tccaagtcaa ttggcctagg   60
   accatacctt attcccttgg ttctacccat atccttctca attttcttca atacccctaa  120
   taattcatga ggtccaagtc ttgtaaagcc tcttaaagca gagttaagaa acttcggttg 180
   atcagtcacg tatgcaggtt 200
<210> 159
   <211> 200
   <212> DNA
   <213> Amaranthus palmeri
<400> 159
   gaagtttagg gtgagctctg agagcctcac tatcccccat gaaaggatat gggaaagacc 60
   atttgtgatg gcaccattga ttgattgtat tgggtctgat gtagaaaatg acactatttg 120
   tacgtggcat tcattatcaa atttttcggg tggaatcttt gaagcatggg gtaaactcgg 180
   tggaagctcc ctaatcggga 200
<210> 160
   <211> 200
   <212> DNA
   <213> Amaranthus palmeri
<400> 160
   tcccgattag ggagcttcca ccgagtttac cccatgcttc aaagattcca cccgaaaaat 60
   ttgataatga atgccacgta caaatagtgt cattttctac atcagaccca atacaatcaa 120
   tcaatggtgc catcacaaat ggtctttccc atatcctttc atgggggata gtgaggctct 180
   cagagctcac cctaaacttc 200
<210> 161
   <211> 200
   <212> DNA
   <213> Amaranthus palmeri
<400> 161
   ctcggtggaa gctccctaat cgggaaggat ggaatgaaaa gggttttgcc cgttggaaat 60
   ctcttatggg attggtctca taaaacctct gttatgggag tcttgaactt gactcctgat 120
   agctttagtg atggcggaag ttttcaatct gtcaatactg cggttgctaa ggttcgtcag 180
   atgatctcag atggggcaga 200
<210> 162
   <211> 200
   <212> DNA
   <213> Amaranthus palmeri
<400> 162
   tctgccccat ctgagatcat ctgacgaacc ttagcaaccg cagtattgac agattgaaaa   60
   cttccgccat cactaaagct atcaggagtc aagttcaaga ctcccataac agaggtttta  120
   tgagaccaat cccataagag atttccaacg ggcaaaaccc ttttcattcc atccttcccg  180
   attagggagc ttccaccgag                                              200
<210> 163
   <211> 200
   <212> DNA
   <213> Amaranthus palmeri
<400> 163
   gtcagatgat ctcagatggg gcagatataa ttgacatcgg ggcacaatca accagaccca   60
   tggcaactag aatttcggct gaagaagagc tagcaagagt agtacctgta ttagaagctg  120
   tcaaggattt gatcgaggaa gaaggaagaa tcttgtcagt ggatacgttt tattctaaag  180
   ttgctttgga ggccgtcaag                                              200
<210> 164
   <211> 200
   <212> DNA
   <213> Amaranthus palmeri
<400> 164
   cttgacggcc tccaaagcaa ctttagaata aaacgtatcc actgacaaga ttcttccttc   60
   ttcctcgatc aaatccttga cagcttctaa tacaggtact actcttgcta gctcttcttc  120
   agccgaaatt ctagttgcca tgggtctggt tgattgtgcc ccgatgtcaa ttatatctgc  180
   cccatctgag atcatctgac                                              200
<210> 165
   <211> 200
   <212> DNA
   <213> Amaranthus palmeri
<400> 165
   taaagttgct ttggaggccg tcaagaaggg ggcacacatt gtgaatgatg tctctagtgg 60
   taaactcgat tccgagatgt ttaatgttgt tgcggacctt aaagttcctt atatagcaat 120
   gcacatgcga ggagatccga cttcaatgca aaactctgag aacttgacct acaatgatgt 180
   ttgtaagcaa gtggcttcgg 200
<210> 166
   <211> 200
   <212> DNA
   <213> Amaranthus palmeri
<400> 166
   ccgaagccac ttgcttacaa acatcattgt aggtcaagtt ctcagagttt tgcattgaag 60
   tcggatctcc tcgcatgtgc attgctatat aaggaacttt aaggtccgca acaacattaa 120
   acatctcgga atcgagttta ccactagaga catcattcac aatgtgtgcc cccttcttga 180
   cggcctccaa agcaacttta 200
<210> 167
   <211> 200
   <212> DNA
   <213> Amaranthus palmeri
<400> 167
   gatgtttgta agcaagtggc ttcggagttg agttctaggg tcatagatgc agaattatcg 60
   ggaattcctg cttggaggat agttattgat cccggcatcg gattttctaa gaatacgaat 120
   caaaatttgg aaattcttag tggtttacaa aagatacggg aagagatagc taagaagagt 180
   ttggcggtgg ctcattgccc 200
<210> 168
   <211> 200
   <212> DNA
   <213> Amaranthus palmeri
<400> 168
   gggcaatgag ccaccgccaa actcttctta gctatctctt cccgtatctt ttgtaaacca 60
   ctaagaattt ccaaattttg attcgtattc ttagaaaatc cgatgccggg atcaataact 120
   atcctccaag caggaattcc cgataattct gcatctatga ccctagaact caactccgaa 180
   gccacttgct tacaaacatc 200
<210> 169
   <211> 200
   <212> DNA
   <213> Amaranthus palmeri
<400> 169
   agagtttggc ggtggctcat tgccccttgc taattggacc ttcaagaaag aggtttctgg 60
   gcgagatttg tgagcgccct gtagcagctg acagggatcc tgctaccatt gcttctataa 120
   ctgctggagt tttaggtggt gcaaacattg taagagtaca taatgttagg gataaccttg 180
   atgctgtcaa gttatgtgat 200
<210> 170
   <211> 200
   <212> DNA
   <213> Amaranthus palmeri
<400> 170
   atcacataac ttgacagcat caaggttatc cctaacatta tgtactctta caatgtttgc 60
   accacctaaa actccagcag ttatagaagc aatggtagca ggatccctgt cagctgctac 120
   agggcgctca caaatctcgc ccagaaacct ctttcttgaa ggtccaatta gcaaggggca 180
   atgagccacc gccaaactct 200
<210> 171
   <211> 200
   <212> DNA
   <213> Amaranthus palmeri
<400> 171
   ggtgtttatt tgggcatggc cttatgtgtg atgttggtaa attttgtggc ttcgtccact 60
   aatctgatgt atcggatctg ttaaagggag ccctctcatc ctatgcttat caatgacttt 120
   ttcatatgcc tatcaaacag atcagattgg atcgggttcg agttcaggtc atatataaac 180
   atgtcagcaa acccttgacc 200
<210> 172
   <211> 200
   <212> DNA
   <213> Amaranthus palmeri
<400> 172
   ggtcaagggt ttgctgacat gtttatatat gacctgaact cgaacccgat ccaatctgat 60
   ctgtttgata ggcatatgaa aaagtcattg ataagcatag gatgagaggg ctccctttaa 120
   cagatccgat acatcagatt agtggacgaa gccacaaaat ttaccaacat cacacataag 180
   gccatgccca aataaacacc 200
<210> 173
   <211> 200
   <212> DNA
   <213> Amaranthus palmeri
<400> 173
   taaacatgtc agcaaaccct tgacccatac ccaacccatt tagttaattg gcttgaaaat   60
   cacaacctta attcgaccag cgacagatca ggttgacctg atttcgaccc acttaaccca  120
   tttataattt ttttttcgat ttaagttttt taacattgat taaatctaat tttttaggct  180
   ttaattttaa ttttatgttt                                              200
<210> 174
   <211> 200
   <212> DNA
   <213> Amaranthus palmeri
<400> 174
   aaacataaaa ttaaaattaa agcctaaaaa attagattta atcaatgtta aaaaacttaa   60
   atcgaaaaaa aaattataaa tgggttaagt gggtcgaaat caggtcaacc tgatctgtcg  120
   ctggtcgaat taaggttgtg attttcaagc caattaacta aatgggttgg gtatgggtca  180
   agggtttgct gacatgttta                                              200
<210> 175
   <211> 200
   <212> DNA
   <213> Amaranthus palmeri
<400> 175
   aggctttaat tttaatttta tgtttttttg tttaatttgt atttactatg aaaaataaga   60
   aactattata tgaactgagt ttagcttaca cttattaatt taactcgaaa ttaacacatt  120
   taattaaatg gttatatagg gttttttagt tttaaaattt caacctgaac ctaatctatt  180
   taataaaaag atcagatctg                                              200
<210> 176
   <211> 200
   <212> DNA
   <213> Amaranthus palmeri
<400> 176
   cagatctgat ctttttatta aatagattag gttcaggttg aaattttaaa actaaaaaac   60
   cctatataac catttaatta aatgtgttaa tttcgagtta aattaataag tgtaagctaa  120
   actcagttca tataatagtt tcttattttt catagtaaat acaaattaaa caaaaaaaca  180
   taaaattaaa attaaagcct                                              200
<210> 177
   <211> 200
   <212> DNA
   <213> Amaranthus palmeri
<400> 177
   ctatttaata aaaagatcag atctgatcaa cccatataat taattgggtc aaaatctcaa   60
   ctcaaaccca tttatttcga attaaattca gatcaaatta gcaggtcgga tcaactagcg  120
   ctaatttttc ctctcaagtt ttttcatatg atgccccttg ttttctttga ataaaattca  180
   ccaatttaaa atccctgccc                                              200
<210> 178
   <211> 200
   <212> DNA
   <213> Amaranthus palmeri
<400> 178
   gggcagggat tttaaattgg tgaattttat tcaaagaaaa caaggggcat catatgaaaa   60
   aacttgagag gaaaaattag cgctagttga tccgacctgc taatttgatc tgaatttaat  120
   tcgaaataaa tgggtttgag ttgagatttt gacccaatta attatatggg ttgatcagat  180
   ctgatctttt tattaaatag                                              200
<210> 179
   <211> 200
   <212> DNA
   <213> Amaranthus palmeri
<400> 179
   attcaccaat ttaaaatccc tgcccttgat ccgttggtat tggtatttgg taaagcacat   60
   atggtaaatt ctactccatt gttatctgta gttttggttc taaaccccaa acaaatccta  120
   ctcctagcac cactaatcct cattactttc tgtttgctgt ctttctctac attcctacta  180
   aattaagacc tagatgcagt                                              200
<210> 180
   <211> 200
   <212> DNA
   <213> Amaranthus palmeri
<400> 180
   actgcatcta ggtcttaatt tagtaggaat gtagagaaag acagcaaaca gaaagtaatg 60
   aggattagtg gtgctaggag taggatttgt ttggggttta gaaccaaaac tacagataac 120
   aatggagtag aatttaccat atgtgcttta ccaaatacca ataccaacgg atcaagggca 180
   gggattttaa attggtgaat 200
<210> 181
   <211> 200
   <212> DNA
   <213> Amaranthus palmeri
<400> 181
   tactaaatta agacctagat gcagtgttat tctgttcgag gttgccgaaa acggtacaat 60
   agttgcctaa tttgcaattc atgtttgttc ttgcgttttc tacattgttt actctattgg 120
   aacaaaggtt gaactttcag cctttcaggg agtattttga tttttctgtc aaattacctt 180
   gccagattta tttggctatt 200
<210> 182
   <211> 200
   <212> DNA
   <213> Amaranthus palmeri
<400> 182
   aatagccaaa taaatctggc aaggtaattt gacagaaaaa tcaaaatact ccctgaaagg 60
   ctgaaagttc aacctttgtt ccaatagagt aaacaatgta gaaaacgcaa gaacaaacat 120
   gaattgcaaa ttaggcaact attgtaccgt tttcggcaac ctcgaacaga ataacactgc 180
   atctaggtct taatttagta 200
<210> 183
   <211> 200
   <212> DNA
   <213> Amaranthus palmeri
<400> 183
   accttgccag atttatttgg ctattttgat tgtgtgtatt tctttgtccc ttgtttccat 60
   ggggctaaac tccttttggt taatcttaaa catttcttct aattttgaat tgcagtggta 120
   tgaagtgttg tttaatatac aatcaatttt tctgaactgg gttaatgaag agaagcattg 180
   tagacatgag gaatgtgttc 200
<210> 184
   <211> 200
   <212> DNA
   <213> Amaranthus palmeri
<400> 184
   gaacacattc ctcatgtcta caatgcttct cttcattaac ccagttcaga aaaattgatt   60
   gtatattaaa caacacttca taccactgca attcaaaatt agaagaaatg tttaagatta  120
   accaaaagga gtttagcccc atggaaacaa gggacaaaga aatacacaca atcaaaatag  180
   ccaaataaat ctggcaaggt                                               200
<210> 185
   <211> 200
   <212> DNA
   <213> Amaranthus palmeri
<400> 185
   cattgtagac atgaggaatg tgttcaagca tctcaaaaca accaaattaa tcccaaatgg   60
   gatcttcaac tgtaatagag gtaaatcaca tatattctca tttaatggtt attgttttat  120
   tttaaggatg ttattatgtt gttatcaatg atagaaataa gagaagcttg agcattcaaa  180
   tgtatctatc actcactggt                                               200
<210> 186
   <211> 200
   <212> DNA
   <213> Amaranthus palmeri
<400> 186
   accagtgagt gatagataca tttgaatgct caagcttctc ttatttctat cattgataac   60
   aacataataa catccttaaa ataaaacaat aaccattaaa tgagaatata tgtgatttac  120
   ctctattaca gttgaagatc ccatttggga ttaatttggt tgttttgaga tgcttgaaca  180
   cattcctcat gtctacaatg                                               200
<210> 187
   <211> 200
   <212> DNA
   <213> Amaranthus palmeri
<400> 187
   tcaaatgtat ctatcactca ctggttgtgg acatctaaaa tatggaccaa gagttttctt   60
   catttcttgt accagaattt gcaaatgtat gattaaagtt taggttggag ttgcaataaa  120
   atttcattac cccgacgcta ttaagtggct cacactgagg cggggtttga gggtcggatg  180
   gtgcaacctt caggagaggt                                               200
<210> 188
   <211> 200
   <212> DNA
   <213> Amaranthus palmeri
<400> 188
   acctctcctg aaggttgcac catccgaccc tcaaaccccg cctcagtgtg agccacttaa   60
   tagcgtcggg gtaatgaaat tttattgcaa ctccaaccta aactttaatc atacatttgc  120
   aaattctggt acaagaaatg aagaaaactc ttggtccata ttttagatgt ccacaaccag  180
   tgagtgatag atacatttga                                              200
<210> 189
   <211> 200
   <212> DNA
   <213> Amaranthus palmeri
<400> 189
   ggatggtgca accttcagga gaggttctaa gcattttcaa catgttcaac cgcagagggt   60
   cccgtaaaat tggaggcttc aatattaaat tatgtagtat gtgttaagtg tttaaagata  120
   taaaaatgtt aatgaataca taatttttaa aattgcacag ggctttcaaa gaaatttttg  180
   attttgctcc gcccatggca                                              200
<210> 190
   <211> 200
   <212> DNA
   <213> Amaranthus palmeri
<400> 190
   tgccatgggc ggagcaaaat caaaaatttc tttgaaagcc ctgtgcaatt ttaaaaatta   60
   tgtattcatt aacattttta tatctttaaa cacttaacac atactacata atttaatatt  120
   gaagcctcca attttacggg accctctgcg gttgaacatg ttgaaaatgc ttagaacctc  180
   tcctgaaggt tgcaccatcc                                              200
<210> 191
   <211> 200
   <212> DNA
   <213> Amaranthus palmeri
<400> 191
   ttttgatttt gctccgccca tggcaacctt tggcaaatct ggatagaatt agagaaattt   60
   acttttatgt atgtataatc tgatgaatga atgaatgaac aatgaagttc atttctgagt  120
   caatgaccta tatactatcg aggtttcgtt aaataggata tataaataaa attagagaca 180
   tcttttatcc ctaatgttgt 200
<210> 192
   <211> 200
   <212> DNA
   <213> Amaranthus palmeri
<400> 192
   acaacattag ggataaaaga tgtctctaat tttatttata tatcctattt aacgaaacct 60
   cgatagtata taggtcattg actcagaaat gaacttcatt gttcattcat tcattcatca 120
   gattatacat acataaaagt aaatttctct aattctatcc agatttgcca aaggttgcca 180
   tgggcggagc aaaatcaaaa 200
<210> 193
   <211> 200
   <212> DNA
   <213> Amaranthus palmeri
<400> 193
   agacatcttt tatccctaat gttgtctgtt ttcttttctg ggcgagactg tttttggaga 60
   aaaacgttat gttgaatgct tgatttactc agaactgatg caaacagatg gataaaaaat 120
   ggtgttttcc tagtgtttat gtttcataaa aggatttcaa attccaatcg tatgaagttt 180
   gaaatactgc tttttgtttt 200
<210> 194
   <211> 200
   <212> DNA
   <213> Amaranthus palmeri
<400> 194
   aaaacaaaaa gcagtatttc aaacttcata cgattggaat ttgaaatcct tttatgaaac 60
   ataaacacta ggaaaacacc attttttatc catctgtttg catcagttct gagtaaatca 120
   agcattcaac ataacgtttt tctccaaaaa cagtctcgcc cagaaaagaa aacagacaac 180
   attagggata aaagatgtct 200
<210> 195
   <211> 200
   <212> DNA
   <213> Amaranthus palmeri
<400> 195
   agtttgaaat actgcttttt gtttttgtag cttcgtgttt tgctttcctt cactcttcac 60
   cagggaacgt tgttgaagtt tgttccaaga agcaagaagt cgtaattgca atcgggagca 120
   atgttggtga cagattagaa aatttcaacc aagctctgca acaaatgaag aaattaggca 180
   tagacatcac aaggcatggt 200
<210> 196
   <211> 200
   <212> DNA
   <213> Amaranthus palmeri
<400> 196
   accatgcctt gtgatgtcta tgcctaattt cttcatttgt tgcagagctt ggttgaaatt 60
   ttctaatctg tcaccaacat tgctcccgat tgcaattacg acttcttgct tcttggaaca 120
   aacttcaaca acgttccctg gtgaagagtg aaggaaagca aaacacgaag ctacaaaaac 180
   aaaaagcagt atttcaaact 200
<210> 197
   <211> 200
   <212> DNA
   <213> Amaranthus palmeri
<400> 197
   aggcatagac atcacaaggc atggttgttt atatgagacg gaacctgcat acgtgactga 60
   tcaaccgaag tttcttaact ctgctttaag aggctttaca agacttggac ctcatgaatt 120
   attaggggta ttgaagaaaa ttgagaagga tatgggtaga accaagggaa taaggtatgg 180
   tcctaggcca attgacttgg 200
<210> 198
   <211> 200
   <212> DNA
   <213> Amaranthus palmeri
<400> 198
   ccaagtcaat tggcctagga ccatacctta ttcccttggt tctacccata tccttctcaa 60
   ttttcttcaa tacccctaat aattcatgag gtccaagtct tgtaaagcct cttaaagcag  120
   agttaagaaa cttcggttga tcagtcacgt atgcaggttc cgtctcatat aaacaaccat 180
   gccttgtgat gtctatgcct 200
<210> 199
   <211> 200
   <212> DNA
   <213> Amaranthus palmeri
<400> 199
   tatggtccta ggccaattga cttggacata ctattttatg ggaagtttag ggtgagctct   60
   gagagcctca ctatccccca tgaaaggata tgggaaagac catttgtgat ggcaccattg  120
   attgattgta ttgggtctga tgtagaaaat gacactattt gtacgtggca ttcattatca  180
   aatttttcgg gtggaatctt                                              200
<210> 200
   <211> 200
   <212> DNA
   <213> Amaranthus palmeri
<400> 200
   aagattccac ccgaaaaatt tgataatgaa tgccacgtac aaatagtgtc attttctaca   60
   tcagacccaa tacaatcaat caatggtgcc atcacaaatg gtctttccca tatcctttca  120
   tgggggatag tgaggctctc agagctcacc ctaaacttcc cataaaatag tatgtccaag  180
   tcaattggcc taggaccata                                              200
<210> 201
   <211> 200
   <212> DNA
   <213> Amaranthus palmeri
<400> 201
   tatcaaattt ttcgggtgga atctttgaag catggggtaa actcggtgga agctccctaa   60
   tcgggaagga tggaatgaaa agggttttgc ccgttggaaa tctcttatgg gattggtctc  120
   ataaaacctc tgttatggga gtcttgaact tgactcctga tagctttagt gatggcggaa  180
   gttttcaatc tgtcaatact                                              200
<210> 202
   <211> 200
   <212> DNA
   <213> Amaranthus palmeri
<400> 202
   agtattgaca gattgaaaac ttccgccatc actaaagcta tcaggagtca agttcaagac   60
   tcccataaca gaggttttat gagaccaatc ccataagaga tttccaacgg gcaaaaccct  120
   tttcattcca tccttcccga ttagggagct tccaccgagt ttaccccatg cttcaaagat  180
   tccacccgaa aaatttgata                                              200
<210> 203
   <211> 200
   <212> DNA
   <213> Amaranthus palmeri
<400> 203
   cggaagtttt caatctgtca atactgcggt tgctaaggtt cgtcagatga tctcagatgg    60
   ggcagatata attgacatcg gggcacaatc aaccagaccc atggcaacta gaatttcggc   120
   tgaagaagag ctagcaagag tagtacctgt attagaagct gtcaaggatt tgatcgagga   180
   agaaggaaga atcttgtcag                                                200
<210> 204
   <211> 200
   <212> DNA
   <213> Amaranthus palmeri
<400> 204
   ctgacaagat tcttccttct tcctcgatca aatccttgac agcttctaat acaggtacta    60
   ctcttgctag ctcttcttca gccgaaattc tagttgccat gggtctggtt gattgtgccc   120
   cgatgtcaat tatatctgcc ccatctgaga tcatctgacg aaccttagca accgcagtat   180
   tgacagattg aaaacttccg                                                200
<210> 205
   <211> 200
   <212> DNA
   <213> Amaranthus palmeri
<400> 205
   gaggaagaag gaagaatctt gtcagtggat acgttttatt ctaaagttgc tttggaggcc    60
   gtcaagaagg gggcacacat tgtgaatgat gtctctagtg gtaaactcga ttccgagatg   120
   tttaatgttg ttgcggacct taaagttcct tatatagcaa tgcacatgcg aggagatccg   180
   acttcaatgc aaaactctga                                                200
<210> 206
   <211> 200
   <212> DNA
   <213> Amaranthus palmeri
<400> 206
   tcagagtttt gcattgaagt cggatctcct cgcatgtgca ttgctatata aggaacttta    60
   aggtccgcaa caacattaaa catctcggaa tcgagtttac cactagagac atcattcaca   120
   atgtgtgccc ccttcttgac ggcctccaaa gcaactttag aataaaacgt atccactgac   180
   aagattcttc cttcttcctc                                                200
<210> 207
   <211> 200
   <212> DNA
   <213> Amaranthus palmeri
<400> 207
   atccgacttc aatgcaaaac tctgagaact tgacctacaa tgatgtttgt aagcaagtgg    60
   cttcggagtt gagttctagg gtcatagatg cagaattatc gggaattcct gcttggagga   120
   tagttattga tcccggcatc ggattttcta agaatacgaa tcaaaatttg gaaattctta   180
   gtggtttaca aaagatacgg                                               200
<210> 208
   <211> 200
   <212> DNA
   <213> Amaranthus palmeri
<400> 208
   ccgtatcttt tgtaaaccac taagaatttc caaattttga ttcgtattct tagaaaatcc    60
   gatgccggga tcaataacta tcctccaagc aggaattccc gataattctg catctatgac   120
   cctagaactc aactccgaag ccacttgctt acaaacatca ttgtaggtca agttctcaga   180
   gttttgcatt gaagtcggat                                               200
<210> 209
   <211> 200
   <212> DNA
   <213> Amaranthus palmeri
<400> 209
   tcttagtggt ttacaaaaga tacgggaaga gatagctaag aagagtttgg cggtggctca    60
   ttgccccttg ctaattggac cttcaagaaa gaggtttctg ggcgagattt gtgagcgccc   120
   tgtagcagct gacagggatc ctgctaccat tgcttctata actgctggag ttttaggtgg   180
   tgcaaacatt gtaagagtac                                               200
<210> 210
   <211> 200
   <212> DNA
   <213> Amaranthus palmeri
<400> 210
   gtactcttac aatgtttgca ccacctaaaa ctccagcagt tatagaagca atggtagcag 60
   gatccctgtc agctgctaca gggcgctcac aaatctcgcc cagaaacctc tttcttgaag 120
   gtccaattag caaggggcaa tgagccaccg ccaaactctt cttagctatc tcttcccgta 180
   tcttttgtaa accactaaga 200
<210> 211
   <211> 200
   <212> DNA
   <213> Amaranthus palmeri
<400> 211
   ggtggtgcaa acattgtaag agtacataat gttagggata accttgatgc tgtcaagtta 60
   tgtgatgcca tactcggaaa aactgattaa ctgcttgttt gtaccacctt gtgaatgatg 120
   tctagtggaa ggttcgattg ggattatgat gaagtccgtt agtttgctcg aaagaagagc 180
   tttagcccgt aaggttatag 200
<210> 212
   <211> 200
   <212> DNA
   <213> Amaranthus palmeri
<400> 212
   ctataacctt acgggctaaa gctcttcttt cgagcaaact aacggacttc atcataatcc 60
   caatcgaacc ttccactaga catcattcac aaggtggtac aaacaagcag ttaatcagtt 120
   tttccgagta tggcatcaca taacttgaca gcatcaaggt tatccctaac attatgtact 180
   cttacaatgt ttgcaccacc 200
<210> 213
   <211> 200
   <212> DNA
   <213> Amaranthus palmeri
<400> 213
   agagctttag cccgtaaggt tatagttctt acagatgtag aatcattggc tatttgccta 60
   tttctgctta gaagatcata gcattgatcc agactttgga ttccatgtag aattatgttg 120
   cgactcatgg tcgaattggc cctttgagga attgttgaag ttccttgagt ctatggttca 180
   atcggctcgg ttcgatgaag 200
<210> 214
   <211> 200
   <212> DNA
   <213> Amaranthus palmeri
<400> 214
   cttcatcgaa ccgagccgat tgaaccatag actcaaggaa cttcaacaat tcctcaaagg   60
   gccaattcga ccatgagtcg caacataatt ctacatggaa tccaaagtct ggatcaatgc  120
   tatgatcttc taagcagaaa taggcaaata gccaatgatt ctacatctgt aagaactata  180
   accttacggg ctaaagctct                                              200
<210> 215
   <211> 200
   <212> DNA
   <213> Amaranthus palmeri
<400> 215
   gttcaatcgg ctcggttcga tgaaggttgg actgtgtcga gacaagatgg gtcaaagaga   60
   ccaatggaca agaagtcgac cttgggcaag cttgatcaag ttacttggac gagcagataa  120
   cagcaggctc tggcgagcat tagacataca tatcatttgt tttttgaagt cagatctgca  180
   atcaatcagt tgcaatggac                                              200
<210> 216
   <211> 200
   <212> DNA
   <213> Amaranthus palmeri
<400> 216
   gtccattgca actgattgat tgcagatctg acttcaaaaa acaaatgata tgtatgtcta   60
   atgctcgcca gagcctgctg ttatctgctc gtccaagtaa cttgatcaag cttgcccaag  120
   gtcgacttct tgtccattgg tctctttgac ccatcttgtc tcgacacagt ccaaccttca  180
   tcgaaccgag ccgattgaac                                              200
<210> 217
   <211> 200
   <212> DNA
   <213> Amaranthus palmeri
<400> 217
   ctgcaatcaa tcagttgcaa tggacagacc atatacggtt gttagttgtt cctgtcactc   60
   tttgagttga attagatcat gcaaacactg taagagtctt gattttgtga agctatgtga  120
   tactgtactt ctcaaaatga aatctttgat atgatgttag cttcaagagt tgtttttccc  180
   cccctcacat tctgttctat                                              200
<210> 218
   <211> 200
   <212> DNA
   <213> Amaranthus palmeri
<400> 218
   atagaacaga atgtgagggg gggaaaaaca actcttgaag ctaacatcat atcaaagatt   60
   tcattttgag aagtacagta tcacatagct tcacaaaatc aagactctta cagtgtttgc  120
   atgatctaat tcaactcaaa gagtgacagg aacaactaac aaccgtatat ggtctgtcca  180
   ttgcaactga ttgattgcag                                              200
<210> 219
   <211> 200
   <212> DNA
   <213> Amaranthus palmeri
<400> 219
   ttccccccct cacattctgt tctatgttgt attgttgtgt gctttttgga attgtattcc   60
   tgtaattaga attattcctg caaaatggta ttcctttata tcatcaaagg agtttttgta  120
   cttctaatca ttgatcttta gatgcaaaac tgaattaata caagtgttct tgatgaaggc  180
   ttattgaaga actcactttt                                              200
<210> 220
   <211> 200
   <212> DNA
   <213> Amaranthus palmeri
<400> 220
   aaaagtgagt tcttcaataa gccttcatca agaacacttg tattaattca gttttgcatc   60
   taaagatcaa tgattagaag tacaaaaact cctttgatga tataaaggaa taccattttg  120
   caggaataat tctaattaca ggaatacaat tccaaaaagc acacaacaat acaacataga  180
   acagaatgtg agggggggaa                                              200
<210> 221
   <211> 200
   <212> DNA
   <213> Amaranthus palmeri
<400> 221
   aaggcttatt gaagaactca ctttttattt atgtctaaac ttgttaaaga tgttctgctt   60
   ccgcccttgc cacgggatgt cgggattgac taagggtggt aatcgaggga ttcctatgct  120
   tgaattgact cgtttaagat tttgcgttga ttttaagtca aatgttttgc taagcataaa  180
   ctattctagc tcgacttgaa                                              200
<210> 222
   <211> 200
   <212> DNA
   <213> Amaranthus palmeri
<400> 222
   ttcaagtcga gctagaatag tttatgctta gcaaaacatt tgacttaaaa tcaacgcaaa   60
   atcttaaacg agtcaattca agcataggaa tccctcgatt accaccctta gtcaatcccg  120
   acatcccgtg gcaagggcgg aagcagaaca tctttaacaa gtttagacat aaataaaaag  180
   tgagttcttc aataagcctt                                              200
<210> 223
   <211> 200
   <212> DNA
   <213> Amaranthus palmeri
<400> 223
   ataaactatt ctagctcgac ttgaataatg aagttttcga gtcaagtccg agctgttctg   60
   taagtttatg tatcttgaag cttcttagtt cttacttctt atccttattt gtttgacaca  120
   ttgaaccgcg ttgatgcttg cttgactcaa cccgttaaag tcttgaattc atcacgaatt  180
   cgagtcgagc aagtttaaac                                              200
<210> 224
   <211> 200
   <212> DNA
   <213> Amaranthus palmeri
<400> 224
   gtttaaactt gctcgactcg aattcgtgat gaattcaaga ctttaacggg ttgagtcaag   60
   caagcatcaa cgcggttcaa tgtgtcaaac aaataaggat aagaagtaag aactaagaag  120
   cttcaagata cataaactta cagaacagct cggacttgac tcgaaaactt cattattcaa  180
   gtcgagctag aatagtttat                                              200
<210> 225
   <211> 200
   <212> DNA
   <213> Amaranthus palmeri
<400> 225
   gaattcgagt cgagcaagtt taaacccaac attaactaag ctctcgaggc ttaatgaggc   60
   aggggtagct caagtttgtt attcaagcaa cttaccatag tattctttta aaaggcatat  120
   agtcagttag ttatttgtta atttcagtag aacgaaagaa gtacattatt aaacttggcc  180
   attgatgctt gagctagaga                                              200
<210> 226
   <211> 200
   <212> DNA
   <213> Amaranthus palmeri
<400> 226
   tctctagctc aagcatcaat ggccaagttt aataatgtac ttctttcgtt ctactgaaat   60
   taacaaataa ctaactgact atatgccttt taaaagaata ctatggtaag ttgcttgaat  120
   aacaaacttg agctacccct gcctcattaa gcctcgagag cttagttaat gttgggttta  180
   aacttgctcg actcgaattc                                              200
<210> 227
   <211> 200
   <212> DNA
   <213> Amaranthus palmeri
<400> 227
   tggccattga tgcttgagct agagagcttc ggttgtgatc gaaacgaatc ctgatttcgc   60
   tcttgatctt tgatgtgaag tgacaaggcc ttcataaaac tcctttattc ttcagcagtg  120
   catacaaaaa tcatgtgtgc ccagctccta gctcacatac cgcgtttttc ccctaatcta  180
   gtgtggtcaa ataaaaacag                                              200
<210> 228
   <211> 200
   <212> DNA
   <213> Amaranthus palmeri
<400> 228
   ctgtttttat ttgaccacac tagattaggg gaaaaacgcg gtatgtgagc taggagctgg   60
   gcacacatga tttttgtatg cactgctgaa gaataaagga gttttatgaa ggccttgtca  120
   cttcacatca aagatcaaga gcgaaatcag gattcgtttc gatcacaacc gaagctctct  180
   agctcaagca tcaatggcca                                              200
<210> 229
   <211> 200
   <212> DNA
   <213> Amaranthus palmeri
<400> 229
   atctagtgtg gtcaaataaa aacagcaaga tgtgttgatg tcatgtgcaa tagtaattat 60
   caggtgaagt aatgcaatat gtagccagtc acctcatatc aaaaaaatgt tgggaggaaa 120
   acgtattcgt tcatcactaa acctgtgttt atacatctaa ctgatcgatc agggtttcaa 180
   aatccaaatt aatacaatgg 200
<210> 230
   <211> 200
   <212> DNA
   <213> Amaranthus palmeri
<400> 230
   ccattgtatt aatttggatt ttgaaaccct gatcgatcag ttagatgtat aaacacaggt 60
   ttagtgatga acgaatacgt ttcctcccca acattttttt gatatgaggt gactggctac 120
   atattgcatt acttcacctg ataattacta ttgcacatga catcaacaca tcttgctgtt 180
   tttatttgac cacactagat 200
<210> 231
   <211> 200
   <212> DNA
   <213> Amaranthus palmeri
<400> 231
   ttcaaaatcc aaattaatac aatggcattt gacgaagata tccagaacac atcccccttg 60
   tttctgctag ataatcaact attctgattc tgctgtaata cctcagcttc ggccctagcc 120
   atgggatcgt cctctttttc tccttcacgc tcctcggcca gtttctttct gtgcagttct 180
   tcagcagctt ctattgaagc 200
<210> 232
   <211> 200
   <212> DNA
   <213> Amaranthus palmeri
<400> 232
   gcttcaatag aagctgctga agaactgcac agaaagaaac tggccgagga gcgtgaagga 60
   gaaaaagagg acgatcccat ggctagggcc gaagctgagg tattacagca gaatcagaat 120
   agttgattat ctagcagaaa caagggggat gtgttctgga tatcttcgtc aaatgccatt 180
   gtattaattt ggattttgaa 200
<210> 233
   <211> 200
   <212> DNA
   <213> Amaranthus rudis
<400> 233
   aatgggagtc ttgaacttga ctcctgatag ctttagtgat ggcggaaggt ttcaatctgt   60
   cgatactgcg gttgctaagg ttcgtcagat gatctctgac ggggcggaca taattgacat  120
   tggggcgcag tcaacaagac ccatggcaac taggatttcg gctgaagaag agctagaaag  180
   agtcgtacct gtattagaag                                              200
<210> 234
   <211> 200
   <212> DNA
   <213> Amaranthus rudis
<400> 234
   cttctaatac aggtacgact ctttctagct cttcttcagc cgaaatccta gttgccatgg   60
   gtcttgttga ctgcgcccca atgtcaatta tgtccgcccc gtcagagatc atctgacgaa  120
   ccttagcaac cgcagtatcg acagattgaa accttccgcc atcactaaag ctatcaggag  180
   tcaagttcaa gactcccatt                                              200
<210> 235
   <211> 200
   <212> DNA
   <213> Amaranthus rudis
<400> 235
   gaaagagtcg tacctgtatt agaagctgtc aaggatttga tcgaggaaga aggaagaatc   60
   ttgtcagtgg atacgtttta ttctaaagtt gcttcggagg ccgtcaagaa gggggcacac  120
   attgtgaatg atgtctctag tgggaaactc gatcccgaga tgtttaatgt tgttgcggac  180
   cttaaagttc cttatatagc                                              200
<210> 236
   <211> 200
   <212> DNA
   <213> Amaranthus rudis
<400> 236
   gctatataag gaactttaag gtccgcaaca acattaaaca tctcgggatc gagtttccca   60
   ctagagacat cattcacaat gtgtgccccc ttcttgacgg cctccgaagc aactttagaa  120
   taaaacgtat ccactgacaa gattcttcct tcttcctcga tcaaatcctt gacagcttct  180
   aatacaggta cgactctttc                                              200
<210> 237
   <211> 200
   <212> DNA
   <213> Amaranthus rudis
<400> 237
   cggaccttaa agttccttat atagcaatgc acatgcgagg agatccgact tcaatgcaaa    60
   attctgagaa cttgacgtac aatgatgtgt gtaagcaagt ggcttcggag ttgagctcta   120
   gggtcgtaga tgcagaatta tcgggaattc ctgcttggag gatggttatt gatcccggca   180
   ttggattttc taagaatacg                                                200
<210> 238
   <211> 200
   <212> DNA
   <213> Amaranthus rudis
<400> 238
   cgtattctta gaaaatccaa tgccgggatc aataaccatc ctccaagcag gaattcccga    60
   taattctgca tctacgaccc tagagctcaa ctccgaagcc acttgcttac acacatcatt   120
   gtacgtcaag ttctcagaat tttgcattga agtcggatct cctcgcatgt gcattgctat   180
   ataaggaact ttaaggtccg                                                200
<210> 239
   <211> 200
   <212> DNA
   <213> Amaranthus rudis
<400> 239
   cggcattgga ttttctaaga atacgaagca aaatttggaa attcttagtg gtttacaaaa    60
   gatacggcaa gagatagcta agaagagttt ggcggtggct cattgcccct tgctaattgg   120
   accttcaaga aagaggtttc tgggcgagat ttgcaatcgc cctgtagcag ctgataggga   180
   tcctgctacc gttgcttcta                                                200
<210> 240
   <211> 200
   <212> DNA
   <213> Amaranthus rudis
<400> 240
   tagaagcaac ggtagcagga tccctatcag ctgctacagg gcgattgcaa atctcgccca    60
   gaaacctctt tcttgaaggt ccaattagca aggggcaatg agccaccgcc aaactcttct   120
   tagctatctc ttgccgtatc ttttgtaaac cactaagaat ttccaaattt tgcttcgtat   180
   tcttagaaaa tccaatgccg                                                200
<210> 241
   <211> 200
   <212> DNA
   <213> Amaranthus rudis
<400> 241
   aggatttcgg ctgaagaaga gctagaaaga gttgtacctg tattggaagc tgttaaggat   60
   gtgatcgagg aagaaggaag aatcttatcg gtggatacgt tttattctaa agttgcttcg  120
   gaggccgtca agaagggggc acacattgtg aatgatgttt ctagtgggaa actcgatccc  180
   gagatgttta atgttgttgc                                              200
<210> 242
   <211> 200
   <212> DNA
   <213> Amaranthus rudis
<400> 242
   gcaacaacat taaacatctc gggatcgagt ttcccactag aaacatcatt cacaatgtgt   60
   gcccccttct tgacggcctc cgaagcaact ttagaataaa acgtatccac cgataagatt  120
   cttccttctt cctcgatcac atccttaaca gcttccaata caggtacaac tctttctagc  180
   tcttcttcag ccgaaatcct                                              200
<210> 243
   <211> 200
   <212> DNA
   <213> Amaranthus rudis
<400> 243
   atcccgagat gtttaatgtt gttgcggacc ttaaagttcc ttatatagca atgcacatgc   60
   gaggagatcc gacttcaatg caaaattctg agaacttgac gtacaatgat gtgtgtaagc  120
   aagtggcttc ggagttgagc tctagggtcg tagatgcaga attatcggga attcctgctt  180
   ggaggatggt tattgatccc                                              200
<210> 244
   <211> 200
   <212> DNA
   <213> Amaranthus rudis
<400> 244
   gggatcaata accatcctcc aagcaggaat tcccgataat tctgcatcta cgaccctaga 60
   gctcaactcc gaagccactt gcttacacac atcattgtac gtcaagttct cagaattttg 120
   cattgaagtc ggatctcctc gcatgtgcat tgctatataa ggaactttaa ggtccgcaac 180
   aacattaaac atctcgggat 200
<210> 245
   <211> 200
   <212> DNA
   <213> Amaranthus rudis
<400> 245
   tgcttggagg atggttattg atcccggcat tggattttct aagaatacga agcaaaattt 60
   ggaaattctt agtggtttac aaaagatacg gcaagagata gctaagaaga gtttggcggt 120
   ggctcattgc cccttgctaa ttggaccttc aagaaagaag gtttctgggc gagatttgca 180
   atcgccctgt agcagctgat 200
<210> 246
   <211> 200
   <212> DNA
   <213> Amaranthus rudis
<400> 246
   atcagctgct acagggcgat tgcaaatctc gcccagaaac cttctttctt gaaggtccaa 60
   ttagcaaggg gcaatgagcc accgccaaac tcttcttagc tatctcttgc cgtatctttt 120
   gtaaaccact aagaatttcc aaattttgct tcgtattctt agaaaatcca atgccgggat 180
   caataaccat cctccaagca 200
<210> 247
   <211> 200
   <212> DNA
   <213> Amaranthus rudis
<400> 247
   attagaggca tcttttatcc ctaatgctgt ccgttttctt ttctgggtga gactattttg 60
   gaagaaaaaa atttatgttc aatgtttaat ttactcagaa ttgatgcaaa cagatggata 120
   aaaatggtgt tttcttatgg tttatgtttc aataaaagga tttcaaattc taatcatatg 180
   aagtttgaaa tactatgtgt 200
<210> 248
   <211> 200
   <212> DNA
   <213> Amaranthus rudis
<400> 248
   acacatagta tttcaaactt catatgatta gaatttgaaa tccttttatt gaaacataaa 60
   ccataagaaa acaccatttt tatccatctg tttgcatcaa ttctgagtaa attaaacatt 120
   gaacataaat ttttttcttc caaaatagtc tcacccagaa aagaaaacgg acagcattag 180
   ggataaaaga tgcctctaat 200
<210> 249
   <211> 200
   <212> DNA
   <213> Amaranthus rudis
<400> 249
   atatgaagtt tgaaatacta tgtgtttttg tagcttcttg tattgctttc cttcactcat 60
   caccagtgaa cgatgttgaa gtttgttcca agaagcaaga agtcgtaatt gcaatcggga 120
   gcaatgttgg tgacagatta gaaaatttca acgaagctct gcaacaaatg aagaaattag 180
   gcatagacat cacaaggcat 200
<210> 250
   <211> 200
   <212> DNA
   <213> Amaranthus rudis
<400> 250
   atgccttgtg atgtctatgc ctaatttctt catttgttgc agagcttcgt tgaaattttc 60
   taatctgtca ccaacattgc tcccgattgc aattacgact tcttgcttct tggaacaaac 120
   ttcaacatcg ttcactggtg atgagtgaag gaaagcaata caagaagcta caaaaacaca 180
   tagtatttca aacttcatat 200
<210> 251
   <211> 200
   <212> DNA
   <213> Amaranthus rudis
<400> 251
   attaggcata gacatcacaa ggcatggttg tttatacgag acggaacctg cttacgtgac 60
   tgatcagccg aagtttctta actctgcttt aagaggcttt acaagacttg ggcctcatga 120
   attattaggg gtattgaaga gaatcgagaa ggatatgggc agaaccaagg gaataaggta 180
   tggtcctagg ccaattgact 200
<210> 252
   <211> 200
   <212> DNA
   <213> Amaranthus rudis
<400> 252
   agtcaattgg cctaggacca taccttattc ccttggttct gcccatatcc ttctcgattc   60
   tcttcaatac ccctaataat tcatgaggcc caagtcttgt aaagcctctt aaagcagagt  120
   taagaaactt cggctgatca gtcacgtaag caggttccgt ctcgtataaa caaccatgcc  180
   ttgtgatgtc tatgcctaat                                              200
<210> 253
   <211> 200
   <212> DNA
   <213> Amaranthus rudis
<400> 253
   cgctactgct gcagctgtca ccgctgcaat tttgaatggt gctaacatag taagggtaca   60
   taatgtagga tatagttcag atgctgcaaa gttttgtgat gcacctatac tcattttacc  120
   catctcgctc ctaatggacc ccagcccctt aataacgtca atgttgtgcc cacatttctt  180
   ggagaaccca atgccaggat                                              200
<210> 254
   <211> 200
   <212> DNA
   <213> Amaranthus rudis
<400> 254
   atcctggcat tgggttctcc aagaaatgtg ggcacaacat tgacgttatt aaggggctgg   60
   ggtccattag gagcgagatg ggtaaaatga gtataggtgc atcacaaaac tttgcagcat  120
   ctgaactata tcctacatta tgtaccctta ctatgttagc accattcaaa attgcagcgg  180
   tgacagctgc agcagtagcg                                              200
<210> 255
   <211> 200
   <212> DNA
   <213> Amaranthus rudis
<400> 255
   ctgagagcct cactattccc catgaaagga tatgggaaag accatttgtg atggcaccat 60
   taattgattg tattgggtct gatgtagaaa atgacactat ttgtacatgg cattcgttat 120
   caaatttttc gggtggaata tttgaagcat gggataaact cggaggaagt tccttaatcg 180
   ggaaggatgg aatgaaaagg 200
<210> 256
   <211> 200
   <212> DNA
   <213> Amaranthus rudis
<400> 256
   ccttttcatt ccatccttcc cgattaagga acttcctccg agtttatccc atgcttcaaa 60
   tattccaccc gaaaaatttg ataacgaatg ccatgtacaa atagtgtcat tttctacatc 120
   agacccaata caatcaatta atggtgccat cacaaatggt ctttcccata tcctttcatg 180
   gggaatagtg aggctctcag 200
<210> 257
   <211> 200
   <212> DNA
   <213> Amaranthus rudis
<400> 257
   aatcgggaag gatggaatga aaagggtttt gcccgttggg aaccgtttat gggattggtc 60
   tcataaaacc tcagtaatgg gagtcttgaa cttgactcct gatagcttta gtgatggcgg 120
   aaggtttcaa tctgtcgata ctgcggttgc taaggttcgt cagatgatct ctgacggggc 180
   ggacataatt gacattgggg 200
<210> 258
   <211> 200
   <212> DNA
   <213> Amaranthus rudis
<400> 258
   ccccaatgtc aattatgtcc gccccgtcag agatcatctg acgaacctta gcaaccgcag 60
   tatcgacaga ttgaaacctt ccgccatcac taaagctatc aggagtcaag ttcaagactc 120
   ccattactga ggttttatga gaccaatccc ataaacggtt cccaacgggc aaaacccttt 180
   tcattccatc cttcccgatt 200
<210> 259
   <211> 200
   <212> DNA
   <213> Amaranthus rudis
<400> 259
   gcgtaccttc actcatcacc agtggacgat gttgaacttt gttctaagaa gcaagaagtc 60
   ataattgcaa tcgggagcaa tgttggtgac agattagaaa attttaacca agctctgcaa 120
   caaatgaaga aattaggcat agacatcaca aggcatggtt gtttatatga gacagaaccc 180
   gcatacgtga ctgatcaacc 200
<210> 260
   <211> 200
   <212> DNA
   <213> Amaranthus rudis
<400> 260
   ggttgatcag tcacgtatgc gggttctgtc tcatataaac aaccatgcct tgtgatgtct 60
   atgcctaatt tcttcatttg ttgcagagct tggttaaaat tttctaatct gtcaccaaca 120
   ttgctcccga ttgcaattat gacttcttgc ttcttagaac aaagttcaac atcgtccact 180
   ggtgatgagt gaaggtacgc 200
<210> 261
   <211> 200
   <212> DNA
   <213> Amaranthus rudis
<400> 261
   aacccgcata cgtgactgat caaccgaagt ttcttaactc tgctttaaga ggctttacaa 60
   gacttgggcc tcatgaatta ttaggggtat tgaagaaaat tgagaagaat atgggtagaa 120
   ccaagggaat aaggtatggt cctaggccaa ttgacttgga catactattt tatgggaagt 180
   ttagggtgag ctctgagagc 200
<210> 262
   <211> 200
   <212> DNA
   <213> Amaranthus rudis
<400> 262
   gctctcagag ctcaccctaa acttcccata aaatagtatg tccaagtcaa ttggcctagg 60
   accatacctt attcccttgg ttctacccat attcttctca attttcttca atacccctaa 120
   taattcatga ggcccaagtc ttgtaaagcc tcttaaagca gagttaagaa acttcggttg 180
   atcagtcacg tatgcgggtt 200
<210> 263
   <211> 200
   <212> DNA
   <213> Amaranthus rudis
<400> 263
   gaagtttagg gtgagctctg agagcctcac tattccccat gaaaggatat gggaaagacc   60
   atttgtgatg gcaccattaa ttgattgtat tgggtctggt gtagaaaatg acactatttg  120
   tacatggcat tcgttatcaa atttttcggg tggaatcttt gaagcatggg ataaactcgg  180
   tggaagttcc ctaatcggga                                              200
<210> 264
   <211> 200
   <212> DNA
   <213> Amaranthus rudis
<400> 264
   tcccgattag ggaacttcca ccgagtttat cccatgcttc aaagattcca cccgaaaaat   60
   ttgataacga atgccatgta caaatagtgt cattttctac accagaccca atacaatcaa  120
   ttaatggtgc catcacaaat ggtctttccc atatcctttc atggggaata gtgaggctct  180
   cagagctcac cctaaacttc                                              200
<210> 265
   <211> 200
   <212> DNA
   <213> Amaranthus rudis
<400> 265
   ctcggtggaa gttccctaat cgggaaggat ggaatgaaaa gggttttgcc cgttgggaac   60
   cgcttatggg attggtctca taaaacctct gtaatgggag tcttgaactt gactcctgat  120
   agctttagtg atggaggaag ttttcaatct gtcgatactg cggttgctaa ggttcgtcag  180
   atgatatcag atggggccga                                              200
<210> 266
   <211> 200
   <212> DNA
   <213> Amaranthus rudis
<400> 266
   tcggccccat ctgatatcat ctgacgaacc ttagcaaccg cagtatcgac agattgaaaa   60
   cttcctccat cactaaagct atcaggagtc aagttcaaga ctcccattac agaggtttta  120
   tgagaccaat cccataagcg gttcccaacg ggcaaaaccc ttttcattcc atccttcccg  180
   attagggaac ttccaccgag                                              200
<210> 267
   <211> 200
   <212> DNA
   <213> Amaranthus rudis
<400> 267
   gtcagatgat atcagatggg gccgacataa ttgacattgg ggcacagtca acaagaccca 60
   tggcaactag gatttcggct gaagaagagc tagaaagagt tgtacctgta ttggaagctg 120
   ttaaggatgt gatcgaggaa gaaggaagaa tcttatcggt ggatactttt tattcaaaag 180
   ttgcttctga ggccgtcaag 200
<210> 268
   <211> 200
   <212> DNA
   <213> Amaranthus rudis
<400> 268
   cttgacggcc tcagaagcaa cttttgaata aaaagtatcc accgataaga ttcttccttc 60
   ttcctcgatc acatccttaa cagcttccaa tacaggtaca actctttcta gctcttcttc 120
   agccgaaatc ctagttgcca tgggtcttgt tgactgtgcc ccaatgtcaa ttatgtcggc 180
   cccatctgat atcatctgac 200
<210> 269
   <211> 200
   <212> DNA
   <213> Amaranthus rudis
<400> 269
   aaaagttgct tctgaggccg tcaagaaggg ggcacacatt gtgaatgatg tctctagtgg 60
   gaaattcgat cctgagatgt tcaatgttgt tgcggacctt aaagttcctt atatagcaat 120
   gcacatgcga ggagatccga cttcaatgca aaattctgag aacttgacgt acagtgatgt 180
   ttgtaagcaa gtggcttcgg 200
<210> 270
   <211> 200
   <212> DNA
   <213> Amaranthus rudis
<400> 270
   ccgaagccac ttgcttacaa acatcactgt acgtcaagtt ctcagaattt tgcattgaag 60
   tcggatctcc tcgcatgtgc attgctatat aaggaacttt aaggtccgca acaacattga 120
   acatctcagg atcgaatttc ccactagaga catcattcac aatgtgtgcc cccttcttga 180
   cggcctcaga agcaactttt 200
<210> 271
   <211> 200
   <212> DNA
   <213> Amaranthus rudis
<400> 271
   gatgtttgta agcaagtggc ttcggagttg agctctaggg tcatagatgc agaattatcg 60
   ggaattcctg cttggagaat agttattgat cccggcattg gattttctaa gaatacgaag 120
   caaaatttgg aaattcttac tggtttacaa aagatacggc tagagatagc taagaagagt 180
   ttggcggtgg ctcattgccc 200
<210> 272
   <211> 200
   <212> DNA
   <213> Amaranthus rudis
<400> 272
   gggcaatgag ccaccgccaa actcttctta gctatctcta gccgtatctt ttgtaaacca 60
   gtaagaattt ccaaattttg cttcgtattc ttagaaaatc caatgccggg atcaataact 120
   attctccaag caggaattcc cgataattct gcatctatga ccctagagct caactccgaa 180
   gccacttgct tacaaacatc 200
<210> 273
   <211> 200
   <212> DNA
   <213> Amaranthus rudis
<400> 273
   agagtttggc ggtggctcat tgccccttgc taattggacc ttcacgaaag aggtttctgg 60
   gtgagatttg taatcgccct gtagcagctg atagggatcc tgctaccgtc gcttctatca 120
   ctgctggagt tttaggtggt gcaaacattg taagagtaca taatgtgagg gataaccttg 180
   atgcagtcaa gttatgtgat 200
<210> 274
   <211> 200
   <212> DNA
   <213> Amaranthus rudis
<400> 274
   atcacataac ttgactgcat caaggttatc cctcacatta tgtactctta caatgtttgc    60
   accacctaaa actccagcag tgatagaagc gacggtagca ggatccctat cagctgctac   120
   agggcgatta caaatctcac ccagaaacct ctttcgtgaa ggtccaatta gcaaggggca   180
   atgagccacc gccaaactct                                               200
<210> 275
   <211> 200
   <212> DNA
   <213> Amaranthus rudis
<400> 275
   gtaatgggag tcttgaactt gactcctgat agctttagtg atggcggaag gtttcaatct    60
   gtcgatactg cggttgctaa ggttcgtcag atgatctctg acggggcgga cataattgac   120
   attggggcgc agtcaacaag acccttggca actaggattt cggctgaaga agagctagaa   180
   agagtcgtac ctgtattaga                                               200
<210> 276
   <211> 200
   <212> DNA
   <213> Amaranthus rudis
<400> 276
   tctaatacag gtacgactct ttctagctct tcttcagccg aaatcctagt tgccaagggt    60
   cttgttgact gcgccccaat gtcaattatg tccgccccgt cagagatcat ctgacgaacc   120
   ttagcaaccg cagtatcgac agattgaaac cttccgccat cactaaagct atcaggagtc   180
   aagttcaaga ctcccattac                                               200
<210> 277
   <211> 200
   <212> DNA
   <213> Amaranthus rudis
<400> 277
   tagaaagagt cgtacctgta ttagaagctg tcaaggattt gatcgaggaa gaaggaagaa    60
   tcttgtcagt ggatacgttt tattctaaag ttgcttcgga ggccgtcaag aagggggcac    120
   acattgtgaa tgatgtttct agtgggaaac tcgatcccga gatgtttaat gttgttgcgg   180
   accttaaagt tccttatata                                               200
<210> 278
   <211> 200
   <212> DNA
   <213> Amaranthus rudis
<400> 278
   tatataagga actttaaggt ccgcaacaac attaaacatc tcgggatcga gtttcccact   60
   agaaacatca ttcacaatgt gtgccccctt cttgacggcc tccgaagcaa ctttagaata  120
   aaacgtatcc actgacaaga ttcttccttc ttcctcgatc aaatccttga cagcttctaa  180
   tacaggtacg actctttcta                                              200
<210> 279
   <211> 200
   <212> DNA
   <213> Amaranthus rudis
<400> 279
   tgcggacctt aaagttcctt atatagcaat gcacatgcga ggagatccga cttcaatgca   60
   aaattctgag aacttgacgt acagtgatgt ttgtaagcaa gtggcttcgg agttgagctc  120
   tagggtcata gatgcagaat tatcgggaat tcctgcttgg agaatagtta ttgatcccgg  180
   cattggattt tctaagaata                                              200
<210> 280
   <211> 200
   <212> DNA
   <213> Amaranthus rudis
<400> 280
   tattcttaga aaatccaatg ccgggatcaa taactattct ccaagcagga attcccgata   60
   attctgcatc tatgacccta gagctcaact ccgaagccac ttgcttacaa acatcactgt  120
   acgtcaagtt ctcagaattt tgcattgaag tcggatctcc tcgcatgtgc attgctatat  180
   aaggaacttt aaggtccgca                                              200
<210> 281
   <211> 200
   <212> DNA
   <213> Amaranthus rudis
<400> 281
   cccggcattg gattttctaa gaatacgaag caaaatttgg aaattcttac tggtttacaa   60
   aagatacggc tagagatagc taagaagagt ttggcggtgg ctcattgccc cttgctaatt  120
   ggaccttcac gaaagaggtt tctgggtgag atttgtaatc gccctgtagc agctgatagg  180
   gatcctgcta ccgtcgcttc                                              200
<210> 282
   <211> 200
   <212> DNA
   <213> Amaranthus rudis
<400> 282
   gaagcgacgg tagcaggatc cctatcagct gctacagggc gattacaaat ctcacccaga 60
   aacctctttc gtgaaggtcc aattagcaag gggcaatgag ccaccgccaa actcttctta 120
   gctatctcta gccgtatctt ttgtaaacca gtaagaattt ccaaattttg cttcgtattc 180
   ttagaaaatc caatgccggg 200
<210> 283
   <211> 200
   <212> DNA
   <213> Amaranthus rudis
<400> 283
   tgatttactc agaattgatg caaacaaatg gataaaaatg gtgttttcct atggtttatg 60
   tttcgataaa aggatttcaa atcccaatcg tatgaagttt gaaatacaac tatgtgtttt 120
   tgtagcttct tgtattgctt tccttcactc atcaccagtg aacgatgttg aagtttgttc 180
   caagaagcaa gaagtcgtaa 200
<210> 284
   <211> 200
   <212> DNA
   <213> Amaranthus rudis
<400> 284
   ttacgacttc ttgcttcttg gaacaaactt caacatcgtt cactggtgat gagtgaagga 60
   aagcaataca agaagctaca aaaacacata gttgtatttc aaacttcata cgattgggat 120
   ttgaaatcct tttatcgaaa cataaaccat aggaaaacac catttttatc catttgtttg 180
   catcaattct gagtaaatca 200
<210> 285
   <211> 200
   <212> DNA
   <213> Amaranthus rudis
<400> 285
   tgttccaaga agcaagaagt cgtaattgca atcgggagca atgttggtga cagattagaa 60
   aatttcaacg aagctctgca acaaatgaag aaattaggca tagacatcac aaggcatggt 120
   tgtttatacg agacggaacc tgcttacgtg actgatcagc cgaagtttct taactctgct 180
   ttaagaggct ttacaagact 200
<210> 286
   <211> 200
   <212> DNA
   <213> Amaranthus rudis
<400> 286
   agtcttgtaa agcctcttaa agcagagtta agaaacttcg gctgatcagt cacgtaagca   60
   ggttccgtct cgtataaaca accatgcctt gtgatgtcta tgcctaattt cttcatttgt  120
   tgcagagctt cgttgaaatt ttctaatctg tcaccaacat tgctcccgat tgcaattacg  180
   acttcttgct tcttggaaca                                              200
<210> 287
   <211> 200
   <212> DNA
   <213> Amaranthus rudis
<400> 287
   ctgctttaag aggctttaca agacttgggc ctcatgaatt attaggggta ttgaagagaa   60
   tcgagaagga tatgggcaga accaagggaa taaggtatgg tcctaggcca attgacttgg  120
   acatactatt ttatgggaag tttagggtga gctctgagag cctcactatt ccccatgaaa  180
   ggatatggga aagaccattt                                              200
<210> 288
   <211> 200
   <212> DNA
   <213> Amaranthus rudis
<400> 288
   aaatggtctt tcccatatcc tttcatgggg aatagtgagg ctctcagagc tcaccctaaa   60
   cttcccataa aatagtatgt ccaagtcaat tggcctagga ccatacctta ttcccttggt  120
   tctgcccata tccttctcga ttctcttcaa tacccctaat aattcatgag gcccaagtct  180
   tgtaaagcct cttaaagcag                                              200
<210> 289
   <211> 200
   <212> DNA
   <213> Amaranthus rudis
<400> 289
   tggcaactag gatttcggct gaagaagagc tagaaagagt cgtacctgta ttagaagctg 60
   tcaaggattt gatcgaggaa gaaggaagaa tcttgtcagt ggatacgttt tattctaaag 120
   ttgcttcgga ggccgtcaag aagggggcac acattgtgaa tgatgtttct agtgggaaac 180
   tcgatcccga gatgtttaat 200
<210> 290
   <211> 200
   <212> DNA
   <213> Amaranthus rudis
<400> 290
   attaaacatc tcgggatcga gtttcccact agaaacatca ttcacaatgt gtgccccctt 60
   cttgacggcc tccgaagcaa ctttagaata aaacgtatcc actgacaaga ttcttccttc 120
   ttcctcgatc aaatccttga cagcttctaa tacaggtacg actctttcta gctcttcttc 180
   agccgaaatc ctagttgcca 200
<210> 291
   <211> 200
   <212> DNA
   <213> Amaranthus rudis
<400> 291
   gaaactcgat cccgagatgt ttaatgttgt tgcggacctt aaagttcctt atatagcaat 60
   gcacatgcga ggagatccga cttcaatgca aaattctgag aacttgacgt acaatgatgt 120
   gtgtaagcaa gtggcttcgg agttgagctc tagggtcgta gatgcagaat tatcgggaat 180
   tcctgcttgg aggatggtta 200
<210> 292
   <211> 200
   <212> DNA
   <213> Amaranthus rudis
<400> 292
   taaccatcct ccaagcagga attcccgata attctgcatc tacgacccta gagctcaact 60
   ccgaagccac ttgcttacac acatcattgt acgtcaagtt ctcagaattt tgcattgaag 120
   tcggatctcc tcgcatgtgc attgctatat aaggaacttt aaggtccgca acaacattaa 180
   acatctcggg atcgagtttc 200
<210> 293
   <211> 200
   <212> DNA
   <213> Amaranthus rudis
<400> 293
   ggaattcctg cttggaggat ggttattgat cccggcattg gattttctaa gaatacgaag    60
   caaaatttgg aaattcttag tggtttacaa aagatacggc aagagatagc taagaagagt   120
   ttggcggtgg ctcattgccc cttgctaatt ggaccttcaa gaaagaggtt tctgggcgag   180
   atttgcaatc gccctgtagc                                               200
<210> 294
   <211> 200
   <212> DNA
   <213> Amaranthus rudis
<400> 294
   gctacagggc gattgcaaat ctcgcccaga aacctctttc ttgaaggtcc aattagcaag    60
   gggcaatgag ccaccgccaa actcttctta gctatctctt gccgtatctt ttgtaaacca   120
   ctaagaattt ccaaattttg cttcgtattc ttagaaaatc caatgccggg atcaataacc   180
   atcctccaag caggaattcc                                               200
<210> 295
   <211> 200
   <212> DNA
   <213> Amaranthus rudis
<400> 295
   gcgagatttg caatcgccct gtagcagctg atagggatcc tgctaccgtt gcttctatca    60
   ctgctggagt tttaggtggt gcaaacattg taagagtaca taatgtgagg gataaccttg   120
   atgcagtcaa gttatgtgat gccatactca gaaaaacgga ttagccgatt tttttggatt   180
   ccagagccga tgttccagat                                               200
<210> 296
   <211> 200
   <212> DNA
   <213> Amaranthus rudis
<400> 296
   atctggaaca tcggctctgg aatccaaaaa aatcggctaa tccgtttttc tgagtatggc    60
   atcacataac ttgactgcat caaggttatc cctcacatta tgtactctta caatgtttgc   120
   accacctaaa actccagcag tgatagaagc aacggtagca ggatccctat cagctgctac   180
   agggcgattg caaatctcgc                                               200
<210> 297
   <211> 200
   <212> DNA
   <213> Amaranthus rudis
<400> 297
   tgtttcgata aaaggatttc aaatcccaat cgtatgaagt ttgaaataca actatgtgtt   60
   tttgcagctt tgttttttgc gtaccttcac tcatcaccag tggacgatgt tgaactttgt  120
   tctaagaagc aagaagtcat aattgcaatc gggagcaatg ttggtgacag attagaaaat  180
   tttaaccaag ctctgcaaca                                              200
<210> 298
   <211> 200
   <212> DNA
   <213> Amaranthus rudis
<400> 298
   tgttgcagag cttggttaaa attttctaat ctgtcaccaa cattgctccc gattgcaatt   60
   atgacttctt gcttcttaga acaaagttca acatcgtcca ctggtgatga gtgaaggtac  120
   gcaaaaaaca aagctgcaaa aacacatagt tgtatttcaa acttcatacg attgggattt  180
   gaaatccttt tatcgaaaca                                              200
<210> 299
   <211> 200
   <212> DNA
   <213> Amaranthus rudis
<400> 299
   aaaattttaa ccaagctctg caacaaatga agaaattagg catagacatc acaaggcatg   60
   gttgtttata tgagacagaa cccgcatacg tgactgatca accgaagttt cttaactctg  120
   ctttaagagg ctttacaaga cttgggcctc atgaattatt aggggtattg aagaaaattg  180
   agaagaatat gggtagaacc                                              200
<210> 300
   <211> 200
   <212> DNA
   <213> Amaranthus rudis
<400> 300
   ggttctaccc atattcttct caattttctt caatacccct aataattcat gaggcccaag 60
   tcttgtaaag cctcttaaag cagagttaag aaacttcggt tgatcagtca cgtatgcggg  120
   ttctgtctca tataaacaac catgccttgt gatgtctatg cctaatttct tcatttgttg  180
   cagagcttgg ttaaaatttt                                              200
<210> 301
   <211> 200
   <212> DNA
   <213> Amaranthus rudis
<400> 301
   aattgagaag aatatgggta gaaccaaggg aataaggtat ggtcctaggc caattgactt 60
   ggacatacta ttttatggga agtttagggt gagctctgag agcctcacta ttccccatga 120
   aaggatatgg gaaagaccat ttgtgatggc accattaatt gattgtattg ggtctggtgt 180
   agaaaatgac actatttgta 200
<210> 302
   <211> 200
   <212> DNA
   <213> Amaranthus rudis
<400> 302
   tacaaatagt gtcattttct acaccagacc caatacaatc aattaatggt gccatcacaa 60
   atggtctttc ccatatcctt tcatggggaa tagtgaggct ctcagagctc accctaaact 120
   tcccataaaa tagtatgtcc aagtcaattg gcctaggacc ataccttatt cccttggttc 180
   tacccatatt cttctcaatt 200
<210> 303
   <211> 200
   <212> DNA
   <213> Amaranthus rudis
<400> 303
   ggtgtagaaa atgacactat ttgtacatgg cattcgttat caaatttttc gggtggaatc 60
   tttgaagcat gggataaact cggtggaagt tccctaatcg ggaaggatgg aatgaaaagg 120
   gttttgcccg ttgggaaccg cttatgggat tggtctcata aaacctctgt aatgggagtc 180
   ttgaacttga ctcctgatag 200
<210> 304
   <211> 200
   <212> DNA
   <213> Amaranthus rudis
<400> 304
   ctatcaggag tcaagttcaa gactcccatt acagaggttt tatgagacca atcccataag 60
   cggttcccaa cgggcaaaac ccttttcatt ccatccttcc cgattaggga acttccaccg 120
   agtttatccc atgcttcaaa gattccaccc gaaaaatttg ataacgaatg ccatgtacaa 180
   atagtgtcat tttctacacc 200
<210> 305
   <211> 200
   <212> DNA
   <213> Amaranthus rudis
<400> 305
   gagtcttgaa cttgactcct gatagcttta gtgatggagg aagttttcaa tctgtcgata 60
   ctgcggttgc taaggttcgt cagatgatat cagatggggc cgacataatt gacattgggg 120
   cacagtcaac aagacccatg gcaactagga tttcggctga agaagagcta gaaagagttg 180
   tacctgtatt ggaagctgtt 200
<210> 306
   <211> 200
   <212> DNA
   <213> Amaranthus rudis
<400> 306
   aacagcttcc aatacaggta caactctttc tagctcttct tcagccgaaa tcctagttgc 60
   catgggtctt gttgactgtg ccccaatgtc aattatgtcg gccccatctg atatcatctg 120
   acgaacctta gcaaccgcag tatcgacaga ttgaaaactt cctccatcac taaagctatc 180
   aggagtcaag ttcaagactc 200
<210> 307
   <211> 200
   <212> DNA
   <213> Amaranthus viridis
<400> 307
   gcagctgata gggatcctgc taccgttgct tctataactg ctggagtttt aggtggtgca 60
   aacattgtaa gagtacataa tgtgagggat aaccttgatg ctgtcaagtt atgtgatgcc 120
   atactcggaa aaactgatta actgcttatt tgtaccacct tgtgaatgac gtctagtgga 180
   aagttcgatt gggatatgat 200
<210> 308
   <211> 200
   <212> DNA
   <213> Amaranthus viridis
<400> 308
   atcatatccc aatcgaactt tccactagac gtcattcaca aggtggtaca aataagcagt 60
   taatcagttt ttccgagtat ggcatcacat aacttgacag catcaaggtt atccctcaca 120
   ttatgtactc ttacaatgtt tgcaccacct aaaactccag cagttataga agcaacggta 180
   gcaggatccc tatcagctgc 200
<210> 309
   <211> 200
   <212> DNA
   <213> Amaranthus viridis
<400> 309
   gtggaaagtt cgattgggat atgatgaagt ccgttagttt gctcgaaagt agagctttag 60
   cccgtaaggt tatagttctt acagatgtag aatcattggc tatttgccta tttttgctta 120
   gaagatcata gcattgatcc agactttgga ttccatctag aattatgttg cgactcatgg 180
   tcgaattggc cctttgagga 200
<210> 310
   <211> 200
   <212> DNA
   <213> Amaranthus viridis
<400> 310
   tcctcaaagg gccaattcga ccatgagtcg caacataatt ctagatggaa tccaaagtct 60
   ggatcaatgc tatgatcttc taagcaaaaa taggcaaata gccaatgatt ctacatctgt 120
   aagaactata accttacggg ctaaagctct actttcgagc aaactaacgg acttcatcat 180
   atcccaatcg aactttccac 200
<210> 311
   <211> 200
   <212> DNA
   <213> Amaranthus viridis
<400> 311
   catggtcgaa ttggcccttt gaggaattgt tgaagttcct tgatctatgg ttcaatcggc 60
   ttggttcgat gaaggttgga ctgtgtcgag acaagatggg tcaaagagac caatggacaa 120
   gaagtcgacc ttgggaaagc ttgatcaagt tacttggacg agcagatagc agcaggactc 180
   tggcgagcat tagacataca 200
<210> 312
   <211> 200
   <212> DNA
   <213> Amaranthus viridis
<400> 312
   tgtatgtcta atgctcgcca gagtcctgct gctatctgct cgtccaagta acttgatcaa 60
   gctttcccaa ggtcgacttc ttgtccattg gtctctttga cccatcttgt ctcgacacag 120
   tccaaccttc atcgaaccaa gccgattgaa ccatagatca aggaacttca acaattcctc 180
   aaagggccaa ttcgaccatg 200
<210> 313
   <211> 200
   <212> DNA
   <213> Amaranthus viridis
<400> 313
   tctgtataac ctaaagattg aaacttttga agatattttt gaagaagtac tctttggctt 60
   tttgatccag aaaatcatat tggtatgaag tgttgtttaa tatacaatca atttttctga 120
   actgggttat taaagagaag cattgtagac atgaggaatg tgttcaagca tctcaaaaca 180
   accaaattaa tcccaaatgg 200
<210> 314
   <211> 200
   <212> DNA
   <213> Amaranthus viridis
<400> 314
   ccatttggga ttaatttggt tgttttgaga tgcttgaaca cattcctcat gtctacaatg 60
   cttctcttta ataacccagt tcagaaaaat tgattgtata ttaaacaaca cttcatacca 120
   atatgatttt ctggatcaaa aagccaaaga gtacttcttc aaaaatatct tcaaaagttt 180
   caatctttag gttatacaga 200
<210> 315
   <211> 200
   <212> DNA
   <213> Amaranthus viridis
<400> 315
   aaacaaccaa attaatccca aatgggatct tcaactgtaa tagagcttcg tgttttgctt 60
   tccttcactc atcaccggtg aacgatgttg aagtttgttc caagaagcaa gaagtcgtaa 120
   ttgcaatcgg gagcaatgtt ggtgacagat tagaaaattt caaccaagct ctgcaacaaa 180
   tgaagaaatt aggcatagac 200
<210> 316
   <211> 200
   <212> DNA
   <213> Amaranthus viridis
<400> 316
   gtctatgcct aatttcttca tttgttgcag agcttggttg aaattttcta atctgtcacc   60
   aacattgctc ccgattgcaa ttacgacttc ttgcttcttg gaacaaactt caacatcgtt  120
   caccggtgat gagtgaagga aagcaaaaca cgaagctcta ttacagttga agatcccatt  180
   tgggattaat ttggttgttt                                                200
<210> 317
   <211> 200
   <212> DNA
   <213> Amaranthus viridis
<400> 317
   acaaatgaag aaattaggca tagacatcac aaggcatggg tgtttatatg agacggaacc   60
   tgcatacgtg actgatcaac cgaagtttct taactctgct ttaagaggct ttacaaaact  120
   tgggcctcat gaattattag gggttttaaa gaaaattgag aaggatatgg gtagaaccaa  180
   gggaataagg tatggtccta                                                200
<210> 318
   <211> 200
   <212> DNA
   <213> Amaranthus viridis
<400> 318
   taggaccata ccttattccc ttggttctac ccatatcctt ctcaattttc tttaaaaccc   60
   ctaataattc atgaggccca agttttgtaa agcctcttaa agcagagtta agaaacttcg  120
   gttgatcagt cacgtatgca ggttccgtct catataaaca cccatgcctt gtgatgtcta  180
   tgcctaattt cttcatttgt                                                200
<210> 319
   <211> 200
   <212> DNA
   <213> Amaranthus viridis
<400> 319
   accaagggaa taaggtatgg tcctaggcca attgacttgg acatactatt ttatgggaaa 60
   tttagggtga gctctgagag cctcactatc ccccatgaaa ggatatggga aagaccattt 120
   gtgatggcac cattgattga ttgtattggg tctgatgtag aaaatgacac tatttgtaca 180
   tggcattcat tatcaaattt 200
<210> 320
   <211> 200
   <212> DNA
   <213> Amaranthus viridis
<400> 320
   aaatttgata atgaatgcca tgtacaaata gtgtcatttt ctacatcaga cccaatacaa 60
   tcaatcaatg gtgccatcac aaatggtctt tcccatatcc tttcatgggg gatagtgagg 120
   ctctcagagc tcaccctaaa tttcccataa aatagtatgt ccaagtcaat tggcctagga 180
   ccatacctta ttcccttggt 200
<210> 321
   <211> 200
   <212> DNA
   <213> Amaranthus viridis
<400> 321
   gtacatggca ttcattatca aatttttggg gtggaatctt tgaagcatgg ggtaaactcg 60
   gtggaagttc cctaatcggg aaggatggaa tgaaaagggt tttgcccgtt ggaaatcgct 120
   tatgggattg gtctcataaa acctctgtaa tgggagtctt gaacttgact cctgatagct 180
   ttagtgatgg cggaagtttt 200
<210> 322
   <211> 200
   <212> DNA
   <213> Amaranthus viridis
<400> 322
   aaaacttccg ccatcactaa agctatcagg agtcaagttc aagactccca ttacagaggt 60
   tttatgagac caatcccata agcgatttcc aacgggcaaa acccttttca ttccatcctt 120
   cccgattagg gaacttccac cgagtttacc ccatgcttca aagattccac ccaaaaaatt 180
   tgataatgaa tgccatgtac 200
<210> 323
   <211> 200
   <212> DNA
   <213> Amaranthus viridis
<400> 323
   tagctttagt gatggcggaa gttttcaatc tgtcgatact gcggttgcta aggttcgtca   60
   gatgatctca gatggggcag atataattga catcggggca caatcaacca gacccatggc  120
   aactaggatt tcggctgaag aagagctaga aagagtagta cctgtattgg aagctgtcaa  180
   ggatttgatc gaggaagaag                                              200
<210> 324
   <211> 200
   <212> DNA
   <213> Amaranthus viridis
<400> 324
   cttcttcctc gatcaaatcc ttgacagctt ccaatacagg tactactctt tctagctctt   60
   cttcagccga aatcctagtt gccatgggtc tggttgattg tgccccgatg tcaattatat  120
   ctgccccatc tgagatcatc tgacgaacct tagcaaccgc agtatcgaca gattgaaaac  180
   ttccgccatc actaaagcta                                              200
<210> 325
   <211> 200
   <212> DNA
   <213> Amaranthus viridis
<400> 325
   gtcaaggatt tgatcgagga agaaggaaga atcttgtcag tggatacgtt ttattctaaa   60
   gttgcttcgg aggccgtcaa gaagggggca cacattgtga atgatgtctc tagtgggaaa  120
   ctcgattccg agatgtttaa tgttgttgcg gaccttaaag ttccttatat agcaatgcac  180
   atgcgaggag atccgacttc                                              200
<210> 326
   <211> 200
   <212> DNA
   <213> Amaranthus viridis
<400> 326
   gaagtcggat ctcctcgcat gtgcattgct atataaggaa ctttaaggtc cgcaacaaca   60
   ttaaacatct cggaatcgag tttcccacta gagacatcat tcacaatgtg tgcccccttc  120
   ttgacggcct ccgaagcaac tttagaataa aacgtatcca ctgacaagat tcttccttct  180
   tcctcgatca aatccttgac                                              200
<210> 327
   <211> 200
   <212> DNA
   <213> Amaranthus viridis
<400> 327
   tgcacatgcg aggagatccg acttcaatgc aaaattctga gaacttgacg tacaatgatg    60
   tttgtaagca agtggctacg gagttgagct ctagggtcat agatgcagaa ttatcgggaa   120
   ttcctgcttg gaggatagtt attgatcccg gcattggatt ttctaagaat acgaatcaaa   180
   atttggaaat tcttcgtggt                                               200
<210> 328
   <211> 200
   <212> DNA
   <213> Amaranthus viridis
<400> 328
   accacgaaga atttccaaat tttgattcgt attcttagaa aatccaatgc cgggatcaat    60
   aactatcctc caagcaggaa ttcccgataa ttctgcatct atgaccctag agctcaactc   120
   cgtagccact tgcttacaaa catcattgta cgtcaagttc tcagaatttt gcattgaagt   180
   cggatctcct cgcatgtgca                                               200
<210> 329
   <211> 200
   <212> DNA
   <213> Ambrosia trifida
<400> 329
   aatgaagcct taagtcaaat gaaaaaatca ggcatagaaa taacaagaca tgcatgttta    60
   tacgaaaccg agccagctta cgtgactgat caacctcttt tcctcaattc tgccatcaga   120
   ggcgttacaa agctgggccc acatgagcta ctatcggccc tcaagaaaat tgaaaaagaa   180
   atgggccgaa ccaaaggtat                                               200
<210> 330
   <211> 200
   <212> DNA
   <213> Ambrosia trifida
<400> 330
   atacctttgg ttcggcccat ttctttttca attttcttga gggccgatag tagctcatgt    60
   gggcccagct ttgtaacgcc tctgatggca gaattgagga aaagaggttg atcagtcacg   120
   taagctggct cggtttcgta taaacatgca tgtcttgtta tttctatgcc tgattttttc   180
   atttgactta aggcttcatt                                               200
<210> 331
   <211> 200
   <212> DNA
   <213> Ambrosia trifida
<400> 331
   aagaaatggg ccgaaccaaa ggtattagat acggcccgcg acccattgac ttagatatac    60
   tgttttatgg taaacacaga gttaactcgg aaattctcac tgttccacat gaaagaatct   120
   tcgagaggcc atttgttatg gctccgttag ttgacttatt ggggtcggaa attgacaatg   180
   atacggttct atgctggcat                                               200
<210> 332
   <211> 200
   <212> DNA
   <213> Ambrosia trifida
<400> 332
   atgccagcat agaaccgtat cattgtcaat ttccgacccc aataagtcaa ctaacggagc    60
   cataacaaat ggcctctcga agattctttc atgtggaaca gtgagaattt ccgagttaac   120
   tctgtgttta ccataaaaca gtatatctaa gtcaatgggt cgcgggccgt atctaatacc   180
   tttggttcgg cccatttctt                                               200
<210> 333
   <211> 200
   <212> DNA
   <213> Ambrosia trifida
<400> 333
   caatgatacg gttctatgct ggcattcttt ctcaaaaaga ggactttttg aatcttggga    60
   aaaattaggt ggcgaatctt tgataggcaa agatgggtta agaagagttt taccagtcaa   120
   taatcggtta tgggattggt caaagaaaac ctctgtcatg ggaattttga acttgactcc   180
   cgatagtttt agcgacggag                                               200
<210> 334
   <211> 200
   <212> DNA
   <213> Ambrosia trifida
<400> 334
   ctccgtcgct aaaactatcg ggagtcaagt tcaaaattcc catgacagag gttttctttg 60
   accaatccca taaccgatta ttgactggta aaactcttct taacccatct ttgcctatca 120
   aagattcgcc acctaatttt tcccaagatt caaaaagtcc tctttttgag aaagaatgcc 180
   agcatagaac cgtatcattg 200
<210> 335
   <211> 200
   <212> DNA
   <213> Ambrosia trifida
<400> 335
   actcccgata gttttagcga cggaggaaag tttgacactg tggggtccgc tatatctcgt 60
   gttagggcca tgatatctga aggggcagac ataattgatc tgggagctca atcgacacgt 120
   ccaatggcaa ccaagatttc agtccaagaa gaactagata ggttaatccc tgttctcgaa 180
   aagattcttg aattacccga 200
<210> 336
   <211> 200
   <212> DNA
   <213> Ambrosia trifida
<400> 336
   tcgggtaatt caagaatctt ttcgagaaca gggattaacc tatctagttc ttcttggact 60
   gaaatcttgg ttgccattgg acgtgtcgat tgagctccca gatcaattat gtctgcccct 120
   tcagatatca tggccctaac acgagatata gcggacccca cagtgtcaaa ctttcctccg 180
   tcgctaaaac tatcgggagt 200
<210> 337
   <211> 200
   <212> DNA
   <213> Ambrosia trifida
<400> 337
   tcgaaaagat tcttgaatta cccgaaattg aaggaaaact gctctctatc gacacgtttt 60
   actcagaagt tgctttagaa gcgattaaga aaggggctca tataatcaat gatgtatcgg 120
   gtggaaatct agattctgat atgtttcggg tagttgctaa tcttgatgtt ccatatattg 180
   ctatgcacat gagaggggac 200
<210> 338
   <211> 200
   <212> DNA
   <213> Ambrosia trifida
<400> 338
   gtcccctctc atgtgcatag caatatatgg aacatcaaga ttagcaacta cccgaaacat 60
   atcagaatct agatttccac ccgatacatc attgattata tgagcccctt tcttaatcgc 120
   ttctaaagca acttctgagt aaaacgtgtc gatagagagc agttttcctt caatttcggg 180
   taattcaaga atcttttcga 200
<210> 339
   <211> 200
   <212> DNA
   <213> Ambrosia trifida
<400> 339
   tattgctatg cacatgagag gggacccatc cacaatgcaa aacagtgaga atttgaagta 60
   tgatgatgtt tgtaaagaag ttggtgatga gttgtatgag cgtgtaagag ctgcagagtt 120
   atgcggtgtg cctgcatgga ggatcattct tgacccaggg atcgggtttt caaagaaaac 180
   cgaagataat ttggatattt 200
<210> 340
   <211> 200
   <212> DNA
   <213> Ambrosia trifida
<400> 340
   aaatatccaa attatcttcg gttttctttg aaaacccgat ccctgggtca agaatgatcc 60
   tccatgcagg cacaccgcat aactctgcag ctcttacacg ctcatacaac tcatcaccaa 120
   cttctttaca aacatcatca tacttcaaat tctcactgtt ttgcattgtg gatgggtccc 180
   ctctcatgtg catagcaata 200
<210> 341
   <211> 200
   <212> DNA
   <213> Ambrosia trifida
<400> 341
   aaaaccgaag ataatttgga tattttgatg ggattaaaga gaattaggag tgagattgca 60
   cggaagagct taggggtgtc acgtgcacct ttgttaatcg gtccttcaag aaagagattt 120
   ttgggggaga tttgtggtcg ggcttctgct gtcgagagag atccagcgac tgttgctgct 180
   gttacatgtg cggttttggg 200
<210> 342
   <211> 200
   <212> DNA
   <213> Ambrosia trifida
<400> 342
   cccaaaaccg cacatgtaac agcagcaaca gtcgctggat ctctctcgac agcagaagcc   60
   cgaccacaaa tctcccccaa aaatctcttt cttgaaggac cgattaacaa aggtgcacgt  120
   gacaccccta agctcttccg tgcaatctca ctcctaattc tctttaatcc catcaaaata  180
   tccaaattat cttcggtttt                                              200
<210> 343
   <211> 200
   <212> DNA
   <213> Ambrosia trifida
<400> 343
   acctcatcac ctccacaaat caccaccacc catctccacc ctccaccacc acccacttca   60
   ttctccccac ccaccgtatc tcatcaccat catacctggt ggattgcagt ttgactctgt  120
   ggggtccgct atatctcgtg ttaggaccat gatatctgaa ggggcggaca taattgatct  180
   gggagctcaa tcgacacgtc                                              200
<210> 344
   <211> 200
   <212> DNA
   <213> Ambrosia trifida
<400> 344
   gacgtgtcga ttgagctccc agatcaatta tgtccgcccc ttcagatatc atggtcctaa   60
   cacgagatat agcggacccc acagagtcaa actgcaatcc accaggtatg atggtgatga  120
   gatacggtgg gtggggagaa tgaagtgggt ggtggtggag ggtggagatg ggtggtggtg  180
   atttgtggag gtgatgaggt                                              200
<210> 345
   <211> 200
   <212> DNA
   <213> Ambrosia trifida
<400> 345
   gacccatcca caatgcaaaa cagtgagaat ttgaagtata atgatgtttg taaagaagtt   60
   ggtgatgagt tgtatgagcg tgtaagagct gcagagttat gcggtgtgcc tgcatggagg  120
   atcattcttg acccagggat cgggttttca aagaaaaccg aagataattt ggatattttg  180
   atgggattaa agagaattag                                              200
<210> 346
   <211> 200
   <212> DNA
   <213> Ambrosia trifida
<400> 346
   ctaattctct ttaatcccat caaaatatcc aaattatctt cggttttctt tgaaaacccg   60
   atccctgggt caagaatgat cctccatgca ggcacaccgc ataactctgc agctcttaca  120
   cgctcataca actcatcacc aacttcttta caaacatcat tatacttcaa attctcactg  180
   ttttgcattg tggatgggtc                                               200
<210> 347
   <211> 200
   <212> DNA
   <213> Ambrosia trifida
<400> 347
   gacccatcca caatgcaaaa cagtgagaat ttgaagtata atgatgtttg taaagaagtt   60
   ggtgatgagt tgtatgagcg tgtaagagct gcagagttat gcggtgtgcc tgcatggagg  120
   atcattcttg acccagggat cgggttttca aagaaaaccg aagataattt ggatattttg  180
   atgggattaa agagaattag                                               200
<210> 348
   <211> 200
   <212> DNA
   <213> Ambrosia trifida
<400> 348
   ctaattctct ttaatcccat caaaatatcc aaattatctt cggttttctt tgaaaacccg   60
   atccctgggt caagaatgat cctccatgca ggcacaccgc ataactctgc agctcttaca  120
   cgctcataca actcatcacc aacttcttta caaacatcat tatacttcaa attctcactg  180
   ttttgcattg tggatgggtc                                               200
<210> 349
   <211> 200
   <212> DNA
   <213> Ambrosia trifida
<400> 349
   ttttgatggg attaaagaga attaggagtg agattgcccg gaagagctta ggggtgtcac   60
   gtgcaccttt gttaatcggt ccttcaagaa agagattttt gggagagatt tgtggtcggg  120
   cttctgctgg tgagagagat ccaacaactg ttgctgctgt tacatgtgcg gttttgggtg  180
   gtgctaatgt tgttcgcgtt                                               200
<210> 350
   <211> 200
   <212> DNA
   <213> Ambrosia trifida
<400> 350
   aacgcgaaca acattagcac cacccaaaac cgcacatgta acagcagcaa cagttgttgg   60
   atctctctca ccagcagaag cccgaccaca aatctctccc aaaaatctct ttcttgaagg  120
   accgattaac aaaggtgcac gtgacacccc taagctcttc cgggcaatct cactcctaat  180
   tctctttaat cccatcaaaa                                              200
<210> 351
   <211> 200
   <212> DNA
   <213> Ambrosia trifida
<400> 351
   gatttgtggt cgggcttctg ctggtgagag agatccaaca actgttgctg ctgttacatg   60
   tgcggttttg ggtggtgcta atgttgttcg cgttcataat gttggagatg atgctgatgc  120
   tgtaaagctt tgtgattcaa tgttgaaccg ggttggaaga acgtaacagc tttttgaaat  180
   ttgattgtgg gttttggtcc                                              200
<210> 352
   <211> 200
   <212> DNA
   <213> Ambrosia trifida
<400> 352
   ggaccaaaac ccacaatcaa atttcaaaaa gctgttacgt tcttccaacc cggttcaaca   60
   ttgaatcaca aagctttaca gcatcagcat catctccaac attatgaacg cgaacaacat  120
   tagcaccacc caaaaccgca catgtaacag cagcaacagt tgttggatct ctctcaccag  180
   cagaagcccg accacaaatc                                              200
<210> 353
   <211> 200
   <212> DNA
   <213> Ambrosia trifida
<400> 353
   gccttaagtc aaatgaagaa atcaggcata gagataacaa gacatgcatg tttatacaaa 60
   accgagccag cttatgtaac tgatcaacct ctttttctta attctgccat cagaggcgtt 120
   acaaagctgg gcccacatga actactatcg gccctcaaga aaatcgaaaa agaattgggc 180
   cgaaccaaag gtattagata 200
<210> 354
   <211> 200
   <212> DNA
   <213> Ambrosia trifida
<400> 354
   tatctaatac ctttggttcg gcccaattct ttttcgattt tcttgagggc cgatagtagt 60
   tcatgtgggc ccagctttgt aacgcctctg atggcagaat taagaaaaag aggttgatca 120
   gttacataag ctggctcggt tttgtataaa catgcatgtc ttgttatctc tatgcctgat 180
   ttcttcattt gacttaaggc 200
<210> 355
   <211> 200
   <212> DNA
   <213> Ambrosia trifida
<400> 355
   tgggccgaac caaaggtatt agatacggcc cgcgacccat tgacttagat atactgtttt 60
   atggtaaaca cagaattaac tcggaaattc tcactgttcc acatgaaaga atcttcgaga 120
   ggccatttgt tatggctccg ttagttgact tattggggtc ggatattgac aatgatacgg 180
   ttctatgctg gcattctttc 200
<210> 356
   <211> 200
   <212> DNA
   <213> Ambrosia trifida
<400> 356
   gaaagaatgc cagcatagaa ccgtatcatt gtcaatatcc gaccccaata agtcaactaa 60
   cggagccata acaaatggcc tctcgaagat tctttcatgt ggaacagtga gaatttccga 120
   gttaattctg tgtttaccat aaaacagtat atctaagtca atgggtcgcg ggccgtatct 180
   aatacctttg gttcggccca 200
<210> 357
   <211> 101
   <212> DNA
   <213> Ambrosia trifida
<400> 357
   ggattgcagt ttgactctgt ggggtccgct atatctcgtg ttaggaccat gatatctgaa 60
   ggggcggaca taattgatct gggagctcaa tcgacacgtc c 101
<210> 358
   <211> 101
   <212> DNA
   <213> Ambrosia trifida
<400> 358
   ggacgtgtcg attgagctcc cagatcaatt atgtccgccc cttcagatat catggtccta 60
   acacgagata tagcggaccc cacagagtca aactgcaatc c 101
<210> 359
   <211> 200
   <212> DNA
   <213> Ambrosia trifida
<400> 359
   tcgttctttc atttctcttc ggatgcttcg atacaagtcc attctcaaga acaagaagta 60
   gtaattgctt taggtagcaa tgtgggtgat aggcttaata actttaatga agccttaagt 120
   caaatgaaaa aatcaggcat agaaataaca agacatgcat gtttatacga aaccgagcca 180
   gcttacgtga ctgatcaacc 200
<210> 360
   <211> 200
   <212> DNA
   <213> Ambrosia trifida
<400> 360
   ggttgatcag tcacgtaagc tggctcggtt tcgtataaac atgcatgtct tgttatttct 60
   atgcctgatt ttttcatttg acttaaggct tcattaaagt tattaagcct atcacccaca 120
   ttgctaccta aagcaattac tacttcttgt tcttgagaat ggacttgtat cgaagcatcc 180
   gaagagaaat gaaagaacga 200
<210> 361
   <211> 200
   <212> DNA
   <213> Ambrosia trifida
<400> 361
   agccagctta cgtgactgat caacctcttt tcctcaattc tgccatcaga ggcgttacaa 60
   agctgggccc acatgagcta ctatcggccc tcaagaaaat tgaaaaagaa atgggccgaa 120
   ccaaaggtat tagatacggc ccgcgaccca ttgacttaga tatactgttt tatggtaaac 180
   acagagttaa ctcggaaatt 200
<210> 362
   <211> 200
   <212> DNA
   <213> Ambrosia trifida
<400> 362
   aatttccgag ttaactctgt gtttaccata aaacagtata tctaagtcaa tgggtcgcgg 60
   gccgtatcta atacctttgg ttcggcccat ttctttttca attttcttga gggccgatag 120
   tagctcatgt gggcccagct ttgtaacgcc tctgatggca gaattgagga aaagaggttg 180
   atcagtcacg taagctggct 200
<210> 363
   <211> 200
   <212> DNA
   <213> Ambrosia trifida
<400> 363
   taaacacaga gttaactcgg aaattctcac tgttccacat gaaagaatct tcgagaggcc 60
   atttgttatg gctccgttag ttgacttatt ggggtcggaa attgacaatg atacggttct 120
   atgctggcat tctttctcaa aaagaggact ttttgaatct tgggaaaaat taggtggcga 180
   atctttgata ggcaaagatg 200
<210> 364
   <211> 200
   <212> DNA
   <213> Ambrosia trifida
<400> 364
   catctttgcc tatcaaagat tcgccaccta atttttccca agattcaaaa agtcctcttt 60
   ttgagaaaga atgccagcat agaaccgtat cattgtcaat ttccgacccc aataagtcaa 120
   ctaacggagc cataacaaat ggcctctcga agattctttc atgtggaaca gtgagaattt 180
   ccgagttaac tctgtgttta 200
<210> 365
   <211> 200
   <212> DNA
   <213> Ambrosia trifida
<400> 365
   ggcgaatctt tgataggcaa agatgggtta agaagagttt taccagtcaa taatcggtta 60
   tgggattggt caaagaaaac ctctgtcatg ggaattttga acttgactcc cgatagtttt 120
   agcgacggag gaaagtttga cactgtgggg tccgctatat ctcgtgttag ggccatgata 180
   tctgaagggg cagacataat 200
<210> 366
   <211> 200
   <212> DNA
   <213> Ambrosia trifida
<400> 366
   attatgtctg ccccttcaga tatcatggcc ctaacacgag atatagcgga ccccacagtg 60
   tcaaactttc ctccgtcgct aaaactatcg ggagtcaagt tcaaaattcc catgacagag 120
   gttttctttg accaatccca taaccgatta ttgactggta aaactcttct taacccatct 180
   ttgcctatca aagattcgcc 200
<210> 367
   <211> 200
   <212> DNA
   <213> Ambrosia trifida
<400> 367
   tgatatctga aggggcagac ataattgatc tgggagctca atcgacacgt ccaatggcaa 60
   ccaagatttc agtccaagaa gaactagata ggttaatccc tgttctcgaa aagattcttg 120
   aattacccga aattgaagga aaactgctct ctatcgacac gttttactca gaagttgctt 180
   tagaagcgat taagaaaggg 200
<210> 368
   <211> 200
   <212> DNA
   <213> Ambrosia trifida
<400> 368
   ccctttctta atcgcttcta aagcaacttc tgagtaaaac gtgtcgatag agagcagttt 60
   tccttcaatt tcgggtaatt caagaatctt ttcgagaaca gggattaacc tatctagttc 120
   ttcttggact gaaatcttgg ttgccattgg acgtgtcgat tgagctccca gatcaattat 180
   gtctgcccct tcagatatca 200
<210> 369
   <211> 200
   <212> DNA
   <213> Ambrosia trifida
<400> 369
   tgctttagaa gcgattaaga aaggggctca tataatcaat gatgtatcgg gtggaaatct   60
   agattctgat atgtttcggg tagttgctaa tcttgatgtt ccatatattg ctatgcacat  120
   gagaggggac ccatccacaa tgcaaaacag tgagaatttg aagtatgatg atgtttgtaa  180
   agaagttggt gatgagttgt                                              200
<210> 370
   <211> 200
   <212> DNA
   <213> Ambrosia trifida
<400> 370
   acaactcatc accaacttct ttacaaacat catcatactt caaattctca ctgttttgca   60
   ttgtggatgg gtcccctctc atgtgcatag caatatatgg aacatcaaga ttagcaacta  120
   cccgaaacat atcagaatct agatttccac ccgatacatc attgattata tgagcccctt  180
   tcttaatcgc ttctaaagca                                              200
<210> 371
   <211> 200
   <212> DNA
   <213> Ambrosia trifida
<400> 371
   tgtaaagaag ttggtgatga gttgtatgag cgtgtaagag ctgcagagtt atgcggtgtg   60
   cctgcatgga ggatcattct tgacccaggg atcgggtttt caaagaaaac cgaagataat  120
   ttggatattt tgatgggatt aaagagaatt aggagtgaga ttgcacggaa gagcttaggg  180
   gtgtcacgtg cacctttgtt                                              200
<210> 372
   <211> 200
   <212> DNA
   <213> Ambrosia trifida
<400> 372
   aacaaaggtg cacgtgacac ccctaagctc ttccgtgcaa tctcactcct aattctcttt   60
   aatcccatca aaatatccaa attatcttcg gttttctttg aaaacccgat ccctgggtca  120
   agaatgatcc tccatgcagg cacaccgcat aactctgcag ctcttacacg ctcatacaac  180
   tcatcaccaa cttctttaca                                              200
<210> 373
   <211> 200
   <212> DNA
   <213> Ambrosia trifida
<400> 373
   ttcgtgtatc tcatgatctc tgtataatgt taaacttatt atagttcaat ataagtcact 60
   gtgtgatcta atttcgtata caattacttt atttgtgttg attctgatcc taaatattgc 120
   taatagtttg tgcttaacta tatatcccgt taaacatcac atttacttta tccctagaag 180
   atttgggcaa gtttttacaa 200
<210> 374
   <211> 200
   <212> DNA
   <213> Ambrosia trifida
<400> 374
   ttgtaaaaac ttgcccaaat cttctaggga taaagtaaat gtgatgttta acgggatata 60
   tagttaagca caaactatta gcaatattta ggatcagaat caacacaaat aaagtaattg 120
   tatacgaaat tagatcacac agtgacttat attgaactat aataagttta acattataca 180
   gagatcatga gatacacgaa 200
<210> 375
   <211> 200
   <212> DNA
   <213> Ambrosia trifida
<400> 375
   agaagatttg ggcaagtttt tacaaattta tggcgacgtt attgtaattt tttaaatcat 60
   tttcgtgcag catctttttc gttctttcat ttctcttcgg atgcttcgat acaagtccat 120
   tctcaagaac aagaagtagt aattgcttta ggtagcaatg tgggtgatag gcttaataac 180
   tttaatgaag ccttaagtca 200
<210> 376
   <211> 200
   <212> DNA
   <213> Ambrosia trifida
<400> 376
   tgacttaagg cttcattaaa gttattaagc ctatcaccca cattgctacc taaagcaatt 60
   actacttctt gttcttgaga atggacttgt atcgaagcat ccgaagagaa atgaaagaac 120
   gaaaaagatg ctgcacgaaa atgatttaaa aaattacaat aacgtcgcca taaatttgta 180
   aaaacttgcc caaatcttct 200
<210> 377
   <211> 200
   <212> DNA
   <213> Conyza canadensis
<400> 377
   gaaataacaa ggcatgcttg tctatacgaa actgaaccag cttatgtgac ggaccagcct   60
   ctttttctca actctgccgt tagagccact acaaagcttg gccctcatga gctactgtcc  120
   attctcaaga aaattgaaaa ggaaatgggt cgaaccaaag ggcttaggta cggcccacga  180
   cccattgacc tagatatatt                                              200
<210> 378
   <211> 200
   <212> DNA
   <213> Conyza canadensis
<400> 378
   aatatatcta ggtcaatggg tcgtgggccg tacctaagcc ctttggttcg acccatttcc   60
   ttttcaattt tcttgagaat ggacagtagc tcatgagggc caagctttgt agtggctcta  120
   acggcagagt tgagaaaaag aggctggtcc gtcacataag ctggttcagt ttcgtataga  180
   caagcatgcc ttgttatttc                                              200
<210> 379
   <211> 200
   <212> DNA
   <213> Conyza canadensis
<400> 379
   cacgacccat tgacctagat atattgttct atggtaaatg caaagttaac tctgatattc   60
   taactgttcc tcatgaaaga atctttgaga ggccattcgt tatggctcca ttagttgact  120
   tgttaggatc agatgtagac aatgatacgg ttctatgctg gcattctttt tcaaaaaatg  180
   ggctttttgg atcttgggaa                                              200
<210> 380
   <211> 200
   <212> DNA
   <213> Conyza canadensis
<400> 380
   ttcccaagat ccaaaaagcc cattttttga aaaagaatgc cagcatagaa ccgtatcatt 60
   gtctacatct gatcctaaca agtcaactaa tggagccata acgaatggcc tctcaaagat 120
   tctttcatga ggaacagtta gaatatcaga gttaactttg catttaccat agaacaatat 180
   atctaggtca atgggtcgtg 200
<210> 381
   <211> 200
   <212> DNA
   <213> Conyza canadensis
<400> 381
   aaatgggctt tttggatctt gggaaacatt aggtggtgaa tcttccatag gaaaagatgg 60
   tttaagaagg gttttacctg tcaatgatcg tttatgggat tggtcaaaga aaacttccgt 120
   catgggtatt ttgaatataa ctcctgatag ttttagtgat ggagggaagt ttgattccat 180
   ggggtccgct ttatctcgtg 200
<210> 382
   <211> 200
   <212> DNA
   <213> Conyza canadensis
<400> 382
   cacgagataa agcggacccc atggaatcaa acttccctcc atcactaaaa ctatcaggag 60
   ttatattcaa aatacccatg acggaagttt tctttgacca atcccataaa cgatcattga 120
   caggtaaaac ccttcttaaa ccatcttttc ctatggaaga ttcaccacct aatgtttccc 180
   aagatccaaa aagcccattt 200
<210> 383
   <211> 200
   <212> DNA
   <213> Conyza canadensis
<400> 383
   tccatggggt ccgctttatc tcgtgttcag accatgatat ctgaaggggt tgacataatt 60
   gatctaggag ctcaatccac acgcccaatg gcgaccaaga tctcagtcga agaggaacta 120
   gataggctaa tacccgttct tgaaaagatt cttgaattac ctgaaattga aggaaagttg 180
   ttgtctgtgg acacatttta 200
<210> 384
   <211> 200
   <212> DNA
   <213> Conyza canadensis
<400> 384
   taaaatgtgt ccacagacaa caactttcct tcaatttcag gtaattcaag aatcttttca   60
   agaacgggta ttagcctatc tagttcctct tcgactgaga tcttggtcgc cattgggcgt  120
   gtggattgag ctcctagatc aattatgtca accccttcag atatcatggt ctgaacacga  180
   gataaagcgg accccatgga                                              200
<210> 385
   <211> 200
   <212> DNA
   <213> Conyza canadensis
<400> 385
   agttgttgtc tgtggacaca ttttactcgg aagttgcttc agaggcaatc aagaaagggg   60
   ctcatatggt caatgatgta tcgggtggaa tgttagattc tgatatgctt catgttgtgg  120
   ctgatctaaa tgttccatat atcactatgc acatgagagg ggacccatcc acaatgcaaa  180
   acagtgagaa tttgaagtat                                              200
<210> 386
   <211> 200
   <212> DNA
   <213> Conyza canadensis
<400> 386
   atacttcaaa ttctcactgt tttgcattgt ggatgggtcc cctctcatgt gcatagtgat   60
   atatggaaca tttagatcag ccacaacatg aagcatatca gaatctaaca ttccacccga  120
   tacatcattg accatatgag cccctttctt gattgcctct gaagcaactt ccgagtaaaa   180
   tgtgtccaca gacaacaact                                              200
<210> 387
   <211> 200
   <212> DNA
   <213> Conyza canadensis
<400> 387
   gcaaaacagt gagaatttga agtatgatga tgttgtcaaa gaagttgggg aggaattgta   60
   tgaacgtgta aggaatgcag agttatgtgg tgttcccgca tggaggatgg ttcttgatcc  120
   agggatcggg ttttcaaaga aaaccgaaga taatttggag atattgatgg gactaaagag  180
   gtttagaagt gagattggac                                              200
<210> 388
   <211> 200
   <212> DNA
   <213> Conyza canadensis
<400> 388
   gtccaatctc acttctaaac ctctttagtc ccatcaatat ctccaaatta tcttcggttt   60
   tctttgaaaa cccgatccct ggatcaagaa ccatcctcca tgcgggaaca ccacataact  120
   ctgcattcct tacacgttca tacaattcct ccccaacttc tttgacaaca tcatcatact  180
   tcaaattctc actgttttgc                                              200
<210> 389
   <211> 200
   <212> DNA
   <213> Conyza canadensis
<400> 389
   aagaggttta gaagtgagat tggacaaaag agcttagggg tgtctcgtgc acctttgtta   60
   atcggacctt caagaaaaag gtttttgggt gagatttgtg gtcggccttc tgctgttgag  120
   agagatccag ggactgtagc tgctgttacc agtgcgattt tgggtggtgc caatatcgtt  180
   agggttcata acattggaca                                              200
<210> 390
   <211> 200
   <212> DNA
   <213> Conyza canadensis
<400> 390
   tgtccaatgt tatgaaccct aacgatattg gcaccaccca aaatcgcact ggtaacagca   60
   gctacagtcc ctggatctct ctcaacagca gaaggccgac cacaaatctc acccaaaaac  120
   ctttttcttg aaggtccgat taacaaaggt gcacgagaca cccctaagct cttttgtcca  180
   atctcacttc taaacctctt                                              200
<210> 391
   <211> 200
   <212> DNA
   <213> Conyza canadensis
<400> 391
   tcgtcctcgg atacttcaat acaagtgcat tctccagaac aagaagtagt aattgcttta   60
   ggtagcaatg tgggtaatag acttaataac tttaatgaag ccttatccca gatgaagaaa  120
   tcggggatag aaataacaag gcatgcttgt ctatacgaaa ctgaaccagc ttatgtgacg  180
   gaccagcctc tttttctcaa                                              200
<210> 392
   <211> 200
   <212> DNA
   <213> Conyza canadensis
<400> 392
   ttgagaaaaa gaggctggtc cgtcacataa gctggttcag tttcgtatag acaagcatgc   60
   cttgttattt ctatccccga tttcttcatc tgggataagg cttcattaaa gttattaagt  120
   ctattaccca cattgctacc taaagcaatt actacttctt gttctggaga atgcacttgt  180
   attgaagtat ccgaggacga                                              200
<210> 393
   <211> 200
   <212> DNA
   <213> Conyza canadensis
<400> 393
   tgacggacca gcctcttttt ctcaactctg ccgttagagc cactacaaag cttggccctc   60
   atgagctact gtccattctc aagaaaattg aaaaggaaat gggtcgaacc aaagggctta  120
   ggtacggccc acgacccatt gacctagata tattgttcta tggtaaatgc aaagttaact  180
   ctgatattct aactgttcct                                              200
<210> 394
   <211> 200
   <212> DNA
   <213> Conyza canadensis
<400> 394
   aggaacagtt agaatatcag agttaacttt gcatttacca tagaacaata tatctaggtc   60
   aatgggtcgt gggccgtacc taagcccttt ggttcgaccc atttcctttt caattttctt  120
   gagaatggac agtagctcat gagggccaag ctttgtagtg gctctaacgg cagagttgag  180
   aaaaagaggc tggtccgtca                                              200
<210> 395
   <211> 200
   <212> DNA
   <213> Conyza canadensis
<400> 395
   taactctgat attctaactg ttcctcatga aagaatcttt gagaggccat tcgttatggc   60
   tccattagtt gacttgttag gatcagatgt agacaatgat acggttctat gctggcattc  120
   tttttcaaaa aatgggcttt ttggatcttg ggaaacatta ggtggtgaat cttccatagg  180
   aaaagatggt ttaagaaggg                                              200
<210> 396
   <211> 200
   <212> DNA
   <213> Conyza canadensis
<400> 396
   cccttcttaa accatctttt cctatggaag attcaccacc taatgtttcc caagatccaa    60
   aaagcccatt ttttgaaaaa gaatgccagc atagaaccgt atcattgtct acatctgatc   120
   ctaacaagtc aactaatgga gccataacga atggcctctc aaagattctt tcatgaggaa   180
   cagttagaat atcagagtta                                               200
<210> 397
   <211> 200
   <212> DNA
   <213> Conyza canadensis
<400> 397
   ataggaaaag atggtttaag aagggtttta cctgtcaatg atcgtttatg ggattggtca    60
   aagaaaactt ccgtcatggg tattttgaat ataactcctg atagttttag tgatggaggg   120
   aagtttgatt ccatggggtc cgctttatct cgtgttcaga ccatgatatc tgaaggggtt   180
   gacataattg atctaggagc                                               200
<210> 398
   <211> 200
   <212> DNA
   <213> Conyza canadensis
<400> 398
   gctcctagat caattatgtc aaccccttca gatatcatgg tctgaacacg agataaagcg    60
   gaccccatgg aatcaaactt ccctccatca ctaaaactat caggagttat attcaaaata   120
   cccatgacgg aagttttctt tgaccaatcc cataaacgat cattgacagg taaaaccctt   180
   cttaaaccat cttttcctat                                               200
<210> 399
   <211> 200
   <212> DNA
   <213> Conyza canadensis
<400> 399
   gggttgacat aattgatcta ggagctcaat ccacacgccc aatggcgacc aagatctcag 60
   tcgaagagga actagatagg ctaatacccg ttcttgaaaa gattcttgaa ttacctgaaa 120
   ttgaaggaaa gttgttgtct gtggacacat tttactcgga agttgcttca gaggcaatca 180
   agaaaggggc tcatatggtc 200
<210> 400
   <211> 200
   <212> DNA
   <213> Conyza canadensis
<400> 400
   gaccatatga gcccctttct tgattgcctc tgaagcaact tccgagtaaa atgtgtccac 60
   agacaacaac tttccttcaa tttcaggtaa ttcaagaatc ttttcaagaa cgggtattag 120
   cctatctagt tcctcttcga ctgagatctt ggtcgccatt gggcgtgtgg attgagctcc 180
   tagatcaatt atgtcaaccc 200
<210> 401
   <211> 200
   <212> DNA
   <213> Conyza canadensis
<400> 401
   aatcaagaaa ggggctcata tggtcaatga tgtatcgggt ggaatgttag attctgatat 60
   gcttcatgtt gtggctgatc taaatgttcc atatatcact atgcacatga gaggggaccc 120
   atccacaatg caaaacagtg agaatttgaa gtatgatgat gttgtcaaag aagttgggga 180
   ggaattgtat gaacgtgtaa 200
<210> 402
   <211> 200
   <212> DNA
   <213> Conyza canadensis
<400> 402
   ttacacgttc atacaattcc tccccaactt ctttgacaac atcatcatac ttcaaattct 60
   cactgttttg cattgtggat gggtcccctc tcatgtgcat agtgatatat ggaacattta 120
   gatcagccac aacatgaagc atatcagaat ctaacattcc acccgataca tcattgacca 180
   tatgagcccc tttcttgatt 200
<210> 403
   <211> 200
   <212> DNA
   <213> Conyza canadensis
<400> 403
   ggggaggaat tgtatgaacg tgtaaggaat gcagagttat gtggtgttcc cgcatggagg 60
   atggttcttg atccagggat cgggttttca aagaaaaccg aagataattt ggagatattg 120
   atgggactaa agaggtttag aagtgagatt ggacaaaaga gcttaggggt gtctcgtgca 180
   cctttgttaa tcggaccttc 200
<210> 404
   <211> 200
   <212> DNA
   <213> Conyza canadensis
<400> 404
   gaaggtccga ttaacaaagg tgcacgagac acccctaagc tcttttgtcc aatctcactt 60
   ctaaacctct ttagtcccat caatatctcc aaattatctt cggttttctt tgaaaacccg 120
   atccctggat caagaaccat cctccatgcg ggaacaccac ataactctgc attccttaca 180
   cgttcataca attcctcccc 200
<210> 405
   <211> 200
   <212> DNA
   <213> Conyza canadensis
<400> 405
   tttcttcttc tagttggagc tcttttacgt ggtgtcttct tttccgagtc atcttcttcc 60
   ccatcagatg gattttctgc agcctttttt ctacgaactg ctgtaatagc aaatgtacta 120
   cgcaatctaa catctgggaa ttgtacaaag tttattgaag atagtgaagg cagattcgac 180
   tgccacctac acatataaga 200
<210> 406
   <211> 200
   <212> DNA
   <213> Conyza canadensis
<400> 406
   tcttatatgt gtaggtggca gtcgaatctg ccttcactat cttcaataaa ctttgtacaa 60
   ttcccagatg ttagattgcg tagtacattt gctattacag cagttcgtag aaaaaaggct 120
   gcagaaaatc catctgatgg ggaagaagat gactcggaaa agaagacacc acgtaaaaga 180
   gctccaacta gaagaagaaa 200
<210> 407
   <211> 200
   <212> DNA
   <213> Conyza canadensis
<400> 407
   tcgactgcca cctacacata taagaaacca ttaacttcta tacttaactc acaatataaa 60
   acatcatttg catgttcgtc aaaaacaata ccatttctta tctttgctca atggatatga 120
   aatctgccaa tacaagatta ttcatttgac ctcaatctag catctgatgt ctattcaagg 180
   tatcgtttat aatgaagatt 200
<210> 408
   <211> 200
   <212> DNA
   <213> Conyza canadensis
<400> 408
   aatcttcatt ataaacgata ccttgaatag acatcagatg ctagattgag gtcaaatgaa 60
   taatcttgta ttggcagatt tcatatccat tgagcaaaga taagaaatgg tattgttttt 120
   gacgaacatg caaatgatgt tttatattgt gagttaagta tagaagttaa tggtttctta 180
   tatgtgtagg tggcagtcga 200
<210> 409
   <211> 200
   <212> DNA
   <213> Conyza canadensis
<400> 409
   caaggtatcg tttataatga agattattgt acttcaaaat actttcttgt gtagtggtat 60
   caaattttga gttaaagtta ctccatttga cctcaaaagc caaataatga gcaacaaaca 120
   acacaatttc acctccctta taccctgcga ctatatttta tcttcaaatc aatccatttc 180
   catgtttatc ctctccatac 200
<210> 410
   <211> 200
   <212> DNA
   <213> Conyza canadensis
<400> 410
   gtatggagag gataaacatg gaaatggatt gatttgaaga taaaatatag tcgcagggta 60
   taagggaggt gaaattgtgt tgtttgttgc tcattatttg gcttttgagg tcaaatggag 120
   taactttaac tcaaaatttg ataccactac acaagaaagt attttgaagt acaataatct 180
   tcattataaa cgataccttg 200
<210> 411
   <211> 200
   <212> DNA
   <213> Conyza canadensis
<400> 411
   atttccatgt ttatcctctc catacagcta cataataagt tttagggaaa aaacaaaact 60
   aaactaaact aagtttacag aattcccatt tcttatccta aaatttacaa gcaattcaac 120
   atatattctt tatttacaac tataaatagc atatgggtat gttggtatat agatatagat 180
   atagatatac atatgtagat 200
<210> 412
   <211> 200
   <212> DNA
   <213> Conyza canadensis
<400> 412
   atctacatat gtatatctat atctatatct atataccaac atacccatat gctatttata 60
   gttgtaaata aagaatatat gttgaattgc ttgtaaattt taggataaga aatgggaatt 120
   ctgtaaactt agtttagttt agttttgttt tttccctaaa acttattatg tagctgtatg 180
   gagaggataa acatggaaat 200
<210> 413
   <211> 200
   <212> DNA
   <213> Conyza canadensis
<400> 413
   tagatataga tatacatatg tagatgtgta ttatatgtgt gtaaatgggg agaaaaaagg 60
   aaacatatat tacctgtgta aagaacaaaa ttgagtaaaa gaaggcaaag aagccatttc 120
   taaccactaa aatcttgggc tttcaagaat cccacttcaa agctaccatt tcacatacca 180
   aacccccctt tctatgggtt 200
<210> 414
   <211> 200
   <212> DNA
   <213> Conyza canadensis
<400> 414
   aacccataga aaggggggtt tggtatgtga aatggtagct ttgaagtggg attcttgaaa 60
   gcccaagatt ttagtggtta gaaatggctt ctttgccttc ttttactcaa ttttgttctt 120
   tacacaggta atatatgttt ccttttttct ccccatttac acacatataa tacacatcta 180
   catatgtata tctatatcta 200
<210> 415
   <211> 200
   <212> DNA
   <213> Conyza canadensis
<400> 415
   taccaaaccc ccctttctat gggtttaaag atgagggctt tacatttatc tcttttacaa 60
   tccaaatttt ccaaaaccga aataatgtac gacattcggt gcacaaggta caaacagaaa 120
   aaactacttt tgtattttag atctcaagaa aggagtggcc tttttggtaa taacatactt 180
   tattcaattg ccacttacct 200
<210> 416
   <211> 200
   <212> DNA
   <213> Conyza canadensis
<400> 416
   aggtaagtgg caattgaata aagtatgtta ttaccaaaaa ggccactcct ttcttgagat 60
   ctaaaataca aaagtagttt tttctgtttg taccttgtgc accgaatgtc gtacattatt 120
   tcggttttgg aaaatttgga ttgtaaaaga gataaatgta aagccctcat ctttaaaccc 180
   atagaaaggg gggtttggta 200
<210> 417
   <211> 200
   <212> DNA
   <213> Conyza canadensis
<400> 417
   tactttattc aattgccact taccttcaag aaattaacaa aattacccac aattaaaaaa 60
   gggtttccca gtttcactca cacttattca agattgatgc aaaataaacc cataaataag 120
   aaggttaaaa tagtgttaaa gccttaaagg tgcttgcttt ttgtgttttt tgtgatgcct 180
   ataaaaagat tcttattgtt 200
<210> 418
   <211> 200
   <212> DNA
   <213> Conyza canadensis
<400> 418
   aacaataaga atctttttat aggcatcaca aaaaacacaa aaagcaagca cctttaaggc   60
   tttaacacta ttttaacctt cttatttatg ggtttatttt gcatcaatct tgaataagtg  120
   tgagtgaaac tgggaaaccc ttttttaatt gtgggtaatt ttgttaattt cttgaaggta  180
   agtggcaatt gaataaagta                                              200
<210> 419
   <211> 200
   <212> DNA
   <213> Conyza canadensis
<400> 419
   tgcctataaa aagattctta ttgttttcaa atttttttat gtcaagtttt cgtttttaac   60
   agtttatata gttatataat ttgcagaaag ccataaagct ctgaacttga atgttttata  120
   gcagctcttt gcagtaatat cttgtagagg taaacctgtt aacccatatt tattaatttg  180
   tacataaaat tgttgaattg                                              200
<210> 420
   <211> 200
   <212> DNA
   <213> Conyza canadensis
<400> 420
   caattcaaca attttatgta caaattaata aatatgggtt aacaggttta cctctacaag   60
   atattactgc aaagagctgc tataaaacat tcaagttcag agctttatgg ctttctgcaa  120
   attatataac tatataaact gttaaaaacg aaaacttgac ataaaaaaat ttgaaaacaa  180
   taagaatctt tttataggca                                              200
<210> 421
   <211> 200
   <212> DNA
   <213> Conyza canadensis
<400> 421
   atttgtacat aaaattgttg aattgaatag agctaccact gggacttgtg tagtcaaact 60
   gtagagcatc cctgtaatgc tggaagcttt atgacctaaa aggatttgta gacttgttat 120
   ctgtggattg gtataaagat tttcaagttg ttattttgaa actttgaata gtatattatg 180
   ctcattactg aatgatacaa 200
<210> 422
   <211> 200
   <212> DNA
   <213> Conyza canadensis
<400> 422
   ttgtatcatt cagtaatgag cataatatac tattcaaagt ttcaaaataa caacttgaaa 60
   atctttatac caatccacag ataacaagtc tacaaatcct tttaggtcat aaagcttcca 120
   gcattacagg gatgctctac agtttgacta cacaagtccc agtggtagct ctattcaatt 180
   caacaatttt atgtacaaat 200
<210> 423
   <211> 200
   <212> DNA
   <213> Conyza canadensis
<400> 423
   ttatgctcat tactgaatga tacaataggg agtcgcgaat gttagaattg ataagaatta 60
   ggatgaagtt tgtgtttgaa cgttccttct gtttataatc ttcatgtcac ttccatagta 120
   ccataaccct agggcaacac gagcctttag attatgaatg ggcacaccca attgtttaga 180
   ccttgtataa atgaagtgaa 200
<210> 424
   <211> 200
   <212> DNA
   <213> Conyza canadensis
<400> 424
   ttcacttcat ttatacaagg tctaaacaat tgggtgtgcc cattcataat ctaaaggctc 60
   gtgttgccct agggttatgg tactatggaa gtgacatgaa gattataaac agaaggaacg 120
   ttcaaacaca aacttcatcc taattcttat caattctaac attcgcgact ccctattgta 180
   tcattcagta atgagcataa 200
<210> 425
   <211> 200
   <212> DNA
   <213> Conyza canadensis
<400> 425
   ttagaccttg tataaatgaa gtgaatatca ttgttataag cattctggtg gtggttttgc 60
   agatttacct ttctctatct aatttgaagg tttgttgatt aaggtgatac aaatgagtgt 120
   tttcaagcag ctaatagctt caagattagt acgcactcat gtctgtggat cttatagagg 180
   tacacttcct cgaacattca 200
<210> 426
   <211> 200
   <212> DNA
   <213> Conyza canadensis
<400> 426
   tgaatgttcg aggaagtgta cctctataag atccacagac atgagtgcgt actaatcttg 60
   aagctattag ctgcttgaaa acactcattt gtatcacctt aatcaacaaa ccttcaaatt 120
   agatagagaa aggtaaatct gcaaaaccac caccagaatg cttataacaa tgatattcac 180
   ttcatttata caaggtctaa 200
<210> 427
   <211> 200
   <212> DNA
   <213> Conyza canadensis
<400> 427
   agaggtacac ttcctcgaac attcatatag cgatgttatt gttaagtttt gaatctgtag 60
   caacattata tataattttt tttgcattgt tttcctgcag catccttttc actctttcat 120
   tcgtcctcgg atacttcaat acaagtgcat tctccagaac aagaagtagt aattgcttta 180
   ggtagcaatg tgggtaatag 200
<210> 428
   <211> 200
   <212> DNA
   <213> Conyza canadensis
<400> 428
   ctattaccca cattgctacc taaagcaatt actacttctt gttctggaga atgcacttgt 60
   attgaagtat ccgaggacga atgaaagagt gaaaaggatg ctgcaggaaa acaatgcaaa 120
   aaaaattata tataatgttg ctacagattc aaaacttaac aataacatcg ctatatgaat 180
   gttcgaggaa gtgtacctct 200
<210> 429
   <211> 200
   <212> DNA
   <213> Conyza canadensis
<400> 429
   ctttaggtag caatgtgggt aatagactta ataactttaa tgaagcctta tcccagatga 60
   agaaatcggg gatagaaata acaaggcatg cttgtctata cgaaactgaa ccagcttatg 120
   tgacggacca gcctcttttt ctcaactctg ccgttagagc cactacaaag cttggccctc 180
   atgagctact gtccattctc 200
<210> 430
   <211> 200
   <212> DNA
   <213> Conyza canadensis
<400> 430
   gagaatggac agtagctcat gagggccaag ctttgtagtg gctctaacgg cagagttgag 60
   aaaaagaggc tggtccgtca cataagctgg ttcagtttcg tatagacaag catgccttgt 120
   tatttctatc cccgatttct tcatctggga taaggcttca ttaaagttat taagtctatt 180
   acccacattg ctacctaaag 200
<210> 431
   <211> 200
   <212> DNA
   <213> Conyza canadensis
<400> 431
   ccctcatgag ctactgtcca ttctcaagaa aattgaaaag gaaatgggtc gaaccaaagg 60
   gcttaggtac ggcccacgac ccattgacct agatatattg ttctatggta aatgcaaagt 120
   taactctgat attctaactg ttcctcatga aagaatcttt gagaggccat tcgttatggc 180
   tccattagtt gacttgttag 200
<210> 432
   <211> 200
   <212> DNA
   <213> Conyza canadensis
<400> 432
   ctaacaagtc aactaatgga gccataacga atggcctctc aaagattctt tcatgaggaa 60
   cagttagaat atcagagtta actttgcatt taccatagaa caatatatct aggtcaatgg 120
   gtcgtgggcc gtacctaagc cctttggttc gacccatttc cttttcaatt ttcttgagaa 180
   tggacagtag ctcatgaggg 200
<210> 433
   <211> 200
   <212> DNA
   <213> Conyza canadensis
<400> 433
   atggctccat tagttgactt gttaggatca gatgtagaca atgatacggt tctatgctgg    60
   cattcttttt caaaaaatgg gctttttgga tcttgggaaa cattaggtgg tgaatcttcc   120
   ataggaaaag atggtttaag aagggtttta cctgtcaatg atcgtttatg ggattggtca   180
   aagaaaactt ccgtcatggg                                               200
<210> 434
   <211> 200
   <212> DNA
   <213> Conyza canadensis
<400> 434
   cccatgacgg aagttttctt tgaccaatcc cataaacgat cattgacagg taaaaccctt    60
   cttaaaccat cttttcctat ggaagattca ccacctaatg tttcccaaga tccaaaaagc   120
   ccattttttg aaaaagaatg ccagcataga accgtatcat tgtctacatc tgatcctaac   180
   aagtcaacta atggagccat                                               200
<210> 435
   <211> 200
   <212> DNA
   <213> Conyza canadensis
<400> 435
   ggtcaaagaa aacttccgtc atgggtattt tgaatataac tcctgatagt tttagtgatg    60
   gagggaagtt tgattccatg ggtccgcttt tatctcgtgt tcagaccatg atatctgaag   120
   gggttgacat aattgatcta ggagctcaat ccacacgccc aatggcgacc aagatctcag   180
   tcgaagagga actagatagg                                               200
<210> 436
   <211> 200
   <212> DNA
   <213> Conyza canadensis
<400> 436
   cctatctagt tcctcttcga ctgagatctt ggtcgccatt gggcgtgtgg attgagctcc    60
   tagatcaatt atgtcaaccc cttcagatat catggtctga acacgagata aagcggaccc   120
   catggaatca aacttccctc catcactaaa actatcagga gttatattca aaatacccat   180
   gacggaagtt ttctttgacc                                               200
<210> 437
   <211> 200
   <212> DNA
   <213> Conyza canadensis
<400> 437
   ctcagtcgaa gaggaactag ataggctaat acccgttctt gaaaagattc ttgaattacc 60
   tgaaattgaa ggaaagttgt tgtctgtgga cacattttac tcggaagttg cttcagaggc 120
   aatcaagaaa ggggctcata tggtcaatga tgtatcgggt ggaatgttag attctgatat 180
   gcttcatgtt gtggctgatc 200
<210> 438
   <211> 200
   <212> DNA
   <213> Conyza canadensis
<400> 438
   gatcagccac aacatgaagc atatcagaat ctaacattcc acccgataca tcattgacca 60
   tatgagcccc tttcttgatt gcctctgaag caacttccga gtaaaatgtg tccacagaca 120
   acaactttcc ttcaatttca ggtaattcaa gaatcttttc aagaacgggt attagcctat 180
   ctagttcctc ttcgactgag 200
<210> 439
   <211> 200
   <212> DNA
   <213> Conyza canadensis
<400> 439
   gatatgcttc atgttgtggc tgatctaaat gttccatata tcactatgca catgagaggg 60
   gacccatcca caatgcaaaa cagtgagaat ttgaagtatg atgatgttgt caaagaagtt 120
   ggggaggaat tgtatgaacg tgtaaggaat gcagagttat gtggtgttcc cgcatggagg 180
   atggttcttg atccagggat 200
<210> 440
   <211> 200
   <212> DNA
   <213> Conyza canadensis
<400> 440
   atccctggat caagaaccat cctccatgcg ggaacaccac ataactctgc attccttaca 60
   cgttcataca attcctcccc aacttctttg acaacatcat catacttcaa attctcactg 120
   ttttgcattg tggatgggtc ccctctcatg tgcatagtga tatatggaac atttagatca 180
   gccacaacat gaagcatatc 200
<210> 441
   <211> 200
   <212> DNA
   <213> Conyza canadensis
<400> 441
   ggaggatggt tcttgatcca gggatcgggt tttcaaagaa aaccgaagat aatttggaga 60
   tattgatggg actaaagagg tttagaagtg agattggaca aaagagctta ggggtgtctc 120
   gtgcaccttt gttaatcgga ccttcaagaa aaaggttttt gggtgagatt tgtggtcggc 180
   cttctgctgt tgagagagat 200
<210> 442
   <211> 200
   <212> DNA
   <213> Conyza canadensis
<400> 442
   atctctctca acagcagaag gccgaccaca aatctcaccc aaaaaccttt ttcttgaagg 60
   tccgattaac aaaggtgcac gagacacccc taagctcttt tgtccaatct cacttctaaa 120
   cctctttagt cccatcaata tctccaaatt atcttcggtt ttctttgaaa acccgatccc 180
   tggatcaaga accatcctcc 200
<210> 443
   <211> 200
   <212> DNA
   <213> Conyza canadensis
<400> 443
   tcggccttct gctgttgaga gagatccagg gactgtagct gctgttacca gtgcgatttt 60
   gggtggtgcc aatatcgtta gggttcataa cattggacat aatgtggatg ctgttaagct 120
   ttgtgattca atgttggatc gagctggtag atcttagtag cttaaagtta tttgacatct 180
   tgttttttat tcaattttct 200
<210> 444
   <211> 200
   <212> DNA
   <213> Conyza canadensis
<400> 444
   agaaaattga ataaaaaaca agatgtcaaa taactttaag ctactaagat ctaccagctc 60
   gatccaacat tgaatcacaa agcttaacag catccacatt atgtccaatg ttatgaaccc 120
   taacgatatt ggcaccaccc aaaatcgcac tggtaacagc agctacagtc cctggatctc 180
   tctcaacagc agaaggccga 200
<210> 445
   <211> 200
   <212> DNA
   <213> Conyza canadensis
<400> 445
   catcttgttt tttattcaat tttctggaca ttactttttg tatttctgtc tggcaaatga 60
   ttttacaggg atatcctaga ggctaaattg tatattacat tcttgatttt ctcacaaagt 120
   tttacttttt cagtttttct tgttcagttt tgtttttgaa aatttgctag atcagattaa 180
   atcgttgctt tatgatcttc 200
<210> 446
   <211> 200
   <212> DNA
   <213> Conyza canadensis
<400> 446
   gaagatcata aagcaacgat ttaatctgat ctagcaaatt ttcaaaaaca aaactgaaca 60
   agaaaaactg aaaaagtaaa actttgtgag aaaatcaaga atgtaatata caatttagcc 120
   tctaggatat ccctgtaaaa tcatttgcca gacagaaata caaaaagtaa tgtccagaaa 180
   attgaataaa aaacaagatg 200
<210> 447
   <211> 200
   <212> DNA
   <213> Conyza canadensis
<400> 447
   attaaatcgt tgctttatga tcttcgtgtc ttttttggga cgctgcgagt ttcatttagt 60
   ggtattgtaa actcctttta aagtttgtac acacttggta tttatgcttc tagttttcgt 120
   ctatgtctgt cctatattgt gtatagtatg tttaaagttc ataaaaagag gataaacgaa 180
   tagcacgtaa atcttataat 200
<210> 448
   <211> 200
   <212> DNA
   <213> Conyza canadensis
<400> 448
   attataagat ttacgtgcta ttcgtttatc ctctttttat gaactttaaa catactatac 60
   acaatatagg acagacatag acgaaaacta gaagcataaa taccaagtgt gtacaaactt 120
   taaaaggagt ttacaatacc actaaatgaa actcgcagcg tcccaaaaaa gacacgaaga 180
   tcataaagca acgatttaat 200
<210> 449
   <211> 200
   <212> DNA
   <213> Conyza canadensis
<400> 449
   acgaatagca cgtaaatctt ataattcgat tccacagagt gcaagttatg ctttcctaaa 60
   tgaaagcaag agtctagaca gtttggtcaa cgttaacatg agactattgt agaatacaac 120
   accacaagaa accaaaactg tcttgtgcat ttctctcttt tccaggttta aaactcaata 180
   ccaagacttg atatcaacta 200
<210> 450
   <211> 200
   <212> DNA
   <213> Conyza canadensis
<400> 450
   tagttgatat caagtcttgg tattgagttt taaacctgga aaagagagaa atgcacaaga 60
   cagttttggt ttcttgtggt gttgtattct acaatagtct catgttaacg ttgaccaaac 120
   tgtctagact cttgctttca tttaggaaag cataacttgc actctgtgga atcgaattat 180
   aagatttacg tgctattcgt 200
<210> 451
   <211> 200
   <212> DNA
   <213> Conyza canadensis
<400> 451
   caataccaag acttgatatc aactattatt ctgcttcaat gattcagctt cagctatagc 60
   cataggatca tctttggtgt caggttcatc cttctcacca tctcgttcct ctgccagttt 120
   ttttctgtgc aattcctctg caacttgctt tgatgcttgg tcttcttttt gaaggcgttg 180
   catctcttct tcttgttgct 200
<210> 452
   <211> 200
   <212> DNA
   <213> Conyza canadensis
<400> 452
   agcaacaaga agaagagatg caacgccttc aaaaagaaga ccaagcatca aagcaagttg   60
   cagaggaatt gcacagaaaa aaactggcag aggaacgaga tggtgagaag gatgaacctg  120
   acaccaaaga tgatcctatg gctatagctg aagctgaatc attgaagcag aataatagtt  180
   gatatcaagt cttggtattg                                              200
<210> 453
   <211> 200
   <212> DNA
   <213> Conyza canadensis
<400> 453
   cgttgcatct cttcttcttg ttgctgtcgt tcaaaccatg tatccgcatc ggcccaaact   60
   gtaatttaga gccaaaacaa catgattaga atgcagattc cagaagccaa attcaaactg  120
   taagaaaaaa aatgtagtga tgatttaact caaaacaact cacacagctc caaaatggat  180
   gggggttcaa attagaggat                                              200
<210> 454
   <211> 200
   <212> DNA
   <213> Conyza canadensis
<400> 454
   atcctctaat ttgaaccccc atccattttg gagctgtgtg agttgttttg agttaaatca   60
   tcactacatt ttttttctta cagtttgaat ttggcttctg gaatctgcat tctaatcatg  120
   ttgttttggc tctaaattac agtttgggcc gatgcggata catggtttga acgacagcaa  180
   caagaagaag agatgcaacg                                              200
<210> 455
   <211> 200
   <212> DNA
   <213> Conyza canadensis
<400> 455
   tggatggggg ttcaaattag aggatagcct aatagataga agcttctaat tatggggaaa   60
   aaaaaacgat catggcaatg tattacacaa tctaaagcaa ccactatgaa taataaagat  120
   aaatctttcc gagagtccac atgccttgat atttcataag ataggctttt tcagaaatca  180
   gaatcttctt ggcaaatata                                              200
<210> 456
   <211> 200
   <212> DNA
   <213> Conyza canadensis
<400> 456
   tatatttgcc aagaagattc tgatttctga aaaagcctat cttatgaaat atcaaggcat   60
   gtggactctc ggaaagattt atctttatta ttcatagtgg ttgctttaga ttgtgtaata  120
   cattgccatg atcgtttttt tttccccata attagaagct tctatctatt aggctatcct  180
   ctaatttgaa cccccatcca                                               200
<210> 457
   <211> 200
   <212> DNA
   <213> Conyza canadensis
<400> 457
   aatcagaatc ttcttggcaa atataacact aaaatcagtc taatgcatgt tattaagtaa   60
   atctttattt gtccatagta taatgccata gattatcgtc aattatccac atcgtcatta  120
   aatatcaagt aaccacatta tgaattgact tgtttaatca aagttcatct tacttcttca  180
   tagatgaaaa agagctatgc                                               200
<210> 458
   <211> 200
   <212> DNA
   <213> Conyza canadensis
<400> 458
   gcatagctct ttttcatcta tgaagaagta agatgaactt tgattaaaca agtcaattca   60
   taatgtggtt acttgatatt taatgacgat gtggataatt gacgataatc tatggcatta  120
   tactatggac aaataaagat ttacttaata acatgcatta gactgatttt agtgttatat  180
   ttgccaagaa gattctgatt                                               200
<210> 459
   <211> 200
   <212> DNA
   <213> Conyza canadensis
<400> 459
   cttcatagat gaaaaagagc tatgcaaata cagaacacat cagttgttct aacatataat 60
   tactgctagc tgtaggaaga aaccaagatg aaacttatct aggattcaaa ttcatgggga 120
   tctgttattt agatgaattt agagaaataa gaaaataagt tgagtgaagt cagtttcgag 180
   gttccaaatt tgtctttatt 200
<210> 460
   <211> 200
   <212> DNA
   <213> Conyza canadensis
<400> 460
   aataaagaca aatttggaac ctcgaaactg acttcactca acttattttc ttatttctct 60
   aaattcatct aaataacaga tccccatgaa tttgaatcct agataagttt catcttggtt 120
   tcttcctaca gctagcagta attatatgtt agaacaactg atgtgttctg tatttgcata 180
   gctctttttc atctatgaag 200
<210> 461
   <211> 200
   <212> DNA
   <213> Conyza canadensis
<400> 461
   tcgaggttcc aaatttgtct ttattagttg agctaacaat attaacctct gaagcatgta 60
   tagtgtactt gtatgaacca cttaaggata taaatgaagc caaatcagtt tcattcgcta 120
   attgagcaaa tgcacgttct gtttgttagt attgaaattt ctttacagga tttctttcaa 180
   ctaaaagcat cacatgtccg 200
<210> 462
   <211> 200
   <212> DNA
   <213> Conyza canadensis
<400> 462
   cggacatgtg atgcttttag ttgaaagaaa tcctgtaaag aaatttcaat actaacaaac 60
   agaacgtgca tttgctcaat tagcgaatga aactgatttg gcttcattta tatccttaag 120
   tggttcatac aagtacacta tacatgcttc agaggttaat attgttagct caactaataa 180
   agacaaattt ggaacctcga 200
<210> 463
   <211> 200
   <212> DNA
   <213> Conyza canadensis
<400> 463
   ttcaactaaa agcatcacat gtccgactct cggtttgata tttgtgacaa gtctaattgg 60
   ttcatcagat gtacttatgg ctaaatttcg aaacttgaaa caataagagc ttagcactct 120
   taaccataaa gggttgctgt taatcttact tgttcttgaa agcaattcca cagtgaagca 180
   tttgttaatt gggaagtttt 200
<210> 464
   <211> 200
   <212> DNA
   <213> Conyza canadensis
<400> 464
   aaaacttccc aattaacaaa tgcttcactg tggaattgct ttcaagaaca agtaagatta 60
   acagcaaccc tttatggtta agagtgctaa gctcttattg tttcaagttt cgaaatttag 120
   ccataagtac atctgatgaa ccaattagac ttgtcacaaa tatcaaaccg agagtcggac 180
   atgtgatgct tttagttgaa 200
<210> 465
   <211> 200
   <212> DNA
   <213> Conyza canadensis
<400> 465
   ttttcttctt ctagttggag ctcttttacg tggtgtcttc ttttccgagt catcttcttc 60
   cccatcagat ggattttctg cagccttttt tctacgaact gctgtaatag caaatgtact 120
   acgcaatcta acatctggga attgtacaaa gtttattgaa gatagtgaag gcagattcga 180
   ctgccaccta cacatataag 200
<210> 466
   <211> 200
   <212> DNA
   <213> Conyza canadensis
<400> 466
   cttatatgtg taggtggcag tcgaatctgc cttcactatc ttcaataaac tttgtacaat 60
   tcccagatgt tagattgcgt agtacatttg ctattacagc agttcgtaga aaaaaggctg 120
   cagaaaatcc atctgatggg gaagaagatg actcggaaaa gaagacacca cgtaaaagag 180
   ctccaactag aagaagaaaa 200
<210> 467
   <211> 200
   <212> DNA
   <213> Conyza canadensis
<400> 467
   ttcgactgcc acctacacat ataagaaacc attaacttct atacttaact cacaatataa   60
   aacatcattt gcatgttcgt caaaaacaat accatttctt atctttgctc aatggatatg  120
   aaatctgcca atacaagatt attcatttga cctcaatcta gcatctgatg tctattcaag  180
   gtatcgttta taatgaagat                                              200
<210> 468
   <211> 200
   <212> DNA
   <213> Conyza canadensis
<400> 468
   atcttcatta taaacgatac cttgaataga catcagatgc tagattgagg tcaaatgaat   60
   aatcttgtat tggcagattt catatccatt gagcaaagat aagaaatggt attgtttttg  120
   acgaacatgc aaatgatgtt ttatattgtg agttaagtat agaagttaat ggtttcttat  180
   atgtgtaggt ggcagtcgaa                                              200
<210> 469
   <211> 200
   <212> DNA
   <213> Conyza canadensis
<400> 469
   tcaaggtatc gtttataatg aagattattg tacttcaaaa tactttcttg tgtagtggta   60
   tcaaattttg agttaaagtt actccatttg acctcaaaag ccaaataatg agcaacaaac  120
   aacacaattt cacctccctt ataccctgcg actatatttt atcttcaaat caatccattt  180
   ccatgtttat cctctccata                                              200
<210> 470
   <211> 200
   <212> DNA
   <213> Conyza canadensis
<400> 470
   tatggagagg ataaacatgg aaatggattg atttgaagat aaaatatagt cgcagggtat   60
   aagggaggtg aaattgtgtt gtttgttgct cattatttgg cttttgaggt caaatggagt  120
   aactttaact caaaatttga taccactaca caagaaagta ttttgaagta caataatctt  180
   cattataaac gataccttga                                              200
<210> 471
   <211> 200
   <212> DNA
   <213> Conyza canadensis
<400> 471
   catttccatg tttatcctct ccatacagct acataataag ttttagggaa aaaacaaaac 60
   taaactaaac taagtttaca gaattcccat ttcttatcct aaaatttaca agcaattcaa 120
   catatattct ttatttacaa ctataaatag catatgggta tgttggtata tagatataga 180
   tatagatata catatgtaga 200
<210> 472
   <211> 200
   <212> DNA
   <213> Conyza canadensis
<400> 472
   tctacatatg tatatctata tctatatcta tataccaaca tacccatatg ctatttatag 60
   ttgtaaataa agaatatatg ttgaattgct tgtaaatttt aggataagaa atgggaattc 120
   tgtaaactta gtttagttta gttttgtttt ttccctaaaa cttattatgt agctgtatgg 180
   agaggataaa catggaaatg 200
<210> 473
   <211> 200
   <212> DNA
   <213> Conyza canadensis
<400> 473
   atagatatag atatacatat gtagatgtgt attatatgtg tgtaaatggg gagaaaaaag 60
   gaaacatata ttacctgtgt aaagaacaaa attgagtaaa agaaggcaaa gaagccattt 120
   ctaaccacta aaatcttggg ctttcaagaa tcccacttca aagctaccat ttcacatacc 180
   aaacccccct ttctatgggt 200
<210> 474
   <211> 200
   <212> DNA
   <213> Conyza canadensis
<400> 474
   acccatagaa aggggggttt ggtatgtgaa atggtagctt tgaagtggga ttcttgaaag 60
   cccaagattt tagtggttag aaatggcttc tttgccttct tttactcaat tttgttcttt 120
   acacaggtaa tatatgtttc cttttttctc cccatttaca cacatataat acacatctac 180
   atatgtatat ctatatctat 200
<210> 475
   <211> 200
   <212> DNA
   <213> Conyza canadensis
<400> 475
   ataccaaacc cccctttcta tgggtttaaa gatgagggct ttacatttat ctcttttaca 60
   atccaaattt tccaaaaccg aaataatgta cgacattcgg tgcacaaggt acaaacagaa 120
   aaaactactt ttgtatttta gatctcaaga aaggagtggc ctttttggta ataacatact 180
   ttattcaatt gccacttacc 200
<210> 476
   <211> 200
   <212> DNA
   <213> Conyza canadensis
<400> 476
   ggtaagtggc aattgaataa agtatgttat taccaaaaag gccactcctt tcttgagatc 60
   taaaatacaa aagtagtttt ttctgtttgt accttgtgca ccgaatgtcg tacattattt 120
   cggttttgga aaatttggat tgtaaaagag ataaatgtaa agccctcatc tttaaaccca 180
   tagaaagggg ggtttggtat 200
<210> 477
   <211> 200
   <212> DNA
   <213> Conyza canadensis
<400> 477
   atactttatt caattgccac ttaccttcaa gaaattaaca aaattaccca caattaaaaa 60
   agggtttccc agtttcactc acacttattc aagattgatg caaaataaac ccataaataa 120
   gaaggttaaa atagtgttaa agccttaaag gtgcttgctt tttgtgtttt ttgtgatgcc 180
   tataaaaaga ttcttattgt 200
<210> 478
   <211> 200
   <212> DNA
   <213> Conyza canadensis
<400> 478
   acaataagaa tctttttata ggcatcacaa aaaacacaaa aagcaagcac ctttaaggct    60
   ttaacactat tttaaccttc ttatttatgg gtttattttg catcaatctt gaataagtgt   120
   gagtgaaact gggaaaccct tttttaattg tgggtaattt tgttaatttc ttgaaggtaa   180
   gtggcaattg aataaagtat                                               200
<210> 479
   <211> 200
   <212> DNA
   <213> Conyza canadensis
<400> 479
   atgcctataa aaagattctt attgttttca aattttttta tgtcaagttt tcgtttttaa    60
   cagtttatat agttatataa tttgcagaaa gccataaagc tctgaacttg aatgttttat   120
   agcagctctt tgcagtaata tcttgtagag gtaaacctgt taacccatat ttattaattt   180
   gtacataaaa ttgttgaatt                                               200
<210> 480
   <211> 200
   <212> DNA
   <213> Conyza canadensis
<400> 480
   aattcaacaa ttttatgtac aaattaataa atatgggtta acaggtttac ctctacaaga    60
   tattactgca aagagctgct ataaaacatt caagttcaga gctttatggc tttctgcaaa   120
   ttatataact atataaactg ttaaaaacga aaacttgaca taaaaaaatt tgaaaacaat   180
   aagaatcttt ttataggcat                                               200
<210> 481
   <211> 200
   <212> DNA
   <213> Conyza canadensis
<400> 481
   aatttgtaca taaaattgtt gaattgaata gagctaccac tgggacttgt gtagtcaaac    60
   tgtagagcat ccctgtaatg ctggaagctt tatgacctaa aaggatttgt agacttgtta   120
   tctgtggatt ggtataaaga ttttcaagtt gttattttga aactttgaat agtatattat   180
   gctcattact gaatgataca                                               200
<210> 482
   <211> 200
   <212> DNA
   <213> Conyza canadensis
<400> 482
   tgtatcattc agtaatgagc ataatatact attcaaagtt tcaaaataac aacttgaaaa 60
   tctttatacc aatccacaga taacaagtct acaaatcctt ttaggtcata aagcttccag 120
   cattacaggg atgctctaca gtttgactac acaagtccca gtggtagctc tattcaattc 180
   aacaatttta tgtacaaatt 200
<210> 483
   <211> 200
   <212> DNA
   <213> Conyza canadensis
<400> 483
   attatgctca ttactgaatg atacaatagg gagtcgcgaa tgttagaatt gataagaatt 60
   aggatgaagt ttgtgtttga acgttccttc tgtttataat cttcatgtca cttccatagt 120
   accataaccc tagggcaaca cgagccttta gattatgaat gggcacaccc aattgtttag 180
   accttgtata aatgaagtga 200
<210> 484
   <211> 200
   <212> DNA
   <213> Conyza canadensis
<400> 484
   tcacttcatt tatacaaggt ctaaacaatt gggtgtgccc attcataatc taaaggctcg 60
   tgttgcccta gggttatggt actatggaag tgacatgaag attataaaca gaaggaacgt 120
   tcaaacacaa acttcatcct aattcttatc aattctaaca ttcgcgactc cctattgtat 180
   cattcagtaa tgagcataat 200
<210> 485
   <211> 200
   <212> DNA
   <213> Conyza canadensis
<400> 485
   tttagacctt gtataaatga agtgaatatc attgttataa gcattctggt ggtggttttg 60
   cagatttacc tttctctatc taatttgaag gtttgttgat taaggtgata caaatgagtg 120
   ttttcaagca gctaatagct tcaagattag tacgcactca tgtctgtgga tcttatagag 180
   gtacacttcc tcgaacatta 200
<210> 486
   <211> 200
   <212> DNA
   <213> Conyza canadensis
<400> 486
   taatgttcga ggaagtgtac ctctataaga tccacagaca tgagtgcgta ctaatcttga 60
   agctattagc tgcttgaaaa cactcatttg tatcacctta atcaacaaac cttcaaatta 120
   gatagagaaa ggtaaatctg caaaaccacc accagaatgc ttataacaat gatattcact 180
   tcatttatac aaggtctaaa 200
<210> 487
   <211> 200
   <212> DNA
   <213> Conyza canadensis
<400> 487
   tagaggtaca cttcctcgaa cattatatag cgatgttatt gttaagtttt gaatctgtag 60
   caacattata tataattttt tttgcattgt tttcctgcag catccttttc actctttcat 120
   tcgtcctcgg atacttcaat acaagtgcat tctccagaac aagaagtagt aattgcttta 180
   ggtagcaatg tgggtaatag 200
<210> 488
   <211> 200
   <212> DNA
   <213> Conyza canadensis
<400> 488
   ctattaccca cattgctacc taaagcaatt actacttctt gttctggaga atgcacttgt 60
   attgaagtat ccgaggacga atgaaagagt gaaaaggatg ctgcaggaaa acaatgcaaa 120
   aaaaattata tataatgttg ctacagattc aaaacttaac aataacatcg ctatataatg 180
   ttcgaggaag tgtacctcta 200
<210> 489
   <211> 200
   <212> DNA
   <213> Conyza canadensis
<400> 489
   ctttaggtag caatgtgggt aatagactta ataactttaa tgaagcctta tcccagatga 60
   agaaatcggg gatagaaata acaaggcatg cttgtctata cgaaactgaa ccagcttatg 120
   tgacggacca gcctcttttt ctcaactctg ccgttagagc cactacaaag cttggccctc 180
   atgagctact gtccattctc 200
<210> 490
   <211> 200
   <212> DNA
   <213> Conyza canadensis
<400> 490
   gagaatggac agtagctcat gagggccaag ctttgtagtg gctctaacgg cagagttgag 60
   aaaaagaggc tggtccgtca cataagctgg ttcagtttcg tatagacaag catgccttgt 120
   tatttctatc cccgatttct tcatctggga taaggcttca ttaaagttat taagtctatt 180
   acccacattg ctacctaaag 200
<210> 491
   <211> 200
   <212> DNA
   <213> Conyza canadensis
<400> 491
   ccctcatgag ctactgtcca ttctcaagaa aattgaaaag gaaatgggtc gaaccaaagg 60
   gcttaggtac ggcccacgac ccattgacct agatatattg ttctatggta aatgcaaagt 120
   taactctgat attctaactg ttcctcatga aagaatcttt gagaggccat tcgttatggc 180
   tccattagtt gacttgttag 200
<210> 492
   <211> 200
   <212> DNA
   <213> Conyza canadensis
<400> 492
   ctaacaagtc aactaatgga gccataacga atggcctctc aaagattctt tcatgaggaa 60
   cagttagaat atcagagtta actttgcatt taccatagaa caatatatct aggtcaatgg 120
   gtcgtgggcc gtacctaagc cctttggttc gacccatttc cttttcaatt ttcttgagaa 180
   tggacagtag ctcatgaggg 200
<210> 493
   <211> 200
   <212> DNA
   <213> Conyza canadensis
<400> 493
   atggctccat tagttgactt gttaggatca gatgtagaca atgatacggt tctatgctgg   60
   cattcttttt caaaaaatgg gctttttgga tcttgggaaa cattaggtgg tgaatcttcc  120
   ataggaaaag atggtttaag aagggtttta cctgtcaatg atcgtttatg ggattggtca  180
   aagaaaactt ccgtcatggg                                              200
<210> 494
   <211> 200
   <212> DNA
   <213> Conyza canadensis
<400> 494
   cccatgacgg aagttttctt tgaccaatcc cataaacgat cattgacagg taaaaccctt   60
   cttaaaccat cttttcctat ggaagattca ccacctaatg tttcccaaga tccaaaaagc  120
   ccattttttg aaaaagaatg ccagcataga accgtatcat tgtctacatc tgatcctaac  180
   aagtcaacta atggagccat                                              200
<210> 495
   <211> 200
   <212> DNA
   <213> Conyza canadensis
<400> 495
   ggtcaaagaa aacttccgtc atgggtattt tgaatataac tcctgatagt tttagtgatg   60
   gagggaagtt tgattccatg gggtccgctt tatctcgtgt tcagaccatg atatctgaag  120
   gggttgacat aattgatcta ggagctcaat ccacacgccc aatggcgacc aagatctcag  180
   tcgaagagga actagatagg                                              200
<210> 496
   <211> 200
   <212> DNA
   <213> Conyza canadensis
<400> 496
   cctatctagt tcctcttcga ctgagatctt ggtcgccatt gggcgtgtgg attgagctcc   60
   tagatcaatt atgtcaaccc cttcagatat catggtctga acacgagata aagcggaccc  120
   catggaatca aacttccctc catcactaaa actatcagga gttatattca aaatacccat  180
   gacggaagtt ttctttgacc                                              200
<210> 497
   <211> 200
   <212> DNA
   <213> Conyza canadensis
<400> 497
   ctcagtcgaa gaggaactag ataggctaat acccgttctt gaaaagattc ttgaattacc 60
   tgaaattgaa ggaaagttgt tgtctgtgga cacattttac tcggaagttg cttcagaggc 120
   aatcaagaaa ggggctcata tggtcaatga tgtatcgggt ggaatgttag attctgatat 180
   gcttcatgtt gtggctgatc 200
<210> 498
   <211> 200
   <212> DNA
   <213> Conyza canadensis
<400> 498
   gatcagccac aacatgaagc atatcagaat ctaacattcc acccgataca tcattgacca 60
   tatgagcccc tttcttgatt gcctctgaag caacttccga gtaaaatgtg tccacagaca 120
   acaactttcc ttcaatttca ggtaattcaa gaatcttttc aagaacgggt attagcctat 180
   ctagttcctc ttcgactgag 200
<210> 499
   <211> 200
   <212> DNA
   <213> Conyza canadensis
<400> 499
   gatatgcttc atgttgtggc tgatctaaat gttccatata tcactatgca catgagaggg 60
   gacccatcca caatgcaaaa cagtgagaat ttgaagtatg atgatgttgt caaagaagtt 120
   ggggaggaat tgtatgaacg tgtaaggaat gcagagttat gtggtgttcc cgcatggagg 180
   atggttcttg atccagggat 200
<210> 500
   <211> 200
   <212> DNA
   <213> Conyza canadensis
<400> 500
   atccctggat caagaaccat cctccatgcg ggaacaccac ataactctgc attccttaca 60
   cgttcataca attcctcccc aacttctttg acaacatcat catacttcaa attctcactg 120
   ttttgcattg tggatgggtc ccctctcatg tgcatagtga tatatggaac atttagatca 180
   gccacaacat gaagcatatc 200
<210> 501
   <211> 200
   <212> DNA
   <213> Conyza canadensis
<400> 501
   ggaggatggt tcttgatcca gggatcgggt tttcaaagaa aaccgaagat aatttggaga    60
   tattgatggg actaaagagg tttagaagtg agattggaca aaagagctta ggggtgtctc   120
   gtgcaccttt gttaatcgga ccttcaagaa aaaggttttt gggtgagatt tgtggtcggc   180
   cttctgctgt tgagagagat                                               200
<210> 502
   <211> 200
   <212> DNA
   <213> Conyza canadensis
<400> 502
   atctctctca acagcagaag gccgaccaca aatctcaccc aaaaaccttt ttcttgaagg    60
   tccgattaac aaaggtgcac gagacacccc taagctcttt tgtccaatct cacttctaaa   120
   cctctttagt cccatcaata tctccaaatt atcttcggtt ttctttgaaa acccgatccc   180
   tggatcaaga accatcctcc                                               200
<210> 503
   <211> 200
   <212> DNA
   <213> Conyza canadensis
<400> 503
   tcggccttct gctgttgaga gagatccagg gactgtagct gctgttacca gtgcgatttt    60
   gggtggtgcc aatatcgtta gggttcataa cattggacat aatgtggatg ctgttaagct   120
   ttgtgattca atgttggatc gagctggtag atcttagtag cttaaagtta tttgacatct   180
   tgttttttat tcaattttct                                                200
<210> 504
   <211> 200
   <212> DNA
   <213> Conyza canadensis
<400> 504
   agaaaattga ataaaaaaca agatgtcaaa taactttaag ctactaagat ctaccagctc 60
   gatccaacat tgaatcacaa agcttaacag catccacatt atgtccaatg ttatgaaccc 120
   taacgatatt ggcaccaccc aaaatcgcac tggtaacagc agctacagtc cctggatctc 180
   tctcaacagc agaaggccga 200
<210> 505
   <211> 200
   <212> DNA
   <213> Conyza canadensis
<400> 505
   catcttgttt tttattcaat tttctggaca ttactttttg tatttctgtc tggcaaatga 60
   ttttacaggg atatcctaga ggctaaattg tatattacat tcttgatttt ctcacaaagt 120
   tttacttttt cagtttttct tgttcagttt tgtttttgaa aatttgctag atcagattaa 180
   atcgttgctt tatgatcttc 200
<210> 506
   <211> 200
   <212> DNA
   <213> Conyza canadensis
<400> 506
   gaagatcata aagcaacgat ttaatctgat ctagcaaatt ttcaaaaaca aaactgaaca 60
   agaaaaactg aaaaagtaaa actttgtgag aaaatcaaga atgtaatata caatttagcc 120
   tctaggatat ccctgtaaaa tcatttgcca gacagaaata caaaaagtaa tgtccagaaa 180
   attgaataaa aaacaagatg 200
<210> 507
   <211> 200
   <212> DNA
   <213> Conyza canadensis
<400> 507
   attaaatcgt tgctttatga tcttcgtgtc ttttttggga cgctgcgagt ttcatttagt 60
   ggtattgtaa actcctttta aagtttgtac acacttggta tttatgcttc tagttttcgt 120
   ctatgtctgt cctatattgt gtatagtatg tttaaagttc ataaaaagag gataaacgaa 180
   tagcacgtaa atcttataat 200
<210> 508
   <211> 200
   <212> DNA
   <213> Conyza canadensis
<400> 508
   attataagat ttacgtgcta ttcgtttatc ctctttttat gaactttaaa catactatac   60
   acaatatagg acagacatag acgaaaacta gaagcataaa taccaagtgt gtacaaactt  120
   taaaaggagt ttacaatacc actaaatgaa actcgcagcg tcccaaaaaa gacacgaaga  180
   tcataaagca acgatttaat                                              200
<210> 509
   <211> 200
   <212> DNA
   <213> Conyza canadensis
<400> 509
   acgaatagca cgtaaatctt ataattcgat tccacagagt gcaagttatg ctttcctaaa   60
   tgaaagcaag agtctagaca gtttggtcaa cgttaacatg agactattgt agaatacaac  120
   accacaagaa accaaaactg tcttgtgcat ttctctcttt tccaggttta aaactcaata  180
   ccaagacttg atatcaacta                                              200
<210> 510
   <211> 200
   <212> DNA
   <213> Conyza canadensis
<400> 510
   tagttgatat caagtcttgg tattgagttt taaacctgga aaagagagaa atgcacaaga   60
   cagttttggt ttcttgtggt gttgtattct acaatagtct catgttaacg ttgaccaaac  120
   tgtctagact cttgctttca tttaggaaag cataacttgc actctgtgga atcgaattat  180
   aagatttacg tgctattcgt                                              200
<210> 511
   <211> 200
   <212> DNA
   <213> Conyza canadensis
<400> 511
   caataccaag acttgatatc aactattatt ctgcttcaat gattcagctt cagctatagc   60
   cataggatca tctttggtgt caggttcatc cttctcacca tctcgttcct ctgccagttt  120
   ttttctgtgc aattcctctg caacttgctt tgatgcttgg tcttcttttt gaaggcgttg  180
   catctcttct tcttgttgct                                              200
<210> 512
   <211> 200
   <212> DNA
   <213> Conyza canadensis
<400> 512
   agcaacaaga agaagagatg caacgccttc aaaaagaaga ccaagcatca aagcaagttg 60
   cagaggaatt gcacagaaaa aaactggcag aggaacgaga tggtgagaag gatgaacctg 120
   acaccaaaga tgatcctatg gctatagctg aagctgaatc attgaagcag aataatagtt 180
   gatatcaagt cttggtattg 200
<210> 513
   <211> 200
   <212> DNA
   <213> Conyza canadensis
<400> 513
   cgttgcatct cttcttcttg ttgctgtcgt tcaaaccatg tatccgcatc ggcccaaact 60
   gtaatttaga gccaaaacaa catgattaga atgcagattc cagaagccaa attcaaactg 120
   taagaaaaaa aatgtagtga tgatttaact caaaacaact cacacagctc caaaatggat 180
   gggggttcaa attagaggat 200
<210> 514
   <211> 200
   <212> DNA
   <213> Conyza canadensis
<400> 514
   atcctctaat ttgaaccccc atccattttg gagctgtgtg agttgttttg agttaaatca 60
   tcactacatt ttttttctta cagtttgaat ttggcttctg gaatctgcat tctaatcatg 120
   ttgttttggc tctaaattac agtttgggcc gatgcggata catggtttga acgacagcaa 180
   caagaagaag agatgcaacg 200
<210> 515
   <211> 200
   <212> DNA
   <213> Conyza canadensis
<400> 515
   tggatggggg ttcaaattag aggatagcct aatagataga agcttctaat tatggggaaa 60
   aaaaaacgat catggcaatg tattacacaa tctaaagcaa ccactatgaa taataaagat 120
   aaatctttcc gagagtccac atgccttgat atttcataag ataggctttt tcagaaatca 180
   gaatcttctt ggcaaatata 200
<210> 516
   <211> 200
   <212> DNA
   <213> Conyza canadensis
<400> 516
   tatatttgcc aagaagattc tgatttctga aaaagcctat cttatgaaat atcaaggcat   60
   gtggactctc ggaaagattt atctttatta ttcatagtgg ttgctttaga ttgtgtaata  120
   cattgccatg atcgtttttt tttccccata attagaagct tctatctatt aggctatcct  180
   ctaatttgaa cccccatcca                                               200
<210> 517
   <211> 200
   <212> DNA
   <213> Conyza canadensis
<400> 517
   aatcagaatc ttcttggcaa atataacact aaaatcagtc taatgcatgt tattaagtaa   60
   atctttattt gtccatagta taatgccata gattatcgtc aattatccac atcgtcatta  120
   aatatcaagt aaccacatta tgaattgact tgtttaatca aagttcatct tacttcttca  180
   tagatgaaaa agagctatgc                                               200
<210> 518
   <211> 200
   <212> DNA
   <213> Conyza canadensis
<400> 518
   gcatagctct ttttcatcta tgaagaagta agatgaactt tgattaaaca agtcaattca   60
   taatgtggtt acttgatatt taatgacgat gtggataatt gacgataatc tatggcatta  120
   tactatggac aaataaagat ttacttaata acatgcatta gactgatttt agtgttatat  180
   ttgccaagaa gattctgatt                                               200
<210> 519
   <211> 200
   <212> DNA
   <213> Conyza canadensis
<400> 519
   cttcatagat gaaaaagagc tatgcaaata cagaacacat cagttgttct aacatataat   60
   tactgctagc tgtaggaaga aaccaagatg aaacttatct aggattcaaa ttcatgggga  120
   tctgttattt agatgaattt agagaaataa gaaaataagt tgagtgaagt cagtttcgag  180
   gttccaaatt tgtctttatt                                               200
<210> 520
   <211> 200
   <212> DNA
   <213> Conyza canadensis
<400> 520
   aataaagaca aatttggaac ctcgaaactg acttcactca acttattttc ttatttctct   60
   aaattcatct aaataacaga tccccatgaa tttgaatcct agataagttt catcttggtt  120
   tcttcctaca gctagcagta attatatgtt agaacaactg atgtgttctg tatttgcata  180
   gctctttttc atctatgaag                                               200
<210> 521
   <211> 200
   <212> DNA
   <213> Conyza canadensis
<400> 521
   tcgaggttcc aaatttgtct ttattagttg agctaacaat attaacctct gaagcatgta   60
   tagtgtactt gtatgaacca cttaaggata taaatgaagc caaatcagtt tcattcgcta  120
   attgagcaaa tgcacgttct gtttgttagt attgaaattt ctttacagga tttctttcaa  180
   ctaaaagcat cacatgtccg                                               200
<210> 522
   <211> 200
   <212> DNA
   <213> Conyza canadensis
<400> 522
   cggacatgtg atgcttttag ttgaaagaaa tcctgtaaag aaatttcaat actaacaaac   60
   agaacgtgca tttgctcaat tagcgaatga aactgatttg gcttcattta tatccttaag  120
   tggttcatac aagtacacta tacatgcttc agaggttaat attgttagct caactaataa  180
   agacaaattt ggaacctcga                                               200
<210> 523
   <211> 200
   <212> DNA
   <213> Conyza canadensis
<400> 523
   ttcaactaaa agcatcacat gtccgactct cggtttgata tttgtgacaa gtctaattgg 60
   ttcatcagat gtacttatgg ctaaatttcg aaacttgaaa caataagagc ttagcactct 120
   taaccataaa gggttgctgt taatcttact tgttcttgaa agcaattcca cagtgaagca 180
   tttgttaatt gggaagtttt 200
<210> 524
   <211> 200
   <212> DNA
   <213> Conyza canadensis
<400> 524
   aaaacttccc aattaacaaa tgcttcactg tggaattgct ttcaagaaca agtaagatta 60
   acagcaaccc tttatggtta agagtgctaa gctcttattg tttcaagttt cgaaatttag 120
   ccataagtac atctgatgaa ccaattagac ttgtcacaaa tatcaaaccg agagtcggac 180
   atgtgatgct tttagttgaa 200
<210> 525
   <211> 200
   <212> DNA
   <213> Digitaria sanguinalis
<400> 525
   aaatcgtaaa atttataact aacatgttcc ttcatgaatc caaaagccaa gtaaacaggt 60
   ccatgagtgc ctttgtgaga ttgcttcgaa tagaatatac ctacggctct taatttcaga 120
   ttctcttagg tagtgtttag gtagcgtttg gaagtagata tttaggcttg gaattaggaa 180
   ttggcattga gatctacgaa 200
<210> 526
   <211> 200
   <212> DNA
   <213> Digitaria sanguinalis
<400> 526
   ttcgtagatc tcaatgccaa ttcctaattc caagcctaaa tatctacttc caaacgctac 60
   ctaaacacta cctaagagaa tctgaaatta agagccgtag gtatattcta ttcgaagcaa 120
   tctcacaaag gcactcatgg acctgtttac ttggcttttg gattcatgaa ggaacatgtt 180
   agttataaat tttacgattt 200
<210> 527
   <211> 200
   <212> DNA
   <213> Digitaria sanguinalis
<400> 527
   aggaattggc attgagatct acgaatacct gccgtttgga tgtctaagaa tttggaattg 60
   gaattgcagc ccaataccaa ccggcattcc ttggcctctc atctgcctgc cctcagtata 120
   gatagagctt atctccttca tcctcgcgga gtggtggcaa gcggcggcga gatccggacc 180
   acatggggtg tgggagcgcg 200
<210> 528
   <211> 200
   <212> DNA
   <213> Digitaria sanguinalis
<400> 528
   cgcgctccca caccccatgt ggtccggatc tcgccgccgc ttgccaccac tccgcgagga 60
   tgaaggagat aagctctatc tatactgagg gcaggcagat gagaggccaa ggaatgccgg 120
   ttggtattgg gctgcaattc caattccaaa ttcttagaca tccaaacggc aggtattcgt 180
   agatctcaat gccaattcct 200
<210> 529
   <211> 200
   <212> DNA
   <213> Digitaria sanguinalis
<400> 529
   ggaccacatg gggtgtggga gcgcggtggc agagtggtgg caagcgatgg cgacggcacc 60
   gtgccgccgt gccacattgg cacggcgacg gacgagtttg gatggtgtca gtacagacca 120
   ctgcccaatt ctatcctttt gtgcatccaa acataaaatc tgaattagga tgctcttttg 180
   attctacaag gcaatgtaat 200
<210> 530
   <211> 200
   <212> DNA
   <213> Digitaria sanguinalis
<400> 530
   attacattgc cttgtagaat caaaagagca tcctaattca gattttatgt ttggatgcac 60
   aaaaggatag aattgggcag tggtctgtac tgacaccatc caaactcgtc cgtcgccgtg 120
   ccaatgtggc acggcggcac ggtgccgtcg ccatcgcttg ccaccactct gccaccgcgc 180
   tcccacaccc catgtggtcc 200
<210> 531
   <211> 200
   <212> DNA
   <213> Digitaria sanguinalis
<400> 531
   ttttgattct acaaggcaat gtaatgacca tccaaacaac agattctgaa ttggagtcaa   60
   ttcaatgtta cgattgtatt ggcatcggac ctaattcaat gtcatgccct ctagtatcta  120
   cattcaaacg gagcggaaaa atctacctcc gtcccaagat tccggcccaa gatcccgact  180
   cttaatactt ttttcatctt                                               200
<210> 532
   <211> 200
   <212> DNA
   <213> Digitaria sanguinalis
<400> 532
   aagatgaaaa aagtattaag agtcgggatc ttgggccgga atcttgggac ggaggtagat   60
   ttttccgctc cgtttgaatg tagatactag agggcatgac attgaattag gtccgatgcc  120
   aatacaatcg taacattgaa ttgactccaa ttcagaatct gttgtttgga tggtcattac  180
   attgccttgt agaatcaaaa                                               200
<210> 533
   <211> 200
   <212> DNA
   <213> Digitaria sanguinalis
<400> 533
   cgactcttaa tacttttttc atctttcgct gaaatttcag cccaagatcc cggcccgcct   60
   ttttgcctcc acgctgggcc tggtgggacg cggccccttc acgagagtcg agaggggaaa  120
   gcggaaaggg aaacaaaaaa actcaacgaa tcgccgccgc cgccttgccc tgcagcgaac  180
   aattaagggc cccgtacctc                                               200
<210> 534
   <211> 200
   <212> DNA
   <213> Digitaria sanguinalis
<400> 534
   gaggtacggg gcccttaatt gttcgctgca gggcaaggcg gcggcggcga ttcgttgagt   60
   ttttttgttt ccctttccgc tttcccctct cgactctcgt gaaggggccg cgtcccacca  120
   ggcccagcgt ggaggcaaaa aggcgggccg ggatcttggg ctgaaatttc agcgaaagat 180
   gaaaaaagta ttaagagtcg 200
<210> 535
   <211> 200
   <212> DNA
   <213> Digitaria sanguinalis
<400> 535
   cgaacaatta agggccccgt acctctctct gcccgttctt ccgctgcact ccgcatctga 60
   gcggacgggt ggggacaccg ccgacggcgc cggggtggcg cctccgtctg tgctccaacc 120
   aaggcagtgg gtgctggttg ctgccaatcg accggccggg catgctctga ccatcgagaa 180
   aatcttcggg taacacaacg 200
<210> 536
   <211> 200
   <212> DNA
   <213> Digitaria sanguinalis
<400> 536
   cgttgtgtta cccgaagatt ttctcgatgg tcagagcatg cccggccggt cgattggcag 60
   caaccagcac ccactgcctt ggttggagca cagacggagg cgccaccccg gcgccgtcgg 120
   cggtgtcccc acccgtccgc tcagatgcgg agtgcagcgg aagaacgggc agagagaggt 180
   acggggccct taattgttcg 200
<210> 537
   <211> 200
   <212> DNA
   <213> Digitaria sanguinalis
<400> 537
   gagaaaatct tcgggtaaca caacgttgtt catttcgttt gtatcgtggc atagttgatt 60
   tccctaactt ttaggatgcg tttcgattca ttgtatctgt ataccaaaac agagttcgat 120
   aacttagatt gaacggtagg cttcagcatc taaggcagca ggtgcaggtc tattctatca 180
   ctctgaaaat tagttttaac 200
<210> 538
   <211> 200
   <212> DNA
   <213> Digitaria sanguinalis
<400> 538
   gttaaaacta attttcagag tgatagaata gacctgcacc tgctgcctta gatgctgaag 60
   cctaccgttc aatctaagtt atcgaactct gttttggtat acagatacaa tgaatcgaaa 120
   cgcatcctaa aagttaggga aatcaactat gccacgatac aaacgaaatg aacaacgttg 180
   tgttacccga agattttctc 200
<210> 539
   <211> 200
   <212> DNA
   <213> Digitaria sanguinalis
<400> 539
   tatcactctg aaaattagtt ttaactgtct gatgatagga ggattagcta atgttgttat 60
   atgaggaggt ttcttgctga ctagagagct atttgaccac ctgatagatt gcctcgttag 120
   caacttagca ttatgacgac gatggcagcg agccatttca tgtagaaaac gaacatgagc 180
   tgagaagctt aatcgtataa 200
<210> 540
   <211> 200
   <212> DNA
   <213> Digitaria sanguinalis
<400> 540
   ttatacgatt aagcttctca gctcatgttc gttttctaca tgaaatggct cgctgccatc 60
   gtcgtcataa tgctaagttg ctaacgaggc aatctatcag gtggtcaaat agctctctag 120
   tcagcaagaa acctcctcat ataacaacat tagctaatcc tcctatcatc agacagttaa 180
   aactaatttt cagagtgata 200
<210> 541
   <211> 200
   <212> DNA
   <213> Digitaria sanguinalis
<400> 541
   tgagctgaga agcttaatcg tataagcttc aattggcttc ctggctgggg aggtttgcta 60
   gagaacttct gtattttgtt cattcattta atctgatgta tgttggcagc tctcctgtca 120
   ggtacagaaa tttagacggc cctgaaacca agcagggcaa cctcaccaat aggggggcag 180
   tcgcaccaca aaggttgctt 200
<210> 542
   <211> 200
   <212> DNA
   <213> Digitaria sanguinalis
<400> 542
   aagcaacctt tgtggtgcga ctgcccccct attggtgagg ttgccctgct tggtttcagg 60
   gccgtctaaa tttctgtacc tgacaggaga gctgccaaca tacatcagat taaatgaatg 120
   aacaaaatac agaagttctc tagcaaacct ccccagccag gaagccaatt gaagcttata 180
   cgattaagct tctcagctca 200
<210> 543
   <211> 200
   <212> DNA
   <213> Digitaria sanguinalis
<400> 543
   ggcagtcgca ccacaaaggt tgcttgcctc cagtggggca tctcctctcc ttacattgag 60
   aggtttattt gacgtctaag atatctgaac tcctgcctcc agttagagaa aaggcccttg 120
   ttcatacctc aaatttgtct ttgctcttct attgaccagg tgtgatggag tgatgcttgc 180
   gtgaaatatc ctacaaggct 200
<210> 544
   <211> 200
   <212> DNA
   <213> Digitaria sanguinalis
<400> 544
   agccttgtag gatatttcac gcaagcatca ctccatcaca cctggtcaat agaagagcaa 60
   agacaaattt gaggtatgaa caagggcctt ttctctaact ggaggcagga gttcagatat 120
   cttagacgtc aaataaacct ctcaatgtaa ggagaggaga tgccccactg gaggcaagca 180
   acctttgtgg tgcgactgcc 200
<210> 545
   <211> 200
   <212> DNA
   <213> Digitaria sanguinalis
<400> 545
   cttgcgtgaa atatcctaca aggctacaat ttatttgaag catttgcttt cttaattatc 60
   atatgttcaa cgatgctcct ccatgctaag gagtccctta agaagatgta tcctgttgct 120
   atgaactact ttggagggct cctcccatcg cattcgtttt caggtactgt tcaaatcttt 180
   tcttttcaaa tcccctatca 200
<210> 546
   <211> 200
   <212> DNA
   <213> Digitaria sanguinalis
<400> 546
   tgatagggga tttgaaaaga aaagatttga acagtacctg aaaacgaatg cgatgggagg   60
   agccctccaa agtagttcat agcaacagga tacatcttct taagggactc cttagcatgg  120
   aggagcatcg ttgaacatat gataattaag aaagcaaatg cttcaaataa attgtagcct  180
   tgtaggatat ttcacgcaag                                              200
<210> 547
   <211> 200
   <212> DNA
   <213> Digitaria sanguinalis
<400> 547
   tcttttcttt tcaaatcccc tatcatgcga tcaagagcag ctctaagctt ctaactcacc   60
   cctttgcatt tctaaacagt gtctgatctt gttcatccgc tgaggttccc aaacagagct  120
   cctagacatg ctattccatt cagggctcgt tcgtttacgc gatgttcgct tgagggatgt  180
   tcaactgacc aagagattgt                                              200
<210> 548
   <211> 200
   <212> DNA
   <213> Digitaria sanguinalis
<400> 548
   acaatctctt ggtcagttga acatccctca agcgaacatc gcgtaaacga acgagccctg   60
   aatggaatag catgtctagg agctctgttt gggaacctca gcggatgaac aagatcagac  120
   actgtttaga aatgcaaagg ggtgagttag aagcttagag ctgctcttga tcgcatgata  180
   ggggatttga aaagaaaaga                                              200
<210> 549
   <211> 200
   <212> DNA
   <213> Digitaria sanguinalis
<400> 549
   gatgttcaac tgaccaagag attgtgattg ctatgggaag caacgtgggt gatggagtca 60
   gtacatttga cagggcatta caaatgatga aaagctcagg cgtgaacatc actaggcatg 120
   cctgtctata tgagactgcc cctgcttatg tgaccgatca gccacggttc ctgaactctg 180
   ccattcgagg tacgactagg 200
<210> 550
   <211> 200
   <212> DNA
   <213> Digitaria sanguinalis
<400> 550
   cctagtcgta cctcgaatgg cagagttcag gaaccgtggc tgatcggtca cataagcagg 60
   ggcagtctca tatagacagg catgcctagt gatgttcacg cctgagcttt tcatcatttg 120
   taatgccctg tcaaatgtac tgactccatc acccacgttg cttcccatag caatcacaat 180
   ctcttggtca gttgaacatc 200
<210> 551
   <211> 200
   <212> DNA
   <213> Digitaria sanguinalis
<400> 551
   ctctgccatt cgaggtacga ctaggctggg acctcacgag ttgcttaaga aactaaagga 60
   aattgagaag gatataggac gcactggtgg aataaggtat ggcccaagac caatcgatct 120
   agatatactt ctgtacggca attcccagat tgatactgag gctctaattg tgccacatga 180
   acgcatccag gagaggccat 200
<210> 552
   <211> 200
   <212> DNA
   <213> Digitaria sanguinalis
<400> 552
   atggcctctc ctggatgcgt tcatgtggca caattagagc ctcagtatca atctgggaat 60
   tgccgtacag aagtatatct agatcgattg gtcttgggcc ataccttatt ccaccagtgc 120
   gtcctatatc cttctcaatt tcctttagtt tcttaagcaa ctcgtgaggt cccagcctag 180
   tcgtacctcg aatggcagag 200
<210> 553
   <211> 200
   <212> DNA
   <213> Digitaria sanguinalis
<400> 553
   catgaacgca tccaggagag gccatttgtt ctagcacctc ttgttgacct tctaggtgca 60
   tctggcaatg atagtgtcga aacaagctgg cactctcttt caaagtgcag tggtggtttc 120
   tttgaattat ggaataaact tgggggtgaa tctataattg gaacagaaag tattaaaagg 180
   gtattacctg ttgggaatca 200
<210> 554
   <211> 200
   <212> DNA
   <213> Digitaria sanguinalis
<400> 554
   tgattcccaa caggtaatac ccttttaata ctttctgttc caattataga ttcaccccca 60
   agtttattcc ataattcaaa gaaaccacca ctgcactttg aaagagagtg ccagcttgtt 120
   tcgacactat cattgccaga tgcacctaga aggtcaacaa gaggtgctag aacaaatggc 180
   ctctcctgga tgcgttcatg 200
<210> 555
   <211> 200
   <212> DNA
   <213> Digitaria sanguinalis
<400> 555
   aaagggtatt acctgttggg aatcatttgt ttgattggtg tgagagaacc ctcgtcatgg 60
   gggtccttaa tctaacacca gacagcttta gtgatggagg taagtttcaa caagtggaag 120
   ctgctatttc tcaggctaaa ttattaattt cagaaggtgc agatatcata gatattggtg 180
   ctcaatctac caggcctttt 200
<210> 556
   <211> 200
   <212> DNA
   <213> Digitaria sanguinalis
<400> 556
   aaaaggcctg gtagattgag caccaatatc tatgatatct gcaccttctg aaattaataa 60
   tttagcctga gaaatagcag cttccacttg ttgaaactta cctccatcac taaagctgtc 120
   tggtgttaga ttaaggaccc ccatgacgag ggttctctca caccaatcaa acaaatgatt 180
   cccaacaggt aatacccttt 200
<210> 557
   <211> 200
   <212> DNA
   <213> Digitaria sanguinalis
<400> 557
   tggtgctcaa tctaccaggc cttttgcaaa gagattatct ccaaacgaag aaattgaaag   60
   attggttcct gttctggatg tgattacgaa aattcctgaa atggagggaa agttgctctc  120
   agtggataca ttctatgcag aagttgcatg tgaagctgtg aaaagaggag ttcacatgat  180
   caatgatgta tctggtggac                                              200
<210> 558
   <211> 200
   <212> DNA
   <213> Digitaria sanguinalis
<400> 558
   gtccaccaga tacatcattg atcatgtgaa ctcctctttt cacagcttca catgcaactt   60
   ctgcatagaa tgtatccact gagagcaact ttccctccat ttcaggaatt ttcgtaatca  120
   catccagaac aggaaccaat ctttcaattt cttcgtttgg agataatctc tttgcaaaag  180
   gcctggtaga ttgagcacca                                              200
<210> 559
   <211> 200
   <212> DNA
   <213> Digitaria sanguinalis
<400> 559
   atgatcaatg atgtatctgg tggacagctt gaccccaaaa ttcttaaagt tgctgctgaa   60
   ctcaaagttc cgtatgttgc aatgcacatg aggggagatc catcaacaat gcaaagtgaa  120
   caaaatttac agtatgacga tgtctgcaag gaagttgctt cagagctata tgcacaggtg  180
   agagaagcag agttatctgg                                              200
<210> 560
   <211> 200
   <212> DNA
   <213> Digitaria sanguinalis
<400> 560
   ccagataact ctgcttctct cacctgtgca tatagctctg aagcaacttc cttgcagaca   60
   tcgtcatact gtaaattttg ttcactttgc attgttgatg gatctcccct catgtgcatt  120
   gcaacatacg gaactttgag ttcagcagca actttaagaa ttttggggtc aagctgtcca  180
   ccagatacat cattgatcat                                              200
<210> 561
   <211> 200
   <212> DNA
   <213> Digitaria sanguinalis
<400> 561
   aggtgagaga agcagagtta tctgggattc cattgtggag gatagttcta gatccaggca 60
   ttgggttctc caagaaatcc aaacataacc ttgaagtaat tatgggattg gaatccatta 120
   ggagggagat gagtaaaatg agtattggtg cttcacatgt gccaatatta ctgggaccct 180
   ctaggaaaag atttttgggt 200
<210> 562
   <211> 200
   <212> DNA
   <213> Digitaria sanguinalis
<400> 562
   acccaaaaat cttttcctag agggtcccag taatattggc acatgtgaag caccaatact 60
   cattttactc atctccctcc taatggattc caatcccata attacttcaa ggttatgttt 120
   ggatttcttg gagaacccaa tgcctggatc tagaactatc ctccacaatg gaatcccaga 180
   taactctgct tctctcacct 200
<210> 563
   <211> 200
   <212> DNA
   <213> Digitaria sanguinalis
<400> 563
   accctctagg aaaagatttt tgggtgaaat atgcaatcgt gaaaatccag ttgagagaga 60
   tgttgctact gttgcagctg tgacggcggg tattttgaat ggtgctaaca tagtaagggt 120
   acataatgca ggatacagtt cagatgctgc aaagttttgt gatgcattga ataagagaag 180
   aagaatggaa gactgaacca 200
<210> 564
   <211> 200
   <212> DNA
   <213> Digitaria sanguinalis
<400> 564
   tggttcagtc ttccattctt cttctcttat tcaatgcatc acaaaacttt gcagcatctg 60
   aactgtatcc tgcattatgt acccttacta tgttagcacc attcaaaata cccgccgtca 120
   cagctgcaac agtagcaaca tctctctcaa ctggattttc acgattgcat atttcaccca 180
   aaaatctttt cctagagggt 200
<210> 565
   <211> 200
   <212> DNA
   <213> Digitaria sanguinalis
<400> 565
   agaagaagaa tggaagactg aaccagctga tcaaaaagat accgagctct gattttattc   60
   gagaaaatgg tgatgcagga tagttactct gctgctcaat gggattctca tattacatca  120
   tttgtggagt attgtttttg taataaataa accagggatg acgtttttcc tgtgtcatct  180
   cctatctagt ttctagatac                                              200
<210> 566
   <211> 200
   <212> DNA
   <213> Digitaria sanguinalis
<400> 566
   gtatctagaa actagatagg agatgacaca ggaaaaacgt catccctggt ttatttatta   60
   caaaaacaat actccacaaa tgatgtaata tgagaatccc attgagcagc agagtaacta  120
   tcctgcatca ccattttctc gaataaaatc agagctcggt atctttttga tcagctggtt  180
   cagtcttcca ttcttcttct                                              200
<210> 567
   <211> 200
   <212> DNA
   <213> Digitaria sanguinalis
<400> 567
   catctcctat ctagtttcta gatacttcaa tgaagcatgg ttcaactcag atcacttgga   60
   aagagtagtc aaggaataag gttacagaat cagttagtaa ttacagattc ttacctcttg  120
   tggtcctatt ttagatgatt ttccagaaca ttttctttcc aatttaataa tatcagaagg  180
   gcctaatttg tgtgtatctt                                              200
<210> 568
   <211> 200
   <212> DNA
   <213> Digitaria sanguinalis
<400> 568
   aagatacaca caaattaggc ccttctgata ttattaaatt ggaaagaaaa tgttctggaa 60
   aatcatctaa aataggacca caagaggtaa gaatctgtaa ttactaactg attctgtaac 120
   cttattcctt gactactctt tccaagtgat ctgagttgaa ccatgcttca ttgaagtatc 180
   tagaaactag ataggagatg 200
<210> 569
   <211> 200
   <212> DNA
   <213> Digitaria sanguinalis
<400> 569
   gctcgttcgt ttacgcgatg ttcgcttgag ggatgttcaa ctgaccaaga gattgtgatt 60
   gctatgggaa gcaacgtggg tgatggagtc agtacatttg acagggcatt acaaatgatg 120
   aaaagctcag gcgtgaacat cactaggcat gcctgtctat atgagactgc ccctgcttat 180
   gtgaccgatc agccacggtt 200
<210> 570
   <211> 200
   <212> DNA
   <213> Digitaria sanguinalis
<400> 570
   aaccgtggct gatcggtcac ataagcaggg gcagtctcat atagacaggc atgcctagtg 60
   atgttcacgc ctgagctttt catcatttgt aatgccctgt caaatgtact gactccatca 120
   cccacgttgc ttcccatagc aatcacaatc tcttggtcag ttgaacatcc ctcaagcgaa 180
   catcgcgtaa acgaacgagc 200
<210> 571
   <211> 200
   <212> DNA
   <213> Digitaria sanguinalis
<400> 571
   cttatgtgac cgatcagcca cggttcctga actctgccat tcgaggtacg actaggctgg 60
   gacctcacga gttgcttaag aaactaaagg aaattgagaa ggatatagga cgcactggtg 120
   gaataaggta tggcccaaga ccaatcgatc tagatatact tctgtacggc aattcccaga 180
   ttgatactga ggctctaatt 200
<210> 572
   <211> 200
   <212> DNA
   <213> Digitaria sanguinalis
<400> 572
   aattagagcc tcagtatcaa tctgggaatt gccgtacaga agtatatcta gatcgattgg 60
   tcttgggcca taccttattc caccagtgcg tcctatatcc ttctcaattt cctttagttt 120
   cttaagcaac tcgtgaggtc ccagcctagt cgtacctcga atggcagagt tcaggaaccg 180
   tggctgatcg gtcacataag 200
<210> 573
   <211> 200
   <212> DNA
   <213> Digitaria sanguinalis
<400> 573
   ccagattgat actgaggctc taattgtgcc acatgaacgc atccaggaga ggccatttgt 60
   tctagcacct cttgttgacc ttctaggtgc atctggcaat gatagtgtcg aaacaagctg 120
   gcactctctt tcaaagtgca gtggtggttt ctttgaatta tggaataaac ttgggggtga 180
   atctataatt ggaacagaaa 200
<210> 574
   <211> 200
   <212> DNA
   <213> Digitaria sanguinalis
<400> 574
   tttctgttcc aattatagat tcacccccaa gtttattcca taattcaaag aaaccaccac 60
   tgcactttga aagagagtgc cagcttgttt cgacactatc attgccagat gcacctagaa 120
   ggtcaacaag aggtgctaga acaaatggcc tctcctggat gcgttcatgt ggcacaatta 180
   gagcctcagt atcaatctgg 200
<210> 575
   <211> 200
   <212> DNA
   <213> Digitaria sanguinalis
<400> 575
   ggtgaatcta taattggaac agaaagtatt aaaagggtat tacctgttgg gaatcatttg 60
   tttgattggt gtgagagaac cctcgtcatg gggtccttta atctaacacc agacagcttt 120
   agtgatggag gtaagtttca acaagtggaa gctgctattt ctcaggctaa attattaatt 180
   tcagaaggtg cagatatcat 200
<210> 576
   <211> 200
   <212> DNA
   <213> Digitaria sanguinalis
<400> 576
   atgatatctg caccttctga aattaataat ttagcctgag aaatagcagc ttccacttgt   60
   tgaaacttac ctccatcact aaagctgtct ggtgttagat taaggacccc catgacgagg  120
   gttctctcac accaatcaaa caaatgattc ccaacaggta ataccctttt aatactttct  180
   gttccaatta tagattcacc                                              200
<210> 577
   <211> 200
   <212> DNA
   <213> Digitaria sanguinalis
<400> 577
   taatttcaga aggtgcagat atcatagata ttggtgctca atctaccagg ccttttgcaa   60
   agagattatc tccaaacgaa gaaattgaaa gattggttcc tgttctggat gtgattacga  120
   aaattcctga aatggaggga aagttgctct cagtggatac attctatgca gaagttgcat  180
   gtgaagctgt gaaaagagga                                              200
<210> 578
   <211> 200
   <212> DNA
   <213> Digitaria sanguinalis
<400> 578
   tcctcttttc acagcttcac atgcaacttc tgcatagaat gtatccactg agagcaactt   60
   tccctccatt tcaggaattt tcgtaatcac atccagaaca ggaaccaatc tttcaatttc  120
   ttcgtttgga gataatctct ttgcaaaagg cctggtagat tgagcaccaa tatctatgat  180
   atctgcacct tctgaaatta                                              200
<210> 579
   <211> 200
   <212> DNA
   <213> Digitaria sanguinalis
<400> 579
   tgcatgtgaa gctgtgaaaa gaggagttca catgatcaat gatgtatctg gtggacagct   60
   tgaccccaaa attcttaaag ttgctgctga actcaaagtt ccgtatgttg caatgcacat  120
   gaggggagat ccatcaacaa tgcaaagtga acaaaattta cagtatgacg atgtctgcaa  180
   ggaagttgct tcagagctat                                              200
<210> 580
   <211> 200
   <212> DNA
   <213> Digitaria sanguinalis
<400> 580
   atagctctga agcaacttcc ttgcagacat cgtcatactg taaattttgt tcactttgca    60
   ttgttgatgg atctcccctc atgtgcattg caacatacgg aactttgagt tcagcagcaa   120
   ctttaagaat tttggggtca agctgtccac cagatacatc attgatcatg tgaactcctc   180
   ttttcacagc ttcacatgca                                               200
<210> 581
   <211> 200
   <212> DNA
   <213> Digitaria sanguinalis
<400> 581
   tgcaaggaag ttgcttcaga gctatatgca caggtgagag aagcagagtt atctgggatt    60
   ccattgtgga ggatagttct agatccaggc attgggttct ccaagaaatc caaacataac   120
   cttgaagtaa ttatgggatt ggaatccatt aggagggaga tgagtaaaat gagtattggt   180
   gcttcacatg tgccaatatt                                               200
<210> 582
   <211> 200
   <212> DNA
   <213> Digitaria sanguinalis
<400> 582
   aatattggca catgtgaagc accaatactc attttactca tctccctcct aatggattcc    60
   aatcccataa ttacttcaag gttatgtttg gatttcttgg agaacccaat gcctggatct   120
   agaactatcc tccacaatgg aatcccagat aactctgctt ctctcacctg tgcatatagc   180
   tctgaagcaa cttccttgca                                               200
<210> 583
   <211> 200
   <212> DNA
   <213> Digitaria sanguinalis
<400> 583
   ttggtgcttc acatgtgcca atattactgg gaccctctag gaaaagattt ttgggtgaaa    60
   tatgcaatcg tgaaaatcca gttgagagag atgttgctac tgttgcagct gtgacggcgg   120
   gtattttgaa tggtgctaac atagtaaggg tacataatgc aggatacagt tcagatgctg   180
   caaagttttg tgatgcattg                                               200
<210> 584
   <211> 200
   <212> DNA
   <213> Digitaria sanguinalis
<400> 584
   caatgcatca caaaactttg cagcatctga actgtatcct gcattatgta cccttactat   60
   gttagcacca ttcaaaatac ccgccgtcac agctgcaaca gtagcaacat ctctctcaac  120
   tggattttca cgattgcata tttcacccaa aaatcttttc ctagagggtc ccagtaatat  180
   tggcacatgt gaagcaccaa                                              200
<210> 585
   <211> 200
   <212> DNA
   <213> Euphorbia heterophylla
<400> 585
   aaatataaat ataaatataa aaataaaaat aaattataat aatataaaat ataaaaatta   60
   atttaaacca tgttttaaaa atagtgttat gaaaaactcc gcttgggcgc cgtctagata  120
   gtttacttag gctgattttt aaaacaaaac cccccgctta acccgcctaa atcggtcaat  180
   tgtttatatc cttatgacta                                              200
<210> 586
   <211> 200
   <212> DNA
   <213> Euphorbia heterophylla
<400> 586
   tagtcataag gatataaaca attgaccgat ttaggcgggt taagcggggg gttttgtttt   60
   aaaaatcagc ctaagtaaac tatctagacg gcgcccaagc ggagtttttc ataacactat  120
   ttttaaaaca tggtttaaat taatttttat attttatatt attataattt atttttattt  180
   ttatatttat atttatattt                                              200
<210> 587
   <211> 200
   <212> DNA
   <213> Euphorbia heterophylla
<400> 587
   tcaattgttt atatccttat gactacttaa aatcgcagcg gtgttatggt gaattatcat 60
   gttttaatga gcttatgaaa tgcaataata aaaaaataac cattaataat ttgatataca 120
   tttatcggga aaaatgaaat aataaaaaat aaaaaaatat caaaaaaatc tataaaatat 180
   ttgtaccttt ttcaattagt 200
<210> 588
   <211> 200
   <212> DNA
   <213> Euphorbia heterophylla
<400> 588
   actaattgaa aaaggtacaa atattttata gatttttttg atattttttt attttttatt 60
   atttcatttt tcccgataaa tgtatatcaa attattaatg gttatttttt tattattgca 120
   tttcataagc tcattaaaac atgataattc accataacac cgctgcgatt ttaagtagtc 180
   ataaggatat aaacaattga 200
<210> 589
   <211> 200
   <212> DNA
   <213> Euphorbia heterophylla
<400> 589
   aatatttgta cctttttcaa ttagtaccca ttttgtatat taaaacaata aaaatgatcc 60
   agattaataa atagaaaaag tattaataac ccgaaaatat caaatatcaa aatataatga 120
   aacatgaaca cgaaaacaca ataaagaagt tggtatcaca aacacaaaga cacccttaaa 180
   gctcctatgt ttgcggagag 200
<210> 590
   <211> 200
   <212> DNA
   <213> Euphorbia heterophylla
<400> 590
   ctctccgcaa acataggagc tttaagggtg tctttgtgtt tgtgatacca acttctttat 60
   tgtgttttcg tgttcatgtt tcattatatt ttgatatttg atattttcgg gttattaata 120
   ctttttctat ttattaatct ggatcatttt tattgtttta atatacaaaa tgggtactaa 180
   ttgaaaaagg tacaaatatt 200
<210> 591
   <211> 200
   <212> DNA
   <213> Euphorbia heterophylla
<400> 591
   ttaaagctcc tatgtttgcg gagagttgta agtcgactaa tttcagattg aaaaagttaa 60
   aaatggccaa cttttttagc tatgaggtta gattagatac gagaatgcct ttgtgaaaag 120
   ctggtcacta tttcttcatt cgcgtggcaa ttaacccggc ataatcgata gccaattcac 180
   ggacaaggtc aaaggtatca 200
<210> 592
   <211> 200
   <212> DNA
   <213> Euphorbia heterophylla
<400> 592
   tgataccttt gaccttgtcc gtgaattggc tatcgattat gccgggttaa ttgccacgcg 60
   aatgaagaaa tagtgaccag cttttcacaa aggcattctc gtatctaatc taacctcata 120
   gctaaaaaag ttggccattt ttaacttttt caatctgaaa ttagtcgact tacaactctc 180
   cgcaaacata ggagctttaa 200
<210> 593
   <211> 200
   <212> DNA
   <213> Euphorbia heterophylla
<400> 593
   ttcacggaca aggtcaaagg tatcagcttt actcaaccct tcgctttgat actaaattgc 60
   tgattcttat tctgattatg ctttatttcg ttacttcatt ttattgtcta tgcacataag 120
   ctgttcgaca aaaagcctga atgaaactgt gagtgctggt acgcggttac aatactcctg 180
   attatgtcca atgattattt 200
<210> 594
   <211> 200
   <212> DNA
   <213> Euphorbia heterophylla
<400> 594
   aaataatcat tggacataat caggagtatt gtaaccgcgt accagcactc acagtttcat 60
   tcaggctttt tgtcgaacag cttatgtgca tagacaataa aatgaagtaa cgaaataaag 120
   cataatcaga ataagaatca gcaatttagt atcaaagcga agggttgagt aaagctgata 180
   cctttgacct tgtccgtgaa 200
<210> 595
   <211> 200
   <212> DNA
   <213> Euphorbia heterophylla
<400> 595
   tcctgattat gtccaatgat tatttgggta ctttaatacc ctaatttgtg aagttcacta   60
   gcgaaagggt agtttcttcc ccggttattt atcattttat gaatccccaa gtctttgaag  120
   tacagctgat taaaatgcaa gtactcataa gttgtcattg tatgtttgaa atccttacgt  180
   tgttggtatt ttacagaatt                                              200
<210> 596
   <211> 200
   <212> DNA
   <213> Euphorbia heterophylla
<400> 596
   aattctgtaa aataccaaca acgtaaggat ttcaaacata caatgacaac ttatgagtac   60
   ttgcatttta atcagctgta cttcaaagac ttggggattc ataaaatgat aaataaccgg  120
   ggaagaaact accctttcgc tagtgaactt cacaaattag ggtattaaag tacccaaata  180
   atcattggac ataatcagga                                              200
<210> 597
   <211> 200
   <212> DNA
   <213> Euphorbia heterophylla
<400> 597
   tacgttgttg gtattttaca gaatttgtac tgaagaagca taaattcata cctagttagg   60
   cttccttgtt cctaatatgc ttctcctcaa gcggctggtg tccactaaac aagggttcaa  120
   taatgccatc aaccgttttg gaggtaatat agttatgcct atgatgcttt aagtcgcttt  180
   tcatcgattt gttacattac                                              200
<210> 598
   <211> 200
   <212> DNA
   <213> Euphorbia heterophylla
<400> 598
   gtaatgtaac aaatcgatga aaagcgactt aaagcatcat aggcataact atattacctc 60
   caaaacggtt gatggcatta ttgaaccctt gtttagtgga caccagccgc ttgaggagaa 120
   gcatattagg aacaaggaag cctaactagg tatgaattta tgcttcttca gtacaaattc 180
   tgtaaaatac caacaacgta 200
<210> 599
   <211> 200
   <212> DNA
   <213> Euphorbia heterophylla
<400> 599
   gcttttcatc gatttgttac attaccaggc taagattctg caattgaagt tagaaatcga 60
   cgagtcttat cttgaaaagt atggattttg ttttgtcacc ctggggccag ttcaagattg 120
   ctttcccagg ttgctgtctc ggcaccacat cctaatccaa aaccctaagg tgatgggtta 180
   atgggtccct tcgcagttat 200
<210> 600
   <211> 200
   <212> DNA
   <213> Euphorbia heterophylla
<400> 600
   ataactgcga agggacccat taacccatca ccttagggtt ttggattagg atgtggtgcc 60
   gagacagcaa cctgggaaag caatcttgaa ctggccccag ggtgacaaaa caaaatccat 120
   acttttcaag ataagactcg tcgatttcta acttcaattg cagaatctta gcctggtaat 180
   gtaacaaatc gatgaaaagc 200
<210> 601
   <211> 200
   <212> DNA
   <213> Euphorbia heterophylla
<400> 601
   ggttaatggg tcccttcgca gttatatttc attcacttaa ctcattagct tccatgtgga 60
   taatcaactc cacacttgcc ccaaaactgt ataaacgtag attttgacct ttggaaatca 120
   ttagctggac aaatcctaac taattagtat caagagccaa aatcttttga ccaagaccaa 180
   atatgttgtt tcattgttgc 200
<210> 602
   <211> 200
   <212> DNA
   <213> Euphorbia heterophylla
<400> 602
   gcaacaatga aacaacatat ttggtcttgg tcaaaagatt ttggctcttg atactaatta 60
   gttaggattt gtccagctaa tgatttccaa aggtcaaaat ctacgtttat acagttttgg 120
   ggcaagtgtg gagttgatta tccacatgga agctaatgag ttaagtgaat gaaatataac 180
   tgcgaaggga cccattaacc 200
<210> 603
   <211> 200
   <212> DNA
   <213> Euphorbia heterophylla
<400> 603
   accaaatatg ttgtttcatt gttgcagcat tgtcatcatc ctttctccat accgaaccaa 60
   atagttcggt ggaagttcat tctcaagagc aagaagtagt aattgcatta ggaagcaatg 120
   tgggagatag agttcataat ttcaaccaag cattgcgatt aatgaaaagt tccggcatta 180
   acgtaactag acatggttgt 200
<210> 604
   <211> 200
   <212> DNA
   <213> Euphorbia heterophylla
<400> 604
   acaaccatgt ctagttacgt taatgccgga acttttcatt aatcgcaatg cttggttgaa 60
   attatgaact ctatctccca cattgcttcc taatgcaatt actacttctt gctcttgaga 120
   atgaacttcc accgaactat ttggttcggt atggagaaag gatgatgaca atgctgcaac 180
   aatgaaacaa catatttggt 200
<210> 605
   <211> 200
   <212> DNA
   <213> Euphorbia heterophylla
<400> 605
   cattaacgta actagacatg gttgtttgta ccagacagca cctgcttatg tcactgacca 60
   acctcacttt ctcaactcag cagttcgagc tttcacgaaa ctcggacccc acgagttatt 120
   aggagtttta aagcagatcg aaaaggactt gggccgtacc aaagggatta ggtacggacc 180
   aaggccaatc gatttggata 200
<210> 606
   <211> 200
   <212> DNA
   <213> Euphorbia heterophylla
<400> 606
   tatccaaatc gattggcctt ggtccgtacc taatcccttt ggtacggccc aagtcctttt 60
   cgatctgctt taaaactcct aataactcgt ggggtccgag tttcgtgaaa gctcgaactg 120
   ctgagttgag aaagtgaggt tggtcagtga cataagcagg tgctgtctgg tacaaacaac 180
   catgtctagt tacgttaatg 200
<210> 607
   <211> 200
   <212> DNA
   <213> Euphorbia heterophylla
<400> 607
   ggaccaaggc caatcgattt ggatatactt ttctatggaa agttccggat taattccgat 60
   acacttattg ttcctcatga gagaatatgg gagagaccct ttgtgatggc cccattggtg 120
   gatttactcg gctcggaaat cgagaatgac acggtcgcca gctggcattc cttgtccggc 180
   ggtctttttg aatcatggga 200
<210> 608
   <211> 200
   <212> DNA
   <213> Euphorbia heterophylla
<400> 608
   tcccatgatt caaaaagacc gccggacaag gaatgccagc tggcgaccgt gtcattctcg 60
   atttccgagc cgagtaaatc caccaatggg gccatcacaa agggtctctc ccatattctc 120
   tcatgaggaa caataagtgt atcggaatta atccggaact ttccatagaa aagtatatcc 180
   aaatcgattg gccttggtcc 200
<210> 609
   <211> 200
   <212> DNA
   <213> Euphorbia heterophylla
<400> 609
   ccggcggtct ttttgaatca tgggaaaagt tgggcggcga aagcctgatc gggaaggacg 60
   gaatgaaaag agttaccccg atcggaaacc atttatggga ttggtcggaa aagacttctg 120
   taatgggaat tatcaattta acccccgata gtttcagcga tgggggcaag ttgacaacta 180
   ttgattctat agtttctaag 200
<210> 610
   <211> 200
   <212> DNA
   <213> Euphorbia heterophylla
<400> 610
   cttagaaact atagaatcaa tagttgtcaa cttgccccca tcgctgaaac tatcgggggt 60
   taaattgata attcccatta cagaagtctt ttccgaccaa tcccataaat ggtttccgat 120
   cggggtaact cttttcattc cgtccttccc gatcaggctt tcgccgccca acttttccca 180
   tgattcaaaa agaccgccgg 200
<210> 611
   <211> 200
   <212> DNA
   <213> Euphorbia heterophylla
<400> 611
   aactattgat tctatagttt ctaaggttcg ctcgatgatt tccgaagggg cggatattgt 60
   cgattttggt gctcaatcga cacgccctat ggctaaaagg atatccccgc aagaggaaat 120
   ggataggcta atccctgtat tggaagcggt tgtgaaaata cctgagatga ccggaaagct 180
   catatcggtc gacacatttt 200
<210> 612
   <211> 200
   <212> DNA
   <213> Euphorbia heterophylla
<400> 612
   aaaatgtgtc gaccgatatg agctttccgg tcatctcagg tattttcaca accgcttcca 60
   atacagggat tagcctatcc atttcctctt gcggggatat ccttttagcc atagggcgtg 120
   tcgattgagc accaaaatcg acaatatccg ccccttcgga aatcatcgag cgaaccttag 180
   aaactataga atcaatagtt 200
<210> 613
   <211> 200
   <212> DNA
   <213> Euphorbia heterophylla
<400> 613
   aagctcatat cggtcgacac attttactcg gaagtcgcct tagaagcggt ccaaagagga 60
   gcaaatctcg taaatgacgt atctgggggg caattagatc caaaaatgac ggaaaatgtc 120
   gctaatctcg aggtaccata tatcttaatg cacatgaggg gggaccccac cacaatgcag 180
   aacactcaga atctaaaata 200
<210> 614
   <211> 200
   <212> DNA
   <213> Euphorbia heterophylla
<400> 614
   tattttagat tctgagtgtt ctgcattgtg gtggggtccc ccctcatgtg cattaagata 60
   tatggtacct cgagattagc gacattttcc gtcatttttg gatctaattg ccccccagat 120
   acgtcattta cgagatttgc tcctctttgg accgcttcta aggcgacttc cgagtaaaat 180
   gtgtcgaccg atatgagctt 200
<210> 615
   <211> 200
   <212> DNA
   <213> Euphorbia heterophylla
<400> 615
   tgcagaacac tcagaatcta aaatacgata atgtttgtaa agaggttgcc tttgagttgt 60
   tttcgagggt taaagaagcc gagatgtccg gaattccagc ctggcgaatg attattgacc 120
   ctggaattgg gttttcgaag aacacgaatc agaatctgga aatcctgatg ggaattccaa 180
   ggatccgggc cgagattggg 200
<210> 616
   <211> 200
   <212> DNA
   <213> Euphorbia heterophylla
<400> 616
   cccaatctcg gcccggatcc ttggaattcc catcaggatt tccagattct gattcgtgtt 60
   cttcgaaaac ccaattccag ggtcaataat cattcgccag gctggaattc cggacatctc 120
   ggcttcttta accctcgaaa acaactcaaa ggcaacctct ttacaaacat tatcgtattt 180
   tagattctga gtgttctgca 200
<210> 617
   <211> 200
   <212> DNA
   <213> Euphorbia heterophylla
<400> 617
   tccaaggatc cgggccgaga ttgggaggga aagcgtgggt atttctcgtg ggccgattct 60
   tattgggcct tcgagaaaga ggtttttggg cgaaatttgc gaacgcccta aagcaactga 120
   aagagacccg gcaactgttg cttctgttac tactgggatt ctgggagggg caaatattgt 180
   tagagttcat aatgttaggg 200
<210> 618
   <211> 200
   <212> DNA
   <213> Euphorbia heterophylla
<400> 618
   ccctaacatt atgaactcta acaatatttg cccctcccag aatcccagta gtaacagaag 60
   caacagttgc cgggtctctt tcagttgctt tagggcgttc gcaaatttcg cccaaaaacc 120
   tctttctcga aggcccaata agaatcggcc cacgagaaat acccacgctt tccctcccaa 180
   tctcggcccg gatccttgga 200
<210> 619
   <211> 200
   <212> DNA
   <213> Euphorbia heterophylla
<400> 619
   attgttagag ttcataatgt tagggataat gtggatgctg tgaagctatg cgactcgatg 60
   atgaaactaa agaggtctac ttaaaaagta tcgggtatag ttcgatttct ttctttccga 120
   atcttttgtt tgactttcag attgtctttg attggcaaaa cagtttcggc tgcggttact 180
   gttgttgttt gttgttgcag 200
<210> 620
   <211> 200
   <212> DNA
   <213> Euphorbia heterophylla
<400> 620
   ctgcaacaac aaacaacaac agtaaccgca gccgaaactg ttttgccaat caaagacaat 60
   ctgaaagtca aacaaaagat tcggaaagaa agaaatcgaa ctatacccga tactttttaa 120
   gtagacctct ttagtttcat catcgagtcg catagcttca cagcatccac attatcccta 180
   acattatgaa ctctaacaat 200
<210> 621
   <211> 200
   <212> DNA
   <213> Euphorbia heterophylla
<400> 621
   ttactgttgt tgtttgttgt tgcagtttag tgttgttgtt tctaattgtg attttaagtt   60
   attgttgtta gctgatttgt acttattgtt tggtgaaaat ataattcacg gttgcggttg  120
   gaggtataaa ttactaaaat agacatattt taaataaatt ataagttata ttgaagggta  180
   tttaggataa ataaaaatcc                                               200
<210> 622
   <211> 200
   <212> DNA
   <213> Euphorbia heterophylla
<400> 622
   ggatttttat ttatcctaaa tacccttcaa tataacttat aatttattta aaatatgtct   60
   attttagtaa tttatacctc caaccgcaac cgtgaattat attttcacca aacaataagt  120
   acaaatcagc taacaacaat aacttaaaat cacaattaga aacaacaaca ctaaactgca  180
   acaacaaaca acaacagtaa                                               200
<210> 623
   <211> 200
   <212> DNA
   <213> Euphorbia heterophylla
<400> 623
   gggtatttag gataaataaa aatccaaacc acaaaaaaaa acccgtaaaa ctattgattt   60
   ggtgaggttt ggaaaaggag ataaatccct tataaaaaaa acaacttcat atatgttttg  120
   aaacttaacc aaacacttgt atacctcctt ttccttttca aacctcaaaa gaccttttta   180
   tagtaagctc ttttgaagtc                                               200
<210> 624
   <211> 200
   <212> DNA
   <213> Euphorbia heterophylla
<400> 624
   gacttcaaaa gagcttacta taaaaaggtc ttttgaggtt tgaaaaggaa aaggaggtat   60
   acaagtgttt ggttaagttt caaaacatat atgaagttgt tttttttata agggatttat   120
   ctccttttcc aaacctcacc aaatcaatag ttttacgggt ttttttttgt ggtttggatt  180
   tttatttatc ctaaataccc                                               200
<210> 625
   <211> 200
   <212> DNA
   <213> Euphorbia heterophylla
<400> 625
   ttttatagta agctcttttg aagtctatac gctattgttt ccgaatcatt ttggactttt   60
   catcgaaatc aacaatactg ctagctagtc atatttcaag atatttggga tgtaaagatc  120
   aaggtagttg ttgttgtatt ctgttctcat cagcaaaatt tgttataact ccaccaagat  180
   ttaatttttt ccaatctgaa                                              200
<210> 626
   <211> 200
   <212> DNA
   <213> Euphorbia heterophylla
<400> 626
   ttcagattgg aaaaaattaa atcttggtgg agttataaca aattttgctg atgagaacag   60
   aatacaacaa caactacctt gatctttaca tcccaaatat cttgaaatat gactagctag  120
   cagtattgtt gatttcgatg aaaagtccaa aatgattcgg aaacaatagc gtatagactt  180
   caaaagagct tactataaaa                                              200
<210> 627
   <211> 200
   <212> DNA
   <213> Euphorbia heterophylla
<400> 627
   aagatttaat tttttccaat ctgaaaatgt cttatgttat agtaagaagt gagatgagtt   60
   tatgtctttg tgaaaagatt ttgcccaatt aatgcaaaaa ttttgtgaca agacaagagg  120
   ggtgaaagag acaagcaaca agagaaagca gtgaaagggg caaatgttag gaaactgtta  180
   gctagaaaag ggtagagggt                                              200
<210> 628
   <211> 200
   <212> DNA
   <213> Euphorbia heterophylla
<400> 628
   accctctacc cttttctagc taacagtttc ctaacatttg cccctttcac tgctttctct   60
   tgttgcttgt ctctttcacc cctcttgtct tgtcacaaaa tttttgcatt aattgggcaa  120
   aatcttttca caaagacata aactcatctc acttcttact ataacataag acattttcag  180
   attggaaaaa attaaatctt                                              200
<210> 629
   <211> 200
   <212> DNA
   <213> Euphorbia heterophylla
<400> 629
   tgttagctag aaaagggtag agggtggcct actttaccaa tttgagcatt aaaaaagcac 60
   atggctatgt attttagacc aaagtcccta ttgttggcag atgaactaag caaccatatg 120
   gttcattaac taactaacta gttattgcaa atttgactgt tccaattcat caaaggactc 180
   tccccataga ccattcaaca 200
<210> 630
   <211> 200
   <212> DNA
   <213> Euphorbia heterophylla
<400> 630
   tgttgaatgg tctatgggga gagtcctttg atgaattgga acagtcaaat ttgcaataac 60
   tagttagtta gttaatgaac catatggttg cttagttcat ctgccaacaa tagggacttt 120
   ggtctaaaat acatagccat gtgctttttt aatgctcaaa ttggtaaagt aggccaccct 180
   ctaccctttt ctagctaaca 200
<210> 631
   <211> 200
   <212> DNA
   <213> Euphorbia heterophylla
<400> 631
   gactctcccc atagaccatt caacacttgg gttctagccc tccctctcac cctcagacaa 60
   cccttgtgaa cctcattttc atcattggtc cccttttttg ttaggactac acttgtctct 120
   ataaaatctc ttctagccat gtgttatgtg tagacttttt cggtttgagg attgggtcgg 180
   gttcgggctg ggtctcgtcg 200
<210> 632
   <211> 200
   <212> DNA
   <213> Euphorbia heterophylla
<400> 632
   cgacgagacc cagcccgaac ccgacccaat cctcaaaccg aaaaagtcta cacataacac 60
   atggctagaa gagattttat agagacaagt gtagtcctaa caaaaaaggg gaccaatgat 120
   gaaaatgagg ttcacaaggg ttgtctgagg gtgagaggga gggctagaac ccaagtgttg 180
   aatggtctat ggggagagtc 200
<210> 633
   <211> 200
   <212> DNA
   <213> Euphorbia heterophylla
<400> 633
   gtcgggttcg ggctgggtct cgtcggactt tttactgcaa acatcaaact agggttctaa 60
   acccatactt cagtactctg gttcggattg aacttgcgag atcattctaa attcaattta 120
   cggctcatag attatatttg agagttttga gttttgggtg gattagattg aaaacgacta 180
   ttttttgttg tataaatata 200
<210> 634
   <211> 200
   <212> DNA
   <213> Euphorbia heterophylla
<400> 634
   tatatttata caacaaaaaa tagtcgtttt caatctaatc cacccaaaac tcaaaactct 60
   caaatataat ctatgagccg taaattgaat ttagaatgat ctcgcaagtt caatccgaac 120
   cagagtactg aagtatgggt ttagaaccct agtttgatgt ttgcagtaaa aagtccgacg 180
   agacccagcc cgaacccgac 200
<210> 635
   <211> 200
   <212> DNA
   <213> Euphorbia heterophylla
<400> 635
   gcagcattgt catcatcctt tctccatacc gaaccaaata gttcggtgga agttcattct 60
   caagagcaag aagtagtaat tgcattagga agcaatgtgg gagatagagt tcataatttc 120
   aaccaagcat tgcaattaat gaaaagttcc ggcattaaca taactagaca tggttgtttg 180
   taccagacag cacctgctta 200
<210> 636
   <211> 200
   <212> DNA
   <213> Euphorbia heterophylla
<400> 636
   taagcaggtg ctgtctggta caaacaacca tgtctagtta tgttaatgcc ggaacttttc 60
   attaattgca atgcttggtt gaaattatga actctatctc ccacattgct tcctaatgca 120
   attactactt cttgctcttg agaatgaact tccaccgaac tatttggttc ggtatggaga 180
   aaggatgatg acaatgctgc 200
<210> 637
   <211> 200
   <212> DNA
   <213> Euphorbia heterophylla
<400> 637
   gtttgtacca gacagcacct gcttatgtca ctgaccaacc tcactttctc aactcagcag 60
   ttcgagcttt cacgaaactc ggaccccacg agttattagg agttttaaag cagatcgaaa 120
   aggacttggg ccgtaccaaa gggattaggt acggaccaag gccaatcgat ttggatatac 180
   ttttctatgg aaagttccgg 200
<210> 638
   <211> 200
   <212> DNA
   <213> Euphorbia heterophylla
<400> 638
   ccggaacttt ccatagaaaa gtatatccaa atcgattggc cttggtccgt acctaatccc 60
   tttggtacgg cccaagtcct tttcgatctg ctttaaaact cctaataact cgtggggtcc 120
   gagtttcgtg aaagctcgaa ctgctgagtt gagaaagtga ggttggtcag tgacataagc 180
   aggtgctgtc tggtacaaac 200
<210> 639
   <211> 200
   <212> DNA
   <213> Euphorbia heterophylla
<400> 639
   tatacttttc tatggaaagt tccggattaa ttccgataca cttattgttc ctcatgagag 60
   aatatgggag agaccctttg tgatggcccc attggtggat ttactcggct cggaaatcga 120
   gaatgacacg gtcgccagct ggcattcctt gtccggcggt ctttttgaat catgggaaaa 180
   gttgggcggc gaaagcctga 200
<210> 640
   <211> 200
   <212> DNA
   <213> Euphorbia heterophylla
<400> 640
   tcaggctttc gccgcccaac ttttcccatg attcaaaaag accgccggac aaggaatgcc 60
   agctggcgac cgtgtcattc tcgatttccg agccgagtaa atccaccaat ggggccatca 120
   caaagggtct ctcccatatt ctctcatgag gaacaataag tgtatcggaa ttaatccgga 180
   actttccata gaaaagtata 200
<210> 641
   <211> 200
   <212> DNA
   <213> Euphorbia heterophylla
<400> 641
   gaaaagttgg gcggcgaaag cctgatcggg aaggacggaa tgaaaagagt taccccgatc 60
   ggaaaccatt tatgggattg gtcggaaaag acttctgtaa tgggaattat caatttaacc 120
   cccgatagtt tcagcgatgg gggcaagttg acaactattg attctatagt ttctaaggtt 180
   cgctcgatga tttccgaagg 200
<210> 642
   <211> 200
   <212> DNA
   <213> Euphorbia heterophylla
<400> 642
   ccttcggaaa tcatcgagcg aaccttagaa actatagaat caatagttgt caacttgccc 60
   ccatcgctga aactatcggg ggttaaattg ataattccca ttacagaagt cttttccgac 120
   caatcccata aatggtttcc gatcggggta actcttttca ttccgtcctt cccgatcagg 180
   ctttcgccgc ccaacttttc 200
<210> 643
   <211> 200
   <212> DNA
   <213> Euphorbia heterophylla
<400> 643
   aggttcgctc gatgatttcc gaaggggcgg atattgtcga ttttggtgct caatcgacac 60
   gccctatggc taaaaggata tccccgcaag aggaaatgga taggctaatc cctgtattgg 120
   aagcggttgt gaaaataccc gagatgaccg aaaagctcat atcggtcgac acattttact 180
   cggaagtcgc cttagaagcg 200
<210> 644
   <211> 200
   <212> DNA
   <213> Euphorbia heterophylla
<400> 644
   cgcttctaag gcgacttccg agtaaaatgt gtcgaccgat atgagctttt cggtcatctc   60
   gggtattttc acaaccgctt ccaatacagg gattagccta tccatttcct cttgcgggga  120
   tatcctttta gccatagggc gtgtcgattg agcaccaaaa tcgacaatat ccgccccttc  180
   ggaaatcatc gagcgaacct                                              200
<210> 645
   <211> 200
   <212> DNA
   <213> Euphorbia heterophylla
<400> 645
   ttactcggaa gtcgccttag aagcggtcca aagaggagca aatctcgtaa atgacgtatc   60
   tggggggcaa ttagatccaa aaatgacgga aaatgtcgct aatctcgagg taccatatat  120
   cttaatgcac atgagggggg accccaccac aatgcagaac actcagaatc taaaatacga  180
   taatgtttgt aaagaggttg                                              200
<210> 646
   <211> 200
   <212> DNA
   <213> Euphorbia heterophylla
<400> 646
   caacctcttt acaaacatta tcgtatttta gattctgagt gttctgcatt gtggtggggt   60
   cccccctcat gtgcattaag atatatggta cctcgagatt agcgacattt tccgtcattt  120
   ttggatctaa ttgcccccca gatacgtcat ttacgagatt tgctcctctt tggaccgctt  180
   ctaaggcgac ttccgagtaa                                              200
<210> 647
   <211> 200
   <212> DNA
   <213> Euphorbia heterophylla
<400> 647
   tacgataatg tttgtaaaga ggttgccttt gagttgtttt cgagggttaa agaagccgag   60
   atgtccggaa ttccagcctg gcgaatgttt attgaccctg gaattgggtt ttcgaagaac  120
   acgaatcaga atctggaaat cctgatggga attccaagga tccgggccga gattgggagg  180
   aaaagcgtgg gtatttctcg                                              200
<210> 648
   <211> 200
   <212> DNA
   <213> Euphorbia heterophylla
<400> 648
   cgagaaatac ccacgctttt cctcccaatc tcggcccgga tccttggaat tcccatcagg   60
   atttccagat tctgattcgt gttcttcgaa aacccaattc cagggtcaat aaacattcgc  120
   caggctggaa ttccggacat ctcggcttct ttaaccctcg aaaacaactc aaaggcaacc  180
   tctttacaaa cattatcgta                                              200
<210> 649
   <211> 200
   <212> DNA
   <213> Euphorbia heterophylla
<400> 649
   ggaggaaaag cgtgggtatt tctcgtgggc cgattcttat tgggccttcg agaaagaggt   60
   ttttgggcga aatttgcgaa cgccctgaag caactgaaag agacccggca actgttgctt  120
   ctgttactac tgggattctg ggaggggcaa atattgttag agttcataat gttagggata  180
   atgtggatgc tgtgaagcta                                              200
<210> 650
   <211> 200
   <212> DNA
   <213> Euphorbia heterophylla
<400> 650
   tagcttcaca gcatccacat tatccctaac attatgaact ctaacaatat ttgcccctcc   60
   cagaatccca gtagtaacag aagcaacagt tgccgggtct ctttcagttg cttcagggcg  120
   ttcgcaaatt tcgcccaaaa acctctttct cgaaggccca ataagaatcg gcccacgaga  180
   aatacccacg cttttcctcc                                              200
<210> 651
   <211> 200
   <212> DNA
   <213> Euphorbia heterophylla
<400> 651
   gcagcattgt catcatcctt tctccatacc gaaccaaata gttcggtgga agttcattct   60
   caagagcaag aagtagtaat tgcattagga agcaatgtgg gagatagagt tcataatttc  120
   aaccaagcat tgcgattaat gaaaagttcc ggcattaacg taactagaca tggttgtttg  180
   taccagacag cacctgctta                                              200
<210> 652
   <211> 200
   <212> DNA
   <213> Euphorbia heterophylla
<400> 652
   taagcaggtg ctgtctggta caaacaacca tgtctagtta cgttaatgcc ggaacttttc   60
   attaatcgca atgcttggtt gaaattatga actctatctc ccacattgct tcctaatgca  120
   attactactt cttgctcttg agaatgaact tccaccgaac tatttggttc ggtatggaga  180
   aaggatgatg acaatgctgc                                              200
<210> 653
   <211> 200
   <212> DNA
   <213> Euphorbia heterophylla
<400> 653
   gtttgtacca gacagcacct gcttatgtca ctgaccaacc tcactttctc aactcagcag   60
   ttcgagcttt cacgaaactc ggaccccacg agttattagg agttttaaag cagatcgaaa  120
   aggacttggg ccgtaccaaa gggattaggt acggaccaag gccaatcgat ttggatatac  180
   ttttctatgg aaagttccgg                                              200
<210> 654
   <211> 200
   <212> DNA
   <213> Euphorbia heterophylla
<400> 654
   ccggaacttt ccatagaaaa gtatatccaa atcgattggc cttggtccgt acctaatccc   60
   tttggtacgg cccaagtcct tttcgatctg ctttaaaact cctaataact cgtggggtcc  120
   gagtttcgtg aaagctcgaa ctgctgagtt gagaaagtga ggttggtcag tgacataagc  180
   aggtgctgtc tggtacaaac                                              200
<210> 655
   <211> 200
   <212> DNA
   <213> Euphorbia heterophylla
<400> 655
   tatacttttc tatggaaagt tccggattaa ttccgataca cttattgttc ctcatgagag   60
   aatatgggag agaccctttg tgatggcccc attggtggat ttactcggct cggaaatcga  120
   gaatgacacg gtcgccagct ggcattcctt gtccggcggt ctttttgaat catgggaaaa  180
   gttgggcggc gaaagcctga                                                200
<210> 656
   <211> 200
   <212> DNA
   <213> Euphorbia heterophylla
<400> 656
   tcaggctttc gccgcccaac ttttcccatg attcaaaaag accgccggac aaggaatgcc   60
   agctggcgac cgtgtcattc tcgatttccg agccgagtaa atccaccaat ggggccatca  120
   caaagggtct ctcccatatt ctctcatgag gaacaataag tgtatcggaa ttaatccgga  180
   actttccata gaaaagtata                                                200
<210> 657
   <211> 200
   <212> DNA
   <213> Euphorbia heterophylla
<400> 657
   gaaaagttgg gcggcgaaag cctgatcggg aaggacggaa tgaaaagagt taccccgatc   60
   ggaaaccatt tatgggattg gtcggaaaag acttctgtaa tgggaattat caatttaacc  120
   cccgatagtt tcagcgatgg gggcaagttg acaactattg attctatagt ttctaaggtt  180
   cgctcgatga tttccgaagg                                                200
<210> 658
   <211> 200
   <212> DNA
   <213> Euphorbia heterophylla
<400> 658
   ccttcggaaa tcatcgagcg aaccttagaa actatagaat caatagttgt caacttgccc   60
   ccatcgctga aactatcggg ggttaaattg ataattccca ttacagaagt cttttccgac  120
   caatcccata aatggtttcc gatcggggta actcttttca ttccgtcctt cccgatcagg  180
   ctttcgccgc ccaacttttc                                                200
<210> 659
   <211> 200
   <212> DNA
   <213> Euphorbia heterophylla
<400> 659
   aggttcgctc gatgatttcc gaaggggcgg atattgtcga ttttggtgct caatcgacac    60
   gccctatggc taaaaggata tccccgcaag aggaaatgga taggctaatc cctgtattgg   120
   aagcggttgt gaaaatacct gagatgaccg gaaagctcat atcggtcgac acattttact   180
   cggaagtcgc cttagaagcg                                                200
<210> 660
   <211> 200
   <212> DNA
   <213> Euphorbia heterophylla
<400> 660
   cgcttctaag gcgacttccg agtaaaatgt gtcgaccgat atgagctttc cggtcatctc    60
   aggtattttc acaaccgctt ccaatacagg gattagccta tccatttcct cttgcgggga   120
   tatcctttta gccatagggc gtgtcgattg agcaccaaaa tcgacaatat ccgccccttc   180
   ggaaatcatc gagcgaacct                                                200
<210> 661
   <211> 200
   <212> DNA
   <213> Euphorbia heterophylla
<400> 661
   ttactcggaa gtcgccttag aagcggtcca aagaggagca aatctcgtaa atgacgtatc    60
   tggggggcaa ttagatccaa aaatgacgga aaatgtcgct aatctcgagg taccatatat   120
   cttaatgcac atgagggggg accccaccac aatgcagaac actcagaatc taaaatacga   180
   taatgtttgt aaagaggttg                                                200
<210> 662
   <211> 200
   <212> DNA
   <213> Euphorbia heterophylla
<400> 662
   caacctcttt acaaacatta tcgtatttta gattctgagt gttctgcatt gtggtggggt 60
   cccccctcat gtgcattaag atatatggta cctcgagatt agcgacattt tccgtcattt 120
   ttggatctaa ttgcccccca gatacgtcat ttacgagatt tgctcctctt tggaccgctt 180
   ctaaggcgac ttccgagtaa 200
<210> 663
   <211> 200
   <212> DNA
   <213> Euphorbia heterophylla
<400> 663
   tacgataatg tttgtaaaga ggttgccttt gagttgtttt cgagggttaa agaagccgag 60
   atgtccggaa ttccagcctg gcgaatgatt attgaccctg gaattgggtt ttcgaagaac 120
   acgaatcaga atctggaaat cctgatggga attccaagga tccgggccga gattgggagg 180
   gaaagcgtgg gtatttctcg 200
<210> 664
   <211> 200
   <212> DNA
   <213> Euphorbia heterophylla
<400> 664
   cgagaaatac ccacgctttc cctcccaatc tcggcccgga tccttggaat tcccatcagg 60
   atttccagat tctgattcgt gttcttcgaa aacccaattc cagggtcaat aatcattcgc 120
   caggctggaa ttccggacat ctcggcttct ttaaccctcg aaaacaactc aaaggcaacc 180
   tctttacaaa cattatcgta 200
<210> 665
   <211> 200
   <212> DNA
   <213> Euphorbia heterophylla
<400> 665
   ggagggaaag cgtgggtatt tctcgtgggc cgattcttat tgggccttcg agaaagaggt 60
   ttttgggcga aatttgcgaa cgccctaaag caactgaaag agacccggca actgttgctt 120
   ctgttactac tgggattctg ggaggggcaa atattgttag agttcataat gttagggata 180
   atgtggatgc tgtgaagcta 200
<210> 666
   <211> 200
   <212> DNA
   <213> Euphorbia heterophylla
<400> 666
   tagcttcaca gcatccacat tatccctaac attatgaact ctaacaatat ttgcccctcc 60
   cagaatccca gtagtaacag aagcaacagt tgccgggtct ctttcagttg ctttagggcg 120
   ttcgcaaatt tcgcccaaaa acctctttct cgaaggccca ataagaatcg gcccacgaga 180
   aatacccacg ctttccctcc 200
<210> 667
   <211> 200
   <212> DNA
   <213> Euphorbia heterophylla
<400> 667
   gttatgatga aagtataagt tgctaatcaa gaagaaagag attttaatgt aattatttaa 60
   ataataaatt cctaccattt taattgtgtg acaattggtt ataatgattc cgttgcatcg 120
   acaactaaaa ctagatagaa ggatcaaaat ataaaatttt aacataatta atacatataa 180
   tatataaatg tataatagat 200
<210> 668
   <211> 200
   <212> DNA
   <213> Euphorbia heterophylla
<400> 668
   atctattata catttatata ttatatgtat taattatgtt aaaattttat attttgatcc 60
   ttctatctag ttttagttgt cgatgcaacg gaatcattat aaccaattgt cacacaatta 120
   aaatggtagg aatttattat ttaaataatt acattaaaat ctctttcttc ttgattagca 180
   acttatactt tcatcataac 200
<210> 669
   <211> 200
   <212> DNA
   <213> Euphorbia heterophylla
<400> 669
   tataatatat aaatgtataa tagatacaat tattattcct cgaattaaaa ttggctaatt 60
   atataataat ataatccaac ctataaaaac ttttgagctt ttaagcttga gatgagcttt 120
   gtaaaaatga ttgtacaagc ccgaccatga aagagagcca agtagggtca aatgcatacc 180
   ctggcttgaa tataaattca 200
<210> 670
   <211> 200
   <212> DNA
   <213> Euphorbia heterophylla
<400> 670
   tgaatttata ttcaagccag ggtatgcatt tgaccctact tggctctctt tcatggtcgg   60
   gcttgtacaa tcatttttac aaagctcatc tcaagcttaa aagctcaaaa gtttttatag  120
   gttggattat attattatat aattagccaa ttttaattcg aggaataata attgtatcta  180
   ttatacattt atatattata                                              200
<210> 671
   <211> 200
   <212> DNA
   <213> Euphorbia heterophylla
<400> 671
   tctggggagt gatggttgga atagatgctt attcctggca gagatcgaaa tgctccattt   60
   tagtttcggg ttttgttaaa atggggattt gttaaattgt ttcaatggat aattcactgt  120
   tgtattagac ttgatattgt tagcagacac tgatttttat gtattcgtga actgaaaagg  180
   ttgattttct tccatgtttt                                              200
<210> 672
   <211> 200
   <212> DNA
   <213> Euphorbia heterophylla
<400> 672
   aaaacatgga agaaaatcaa ccttttcagt tcacgaatac ataaaaatca gtgtctgcta   60
   acaatatcaa gtctaataca acagtgaatt atccattgaa acaatttaac aaatccccat  120
   tttaacaaaa cccgaaacta aaatggagca tttcgatctc tgccaggaat aagcatctat  180
   tccaaccatc actccccaga                                              200
<210> 673
   <211> 200
   <212> DNA
   <213> Euphorbia heterophylla
<400> 673
   aaaggttgat tttcttccat gttttgtctg atttatgatg gaacttcatt tcttagtggt 60
   taatgttggt atttccttct gttgtttgat ttgtatctgt aattggtacc atgttatatg 120
   caatctttta aaaaatgttt taaaaagcgc tatacgtggc agctgagact gttgagactt 180
   gaaaacgcac gggcatgtgc 200
<210> 674
   <211> 200
   <212> DNA
   <213> Euphorbia heterophylla
<400> 674
   gcacatgccc gtgcgttttc aagtctcaac agtctcagct gccacgtata gcgcttttta 60
   aaacattttt taaaagattg catataacat ggtaccaatt acagatacaa atcaaacaac 120
   agaaggaaat accaacatta accactaaga aatgaagttc catcataaat cagacaaaac 180
   atggaagaaa atcaaccttt 200
<210> 675
   <211> 200
   <212> DNA
   <213> Euphorbia heterophylla
<400> 675
   ttaaagcaga tcgaaaagga cttgggccgt accaaaggga ttaggtacgg accaaggcca 60
   atcgatttgg atatactttt tctatggaaa gttccggatt aattccgata cacttattgt 120
   tcctcatgag agaatatggg agagaccctt tgtgatggcc ccattggtgg atttactcgg 180
   ctcggaaatc gagaatgaca 200
<210> 676
   <211> 200
   <212> DNA
   <213> Euphorbia heterophylla
<400> 676
   tgtcattctc gatttccgag ccgagtaaat ccaccaatgg ggccatcaca aagggtctct 60
   cccatattct ctcatgagga acaataagtg tatcggaatt aatccggaac tttccataga 120
   aaaagtatat ccaaatcgat tggccttggt ccgtacctaa tccctttggt acggcccaag 180
   tccttttcga tctgctttaa 200
<210> 677
   <211> 200
   <212> DNA
   <213> Euphorbia heterophylla
<400> 677
   ctcggctcgg aaatcgagaa tgacacggtc gccagctggc attccttgtc cggcggtctt    60
   tttgaatcat gggaaaagtt gggcggcgaa agcctgatcg ggaaggacgg aatgaaaaga   120
   gttaccccga tcggaaacca tttatgggat tggtcggaaa agacttctgt aatgggaatt   180
   atcaatttaa cccccgatag                                               200
<210> 678
   <211> 200
   <212> DNA
   <213> Euphorbia heterophylla
<400> 678
   ctatcggggg ttaaattgat aattcccatt acagaagtct tttccgacca atcccataaa    60
   tggtttccga tcggggtaac tcttttcatt ccgtccttcc cgatcaggct ttcgccgccc   120
   aacttttccc atgattcaaa aagaccgccg gacaaggaat gccagctggc gaccgtgtca   180
   ttctcgattt ccgagccgag                                               200
<210> 679
   <211> 200
   <212> DNA
   <213> Euphorbia heterophylla
<400> 679
   gaattatcaa tttaaccccc gatagtttca gcgatggggg caagttgaca actattgatt    60
   ctatagtttc taaggttcgc tcgatgattt ccgaaggggc ggatattgtc gattttggtg   120
   ctcaatcgac acgccctatg gctaaaagga tatccccgca agaggaaatg gataggctaa   180
   tccctgtatt ggaagcggtt                                               200
<210> 680
   <211> 200
   <212> DNA
   <213> Euphorbia heterophylla
<400> 680
   aaccgcttcc aatacaggga ttagcctatc catttcctct tgcggggata tccttttagc    60
   catagggcgt gtcgattgag caccaaaatc gacaatatcc gccccttcgg aaatcatcga   120
   gcgaacctta gaaactatag aatcaatagt tgtcaacttg cccccatcgc tgaaactatc   180
   gggggttaaa ttgataattc                                               200
<210> 681
   <211> 200
   <212> DNA
   <213> Euphorbia heterophylla
<400> 681
   tttgtatatt aaaacaataa aaatgatcca gattaataaa tagaaaaagt attaataacc   60
   cgaaaatatc aaatatcaaa atataatgaa acatgaacac gaaatcacaa taaagaagtt  120
   ggtatcacaa acacaaagac acccttaaag ctcctatgtt tgtggagagt tgtaagtcga  180
   ctaatttcag attgcaaaag                                              200
<210> 682
   <211> 200
   <212> DNA
   <213> Euphorbia heterophylla
<400> 682
   cttttgcaat ctgaaattag tcgacttaca actctccaca aacataggag ctttaagggt   60
   gtctttgtgt ttgtgatacc aacttcttta ttgtgatttc gtgttcatgt ttcattatat  120
   tttgatattt gatattttcg ggttattaat actttttcta tttattaatc tggatcattt  180
   ttattgtttt aatatacaaa                                              200
<210> 683
   <211> 200
   <212> DNA
   <213> Euphorbia heterophylla
<400> 683
   gtcgactaat ttcagattgc aaaagttaaa aatggccaac ttttttagct atgaggttag   60
   attagatacg agaatgcctt ggtgaaaagc tggtcactgt ttcttcattc gcgtggcaat  120
   taacccggca taatcgatac ccaattcacg gacaaggtca aaggtatcag ctttactcaa  180
   cccttcgctt tgatactaaa                                              200
<210> 684
   <211> 200
   <212> DNA
   <213> Euphorbia heterophylla
<400> 684
   tttagtatca aagcgaaggg ttgagtaaag ctgatacctt tgaccttgtc cgtgaattgg   60
   gtatcgatta tgccgggtta attgccacgc gaatgaagaa acagtgacca gcttttcacc  120
   aaggcattct cgtatctaat ctaacctcat agctaaaaaa gttggccatt tttaactttt  180
   gcaatctgaa attagtcgac                                              200
<210> 685
   <211> 200
   <212> DNA
   <213> Euphorbia heterophylla
<400> 685
   ctcaaccctt cgctttgata ctaaattgct gattcttatt ctgattatgc tttatttcgt   60
   tacttcattt tattgtctat gcacataagc tgttcgacaa aaagcctgaa tgaaactgtg  120
   agtgctggta cgcggttaca atactcctga ttatgtccaa tgattatttg ggtactttaa  180
   taccctaatt tgtgaagttc                                              200
<210> 686
   <211> 200
   <212> DNA
   <213> Euphorbia heterophylla
<400> 686
   gaacttcaca aattagggta ttaaagtacc caaataatca ttggacataa tcaggagtat   60
   tgtaaccgcg taccagcact cacagtttca ttcaggcttt ttgtcgaaca gcttatgtgc  120
   atagacaata aaatgaagta acgaaataaa gcataatcag aataagaatc agcaatttag  180
   tatcaaagcg aagggttgag                                              200
<210> 687
   <211> 200
   <212> DNA
   <213> Euphorbia heterophylla
<400> 687
   tttaataccc taatttgtga agttcactag cgaaagggta gtttcttccc cggttattta   60
   tcattttatg aatccccaag tctttgaagt acagctgatt aaaatgcaag tactcataag  120
   ttgtcattgt atgtttgaag tccttacatt tttggtattt tacagaattt gtactgaaga  180
   agcataaatt catacctagt                                              200
<210> 688
   <211> 200
   <212> DNA
   <213> Euphorbia heterophylla
<400> 688
   actaggtatg aatttatgct tcttcagtac aaattctgta aaataccaaa aatgtaagga   60
   cttcaaacat acaatgacaa cttatgagta cttgcatttt aatcagctgt acttcaaaga  120
   cttggggatt cataaaatga taaataaccg gggaagaaac taccctttcg ctagtgaact  180
   tcacaaatta gggtattaaa                                              200
<210> 689
   <211> 200
   <212> DNA
   <213> Euphorbia heterophylla
<400> 689
   gaagaagcat aaattcatac ctagttaggc ttccttgttc ctaatatgct tctcctcaag 60
   cggctggtgt ccactaaaca agggttcaat aatgccatca accgttttgg aggtaatata 120
   gttatgccta tgatgcttta agtcgctttt catcgatttg ttacattacc aggctaagat 180
   tctgcaattg aagttagaaa 200
<210> 690
   <211> 200
   <212> DNA
   <213> Euphorbia heterophylla
<400> 690
   tttctaactt caattgcaga atcttagcct ggtaatgtaa caaatcgatg aaaagcgact 60
   taaagcatca taggcataac tatattacct ccaaaacggt tgatggcatt attgaaccct 120
   tgtttagtgg acaccagccg cttgaggaga agcatattag gaacaaggaa gcctaactag 180
   gtatgaattt atgcttcttc 200
<210> 691
   <211> 200
   <212> DNA
   <213> Euphorbia heterophylla
<400> 691
   aagattctgc aattgaagtt agaaatcgac gagtcttgtc ttgaaaagta tggattttgt 60
   tttgtcaccc tggggccggt tcaagattgc tttcccaggt tgctgtcttg gcatcacgtc 120
   ctaatccaaa accctaaggt gatgggttaa tgggtccctt cgcagttata tatcattcac 180
   ttaactcatt aacttccatg 200
<210> 692
   <211> 200
   <212> DNA
   <213> Euphorbia heterophylla
<400> 692
   catggaagtt aatgagttaa gtgaatgata tataactgcg aagggaccca ttaacccatc 60
   accttagggt tttggattag gacgtgatgc caagacagca acctgggaaa gcaatcttga 120
   accggcccca gggtgacaaa acaaaatcca tacttttcaa gacaagactc gtcgatttct 180
   aacttcaatt gcagaatctt 200
<210> 693
   <211> 200
   <212> DNA
   <213> Euphorbia heterophylla
<400> 693
   ttcacttaac tcattaactt ccatgtggat aatcaactcc acacttgccc caacactgta 60
   taaacgtaga ttttgacctt tggaaatcat tagctggaca aatcctaact aattagtatc 120
   aagagccaaa atcttttgac caagaccaaa atatgttgtt tcattgttgc agcattgtca 180
   tcatcctttc tccataccga 200
<210> 694
   <211> 200
   <212> DNA
   <213> Euphorbia heterophylla
<400> 694
   tcggtatgga gaaaggatga tgacaatgct gcaacaatga aacaacatat tttggtcttg 60
   gtcaaaagat tttggctctt gatactaatt agttaggatt tgtccagcta atgatttcca 120
   aaggtcaaaa tctacgttta tacagtgttg gggcaagtgt ggagttgatt atccacatgg 180
   aagttaatga gttaagtgaa 200
<210> 695
   <211> 200
   <212> DNA
   <213> Euphorbia heterophylla
<400> 695
   tgtcatcatc ctttctccat accgaaccaa atagttcggt ggaagttcat tctcaagagc 60
   aagaagtagt aattgcatta ggaagcaatg tgggagatag agttcataat ttcaaccaag 120
   cattgcaatt aatgaaaagt tccggcatta acataactag acatggttgt ttgtaccaga 180
   cagcacctgc ttatgtcact 200
<210> 696
   <211> 200
   <212> DNA
   <213> Euphorbia heterophylla
<400> 696
   agtgacataa gcaggtgctg tctggtacaa acaaccatgt ctagttatgt taatgccgga 60
   acttttcatt aattgcaatg cttggttgaa attatgaact ctatctccca cattgcttcc 120
   taatgcaatt actacttctt gctcttgaga atgaacttcc accgaactat ttggttcggt 180
   atggagaaag gatgatgaca 200
<210> 697
   <211> 200
   <212> DNA
   <213> Euphorbia heterophylla
<400> 697
   ccagacagca cctgcttatg tcactgacca acctcacttt ctcaactcag cagttcgagc 60
   tttcacgaaa ctcggacccc acgagttatt aggagtttta aagcagatcg aaaaggactt 120
   gggccgtacc aaagggatta ggtacggacc aaggccaatc gatttggata tacttttcta 180
   tggaaagttc cggattaatt 200
<210> 698
   <211> 200
   <212> DNA
   <213> Euphorbia heterophylla
<400> 698
   aattaatccg gaactttcca tagaaaagta tatccaaatc gattggcctt ggtccgtacc 60
   taatcccttt ggtacggccc aagtcctttt cgatctgctt taaaactcct aataactcgt 120
   ggggtccgag tttcgtgaaa gctcgaactg ctgagttgag aaagtgaggt tggtcagtga 180
   cataagcagg tgctgtctgg 200
<210> 699
   <211> 200
   <212> DNA
   <213> Euphorbia heterophylla
<400> 699
   ttctatggaa agttccggat taattccgat acacttattg ttcctcatga gagaatatgg 60
   gagagaccct ttgtgatggc cccattggtg gatttactcg gctcggaaat cgagaatgac 120
   acggtcgcca gctggcattc cttgtccggc ggtctttttg aatcatggga aaagttgggc 180
   ggcgaaagcc tgatcgggaa 200
<210> 700
   <211> 200
   <212> DNA
   <213> Euphorbia heterophylla
<400> 700
   ttcccgatca ggctttcgcc gcccaacttt tcccatgatt caaaaagacc gccggacaag   60
   gaatgccagc tggcgaccgt gtcattctcg atttccgagc cgagtaaatc caccaatggg  120
   gccatcacaa agggtctctc ccatattctc tcatgaggaa caataagtgt atcggaatta  180
   atccggaact ttccatagaa                                              200
<210> 701
   <211> 200
   <212> DNA
   <213> Euphorbia heterophylla
<400> 701
   tgggcggcga aagcctgatc gggaaggacg gaatgaaaag agttaccccg atcggaaacc   60
   atttatggga ttggtcggaa aagacttctg taatgggaat tatcaattta acccccgata  120
   gtttcagcga tgggggcaag ttgacaacta ttgattctat agtttctaag gttcgctcga  180
   tgatttccga aggggcggat                                              200
<210> 702
   <211> 200
   <212> DNA
   <213> Euphorbia heterophylla
<400> 702
   atccgcccct tcggaaatca tcgagcgaac cttagaaact atagaatcaa tagttgtcaa   60
   cttgccccca tcgctgaaac tatcgggggt taaattgata attcccatta cagaagtctt  120
   ttccgaccaa tcccataaat ggtttccgat cggggtaact cttttcattc cgtccttccc  180
   gatcaggctt tcgccgccca                                              200
<210> 703
   <211> 200
   <212> DNA
   <213> Euphorbia heterophylla
<400> 703
   ctcgatgatt tccgaagggg cggatattgt cgattttggt gctcaatcga cacgccctat   60
   ggctaaaagg atatccccgc aagaggaaat ggataggcta atccctgtat tggaagcggt  120
   tgtgaaaata cccgagatga ccgaaaagct catatcggtc gacacatttt actcggaagt  180
   cgccttagaa gcggtccaaa                                              200
<210> 704
   <211> 200
   <212> DNA
   <213> Euphorbia heterophylla
<400> 704
   tttggaccgc ttctaaggcg acttccgagt aaaatgtgtc gaccgatatg agcttttcgg   60
   tcatctcggg tattttcaca accgcttcca atacagggat tagcctatcc atttcctctt  120
   gcggggatat ccttttagcc atagggcgtg tcgattgagc accaaaatcg acaatatccg  180
   ccccttcgga aatcatcgag                                               200
<210> 705
   <211> 200
   <212> DNA
   <213> Euphorbia heterophylla
<400> 705
   gaagtcgcct tagaagcggt ccaaagagga gcaaatctcg taaatgacgt atctgggggg   60
   caattagatc caaaaatgac ggaaaatgtc gctaatctcg aggtaccata tatcttaatg  120
   cacatgaggg gggaccccac cacaatgcag aacactcaga atctaaaata cgataatgtt  180
   tgtaaagagg ttgcctttga                                               200
<210> 706
   <211> 200
   <212> DNA
   <213> Euphorbia heterophylla
<400> 706
   tcaaaggcaa cctctttaca aacattatcg tattttagat tctgagtgtt ctgcattgtg   60
   gtggggtccc ccctcatgtg cattaagata tatggtacct cgagattagc gacattttcc  120
   gtcatttttg gatctaattg ccccccagat acgtcattta cgagatttgc tcctctttgg  180
   accgcttcta aggcgacttc                                               200
<210> 707
   <211> 200
   <212> DNA
   <213> Euphorbia heterophylla
<400> 707
   atgtttgtaa agaggttgcc tttgagttgt tttcgagggt taaagaagcc gagatgtccg   60
   gaattccagc ctggcgaatg tttattgacc ctggaattgg gttttcgaag aacacgaatc  120
   agaatctgga aatcctgatg ggaattccaa ggatccgggc cgagattggg aggaaaagcg  180
   tgggtatttc tcgtgggccg                                               200
<210> 708
   <211> 200
   <212> DNA
   <213> Euphorbia heterophylla
<400> 708
   cggcccacga gaaataccca cgcttttcct cccaatctcg gcccggatcc ttggaattcc 60
   catcaggatt tccagattct gattcgtgtt cttcgaaaac ccaattccag ggtcaataaa 120
   cattcgccag gctggaattc cggacatctc ggcttcttta accctcgaaa acaactcaaa 180
   ggcaacctct ttacaaacat 200
<210> 709
   <211> 200
   <212> DNA
   <213> Euphorbia heterophylla
<400> 709
   aagcgtgggt atttctcgtg ggccgattct tattgggcct tcgagaaaga ggtttttggg 60
   cgaaatttgc gaacgccctg aagcaactga aagagacccg gcaactgttg cttctgttac 120
   tactgggatt ctgggagggg caaatattgt tagagttcat aatgttaggg ataatgtgga 180
   tgctgtgaag ctatgtgact 200
<210> 710
   <211> 200
   <212> DNA
   <213> Euphorbia heterophylla
<400> 710
   agtcacatag cttcacagca tccacattat ccctaacatt atgaactcta acaatatttg 60
   cccctcccag aatcccagta gtaacagaag caacagttgc cgggtctctt tcagttgctt 120
   cagggcgttc gcaaatttcg cccaaaaacc tctttctcga aggcccaata agaatcggcc 180
   cacgagaaat acccacgctt 200
<210> 711
   <211> 200
   <212> DNA
   <213> Euphorbia heterophylla
<400> 711
   gtggatgctg tgaagctatg tgactcgatg atgaaactaa agaggtctac ttaaaaatta 60
   tcgggtatag ttcgatttct ttctttccga atcttttgtt tgactttcag attgtctttg 120
   attggcaaaa cagtttcggc tgcggttact gtttgctgtt gcagtttacc gttgttgttt 180
   ctaattgtga ttttaagtta 200
<210> 712
   <211> 200
   <212> DNA
   <213> Euphorbia heterophylla
<400> 712
   taacttaaaa tcacaattag aaacaacaac ggtaaactgc aacagcaaac agtaaccgca 60
   gccgaaactg ttttgccaat caaagacaat ctgaaagtca aacaaaagat tcggaaagaa 120
   agaaatcgaa ctatacccga taatttttaa gtagacctct ttagtttcat catcgagtca 180
   catagcttca cagcatccac 200
<210> 713
   <211> 200
   <212> DNA
   <213> Euphorbia heterophylla
<400> 713
   tgtttctaat tgtgatttta agttattgtt gttagttgat ttgtacttag tgcttggtga 60
   aaatataatt cacggttgcg gttggaggta taaattacta aaatagacat attttaaata 120
   aattagaagt tatattgaag ggtatttagg ataaataaaa gtccaaacca caaaaaaaaa 180
   aaaccgtaaa actattgatt 200
<210> 714
   <211> 200
   <212> DNA
   <213> Euphorbia heterophylla
<400> 714
   aatcaatagt tttacggttt tttttttttg tggtttggac ttttatttat cctaaatacc 60
   cttcaatata acttctaatt tatttaaaat atgtctattt tagtaattta tacctccaac 120
   cgcaaccgtg aattatattt tcaccaagca ctaagtacaa atcaactaac aacaataact 180
   taaaatcaca attagaaaca 200
<210> 715
   <211> 200
   <212> DNA
   <213> Euphorbia heterophylla
<400> 715
   aaaaaaaacc gtaaaactat tgatttggtg aggtttggaa aaggagataa atcccttatg 60
   aaaagcaact ccatatatgt tttgaaactt aaccaaacac ttgtatacct ccttttcctt 120
   ttcaaacctc aaaagacctt tttatagtaa gctcttttga agtatatacg ctattgtttc 180
   cgaatcattt tggacttttc 200
<210> 716
   <211> 200
   <212> DNA
   <213> Euphorbia heterophylla
<400> 716
   gaaaagtcca aaatgattcg gaaacaatag cgtatatact tcaaaagagc ttactataaa 60
   aaggtctttt gaggtttgaa aaggaaaagg aggtatacaa gtgtttggtt aagtttcaaa 120
   acatatatgg agttgctttt cataagggat ttatctcctt ttccaaacct caccaaatca 180
   atagttttac ggtttttttt 200
<210> 717
   <211> 200
   <212> DNA
   <213> Euphorbia heterophylla
<400> 717
   gtttccgaat cattttggac ttttcgtcga aatcaacaat actgttagct agtcatattt 60
   caagatattt gggatgtaaa gatcaaggta gttgttgttg tattctgttc tcatcagcaa 120
   aatttgttat aactccacca agatttaatt ttttccaatc tgaaaatgtc ttatgttata 180
   gtaagaagtg agatgagttt 200
<210> 718
   <211> 200
   <212> DNA
   <213> Euphorbia heterophylla
<400> 718
   aaactcatct cacttcttac tataacataa gacattttca gattggaaaa aattaaatct 60
   tggtggagtt ataacaaatt ttgctgatga gaacagaata caacaacaac taccttgatc 120
   tttacatccc aaatatcttg aaatatgact agctaacagt attgttgatt tcgacgaaaa 180
   gtccaaaatg attcggaaac 200
<210> 719
   <211> 200
   <212> DNA
   <213> Euphorbia heterophylla
<400> 719
   ttatagtaag aagtgagatg agtttatgtc tttgtgaaaa gattttgccc aattaatgca   60
   aaaattttgt gacaagacaa gaggggtgaa agagacaagc aacaagagaa agcagtgaaa  120
   ggggcaaatg ttaggaaact gttagctaga aaagggtaga gggtggccta ctttaccaat  180
   ttgagcatta aaaaagcaca                                              200
<210> 720
   <211> 200
   <212> DNA
   <213> Euphorbia heterophylla
<400> 720
   tgtgcttttt taatgctcaa attggtaaag taggccaccc tctacccttt tctagctaac   60
   agtttcctaa catttgcccc tttcactgct ttctcttgtt gcttgtctct ttcacccctc  120
   ttgtcttgtc acaaaatttt tgcattaatt gggcaaaatc ttttcacaaa gacataaact  180
   catctcactt cttactataa                                              200
<210> 721
   <211> 200
   <212> DNA
   <213> Euphorbia heterophylla
<400> 721
   ccaatttgag cattaaaaaa gcacatggct atgtatttta gaccaaagtc cctattgttg   60
   gcagatgaac taagcaacca tatggttcat taactaacta actagttatt gcaaatttga  120
   ctgttccaat tcatcaaagg actctcccca tagaccattc aacacttggg ttctagccct  180
   ccctctcacc ctcagacaac                                              200
<210> 722
   <211> 200
   <212> DNA
   <213> Euphorbia heterophylla
<400> 722
   gttgtctgag ggtgagaggg agggctagaa cccaagtgtt gaatggtcta tggggagagt   60
   cctttgatga attggaacag tcaaatttgc aataactagt tagttagtta atgaaccata  120
   tggttgctta gttcatctgc caacaatagg gactttggtc taaaatacat agccatgtgc  180
   ttttttaatg ctcaaattgg                                               200
<210> 723
   <211> 200
   <212> DNA
   <213> Euphorbia heterophylla
<400> 723
   gccctccctc tcaccctcag acaacccttg tgaacctcat tttcatcatt ggtccccttt   60
   tttgttagga ctacacttgt ctctataaaa tctcttctag ccatgtgtta tgtgtagact  120
   ttttcggttt gaggattggg tcgggttcgg gctgggtctc gtcgggcttt ttactgcaaa  180
   catcaaacta gggttctaaa                                              200
<210> 724
   <211> 200
   <212> DNA
   <213> Euphorbia heterophylla
<400> 724
   tttagaaccc tagtttgatg tttgcagtaa aaagcccgac gagacccagc ccgaacccga   60
   cccaatcctc aaaccgaaaa agtctacaca taacacatgg ctagaagaga ttttatagag  120
   acaagtgtag tcctaacaaa aaaggggacc aatgatgaaa atgaggttca caagggttgt  180
   ctgagggtga gagggagggc                                              200
<210> 725
   <211> 200
   <212> DNA
   <213> Euphorbia heterophylla
<400> 725
   gcaaacatca aactagggtt ctaaacccat acttcagtac tctggttcgg attgaacttg   60
   cgagatcatt ctaaattcaa tttacggctc atagattata tttgagagtt ttgagttttg  120
   ggtggattag attgaaaacg actatttttt gttgtataaa tatatctaaa aagttatata  180
   attacattct ttattggaat                                              200
<210> 726
   <211> 200
   <212> DNA
   <213> Euphorbia heterophylla
<400> 726
   attccaataa agaatgtaat tatataactt tttagatata tttatacaac aaaaaatagt 60
   cgttttcaat ctaatccacc caaaactcaa aactctcaaa tataatctat gagccgtaaa 120
   ttgaatttag aatgatctcg caagttcaat ccgaaccaga gtactgaagt atgggtttag 180
   aaccctagtt tgatgtttgc 200
<210> 727
   <211> 200
   <212> DNA
   <213> Euphorbia heterophylla
<400> 727
   atataattac attctttatt ggaattcatc aatatatata agaatttgac tttgttgtag 60
   ccattttgaa agttcgataa gctactccct tcgtctagtt tcaatagttc atttaaaagt 120
   ttcattaatt ctgttttcaa ttgtcaattt aaattttcat aatatttttc accatttttt 180
   tttccaattt gcccttgtat 200
<210> 728
   <211> 200
   <212> DNA
   <213> Euphorbia heterophylla
<400> 728
   atacaagggc aaattggaaa aaaaaatggt gaaaaatatt atgaaaattt aaattgacaa 60
   ttgaaaacag aattaatgaa acttttaaat gaactattga aactagacga agggagtagc 120
   ttatcgaact ttcaaaatgg ctacaacaaa gtcaaattct tatatatatt gatgaattcc 180
   aataaagaat gtaattatat 200
<210> 729
   <211> 200
   <212> DNA
   <213> Euphorbia heterophylla
<400> 729
   ttttttttcc aatttgccct tgtatttatt gagaaagaga ttatgttatg atgaaagtat 60
   aagttgctaa tcaagaagaa agagatttta atgtaattat ttaaataata aattcctacc 120
   attttaattg tgtgacaatt ggttataatg attccgttgc atcgacaact aaaactagat 180
   agaaggatca aaatataaaa 200
<210> 730
   <211> 200
   <212> DNA
   <213> Euphorbia heterophylla
<400> 730
   ttttatattt tgatccttct atctagtttt agttgtcgat gcaacggaat cattataacc   60
   aattgtcaca caattaaaat ggtaggaatt tattatttaa ataattacat taaaatctct  120
   ttcttcttga ttagcaactt atactttcat cataacataa tctctttctc aataaataca  180
   agggcaaatt ggaaaaaaaa                                              200
<210> 731
   <211> 200
   <212> DNA
   <213> Euphorbia heterophylla
<400> 731
   tagatagaag gatcaaaata taaaatttta acataattaa tacatataat atataaatgt   60
   ataatagata caattattat tcctcgaatt aaaattggct aattatataa taatataatc  120
   caacctataa aaacttttga gcttttaagc ttgagatgag ctttgtaaaa atgattgtac  180
   aagcccgacc atgaaagaga                                              200
<210> 732
   <211> 200
   <212> DNA
   <213> Euphorbia heterophylla
<400> 732
   tctctttcat ggtcgggctt gtacaatcat ttttacaaag ctcatctcaa gcttaaaagc   60
   tcaaaagttt ttataggttg gattatatta ttatataatt agccaatttt aattcgagga  120
   ataataattg tatctattat acatttatat attatatgta ttaattatgt taaaatttta  180
   tattttgatc cttctatcta                                              200
<210> 733
   <211> 200
   <212> DNA
   <213> Kochia scoparia
<400> 733
   gaatgtgctt aggcggctgc aatcagccag tttgatttgg aagggaattt ccaagtatga   60
   tagaggtaaa gcacagttgt ttcatttttt gactatatat ttaatttggg aatctgtact  120
   gtttcatttt gtaactttat tttgatcctg agttattgaa tctgtcatgt ctcattttga  180
   gacggtattt tgaatctgag                                              200
<210> 734
   <211> 200
   <212> DNA
   <213> Kochia scoparia
<400> 734
   ctcagattca aaataccgtc tcaaaatgag acatgacaga ttcaataact caggatcaaa   60
   ataaagttac aaaatgaaac agtacagatt cccaaattaa atatatagtc aaaaaatgaa  120
   acaactgtgc tttacctcta tcatacttgg aaattccctt ccaaatcaaa ctggctgatt  180
   gcagccgcct aagcacattc                                              200
<210> 735
   <211> 200
   <212> DNA
   <213> Kochia scoparia
<400> 735
   tttgagacgg tattttgaat ctgagtctgg atatgaaatt atgaataggc cataggtatt   60
   tataaatata gggtttaagt tgatgatctg gatgctatca gagctctatt atgcaggatt  120
   acaaatcata atttcattgt gaaatttact tctttatgtg tattcacttg aagattcata  180
   attttgatgt attcataaat                                              200
<210> 736
   <211> 200
   <212> DNA
   <213> Kochia scoparia
<400> 736
   atttatgaat acatcaaaat tatgaatctt caagtgaata cacataaaga agtaaatttc   60
   acaatgaaat tatgatttgt aatcctgcat aatagagctc tgatagcatc cagatcatca  120
   acttaaaccc tatatttata aatacctatg gcctattcat aatttcatat ccagactcag  180
   attcaaaata ccgtctcaaa                                              200
<210> 737
   <211> 200
   <212> DNA
   <213> Kochia scoparia
<400> 737
   tcataatttt gatgtattca taaatttgat gtgccctaaa cagaaagagt cagcatgtgc 60
   tgaaagtata agggggaaaa ccaagatgta taaatctttt ccttgatgag ttgcttatat 120
   gattgatgtc atgattctaa aatgccttta cctaattcaa atggatttta aagattacgt 180
   atgtactttt gcagcttcaa 200
<210> 738
   <211> 200
   <212> DNA
   <213> Kochia scoparia
<400> 738
   ttgaagctgc aaaagtacat acgtaatctt taaaatccat ttgaattagg taaaggcatt 60
   ttagaatcat gacatcaatc atataagcaa ctcatcaagg aaaagattta tacatcttgg 120
   ttttccccct tatactttca gcacatgctg actctttctg tttagggcac atcaaattta 180
   tgaatacatc aaaattatga 200
<210> 739
   <211> 200
   <212> DNA
   <213> Kochia scoparia
<400> 739
   tacgtatgta cttttgcagc ttcaagtctt gcttttttgc actcatcacc agaaactact 60
   attgaggttt gttctaaaga gcatgaagtt gtaattgctc tagggagcaa tgtaggagac 120
   agattagata actttaacca agctctgcag ctaatgaaga aattaggagt aaatatcaca 180
   aggcatggct gcttatacga 200
<210> 740
   <211> 200
   <212> DNA
   <213> Kochia scoparia
<400> 740
   tcgtataagc agccatgcct tgtgatattt actcctaatt tcttcattag ctgcagagct 60
   tggttaaagt tatctaatct gtctcctaca ttgctcccta gagcaattac aacttcatgc 120
   tctttagaac aaacctcaat agtagtttct ggtgatgagt gcaaaaaagc aagacttgaa 180
   gctgcaaaag tacatacgta 200
<210> 741
   <211> 200
   <212> DNA
   <213> Kochia scoparia
<400> 741
   tcacaaggca tggctgctta tacgagacag atcctgccta tgtgactgat caaccaaagt 60
   ttcttaactc tgcagtgagg ggcttcacaa aacttgggcc tctagaacta ttagggatgc 120
   tgaagaaaat tgagaaggat atgggtcgaa ctaatggaat aagatatggc cctaggccaa 180
   ttgacttaga cattcttttt 200
<210> 742
   <211> 200
   <212> DNA
   <213> Kochia scoparia
<400> 742
   aaaaagaatg tctaagtcaa ttggcctagg gccatatctt attccattag ttcgacccat 60
   atccttctca attttcttca gcatccctaa tagttctaga ggcccaagtt ttgtgaagcc 120
   cctcactgca gagttaagaa actttggttg atcagtcaca taggcaggat ctgtctcgta 180
   taagcagcca tgccttgtga 200
<210> 743
   <211> 200
   <212> DNA
   <213> Kochia scoparia
<400> 743
   gccaattgac ttagacattc ttttttatgg gaagtttagg gtaagctctg agaaacttac 60
   agttccgcat gaaaggatat gggaaagacc ctttgtgatg gcaccactaa ttgatataat 120
   tggatcggat gtagaaaatg acactgttgc tgcatggcat tcattatcaa aattttctgg 180
   tggactattt gaagcgtggg 200
<210> 744
   <211> 200
   <212> DNA
   <213> Kochia scoparia
<400> 744
   cccacgcttc aaatagtcca ccagaaaatt ttgataatga atgccatgca gcaacagtgt 60
   cattttctac atccgatcca attatatcaa ttagtggtgc catcacaaag ggtctttccc 120
   atatcctttc atgcggaact gtaagtttct cagagcttac cctaaacttc ccataaaaaa 180
   gaatgtctaa gtcaattggc 200
<210> 745
   <211> 200
   <212> DNA
   <213> Kochia scoparia
<400> 745
   tctggtggac tatttgaagc gtgggataaa cttggtggag attcactcat tgggaaggac    60
   ggaatgagta gggttttgcc agttgggaac cacttgtggg attggtcgtg caaaacctct   120
   gtaatgggag tcttgaattt gactccggac agctttagtg atggaggaaa gtttctacct   180
   gtagaaaatg cagtttctca                                               200
<210> 746
   <211> 200
   <212> DNA
   <213> Kochia scoparia
<400> 746
   tgagaaactg cattttctac aggtagaaac tttcctccat cactaaagct gtccggagtc    60
   aaattcaaga ctcccattac agaggttttg cacgaccaat cccacaagtg gttcccaact   120
   ggcaaaaccc tactcattcc gtccttccca atgagtgaat ctccaccaag tttatcccac   180
   gcttcaaata gtccaccaga                                               200
<210> 747
   <211> 200
   <212> DNA
   <213> Kochia scoparia
<400> 747
   tacctgtaga aaatgcagtt tctcaggttc gtcaaatgat ctcagaaggt gctgacataa    60
   ttgatattgg agcgcaatca acaaggccca tggcaactag gatttctgct gaggaagagc   120
   tggaaagact agtccctgtt ttagaaggtg tcaaggatgt tatcgaggaa gaaggaagaa   180
   tgttatcagt ggatacattt                                               200
<210> 748
   <211> 200
   <212> DNA
   <213> Kochia scoparia
<400> 748
   aaatgtatcc actgataaca ttcttccttc ttcctcgata acatccttga caccttctaa    60
   aacagggact agtctttcca gctcttcctc agcagaaatc ctagttgcca tgggccttgt   120
   tgattgcgct ccaatatcaa ttatgtcagc accttctgag atcatttgac gaacctgaga   180
   aactgcattt tctacaggta                                               200
<210> 749
   <211> 200
   <212> DNA
   <213> Kochia scoparia
<400> 749
   aagaatgtta tcagtggata cattttactc aaaagttgct tctgaagctg tcaataaggg   60
   agcacatatg gtgaatgatg tttcgagtgg aaagctagat cctgagatgt tcaatgttgt  120
   tgcagggctt aaagtgcctt atatagcaat gcacatgcga ggtgatcctt cttcaatgca  180
   aaatgctgat aatttgacat                                              200
<210> 750
   <211> 200
   <212> DNA
   <213> Kochia scoparia
<400> 750
   atgtcaaatt atcagcattt tgcattgaag aaggatcacc tcgcatgtgc attgctatat   60
   aaggcacttt aagccctgca acaacattga acatctcagg atctagcttt ccactcgaaa  120
   catcattcac catatgtgct cccttattga cagcttcaga agcaactttt gagtaaaatg  180
   tatccactga taacattctt                                              200
<210> 751
   <211> 200
   <212> DNA
   <213> Kochia scoparia
<400> 751
   atgcaaaatg ctgataattt gacatacaat gatgtttgta aggaggtggc tttagagttg   60
   agctctagga ttacagatgc agaattatca ggtattcctg cttggaggat agttattgat  120
   cctggcattg gattttccaa gaatacaaag caaaatttgg aaattctcac agggttgaaa  180
   agaattcggc aagaaatagc                                              200
<210> 752
   <211> 200
   <212> DNA
   <213> Kochia scoparia
<400> 752
   gctatttctt gccgaattct tttcaaccct gtgagaattt ccaaattttg ctttgtattc   60
   ttggaaaatc caatgccagg atcaataact atcctccaag caggaatacc tgataattct  120
   gcatctgtaa tcctagagct caactctaaa gccacctcct tacaaacatc attgtatgtc  180
   aaattatcag cattttgcat                                              200
<210> 753
   <211> 200
   <212> DNA
   <213> Kochia scoparia
<400> 753
   tgaaaagaat tcggcaagaa atagcaaaaa agagtttggc tgtggctcat ggtcccttac   60
   tgattggacc ttcaagaaag aggtttttgg gtgagatctg caatcgtcct gtagcatctg  120
   atagagatcc ggcaactgtt gcttctatca ccgctggagt tttgggtggt gcaaacattg  180
   taagagtaca taatgttcgg                                              200
<210> 754
   <211> 200
   <212> DNA
   <213> Kochia scoparia
<400> 754
   ccgaacatta tgtactctta caatgtttgc accacccaaa actccagcgg tgatagaagc   60
   aacagttgcc ggatctctat cagatgctac aggacgattg cagatctcac ccaaaaacct  120
   ctttcttgaa ggtccaatca gtaagggacc atgagccaca gccaaactct tttttgctat  180
   ttcttgccga attcttttca                                              200
<210> 755
   <211> 200
   <212> DNA
   <213> Kochia scoparia
<400> 755
   cattgtaaga gtacataatg ttcgggatag ccttgatggt gtcaagctat gtgatgcaat   60
   gcttgaatat cagtataact gaagattctt agattcttag attcttagat tctaagagtt  120
   gattttatag attattgtct ttgttgtgtg ccttcggcag atatgaataa tatgattgta  180
   attttgttgc ttatgatttt                                              200
<210> 756
   <211> 200
   <212> DNA
   <213> Kochia scoparia
<400> 756
   aaaatcataa gcaacaaaat tacaatcata ttattcatat ctgccgaagg cacacaacaa 60
   agacaataat ctataaaatc aactcttaga atctaagaat ctaagaatct aagaatcttc 120
   agttatactg atattcaagc attgcatcac atagcttgac accatcaagg ctatcccgaa 180
   cattatgtac tcttacaatg 200
<210> 757
   <211> 200
   <212> DNA
   <213> Kochia scoparia
<400> 757
   ttgtaatttt gttgcttatg attttcttcc tacctttctt ttatcataga atttagatta 60
   aaaaatgtac ttgaatttca taaatggtga aataaagttt gtcggtgctt catgcaatac 120
   gacttttcta gtttgcattg ctctatcata ggggctgtaa gcagattagc ccagtcaatt 180
   cacattagat ttagacggcc 200
<210> 758
   <211> 200
   <212> DNA
   <213> Kochia scoparia
<400> 758
   ggccgtctaa atctaatgtg aattgactgg gctaatctgc ttacagcccc tatgatagag 60
   caatgcaaac tagaaaagtc gtattgcatg aagcaccgac aaactttatt tcaccattta 120
   tgaaattcaa gtacattttt taatctaaat tctatgataa aagaaaggta ggaagaaaat 180
   cataagcaac aaaattacaa 200
<210> 759
   <211> 200
   <212> DNA
   <213> Kochia scoparia
<400> 759
   caattcacat tagatttaga cggccggtgc aaaaacagca taatgttgct aacgctgttt 60
   atacatttaa actatatgat ctctaagagc aaaatacaaa ataatacaat gtgtttcatg 120
   aagatgtcca atcatttctc cctcaaaaaa agatcgtgat gaacaatcag gtactttgat 180
   tttgttgtag aacctcagct 200
<210> 760
   <211> 200
   <212> DNA
   <213> Kochia scoparia
<400> 760
   agctgaggtt ctacaacaaa atcaaagtac ctgattgttc atcacgatct ttttttgagg 60
   gagaaatgat tggacatctt catgaaacac attgtattat tttgtatttt gctcttagag 120
   atcatatagt ttaaatgtat aaacagcgtt agcaacatta tgctgttttt gcaccggccg 180
   tctaaatcta atgtgaattg 200
<210> 761
   <211> 200
   <212> DNA
   <213> Kochia scoparia
<400> 761
   ttgattttgt tgtagaacct cagcttcagc cctagccatg ggatcatctt tttctccttc 60
   tcgctcctca gccaatttct ttctgtgcat ctcctctgca gcttctattg aagctttgtg 120
   ctcagcctgg agacgctgca tttcctcttc catctgctgt ctttcgaacc atgtatcagc 180
   atccgcccaa actgcagaaa 200
<210> 762
   <211> 200
   <212> DNA
   <213> Kochia scoparia
<400> 762
   tttctgcagt ttgggcggat gctgatacat ggttcgaaag acagcagatg gaagaggaaa 60
   tgcagcgtct ccaggctgag cacaaagctt caatagaagc tgcagaggag atgcacagaa 120
   agaaattggc tgaggagcga gaaggagaaa aagatgatcc catggctagg gctgaagctg 180
   aggttctaca acaaaatcaa 200
<210> 763
   <211> 200
   <212> DNA
   <213> Kochia scoparia
<400> 763
   tcagcatccg cccaaactgc agaaataatt taaacaagtt aatcatacgg agtgtgcagc 60
   atggcaaatt ggcaataaga tggtgcaagt aacaagctca aatgatccac catagccaaa 120
   ttcctaacca ttggactgtg tcatatactc atatcagcta gtagaaacat tctcaccata 180
   accctaacta ttcgaggata 200
<210> 764
   <211> 200
   <212> DNA
   <213> Kochia scoparia
<400> 764
   tatcctcgaa tagttagggt tatggtgaga atgtttctac tagctgatat gagtatatga   60
   cacagtccaa tggttaggaa tttggctatg gtggatcatt tgagcttgtt acttgcacca  120
   tcttattgcc aatttgccat gctgcacact ccgtatgatt aacttgttta aattatttct  180
   gcagtttggg cggatgctga                                              200
<210> 765
   <211> 200
   <212> DNA
   <213> Kochia scoparia
<400> 765
   ccataaccct aactattcga ggataagtga taccattcaa caatagtaaa gatcaaatca   60
   ggtattacta gacctatcaa agtaatacgg agtagttgtt attgttacag agttaggaag  120
   aatcctattt tcccatttct cctatcaatc ctaaactttt gtgactgaac tatcgacata  180
   tagtgtgaag aacatgaatg                                              200
<210> 766
   <211> 200
   <212> DNA
   <213> Kochia scoparia
<400> 766
   cattcatgtt cttcacacta tatgtcgata gttcagtcac aaaagtttag gattgatagg   60
   agaaatggga aaataggatt cttcctaact ctgtaacaat aacaactact ccgtattact  120
   ttgataggtc tagtaatacc tgatttgatc tttactattg ttgaatggta tcacttatcc  180
   tcgaatagtt agggttatgg                                              200
<210> 767
   <211> 200
   <212> DNA
   <213> Kochia scoparia
<400> 767
   acatatagtg tgaagaacat gaatggcaga agtcacaact ctgattcctg agtcctgact   60
   ataaactatt ggatgactgt gatacatcat tagtccttta agcattgagt tactctattc  120
   tacatagtta aattatatag attccaagat ccgaatccat gaattatcaa ttaccataaa  180
   ttgatcttct tcctttgagt                                              200
<210> 768
   <211> 200
   <212> DNA
   <213> Kochia scoparia
<400> 768
   actcaaagga agaagatcaa tttatggtaa ttgataattc atggattcgg atcttggaat    60
   ctatataatt taactatgta gaatagagta actcaatgct taaaggacta atgatgtatc   120
   acagtcatcc aatagtttat agtcaggact caggaatcag agttgtgact tctgccattc   180
   atgttcttca cactatatgt                                               200
<210> 769
   <211> 200
   <212> DNA
   <213> Kochia scoparia
<400> 769
   ataaattgat cttcttcctt tgagttttgt caacagttcc tactccgaaa tatgtcttaa    60
   atctagaagc aatagacact tatcaatgat acagcaatag acgaattcat ctacccaagg   120
   cagcatcaac aacagctgcc gtttgcgata atcgaaacta tcatagtgtc cttttcacta   180
   ttagtgcaga tgttatctag                                               200
<210> 770
   <211> 200
   <212> DNA
   <213> Kochia scoparia
<400> 770
   ctagataaca tctgcactaa tagtgaaaag gacactatga tagtttcgat tatcgcaaac    60
   ggcagctgtt gttgatgctg ccttgggtag atgaattcgt ctattgctgt atcattgata   120
   agtgtctatt gcttctagat ttaagacata tttcggagta ggaactgttg acaaaactca   180
   aaggaagaag atcaatttat                                               200
<210> 771
   <211> 200
   <212> DNA
   <213> Kochia scoparia
<400> 771
   cactattagt gcagatgtta tctagggtct aaacctctat gtttctacat tcctggaaaa    60
   tcttattctg tactgcataa tcctctcaca aaaaattcgc caatgaatga caaattagct   120
   atccagaatt gcagattgat tgaatcaaat tgatcatggc agcagcacaa ttgcagcagt   180
   tatcaattga agcacaatat                                               200
<210> 772
   <211> 200
   <212> DNA
   <213> Kochia scoparia
<400> 772
   atattgtgct tcaattgata actgctgcaa ttgtgctgct gccatgatca atttgattca    60
   atcaatctgc aattctggat agctaatttg tcattcattg gcgaattttt tgtgagagga   120
   ttatgcagta cagaataaga ttttccagga atgtagaaac atagaggttt agaccctaga   180
   taacatctgc actaatagtg                                               200
<210> 773
   <211> 200
   <212> DNA
   <213> Kochia scoparia
<400> 773
   gcagttatca attgaagcac aatattacct ctcaatcata acaatcaaag cacagaaatt    60
   ccaatcaata actccacaat atagtatcaa tctctaactc agaaactaaa cagaagcaaa   120
   aacccattca atcaaagcaa cagtaaaatt agggtttgaa ttagaaagaa agagagggaa   180
   gagaaacata ctgggttgcg                                               200
<210> 774
   <211> 200
   <212> DNA
   <213> Kochia scoparia
<400> 774
   cgcaacccag tatgtttctc ttccctctct ttctttctaa ttcaaaccct aattttactg    60
   ttgctttgat tgaatgggtt tttgcttctg tttagtttct gagttagaga ttgatactat   120
   attgtggagt tattgattgg aatttctgtg ctttgattgt tatgattgag aggtaatatt   180
   gtgcttcaat tgataactgc                                               200
<210> 775
   <211> 200
   <212> DNA
   <213> Kochia scoparia
<400> 775
   gggaagagaa acatactggg ttgcgcagcc catccctgtt caccaccctt aatcttctcg 60
   ggaagagcgt aattgacagt gagaacacga ccgtaaagct cagcaccatc catgttatcc 120
   atggcagaag aagcgtcgtc tctctccaag aaagtgacga aaccgaaaga acgatgcttg 180
   ttggtggcca aatcaagagg 200
<210> 776
   <211> 200
   <212> DNA
   <213> Kochia scoparia
<400> 776
   cctcttgatt tggccaccaa caagcatcgt tctttcggtt tcgtcacttt cttggagaga 60
   gacgacgctt cttctgccat ggataacatg gatggtgctg agctttacgg tcgtgttctc 120
   actgtcaatt acgctcttcc cgagaagatt aagggtggtg aacagggatg ggctgcgcaa 180
   cccagtatgt ttctcttccc 200
<210> 777
   <211> 200
   <212> DNA
   <213> Kochia scoparia
<400> 777
   gcagcttcaa gtcttgcttt tttgcactca tcaccagaaa ctactattga ggtttgttct 60
   aaagagcatg aagttgtaat tgctctaggg agcaatgtag gagacagatt agataacttt 120
   aaccaagctc tgcagctaat gaagaaatta ggagtaaata tcacaaggca tggctgctta 180
   tacgagacag atcctgccta 200
<210> 778
   <211> 200
   <212> DNA
   <213> Kochia scoparia
<400> 778
   taggcaggat ctgtctcgta taagcagcca tgccttgtga tatttactcc taatttcttc 60
   attagctgca gagcttggtt aaagttatct aatctgtctc ctacattgct ccctagagca 120
   attacaactt catgctcttt agaacaaacc tcaatagtag tttctggtga tgagtgcaaa 180
   aaagcaagac ttgaagctgc 200
<210> 779
   <211> 200
   <212> DNA
   <213> Kochia scoparia
<400> 779
   gcttatacga gacagatcct gcctatgtga ctgatcaacc aaagtttctt aactctgcag 60
   tgaggggctt cacaaaactt gggcctctag aactattagg gatgctgaag aaaattgaga 120
   aggatatggg tcgaactaat ggaataagat atggccctag gccaattgac ttagacattc 180
   ttttttatgg gaagtttagg 200
<210> 780
   <211> 200
   <212> DNA
   <213> Kochia scoparia
<400> 780
   cctaaacttc ccataaaaaa gaatgtctaa gtcaattggc ctagggccat atcttattcc 60
   attagttcga cccatatcct tctcaatttt cttcagcatc cctaatagtt ctagaggccc 120
   aagttttgtg aagcccctca ctgcagagtt aagaaacttt ggttgatcag tcacataggc 180
   aggatctgtc tcgtataagc 200
<210> 781
   <211> 200
   <212> DNA
   <213> Kochia scoparia
<400> 781
   cattcttttt tatgggaagt ttagggtaag ctctgagaaa cttacagttc cgcatgaaag 60
   gatatgggaa agaccctttg tgatggcacc actaattgat ataattggat cggatgtaga 120
   aaatgacact gttgctgcat ggcattcatt atcaaaattt tctggtggac tatttgaagc 180
   gtgggataaa cttggtggag 200
<210> 782
   <211> 200
   <212> DNA
   <213> Kochia scoparia
<400> 782
   ctccaccaag tttatcccac gcttcaaata gtccaccaga aaattttgat aatgaatgcc 60
   atgcagcaac agtgtcattt tctacatccg atccaattat atcaattagt ggtgccatca 120
   caaagggtct ttcccatatc ctttcatgcg gaactgtaag tttctcagag cttaccctaa 180
   acttcccata aaaaagaatg 200
<210> 783
   <211> 200
   <212> DNA
   <213> Kochia scoparia
<400> 783
   gaagcgtggg ataaacttgg tggagattca ctcattggga aggacggaat gagtagggtt 60
   ttgccagttg ggaaccactt gtgggattgg tcgtgcaaaa cctctgtaat gggagtcttg 120
   aatttgactc cggacagctt tagtgatgga ggaaagtttc tacctgtaga aaatgcagtt 180
   tctcaggttc gtcaaatgat 200
<210> 784
   <211> 200
   <212> DNA
   <213> Kochia scoparia
<400> 784
   atcatttgac gaacctgaga aactgcattt tctacaggta gaaactttcc tccatcacta 60
   aagctgtccg gagtcaaatt caagactccc attacagagg ttttgcacga ccaatcccac 120
   aagtggttcc caactggcaa aaccctactc attccgtcct tcccaatgag tgaatctcca 180
   ccaagtttat cccacgcttc 200
<210> 785
   <211> 200
   <212> DNA
   <213> Kochia scoparia
<400> 785
   cagtttctca ggttcgtcaa atgatctcag aaggtgctga cataattgat attggagcgc 60
   aatcaacaag gcccatggca actaggattt ctgctgagga agagctggaa agactagtcc 120
   ctgttttaga aggtgtcaag gatgttatcg aggaagaagg aagaatgtta tcagtggata 180
   cattttactc aaaagttgct 200
<210> 786
   <211> 200
   <212> DNA
   <213> Kochia scoparia
<400> 786
   agcaactttt gagtaaaatg tatccactga taacattctt ccttcttcct cgataacatc 60
   cttgacacct tctaaaacag ggactagtct ttccagctct tcctcagcag aaatcctagt 120
   tgccatgggc cttgttgatt gcgctccaat atcaattatg tcagcacctt ctgagatcat 180
   ttgacgaacc tgagaaactg 200
<210> 787
   <211> 200
   <212> DNA
   <213> Kochia scoparia
<400> 787
   ggatacattt tactcaaaag ttgcttctga agctgtcaat aagggagcac atatggtgaa 60
   tgatgtttcg agtggaaagc tagatcctga gatgttcaat gttgttgcag ggcttaaagt 120
   gccttatata gcaatgcaca tgcgaggtga tccttcttca atgcaaaatg ctgataattt 180
   gacatacaat gatgtttgta 200
<210> 788
   <211> 200
   <212> DNA
   <213> Kochia scoparia
<400> 788
   tacaaacatc attgtatgtc aaattatcag cattttgcat tgaagaagga tcacctcgca 60
   tgtgcattgc tatataaggc actttaagcc ctgcaacaac attgaacatc tcaggatcta 120
   gctttccact cgaaacatca ttcaccatat gtgctccctt attgacagct tcagaagcaa 180
   cttttgagta aaatgtatcc 200
<210> 789
   <211> 200
   <212> DNA
   <213> Kochia scoparia
<400> 789
   aatttgacat acaatgatgt ttgtaaggag gtggctttag agttgagctc taggattaca 60
   gatgcagaat tatcaggtat tcctgcttgg aggatagtta ttgatcctgg cattggattt 120
   tccaagaata caaagcaaaa tttggaaatt ctcacagggt tgaaaagaat tcggcaagaa 180
   atagcaaaaa agagtttggc 200
<210> 790
   <211> 200
   <212> DNA
   <213> Kochia scoparia
<400> 790
   gccaaactct tttttgctat ttcttgccga attcttttca accctgtgag aatttccaaa 60
   ttttgctttg tattcttgga aaatccaatg ccaggatcaa taactatcct ccaagcagga 120
   atacctgata attctgcatc tgtaatccta gagctcaact ctaaagccac ctccttacaa 180
   acatcattgt atgtcaaatt 200
<210> 791
   <211> 200
   <212> DNA
   <213> Kochia scoparia
<400> 791
   aagaaatagc aaaaaagagt ttggctgtgg ctcatggtcc cttactgatt ggaccttcaa 60
   gaaagaggtt tttgggtgag atctgcaatc gtcctgtagc atctgataga gatccggcaa 120
   ctgttgcttc tatcaccgct ggagttttgg gtggtgcaaa cattgtaaga gtacataatg 180
   ttcgggatag ccttgatggt 200
<210> 792
   <211> 200
   <212> DNA
   <213> Kochia scoparia
<400> 792
   accatcaagg ctatcccgaa cattatgtac tcttacaatg tttgcaccac ccaaaactcc 60
   agcggtgata gaagcaacag ttgccggatc tctatcagat gctacaggac gattgcagat 120
   ctcacccaaa aacctctttc ttgaaggtcc aatcagtaag ggaccatgag ccacagccaa 180
   actctttttt gctatttctt 200
<210> 793
   <211> 200
   <212> DNA
   <213> Kochia scoparia
<400> 793
   taattcaaat ggattttaaa gattacgtat gtacttttgc agcttcaagt cttgcttttt 60
   tgcactcatc accagaaact actattgagg tttgttctaa agagcatgaa gttgtaattg 120
   ctctagggag caatgtagga gacagattag ataactttaa ccaagctctg cagctaatga 180
   agaaattagg agtaaatatc 200
<210> 794
   <211> 200
   <212> DNA
   <213> Kochia scoparia
<400> 794
   gatatttact cctaatttct tcattagctg cagagcttgg ttaaagttat ctaatctgtc 60
   tcctacattg ctccctagag caattacaac ttcatgctct ttagaacaaa cctcaatagt 120
   agtttctggt gatgagtgca aaaaagcaag acttgaagct gcaaaagtac atacgtaatc 180
   tttaaaatcc atttgaatta 200
<210> 795
   <211> 200
   <212> DNA
   <213> Kochia scoparia
<400> 795
   aatgaagaaa ttaggagtaa atatcacaag gcatggctgc ttatacgaga cagatcctgc 60
   ctatgtgact gatcaaccaa agtttcttaa ctctgcagtg aggggcttca caaaacttgg 120
   gcctctagaa ctattaggga tgctgaagaa aattgagaag gatatgggtc gaactaatgg 180
   aataagatat ggccctaggc 200
<210> 796
   <211> 200
   <212> DNA
   <213> Kochia scoparia
<400> 796
   gcctagggcc atatcttatt ccattagttc gacccatatc cttctcaatt ttcttcagca 60
   tccctaatag ttctagaggc ccaagttttg tgaagcccct cactgcagag ttaagaaact 120
   ttggttgatc agtcacatag gcaggatctg tctcgtataa gcagccatgc cttgtgatat 180
   ttactcctaa tttcttcatt 200
<210> 797
   <211> 200
   <212> DNA
   <213> Kochia scoparia
<400> 797
   aatggaataa gatatggccc taggccaatt gacttagaca ttctttttta tgggaagttt 60
   agggtaagct ctgagaaact tacagttccg catgaaagga tatgggaaag accctttgtg 120
   atggcaccac taattgatat aattggatcg gatgtagaaa atgacactgt tgctgcatgg 180
   cattcattat caaaattttc 200
<210> 798
   <211> 200
   <212> DNA
   <213> Kochia scoparia
<400> 798
   gaaaattttg ataatgaatg ccatgcagca acagtgtcat tttctacatc cgatccaatt    60
   atatcaatta gtggtgccat cacaaagggt ctttcccata tcctttcatg cggaactgta   120
   agtttctcag agcttaccct aaacttccca taaaaaagaa tgtctaagtc aattggccta   180
   gggccatatc ttattccatt                                               200
<210> 799
   <211> 200
   <212> DNA
   <213> Kochia scoparia
<400> 799
   catggcattc attatcaaaa ttttctggtg gactatttga agcgtgggat aaacttggtg    60
   gagattcact cattgggaag gacggaatga gtagggtttt gccagttggg aaccacttgt   120
   gggattggtc gtgcaaaacc tctgtaatgg gagtcttgaa tttgactccg gacagcttta   180
   gtgatggagg aaagtttcta                                               200
<210> 800
   <211> 200
   <212> DNA
   <213> Kochia scoparia
<400> 800
   tagaaacttt cctccatcac taaagctgtc cggagtcaaa ttcaagactc ccattacaga    60
   ggttttgcac gaccaatccc acaagtggtt cccaactggc aaaaccctac tcattccgtc   120
   cttcccaatg agtgaatctc caccaagttt atcccacgct tcaaatagtc caccagaaaa   180
   ttttgataat gaatgccatg                                               200
<210> 801
   <211> 200
   <212> DNA
   <213> Kochia scoparia
<400> 801
   ctttagtgat ggaggaaagt ttctacctgt agaaaatgca gtttctcagg ttcgtcaaat 60
   gatctcagaa ggtgctgaca taattgatat tggagcgcaa tcaacaaggc ccatggcaac 120
   taggatttct gctgaggaag agctggaaag actagtccct gttttagaag gtgtcaagga 180
   tgttatcgag gaagaaggaa 200
<210> 802
   <211> 200
   <212> DNA
   <213> Kochia scoparia
<400> 802
   ttccttcttc ctcgataaca tccttgacac cttctaaaac agggactagt ctttccagct 60
   cttcctcagc agaaatccta gttgccatgg gccttgttga ttgcgctcca atatcaatta 120
   tgtcagcacc ttctgagatc atttgacgaa cctgagaaac tgcattttct acaggtagaa 180
   actttcctcc atcactaaag 200
<210> 803
   <211> 200
   <212> DNA
   <213> Kochia scoparia
<400> 803
   aaggatgtta tcgaggaaga aggaagaatg ttatcagtgg atacatttta ctcaaaagtt 60
   gcttctgaag ctgtcaataa gggagcacat atggtgaatg atgtttcgag tggaaagcta 120
   gatcctgaga tgttcaatgt tgttgcaggg cttaaagtgc cttatatagc aatgcacatg 180
   cgaggtgatc cttcttcaat 200
<210> 804
   <211> 200
   <212> DNA
   <213> Kochia scoparia
<400> 804
   attgaagaag gatcacctcg catgtgcatt gctatataag gcactttaag ccctgcaaca 60
   acattgaaca tctcaggatc tagctttcca ctcgaaacat cattcaccat atgtgctccc 120
   ttattgacag cttcagaagc aacttttgag taaaatgtat ccactgataa cattcttcct 180
   tcttcctcga taacatcctt 200
<210> 805
   <211> 200
   <212> DNA
   <213> Kochia scoparia
<400> 805
   acatgcgagg tgatccttct tcaatgcaaa atgctgataa tttgacatac aatgatgttt 60
   gtaaggaggt ggctttagag ttgagctcta ggattacaga tgcagaatta tcaggtattc 120
   ctgcttggag gatagttatt gatcctggca ttggattttc caagaataca aagcaaaatt 180
   tggaaattct cacagggttg 200
<210> 806
   <211> 200
   <212> DNA
   <213> Kochia scoparia
<400> 806
   caaccctgtg agaatttcca aattttgctt tgtattcttg gaaaatccaa tgccaggatc 60
   aataactatc ctccaagcag gaatacctga taattctgca tctgtaatcc tagagctcaa 120
   ctctaaagcc acctccttac aaacatcatt gtatgtcaaa ttatcagcat tttgcattga 180
   agaaggatca cctcgcatgt 200
<210> 807
   <211> 200
   <212> DNA
   <213> Kochia scoparia
<400> 807
   aaatttggaa attctcacag ggttgaaaag aattcggcaa gaaatagcaa aaaagagttt 60
   ggctgtggct catggtccct tactgattgg accttcaaga aagaggtttt tgggtgagat 120
   ctgcaatcgt cctgtagcat ctgatagaga tccggcaact gttgcttcta tcaccgctgg 180
   agttttgggt ggtgcaaaca 200
<210> 808
   <211> 200
   <212> DNA
   <213> Kochia scoparia
<400> 808
   tgtttgcacc acccaaaact ccagcggtga tagaagcaac agttgccgga tctctatcag 60
   atgctacagg acgattgcag atctcaccca aaaacctctt tcttgaaggt ccaatcagta 120
   agggaccatg agccacagcc aaactctttt ttgctatttc ttgccgaatt cttttcaacc 180
   ctgtgagaat ttccaaattt 200
<210> 809
   <211> 200
   <212> DNA
   <213> Kochia scoparia
<400> 809
   gctggagttt tgggtggtgc aaacattgta agagtacata atgttcggga tagccttgat 60
   ggtgtcaagc tatgtgatgc aatgcttgaa tatcagtata actgaagatt cttagattct 120
   tagattctta gattctaaga gttgatttta tagattattg tctttgttgt gtgccttcgg 180
   cagatatgaa taatatgatt 200
<210> 810
   <211> 200
   <212> DNA
   <213> Kochia scoparia
<400> 810
   aatcatatta ttcatatctg ccgaaggcac acaacaaaga caataatcta taaaatcaac 60
   tcttagaatc taagaatcta agaatctaag aatcttcagt tatactgata ttcaagcatt 120
   gcatcacata gcttgacacc atcaaggcta tcccgaacat tatgtactct tacaatgttt 180
   gcaccaccca aaactccagc 200
<210> 811
   <211> 200
   <212> DNA
   <213> Kochia scoparia
<400> 811
   ttcggcagat atgaataata tgattgtaat tttgttgctt atgattttct tcctaccttt 60
   cttttatcat agaatttaga ttaaaaaatg tacttgaatt tcataaatgg tgaaataaag 120
   tttgtcggtg cttcatgcaa tacgactttt ctagtttgca ttgctctatc ataggggctg 180
   taagcagatt agcccagtca 200
<210> 812
   <211> 200
   <212> DNA
   <213> Kochia scoparia
<400> 812
   tgactgggct aatctgctta cagcccctat gatagagcaa tgcaaactag aaaagtcgta 60
   ttgcatgaag caccgacaaa ctttatttca ccatttatga aattcaagta cattttttaa 120
   tctaaattct atgataaaag aaaggtagga agaaaatcat aagcaacaaa attacaatca 180
   tattattcat atctgccgaa 200
<210> 813
   <211> 200
   <212> DNA
   <213> Kochia scoparia
<400> 813
   ggctgtaagc agattagccc agtcaattca cattagattt agacggccgg tgcaaaaaca    60
   gcataatgtt gctaacgctg tttatacatt taaactatat gatctctaag agcaaaatac   120
   aaaataatac aatgtgtttc atgaagatgt ccaatcattt ctccctcaaa aaaagatcgt   180
   gatgaacaat caggtacttt                                               200
<210> 814
   <211> 200
   <212> DNA
   <213> Kochia scoparia
<400> 814
   aaagtacctg attgttcatc acgatctttt tttgagggag aaatgattgg acatcttcat    60
   gaaacacatt gtattatttt gtattttgct cttagagatc atatagttta aatgtataaa   120
   cagcgttagc aacattatgc tgtttttgca ccggccgtct aaatctaatg tgaattgact   180
   gggctaatct gcttacagcc                                               200
<210> 815
   <211> 200
   <212> DNA
   <213> Kochia scoparia
<400> 815
   atcgtgatga acaatcaggt actttgattt tgttgtagaa cctcagcttc agccctagcc    60
   atgggatcat ctttttctcc ttctcgctcc tcagccaatt tctttctgtg catctcctct   120
   gcagcttcta ttgaagcttt gtgctcagcc tggagacgct gcatttcctc ttccatctgc   180
   tgtctttcga accatgtatc                                               200
<210> 816
   <211> 200
   <212> DNA
   <213> Kochia scoparia
<400> 816
   gatacatggt tcgaaagaca gcagatggaa gaggaaatgc agcgtctcca ggctgagcac    60
   aaagcttcaa tagaagctgc agaggagatg cacagaaaga aattggctga ggagcgagaa   120
   ggagaaaaag atgatcccat ggctagggct gaagctgagg ttctacaaca aaatcaaagt   180
   acctgattgt tcatcacgat                                               200
<210> 817
   <211> 200
   <212> DNA
   <213> Kochia scoparia
<400> 817
   tctgctgtct ttcgaaccat gtatcagcat ccgcccaaac tgcagaaata atttaaacaa   60
   gttaatcata cggagtgtgc agcatggcaa attggcaata agatggtgca agtaacaagc  120
   tcaaatgatc caccatagcc aaattcctaa ccattggact gtgtcatata ctcatatcag  180
   ctagtagaaa cattctcacc                                              200
<210> 818
   <211> 200
   <212> DNA
   <213> Kochia scoparia
<400> 818
   ggtgagaatg tttctactag ctgatatgag tatatgacac agtccaatgg ttaggaattt   60
   ggctatggtg gatcatttga gcttgttact tgcaccatct tattgccaat ttgccatgct  120
   gcacactccg tatgattaac ttgtttaaat tatttctgca gtttgggcgg atgctgatac  180
   atggttcgaa agacagcaga                                              200
<210> 819
   <211> 200
   <212> DNA
   <213> Kochia scoparia
<400> 819
   atcagctagt agaaacattc tcaccataac cctaactatt cgaggataag tgataccatt   60
   caacaatagt aaagatcaaa tcaggtatta ctagacctat caaagtaata cggagtagtt  120
   gttattgtta cagagttagg aagaatccta ttttcccatt tctcctatca atcctaaact  180
   tttgtgactg aactatcgac                                              200
<210> 820
   <211> 200
   <212> DNA
   <213> Kochia scoparia
<400> 820
   gtcgatagtt cagtcacaaa agtttaggat tgataggaga aatgggaaaa taggattctt 60
   cctaactctg taacaataac aactactccg tattactttg ataggtctag taatacctga 120
   tttgatcttt actattgttg aatggtatca cttatcctcg aatagttagg gttatggtga 180
   gaatgtttct actagctgat 200
<210> 821
   <211> 200
   <212> DNA
   <213> Kochia scoparia
<400> 821
   aaacttttgt gactgaacta tcgacatata gtgtgaagaa catgaatggc agaagtcaca 60
   actctgattc ctgagtcctg actataaact attggatgac tgtgatacat cattagtcct 120
   ttaagcattg agttactcta ttctacatag ttaaattata tagattccaa gatccgaatc 180
   catgaattat caattaccat 200
<210> 822
   <211> 200
   <212> DNA
   <213> Kochia scoparia
<400> 822
   atggtaattg ataattcatg gattcggatc ttggaatcta tataatttaa ctatgtagaa 60
   tagagtaact caatgcttaa aggactaatg atgtatcaca gtcatccaat agtttatagt 120
   caggactcag gaatcagagt tgtgacttct gccattcatg ttcttcacac tatatgtcga 180
   tagttcagtc acaaaagttt 200
<210> 823
   <211> 200
   <212> DNA
   <213> Kochia scoparia
<400> 823
   gaatccatga attatcaatt accataaatt gatcttcttc ctttgagttt tgtcaacagt 60
   tcctactccg aaatatgtct taaatctaga agcaatagac acttatcaat gatacagcaa 120
   tagacgaatt catctaccca aggcagcatc aacaacagct gccgtttgcg ataatcgaaa 180
   ctatcatagt gtccttttca 200
<210> 824
   <211> 200
   <212> DNA
   <213> Kochia scoparia
<400> 824
   tgaaaaggac actatgatag tttcgattat cgcaaacggc agctgttgtt gatgctgcct 60
   tgggtagatg aattcgtcta ttgctgtatc attgataagt gtctattgct tctagattta 120
   agacatattt cggagtagga actgttgaca aaactcaaag gaagaagatc aatttatggt 180
   aattgataat tcatggattc 200
<210> 825
   <211> 200
   <212> DNA
   <213> Kochia scoparia
<400> 825
   cgaaactatc atagtgtcct tttcactatt agtgcagatg ttatctaggg tctaaacctc 60
   tatgtttcta cattcctgga aaatcttatt ctgtactgca taatcctctc acaaaaaatt 120
   cgccaatgaa tgacaaatta gctatccaga attgcagatt gattgaatca aattgatcat 180
   ggcagcagca caattgcagc 200
<210> 826
   <211> 200
   <212> DNA
   <213> Kochia scoparia
<400> 826
   gctgcaattg tgctgctgcc atgatcaatt tgattcaatc aatctgcaat tctggatagc 60
   taatttgtca ttcattggcg aattttttgt gagaggatta tgcagtacag aataagattt 120
   tccaggaatg tagaaacata gaggtttaga ccctagataa catctgcact aatagtgaaa 180
   aggacactat gatagtttcg 200
<210> 827
   <211> 200
   <212> DNA
   <213> Kochia scoparia
<400> 827
   atcatggcag cagcacaatt gcagcagtta tcaattgaag cacaatatta cctctcaatc 60
   ataacaatca aagcacagaa attccaatca ataactccac aatatagtat caatctctaa 120
   ctcagaaact aaacagaagc aaaaacccat tcaatcaaag caacagtaaa attagggttt 180
   gaattagaaa gaaagagagg 200
<210> 828
   <211> 200
   <212> DNA
   <213> Kochia scoparia
<400> 828
   cctctctttc tttctaattc aaaccctaat tttactgttg ctttgattga atgggttttt   60
   gcttctgttt agtttctgag ttagagattg atactatatt gtggagttat tgattggaat  120
   ttctgtgctt tgattgttat gattgagagg taatattgtg cttcaattga taactgctgc  180
   aattgtgctg ctgccatgat                                              200
<210> 829
   <211> 200
   <212> DNA
   <213> Kochia scoparia
<400> 829
   ggtttgaatt agaaagaaag agagggaaga gaaacatact gggttgcgca gcccatccct   60
   gttcaccacc cttaatcttc tcgggaagag cgtaattgac agtgagaaca cgaccgtaaa  120
   gctcagcacc atccatgtta tccatggcag aagaagcgtc gtctctctcc aagaaagtga  180
   cgaaaccgaa agaacgatgc                                              200
<210> 830
   <211> 200
   <212> DNA
   <213> Kochia scoparia
<400> 830
   gcatcgttct ttcggtttcg tcactttctt ggagagagac gacgcttctt ctgccatgga   60
   taacatggat ggtgctgagc tttacggtcg tgttctcact gtcaattacg ctcttcccga  120
   gaagattaag ggtggtgaac agggatgggc tgcgcaaccc agtatgtttc tcttccctct  180
   ctttctttct aattcaaacc                                              200
<210> 831
   <211> 200
   <212> DNA
   <213> Lolium multiflorum
<400> 831
   acgaaagctt tgaactctca agtggtgcaa catagataga ttatatatat ttcgaatata   60
   attcgaatga acataaaaat tacatataaa aactttaaaa caaacttaaa ctacttcttc  120
   ttcctcgatg gccccgcctc atcgtcgggg cggcgacgct tccggctcgt cacctcctcg  180
   tcggaggaag ttgggtcctc                                              200
<210> 832
   <211> 200
   <212> DNA
   <213> Lolium multiflorum
<400> 832
   gaggacccaa cttcctccga cgaggaggtg acgagccgga agcgtcgccg ccccgacgat   60
   gaggcggggc catcgaggaa gaagaagtag tttaagtttg ttttaaagtt tttatatgta  120
   atttttatgt tcattcgaat tatattcgaa atatatataa tctatctatg ttgcaccact  180
   tgagagttca aagctttcgt                                              200
<210> 833
   <211> 200
   <212> DNA
   <213> Lolium multiflorum
<400> 833
   cctcgtcgga ggaagttggg tcctcctcct cgtcagtgtc ctcagcctct tcgtcgtcct   60
   cctcctcttc ctcggcctct tcgtcggcct gctccttggc ctcttcgtcc tcctcctcgt  120
   cgtcatccca tgcgtcctcc tcctcgtcgt catcccattc gtccttgctg gccggcgggg  180
   gggaatcgtc gctgctggac                                              200
<210> 834
   <211> 200
   <212> DNA
   <213> Lolium multiflorum
<400> 834
   gtccagcagc gacgattccc ccccgccggc cagcaaggac gaatgggatg acgacgagga   60
   ggaggacgca tgggatgacg acgaggagga ggacgaagag gccaaggagc aggccgacga  120
   agaggccgag gaagaggagg aggacgacga agaggctgag gacactgacg aggaggagga  180
   cccaacttcc tccgacgagg                                              200
<210> 835
   <211> 200
   <212> DNA
   <213> Lolium multiflorum
<400> 835
   cgggggggaa tcgtcgctgc tggacgatgc agctttatcc caccaatggc gccatccagg   60
   cggcttcccc tcgctgtcgg tgtccgatgg ccaagagaga tcgctcatgg ttgacggagg  120
   atggtgagga gagaacagat gaatggcgtc ggccgtcgta aaccgtatat gtaggtctct  180
   cgacgaagag agcggcggtt                                              200
<210> 836
   <211> 200
   <212> DNA
   <213> Lolium multiflorum
<400> 836
   aaccgccgct ctcttcgtcg agagacctac atatacggtt tacgacggcc gacgccattc   60
   atctgttctc tcctcaccat cctccgtcaa ccatgagcga tctctcttgg ccatcggaca  120
   ccgacagcga ggggaagccg cctggatggc gccattggtg ggataaagct gcatcgtcca  180
   gcagcgacga ttcccccccg                                              200
<210> 837
   <211> 200
   <212> DNA
   <213> Lolium multiflorum
<400> 837
   tctctcgacg aagagagcgg cggttgctct tccgcggagt tcgtgctcca ttacagcggt   60
   tctcgcatcg aggccacttc ggccgttccc gacgagtcgt ttgggctctc cagaccactt  120
   tgcgacggtt caatgcgtcg agggcactcc gacgattgcc cttcccggtg actgcgccgt  180
   cgctatgcac gcggtgtgtg                                              200
<210> 838
   <211> 200
   <212> DNA
   <213> Lolium multiflorum
<400> 838
   cacacaccgc gtgcatagcg acggcgcagt caccgggaag ggcaatcgtc ggagtgccct   60
   cgacgcattg aaccgtcgca aagtggtctg gagagcccaa acgactcgtc gggaacggcc  120
   gaagtggcct cgatgcgaga accgctgtaa tggagcacga actccgcgga agagcaaccg  180
   ccgctctctt cgtcgagaga                                              200
<210> 839
   <211> 200
   <212> DNA
   <213> Lolium multiflorum
<400> 839
   gccgtcgcta tgcacgcggt gtgtgcgcaa ccatgggctc gcggctggga aaatgggcct 60
   ccccaggcca caaaatacat ccatccggcg ataactaacg ccggatttcg gcctgggagc 120
   cccaacggct gggatgctct tagcccgtgg aacacattcc cgaatcggta ccgacggtct 180
   catctctctc gccctgatct 200
<210> 840
   <211> 200
   <212> DNA
   <213> Lolium multiflorum
<400> 840
   agatcagggc gagagagatg agaccgtcgg taccgattcg ggaatgtgtt ccacgggcta 60
   agagcatccc agccgttggg gctcccaggc cgaaatccgg cgttagttat cgccggatgg 120
   atgtattttg tggcctgggg aggcccattt tcccagccgc gagcccatgg ttgcgcacac 180
   accgcgtgca tagcgacggc 200
<210> 841
   <211> 200
   <212> DNA
   <213> Lolium multiflorum
<400> 841
   ggtctcatct ctctcgccct gatctatctc tcttccgcga tctggaaggg ccgacggcgt 60
   ggcggcgcaa ccacgggcgc tgatgggcgg cggcgcgcag tctgttaagc aggacgcctg 120
   cttctcgctc cgtcctccct ctccaaccga gcggcacgag gtagacccgg agaccccaac 180
   tccgtcctag cccagtggcg 200
<210> 842
   <211> 200
   <212> DNA
   <213> Lolium multiflorum
<400> 842
   cgccactggg ctaggacgga gttggggtct ccgggtctac ctcgtgccgc tcggttggag 60
   agggaggacg gagcgagaag caggcgtcct gcttaacaga ctgcgcgccg ccgcccatca 120
   gcgcccgtgg ttgcgccgcc acgccgtcgg cccttccaga tcgcggaaga gagatagatc 180
   agggcgagag agatgagacc 200
<210> 843
   <211> 200
   <212> DNA
   <213> Lolium multiflorum
<400> 843
   ccaactccgt cctagcccag tggcgcccgt gaggcgtcat ctccggcgcc ctctccatct 60
   ccgtctgctt cgcacttgta tgcacatact cttcctcctt accccagttt cttggtcttg 120
   gcttgtttga ttacgatttc tagggttctt ggcatgctcg gtagttgcag atttgtcttc 180
   tgttgggtgt ttgtttccat 200
<210> 844
   <211> 200
   <212> DNA
   <213> Lolium multiflorum
<400> 844
   atggaaacaa acacccaaca gaagacaaat ctgcaactac cgagcatgcc aagaacccta 60
   gaaatcgtaa tcaaacaagc caagaccaag aaactggggt aaggaggaag agtatgtgca 120
   tacaagtgcg aagcagacgg agatggagag ggcgccggag atgacgcctc acgggcgcca 180
   ctgggctagg acggagttgg 200
<210> 845
   <211> 200
   <212> DNA
   <213> Lolium multiflorum
<400> 845
   tcttctgttg ggtgtttgtt tccatggcct tggctgcatc catcgtttcg tattatgtag 60
   ttgtttgttt ccatgccatt gcttgtatcc atcaattgga aagggaagaa agggctacag 120
   ggatattagg aattagatca gtccttccta gctgattttc tctgacaggt tgttagtctc 180
   gcgcatgtgg tggacttctt 200
<210> 846
   <211> 200
   <212> DNA
   <213> Lolium multiflorum
<400> 846
   aagaagtcca ccacatgcgc gagactaaca acctgtcaga gaaaatcagc taggaaggac 60
   tgatctaatt cctaatatcc ctgtagccct ttcttccctt tccaattgat ggatacaagc 120
   aatggcatgg aaacaaacaa ctacataata cgaaacgatg gatgcagcca aggccatgga 180
   aacaaacacc caacagaaga 200
<210> 847
   <211> 200
   <212> DNA
   <213> Lolium multiflorum
<400> 847
   gtctcgcgca tgtggtggac ttcttgtgct tttggctagc tccaagtctg atgtgtagct   60
   gctttctgag attttccaca taaacgttgc tatggctagt aagttgtggc taactaaatc  120
   cttgggcaca agctagtaat ctgaatggca tatccgcccc catgccttta attgttgcct  180
   aacgatgggc taggttctct                                              200
<210> 848
   <211> 200
   <212> DNA
   <213> Lolium multiflorum
<400> 848
   agagaaccta gcccatcgtt aggcaacaat taaaggcatg ggggcggata tgccattcag   60
   attactagct tgtgcccaag gatttagtta gccacaactt actagccata gcaacgttta  120
   tgtggaaaat ctcagaaagc agctacacat cagacttgga gctagccaaa agcacaagaa  180
   gtccaccaca tgcgcgagac                                              200
<210> 849
   <211> 200
   <212> DNA
   <213> Lolium multiflorum
<400> 849
   tgcctaacga tgggctaggt tctcttattt attcattttt gttaaaagaa gaacttgggc   60
   atgctaattc agcaaaatag aaaaaatatg tgttgtttct gtatatagag gttcttggtt  120
   ttcccttgcc gattctgacc gatttgcggg tttttttttt ttttgtcttt cctcatcgtc  180
   aggcaagaag agcaagatgt                                              200
<210> 850
   <211> 200
   <212> DNA
   <213> Lolium multiflorum
<400> 850
   acatcttgct cttcttgcct gacgatgagg aaagacaaaa aaaaaaaaaa cccgcaaatc 60
   ggtcagaatc ggcaagggaa aaccaagaac ctctatatac agaaacaaca catatttttt 120
   ctattttgct gaattagcat gcccaagttc ttcttttaac aaaaatgaat aaataagaga 180
   acctagccca tcgttaggca 200
<210> 851
   <211> 200
   <212> DNA
   <213> Lolium multiflorum
<400> 851
   tcgtcaggca agaagagcaa gatgtgacag aggatttaag cagcagcaga actagacctg 60
   gctcctatgc tctaagatgc ttcgtgccaa ggacacattc aggaagatgt actcagctgc 120
   caagggctac tactgcggag ggctagcgtc tcctaatgcc ttctcaggta gcttgcccaa 180
   agttccccaa taataactag 200
<210> 852
   <211> 200
   <212> DNA
   <213> Lolium multiflorum
<400> 852
   ctagttatta ttggggaact ttgggcaagc tacctgagaa ggcattagga gacgctagcc 60
   ctccgcagta gtagcccttg gcagctgagt acatcttcct gaatgtgtcc ttggcacgaa 120
   gcatcttaga gcataggagc caggtctagt tctgctgctg cttaaatcct ctgtcacatc 180
   ttgctcttct tgcctgacga 200
<210> 853
   <211> 200
   <212> DNA
   <213> Lolium multiflorum
<400> 853
   cccaaagttc cccaataata actagggaac atctcttttc cacctctttc acattccaac 60
   caaagacagt cctaactcgt ttcccctctg aacataccag cattggatct tcggcatcct 120
   ctcagctcct caagaaaacc accaggccac gctaacctgt tcagggtccg ttcatacacg 180
   caatgcgccc tcaccaacga 200
<210> 854
   <211> 200
   <212> DNA
   <213> Lolium multiflorum
<400> 854
   tcgttggtga gggcgcattg cgtgtatgaa cggaccctga acaggttagc gtggcctggt   60
   ggttttcttg aggagctgag aggatgccga agatccaatg ctggtatgtt cagaggggaa  120
   acgagttagg actgtctttg gttggaatgt gaaagaggtg gaaaagagat gttccctagt  180
   tattattggg gaactttggg                                               200
<210> 855
   <211> 200
   <212> DNA
   <213> Lolium multiflorum
<400> 855
   acacgcaatg cgccctcacc aacgattccg ccgaccagga ggtcgtgatt gccctgggca   60
   gcaacgtggg agacagggtc agcatgttcg acagggcgct gcggctgatg aggagctcgg  120
   gcatcaaggt caccaggcac gcctgcctgt acgagaccgc cccagcctac gtgaccgacc  180
   agccgcggtt cctcaactct                                               200
<210> 856
   <211> 200
   <212> DNA
   <213> Lolium multiflorum
<400> 856
   agagttgagg aaccgcggct ggtcggtcac gtaggctggg gcggtctcgt acaggcaggc   60
   gtgcctggtg accttgatgc ccgagctcct catcagccgc agcgccctgt cgaacatgct  120
   gaccctgtct cccacgttgc tgcccagggc aatcacgacc tcctggtcgg cggaatcgtt  180
   ggtgagggcg cattgcgtgt                                               200
<210> 857
   <211> 200
   <212> DNA
   <213> Lolium multiflorum
<400> 857
   cgaccagccg cggttcctca actctgccgt taggggcacg acgaagctgg ggcctcacga   60
   gttgctcagg aagctcaagg agatcgagaa ggatatagga cgtaccgccg gggtaaggta  120
   cggcccgagg ccgatcgatc tggacattct tctgtatggg gactcccgga tcaagaccga  180
   gtctctgatt gtgccgcatg                                               200
<210> 858
   <211> 200
   <212> DNA
   <213> Lolium multiflorum
<400> 858
   catgcggcac aatcagagac tcggtcttga tccgggagtc cccatacaga agaatgtcca 60
   gatcgatcgg cctcgggccg taccttaccc cggcggtacg tcctatatcc ttctcgatct 120
   ccttgagctt cctgagcaac tcgtgaggcc ccagcttcgt cgtgccccta acggcagagt 180
   tgaggaaccg cggctggtcg 200
<210> 859
   <211> 200
   <212> DNA
   <213> Lolium multiflorum
<400> 859
   accgagtctc tgattgtgcc gcatgaacgc atccatgaga gaccgttcgt cttggcgcct 60
   cttgttgacc tcctgggttc gtcggctgag gacggtatgg agaaaagctg gcactctctc 120
   tcgaagtgca gtggcgggtt ctttgatttg tggaacaagc tcggcggtga atctgttgtt 180
   ggaacagaag gcattaaaag 200
<210> 860
   <211> 200
   <212> DNA
   <213> Lolium multiflorum
<400> 860
   cttttaatgc cttctgttcc aacaacagat tcaccgccga gcttgttcca caaatcaaag 60
   aacccgccac tgcacttcga gagagagtgc cagcttttct ccataccgtc ctcagccgac 120
   gaacccagga ggtcaacaag aggcgccaag acgaacggtc tctcatggat gcgttcatgc 180
   ggcacaatca gagactcggt 200
<210> 861
   <211> 200
   <212> DNA
   <213> Lolium multiflorum
<400> 861
   ttgttggaac agaaggcatt aaaagggtca tgtctgttgg aaatacgctg ttggactggc 60
   gtcagaggac ccttgttatg ggggtgctta acctcacgcc agacagcttc agcgacggag 120
   gtaagtttca agaagtggaa gctgccattt cccaggccag gttgttgata tcagaaggtg 180
   ctgacataat tgatattggt 200
<210> 862
   <211> 200
   <212> DNA
   <213> Lolium multiflorum
<400> 862
   accaatatca attatgtcag caccttctga tatcaacaac ctggcctggg aaatggcagc 60
   ttccacttct tgaaacttac ctccgtcgct gaagctgtct ggcgtgaggt taagcacccc 120
   cataacaagg gtcctctgac gccagtccaa cagcgtattt ccaacagaca tgaccctttt 180
   aatgccttct gttccaacaa 200
<210> 863
   <211> 200
   <212> DNA
   <213> Lolium multiflorum
<400> 863
   aggtgctgac ataattgata ttggtgccca gtccaccagg ccctttgcaa ggaggctatc 60
   cgcagaagaa gagcttgaga ggctggtccc tgttctggat gctatcatga aactcccaga 120
   gatggaaggg aagttgcttt cggtagacac gttctacgcg caagtcgctg ctgaagctgt 180
   aaaaagagga gccaccatga 200
<210> 864
   <211> 200
   <212> DNA
   <213> Lolium multiflorum
<400> 864
   tcatggtggc tcctcttttt acagcttcag cagcgacttg cgcgtagaac gtgtctaccg 60
   aaagcaactt cccttccatc tctgggagtt tcatgatagc atccagaaca gggaccagcc 120
   tctcaagctc ttcttctgcg gatagcctcc ttgcaaaggg cctggtggac tgggcaccaa 180
   tatcaattat gtcagcacct 200
<210> 865
   <211> 200
   <212> DNA
   <213> Lolium multiflorum
<400> 865
   gctgtaaaaa gaggagccac catgatcaat gatgtatctg gtgggcagct tgacccaagt 60
   attcttcaag ttgttgctga actgggagtt ccgtatgtta ccatgcacat gagaggcgat 120
   ccatcaacta tgcagaatga acagaatcta cagtatgatg atgtctgcaa agaagttgct 180
   tctgagttat acgcccggtt 200
<210> 866
   <211> 200
   <212> DNA
   <213> Lolium multiflorum
<400> 866
   aaccgggcgt ataactcaga agcaacttct ttgcagacat catcatactg tagattctgt 60
   tcattctgca tagttgatgg atcgcctctc atgtgcatgg taacatacgg aactcccagt 120
   tcagcaacaa cttgaagaat acttgggtca agctgcccac cagatacatc attgatcatg 180
   gtggctcctc tttttacagc 200
<210> 867
   <211> 200
   <212> DNA
   <213> Lolium multiflorum
<400> 867
   ttgcttctga gttatacgcc cggttgagag cagcagagct ttctggaatt cctttgtgga 60
   ggattattct tgaccctggc attgggtttt ctaagaaatc cacacagaat attgaagtaa 120
   tcgcgggttt ggaatccatt agagaagaga tgggtaaaat gagcctaggt gcttcacatg 180
   tgccaatatt acttggaccc 200
<210> 868
   <211> 200
   <212> DNA
   <213> Lolium multiflorum
<400> 868
   gggtccaagt aatattggca catgtgaagc acctaggctc attttaccca tctcttctct 60
   aatggattcc aaacccgcga ttacttcaat attctgtgtg gatttcttag aaaacccaat 120
   gccagggtca agaataatcc tccacaaagg aattccagaa agctctgctg ctctcaaccg 180
   ggcgtataac tcagaagcaa 200
<210> 869
   <211> 200
   <212> DNA
   <213> Lolium multiflorum
<400> 869
   acatgtgcca atattacttg gaccctcgag gaaaagtttc ctaggtgaaa tatgtagtcg 60
   cgccgatcca gttcagagag atgctgctac tgcttctgcc gttacaattg cgatcttgaa 120
   tggcgctaat atagtaaggg tccataatgt tagatacaat gtggatgctg caaaggtctc 180
   tgatgcatta ctcaagtaca 200
<210> 870
   <211> 200
   <212> DNA
   <213> Lolium multiflorum
<400> 870
   tgtacttgag taatgcatca gagacctttg cagcatccac attgtatcta acattatgga 60
   cccttactat attagcgcca ttcaagatcg caattgtaac ggcagaagca gtagcagcat 120
   ctctctgaac tggatcggcg cgactacata tttcacctag gaaacttttc ctcgagggtc 180
   caagtaatat tggcacatgt 200
<210> 871
   <211> 200
   <212> DNA
   <213> Lolium multiflorum
<400> 871
   gtctctgatg cattactcaa gtacagaaga aaataataga aagtatacag cccggataga 60
   agccatacca gttgccagtt ttgtgcaagg gaatgctgat gtggagctca aaccatatgg 120
   ggtgtatcat cacttaataa gatcaaagca tgcgtgaata actattacag tgaacaagag 180
   atatacagtt ttttcgtgcg 200
<210> 872
   <211> 200
   <212> DNA
   <213> Lolium multiflorum
<400> 872
   cgcacgaaaa aactgtatat ctcttgttca ctgtaatagt tattcacgca tgctttgatc 60
   ttattaagtg atgatacacc ccatatggtt tgagctccac atcagcattc ccttgcacaa 120
   aactggcaac tggtatggct tctatccggg ctgtatactt tctattattt tcttctgtac 180
   ttgagtaatg catcagagac 200
<210> 873
   <211> 200
   <212> DNA
   <213> Lolium multiflorum
<400> 873
   aagagatata cagttttttc gtgcgcctcc tccttcgtag aatcttcaaa gaagcaaagg 60
   taacacagac ctaggcctct ggggttggta tatgatgtaa ccagttttgt gagggtttaa 120
   taattatcta atagtgagac ctaagagtaa gacctttata atatactttg cttggaattt 180
   cctacttgag agcatcattt 200
<210> 874
   <211> 200
   <212> DNA
   <213> Lolium multiflorum
<400> 874
   aaatgatgct ctcaagtagg aaattccaag caaagtatat tataaaggtc ttactcttag 60
   gtctcactat tagataatta ttaaaccctc acaaaactgg ttacatcata taccaacccc 120
   agaggcctag gtctgtgtta cctttgcttc tttgaagatt ctacgaagga ggaggcgcac 180
   gaaaaaactg tatatctctt 200
<210> 875
   <211> 200
   <212> DNA
   <213> Lolium multiflorum
<400> 875
   agctcctcaa gaaaaccacc aggccacgct aacctgttca gggtccgttc atacacgcaa 60
   tgcgccctca ccaacgattc cgccgaccag gaggtcgtga ttgccctggg cagcaacgtg 120
   ggagacaggg tcagcatgtt cgacagggcg ctgcggctga tgaggagctc gggcatcaag 180
   gtcaccaggc acgcctgcct 200
<210> 876
   <211> 200
   <212> DNA
   <213> Lolium multiflorum
<400> 876
   aggcaggcgt gcctggtgac cttgatgccc gagctcctca tcagccgcag cgccctgtcg 60
   aacatgctga ccctgtctcc cacgttgctg cccagggcaa tcacgacctc ctggtcggcg 120
   gaatcgttgg tgagggcgca ttgcgtgtat gaacggaccc tgaacaggtt agcgtggcct 180
   ggtggttttc ttgaggagct 200
<210> 877
   <211> 200
   <212> DNA
   <213> Lolium multiflorum
<400> 877
   tcaaggtcac caggcacgcc tgcctgtacg agaccgcccc agcctacgtg accgaccagc   60
   cgcggttcct caactctgcc gttaggggca cgacgaagct ggggcctcac gagttgctca  120
   ggaagctcaa ggagatcgag aaggatatag gacgtaccgc cggggtaagg tacggcccga  180
   ggccgatcga tctggacatt                                              200
<210> 878
   <211> 200
   <212> DNA
   <213> Lolium multiflorum
<400> 878
   aatgtccaga tcgatcggcc tcgggccgta ccttaccccg gcggtacgtc ctatatcctt   60
   ctcgatctcc ttgagcttcc tgagcaactc gtgaggcccc agcttcgtcg tgcccctaac  120
   ggcagagttg aggaaccgcg gctggtcggt cacgtaggct ggggcggtct cgtacaggca  180
   ggcgtgcctg gtgaccttga                                              200
<210> 879
   <211> 200
   <212> DNA
   <213> Lolium multiflorum
<400> 879
   cccgaggccg atcgatctgg acattcttct gtatggggac tcccggatca agaccgagtc   60
   tctgattgtg ccgcatgaac gcatccatga gagaccgttc gtcttggcgc ctcttgttga  120
   cctcctgggt tcgtcggctg aggacggtat ggagaaaagc tggcactctc tctcgaagtg  180
   cagtggcggg ttctttgatt                                              200
<210> 880
   <211> 200
   <212> DNA
   <213> Lolium multiflorum
<400> 880
   aatcaaagaa cccgccactg cacttcgaga gagagtgcca gcttttctcc ataccgtcct   60
   cagccgacga acccaggagg tcaacaagag gcgccaagac gaacggtctc tcatggatgc  120
   gttcatgcgg cacaatcaga gactcggtct tgatccggga gtccccatac agaagaatgt  180
   ccagatcgat cggcctcggg                                              200
<210> 881
   <211> 200
   <212> DNA
   <213> Lolium multiflorum
<400> 881
   aagtgcagtg gcgggttctt tgatttgtgg aacaagctcg gcggtgaatc tgttgttgga   60
   acagaaggca ttaaaagggt catgtctgtt ggaaatacgc tgttggactg gcgtcagagg  120
   acccttgtta tgggggtgct taacctcacg ccagacagct tcagcgacgg aggtaagttt  180
   caagaagtgg aagctgccat                                              200
<210> 882
   <211> 200
   <212> DNA
   <213> Lolium multiflorum
<400> 882
   atggcagctt ccacttcttg aaacttacct ccgtcgctga agctgtctgg cgtgaggtta   60
   agcaccccca taacaagggt cctctgacgc cagtccaaca gcgtatttcc aacagacatg  120
   acccttttaa tgccttctgt tccaacaaca gattcaccgc cgagcttgtt ccacaaatca  180
   aagaacccgc cactgcactt                                              200
<210> 883
   <211> 200
   <212> DNA
   <213> Lolium multiflorum
<400> 883
   agtttcaaga agtggaagct gccatttccc aggccaggtt gttgatatca gaaggtgctg   60
   acataattga tattggtgcc cagtccacca ggccctttgc aaggaggcta tccgcagaag  120
   aagagcttga gaggctggtc cctgttctgg atgctatcat gaaactccca gagatggaag  180
   ggaagttgct ttcggtagac                                              200
<210> 884
   <211> 200
   <212> DNA
   <213> Lolium multiflorum
<400> 884
   gtctaccgaa agcaacttcc cttccatctc tgggagtttc atgatagcat ccagaacagg 60
   gaccagcctc tcaagctctt cttctgcgga tagcctcctt gcaaagggcc tggtggactg 120
   ggcaccaata tcaattatgt cagcaccttc tgatatcaac aacctggcct gggaaatggc 180
   agcttccact tcttgaaact 200
<210> 885
   <211> 200
   <212> DNA
   <213> Lolium multiflorum
<400> 885
   ggaagggaag ttgctttcgg tagacacgtt ctacgcgcaa gtcgctgctg aagctgtaaa 60
   aagaggagcc accatgatca atgatgtatc tggtgggcag cttgacccaa gtattcttca 120
   agttgttgct gaactgggag ttccgtatgt taccatgcac atgagaggcg atccatcaac 180
   tatgcagaat gaacagaatc 200
<210> 886
   <211> 200
   <212> DNA
   <213> Lolium multiflorum
<400> 886
   gattctgttc attctgcata gttgatggat cgcctctcat gtgcatggta acatacggaa 60
   ctcccagttc agcaacaact tgaagaatac ttgggtcaag ctgcccacca gatacatcat 120
   tgatcatggt ggctcctctt tttacagctt cagcagcgac ttgcgcgtag aacgtgtcta 180
   ccgaaagcaa cttcccttcc 200
<210> 887
   <211> 200
   <212> DNA
   <213> Lolium multiflorum
<400> 887
   tcaactatgc agaatgaaca gaatctacag tatgatgatg tctgcaaaga agttgcttct 60
   gagttatacg cccggttgag agcagcagag ctttctggaa ttcctttgtg gaggattatt 120
   cttgaccctg gcattgggtt ttctaagaaa tccacacaga atattgaagt aatcgcgggt 180
   ttggaatcca ttagagaaga 200
<210> 888
   <211> 200
   <212> DNA
   <213> Lolium multiflorum
<400> 888
   tcttctctaa tggattccaa acccgcgatt acttcaatat tctgtgtgga tttcttagaa 60
   aacccaatgc cagggtcaag aataatcctc cacaaaggaa ttccagaaag ctctgctgct 120
   ctcaaccggg cgtataactc agaagcaact tctttgcaga catcatcata ctgtagattc 180
   tgttcattct gcatagttga 200
<210> 889
   <211> 200
   <212> DNA
   <213> Lolium multiflorum
<400> 889
   cgggtttgga atccattaga gaagagatgg gtaaaatgag cctaggtgct tcacatgtgc 60
   caatattact tggaccctcg aggaaaagtt tcctaggtga aatatgtagt cgcgccgatc 120
   cagttcagag agatgctgct actgcttctg ccgttacaat tgcgatcttg aatggcgcta 180
   atatagtaag ggtccataat 200
<210> 890
   <211> 200
   <212> DNA
   <213> Lolium multiflorum
<400> 890
   attatggacc cttactatat tagcgccatt caagatcgca attgtaacgg cagaagcagt 60
   agcagcatct ctctgaactg gatcggcgcg actacatatt tcacctagga aacttttcct 120
   cgagggtcca agtaatattg gcacatgtga agcacctagg ctcattttac ccatctcttc 180
   tctaatggat tccaaacccg 200
<210> 891
   <211> 200
   <212> DNA
   <213> Lolium multiflorum
<400> 891
   aggatatagg gcgcaccgcc gggataaggt acggtccgag gccgatcgat ctggacattc 60
   ttctgtatgg ggattcccgg atcaagaccg agtctctgac tgtgccgcat gaacgcatcc 120
   atgagagacc gttcgtcttg gcgcctcttg ttgacctcct gggttcatcg ggtgaggatg 180
   gtatggagag aagatggcac 200
<210> 892
   <211> 200
   <212> DNA
   <213> Lolium multiflorum
<400> 892
   gtgccatctt ctctccatac catcctcacc cgatgaaccc aggaggtcaa caagaggcgc    60
   caagacgaac ggtctctcat ggatgcgttc atgcggcaca gtcagagact cggtcttgat   120
   ccgggaatcc ccatacagaa gaatgtccag atcgatcggc ctcggaccgt accttatccc   180
   ggcggtgcgc cctatatcct                                               200
<210> 893
   <211> 200
   <212> DNA
   <213> Lolium multiflorum
<400> 893
   agggtcatgt ctgttggaaa tacgctgttg gattggcggg agaggaccct ggttatgggg    60
   gtgcttaacc tcacgccaga cagctttagc gatggaggta agtttcaaga ggtggaagct   120
   gccatttctc aggccaggct gttaatttca gaaggtgcag gcataattga tattggcgct   180
   cagtccacca ggccctttgc                                               200
<210> 894
   <211> 200
   <212> DNA
   <213> Lolium multiflorum
<400> 894
   gcaaagggcc tggtggactg agcgccaata tcaattatgc ctgcaccttc tgaaattaac    60
   agcctggcct gagaaatggc agcttccacc tcttgaaact tacctccatc gctaaagctg   120
   tctggcgtga ggttaagcac ccccataacc agggtcctct cccgccaatc caacagcgta    180
   tttccaacag acatgaccct                                               200
<210> 895
   <211> 200
   <212> DNA
   <213> Lolium multiflorum
<400> 895
   gcgctcagtc caccaggccc tttgcaaggt ggctatctgc agaagaagag cttgagaggc    60
   tggtccctgt tcttaatgct atcatgaaac tccccgagat ggaagggaag ttgctttcag   120
   tagatacgtt ctacgcgcaa gtcgcggctg aagccgtgaa aagaggggcc accatgatca   180
   atgatgtatc tggtgggcag                                               200
<210> 896
   <211> 200
   <212> DNA
   <213> Lolium multiflorum
<400> 896
   ctgcccacca gatacatcat tgatcatggt ggcccctctt ttcacggctt cagccgcgac 60
   ttgcgcgtag aacgtatcta ctgaaagcaa cttcccttcc atctcgggga gtttcatgat 120
   agcattaaga acagggacca gcctctcaag ctcttcttct gcagatagcc accttgcaaa 180
   gggcctggtg gactgagcgc 200
<210> 897
   <211> 200
   <212> DNA
   <213> Lolium multiflorum
<400> 897
   gatcaatgat gtatctggtg ggcagcttga cccagatatt cttcaagttg ttgctgaatt 60
   gggagttccg tatgttgcca tgcacatgag aggggatcca tcaactatgc agaatgagca 120
   gaatttacag tatgatgatg tctgcaaaga agttgcttct gagttatact ctcggttgag 180
   agcagcagag ttgtctggaa 200
<210> 898
   <211> 200
   <212> DNA
   <213> Lolium multiflorum
<400> 898
   ttccagacaa ctctgctgct ctcaaccgag agtataactc agaagcaact tctttgcaga 60
   catcatcata ctgtaaattc tgctcattct gcatagttga tggatcccct ctcatgtgca 120
   tggcaacata cggaactccc aattcagcaa caacttgaag aatatctggg tcaagctgcc 180
   caccagatac atcattgatc 200
<210> 899
   <211> 200
   <212> DNA
   <213> Lolium multiflorum
<400> 899
   ttgagagcag cagagttgtc tggaattcct ttgtggagga ttattcttga ccctggcatt 60
   gggttttcca agaaatccac acagaatatt gaagtaatcg cgggcttgga atccattaga 120
   gaagagatgg gtaaaatgag tctaggtgct tcacatgtgc caatattact tggaccctcc 180
   aggaaacgtt tcttagggca 200
<210> 900
   <211> 200
   <212> DNA
   <213> Lolium multiflorum
<400> 900
   tgccctaaga aacgtttcct ggagggtcca agtaatattg gcacatgtga agcacctaga   60
   ctcattttac ccatctcttc tctaatggat tccaagcccg cgattacttc aatattctgt  120
   gtggatttct tggaaaaccc aatgccaggg tcaagaataa tcctccacaa aggaattcca  180
   gacaactctg ctgctctcaa                                              200
<210> 901
   <211> 200
   <212> DNA
   <213> Lolium multiflorum
<400> 901
   cgctaacctg ttcagggtcc gttcgtacat gcaatgcgcg gtcaccaacg attccgccga   60
   ccaagagatc gtgattgccc tgggcagcaa cgtgggagac agggtcagca cgttcgacag  120
   ggcgctgcgg ctgatgagga gctcgggcat caggatcacc aggcacgcct gcctgtacga  180
   gaccgcccct gcttacgtga                                              200
<210> 902
   <211> 200
   <212> DNA
   <213> Lolium multiflorum
<400> 902
   tcacgtaagc aggggcggtc tcgtacaggc aggcgtgcct ggtgatcctg atgcccgagc   60
   tcctcatcag ccgcagcgcc ctgtcgaacg tgctgaccct gtctcccacg ttgctgccca  120
   gggcaatcac gatctcttgg tcggcggaat cgttggtgac cgcgcattgc atgtacgaac  180
   ggaccctgaa caggttagcg                                              200
<210> 903
   <211> 200
   <212> DNA
   <213> Lolium multiflorum
<400> 903
   gatatagggc gcaccgccgg gataaggtac ggtccgaggc cgatcgatct ggacattctt 60
   ctgtatgggg attcccggat caagaccgag tctctgactg tgccgcatga acgcatccat 120
   gagagaccgt tcgtcttggc gcctcttgtt gacctcctgg gttcatcggg tgaggatggt 180
   atggagagaa gatggcactc 200
<210> 904
   <211> 200
   <212> DNA
   <213> Lolium multiflorum
<400> 904
   gagtgccatc ttctctccat accatcctca cccgatgaac ccaggaggtc aacaagaggc 60
   gccaagacga acggtctctc atggatgcgt tcatgcggca cagtcagaga ctcggtcttg 120
   atccgggaat ccccatacag aagaatgtcc agatcgatcg gcctcggacc gtaccttatc 180
   ccggcggtgc gccctatatc 200
<210> 905
   <211> 150
   <212> DNA
   <213> Lolium multiflorum
<400> 905
   gattccgccg accaagagat cgtgattgcc ctgggcagca acgtgggaga cagggtcagc 60
   acgttcgaca gggcgctgcg gctgatgagg agctcgggca tcaggatcac caggcacgcc 120
   tgcctgtacg agaccgcccc tgcttacgtg 150
<210> 906
   <211> 150
   <212> DNA
   <213> Lolium multiflorum
<400> 906
   cacgtaagca ggggcggtct cgtacaggca ggcgtgcctg gtgatcctga tgcccgagct 60
   cctcatcagc cgcagcgccc tgtcgaacgt gctgaccctg tctcccacgt tgctgcccag 120
   ggcaatcacg atctcttggt cggcggaatc 150
<210> 907
   <211> 21
   <212> DNA
   <213> Unknown
<220>
   <223> Found in multiple species
<400> 907
   ttatgtactc ttacaatgtt t 21
<210> 908
   <211> 21
   <212> DNA
   <213> Unknown
<220>
   <223> Found in multiple species
<400> 908
   tgtactctta caatgtttgc a 21
<210> 909
   <211> 21
   <212> DNA
   <213> Unknown
<220>
   <223> Found in multiple species
<400> 909
   tggtgcaaac attgtaagag t 21
<210> 910
   <211> 21
   <212> DNA
   <213> Unknown
<220>
   <223> Found in multiple species
<400> 910
   tgaaaggata tgggaaagac c 21
<210> 911
   <211> 21
   <212> DNA
   <213> Unknown
<220>
   <223> Found in multiple species
<400> 911
   tctttcccat atcctttcat g 21
<210> 912
   <211> 21
   <212> DNA
   <213> Unknown
<220>
   <223> Found in multiple species
<400> 912
   tcttacaatg tttgcaccac c 21
<210> 913
   <211> 21
   <212> DNA
   <213> Unknown
<220>
   <223> Found in multiple species
<400> 913
   tatgtactct tacaatgttt g 21
<210> 914
   <211> 21
   <212> DNA
   <213> Unknown
<220>
   <223> Found in multiple species
<400> 914
   tactcttaca atgtttgcac c 21
<210> 915
   <211> 21
   <212> DNA
   <213> Unknown
<220>
   <223> Found in multiple species
<400> 915
   gtggtgcaaa cattgtaaga g 21
<210> 916
   <211> 21
   <212> DNA
   <213> Unknown
<220>
   <223> Found in multiple species
<400> 916
   gtgcaaacat tgtaagagta c 21
<210> 917
   <211> 21
   <212> DNA
   <213> Unknown
<220>
   <223> Found in multiple species
<400> 917
   gtctttccca tatcctttca t 21
<210> 918
   <211> 21
   <212> DNA
   <213> Unknown
<220>
   <223> Found in multiple species
<400> 918
   gcaaacattg taagagtaca t 21
<210> 919
   <211> 21
   <212> DNA
   <213> Unknown
<220>
   <223> Found in multiple species
<400> 919
   cattgtaaga gtacataatg t 21
<210> 920
   <211> 21
   <212> DNA
   <213> Unknown
<220>
   <223> Found in multiple species
<400> 920
   cattatgtac tcttacaatg t 21
<210> 921
   <211> 21
   <212> DNA
   <213> Unknown
<220>
   <223> Found in multiple species
<400> 921
   attatgtact cttacaatgt t 21
<210> 922
   <211> 21
   <212> DNA
   <213> Unknown
<220>
   <223> Found in multiple species
<400> 922
   ttttctacat cagacccaat a 21
<210> 923
   <211> 21
   <212> DNA
   <213> Unknown
<220>
   <223> Found in multiple species
<400> 923
   ttttcattcc atccttcccg a 21
<210> 924
   <211> 21
   <212> DNA
   <213> Unknown
<220>
   <223> Found in multiple species
<400> 924
   ttttcaatct gtcgatactg c 21
<210> 925
   <211> 21
   <212> DNA
   <213> Unknown
<220>
   <223> Found in multiple species
<400> 925
   ttttatgaga ccaatcccat a 21
<210> 926
   <211> 21
   <212> DNA
   <213> Unknown
<220>
   <223> Found in multiple species
<400> 926
   ttttaggtgg tgcaaacatt g 21
<210> 927
   <211> 21
   <212> DNA
   <213> Unknown
<220>
   <223> Found in multiple species
<400> 927
   tttgcaccac ctaaaactcc a 21
<210> 928
   <211> 21
   <212> DNA
   <213> Unknown
<220>
   <223> Found in multiple species
<400> 928
   tttctacatc agacccaata c 21
<210> 929
   <211> 21
   <212> DNA
   <213> Unknown
<220>
   <223> Found in multiple species
<400> 929
   tttcccatat cctttcatgg g 21
<210> 930
   <211> 21
   <212> DNA
   <213> Unknown
<220>
   <223> Found in multiple species
<400> 930
   tttcattcca tccttcccga t 21
<210> 931
   <211> 21
   <212> DNA
   <213> Unknown
<220>
   <223> Found in multiple species
<400> 931
   tttcaatctg tcgatactgc g 21
<210> 932
   <211> 21
   <212> DNA
   <213> Unknown
<220>
   <223> Found in multiple species
<400> 932
   tttatgagac caatcccata a 21
<210> 933
   <211> 21
   <212> DNA
   <213> Unknown
<220>
   <223> Found in multiple species
<400> 933
   tttaggtggt gcaaacattg t 21
<210> 934
   <211> 21
   <212> DNA
   <213> Unknown
<220>
   <223> Found in multiple species
<400> 934
   tttagggtga gctctgagag c 21
<210> 935
   <211> 21
   <212> DNA
   <213> Unknown
<220>
   <223> Found in multiple species
<400> 935
   ttgtattggg tctgatgtag a 21
<210> 936
   <211> 21
   <212> DNA
   <213> Unknown
<220>
   <223> Found in multiple species
<400> 936
   ttgtaagagt acataatgtg a 21
<210> 937
   <211> 21
   <212> DNA
   <213> Unknown
<220>
   <223> Found in multiple species
<400> 937
   ttggtctcat aaaacctctg t 21
<210> 938
   <211> 21
   <212> DNA
   <213> Unknown
<220>
   <223> Found in multiple species
<400> 938
   ttgggtctga tgtagaaaat g 21
<210> 939
   <211> 21
   <212> DNA
   <213> Unknown
<220>
   <223> Found in multiple species
<400> 939
   ttggcctagg accatacctt a 21
<210> 940
   <211> 21
   <212> DNA
   <213> Unknown
<220>
   <223> Found in multiple species
<400> 940
   ttggacatac tattttatgg g 21
<210> 941
   <211> 21
   <212> DNA
   <213> Unknown
<220>
   <223> Found in multiple species
<400> 941
   ttgctaaggt tcgtcagatg a 21
<210> 942
   <211> 21
   <212> DNA
   <213> Unknown
<220>
   <223> Found in multiple species
<400> 942
   ttgcaccacc taaaactcca g 21
<210> 943
   <211> 21
   <212> DNA
   <213> Unknown
<220>
   <223> Found in multiple species
<400> 943
   ttgattgtat tgggtctgat g 21
<210> 944
   <211> 21
   <212> DNA
   <213> Unknown
<220>
   <223> Found in multiple species
<400> 944
   ttgacttgga catactattt t 21
<210> 945
   <211> 21
   <212> DNA
   <213> Unknown
<220>
   <223> Found in multiple species
<400> 945
   ttgactcctg atagctttag t 21
<210> 946
   <211> 21
   <212> DNA
   <213> Unknown
<220>
   <223> Found in multiple species
<400> 946
   ttgaacttga ctcctgatag c 21
<210> 947
   <211> 21
   <212> DNA
   <213> Unknown
<220>
   <223> Found in multiple species
<400> 947
   ttctacatca gacccaatac a 21
<210> 948
   <211> 21
   <212> DNA
   <213> Unknown
<220>
   <223> Found in multiple species
<400> 948
   ttccgccatc actaaagcta t 21
<210> 949
   <211> 21
   <212> DNA
   <213> Unknown
<220>
   <223> Found in multiple species
<400> 949
   ttccctaatc gggaaggatg g 21
<210> 950
   <211> 21
   <212> DNA
   <213> Unknown
<220>
   <223> Found in multiple species
<400> 950
   ttcccgatta gggaacttcc a 21
<210> 951
   <211> 21
   <212> DNA
   <213> Unknown
<220>
   <223> Found in multiple species
<400> 951
   ttcccatatc ctttcatggg g 21
<210> 952
   <211> 21
   <212> DNA
   <213> Unknown
<220>
   <223> Found in multiple species
<400> 952
   ttcccataaa atagtatgtc c 21
<210> 953
   <211> 21
   <212> DNA
   <213> Unknown
<220>
   <223> Found in multiple species
<400> 953
   ttccatcctt cccgattagg g 21
<210> 954
   <211> 21
   <212> DNA
   <213> Unknown
<220>
   <223> Found in multiple species
<400> 954
   ttcattccat ccttcccgat t 21
<210> 955
   <211> 21
   <212> DNA
   <213> Unknown
<220>
   <223> Found in multiple species
<400> 955
   ttcaatctgt cgatactgcg g 21
<210> 956
   <211> 21
   <212> DNA
   <213> Unknown
<220>
   <223> Found in multiple species
<400> 956
   ttcaagactc ccattacaga g 21
<210> 957
   <211> 21
   <212> DNA
   <213> Unknown
<220>
   <223> Found in multiple species
<400> 957
   ttatgagacc aatcccataa g 21
<210> 958
   <211> 21
   <212> DNA
   <213> Unknown
<220>
   <223> Found in multiple species
<400> 958
   ttatccctca cattatgtac t 21
<210> 959
   <211> 21
   <212> DNA
   <213> Unknown
<220>
   <223> Found in multiple species
<400> 959
   ttatatagca atgcacatgc g 21
<210> 960
   <211> 21
   <212> DNA
   <213> Unknown
<220>
   <223> Found in multiple species
<400> 960
   ttaggtggtg caaacattgt a 21
<210> 961
   <211> 21
   <212> DNA
   <213> Unknown
<220>
   <223> Found in multiple species
<400> 961
   ttagggtgag ctctgagagc c 21
<210> 962
   <211> 21
   <212> DNA
   <213> Unknown
<220>
   <223> Found in multiple species
<400> 962
   ttagggaact tccaccgagt t 21
<210> 963
   <211> 21
   <212> DNA
   <213> Unknown
<220>
   <223> Found in multiple species
<400> 963
   ttagcaaccg cagtatcgac a 21
<210> 964
   <211> 21
   <212> DNA
   <213> Unknown
<220>
   <223> Found in multiple species
<400> 964
   ttacaatgtt tgcaccacct a 21
<210> 965
   <211> 21
   <212> DNA
   <213> Unknown
<220>
   <223> Found in multiple species
<400> 965
   tgtttgcacc acctaaaact c 21
<210> 966
   <211> 21
   <212> DNA
   <213> Unknown
<220>
   <223> Found in multiple species
<400> 966
   tgtgagggat aaccttgatg c 21
<210> 967
   <211> 21
   <212> DNA
   <213> Unknown
<220>
   <223> Found in multiple species
<400> 967
   tgtccaagtc aattggccta g 21
<210> 968
   <211> 21
   <212> DNA
   <213> Unknown
<220>
   <223> Found in multiple species
<400> 968
   tgtcattttc tacatcagac c 21
<210> 969
   <211> 21
   <212> DNA
   <213> Unknown
<220>
   <223> Found in multiple species
<400> 969
   tgtagaaaat gacactattt g 21
<210> 970
   <211> 21
   <212> DNA
   <213> Unknown
<220>
   <223> Found in multiple species
<400> 970
   tgtaagagta cataatgtga g 21
<210> 971
   <211> 21
   <212> DNA
   <213> Unknown
<220>
   <223> Found in multiple species
<400> 971
   tggtgccatc acaaatggtc t 21
<210> 972
   <211> 21
   <212> DNA
   <213> Unknown
<220>
   <223> Found in multiple species
<400> 972
   tggtctttcc catatccttt c 21
<210> 973
   <211> 21
   <212> DNA
   <213> Unknown
<220>
   <223> Found in multiple species
<400> 973
   tggtcctagg ccaattgact t 21
<210> 974
   <211> 21
   <212> DNA
   <213> Unknown
<220>
   <223> Found in multiple species
<400> 974
   tgggtctgat gtagaaaatg a 21
<210> 975
   <211> 21
   <212> DNA
   <213> Unknown
<220>
   <223> Found in multiple species
<400> 975
   tgggattggt ctcataaaac c 21
<210> 976
   <211> 21
   <212> DNA
   <213> Unknown
<220>
   <223> Found in multiple species
<400> 976
   tgggagtctt gaacttgact c 21
<210> 977
   <211> 21
   <212> DNA
   <213> Unknown
<220>
   <223> Found in multiple species
<400> 977
   tgggaaagac catttgtgat g 21
<210> 978
   <211> 21
   <212> DNA
   <213> Unknown
<220>
   <223> Found in multiple species
<400> 978
   tggcctagga ccatacctta t 21
<210> 979
   <211> 21
   <212> DNA
   <213> Unknown
<220>
   <223> Found in multiple species
<400> 979
   tggacatact attttatggg a 21
<210> 980
   <211> 21
   <212> DNA
   <213> Unknown
<220>
   <223> Found in multiple species
<400> 980
   tggaatgaaa agggttttgc c 21
<210> 981
   <211> 21
   <212> DNA
   <213> Unknown
<220>
   <223> Found in multiple species
<400> 981
   tgctggagtt ttaggtggtg c 21
<210> 982
   <211> 21
   <212> DNA
   <213> Unknown
<220>
   <223> Found in multiple species
<400> 982
   tgctaaggtt cgtcagatga t 21
<210> 983
   <211> 21
   <212> DNA
   <213> Unknown
<220>
   <223> Found in multiple species
<400> 983
   tgcggttgct aaggttcgtc a 21
<210> 984
   <211> 21
   <212> DNA
   <213> Unknown
<220>
   <223> Found in multiple species
<400> 984
   tgccatcaca aatggtcttt c 21
<210> 985
   <211> 21
   <212> DNA
   <213> Unknown
<220>
   <223> Found in multiple species
<400> 985
   tgcaccacct aaaactccag c 21
<210> 986
   <211> 21
   <212> DNA
   <213> Unknown
<220>
   <223> Found in multiple species
<400> 986
   tgattgtatt gggtctgatg t 21
<210> 987
   <211> 21
   <212> DNA
   <213> Unknown
<220>
   <223> Found in multiple species
<400> 987
   tgatgtagaa aatgacacta t 21
<210> 988
   <211> 21
   <212> DNA
   <213> Unknown
<220>
   <223> Found in multiple species
<400> 988
   tgatagcttt agtgatggcg g 21
<210> 989
   <211> 21
   <212> DNA
   <213> Unknown
<220>
   <223> Found in multiple species
<400> 989
   tgaggctctc agagctcacc c 21
<210> 990
   <211> 21
   <212> DNA
   <213> Unknown
<220>
   <223> Found in multiple species
<400> 990
   tgagctctga gagcctcact a 21
<210> 991
   <211> 21
   <212> DNA
   <213> Unknown
<220>
   <223> Found in multiple species
<400> 991
   tgacttggac atactatttt a 21
<210> 992
   <211> 21
   <212> DNA
   <213> Unknown
<220>
   <223> Found in multiple species
<400> 992
   tgactcctga tagctttagt g 21
<210> 993
   <211> 21
   <212> DNA
   <213> Unknown
<220>
   <223> Found in multiple species
<400> 993
   tgaacttgac tcctgatagc t 21
<210> 994
   <211> 21
   <212> DNA
   <213> Unknown
<220>
   <223> Found in multiple species
<400> 994
   tgaaaagggt tttgcccgtt g 21
<210> 995
   <211> 21
   <212> DNA
   <213> Unknown
<220>
   <223> Found in multiple species
<400> 995
   tcttgaactt gactcctgat a 21
<210> 996
   <211> 21
   <212> DNA
   <213> Unknown
<220>
   <223> Found in multiple species
<400> 996
   tctgtcgata ctgcggttgc t 21
<210> 997
   <211> 21
   <212> DNA
   <213> Unknown
<220>
   <223> Found in multiple species
<400> 997
   tctgatgtag aaaatgacac t 21
<210> 998
   <211> 21
   <212> DNA
   <213> Unknown
<220>
   <223> Found in multiple species
<400> 998
   tctgacgaac cttagcaacc g 21
<210> 999
   <211> 21
   <212> DNA
   <213> Unknown
<220>
   <223> Found in multiple species
<400> 999
   tcggtggaag ttccctaatc g 21
<210> 1000
   <211> 21
   <212> DNA
   <213> Unknown
<220>
   <223> Found in multiple species
<400> 1000
   tcgcatgtgc attgctatat a 21
<210> 1001
   <211> 21
   <212> DNA
   <213> Unknown
<220>
   <223> Found in multiple species
<400> 1001
   tcgatactgc ggttgctaag g 21
<210> 1002
   <211> 21
   <212> DNA
   <213> Unknown
<220>
   <223> Found in multiple species
<400> 1002
   tccttcccga ttagggaact t 21
<210> 1003
   <211> 21
   <212> DNA
   <213> Unknown
<220>
   <223> Found in multiple species
<400> 1003
   tcctgatagc tttagtgatg g 21
<210> 1004
   <211> 21
   <212> DNA
   <213> Unknown
<220>
   <223> Found in multiple species
<400> 1004
   tcctaggcca attgacttgg a 21
<210> 1005
   <211> 21
   <212> DNA
   <213> Unknown
<220>
   <223> Found in multiple species
<400> 1005
   tccgccatca ctaaagctat c 21
<210> 1006
   <211> 21
   <212> DNA
   <213> Unknown
<220>
   <223> Found in multiple species
<400> 1006
   tccctcacat tatgtactct t 21
<210> 1007
   <211> 21
   <212> DNA
   <213> Unknown
<220>
   <223> Found in multiple species
<400> 1007
   tccctaatcg ggaaggatgg a 21
<210> 1008
   <211> 21
   <212> DNA
   <213> Unknown
<220>
   <223> Found in multiple species
<400> 1008
   tcccgattag ggaacttcca c 21
<210> 1009
   <211> 21
   <212> DNA
   <213> Unknown
<220>
   <223> Found in multiple species
<400> 1009
   tcattccatc cttcccgatt a 21
<210> 1010
   <211> 21
   <212> DNA
   <213> Unknown
<220>
   <223> Found in multiple species
<400> 1010
   tcaggagtca agttcaagac t 21
<210> 1011
   <211> 21
   <212> DNA
   <213> Unknown
<220>
   <223> Found in multiple species
<400> 1011
   tcactaaagc tatcaggagt c 21
<210> 1012
   <211> 21
   <212> DNA
   <213> Unknown
<220>
   <223> Found in multiple species
<400> 1012
   tcacaaatgg tctttcccat a 21
<210> 1013
   <211> 21
   <212> DNA
   <213> Unknown
<220>
   <223> Found in multiple species
<400> 1013
   tcaattggcc taggaccata c 21
<210> 1014
   <211> 21
   <212> DNA
   <213> Unknown
<220>
   <223> Found in multiple species
<400> 1014
   tcaatctgtc gatactgcgg t 21
<210> 1015
   <211> 21
   <212> DNA
   <213> Unknown
<220>
   <223> Found in multiple species
<400> 1015
   tcaagttatg tgatgccata c 21
<210> 1016
   <211> 21
   <212> DNA
   <213> Unknown
<220>
   <223> Found in multiple species
<400> 1016
   tcaaggttat ccctcacatt a 21
<210> 1017
   <211> 21
   <212> DNA
   <213> Unknown
<220>
   <223> Found in multiple species
<400> 1017
   tcaagactcc cattacagag g 21
<210> 1018
   <211> 21
   <212> DNA
   <213> Unknown
<220>
   <223> Found in multiple species
<400> 1018
   tatgtccaag tcaattggcc t 21
<210> 1019
   <211> 21
   <212> DNA
   <213> Unknown
<220>
   <223> Found in multiple species
<400> 1019
   tatggtccta ggccaattga c 21
<210> 1020
   <211> 21
   <212> DNA
   <213> Unknown
<220>
   <223> Found in multiple species
<400> 1020
   tatggcatca cataacttga c 21
<210> 1021
   <211> 21
   <212> DNA
   <213> Unknown
<220>
   <223> Found in multiple species
<400> 1021
   tatgagacca atcccataag c 21
<210> 1022
   <211> 21
   <212> DNA
   <213> Unknown
<220>
   <223> Found in multiple species
<400> 1022
   tatcgacaga ttgaaaactt c 21
<210> 1023
   <211> 21
   <212> DNA
   <213> Unknown
<220>
   <223> Found in multiple species
<400> 1023
   tatccctcac attatgtact c 21
<210> 1024
   <211> 21
   <212> DNA
   <213> Unknown
<220>
   <223> Found in multiple species
<400> 1024
   tatagcaatg cacatgcgag g 21
<210> 1025
   <211> 21
   <212> DNA
   <213> Unknown
<220>
   <223> Found in multiple species
<400> 1025
   tagtgtcatt ttctacatca g 21
<210> 1026
   <211> 21
   <212> DNA
   <213> Unknown
<220>
   <223> Found in multiple species
<400> 1026
   tagtatgtcc aagtcaattg g 21
<210> 1027
   <211> 21
   <212> DNA
   <213> Unknown
<220>
   <223> Found in multiple species
<400> 1027
   tagggtgagc tctgagagcc t 21
<210> 1028
   <211> 21
   <212> DNA
   <213> Unknown
<220>
   <223> Found in multiple species
<400> 1028
   tagggaactt ccaccgagtt t 21
<210> 1029
   <211> 21
   <212> DNA
   <213> Unknown
<220>
   <223> Found in multiple species
<400> 1029
   taggccaatt gacttggaca t 21
<210> 1030
   <211> 21
   <212> DNA
   <213> Unknown
<220>
   <223> Found in multiple species
<400> 1030
   taggaccata ccttattccc t 21
<210> 1031
   <211> 21
   <212> DNA
   <213> Unknown
<220>
   <223> Found in multiple species
<400> 1031
   tagctttagt gatggcggaa g 21
<210> 1032
   <211> 21
   <212> DNA
   <213> Unknown
<220>
   <223> Found in multiple species
<400> 1032
   tagcaaccgc agtatcgaca g 21
<210> 1033
   <211> 21
   <212> DNA
   <213> Unknown
<220>
   <223> Found in multiple species
<400> 1033
   tagaaaatga cactatttgt a 21
<210> 1034
   <211> 21
   <212> DNA
   <213> Unknown
<220>
   <223> Found in multiple species
<400> 1034
   tactgcggtt gctaaggttc g 21
<210> 1035
   <211> 21
   <212> DNA
   <213> Unknown
<220>
   <223> Found in multiple species
<400> 1035
   tacatcagac ccaatacaat c 21
<210> 1036
   <211> 21
   <212> DNA
   <213> Unknown
<220>
   <223> Found in multiple species
<400> 1036
   tacataatgt gagggataac c 21
<210> 1037
   <211> 21
   <212> DNA
   <213> Unknown
<220>
   <223> Found in multiple species
<400> 1037
   taaggttcgt cagatgatct c 21
<210> 1038
   <211> 21
   <212> DNA
   <213> Unknown
<220>
   <223> Found in multiple species
<400> 1038
   taagagtaca taatgtgagg g 21
<210> 1039
   <211> 21
   <212> DNA
   <213> Unknown
<220>
   <223> Found in multiple species
<400> 1039
   taaagctatc aggagtcaag t 21
<210> 1040
   <211> 21
   <212> DNA
   <213> Unknown
<220>
   <223> Found in multiple species
<400> 1040
   taaactcggt ggaagttccc t 21
<210> 1041
   <211> 21
   <212> DNA
   <213> Unknown
<220>
   <223> Found in multiple species
<400> 1041
   gttttcaatc tgtcgatact g 21
<210> 1042
   <211> 21
   <212> DNA
   <213> Unknown
<220>
   <223> Found in multiple species
<400> 1042
   gttttatgag accaatccca t 21
<210> 1043
   <211> 21
   <212> DNA
   <213> Unknown
<220>
   <223> Found in multiple species
<400> 1043
   gttttaggtg gtgcaaacat t 21
<210> 1044
   <211> 21
   <212> DNA
   <213> Unknown
<220>
   <223> Found in multiple species
<400> 1044
   gtttgcacca cctaaaactc c 21
<210> 1045
   <211> 21
   <212> DNA
   <213> Unknown
<220>
   <223> Found in multiple species
<400> 1045
   gttgctaagg ttcgtcagat g 21
<210> 1046
   <211> 21
   <212> DNA
   <213> Unknown
<220>
   <223> Found in multiple species
<400> 1046
   gttccctaat cgggaaggat g 21
<210> 1047
   <211> 21
   <212> DNA
   <213> Unknown
<220>
   <223> Found in multiple species
<400> 1047
   gttatccctc acattatgta c 21
<210> 1048
   <211> 21
   <212> DNA
   <213> Unknown
<220>
   <223> Found in multiple species
<400> 1048
   gtgtcatttt ctacatcaga c 21
<210> 1049
   <211> 21
   <212> DNA
   <213> Unknown
<220>
   <223> Found in multiple species
<400> 1049
   gtggaatctt tgaagcatgg g 21
<210> 1050
   <211> 21
   <212> DNA
   <213> Unknown
<220>
   <223> Found in multiple species
<400> 1050
   gtgccatcac aaatggtctt t 21
<210> 1051
   <211> 21
   <212> DNA
   <213> Unknown
<220>
   <223> Found in multiple species
<400> 1051
   gtgaggctct cagagctcac c 21
<210> 1052
   <211> 21
   <212> DNA
   <213> Unknown
<220>
   <223> Found in multiple species
<400> 1052
   gtgagctctg agagcctcac t 21
<210> 1053
   <211> 21
   <212> DNA
   <213> Unknown
<220>
   <223> Found in multiple species
<400> 1053
   gtcttgaact tgactcctga t 21
<210> 1054
   <211> 21
   <212> DNA
   <213> Unknown
<220>
   <223> Found in multiple species
<400> 1054
   gtcgatactg cggttgctaa g 21
<210> 1055
   <211> 21
   <212> DNA
   <213> Unknown
<220>
   <223> Found in multiple species
<400> 1055
   gtcctaggcc aattgacttg g 21
<210> 1056
   <211> 21
   <212> DNA
   <213> Unknown
<220>
   <223> Found in multiple species
<400> 1056
   gtccaagtca attggcctag g 21
<210> 1057
   <211> 21
   <212> DNA
   <213> Unknown
<220>
   <223> Found in multiple species
<400> 1057
   gtcattttct acatcagacc c 21
<210> 1058
   <211> 21
   <212> DNA
   <213> Unknown
<220>
   <223> Found in multiple species
<400> 1058
   gtatgtccaa gtcaattggc c 21
<210> 1059
   <211> 21
   <212> DNA
   <213> Unknown
<220>
   <223> Found in multiple species
<400> 1059
   gtatcgacag attgaaaact t 21
<210> 1060
   <211> 21
   <212> DNA
   <213> Unknown
<220>
   <223> Found in multiple species
<400> 1060
   gtagaaaatg acactatttg t 21
<210> 1061
   <211> 21
   <212> DNA
   <213> Unknown
<220>
   <223> Found in multiple species
<400> 1061
   gtacaaatag tgtcattttc t 21
<210> 1062
   <211> 21
   <212> DNA
   <213> Unknown
<220>
   <223> Found in multiple species
<400> 1062
   gtaagagtac ataatgtgag g 21
<210> 1063
   <211> 21
   <212> DNA
   <213> Unknown
<220>
   <223> Found in multiple species
<400> 1063
   ggttgctaag gttcgtcaga t 21
<210> 1064
   <211> 21
   <212> DNA
   <213> Unknown
<220>
   <223> Found in multiple species
<400> 1064
   ggtggaatct ttgaagcatg g 21
<210> 1065
   <211> 21
   <212> DNA
   <213> Unknown
<220>
   <223> Found in multiple species
<400> 1065
   ggtggaagtt ccctaatcgg g 21
<210> 1066
   <211> 21
   <212> DNA
   <213> Unknown
<220>
   <223> Found in multiple species
<400> 1066
   ggtgccatca caaatggtct t 21
<210> 1067
   <211> 21
   <212> DNA
   <213> Unknown
<220>
   <223> Found in multiple species
<400> 1067
   ggtcctaggc caattgactt g 21
<210> 1068
   <211> 21
   <212> DNA
   <213> Unknown
<220>
   <223> Found in multiple species
<400> 1068
   ggtatggtcc taggccaatt g 21
<210> 1069
   <211> 21
   <212> DNA
   <213> Unknown
<220>
   <223> Found in multiple species
<400> 1069
   gggtggaatc tttgaagcat g 21
<210> 1070
   <211> 21
   <212> DNA
   <213> Unknown
<220>
   <223> Found in multiple species
<400> 1070
   gggcaaaacc cttttcattc c 21
<210> 1071
   <211> 21
   <212> DNA
   <213> Unknown
<220>
   <223> Found in multiple species
<400> 1071
   gggattggtc tcataaaacc t 21
<210> 1072
   <211> 21
   <212> DNA
   <213> Unknown
<220>
   <223> Found in multiple species
<400> 1072
   gggagtcttg aacttgactc c 21
<210> 1073
   <211> 21
   <212> DNA
   <213> Unknown
<220>
   <223> Found in multiple species
<400> 1073
   gggaataagg tatggtccta g 21
<210> 1074
   <211> 21
   <212> DNA
   <213> Unknown
<220>
   <223> Found in multiple species
<400> 1074
   gggaaggatg gaatgaaaag g 21
<210> 1075
   <211> 21
   <212> DNA
   <213> Unknown
<220>
   <223> Found in multiple species
<400> 1075
   gggaaagacc atttgtgatg g 21
<210> 1076
   <211> 21
   <212> DNA
   <213> Unknown
<220>
   <223> Found in multiple species
<400> 1076
   ggcctaggac cataccttat t 21
<210> 1077
   <211> 21
   <212> DNA
   <213> Unknown
<220>
   <223> Found in multiple species
<400> 1077
   ggattggtct cataaaacct c 21
<210> 1078
   <211> 21
   <212> DNA
   <213> Unknown
<220>
   <223> Found in multiple species
<400> 1078
   ggatggaatg aaaagggttt t 21
<210> 1079
   <211> 21
   <212> DNA
   <213> Unknown
<220>
   <223> Found in multiple species
<400> 1079
   ggatatggga aagaccattt g 21
<210> 1080
   <211> 21
   <212> DNA
   <213> Unknown
<220>
   <223> Found in multiple species
<400> 1080
   ggagtcttga acttgactcc t 21
<210> 1081
   <211> 21
   <212> DNA
   <213> Unknown
<220>
   <223> Found in multiple species
<400> 1081
   ggaataaggt atggtcctag g 21
<210> 1082
   <211> 21
   <212> DNA
   <213> Unknown
<220>
   <223> Found in multiple species
<400> 1082
   ggaagttttc aatctgtcga t 21
<210> 1083
   <211> 21
   <212> DNA
   <213> Unknown
<220>
   <223> Found in multiple species
<400> 1083
   ggaagttccc taatcgggaa g 21
<210> 1084
   <211> 21
   <212> DNA
   <213> Unknown
<220>
   <223> Found in multiple species
<400> 1084
   ggaaggatgg aatgaaaagg g 21
<210> 1085
   <211> 21
   <212> DNA
   <213> Unknown
<220>
   <223> Found in multiple species
<400> 1085
   ggaaagacca tttgtgatgg c 21
<210> 1086
   <211> 21
   <212> DNA
   <213> Unknown
<220>
   <223> Found in multiple species
<400> 1086
   gctaaggttc gtcagatgat c 21
<210> 1087
   <211> 21
   <212> DNA
   <213> Unknown
<220>
   <223> Found in multiple species
<400> 1087
   gcggttgcta aggttcgtca g 21
<210> 1088
   <211> 21
   <212> DNA
   <213> Unknown
<220>
   <223> Found in multiple species
<400> 1088
   gcctaggacc ataccttatt c 21
<210> 1089
   <211> 21
   <212> DNA
   <213> Unknown
<220>
   <223> Found in multiple species
<400> 1089
   gccatcacta aagctatcag g 21
<210> 1090
   <211> 21
   <212> DNA
   <213> Unknown
<220>
   <223> Found in multiple species
<400> 1090
   gccaattgac ttggacatac t 21
<210> 1091
   <211> 21
   <212> DNA
   <213> Unknown
<220>
   <223> Found in multiple species
<400> 1091
   gcatgtgcat tgctatataa g 21
<210> 1092
   <211> 21
   <212> DNA
   <213> Unknown
<220>
   <223> Found in multiple species
<400> 1092
   gcaaccgcag tatcgacaga t 21
<210> 1093
   <211> 21
   <212> DNA
   <213> Unknown
<220>
   <223> Found in multiple species
<400> 1093
   gcaaaaccct tttcattcca t 21
<210> 1094
   <211> 21
   <212> DNA
   <213> Unknown
<220>
   <223> Found in multiple species
<400> 1094
   gattggtctc ataaaacctc t 21
<210> 1095
   <211> 21
   <212> DNA
   <213> Unknown
<220>
   <223> Found in multiple species
<400> 1095
   gattagggaa cttccaccga g 21
<210> 1096
   <211> 21
   <212> DNA
   <213> Unknown
<220>
   <223> Found in multiple species
<400> 1096
   gatgtagaaa atgacactat t 21
<210> 1097
   <211> 21
   <212> DNA
   <213> Unknown
<220>
   <223> Found in multiple species
<400> 1097
   gatggaatga aaagggtttt g 21
<210> 1098
   <211> 21
   <212> DNA
   <213> Unknown
<220>
   <223> Found in multiple species
<400> 1098
   gatatgggaa agaccatttg t 21
<210> 1099
   <211> 21
   <212> DNA
   <213> Unknown
<220>
   <223> Found in multiple species
<400> 1099
   gatactgcgg ttgctaaggt t 21
<210> 1100
   <211> 21
   <212> DNA
   <213> Unknown
<220>
   <223> Found in multiple species
<400> 1100
   gagtcttgaa cttgactcct g 21
<210> 1101
   <211> 21
   <212> DNA
   <213> Unknown
<220>
   <223> Found in multiple species
<400> 1101
   gagtatggca tcacataact t 21
<210> 1102
   <211> 21
   <212> DNA
   <213> Unknown
<220>
   <223> Found in multiple species
<400> 1102
   gaggctctca gagctcaccc t 21
<210> 1103
   <211> 21
   <212> DNA
   <213> Unknown
<220>
   <223> Found in multiple species
<400> 1103
   gagctctgag agcctcacta t 21
<210> 1104
   <211> 21
   <212> DNA
   <213> Unknown
<220>
   <223> Found in multiple species
<400> 1104
   gacttggaca tactatttta t 21
<210> 1105
   <211> 21
   <212> DNA
   <213> Unknown
<220>
   <223> Found in multiple species
<400> 1105
   gactcccatt acagaggttt t 21
<210> 1106
   <211> 21
   <212> DNA
   <213> Unknown
<220>
   <223> Found in multiple species
<400> 1106
   gacgaacctt agcaaccgca g 21
<210> 1107
   <211> 21
   <212> DNA
   <213> Unknown
<220>
   <223> Found in multiple species
<400> 1107
   gaccatttgt gatggcacca t 21
<210> 1108
   <211> 21
   <212> DNA
   <213> Unknown
<220>
   <223> Found in multiple species
<400> 1108
   gaatgaaaag ggttttgccc g 21
<210> 1109
   <211> 21
   <212> DNA
   <213> Unknown
<220>
   <223> Found in multiple species
<400> 1109
   gaagttccct aatcgggaag g 21
<210> 1110
   <211> 21
   <212> DNA
   <213> Unknown
<220>
   <223> Found in multiple species
<400> 1110
   gaaggatgga atgaaaaggg t 21
<210> 1111
   <211> 21
   <212> DNA
   <213> Unknown
<220>
   <223> Found in multiple species
<400> 1111
   gaacttgact cctgatagct t 21
<210> 1112
   <211> 21
   <212> DNA
   <213> Unknown
<220>
   <223> Found in multiple species
<400> 1112
   gaaccttagc aaccgcagta t 21
<210> 1113
   <211> 21
   <212> DNA
   <213> Unknown
<220>
   <223> Found in multiple species
<400> 1113
   gaaaagggtt ttgcccgttg g 21
<210> 1114
   <211> 21
   <212> DNA
   <213> Unknown
<220>
   <223> Found in multiple species
<400> 1114
   cttttcattc catccttccc g 21
<210> 1115
   <211> 21
   <212> DNA
   <213> Unknown
<220>
   <223> Found in multiple species
<400> 1115
   ctttcccata tcctttcatg g 21
<210> 1116
   <211> 21
   <212> DNA
   <213> Unknown
<220>
   <223> Found in multiple species
<400> 1116
   cttggacata ctattttatg g 21
<210> 1117
   <211> 21
   <212> DNA
   <213> Unknown
<220>
   <223> Found in multiple species
<400> 1117
   cttgactcct gatagcttta g 21
<210> 1118
   <211> 21
   <212> DNA
   <213> Unknown
<220>
   <223> Found in multiple species
<400> 1118
   cttgaacttg actcctgata g 21
<210> 1119
   <211> 21
   <212> DNA
   <213> Unknown
<220>
   <223> Found in multiple species
<400> 1119
   cttacaatgt ttgcaccacc t 21
<210> 1120
   <211> 21
   <212> DNA
   <213> Unknown
<220>
   <223> Found in multiple species
<400> 1120
   ctgctggagt tttaggtggt g 21
<210> 1121
   <211> 21
   <212> DNA
   <213> Unknown
<220>
   <223> Found in multiple species
<400> 1121
   ctgatagctt tagtgatggc g 21
<210> 1122
   <211> 21
   <212> DNA
   <213> Unknown
<220>
   <223> Found in multiple species
<400> 1122
   ctcgcatgtg cattgctata t 21
<210> 1123
   <211> 21
   <212> DNA
   <213> Unknown
<220>
   <223> Found in multiple species
<400> 1123
   ctcctgatag ctttagtgat g 21
<210> 1124
   <211> 21
   <212> DNA
   <213> Unknown
<220>
   <223> Found in multiple species
<400> 1124
   ctacatcaga cccaatacaa t 21
<210> 1125
   <211> 21
   <212> DNA
   <213> Unknown
<220>
   <223> Found in multiple species
<400> 1125
   ctaatcggga aggatggaat g 21
<210> 1126
   <211> 21
   <212> DNA
   <213> Unknown
<220>
   <223> Found in multiple species
<400> 1126
   ctaaggttcg tcagatgatc t 21
<210> 1127
   <211> 21
   <212> DNA
   <213> Unknown
<220>
   <223> Found in multiple species
<400> 1127
   cggtggaagt tccctaatcg g 21
<210> 1128
   <211> 21
   <212> DNA
   <213> Unknown
<220>
   <223> Found in multiple species
<400> 1128
   cgcttatggg attggtctca t 21
<210> 1129
   <211> 21
   <212> DNA
   <213> Unknown
<220>
   <223> Found in multiple species
<400> 1129
   cgatactgcg gttgctaagg t 21
<210> 1130
   <211> 21
   <212> DNA
   <213> Unknown
<220>
   <223> Found in multiple species
<400> 1130
   ccttatatag caatgcacat g 21
<210> 1131
   <211> 21
   <212> DNA
   <213> Unknown
<220>
   <223> Found in multiple species
<400> 1131
   cctaatcggg aaggatggaa t 21
<210> 1132
   <211> 21
   <212> DNA
   <213> Unknown
<220>
   <223> Found in multiple species
<400> 1132
   catcaaggtt atccctcaca t 21
<210> 1133
   <211> 21
   <212> DNA
   <213> Unknown
<220>
   <223> Found in multiple species
<400> 1133
   cataatgtga gggataacct t 21
<210> 1134
   <211> 21
   <212> DNA
   <213> Unknown
<220>
   <223> Found in multiple species
<400> 1134
   cataaaatag tatgtccaag t 21
<210> 1135
   <211> 21
   <212> DNA
   <213> Unknown
<220>
   <223> Found in multiple species
<400> 1135
   cagaggtttt atgagaccaa t 21
<210> 1136
   <211> 21
   <212> DNA
   <213> Unknown
<220>
   <223> Found in multiple species
<400> 1136
   cacattatgt actcttacaa t 21
<210> 1137
   <211> 21
   <212> DNA
   <213> Unknown
<220>
   <223> Found in multiple species
<400> 1137
   cacaaatggt ctttcccata t 21
<210> 1138
   <211> 21
   <212> DNA
   <213> Unknown
<220>
   <223> Found in multiple species
<400> 1138
   caattgactt ggacatacta t 21
<210> 1139
   <211> 21
   <212> DNA
   <213> Unknown
<220>
   <223> Found in multiple species
<400> 1139
   caatctgtcg atactgcggt t 21
<210> 1140
   <211> 21
   <212> DNA
   <213> Unknown
<220>
   <223> Found in multiple species
<400> 1140
   caagttatgt gatgccatac t 21
<210> 1141
   <211> 21
   <212> DNA
   <213> Unknown
<220>
   <223> Found in multiple species
<400> 1141
   caaggttatc cctcacatta t 21
<210> 1142
   <211> 21
   <212> DNA
   <213> Unknown
<220>
   <223> Found in multiple species
<400> 1142
   caagactccc attacagagg t 21
<210> 1143
   <211> 21
   <212> DNA
   <213> Unknown
<220>
   <223> Found in multiple species
<400> 1143
   caaccgcagt atcgacagat t 21
<210> 1144
   <211> 21
   <212> DNA
   <213> Unknown
<220>
   <223> Found in multiple species
<400> 1144
   attttctaca tcagacccaa t 21
<210> 1145
   <211> 21
   <212> DNA
   <213> Unknown
<220>
   <223> Found in multiple species
<400> 1145
   attggcctag gaccatacct t 21
<210> 1146
   <211> 21
   <212> DNA
   <213> Unknown
<220>
   <223> Found in multiple species
<400> 1146
   attgattgta ttgggtctga t 21
<210> 1147
   <211> 21
   <212> DNA
   <213> Unknown
<220>
   <223> Found in multiple species
<400> 1147
   attgacttgg acatactatt t 21
<210> 1148
   <211> 21
   <212> DNA
   <213> Unknown
<220>
   <223> Found in multiple species
<400> 1148
   attagggaac ttccaccgag t 21
<210> 1149
   <211> 21
   <212> DNA
   <213> Unknown
<220>
   <223> Found in multiple species
<400> 1149
   atgtttgcac cacctaaaac t 21
<210> 1150
   <211> 21
   <212> DNA
   <213> Unknown
<220>
   <223> Found in multiple species
<400> 1150
   atgtagaaaa tgacactatt t 21
<210> 1151
   <211> 21
   <212> DNA
   <213> Unknown
<220>
   <223> Found in multiple species
<400> 1151
   atggtctttc ccatatcctt t 21
<210> 1152
   <211> 21
   <212> DNA
   <213> Unknown
<220>
   <223> Found in multiple species
<400> 1152
   atggtcctag gccaattgac t 21
<210> 1153
   <211> 21
   <212> DNA
   <213> Unknown
<220>
   <223> Found in multiple species
<400> 1153
   atgggagtct tgaacttgac t 21
<210> 1154
   <211> 21
   <212> DNA
   <213> Unknown
<220>
   <223> Found in multiple species
<400> 1154
   atgggaaaga ccatttgtga t 21
<210> 1155
   <211> 21
   <212> DNA
   <213> Unknown
<220>
   <223> Found in multiple species
<400> 1155
   atgaaaaggg ttttgcccgt t 21
<210> 1156
   <211> 21
   <212> DNA
   <213> Unknown
<220>
   <223> Found in multiple species
<400> 1156
   atccttcccg attagggaac t 21
<210> 1157
   <211> 21
   <212> DNA
   <213> Unknown
<220>
   <223> Found in multiple species
<400> 1157
   atccctcaca ttatgtactc t 21
<210> 1158
   <211> 21
   <212> DNA
   <213> Unknown
<220>
   <223> Found in multiple species
<400> 1158
   atcactaaag ctatcaggag t 21
<210> 1159
   <211> 21
   <212> DNA
   <213> Unknown
<220>
   <223> Found in multiple species
<400> 1159
   atcaaggtta tccctcacat t 21
<210> 1160
   <211> 21
   <212> DNA
   <213> Unknown
<220>
   <223> Found in multiple species
<400> 1160
   agttttcaat ctgtcgatac t 21
<210> 1161
   <211> 21
   <212> DNA
   <213> Unknown
<220>
   <223> Found in multiple species
<400> 1161
   aggttatccc tcacattatg t 21
<210> 1162
   <211> 21
   <212> DNA
   <213> Unknown
<220>
   <223> Found in multiple species
<400> 1162
   aggtatggtc ctaggccaat t 21
<210> 1163
   <211> 21
   <212> DNA
   <213> Unknown
<220>
   <223> Found in multiple species
<400> 1163
   aggatggaat gaaaagggtt t 21
<210> 1164
   <211> 21
   <212> DNA
   <213> Unknown
<220>
   <223> Found in multiple species
<400> 1164
   aggatatggg aaagaccatt t 21
<210> 1165
   <211> 21
   <212> DNA
   <213> Unknown
<220>
   <223> Found in multiple species
<400> 1165
   aggaccatac cttattccct t 21
<210> 1166
   <211> 21
   <212> DNA
   <213> Unknown
<220>
   <223> Found in multiple species
<400> 1166
   agactcccat tacagaggtt t 21
<210> 1167
   <211> 21
   <212> DNA
   <213> Unknown
<220>
   <223> Found in multiple species
<400> 1167
   actgctggag ttttaggtgg t 21
<210> 1168
   <211> 21
   <212> DNA
   <213> Unknown
<220>
   <223> Found in multiple species
<400> 1168
   actgcggttg ctaaggttcg t 21
<210> 1169
   <211> 21
   <212> DNA
   <213> Unknown
<220>
   <223> Found in multiple species
<400> 1169
   acgggcaaaa cccttttcat t 21
<210> 1170
   <211> 21
   <212> DNA
   <213> Unknown
<220>
   <223> Found in multiple species
<400> 1170
   accatttgtg atggcaccat t 21
<210> 1171
   <211> 21
   <212> DNA
   <213> Unknown
<220>
   <223> Found in multiple species
<400> 1171
   aattgacttg gacatactat t 21
<210> 1172
   <211> 21
   <212> DNA
   <213> Unknown
<220>
   <223> Found in multiple species
<400> 1172
   aatggtcttt cccatatcct t 21
<210> 1173
   <211> 21
   <212> DNA
   <213> Unknown
<220>
   <223> Found in multiple species
<400> 1173
   aaggatggaa tgaaaagggt t 21
<210> 1174
   <211> 21
   <212> DNA
   <213> Unknown
<220>
   <223> Found in multiple species
<400> 1174
   aagactccca ttacagaggt t 21
<210> 1175
   <211> 21
   <212> DNA
   <213> Unknown
<220>
   <223> Found in multiple species
<400> 1175
   aacttgactc ctgatagctt t 21
<210> 1176
   <211> 25
   <212> DNA
   <213> Amaranthus palmeri
<400> 1176
   ttttattcta aagttgcttc ggagg 25
<210> 1177
   <211> 25
   <212> DNA
   <213> Amaranthus palmeri
<400> 1177
   ccgtcaagaa gggggcacac attgt 25
<210> 1178
   <211> 25
   <212> DNA
   <213> Amaranthus palmeri
<400> 1178
   gaatgatgtc tctagtggga aactc 25
<210> 1179
   <211> 25
   <212> DNA
   <213> Amaranthus palmeri
<400> 1179
   gattccgaga tgtttaatgt tgttg 25
<210> 1180
   <211> 25
   <212> DNA
   <213> Amaranthus palmeri
<400> 1180
   cggaccttaa agttccttat atagc 25
<210> 1181
   <211> 25
   <212> DNA
   <213> Amaranthus palmeri
<400> 1181
   aatgcacatg cgaggagatc cgact 25
<210> 1182
   <211> 25
   <212> DNA
   <213> Amaranthus palmeri
<400> 1182
   tcaatgcaaa actctgagaa cttga 25
<210> 1183
   <211> 25
   <212> DNA
   <213> Amaranthus palmeri
<400> 1183
   cctacaatga tgtttgtaag caagt 25
<210> 1184
   <211> 25
   <212> DNA
   <213> Amaranthus palmeri
<400> 1184
   ggcttcggag ttgagttcta gggtc 25
<210> 1185
   <211> 25
   <212> DNA
   <213> Amaranthus palmeri
<400> 1185
   atagatgcag aattatcggg aattc 25
<210> 1186
   <211> 25
   <212> DNA
   <213> Amaranthus palmeri
<400> 1186
   ctgcttggag gatagttatt gatcc 25
<210> 1187
   <211> 25
   <212> DNA
   <213> Amaranthus palmeri
<400> 1187
   cggcatcgga ttttctaaga atacg 25
<210> 1188
   <211> 25
   <212> DNA
   <213> Amaranthus palmeri
<400> 1188
   aatcaaaatt tggaaattct tagtg 25
<210> 1189
   <211> 25
   <212> DNA
   <213> Amaranthus palmeri
<400> 1189
   gtttacaaaa gatacgggaa gagat 25
<210> 1190
   <211> 25
   <212> DNA
   <213> Amaranthus palmeri
<400> 1190
   agctaagaag agtttggcgg tggct 25
<210> 1191
   <211> 25
   <212> DNA
   <213> Amaranthus palmeri
<400> 1191
   cattgcccct tgctaattgg acctt 25
<210> 1192
   <211> 25
   <212> DNA
   <213> Amaranthus palmeri
<400> 1192
   caagaaagag gtttctgggc gagat 25
<210> 1193
   <211> 25
   <212> DNA
   <213> Amaranthus palmeri
<400> 1193
   ttgtgagcgc cctgtagcag ctgac 25
<210> 1194
   <211> 25
   <212> DNA
   <213> Amaranthus palmeri
<400> 1194
   agggatcctg ctaccattgc ttcta 25
<210> 1195
   <211> 25
   <212> DNA
   <213> Amaranthus palmeri
<400> 1195
   taactgctgg agttttaggt ggtgc 25
<210> 1196
   <211> 25
   <212> DNA
   <213> Amaranthus palmeri
<400> 1196
   aaacattgta agagtacata atgtt 25
<210> 1197
   <211> 25
   <212> DNA
   <213> Amaranthus palmeri
<400> 1197
   agggataacc ttgatgctgt caagt 25
<210> 1198
   <211> 25
   <212> DNA
   <213> Amaranthus palmeri
<400> 1198
   tatgtgatgc catactcgga aaaac 25
<210> 1199
   <211> 25
   <212> DNA
   <213> Amaranthus palmeri
<400> 1199
   tgattaactg cttgtttgta ccacc 25
<210> 1200
   <211> 25
   <212> DNA
   <213> Amaranthus palmeri
<400> 1200
   ttgtgaatga tgtctagtgg aaggt 25
<210> 1201
   <211> 25
   <212> DNA
   <213> Amaranthus palmeri
<400> 1201
   tcgattggga ttatgatgaa gtccg 25
<210> 1202
   <211> 25
   <212> DNA
   <213> Amaranthus palmeri
<400> 1202
   ttagtttgct cgaaagtaga gcttt 25
<210> 1203
   <211> 25
   <212> DNA
   <213> Amaranthus palmeri
<400> 1203
   agcccgtaag gctatagttc ttaca 25
<210> 1204
   <211> 25
   <212> DNA
   <213> Amaranthus palmeri
<400> 1204
   gatgtagaat cattggctat ttgcc 25
<210> 1205
   <211> 25
   <212> DNA
   <213> Amaranthus palmeri
<400> 1205
   tatttctgct tagaagatca tagca 25
<210> 1206
   <211> 25
   <212> DNA
   <213> Amaranthus palmeri
<400> 1206
   ttgatccaga ctttggattc catgt 25
<210> 1207
   <211> 25
   <212> DNA
   <213> Amaranthus palmeri
<400> 1207
   agaattatgt tgcgactcat ggtcg 25
<210> 1208
   <211> 25
   <212> DNA
   <213> Amaranthus palmeri
<400> 1208
   aattggccct ttgaggaatt gttga 25
<210> 1209
   <211> 25
   <212> DNA
   <213> Amaranthus palmeri
<400> 1209
   agttccttga gtctatggtt caatc 25
<210> 1210
   <211> 25
   <212> DNA
   <213> Amaranthus palmeri
<400> 1210
   ggctcggttc gatgaaggtt ggact 25
<210> 1211
   <211> 25
   <212> DNA
   <213> Amaranthus palmeri
<400> 1211
   gtgtcgagac aagatgggtc aaaga 25
<210> 1212
   <211> 25
   <212> DNA
   <213> Amaranthus palmeri
<400> 1212
   gaccaatgga caagaagtcg acctt 25
<210> 1213
   <211> 25
   <212> DNA
   <213> Amaranthus palmeri
<400> 1213
   gggcaagctt gatcgagtta cttgg 25
<210> 1214
   <211> 25
   <212> DNA
   <213> Amaranthus palmeri
<400> 1214
   acaagcagat aacagcaggc tctgg 25
<210> 1215
   <211> 25
   <212> DNA
   <213> Amaranthus palmeri
<400> 1215
   cgaccattag acatacatat cattt 25
<210> 1216
   <211> 25
   <212> DNA
   <213> Amaranthus palmeri
<400> 1216
   gttttttgaa gtcagatctg caatc 25
<210> 1217
   <211> 25
   <212> DNA
   <213> Amaranthus palmeri
<400> 1217
   aatcagttgc aatggacaaa ccata 25
<210> 1218
   <211> 25
   <212> DNA
   <213> Amaranthus palmeri
<400> 1218
   tacggttatt agttgttcct gtcac 25
<210> 1219
   <211> 25
   <212> DNA
   <213> Amaranthus palmeri
<400> 1219
   tgtttgagtt gaattagatc atgca 25
<210> 1220
   <211> 25
   <212> DNA
   <213> Amaranthus palmeri
<400> 1220
   aacattgtaa gaatcttgat catgt 25
<210> 1221
   <211> 25
   <212> DNA
   <213> Amaranthus palmeri
<400> 1221
   gaagctatgt gatactgtac ttctc 25
<210> 1222
   <211> 25
   <212> DNA
   <213> Amaranthus palmeri
<400> 1222
   aaaatgaaat ctttgatatg atgtt 25

## Description

Sequences of the sequence listing that are not cited in the claims are submitted for comparative purposes.

### FIELD

The methods and compositions generally relate to the field of weed management. More specifically, relate to 7,8-dihydropteroate synthase inhibitors (DHPS) genes in plants and compositions containing polynucleotide molecules for modulating and/or regulating their expression. Further provided are methods and compositions useful for weed control.

### BACKGROUND

Weeds are plants that compete with cultivated plants in an agronomic environment and cost farmers billions of dollars annually in crop losses and the expense of efforts to keep weeds under control. Weeds also serve as hosts for crop diseases and insect pests. The losses caused by weeds in agricultural production environments include decreases in crop yield, reduced crop quality, increased irrigation costs, increased harvesting costs, reduced land value, injury to livestock, and crop damage from insects and diseases harbored by the weeds. The principal means by which weeds cause these effects are: 1) competing with crop plants for water, nutrients, sunlight and other essentials for growth and development, 2) production of toxic or irritant chemicals that cause human or animal health problem, 3) production of immense quantities of seed or vegetative reproductive parts or both that contaminate agricultural products and perpetuate the species in agricultural lands, and 4) production on agricultural and nonagricultural lands of vast amounts of vegetation that must be disposed of. Herbicide tolerant weeds are a problem with nearly all herbicides in use, there is a need to effectively manage these weeds. There are over 365 weed biotypes currently identified as being herbicide resistant to one or more herbicides by the Herbicide Resistance Action Committee (HRAC), the North American Herbicide Resistance Action Committee (NAHRAC), and the Weed Science Society of America (WSSA).

The 7,8-dihydropteroate synthase (DHPS) is an enzyme involved in folic acid synthesis which is needed for purine nucleotide biosynthesis. This enzyme is the target of herbicides that include the carbamate chemical family.

### BRIEF DESCRIPTION OF THE FIGURES

The following drawings form part of the present specification and are included to further demonstrate certain methods, compositions or results. They may be better understood by reference to one or more of these drawings in combination with the detailed description of specific embodiments presented herein. The invention can be more fully understood from the following description of the figures:
FIGURE 1. Treatment with oligonucleotide pools followed by Prowl (pendimethalin)

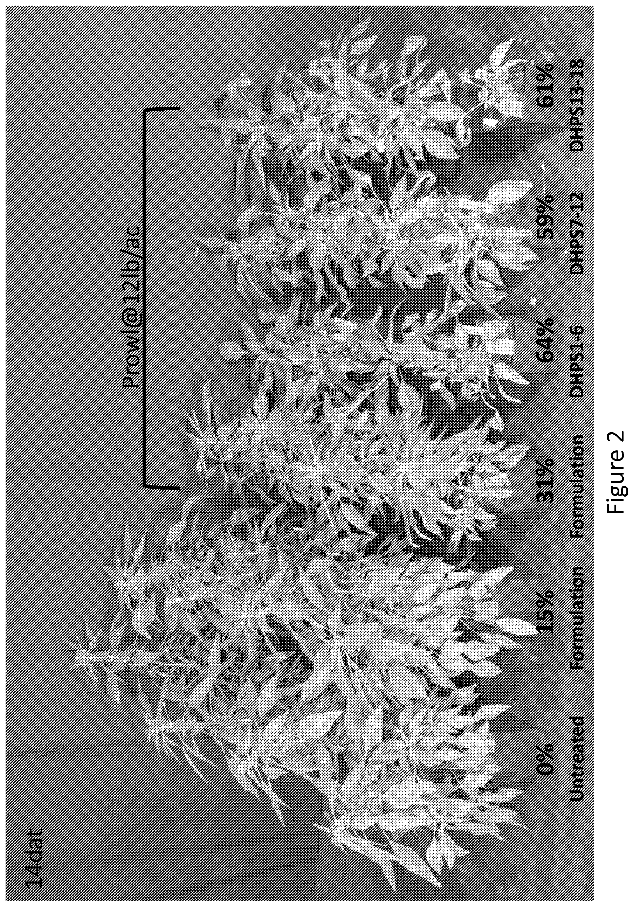

FIGURE 2. Treatment of Palmer Amaranth with 3 oligonucleotide pools followed by Prowl herbicide at 13.45 kg/ha (12 lb/ac) rate

### SUMMARY

In one aspect, the invention provides a method of plant control comprising: treating a plant with a composition comprising a double-stranded RNA (dsRNA) polynucleotide and a transfer agent, wherein said dsRNA polynucleotide is identical or complementary to an RNA sequence of a 7,8-dihydropteroate synthase (DHPS) gene sequence, wherein said DHPS gene sequence is selected from the group consisting of SEQ ID NOs:1, 2, 5, 9, 11, 18, 19, and a polynucleotide fragment thereof with at least 18 contiguous nucleotides from said DHPS gene sequence, wherein said transfer agent is an organosilicone composition or an organosilicone compound contained therein and conditions the surface of said plant for permeation by said dsRNA polynucleotide, and whereby said plant's growth, development, or reproductive ability is suppressed or delayed or said plant is more sensitive to a DHPS inhibitor herbicide or a mitosis inhibitor herbicide as a result of said dsRNA polynucleotide containing composition relative to an untreated plant.

In a related aspect, the present invention provides a composition comprising a dsRNA polynucleotide and a transfer agent, wherein said dsRNA polynucleotide is identical or complementary to an RNA sequence of a DHPS gene sequence, wherein said DHPS gene sequence is selected from the group consisting of SEQ ID NOs:1, 2, 5, 9, 11, 18, 19, and a polynucleotide fragment thereof with at least 18 contiguous nucleotides from said DHPS gene sequence, wherein said transfer agent is an organosilicone composition or an organosilicone compound contained therein and conditions the surface of a plant for permeation by said dsRNA polynucleotide, and whereby said plant treated with said composition has its growth, development, or reproductive ability regulated, suppressed or delayed or said plant is more sensitive to a DHPS inhibitor herbicide or a mitosis inhibitor herbicide as a result of said dsRNA polynucleotide containing composition relative to an untreated plant.

In a further embodiment the present invention also provides a method of reducing expression of a DHPS gene in a plant comprising: external application to said plant of a composition comprising a dsRNA polynucleotide and a transfer agent, wherein said dsRNA polynucleotide is identical or complementary to an RNA sequence of a DHPS gene sequence, wherein said DHPS gene sequence is selected from the group consisting of SEQ ID NOs: 1, 2, 5, 9, 11, 18, 19, and a polynucleotide fragment thereof with at least 18 contiguous nucleotides from said DHPS gene sequence, wherein said transfer agent is an organosilicone composition or an organosilicone compound contained therein and conditions the surface of said plant for permeation by said dsRNA polynucleotide, and whereby said expression of said DHPS gene is reduced relative to an untreated plant.

Alternatively, the present invention provides methods of identifying dsRNA polynucleotides useful in modulating DHPS gene expression when externally treating a plant comprising: a) providing a plurality of dsRNA polynucleotides that comprise a region identical or complementary to a polynucleotide fragment of at least 18 contiguous nucleotides in length that is identical or complementary to a DHPS gene sequence selected from the group consisting of SEQ ID NOs:1, 2, 5, 9, 11, 18, and 19; b) externally treating said plant with a composition comprising one or more of said dsRNA polynucleotides and a transfer agent, wherein said transfer agent is an organosilicone composition or an organosilicone compound contained therein and conditions the surface of said plant for permeation by said one or more of said dsRNA polynucleotides; c) analyzing said plant, or extract for modulation of DHPS gene expression, and d) said plant treated with said composition has its growth, development or reproductive ability regulated, suppressed, or delayed or said plant is more sensitive to a DHPS inhibitor herbicide or a mitosis inhibitor herbicide as a result of said composition, relative to an untreated plant.

In yet another embodiment, the present invention provides an agricultural chemical composition comprising an admixture of a dsRNA polynucleotide, a transfer agent, a DHPS inhibitor herbicide or a mitosis inhibitor herbicide, and a co-herbicide, wherein said dsRNA polynucleotide is identical or complementary to a portion of an RNA sequence of a DHPS gene sequence, wherein said DHPS gene sequence is selected from the group consisting of SEQ ID NOs: 1, 2, 5, 9, 11, 18, 19, and a polynucleotide fragment thereof with at least 18 contiguous nucleotides from said DHPS gene sequence, wherein said transfer agent is an organosilicone composition or an organosiliocone compound contained therein and conditions a surface of a plant for permeation by said dsRNA polynucleotide, and whereby said plant treated with said composition has its growth, development, or reproductive ability regulated, suppressed, or delayed or said plant is more sensitive to said DHPS inhibitor herbicide or said mitosis inhibitor herbicide as a result of said dsRNA polynucleotide containing composition, relative to an untreated plant.

In a further aspect the invention provides an agricultural chemical composition comprising an admixture of a dsRNA polynucleotide, a transfer agent, a DHPS inhibitor herbicide, and a pesticide, wherein said dsRNA polynucleotide is identical or complementary to a portion of an RNA sequence of a DHPS gene sequence, wherein said DHPS gene sequence is selected from the group consisting of SEQ ID NOs:1, 2, 5, 9, 11, 18, 19, and a polynucleotide fragment thereof with at least 18 contiguous nucleotides from said DHPS gene sequence, wherein said transfer agent is an organosilicone composition or an organosilicone compound contained therein and conditions a surface of a plant for permeation by said dsRNA polynucleotide, whereby said plant treated with said composition has its growth, development, or reproductive ability regulated, suppressed, or delayed or said plant is more sensitive to said DHPS inhibitor herbicide or a mitosis inhibitor herbicide as a result of said dsRNA polynucleotide containing composition relative to an untreated plant.

### DETAILED DESCRIPTION

Provided are methods and compositions containing a polynucleotide that provide for regulation, repression or delay of 7,8-dihydropteroate synthase (DHPS) gene expression and enhanced control of weedy plant species amd importantly DHPS inhibitor resistant weed biotypes. Aspects of the method can be applied to manage various weedy plants in agronomic and other cultivated environments.

The following definitions and methods are provided to better define the present invention and to guide those of ordinary skill in the art. Unless otherwise noted, terms are to be understood according to conventional usage by those of ordinary skill in the relevant art. Where a term is provided in the singular, the inventors also contemplate aspects described by the plural of that term.

By "non-transcribable" polynucleotides is meant that the polynucleotides do not comprise a complete polymerase II transcription unit.

As used herein "solution" refers to homogeneous mixtures and non-homogeneous mixtures such as suspensions, colloids, micelles, and emulsions.

Weedy plants are plants that compete with cultivated plants, those of particular importance include, but are not limited to important invasive and noxious weeds and herbicide resistant biotypes in crop production, such as, *Amaranthus* species *-A. albus*, *A. blitoides, A. hybridus*, *A. palmeri*, *A. powellii, A. retroflexus*, *A. spinosus, A. tuberculatus*, and *A. viridis*; *Ambrosia* species - *A. trifida*, *A. artemisifolia*; *Lolium* species *-L. multiflorum*, *L. rigidium*, *L perenne*; *Digitaria* species *-D. insularis*; *Euphorbia* species *-E. heterophylla*; *Kochia species - K. scoparia*; *Sorghum* species *-S. halepense*; *Conyza* species *-C. bonariensis*, *C. canadensis*, *C. sumatrensis*; *Chloris* species *-C. truncate*; *Echinochola* species - *E. colona*, *E. crus*-*galli*; *Eleusine species -E. indica; Poa* species *-P. annua*; *Plantago* species *-P. lanceolata*; *Avena species - A. fatua*; *Chenopodium* species - *C*. *album*; *Setaria* species - *S. viridis*, *Abutilon theophrasti*, *Ipomoea* species, *Sesbania,* species, *Cassia* species, *Sida* species, *Brachiaria*, species and *Solanum* species.

Additional weedy plant species found in cultivated areas include *Alopecurus myosuroides*, *Avena sterilis*, *Avena sterilis ludoviciana, Brachiaria plantaginea, Bromus diandrus*, *Bromus ragidus*, *Cynosurus echinatus*, *Digitaria ciliaris*, *Digitaria ischaemum*, *Digitaria sanguinalis*, *Echinochloa oryzicola, Echinochloa phyllopogon, Eriochloa punctata*, *Hordeum glaucum, Hordeum leporinum, Ischaemum rugosum, Leptochloa chinensis, Lolium persicum*, , *Phalaris minor, Phalaris paradoxa*, *Rottboellia exalta*, *Setaria faberi*, *Setaria viridis var, robusta-alba schreiber, Setaria viridis var, robusta -purpurea*, *Snowdenia polystachea, Sorghum sudanese, Alisma plantago-aquatica*, *Amaranthus lividus, Amaranthus quitensis, Ammania auriculata*, *Ammania coccinea, Anthemis cotula, Apera spica-venti*, *Bacopa rotundifolia*, *Bidens pilosa, Bidens subalternans, Brassica tournefortii, Bromus tectorum, Camelina microcarpa, Chrysanthemum coronarium, Cuscuta campestris, Cyperus difformis*, *Damasonium minus, Descurainia sophia, Diplotaxis tenuifolia, Echium plantagineum, Elatine triandra var, pedicellata, Euphorbia heterophylla, Fallopia convolvulus, Fimbristylis miliacea*, *Galeopsis tetrahit, Galium spurium, Helianthus annuus*, *Iva xanthifolia, Ixophorus unisetus, Ipomoea indica, Ipomoea purpurea, Ipomoea sepiaria*, *Ipomoea aquatic*, *Ipomoea triloba, Lactuca serriola, Limnocharis flava*, *Limnophila erecta*, *Limnophila sessiliflora, Lindernia dubia*, *Lindernia dubia var, major, Lindernia micrantha*, *Lindernia procumbens, Mesembryanthemum crystallinum*, *Monochoria korsakowii*, *Monochoria vaginalis, Neslia paniculata, Papaver rhoeas, Parthenium hysterophorus*, *Pentzia suffruticosa*, *Phalaris minor, Raphanus raphanistrum, Raphanus sativus, Rapistrum rugosum, Rotala indica var, uliginosa, Sagittaria guyanensis, Sagittaria montevidensis, Sagittaria pygmaea, Salsola iberica*, *Scirpusjuncoides var, ohwianus, Scirpus mucronatus, Setaria lutescens, Sida spinosa, Sinapis arvensis*, *Sisymbrium orientale, Sisymbrium thellungii, Solanum ptycanthum, Sonchus asper*, *Sonchus oleraceus, Sorghum bicolor, Stellaria media*, *Thlaspi arvense*, *Xanthium strumarium, Arctotheca calendula, Conyza sumatrensis, Crassocephalum crepidiodes, Cuphea carthagenenis, Epilobium adenocaulon*, *Erigeron philadelphicus, Landoltia punctata, Lepidium virginicum, Monochoria korsakowii*, *Solanum americanum*, *Solanum nigrum, Vulpia bromoides*, *Youngia japonica, Hydrilla verticillata*, *Carduus nutans, Carduus pycnocephalus, Centaurea solstitialis, Cirsium arvense*, *Commelina diffusa*, *Convolvulus arvensis*, *Daucus carota, Digitaria ischaemum*, *Echinochloa crus-pavonis*, *Fimbristylis miliacea*, *Galeopsis tetrahit, Galium spurium, Limnophila erecta*, *Matricaria perforate, Papaver rhoeas, Ranunculus acris*, *Soliva sessilis, Sphenoclea zeylanica, Stellaria media*, *Nassella trichotoma, Stipa neesiana, Agrostis stolonifera, Polygonum aviculare*, *Alopecurus japonicus*, *Beckmannia syzigachne, Bromus tectorum, Chloris inflate, Echinochloa erecta*, *Portulaca oleracea, and Senecio vulgaris.* It is believed that all plants contain an DHPS gene in their genome, which can be isolated and polynucleotides made according to the methods that are useful for regulating, suppressing or delaying the expression of the target DHPS gene in the plants and the growth or development of the treated plants.

Some cultivated plants may also be weedy plants when they occur in unwanted environments. For example, corn plants growing in a soybean field. Transgenic crops with one or more herbicide tolerances will need specialized methods of management to control weeds and volunteer crop plants.

A "trigger" or "trigger polynucleotide" is a polynucleotide molecule that is homologous or complementary to a target gene polynucleotide. The trigger polynucleotide molecules modulate expression of the target gene when topically applied to a plant surface with a transfer agent, whereby a plant treated with said composition has its growth or development or reproductive ability regulated, suppressed or delayed or said plant is more sensitive to a DHPS inhibitor herbicide or mitosis inhibitor herbicide as a result of said polynucleotide containing composition relative to a plant not treated with a composition containing the trigger molecule.

It is contemplated that the compositions will contain one or more polynucleotides and one or more herbicides that include but not limited to DHPS gene trigger polynucleotides and a DHPS inhibitor herbicide and anyone or more additional herbicide target gene trigger polynucleotides and the related herbicides and anyone or more additional essential gene trigger polynucleotides. Essential genes are genes in a plant that provide key enzymes or other proteins, for example, a biosynthetic enzyme, metabolizing enzyme, receptor, signal transduction protein, structural gene product, transcription factor, or transport protein; or regulating RNAs, such as, microRNAs, that are essential to the growth or survival of the organism or cell or involved in the normal growth and development of the plant (Meinke, et al., Trends Plant Sci. 2008 Sep;13(9):483-91). The suppression of an essential gene enhances the effect of a herbicide that affects the function of a gene product different than the suppressed essential gene. The compositions can include various trigger polynucleotides that modulate the expression of an essential gene other than DHPS.

Herbicides, for which transgenes for plant tolerance have been demonstrated, include but are not limited to: auxin-like herbicides, glyphosate, glufosinate, sulfonylureas, imidazolinones, bromoxynil, delapon, dicamba, cyclohezanedione, protoporphyrionogen oxidase inhibitors, 4-hydroxyphenyl-pyruvate-dioxygenase inhibitors herbicides. For example, transgenes and their polynucleotide molecules that encode proteins involved in herbicide tolerance are known in the art, and include, but are not limited to an 5-enolpyruvylshikimate-3-phosphate synthase (EPSPS), for example, as more fully described in U.S. Pat. Nos. 7,807,791 (SEQ ID NO:5); 6,248,876 B1; 5,627,061; 5,804,425; 5,633,435; 5,145,783; 4,971,908; 5,392,910; 5,188,642; 4,940,835; 5,866,775; 6,225,114 B1; 6,130,366; 5,390,667; 4,535,060; 4,769,061; 5,633,448; 5,510,471; U.S. Pat. No. RE36,449; U.S. Pat. Nos. RE37,287 E; and 5,491,288; tolerance to sulfonylurea and/or imidazolinone, for example, as described more fully in U.S. Pat. Nos. 5,605,011; 5,013,659; 5,141,870; 5,767,361; 5,739,180; 5,304,732; 4,761,373; 5,339,107; 5,928,937; and 5,378,824; and international publication WO 96/33270; tolerance to hydroxyphenylpyruvatedioxygenases inhibitiong herbicides in plants are described in U.S. Pat. Nos. 6,245,968 B1; 6,268,549, and 6,069,115; US Pat.Pub. 20110191897 and US7,392,379 SEQ ID NO:3; US7,935,869; US7,304,209, SEQ ID NO:1, 3,5 and 15; aryloxyalkanoate dioxygenase polynucleotides, which confer tolerance to 2,4-D and other phenoxy auxin herbicides as well as to aryloxyphenoxypropionate herbicides as described, for example, in WO2005/107437; US7,838,733 SEQ ID NO:5;) and dicamba-tolerance polynucleotides as described, for example, in Herman et al. (2005) J. Biol. Chem. 280: 24759-24767. Other examples of herbicide-tolerance traits include those conferred by polynucleotides encoding an exogenous phosphinothricin acetyltransferase, as described in U.S. Pat. Nos. 5,969,213; 5,489,520; 5,550,398; 5,874,265; 5,919,675; 5,561,236; 5,648,477; 5,639,024; 6,177,616; and 5,879,903. Plants containing an exogenous phosphinothricin acetyltransferase can exhibit improved tolerance to glufosinate herbicides, which inhibit the enzyme glutamine synthase. Additionally, herbicide-tolerance polynucleotides include those conferred by polynucleotides conferring altered protoporphyrinogen oxidase (protox) activity, as described in U.S. Pat. Nos. 6,288,306 B1; 6,282,837 B1; and 5,767,373; and WO 01/12825. Plants containing such polynucleotides can exhibit improved tolerance to any of a variety of herbicides which target the protox enzyme (also referred to as protox inhibitors). Polynucleotides encoding a glyphosate oxidoreductase and a glyphosate-N-acetyl transferase (GOX described in U.S. Patent 5,393,175 and GAT described in U.S. Patent publication 20030083480, dicamba monooxygenase U.S. Patent publication 20030135879
; a polynucleotide molecule encoding bromoxynil nitrilase (*Bxn* described in U.S. Patent No. 4,810,648 for Bromoxynil tolerance
; a polynucleotide molecule encoding phytoene desaturase (*crtI*) described in Misawa et al, (1993) Plant J. 4:833-840 and Misawa et al, (1994) Plant J. 6:481-489 for norflurazon tolerance; a polynucleotide molecule encoding acetohydroxyacid synthase (AHAS, *aka* ALS) described in Sathasiivan et al. (1990) Nucl. Acids Res. 18:398-2193 for tolerance to sulfonylurea herbicides; and the *bar* gene described in DeBlock, et al. (1987) EMBO J. 6:2513-2519 for glufosinate and bialaphos tolerance. The transgenic coding regions and regulatory elements of the herbicide tolerance genes are targets in which polynucleotide triggers and herbicides can be included in the compositions.

DHPS inhibitor herbicides include but are not limited to carbamates and asulam. Mitosis inhibitor herbicides include but are not limited to dinitroaniline herbicides for example benfluralin, butralin, dinitramine, ethalfluralin, oryzalin, pendimethalin, and trifluralin. Additional mitosis inhibitor herbicides also include but are not limited to Phosphoroamidates, Pyridines, Benzamides, and Benzenedicarboxylic acids.

Numerous herbicides with similar or different modes of action (herein referred to as co-herbicides) are available that can be added to the compositions, for example, members of the herbicide families that include but are not limited to amide herbicides, aromatic acid herbicides, arsenical herbicides, benzothiazole herbicides, benzoylcyclohexanedione herbicides, benzofuranyl alkylsulfonate herbicides, carbamate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides. In particular, the rates of use of the added herbicides can be reduced in compositions comprising the polynucleotides. Use rate reductions of the additional added herbicides can be 10-25 percent, 26-50 percent, 51-75 percent or more can be achieved that enhance the activity of the polynucleotides and herbicide composition and is contemplated. Representative co-herbicides of the families include but are not limited to acetochlor, acifluorfen, acifluorfen-sodium, aclonifen, acrolein, alachlor, alloxydim, allyl alcohol, ametryn, amicarbazone, amidosulfuron, aminopyralid, amitrole, ammonium sulfamate, anilofos, asulam, atraton, atrazine, azimsulfuron, BCPC, beflubutamid, benazolin, benfluralin, benfuresate, bensulfuron, bensulfuron-methyl, bensulide, bentazone, benzfendizone, benzobicyclon, benzofenap, bifenox, bilanafos, bispyribac, bispyribac-sodium, borax, bromacil, bromobutide, bromoxynil, butachlor, butafenacil, butamifos, butralin, butroxydim, butylate, cacodylic acid, calcium chlorate, cafenstrole, carbetamide, carfentrazone, carfentrazone-ethyl, CDEA, CEPC, chlorflurenol, chlorflurenol-methyl, chloridazon, chlorimuron, chlorimuron-ethyl, chloroacetic acid, chlorotoluron, chlorpropham, chlorsulfuron, chlorthal, chlorthal-dimethyl, cinidon-ethyl, cinmethylin, cinosulfuron, cisanilide, clethodim, clodinafop, clodinafop-propargyl, clomazone, clomeprop, clopyralid, cloransulam, cloransulam-methyl, CMA, 4-CPB, CPMF, 4-CPP, CPPC, cresol, cumyluron, cyanamide, cyanazine, cycloate, cyclosulfamuron, cycloxydim, cyhalofop, cyhalofop-butyl, 2,4-D, 3,4-DA, daimuron, dalapon, dazomet, 2,4-DB, 3,4-DB, 2,4-DEB, desmedipham, dicamba, dichlobenil, ortho-dichlorobenzene, para-dichlorobenzene, dichlorprop, dichlorprop-P, diclofop, diclofop-methyl, diclosulam, difenzoquat, difenzoquat metilsulfate, diflufenican, diflufenzopyr, dimefuron, dimepiperate, dimethachlor, dimethametryn, dimethenamid, dimethenamid-P, dimethipin, dimethylarsinic acid, dinitramine, dinoterb, diphenamid, diquat, diquat dibromide, dithiopyr, diuron, DNOC, 3,4-DP, DSMA, EBEP, endothal, EPTC, esprocarb, ethalfluralin, ethametsulfuron, ethametsulfuron-methyl, ethofumesate, ethoxyfen, ethoxysulfuron, etobenzanid, fenoxaprop-P, fenoxaprop-P-ethyl, fentrazamide, ferrous sulfate, flamprop-M, flazasulfuron, florasulam, fluazifop, fluazifop-butyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, fluoroglycofen, fluoroglycofen-ethyl, flupropanate, flupyrsulfuron, flupyrsulfuron-methyl-sodium, flurenol, fluridone, fluorochloridone, fluoroxypyr, flurtamone, fluthiacet, fluthiacet-methyl, fomesafen, foramsulfuron, fosamine, glufosinate, glufosinate-ammonium, glyphosate, halosulfuron, halosulfuron-methyl, haloxyfop, haloxyfop-P, HC-252, hexazinone, imazamethabenz, imazamethabenz-methyl, imazamox, imazapic, imazapyr, imazaquin, imazethapyr, imazosulfuron, indanofan, iodomethane, iodosulfuron, iodosulfuron-methyl-sodium, ioxynil, isoproturon, isouron, isoxaben, isoxachlortole, isoxaflutole, karbutilate, lactofen, lenacil, linuron, MAA, MAMA, MCPA, MCPA-thioethyl, MCPB, mecoprop, mecoprop-P, mefenacet, mefluidide, mesosulfuron, mesosulfuron-methyl, mesotrione, metam, metamifop, metamitron, metazachlor, methabenzthiazuron, methylarsonic acid, methyldymron, methyl isothiocyanate, metobenzuron, metolachlor, S-metolachlor, metosulam, metoxuron, metribuzin, metsulfuron, metsulfuron-methyl, MK-66, molinate, monolinuron, MSMA, naproanilide, napropamide, naptalam, neburon, nicosulfuron, nonanoic acid, norflurazon, oleic acid (fatty acids), orbencarb, orthosulfamuron, oryzalin, oxadiargyl, oxadiazon, oxasulfuron, oxaziclomefone, oxyfluorfen, paraquat, paraquat dichloride, pebulate, pendimethalin, penoxsulam, pentachlorophenol, pentanochlor, pentoxazone, pethoxamid, petrolium oils, phenmedipham, phenmedipham-ethyl, picloram, picolinafen, pinoxaden, piperophos, potassium arsenite, potassium azide, pretilachlor, primisulfuron, primisulfuron-methyl, prodiamine, profluazol, profoxydim, prometon, prometryn, propachlor, propanil, propaquizafop, propazine, propham, propisochlor, propoxycarbazone, propoxycarbazone-sodium, propyzamide, prosulfocarb, prosulfuron, pyraclonil, pyraflufen, pyraflufen-ethyl, pyrazolynate, pyrazosulfuron, pyrazosulfuron-ethyl, pyrazoxyfen, pyribenzoxim, pyributicarb, pyridafol, pyridate, pyriftalid, pyriminobac, pyriminobac-methyl, pyrimisulfan, pyrithiobac, pyrithiobac-sodium, quinclorac, quinmerac, quinoclamine, quizalofop, quizalofop-P, rimsulfuron, sethoxydim, siduron, simazine, simetryn, SMA, sodium arsenite, sodium azide, sodium chlorate, sulcotrione, sulfentrazone, sulfometuron, sulfometuron-methyl, sulfosate, sulfosulfuron, sulfuric acid, tar oils, 2,3,6-TBA, TCA, TCA-sodium, tebuthiuron, tepraloxydim, terbacil, terbumeton, terbuthylazine, terbutryn, thenylchlor, thiazopyr, thifensulfuron, thifensulfuron-methyl, thiobencarb, tiocarbazil, topramezone, tralkoxydim, tri-allate, triasulfuron, triaziflam, tribenuron, tribenuron-methyl, tricamba, triclopyr, trietazine, trifloxysulfuron, trifloxysulfuron-sodium, trifluralin, triflusulfuron, triflusulfuron-methyl, trihydroxytriazine, tritosulfuron, [3-[2-chloro-4-fluoro-5-(-methyl-6-trifluoromethyl-2,4-dioxo-,2,3,4-t- etrahydropyrimidin-3-yl)phenoxy]-2-pyridyloxy]acetic acid ethyl ester (CAS RN 353292-3-6), 4-[(4,5-dihydro-3-methoxy-4-methyl-5-oxo)-H-,2,4-triazol--ylcarbonyl-sulfamoyl]-5-methylthiophene-3-carboxylic acid (BAY636), BAY747 (CAS RN 33504-84-2), topramezone (CAS RN 2063-68-8), 4-hydroxy-3-[[2-[(2-methoxyethoxy)methyl]-6-(trifluoro-methyl)-3-pyridi- nyl]carbonyl]-bicyclo[3.2.]oct-3-en-2-one (CAS RN 35200-68-5), and 4-hydroxy-3-[[2-(3-methoxypropyl)-6-(difluoromethyl)-3-pyridinyl]carbon- yl]-bicyclo[3.2.]oct-3-en-2-one. Additionally, including herbicidal compounds of unspecified modes of action as described in CN101279950A, CN101279951A, DE10000600A1, DE10116399A1, DE102004054666A1, DE102005014638A1, DE102005014906A1, DE102007012168A1, DE102010042866A1, DE10204951A1, DE10234875A1, DE10234876A1, DE10256353A1, DE10256354A1, DE10256367A1, EP1157991A2, EP1238586A1, EP2147919A1, EP2160098A2, JP03968012B2, JP2001253874A, JP2002080454A, JP2002138075A, JP2002145707A, JP2002220389A, JP2003064059A, JP2003096059A, JP2004051628A, JP2004107228A, JP2005008583A, JP2005239675A, JP2005314407A, JP2006232824A, JP2006282552A, JP2007153847A, JP2007161701A, JP2007182404A, JP2008074840A, JP2008074841A, JP2008133207A, JP2008133218A, JP2008169121A, JP2009067739A, JP2009114128A, JP2009126792A, JP2009137851A, US20060111241A1, US20090036311A1, US20090054240A1, US20090215628A1, US20100099561A1, US20100152443A1, US20110105329A1, US20110201501A1, WO2001055066A2, WO2001056975A1, WO2001056979A1, WO2001090071A2, WO2001090080A1, WO2002002540A1 , WO2002028182A1, WO2002040473A1, WO2002044173A2, WO2003000679A2, WO2003006422A1 , WO2003013247A1, WO2003016308A1, WO2003020704A1, WO2003022051A1, WO2003022831A1, WO2003022843A1, WO2003029243A2, WO2003037085A1, WO2003037878A1, WO2003045878A2, WO2003050087A2, WO2003051823A1, WO2003051824A1, WO2003051846A2, WO2003076409A1, WO2003087067A1, WO2003090539A1, WO2003091217A1, WO2003093269A2, WO2003104206A2, WO2004002947A1, WO2004002981A2, WO2004011429A1, WO2004029060A1, WO2004035545A2, WO2004035563A1, WO2004035564A1, WO2004037787A1, WO2004067518A1, WO2004067527A1, WO2004077950A1, WO2005000824A1, WO2005007627A1, WO2005040152A1, WO2005047233A1, WO2005047281A1, WO2005061443A2, WO2005061464A1, WO2005068434A1, WO2005070889A1, WO2005089551A1, WO2005095335A1, WO2006006569A1, WO2006024820A1, WO2006029828A1, WO2006029829A1, WO2006037945A1, WO2006050803A1, WO2006090792A1, WO2006123088A2, WO2006125687A1, WO2006125688A1, WO2007003294A1, WO2007026834A1, WO2007071900A1, WO2007077201A1, WO2007077247A1, WO2007096576A1, WO2007119434A1, WO2007134984A1, WO2008009908A1, WO2008029084A1, WO2008059948A1, WO2008071918A1, WO2008074991A1, WO2008084073A1, WO2008100426A2, WO2008102908A1, WO2008152072A2, WO2008152073A2, WO2009000757A1, WO2009005297A2, WO2009035150A2, WO2009063180A1, WO2009068170A2, WO2009068171A2, WO2009086041A1, WO2009090401A2, WO2009090402A2, WO2009115788A1, WO2009116558A1, WO2009152995A1, WO2009158258A1, WO2010012649A1, WO2010012649A1, WO2010026989A1, WO2010034153A1, WO2010049270A1, WO2010049369A1, WO2010049405A1, WO2010049414A1, WO2010063422A1, WO2010069802A1, WO2010078906A2, WO2010078912A1, WO2010104217A1, WO2010108611A1, WO2010112826A3, WO2010116122A3, WO2010119906A1, WO2010130970A1, WO2011003776A2, WO2011035874A1, WO2011065451A1

An agronomic field in need of plant control is treated by application of the composition directly to the surface of the growing plants, such as by a spray. For example, the method is applied to control weeds in a field of crop plants by spraying the field with the composition.. The composition can be provided as a tank mix, a sequential treatment of components (generally the polynucleotide containing composition followed by the herbicide), or a simultaneous treatment or mixing of one or more of the components of the composition from separate containers. Treatment of the field can occur as often as needed to provide weed control and the components of the composition can be adjusted to target specific weed species or weed families through utilization of specific polynucleotides or polynucleotide compositions capable of selectively targeting the specific species or plant family to be controlled. The composition can be applied at effective use rates according to the time of application to the field, for example, preplant, at planting, post planting, postharvest. DHPS inhibitor herbicides can be applied to a field at rates of 500 to 3000 g ai/ha (active ingredient per hectare) or more. The polynucleotides of the composition can be applied at rates of 1 to 30 grams per acre depending on the number of trigger molecules needed for the scope of weeds in the field.

Crop plants in which weed control is needed include but are not limited to, i) corn, soybean, cotton, canola, sugar beet, alfalfa, sugarcane, rice, and wheat; ii) vegetable plants including, but not limited to, tomato, sweet pepper, hot pepper, melon, watermelon, cucumber, eggplant, cauliflower, broccoli, lettuce, spinach, onion, peas, carrots, sweet corn, Chinese cabbage, leek, fennel, pumpkin, squash or gourd, radish, Brussels sprouts, tomatillo, garden beans, dry beans, or okra; iii) culinary plants including, but not limited to, basil, parsley, coffee, or tea; or , iv) fruit plants including but not limited to apple, pear, cherry, peach, plum, apricot, banana, plantain, table grape, wine grape, citrus, avocado, mango, or berry; v) a tree grown for ornamental or commercial use, including, but not limited to, a fruit or nut tree; or, vi) an ornamental plant (e. g., an ornamental flowering plant or shrub or turf grass). The methods and compositions provided herein can also be applied to plants produced by a cutting, cloning, or grafting process (i. e., a plant not grown from a seed) include fruit trees and plants that include, but are not limited to, citrus, apples, avocados, tomatoes, eggplant, cucumber, melons, watermelons, and grapes as well as various ornamental plants.

### Pesticidal Mixtures

The polynucleotide compositions may also be used as mixtures with various agricultural chemicals and/or insecticides, miticides and fungicides, pesticidal and biopesticidal agents. Examples include but are not limited to azinphos-methyl, acephate, isoxathion, isofenphos, ethion, etrimfos, oxydemeton-methyl, oxydeprofos, quinalphos, chlorpyrifos, chlorpyrifos-methyl, chlorfenvinphos, cyanophos, dioxabenzofos, dichlorvos, disulfoton, dimethylvinphos, dimethoate, sulprofos, diazinon, thiometon, tetrachlorvinphos, temephos, tebupirimfos, terbufos, naled, vamidothion, pyraclofos, pyridafenthion, pirimiphos-methyl, fenitrothion, fenthion, phenthoate, flupyrazophos, prothiofos, propaphos, profenofos, phoxime, phosalone, phosmet, formothion, phorate, malathion, mecarbam, mesulfenfos, methamidophos, methidathion, parathion, methyl parathion, monocrotophos, trichlorphon, EPN, isazophos, isamidofos, cadusafos, diamidaphos, dichlofenthion, thionazin, fenamiphos, fosthiazate, fosthietan, phosphocarb, DSP, ethoprophos, alanycarb, aldicarb, isoprocarb, ethiofencarb, carbaryl, carbosulfan, xylylcarb, thiodicarb, pirimicarb, fenobucarb, furathiocarb, propoxur, bendiocarb, benfuracarb, methomyl, metolcarb, XMC, carbofuran, aldoxycarb, oxamyl, acrinathrin, allethrin, esfenvalerate, empenthrin, cycloprothrin, cyhalothrin, gamma-cyhalothrin, lambda-cyhalothrin, cyfluthrin, beta-cyfluthrin, cypermethrin, alpha-cypermethrin, zeta-cypermethrin, silafluofen, tetramethrin, tefluthrin, deltamethrin, tralomethrin, bifenthrin, phenothrin, fenvalerate, fenpropathrin, furamethrin, prallethrin, flucythrinate, fluvalinate, flubrocythrinate, permethrin, resmethrin, ethofenprox, cartap, thiocyclam, bensultap, acetamiprid, imidacloprid, clothianidin, dinotefuran, thiacloprid, thiamethoxam, nitenpyram, chlorfluazuron, diflubenzuron, teflubenzuron, triflumuron, novaluron, noviflumuron, bistrifluoron, fluazuron, flucycloxuron, flufenoxuron, hexaflumuron, lufenuron, chromafenozide, tebufenozide, halofenozide, methoxyfenozide, diofenolan, cyromazine, pyriproxyfen, buprofezin, methoprene, hydroprene, kinoprene, triazamate, endosulfan, chlorfenson, chlorobenzilate, dicofol, bromopropylate, acetoprole, fipronil, ethiprole, pyrethrin, rotenone, nicotine sulphate, BT (Bacillus Thuringiensis) agent, spinosad, abamectin, acequinocyl, amidoflumet, amitraz, etoxazole, chinomethionat, clofentezine, fenbutatin oxide, dienochlor, cyhexatin, spirodiclofen, spiromesifen, tetradifon, tebufenpyrad, binapacryl, bifenazate, pyridaben, pyrimidifen, fenazaquin, fenothiocarb, fenpyroximate, fluacrypyrim, fluazinam, flufenzin, hexythiazox, propargite, benzomate, polynactin complex, milbemectin, lufenuron, mecarbam, methiocarb, mevinphos, halfenprox, azadirachtin, diafenthiuron, indoxacarb, emamectin benzoate, potassium oleate, sodium oleate, chlorfenapyr, tolfenpyrad, pymetrozine, fenoxycarb, hydramethylnon, hydroxy propyl starch, pyridalyl, flufenerim, flubendiamide, flonicamid, metaflumizole, lepimectin, TPIC, albendazole, oxibendazole, oxfendazole, trichlamide, fensulfothion, fenbendazole, levamisole hydrochloride, morantel tartrate, dazomet, metam-sodium, triadimefon, hexaconazole, propiconazole, ipconazole, prochloraz, triflumizole, tebuconazole, epoxiconazole, difenoconazole, flusilazole, triadimenol, cyproconazole, metconazole, fluquinconazole, bitertanol, tetraconazole, triticonazole, flutriafol, penconazole, diniconazole, fenbuconazole, bromuconazole, imibenconazole, simeconazole, myclobutanil, hymexazole, imazalil, furametpyr, thifluzamide, etridiazole, oxpoconazole, oxpoconazole fumarate, pefurazoate, prothioconazole, pyrifenox, fenarimol, nuarimol, bupirimate, mepanipyrim, cyprodinil, pyrimethanil, metalaxyl, mefenoxam, oxadixyl, benalaxyl, thiophanate, thiophanate-methyl, benomyl, carbendazim, fuberidazole, thiabendazole, manzeb, propineb, zineb, metiram, maneb, ziram, thiuram, chlorothalonil, ethaboxam, oxycarboxin, carboxin, flutolanil, silthiofam, mepronil, dimethomorph, fenpropidin, fenpropimorph, spiroxamine, tridemorph, dodemorph, flumorph, azoxystrobin, kresoxim-methyl, metominostrobin, orysastrobin, fluoxastrobin, trifloxystrobin, dimoxystrobin, pyraclostrobin, picoxystrobin, iprodione, procymidone, vinclozolin, chlozolinate, flusulfamide, dazomet, methyl isothiocyanate, chloropicrin, methasulfocarb, hydroxyisoxazole, potassium hydroxyisoxazole, echlomezol, D-D, carbam, basic copper chloride, basic copper sulfate, copper nonylphenolsulfonate, oxine copper, DBEDC, anhydrous copper sulfate, copper sulfate pentahydrate, cupric hydroxide, inorganic sulfur, wettable sulfur, lime sulfur, zinc sulfate, fentin, sodium hydrogen carbonate, potassium hydrogen carbonate, sodium hypochlorite, silver, edifenphos, tolclofos-methyl, fosetyl, iprobenfos, dinocap, pyrazophos, carpropamid, fthalide, tricyclazole, pyroquilon, diclocymet, fenoxanil, kasugamycin, validamycin, polyoxins, blasticiden S, oxytetracycline, mildiomycin, streptomycin, rape seed oil, machine oil, benthiavalicarbisopropyl, iprovalicarb, propamocarb, diethofencarb, fluoroimide, fludioxanil, fenpiclonil, quinoxyfen, oxolinic acid, chlorothalonil, captan, folpet, probenazole, acibenzolar-S-methyl, tiadinil, cyflufenamid, fenhexamid, diflumetorim, metrafenone, picobenzamide, proquinazid, famoxadone, cyazofamid, fenamidone, zoxamide, boscalid, cymoxanil, dithianon, fluazinam, dichlofluanide, triforine, isoprothiolane, ferimzone, diclomezine, tecloftalam, pencycuron, chinomethionat, iminoctadine acetate, iminoctadine albesilate, ambam, polycarbamate, thiadiazine, chloroneb, nickel dimethyldithiocarbamate, guazatine, dodecylguanidine-acetate, quintozene, tolylfluanid, anilazine, nitrothalisopropyl, fenitropan, dimethirimol, benthiazole, harpin protein, flumetover, mandipropamide and penthiopyrad.

### Polynucleotides

As used herein, the term "DNA", "DNA molecule", "DNA polynucleotide molecule" refers to a single-stranded DNA (ssDNA) or double-stranded DNA (dsDNA) molecule of genomic or synthetic origin, such as, a polymer of deoxyribonucleotide bases or a DNA polynucleotide molecule. As used herein, the term "DNA sequence", "DNA nucleotide sequence" or "DNA polynucleotide sequence" refers to the nucleotide sequence of a DNA molecule. As used herein, the term "RNA", "RNA molecule", "RNA polynucleotide molecule" refers to a single-stranded RNA (ssRNA) or double-stranded RNA (dsRNA) molecule of genomic or synthetic origin, such as, a polymer of ribonucleotide bases that comprise single or double stranded regions. Unless otherwise stated, nucleotide sequences in the text of this specification are given, when read from left to right, in the 5' to 3' direction. The nomenclature used herein is that required by Title 37 of the United States Code of Federal Regulations § 1.822 and set forth in the tables in WIPO Standard ST.25 (1998), Appendix 2, Tables 1 and 3.

As used herein, "polynucleotide" refers to a DNA or RNA molecule containing multiple nucleotides and generally refers both to "oligonucleotides" (a polynucleotide molecule of typically 50 or fewer nucleotides in length) and polynucleotides of 51 or more nucleotides. Embodiments include compositions including oligonucleotides having a length of 18-25 nucleotides (18-mers, 19-mers, 20-mers, 21-mers, 22-mers, 23-mers, 24-mers, or 25-mers), for example, oligonucleotides of Table 3 (SEQ ID NO:907-1175) pr fragments thereof or medium-length polynucleotides having a length of 26 or more nucleotides (polynucleotides of 26, 27, 28, 29, 30, 39, 32, 33, 34, 35, 36, 37, 38, 39, 40, 41, 42, 43, 44, 45, 39, 47, 48, 49, 50, 51, 52, 53, 54, 55, 56, 57, 58, 59, 60, about 65, about 70, about 75, about 80, about 85, about 90, about 95, about 100, about 110, about 120, about 130, about 140, about 150, about 160, about 170, about 180, about 190, about 200, about 210, about 220, about 230, about 240, about 250, about 260, about 270, about 280, about 290, or about 300 nucleotides), for example, polynucleotides of Table 2 (SEQ ID NO: 55-906) or fragments thereof or long polynucleotides having a length greater than about 300 nucleotides (for example, polynucleotides of between about 300 to about 400 nucleotides, between about 400 to about 500 nucleotides, between about 500 to about 600 nucleotides, between about 600 to about 700 nucleotides, between about 700 to about 800 nucleotides, between about 800 to about 900 nucleotides, between about 900 to about 1000 nucleotides, between about 300 to about 500 nucleotides, between about 300 to about 600 nucleotides, between about 300 to about 700 nucleotides, between about 300 to about 800 nucleotides, between about 300 to about 900 nucleotides, or about 1000 nucleotides in length, or even greater than about 1000 nucleotides in length, for example up to the entire length of a target gene including coding or non-coding or both coding and non-coding portions of the target gene), for example, polynucleotides of Table 1 (SEQ ID NO:1-54), wherein the selected polynucleotides or fragments thereof homologous or complementary to SEQ ID NO:1-54 suppresses, represses or otherwise delay the expression of the target DHPS gene. A target gene comprises any polynucleotide molecule in a plant cell or fragment thereof for which the modulation of the expression of the target gene is provided by the methods and compositions. Where a polynucleotide is double-stranded, its length can be similarly described in terms of base pairs. Oligonucleotides and polynucleotides can be made that are essentially identical or essentially complementary to adjacent genetic elements of a gene, for example, spanning the junction region of an intron and exon, the junction region of a promoter and a transcribed region, the junction region of a 5' leader and a coding sequence, the junction of a 3' untranslated region and a coding sequence.

Polynucleotide compositions used in the various embodiments include compositions including oligonucleotides or polynucleotides or a mixture of both, including RNA or DNA or RNA/DNA hybrids or chemically modified oligonucleotides or polynucleotides or a mixture thereof. In some embodiments, the polynucleotide may be a combination of ribonucleotides and deoxyribonucleotides, for example, synthetic polynucleotides consisting mainly of ribonucleotides but with one or more terminal deoxyribonucleotides or synthetic polynucleotides consisting mainly of deoxyribonucleotides but with one or more terminal dideoxyribonucleotides. In some embodiments, the polynucleotide includes non-canonical nucleotides such as inosine, thiouridine, or pseudouridine. In some embodiments, the polynucleotide includes chemically modified nucleotides. Examples of chemically modified oligonucleotides or polynucleotides are well known in the art; see, for example, US Patent Publication 20110171287, US Patent Publication 20110171176, and US Patent Publication 20110152353, US Patent Publication, 20110152339, US Patent Publication 20110160082 . For example, including but not limited to the naturally occurring phosphodiester backbone of an oligonucleotide or polynucleotide can be partially or completely modified with phosphorothioate, phosphorodithioate, or methylphosphonate internucleotide linkage modifications, modified nucleoside bases or modified sugars can be used in oligonucleotide or polynucleotide synthesis, and oligonucleotides or polynucleotides can be labeled with a fluorescent moiety (for example, fluorescein or rhodamine) or other label (for example, biotin).

The polynucleotides can be single- or double-stranded RNA or single- or double-stranded DNA or double-stranded DNA/RNA hybrids or modified analogues thereof, and can be of oligonucleotide lengths or longer. In more specific embodiments, the polynucleotides that provide single-stranded RNA in the plant cell are selected from the group consisting of (a) a single-stranded RNA molecule (ssRNA), (b) a single-stranded RNA molecule that self-hybridizes to form a double-stranded RNA molecule, (c) a double-stranded RNA molecule (dsRNA), (d) a single-stranded DNA molecule (ssDNA), (e) a single-stranded DNA molecule that self-hybridizes to form a double-stranded DNA molecule, and (f) a single-stranded DNA molecule including a modified Pol III gene that is transcribed to an RNA molecule, (g) a double-stranded DNA molecule (dsDNA), (h) a double-stranded DNA molecule including a modified Pol III gene that is transcribed to an RNA molecule, (i) a double-stranded, hybridized RNA/DNA molecule, or combinations thereof. In some embodiments these polynucleotides include chemically modified nucleotides or non-canonical nucleotides. In some embodiments, the oligonucleotides may be blunt-ended or may comprise a 3' overhang of from 1-5 nucleotides of at least one or both of the strands. Other configurations of the oligonucleotide are known in the field and are contemplated herein. In embodiments of the method the polynucleotides include double-stranded DNA formed by intramolecular hybridization, double-stranded DNA formed by intermolecular hybridization, double-stranded RNA formed by intramolecular hybridization, or double-stranded RNA formed by intermolecular hybridization. In one embodiment the polynucleotides include single-stranded DNA or single-stranded RNA that self-hybridizes to form a hairpin structure having an at least partially double-stranded structure including at least one segment that will hybridize to RNA transcribed from the gene targeted for suppression. Not intending to be bound by any mechanism, it is believed that such polynucleotides are or will produce single-stranded RNA with at least one segment that will hybridize to RNA transcribed from the gene targeted for suppression. In certain other embodiments the polynucleotides further includes a promoter, generally a promoter functional in a plant, for example, a pol II promoter, a pol III promoter, a pol IV promoter, or a pol V promoter.

The term "gene" refers to components that comprise chromosomal DNA, plasmid DNA, cDNA, intron and exon DNA, artificial DNA polynucleotide, or other DNA that encodes a peptide, polypeptide, protein, or RNA transcript molecule, and the genetic elements flanking the coding sequence that are involved in the regulation of expression, such as, promoter regions, 5' leader regions, 3' untranslated region that may exist as native genes or transgenes in a plant genome. The gene or a fragment thereof is isolated and subjected to polynucleotide sequencing methods that determines the order of the nucleotides that comprise the gene. Any of the components of the gene are potential targets for a trigger oligonucleotide and polynucleotides.

The trigger polynucleotide molecules are designed to modulate expression by inducing regulation or suppression of an endogenous DHPS gene in a plant and are designed to have a nucleotide sequence essentially identical or essentially complementary to the nucleotide sequence of an endogenous DHPS gene of a plant or to the sequence of RNA transcribed from an endogenous DHPS gene of a plant, including a transgene in a plant that provides for a herbicide resistant DHPS enzyme, which can be coding sequence or non-coding sequence. Effective molecules that modulate expression are referred to as "a trigger molecule, or trigger polynucleotides". By "essentially identical" or "essentially complementary" is meant that the trigger polynucleotides (or at least one strand of a double-stranded polynucleotide or portion thereof, or a portion of a single strand polynucleotide) are designed to hybridize to the endogenous gene noncoding sequence or to RNA transcribed (known as messenger RNA or an RNA transcript) from the endogenous gene to effect regulation or suppression of expression of the endogenous gene. Trigger molecules are identified by "tiling" the gene targets with partially overlapping probes or non-overlapping probes of antisense or sense polynucleotides that are essentially identical or essentially complementary to the nucleotide sequence of an endogenous gene. Multiple target sequences can be aligned and sequence regions with homology in common are identified as potential trigger molecules for the multiple targets. Multiple trigger molecules of various lengths, for example 18-25 nucleotides, 26-50 nucleotides, 51-100 nucleotides, 101-200 nucleotides, 201-300 nucleotides or more can be pooled into a few treatments in order to investigate polynucleotide molecules that cover a portion of a gene sequence (for example, a portion of a coding versus a portion of a noncoding region, or a 5' versus a 3' portion of a gene) or an entire gene sequence including coding and noncoding regions of a target gene. Polynucleotide molecules of the pooled trigger molecules can be divided into smaller pools or single molecules inorder to identify trigger molecules that provide the desired effect.

The target gene RNA and DNA polynucleotide molecules (Table 1, SEQ ID NO:1-54) are sequenced by any number of available methods and equipment. Some of the sequencing technologies are available commercially, such as the sequencing-by-hybridization platform from Affymetrix Inc. (Sunnyvale, Calif.) and the sequencing-by-synthesis platforms from 454 Life Sciences (Bradford, Conn.), Illumina/Solexa (Hayward, Calif.) and Helicos Biosciences (Cambridge, Mass.), and the sequencing-by-ligation platform from Applied Biosystems (Foster City, Calif.), as described below. In addition to the single molecule sequencing performed using sequencing-by-synthesis of Helicos Biosciences, other single molecule sequencing technologies are encompassed by the method and include the SMRT™ technology of Pacific Biosciences, the Ion Torrent™. technology, and nanopore sequencing being developed for example, by Oxford Nanopore Technologies. A DHPS target gene comprising DNA or RNA can be isolated using primers or probes essentially complementary or essentially homologous to SEQ ID NO:1-54 or a fragment thereof. A polymerase chain reaction (PCR) gene fragment can be produced using primers essentially complementary or essentially homologous to SEQ ID NO:1-54 or a fragment thereof.that is useful to isolate a DHPS gene from a plant genome.

Embodiments of functional single-stranded polynucleotides functional have sequence complementarity that need not be 100 percent, but is at least sufficient to permit hybridization to RNA transcribed from the target gene or DNA of the target gene to form a duplex to permit a gene silencing mechanism. Thus, in embodiments, a polynucleotide fragment is designed to be essentially identical to, or essentially complementary to, a sequence of 18 or more contiguous nucleotides in either the target DHPS gene sequence or messenger RNA transcribed from the target gene. By "essentially identical" is meant having 100 percent sequence identity or at least about 83, 84, 85, 86, 87, 88, 89, 90, 91, 92, 93, 94, 95, 96, 97, 98, or 99 percent sequence identity when compared to the sequence of 18 or more contiguous nucleotides in either the target gene or RNA transcribed from the target gene; by "essentially complementary" is meant having 100 percent sequence complementarity or at least about 83, 84, 85, 86, 87, 88, 89, 90, 91, 92, 93, 94, 95, 96, 97, 98, or 99 percent sequence complementarity when compared to the sequence of 18 or more contiguous nucleotides in either the target gene or RNA transcribed from the target gene. In some embodiments, polynucleotide molecules are designed to have 100 percent sequence identity with or complementarity to one allele or one family member of a given target gene (coding or non-coding sequence of a gene); in other embodiments the polynucleotide molecules are designed to have 100 percent sequence identity with or complementarity to multiple alleles or family members of a given target gene. The trigger polynucleotide sequences in the sequence listing SEQ ID NO: 1-1222 or table 1, 2 or 3 maybe complementary or homologous to a portion of the DHPS target gene sequence.

In certain embodiments, the polynucleotides used in the compositions that are essentially identical or essentially complementary to the target gene or transcript will comprise the predominant nucleic acid in the composition. Thus in certain embodiments, the polynucleotides that are essentially identical or essentially complementary to the target gene or transcript will comprise at least about 50%, 75%, 95%, 98% or 100% of the nucleic acids provided in the composition by either mass or molar concentration. However, in certain embodiments, the polynucleotides that are essentially identical or essentially complementary to the target gene or transcript can comprise at least about 1% to about 50%, about 10% to about 50%, about 20% to about 50%, or about 30% to about 50% of the nucleic acids provided in the composition by either mass or molar concentration. Also provided are compositions where the polynucleotides that are essentially identical or essentially complementary to the target gene or transcript can comprise at least about 1% to 100%, about 10% to 100%, about 20% to about 100%, about 30% to about 50%, or about 50% to a 100% of the nucleic acids provided in the composition by either mass or molar concentration.

"Identity" refers to the degree of similarity between two polynucleic acid or protein sequences. An alignment of the two sequences is performed by a suitable computer program. A widely used and accepted computer program for performing sequence alignments is CLUSTALW v1.6 (Thompson, et al. Nucl. Acids Res., 22: 3973-3980, 1994). The number of matching bases or amino acids is divided by the total number of bases or amino acids, and multiplied by 100 to obtain a percent identity. For example, if two 580 base pair sequences had 145 matched bases, they would be 25 percent identical. If the two compared sequences are of different lengths, the number of matches is divided by the shorter of the two lengths. For example, if there are 100 matched amino acids between a 200 and a 400 amino acid protein, they are 50 percent identical with respect to the shorter sequence. If the shorter sequence is less than 150 bases or 50 amino acids in length, the number of matches are divided by 150 (for nucleic acid bases) or 50 (for amino acids), and multiplied by 100 to obtain a percent identity.

Trigger molecules for specific gene family members can be identified from coding and/or non-coding sequences of gene families of a plant or multiple plants, by aligning and selecting 200-300 polynucleotide fragments from the least homologous regions amongst the aligned sequences and evaluated using topically applied polynucleotides (as sense or anti-sense ssDNA or ssRNA, dsRNA, or dsDNA) to determine their relative effectiveness in inducing the herbicidal phenotype. The effective segments are further subdivided into 50-60 polynucleotide fragments, prioritized by least homology, and reevaluated using topically applied polynucleotides. The effective 50-60 polynucleotide fragments are subdivided into 19-30 polynucleotide fragments, prioritized by least homology, and again evaluated for induction of the yield/quality phenotype. Once relative effectiveness is determined, the fragments are utilized singly, or again evaluated in combination with one or more other fragments to determine the trigger composition or mixture of trigger polynucleotides for providing the yield/quality phenotype.

Trigger molecules for broad activity can be identified from coding and/or non-coding sequences of gene families of a plant or multiple plants, by aligning and selecting 200-300 polynucleotide fragments from the most homologous regions amongst the aligned sequences and evaluated using topically applied polynucleotides (as sense or anti-sense ssDNA or ssRNA, dsRNA, or dsDNA) to determine their relative effectiveness in inducing the yield/quality phenotype. The effective segments are subdivided into 50-60 polynucleotide fragments, prioritized by most homology, and reevaluated using topically applied polynucleotides. The effective 50-60 polynucleotide fragments are subdivided into 19-30 polynucleotide fragments, prioritized by most homology, and again evaluated for induction of the yield/quality phenotype. Once relative effectiveness is determined, the fragments may be utilized singly, or in combination with one or more other fragments to determine the trigger composition or mixture of trigger polynucleotides for providing the yield/quality phenotype.

Methods of making polynucleotides are well known in the art. Chemical synthesis, in vivo synthesis and in vitro synthesis methods and compositions are known in the art and include various viral elements, microbial cells, modified polymerases, and modified nucleotides. Commercial preparation of oligonucleotides often provides two deoxyribonucleotides on the 3' end of the sense strand. Long polynucleotide molecules can be synthesized from commercially available kits, for example, kits from Applied Biosystems/Ambion (Austin, TX) have DNA ligated on the 5' end in a microbial expression cassette that includes a bacterial T7 polymerase promoter that makes RNA strands that can be assembled into a dsRNA and kits provided by various manufacturers that include T7 RiboMax Express (Promega, Madison, WI), AmpliScribe T7-Flash (Epicentre, Madison, WI), and TranscriptAid T7 High Yield (Fermentas, Glen Burnie, MD). dsRNA molecules can be produced from microbial expression cassettes in bacterial cells (Ongvarrasopone et al. ScienceAsia 33:35-39; Yin, Appl. Microbiol. Biotechnol 84:323-333, 2009; Liu et al., BMC Biotechnology 10:85, 2010) that have regulated or deficient RNase III enzyme activity or the use of various viral vectors to produce sufficient quantities of dsRNA. DHPS gene fragments are inserted into the microbial expression cassettes in a position in which the fragments are express to produce ssRNA or dsRNA useful in the methods described herein to regulate expression on a target DHPS gene. Long polynucleotide molecules can also be assembled from multiple RNA or DNA fragments. In some embodiments design parameters such as Reynolds score (Reynolds et al. Nature Biotechnology 22, 326 - 330 (2004),Tuschl rules (Pei and Tuschl, Nature Methods 3(9): 670-676, 2006), i-score (Nucleic Acids Res 35: e123, 2007), i-Score Designer tool and associated algorithms (Nucleic Acids Res 32: 936-948, 2004. Biochem Biophys Res Commun 316: 1050-1058, 2004, Nucleic Acids Res 32: 893-901, 2004, Cell Cycle 3: 790-5, 2004, Nat Biotechnol 23: 995-1001, 2005, Nucleic Acids Res 35: e27, 2007, BMC Bioinformatics 7: 520, 2006, Nucleic Acids Res 35: e123, 2007, Nat Biotechnol 22: 326-330, 2004) are known in the art and may be used in selecting polynucleotide sequences effective in gene silencing. In some embodiments random design or empirical selection of polynucleotide sequences is used in selecting polynucleotide sequences effective in gene silencing. In some embodiments the sequence of a polynucleotide is screened against the genomic DNA of the intended plant to minimize unintentional silencing of other genes.

The polynucleotide compositions are useful in compositions, such as solutions of polynucleotide molecules, at low concentrations, alone or in combination with other components either in the same solution or in separately applied solutions that provide a permeability-enhancing agent. While there is no upper limit on the concentrations and dosages of polynucleotide molecules that can useful in the methods, lower effective concentrations and dosages will generally be sought for efficiency. The concentrations can be adjusted in consideration of the volume of spray or treatment applied to plant leaves or other plant part surfaces, such as flower petals, stems, tubers, fruit, anthers, pollen, or seed. In one embodiment, a useful treatment for herbaceous plants using 25-mer oligonucleotide molecules is about 1 nanomole (nmol) of oligonucleotide molecules per plant, for example, from about 0.05 to 1 nmol per plant. Other embodiments for herbaceous plants include useful ranges of about 0.05 to about 100 nmol, or about 0.1 to about 20 nmol, or about 1 nmol to about 10 nmol of polynucleotides per plant. Very large plants, trees, or vines may require correspondingly larger amounts of polynucleotides. When using long dsRNA molecules that can be processed into multiple oligonucleotides, lower concentrations can be used. To illustrate embodiments, the factor 1X, when applied to oligonucleotide molecules is arbitrarily used to denote a treatment of 0.8 nmol of polynucleotide molecule per plant; 10X, 8 nmol of polynucleotide molecule per plant; and 100X, 80 nmol of polynucleotide molecule per plant.

The trigger polynucleotide and oligonucleotide molecule compositions are useful in compositions, such as liquids that comprise these polynucleotide molecules, alone or in combination with other components, for example one or more herbicide molecules, either in the same liquid or in separately applied liquids that also provide a transfer agent. As used herein, a transfer agent is an agent that, when combined with a polynucleotide in a composition that is topically applied to a target plant surface, enables the polynucleotide to enter a plant cell. In certain embodiments, a transfer agent is an agent that conditions the surface of plant tissue, e. g., leaves, stems, roots, flowers, or fruits, to permeation by the polynucleotide molecules into plant cells. The transfer of polynucleotides into plant cells can be facilitated by the prior or contemporaneous application of a polynucleotide-transferring agent to the plant tissue. In some embodiments the transferring agent is applied subsequent to the application of the polynucleotide composition. The polynucleotide transfer agent enables a pathway for polynucleotides through cuticle wax barriers, stomata and/or cell wall or membrane barriers into plant cells. Suitable transfer agents to facilitate transfer of the polynucleotide into a plant cell include agents that increase permeability of the exterior of the plant or that increase permeability of plant cells to oligonucleotides or polynucleotides. Such agents to facilitate transfer of the composition into a plant cell include a chemical agent, or a physical agent, or combinations thereof. Chemical agents for conditioning or transfer include (a) surfactants, (b) an organic solvent or an aqueous solution or aqueous mixtures of organic solvents, (c) oxidizing agents, (d) acids, (e) bases, (f) oils, (g) enzymes, or combinations thereof. Embodiments of the method can optionally include an incubation step, a neutralization step (e.g., to neutralize an acid, base, or oxidizing agent, or to inactivate an enzyme), a rinsing step, or combinations thereof. Embodiments of agents or treatments for conditioning of a plant to permeation by polynucleotides include emulsions, reverse emulsions, liposomes, and other micellar-like compositions. Embodiments of agents or treatments for conditioning of a plant to permeation by polynucleotides include counter-ions or other molecules that are known to associate with nucleic acid molecules, *e. g.,* inorganic ammonium ions, alkyl ammonium ions, lithium ions, polyamines such as spermine, spermidine, or putrescine, and other cations. Organic solvents useful in conditioning a plant to permeation by polynucleotides include DMSO, DMF, pyridine, *N*-pyrrolidine, hexamethylphosphoramide, acetonitrile, dioxane, polypropylene glycol, other solvents miscible with water or that will dissolve phosphonucleotides in non-aqueous systems (such as is used in synthetic reactions). Naturally derived or synthetic oils with or without surfactants or emulsifiers can be used, *e. g.,* plant-sourced oils, crop oils (such as those listed in the 9^{th} Compendium of Herbicide Adjuvants, publicly available on the worldwide web (internet) at herbicide.adjuvants.com can be used, *e. g.,* paraffinic oils, polyol fatty acid esters, or oils with short-chain molecules modified with amides or polyamines such as polyethyleneimine or *N-*pyrrolidine. Transfer agents include, but are not limited to, organosilicone preparations.

Ligands can be tethered to a polynucleotide, for example a dsRNA, ssRNA, dsDNA or ssDNA. Ligands in general can include modifiers, e.g., for enhancing uptake; diagnostic compounds or reporter groups e.g., for monitoring distribution; cross-linking agents; nuclease-resistance conferring moieties; and natural or unusual nucleobases. General examples include lipophiles, lipids (e.g., cholesterol, a bile acid, or a fatty acid (e.g., lithocholic-oleyl, lauroyl, docosnyl, stearoyl, palmitoyl, myristoyl oleoyl, linoleoyl), steroids (e.g., uvaol, hecigenin, diosgenin), terpenes (e.g., triterpenes, e.g., sarsasapogenin, Friedelin, epifriedelanol derivatized lithocholic acid), vitamins (e.g., folic acid, vitamin A, biotin, pyridoxal), carbohydrates, proteins, protein binding agents, integrin targeting molecules, polycationics, peptides, polyamines, and peptide mimics. The ligand may also be a recombinant or synthetic molecule, such as a synthetic polymer, e.g., polyethylene glycol (PEG), PEG-40K, PEG-20K and PEG-5K. Other examples of ligands include lipophilic molecules, e.g, cholesterol, cholic acid, adamantane acetic acid, 1-pyrene butyric acid, dihydrotestosterone, glycerol (e.g., esters and ethers thereof, e.g., C.sub.10, C.sub.11, C.sub.12, C.sub.13, C.sub.14, C.sub.15, C.sub.16, C.sub.17, C.sub.18, C.sub.19, or C.sub.20 alkyl; e.g., lauroyl, docosnyl, stearoyl, oleoyl, linoleoyl 1,3-bis-O(hexadecyl)glycerol, 1,3-bis-O(octaadecyl)glycerol), geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dodecanoyl, lithocholyl, 5.beta.-cholanyl, N,N-distearyl-lithocholamide, 1,2-di-O-stearoylglyceride, dimethoxytrityl, or phenoxazine) and PEG (e.g., PEG-5K, PEG-20K, PEG-40K). Preferred lipophilic moieties include lipid, cholesterols, oleyl, retinyl, or cholesteryl residues.

Conjugating a ligand to a dsRNA can enhance its cellular absorption, lipophilic compounds that have been conjugated to oligonucleotides include 1-pyrene butyric acid, 1,3-bis-O-(hexadecyl)glycerol, and menthol. One example of a ligand for receptor-mediated endocytosis is folic acid. Folic acid enters the cell by folate-receptor-radiated endocytosis. dsRNA compounds bearing folic acid would be efficiently transported into the cell via the folate-receptor-mediated endocytosis. Other ligands that have been conjugated to oligonucleotides include polyethylene glycols, carbohydrate clusters, cross-linking agents, porphyrin conjugates, delivery peptides and lipids such as cholesterol. In certain instances, conjugation of a cationic ligand to oligonucleotides results in improved resistance to nucleases. Representative examples of cationic ligands are propylammonium and dimethylpropylammonium. Interestingly, antisense oligonucleotides were reported to retain their high binding affinity to mRNA when the cationic ligand was dispersed, throughout the oligonucleotide. See M. Manoharan Antisense & Nucleic Acid Drug Development 2002, 12, 103 and references therein.

A biologic delivery can be accomplished by a variety of methods including, without limitation, (1) loading liposomes with a dsRNA acid molecule provided herein and (2) complexing a dsRNA molecule with lipids or liposomes to form nucleic acid-lipid or nucleic acid-liposome complexes. The liposome can be composed of cationic and neutral lipids commonly used to transfect cells in vitro. Cationic lipids can complex (e.g., charge-associate) with negatively charged, nucleic acids to form liposomes. Examples of cationic liposomes include, without limitation, lipofectin, lipofectamine, lipofectace, and DOTAP. Procedures for forming liposomes are well known in the art. Liposome compositions can be formed, for example, from phosphatidylcholine, dimyristoyl phosphatidylcholine, dipalmitoyl phosphatidylcholine, dimyristoyl phosphatidyl glycerol, dioleoyl phosphatidylethanolamine or liposomes comprising dihydrosphingomyelin (DHSM) Numerous lipophilic agents are commercially available, including Lipofectin.RTM. (Invitrogen/Life Technologies, Carlsbad, Calif.) and Effectene™ (Qiagen, Valencia, Calif.), In addition, systemic delivery methods can be optimized using commercially available cationic lipids such as DDAB or DOTAP, each of which can be mixed with a neutral lipid such as DOPE or cholesterol. In some eases, liposomes such as those described by Templeton et al. Nature Biotechnology, 15:647-652 (1997) can be used. In other embodiments, polycations such as polyethyleneimine can be used to achieve delivery in vivo and ex vivo (Boletta et al., J. Am Soc. Nephrol. 7:1728, 1996). Additional information regarding the use of liposomes to deliver nucleic acids can be found in U.S. Pat. No. 6,271,359, PCT Publication WO 96/40964 and Morrissey, D. et al., 2005. Nature Biotechnol. 23(8):1002-7.

In certain embodiments, an organosilicone preparation that is commercially available as Silwet® L-77 surfactant having CAS Number 27306-78-1 and EPA Number: CAL.REG.NO. 5905-50073-AA, and currently available from Momentive Performance Materials, Albany, New York can be used to prepare a polynucleotide composition. In certain embodiments where a Silwet L-77 organosilicone preparation is used as a pre-spray treatment of plant leaves or other plant surfaces, freshly made concentrations in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation comprising Silwet L-77 in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0. 1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

In certain embodiments, any of the commercially available organosilicone preparations provided such as the following Breakthru S 321, Breakthru S 200 Cat# 67674-67-3, Breakthru OE 441 Cat#68937-55-3, Breakthru S 278 Cat #27306-78-1, Breakthru S 243, Breakthru S 233 Cat#134180-76-0, available from manufacturer Evonik Goldschmidt (Germany), Silwet® HS 429, Silwet® HS 312, Silwet® HS 508, Silwet® HS 604 (Momentive Performance Materials, Albany, New York) can be used as transfer agents in a polynucleotide composition. In certain embodiments where an organosilicone preparation is used as a pre-spray treatment of plant leaves or other surfaces, freshly made concentrations in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Organosilicone preparations used in the methods and compositions provided herein can comprise one or more effective organosilicone compounds. As used herein, the phrase "effective organosilicone compound" is used to describe any organosilicone compound that is found in an organosilicone preparation that enables a polynucleotide to enter a plant cell. In certain embodiments, an effective organosilicone compound can enable a polynucleotide to enter a plant cell in a manner permitting a polynucleotide mediated suppression of a target gene expression in the plant cell. In general, effective organosilicone compounds include, but are not limited to, compounds that can comprise: i) a trisiloxane head group that is covalently linked to, ii) an alkyl linker including, but not limited to, an n-propyl linker, that is covalently linked to, iii) a poly glycol chain, that is covalently linked to, iv) a terminal group. Trisiloxane head groups of such effective organosilicone compounds include, but are not limited to, heptamethyltrisiloxane. Alkyl linkers can include, but are not limited to, an n-propyl linker. Poly glycol chains include, but are not limited to, polyethylene glycol or polypropylene glycol. Poly glycol chains can comprise a mixture that provides an average chain length "n" of about "7.5". In certain embodiments, the average chain length "n" can vary from about 5 to about 14. Terminal groups can include, but are not limited to, alkyl groups such as a methyl group. Effective organosilicone compounds are believed to include, but are not limited to, trisiloxane ethoxylate surfactants or polyalkylene oxide modified heptamethyl trisiloxane. 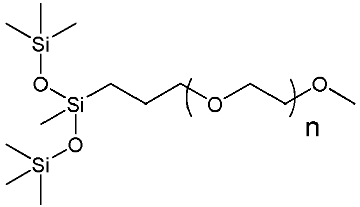 (Compound I: polyalkyleneoxide heptamethyltrisiloxane, average n=7.5).

In certain embodiments, an organosilicone preparation that comprises an organosilicone compound comprising a trisiloxane head group is used in the methods and compositions provided herein. In certain embodiments, an organosilicone preparation that comprises an organosilicone compound comprising a heptamethyltrisiloxane head group is used in the methods and compositions provided herein. In certain embodiments, an organosilicone composition that comprises Compound I is used in the methods and compositions provided herein. In certain embodiments, an organosilicone composition that comprises Compound I is used in the methods and compositions provided herein. In certain embodiments of the methods and compositions provided herein, a composition that comprises a polynucleotide molecule and one or more effective organosilicone compound in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Compositions include but are not limited components that are one or more polynucleotides essentially identical to, or essentially complementary to a DHPS gene sequence (promoter, intron, exon, 5' untranslated region, 3' untranslated region), a transfer agent that provides for the polynucleotide to enter a plant cell, a herbicide that complements the action of the polynucleotide, one or more additional herbicides that further enhance the herbicide activity of the composition or provide an additional mode of action different from the complementing herbicide, various salts and stabilizing agents that enhance the utility of the composition as an admixture of the components of the composition.

The methods include one or more applications of a polynucleotide composition and one or more applications of a permeability-enhancing agent for conditioning of a plant to permeation by polynucleotides. When the agent for conditioning to permeation is an organosilicone composition or compound contained therein, embodiments of the polynucleotide molecules are double-stranded RNA oligonucleotides, single-stranded RNA oligonucleotides, double-stranded RNA polynucleotides, single-stranded RNA polynucleotides, double-stranded DNA oligonucleotides, single-stranded DNA oligonucleotides, double-stranded DNA polynucleotides, single-stranded DNA polynucleotides, chemically modified RNA or DNA oligonucleotides or polynucleotides or mixtures thereof.

Compositions and methods are useful for modulating the expression of an endogenous DHPS gene or transgenic DHPS gene (for example US Patent No. 6,121,513) in a plant cell. In various embodiments, a DHPS gene includes coding (protein-coding or translatable) sequence, non-coding (non-translatable) sequence, or both coding and non-coding sequence. Compositions can include polynucleotides and oligonucleotides designed to target multiple genes, or multiple segments of one or more genes. The target gene can include multiple consecutive segments of a target gene, multiple non-consecutive segments of a target gene, multiple alleles of a target gene, or multiple target genes from one or more species.

An aspect provides a method for modulating expression of a DHPS gene in a plant including (a) conditioning of a plant to permeation by polynucleotides and (b) treatment of the plant with the polynucleotide molecules, wherein the polynucleotide molecules include at least one segment of 18 or more contiguous nucleotides cloned from or otherwise identified from the target DHPS gene in either anti-sense or sense orientation, whereby the polynucleotide molecules permeate the interior of the plant and induce modulation of the target gene. The conditioning and polynucleotide application can be performed separately or in a single step. When the conditioning and polynucleotide application are performed in separate steps, the conditioning can precede or can follow the polynucleotide application within minutes, hours, or days. In some embodiments more than one conditioning step or more than one polynucleotide molecule application can be performed on the same plant. In embodiments of the method, the segment can be cloned or identified from (a) coding (protein-encoding), (b) non-coding (promoter and other gene related molecules), or (c) both coding and non-coding parts of the target gene. Non-coding parts include DNA, such as promoter regions or the RNA transcribed by the DNA that provide RNA regulatory molecules, including but not limited to: introns, 5' or 3' untranslated regions, and microRNAs (miRNA), *trans*-acting siRNAs, natural anti-sense siRNAs, and other small RNAs with regulatory function or RNAs having structural or enzymatic function including but not limited to: ribozymes, ribosomal RNAs, t-RNAs, aptamers, and riboswitches.

The following examples are included to demonstrate examples of certain preferred embodiments.

### EXAMPLES

### Example 1. Polynucleotides related to the DHPS gene sequences.

The target DHPS polynucleotide molecule naturally occurs in the genome of plants that include but are not limited to *Amaranthus palmeri*, *Amaranthus rudis, Amaranthus hybrids*, *Amaranthus lividus*, *Amaranthus viridis*, *Ambrosia trifida*, *Conyza candensis*, *Digitaria sanguinalis*, *Euphorbia heterophylla*, *Kochia scoparia*, *Lolium multiflorum*, and include molecules related to the expression of a polypeptide identified as a DHPS, that include genomic DNA (gDNA) and coding cDNAs comprising coding and noncoding regions of a DHPS gene and fragments thereof as shown in Table 1.

Polynucleotide molecules were extracted from these plant species by methods standard in the field, for example, total RNA was extracted using Trizol Reagent (Invitrogen Corp, Carlsbad, CA Cat. No. 15596-018), following the manufacturer's protocol or modifications thereof by those skilled in the art of polynucleotide extraction that may enhance recover or purity of the extracted RNA. Briefly, start with 1 gram of ground plant tissue for extraction. Prealiquot 10 milliliters (mL) Trizol reagent to 15 mL conical tubes, add ground powder to tubes and shake to homogenize. Incubate the homogenized samples for 5 minutes (min) at room temperature (RT) and then add 3 mL of chloroform. Shakes tubes vigorously by hand for 15-30 seconds (sec) and incubate at RT for 3 min. Centrifuge the tubes at 7,000 revolutions per minute (rpm) for 10 min at 4 degrees C. Transfer the aqueous phase to a new 1.5 mL tube and add 1 volume of cold isopropanol. Incubate the samples for 20-30 min at RT and centrifuge at 10,000 rpm for 10 min at 4 degrees C. Wash pellet with Sigma-grade 80 percent ethanol. Remove the supernatant and briefly air-dry the pellet. Dissolve the RNA pellet in approximately 200 microliters of DEPC treated water. Heat briefly at 65C to dissolve pellet and vortex or pipet to resuspend RNA pellet and then adjust RNA concentration to 1-2 microgram/microliter.

DNA was extracted using EZNA SP Plant DNA Mini kit (Omega Biotek, Norcross GA, Cat#D5511) and Lysing Matrix E tubes (Q-Biogen, Cat#6914), following the manufacturer's protocol or modifications thereof by those skilled in the art of polynucleotide extraction that may enhance recover or purity of the extracted DNA. Briefly, aliquot ground tissue to a Lysing Matrix E tube on dry ice, add 800µl Buffer SP1 to each sample, homogenize in a bead beater for 35-45sec, incubate on ice for 45-60 sec, centrifuge at ≥14000 rpm for 1min at RT, add 10 microliter RNase A to the lysate, incubate at 65°C for 10min, centrifuge for 1min at RT, add 280µl Buffer SP2 and vortex to mix, incubate the samples on ice for 5min, centrifuge at ≥10,000g for 10 min at RT, transfer the supernatant to a homogenizer column in a 2ml collection tube, centrifuge at 10,000g for 2 min at RT, transfer the cleared lysate into a 1.5ml microfuge tube, add 1.5 volumes Buffer SP3 to the cleared lysate, vortex immediately to obtain a homogeneous mixture, transfer up to 650µl supernatant to the Hi-Bind column, centrifuge at 10,000g for 1min, repeat, apply 100µl 65°C Elution Buffer to the column, centrifuge at 10,000g for 5min at RT.

Next-generation DNA sequencers, such as the 454-FLX (Roche, Branford, CT), the SOLiD (Applied Biosystems,), and the Genome Analyzer (HiSeq2000, Illumina, San Diego, CA) were used to provide polynucleotide sequence from the DNA and RNA extracted from the plant tissues. Raw sequence data was assembled into contigs. The contig sequence was used to identify trigger molecules that can be applied to a plant to enable regulation of the gene expression.

The target DNA sequence isolated from genomic (gDNA) and coding DNA (cDNA) from the various weedy plant species for the DHPS gene and the assembled contigs as set forth in SEQ ID NOs 1-54 and Table 1.

### Example 2. Polynucleotides related to the trigger molecules

The gene sequences and fragments of Table 1 were divided into 200 polynucleotide (200-mer) lengths with 25 polynucleotide overlapping regions and are shown in Table 2, SEQ ID NO:55-906. These polynucleotides are tested to select the most efficacious trigger regions across the length of any target sequence. The trigger polynucleotides are constructed as sense or anti-sense ssDNA or ssRNA, dsRNA, or dsDNA, or dsDNA/RNA hybrids and combined with an organosilicone based transfer agent to provide a polynucleotide preparation. The polynucleotides are combined into sets of two to three polynucleotides per set, using 4-8 nmol of each polynucleotide. Each polynucleotide set is prepared with the transfer agent and applied to a plant or a field of plants in combination with a DHPS inhibitor containing herbicide, or followed by a DHPS inhibitor treatment one to three days after the polynucleotide application, to determine the effect on the plant's susceptibility to an DHPS inhibitor. The effect is measured as stunting the growth and/or killing of the plant and is measured 8-14 days after treatment with the polynucleotide set and DHPS inhibitor. The most efficacious sets are identified and the individual polynucleotides are tested in the same methods as the sets are and the most efficacious single 200-mer identified. The 200-mer sequence is divided into smaller sequences of 50-70-mer regions with 10-15 polynucleotide overlapping regions and the polynucleotides tested individually. The most efficacious 50-70-mer is further divided into smaller sequences of 25-mer regions with a 12 to 13 polynucleotide overlapping region and tested for efficacy in combination with DHPS inhibitor treatment. By this method it is possible to identify an oligonucleotide or several oligonucleotides that are the most efficacious trigger molecule to effect plant sensitivity to a DHPS inhibitor or modulation of an DHPS gene expression. The modulation of DHPS gene expression is determined by the detection of DHPS siRNA moleclules specific to a DHPS gene or by an observation of a reduction in the amount of DHPS RNA transcript produced relative to an untreated plant or by merely observing the anticipated phenotype of the application of the trigger with the DHPS inhibitor containing herbicide. Detection of siRNA can be accomplished, for example, using kits such as mirVana (Ambion, Austin TX) and mirPremier (Sigma-Aldrich, St Louis, MO).

The target DNA sequence isolated from genomic (gDNA) and coding DNA (cDNA) from the various weedy plant species for the DHPS gene and the assembled contigs as set forth in SEQ ID NOs 1-54 were divided into polynucleotide fragments as shown in Table 2 and as set forth in SEQ ID NOs 55-906.

The gene sequences and fragments of Table 1 were compared and 21-mers of contiguous polynucleotides were identified that had homology across the various DHPS gene sequences. The purpose is to identify trigger molecules that are useful as herbicidal molecules or in combination with a DHPS inhibitor herbicide across a broad range of weed species. The sequences shown in Table 3 represent the 21-mers that were present in the DHPS gene of at least eight of the weed species of Table 1. It is contemplated that additional 21-mers can be selected from the sequences of Table 1 that are specific for a single weed species or a few weeds species within a genus or trigger molecules that are at least 18 contiguous nucleotides, at least 19 contiguous nucleotides, at least 20 contiguous nucleotides or at least 21 contiguous nucleotides in length and at least 85 percent identical to an DHPS gene sequence selected from the group consisting of SEQ ID NOs:1-54 or fragment thereof.

By this method it is possible to identify an oligonucleotide or several oligonucleotides that are the most efficacious trigger molecule to effect plant sensitivity to DHPS inhibitor or modulation of DHPS gene expression. The modulation of DHPS gene expression is determined by the detection of DHPS siRNA moleclules specific to DHPS gene or by an observation of a reduction in the amount of DHPS RNA transcript produced relative to an untreated plant. Detection of siRNA can be accomplished, for example, using kits such as mirVana (Ambion, Austin TX) and mirPremier (Sigma-Aldrich, St Louis, MO).

The target DNA sequence isolated from genomic (gDNA) and coding DNA (cDNA) from the various weedy plant species for the DHPS gene and the assembled contigs as set forth in SEQ ID NOs 1-54 were divided into fragments as shown in Table 3 and as set forth in SEQ ID NOs 907-1175.

### Example 3. Methods related to treating plants or plant parts with a topical mixture of the trigger molecules.

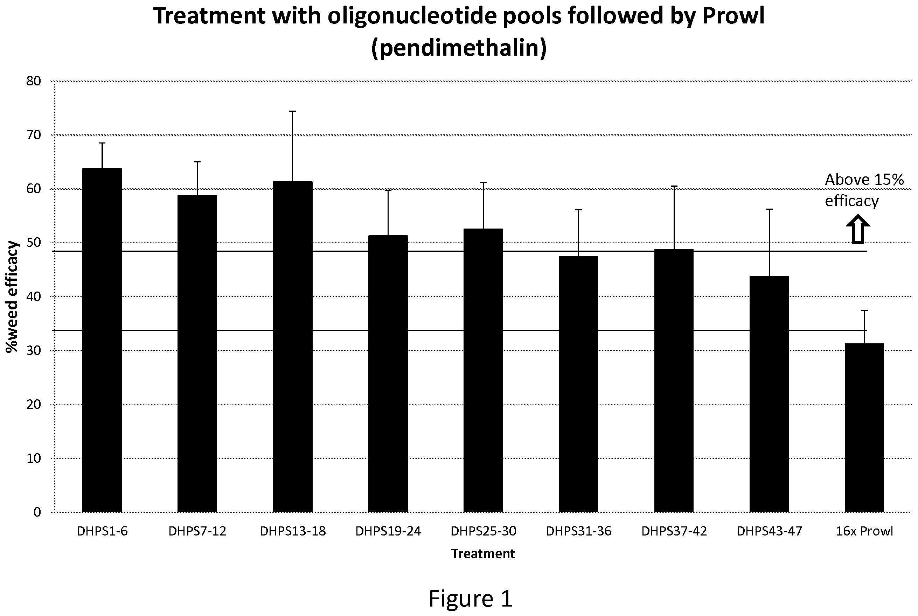

Glyphosate-sensitive Palmer amaranth (*A. palmeri* R-22) plants were grown in the greenhouse (30 / 20 C day/night T; 14 hour photoperiod) in 10.16 cm (4 inch) square pots containing Sun Crro® Redi-Earth and 3.5 kg/cubic meter Osmocote® 14-14-14 fertilizer. Palmer amaranth plants at 5 to 10 cm in height were pre-treated with a mixture of short (25mer) dsDNA trigger oligonucleotides targeting DHPS coding or noncoding regions using 4 nmol of each oligonucleotide and pooling 5-6 oligonucleotides in each treatment, formulated in 20 millimolar sodium phosphate buffer (pH 6.8) containing 2% ammonium sulfate and 1% Silwet L-77. Plants were treated manually by pipetting 10 µL of polynucleotide solution on four fully expanded mature leaves, for a total of 40 microliters of solution per plant. There were eight treatment pools, DHPS1-6, DHPS7-12, DHPS13-18, DHPS19-24, DHPS25-30, DHPS31-36, DHPS37-42, and DHPS43-47 (Table 4). Twenty-four hours later, the plants were treated with pendimethalin (Prowl®, BASF, this herbicide functions as a mitosis inhibitor similar to asulam a known DHPS inhibitor, other dinitroaniline herbicides that function to inhibit mitosis include but are not limited to Sonalan® (ethalfluralin), Squadron1®, Steel1®, Treflan®/Trilin®/Tri-4) at a rate of 13.45 kg/ha (12 lb/ac). Four replications of each treatment was conducted. Plant height was determined just before polynucleotide treatment and at intervals upto fourteen days after herbicide treatments to determine effect of the oligonucleotide and herbicide treatments. The results were expressed as percent reduction in height relative to the untreated control (no formulation and no trigger molecules), another treatment was the formulation control which is herbicide plus buffer plus ammonium plus Silwet. Figure 1 illustrates the results of this test. Three of the pooled oligonucleotides demonstrated an enhancement of the herbicide activity, these are DHPSl-6, DHPS7-12, and DHPS13-18. Further testing of single oligonucleotides from DHPS1-6 and DHPS7-12 demonstrated that DHPS 1 (SEQ ID NO:1176) and DHPS11(SEQ ID NO:1186) had the highest activity among the oligonucleotides in those respective pools with these 2 oligonucleotides providing greater than 15 percent increase in herbicide injury.

**Table 4. DHPS dsDNA oligonucleotides.**

| trigger name | SEQ ID NO: | Sense sequence | Antisense sequence |
|---|---|---|---|
| DHPS1 | 1176 | TTTTATTCTAAAGTTGCttcGGAGG | CCTCCGAAGCAACTTTAGAATAAAA |
| DHPS2 | 1177 | CCGTCAAGAAGGGGGCACACATTGT | ACAATGTGTGCCCCCTTCTTGACGG |
| DHPS3 | 1178 | GAATGATGTCTCtaGTGGgAAACTC | GAGTTTCCCACTAGAGACATCATTC |
| DHPS4 | 1179 | GATTCCGAGATGTTTAATGTTGTTG | CAACAACATTAAACATCTCGGAATC |
| DHPS5 | 1180 | CGGACCTTAAAGTTCCTTATATAGC | GCTATATAAGGAACTTTAAGGTCCG |
| DHPS6 | 1181 | AATGCACATGCGAGGAGATCCGACT | AGTCGGATCTCCTCGCATGTGCATT |
| DHPS7 | 1182 | TCAATGCAAAACTCTGAGAACTTGA | TCAAGTTCTCAGAGTTTTGCATTGA |
| DHPS8 | 1183 | CCTACAATGATGTTTGTAAGCAAGT | ACTTGCTTACAAACATCATTGTAGG |
| DHPS9 | 1184 | GGCTTCGGAGTTGAGTTCTAGGGTc | GACCCTAGAACTCAACTCCGAAGCC |
| DHPS10 | 1185 | ATAGATGCAGAATTATCGGGAATTC | GAATTCCCGATAATTCTGCATCTAT |
| DHPS11 | 1186 | CTGCTTGGAGGATAGTTATTGATCC | GGATCAATAACTATCCTCCAAGCAG |
| DHPS12 | 1187 | CGGCATCGGATTTTCTAAGAATACG | CGTATTCTTAGAAAATCCGATGCCG |
| DHPS13 | 1188 | AATCAAAATTTGGAAATTCTTAGTG | CACTAAGAATTTCCAAATTTTGATT |
| DHPS14 | 1189 | GTTTACAAAAGATACGGGAAGAGAT | ATCTCTTCCCGTATCTTTTGTAAAC |
| DHPS15 | 1190 | AGCTAAGAAGAGTTTGGCGGTGGCT | AGCCACCGCCAAACTCTTCTTAGCT |
| DHPS16 | 1191 | CATTGCCCCTTGCTAATTGGACCTT | AAGGTCCAATTAGCAAGGGGCAATG |
| DHPS17 | 1192 | CAAGAAAGAGGTTTCTGGGCGAGAT | ATCTCGCCCAGAAACCTCTTTCTTG |
| DHPS18 | 1193 | TTGTGAGCGCCCTGTAGCAGCTGAC | GTCAGCTGCTACAGGGCGCTCACAA |
| DHPS19 | 1194 | AGGGATCCTGCTACCATTGCTTCTA | TAGAAGCAATGGTAGCAGGATCCCT |
| DHPS20 | 1195 | TAACTGCTGGAGTTTTAGGTGGTGC | GCACCACCTAAAACTCCAGCAGTTA |
| DHPS21 | 1196 | AAACATTGTAAGAGTACATAATGTt | AACATTATGTACTCTTACAATGTTT |
| DHPS22 | 1197 | AGGGATAACCTTGATGCTGTCAAGT | ACTTGACAGCATCAAGGTTATCCCT |
| DHPS23 | 1198 | TATGTGATGCCATACTCGGAAAAAC | GTTTTTCCGAGTATGGCATCACATA |
| DHPS24 | 1199 | TGATTAACTGCTTGTTTGTACCACC | GGTGGTACAAACAAGCAGTTAATCA |
| DHPS25 | 1200 | TTGTGAATGATGTCTAGTGGAAGGT | ACCTTCCACTAGACATCATTCACAA |
| DHPS26 | 1201 | TCGATTGGGATTATGATGAAGTCCG | CGGACTTCATCATAATCCCAATCGA |
| DHPS27 | 1202 | TTAGTTTGCTCGAAAGtaGAGCTTT | AAAGCTCTACTTTCGAGCAAACTAA |
| DHPS28 | 1203 | AGCCCGTAAGGctATAGTTCTTACA | TGTAAGAACTATAGCCTTACGGGCT |
| DHPS29 | 1204 | GATGTAGAATCATTGGCTATTTGCC | GGCAAATAGCCAATGATTCTACATC |
| DHPS30 | 1205 | TATTTCTGCTTAGAAGATCATAGCA | TGCTATGATCTTCTAAGCAGAAATA |
| DHPS31 | 1206 | TTGATCCAGACTTTGGATTCCATGT | ACATGGAATCCAAAGTCTGGATCAA |
| DHPS32 | 1207 | AGAATTATGTTGCGACTCATGGTCG | CGACCATGAGTCGCAACATAATTCT |
| DHPS33 | 1208 | AATTGGCCCTTTGAGGAATTGTTGA | TCAACAATTCCTCAAAGGGCCAATT |
| DHPS34 | 1209 | AGTTCCTTGAGTCTATGGTTCAATC | GATTGAACCATAGACTCAAGGAACT |
| DHPS35 | 1210 | GGCTCGGTTCGATGAAGGTTGGACT | AGTCCAACCTTCATCGAACCGAGCC |
| DHPS36 | 1211 | GTGTCGAGACAAGATGGGTCAAAGA | TCTTTGACCCATCTTGTCTCGACAC |
| DHPS37 | 1212 | GACCAATGGACAAGAAGTCGACCTT | AAGGTCGACTTCTTGTCCATTGGTC |
| DHPS38 | 1213 | GGGCAAGCTTGATCgaGTTACTTGG | CCAAGTAACTCGATCAAGCTTGCCC |
| DHPS39 | 1214 | ACAaGCAGATAACAGCAGGCTCTGG | CCAGAGCCTGCTGTTATCTGCTTGT |
| DHPS40 | 1215 | CGACcATTAGACATACATATCATTt | AAATGATATGTATGTCTAATGGTCG |
| DHPS41 | 1216 | GTTTTTtGAAGTCAGATCTGCAATC | GATTGCAGATCTGACTTCAAAAAAC |
| DHPS42 | 1217 | AATCAGTTGCAATGGACAAaCCATA | TATGGTTTGTCCATTGCAACTGATT |
| DHPS43 | 1218 | TACGGTTaTTagTTGTTCCTGTCAC | GTGACAGGAACAACTAATAACCGTA |
| DHPS44 | 1219 | TgTTTGAGTTGAATTAGATCATGCA | TGCATGATCTAATTCAACTCAAACA |
| DHPS45 | 1220 | AACATTGTAAGAATCTTGATCATGT | ACATGATCAAGATTCTTACAATGTT |
| DHPS46 | 1221 | GAAGCTATGTGATACTGTACTTCTC | GAGAAGTACAGTATCACATAGCTTC |
| DHPS47 | 1222 | AAAATGAAATCTTTGATATGATGTT | AACATCATATCAAAGATTTCATTTT |

### Example 4. A method to control weeds in a field.

A method to control weeds in a field comprises the use of trigger polynucleotides that can modulate the expression of a DHPS gene in one or more target weed plant species. In Table 3, an analysis of DHPS gene sequences from multiple plant species provided a collection of 21-mer polynucleotides that were common to at least 4 of the species and can be used in compositions to affect the growth or develop or sensitivity to DHPS inhibitor herbicide to control multiple weed species in a field. Other oligonucleotide segments can be selected from the disclosed gene sequences that are more specific to a particular weed species, for example, an oligonucleotide that has a sequence that is homologous or complementary to a DHPS gene of three weeds species, or of two weed species, or of only one weed species. A composition containing 1 or 2 or 3 or 4 or more of the polynucleotides of Table 3 would enable broad activity of the composition against the multiple weed species that occur in a field environment.

The method includes creating a composition that comprises components that include at least one polynucleotide of Table 3 (SEQ ID NO:907-1175) or any other effective gene expression modulating polynucleotide essentially identical or essentially complementary to SEQ ID NO:1-54 or fragment thereof, a transfer agent that mobilizes the polynucleotide into a plant cell and a DHPS inhibiting herbicide and optionally a polynucleotide that modulates the expression of an essential gene and optionally a co-herbicide that has a different mode of action relative to a DHPS inhibitor. The polynucleotide of the composition includes a dsRNA, ssDNA or dsDNA or a combination thereof. A composition containing a polynucleotide can have a use rate of about 1 to 30 grams or more per acre depending on the size of the polynucleotide and the number of polynucleotides in the composition. The composition may include one or more additional co-herbicides as needed to provide effective multi-species weed control. Crop safety can be enhanced by reducing the amount of effective herbicide needed to control weeds in the field. A field of crop plants or a turf grass environment in need of weedy plant control is treated by spray application of the composition. The composition can be provided as a tank mix, a sequential treatment of components (generally the polynucleotide followed by the herbicide), a simultaneous treatment or mixing of one or more of the components of the composition from separate containers. Treatment of the field can occur as often as needed to provide weed control and the components of the composition can be adjusted to target specific weed species or weed families.

### Example 5. Herbicidal Compositions comprising pesticidal agents

A method of controlling weeds and plant pest and pathogens in a field of DHPS inhibitor tolerant crop plants is provided, wherein the method comprises applying a composition comprising a DHPS trigger oligonucleotide, a DHPS inhibitor composition and an admixture of a pest control agent. For example, the admixture comprises insecticides, fungicides, nematocides, bactericides, acaricides, growth regulators, chemosterilants, semiochemicals, repellents, attractants, pheromones, feeding stimulants or other biologically active compounds or biological agents, such as, microorganisms.

For example, the admixture comprises a fungicide compound for use on a DHPS inhibitor tolerant crop plant to prevent or control plant disease caused by a plant fungal pathogen, The fungicide compound of the admixture may be a systemic or contact fungicide or mixtures of each. More particularly the fungicide compound includes, but is not limited to members of the chemical groups strobilurins, triazoles, chloronitriles, carboxamides and mixtures thereof. The composition may additional have an admixture comprises an insecticidal compound or agent.

The DHPS trigger oligonucleotides and DHPS inhibitor or mitosis inhibitor herbicide (for example, asulam, or other dinitroaniline herbicides) tank mixes with fungicides, insecticides or both are tested for use in soybean and corn for control of foliar diseases and pests. Testing is conducted to develop a method for use of mixtures of the trigger oligonucleotides and asulam formulation and various commercially available fungicides for weed control and pest control. The field plots are planted with soybeans or corn. All plots receive a post plant application of the DHPS trigger + asulam about 3 weeks after planting. The mixtures of trigger + asulam or trigger + asulam + fungicide + insecticides are used to treat the plots at the R1 stage of soybean development (first flowering) or tassel stage of corn. Data is taken for percent weed control at 7 and 21 days after R1 treatment, soybean safety (% necrosis, chlorosis, growth rate): 5 days after treatment, disease rating, pest ratings and yield (bushels/Acre). These mixtures and treatments are designed to provide simultaneous weed and pest control, such as fungal pest control, for example, leaf rust disease; and insect pest control, for example, aphids, armyworms, loopers, beetles, stinkbugs, and leaf hoppers.

Agricultural chemicals are provided in containers suitable for safe storage, transportation and distribution, stability of the chemical compositions, mixing with solvents and instructions for use. A container of a mixture of a trigger oligonucleotide + herbicice + fungicide compound, or a mixture of a trigger oligonucleotide + herbicide compound and an insecticide compound, or a trigger oligonucleotide + a herbicide compound and a fungicide compound and an insecticide compound (for example, lambda-cyhalothrin, Warrier®). The container may further provide instructions on the effective use of the mixture. Containers can be of any material that is suitable for the storage of the chemical mixture. Containers can be of any material that is suitable for the shipment of the chemical mixture. The material can be of cardboard, plastic, metal, or a composite of these materials. The container can have a volume of 0.5 liter, 1 liter, 2 liter, 3-5 liter, 5-10 liter, 10-20 liter, 20-50 liter or more depending upon the need. A tank mix of a trigger oligonucleotide + herbicide compound and a fungicide compound is provided, methods of application to the crop to achieve an effective dose of each compound are known to those skilled in the art and can be refined and further developed depending on the crop, weather conditions, and application equipment used.

Insecticides, fungicides, nematocides, bactericides, acaricides, growth regulators, chemosterilants, semiochemicals, repellents, attractants, pheromones, feeding stimulants or other biologically active compounds can be added to the trigger oligonucleotide to form a multi-component pesticide giving an even broader spectrum of agricultural protection. Examples of such agricultural protectants with which compounds of this invention can be formulated are: insecticides such as abamectin, acephate, azinphos-methyl, bifenthrin, buprofezin, carbofuran, chlorfenapyr, chlorpyrifos, chlorpyrifos-methyl, cyfluthrin, beta-cyfluthrin, cyhalothrin, lambda-cyhalothrin, deltamethrin, diafenthiuron, diazinon, diflubenzuron, dimethoate, esfenvalerate, fenoxycarb, fenpropathrin, fenvalerate, fipronil, flucythrinate, tau-fluvalinate, fonophos, imidacloprid, isofenphos, malathion, metaldehyde, methamidophos, methidathion, methomyl, methoprene, methoxychlor, methyl 7-chloro-2,5-dihydro-2-[[N-(methoxycarbonyl)-N-[4-(trifluoromethoxy)phenyl ]amino]carbonyl]indeno[1,2-e][1,3,4]oxadiazine-4a(3H)-carboxylate (DPX-JW062), monocrotophos, oxamyl, parathion, parathion-methyl, permethrin, phorate, phosalone, phosmet, phosphamidon, pirimicarb, profenofos, rotenone, sulprofos, tebufenozide, tefluthrin, terbufos, tetrachlorvinphos, thiodicarb, tralomethrin, trichlorfon and triflumuron; most preferably a DHPS inhibitor compound is formulated with a fungicide compound or combinations of fungicides, such as azoxystrobin, benomyl, blasticidin-S, Bordeaux mixture (tribasic copper sulfate), bromuconazole, captafol, captan, carbendazim, chloroneb, chlorothalonil, copper oxychloride, copper salts, cymoxanil, cyproconazole, cyprodinil (CGA 219417), diclomezine, dicloran, difenoconazole, dimethomorph, diniconazole, diniconazole-M, dodine, edifenphos, epoxiconazole (BAS 480F), famoxadone, fenarimol, fenbuconazole, fenpiclonil, fenpropidin, fenpropimorph, fluazinam, fluquinconazole, flusilazole, flutolanil, flutriafol, folpet, fosetyl-aluminum, furalaxyl, hexaconazole, ipconazole, iprobenfos, iprodione, isoprothiolane, kasugamycin, kresoxim-methyl, mancozeb, maneb, mepronil, metalaxyl, metconazole, S-methyl 7-benzothiazolecarbothioate (CGA 245704), myclobutanil, neo-asozin (ferric methanearsonate), oxadixyl, penconazole, pencycuron, probenazole, prochloraz, propiconazole, pyrifenox, pyroquilon, quinoxyfen, spiroxamine (KWG4168), sulfur, tebuconazole, tetraconazole, thiabendazole, thiophanate-methyl, thiram, triadimefon, triadimenol, tricyclazole, trifloxystrobin, triticonazole, validamycin and vinclozolin; combinations of fungicides are common for example, cyproconazole and azoxystrobin, difenoconazole, and metalaxyl-M, fludioxonil and metalaxyl-M, mancozeb and metalaxyl-M, copper hydroxide and metalaxyl-M, cyprodinil and fludioxonil, cyproconazole and propiconazole; commercially available fungicide formulations for control of Asian soybean rust disease include, but are not limited to Quadris® (Syngenta Corp), Bravo® (Syngenta Corp), Echo 720® (Sipcam Agro Inc), Headline® 2.09EC (BASF Corp), Tilt® 3.6EC (Syngenta Corp), PropiMax™ 3.6EC (Dow AgroSciences), Bumper® 41.8EC (MakhteshimAgan), Folicur® 3.6F (Bayer CropScience), Laredo® 25EC (Dow AgroSciences), Laredo™ 25EW (Dow AgroSciences), Stratego® 2.08F (Bayer Corp), Domark™ 125SL (Sipcam Agro USA), and Pristine®38%WDG (BASF Corp) these can be combined with DHPS inhibitor compositions as described in the present invention to provide enhanced protection from fungal disease; nematocides such as aldoxycarb and fenamiphos; bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad; and biological agents such as *Bacillus thuringiensis*, *Bacillus thuringiensis* delta endotoxin, baculovirus, and entomopathogenic bacteria, virus and fungi.

**Table 1**

| SEQ ID NO | SPECIES | TYPE | LENGTH |
|---|---|---|---|
| 1 | Amaranthus hybridus | cDNAContig | 672 |
| 2 | Amaranthus lividus | cDNAContig | 458 |
| 3 | Amaranthus palmeri | gDNAContig | 5590 |
| 4 | Amaranthus palmeri | gDNAContig | 5515 |
| 5 | Amaranthus palmeri | cDNAContig | 1431 |
| 6 | Amaranthus palmeri | cDNAContig | 1431 |
| 7 | Amaranthus palmeri | cDNAContig | 1163 |
| 8 | Amaranthus rudis | cDNAContig | 1431 |
| 9 | Amaranthus rudis | gDNAContig | 1059 |
| 10 | Amaranthus rudis | gDNAContig | 792 |
| 11 | Amaranthus rudis | gDNAContig | 764 |
| 12 | Amaranthus rudis | gDNAContig | 755 |
| 13 | Amaranthus rudis | cDNAContig | 602 |
| 14 | Amaranthus rudis | gDNAContig | 584 |
| 15 | Amaranthus rudis | gDNAContig | 570 |
| 16 | Amaranthus rudis | cDNAContig | 411 |
| 17 | Amaranthus rudis | gDNAContig | 260 |
| 18 | Amaranthus viridis | cDNAContig | 1504 |
| 19 | Amaranthus viridis | cDNAContig | 655 |
| 20 | Ambrosia trifida | cDNAContig | 1413 |
| 21 | Ambrosia trifida | gDNAContig | 1328 |
| 22 | Ambrosia trifida | gDNAContig | 486 |
| 23 | Ambrosia trifida | cDNAContig | 486 |
| 24 | Ambrosia trifida | cDNAContig | 410 |
| 25 | Ambrosia trifida | gDNAContig | 381 |
| 26 | Ambrosia trifida | gDNAContig | 304 |
| 27 | Ambrosia trifida | gDNAContig | 255 |
| 28 | Ambrosia trifida | gDNAContig | 201 |
| 29 | Ambrosia trifida | cDNAContig | 101 |
| 30 | Conyza canadensis | gDNAContig | 5446 |
| 31 | Conyza canadensis | gDNAContig | 5446 |
| 32 | Conyza canadensis | cDNAContig | 1401 |
| 33 | Conyza canadensis | cDNAContig | 1262 |
| 34 | Euphorbia heterophylla | gDNAContig | 4643 |
| 35 | Euphorbia heterophylla | gDNAContig | 4556 |
| 36 | Euphorbia heterophylla | cDNAContig | 1425 |
| 37 | Euphorbia heterophylla | cDNAContig | 1425 |
| 38 | Euphorbia heterophylla | cDNAContig | 681 |
| 39 | Euphorbia heterophylla | gDNAContig | 444 |
| 40 | Euphorbia heterophylla | gDNAContig | 379 |
| 41 | Digitaria sanguinalis | gDNAContig | 4031 |
| 42 | Digitaria sanguinalis | cDNAContig | 1428 |
| 43 | Kochia scoparia | gDNAContig | 4007 |
| 44 | Kochia scoparia | gDNAContig | 3505 |
| 45 | Kochia scoparia | cDNAContig | 1446 |
| 46 | Kochia scoparia | cDNAContig | 1446 |
| 47 | Lolium multiflorum | gDNAContig | 3920 |
| 48 | Lolium multiflorum | cDNAContig | 1467 |
| 49 | Lolium multiflorum | gDNAContig | 834 |
| 50 | Lolium multiflorum | cDNAContig | 834 |
| 51 | Lolium multiflorum | gDNAContig | 212 |
| 52 | Lolium multiflorum | cDNAContig | 210 |
| 53 | Lolium multiflorum | gDNAContig | 201 |
| 54 | Lolium multiflorum | cDNAContig | 150 |

**Table 2**

| SEQ ID NO | Species | Name \| Reference | Type | Start | End |
|---|---|---|---|---|---|
| 55 | Amaranthus hybridus | DHP_A1_1 | cDNAContig | 1 | 200 |
| 56 | Amaranthus hybridus | DHP_A1_1 | cDNAContig | 1 | 200 |
| 57 | Amaranthus hybridus | DHP_A1_2 | cDNAContig | 176 | 375 |
| 58 | Amaranthus hybridus | DHP_A1_2 | cDNAContig | 176 | 375 |
| 59 | Amaranthus hybridus | DHP_A1_3 | cDNAContig | 351 | 550 |
| 60 | Amaranthus hybridus | DHP_A1_3 | cDNAContig | 351 | 550 |
| 61 | Amaranthus lividus | DHP_A2_1 | cDNAContig | 1 | 200 |
| 62 | Amaranthus lividus | DHP_A2_1 | cDNAContig | 1 | 200 |
| 63 | Amaranthus lividus | DHP_A2_2 | cDNAContig | 176 | 375 |
| 64 | Amaranthus lividus | DHP_A2_2 | cDNAContig | 176 | 375 |
| 65 | Amaranthus palmeri | DHP_A4_1 | gDNAContig | 1 | 200 |
| 66 | Amaranthus palmeri | DHP_A4_1 | gDNAContig | 1 | 200 |
| 67 | Amaranthus palmeri | DHP_A4_2 | gDNAContig | 176 | 375 |
| 68 | Amaranthus palmeri | DHP_A4_2 | gDNAContig | 176 | 375 |
| 69 | Amaranthus palmeri | DHP_A4_3 | gDNAContig | 351 | 550 |
| 70 | Amaranthus palmeri | DHP_A4_3 | gDNAContig | 351 | 550 |
| 71 | Amaranthus palmeri | DHP_A4_4 | gDNAContig | 526 | 725 |
| 72 | Amaranthus palmeri | DHP_A4_4 | gDNAContig | 526 | 725 |
| 73 | Amaranthus palmeri | DHP_A4_5 | gDNAContig | 701 | 900 |
| 74 | Amaranthus palmeri | DHP_A4_5 | gDNAContig | 701 | 900 |
| 75 | Amaranthus palmeri | DHP_A4_6 | gDNAContig | 876 | 1075 |
| 76 | Amaranthus palmeri | DHP_A4_6 | gDNAContig | 876 | 1075 |
| 77 | Amaranthus palmeri | DHP_A4_7 | gDNAContig | 1051 | 1250 |
| 78 | Amaranthus palmeri | DHP_A4_7 | gDNAContig | 1051 | 1250 |
| 79 | Amaranthus palmeri | DHP_A4_8 | gDNAContig | 1226 | 1425 |
| 80 | Amaranthus palmeri | DHP_A4_8 | gDNAContig | 1226 | 1425 |
| 81 | Amaranthus palmeri | DHP_A4_9 | gDNAContig | 1401 | 1600 |
| 82 | Amaranthus palmeri | DHP_A4_9 | gDNAContig | 1401 | 1600 |
| 83 | Amaranthus palmeri | DHP_A4_10 | gDNAContig | 1576 | 1775 |
| 84 | Amaranthus palmeri | DHP_A4_10 | gDNAContig | 1576 | 1775 |
| 85 | Amaranthus palmeri | DHP_A4_11 | gDNAContig | 1751 | 1950 |
| 86 | Amaranthus palmeri | DHP_A4_11 | gDNAContig | 1751 | 1950 |
| 87 | Amaranthus palmeri | DHP_A4_12 | gDNAContig | 1926 | 2125 |
| 88 | Amaranthus palmeri | DHP_A4_12 | gDNAContig | 1926 | 2125 |
| 89 | Amaranthus palmeri | DHP_A4_13 | gDNAContig | 2101 | 2300 |
| 90 | Amaranthus palmeri | DHP_A4_13 | gDNAContig | 2101 | 2300 |
| 91 | Amaranthus palmeri | DHP_A4_14 | gDNAContig | 2276 | 2475 |
| 92 | Amaranthus palmeri | DHP_A4_14 | gDNAContig | 2276 | 2475 |
| 93 | Amaranthus palmeri | DHP_A4_15 | gDNAContig | 2451 | 2650 |
| 94 | Amaranthus palmeri | DHP_A4_15 | gDNAContig | 2451 | 2650 |
| 95 | Amaranthus palmeri | DHP_A4_16 | gDNAContig | 2626 | 2825 |
| 96 | Amaranthus palmeri | DHP_A4_16 | gDNAContig | 2626 | 2825 |
| 97 | Amaranthus palmeri | DHP_A4_17 | gDNAContig | 2801 | 3000 |
| 98 | Amaranthus palmeri | DHP_A4_17 | gDNAContig | 2801 | 3000 |
| 99 | Amaranthus palmeri | DHP_A4_18 | gDNAContig | 2976 | 3175 |
| 100 | Amaranthus palmeri | DHP_A4_18 | gDNAContig | 2976 | 3175 |
| 101 | Amaranthus palmeri | DHP_A4_19 | gDNAContig | 3151 | 3350 |
| 102 | Amaranthus palmeri | DHP_A4_19 | gDNAContig | 3151 | 3350 |
| 103 | Amaranthus palmeri | DHP_A4_20 | gDNAContig | 3326 | 3525 |
| 104 | Amaranthus palmeri | DHP_A4_20 | gDNAContig | 3326 | 3525 |
| 105 | Amaranthus palmeri | DHP_A4_21 | gDNAContig | 3501 | 3700 |
| 106 | Amaranthus palmeri | DHP_A4_21 | gDNAContig | 3501 | 3700 |
| 107 | Amaranthus palmeri | DHP_A4_22 | gDNAContig | 3676 | 3875 |
| 108 | Amaranthus palmeri | DHP_A4_22 | gDNAContig | 3676 | 3875 |
| 109 | Amaranthus palmeri | DHP_A4_23 | gDNAContig | 3851 | 4050 |
| 110 | Amaranthus palmeri | DHP_A4_23 | gDNAContig | 3851 | 4050 |
| 111 | Amaranthus palmeri | DHP_A4_24 | gDNAContig | 4026 | 4225 |
| 112 | Amaranthus palmeri | DHP_A4_24 | gDNAContig | 4026 | 4225 |
| 113 | Amaranthus palmeri | DHP_A4_25 | gDNAContig | 4201 | 4400 |
| 114 | Amaranthus palmeri | DHP_A4_25 | gDNAContig | 4201 | 4400 |
| 115 | Amaranthus palmeri | DHP_A4_26 | gDNAContig | 4376 | 4575 |
| 116 | Amaranthus palmeri | DHP_A4_26 | gDNAContig | 4376 | 4575 |
| 117 | Amaranthus palmeri | DHP_A4_27 | gDNAContig | 4551 | 4750 |
| 118 | Amaranthus palmeri | DHP_A4_27 | gDNAContig | 4551 | 4750 |
| 119 | Amaranthus palmeri | DHP_A4_28 | gDNAContig | 4726 | 4925 |
| 120 | Amaranthus palmeri | DHP_A4_28 | gDNAContig | 4726 | 4925 |
| 121 | Amaranthus palmeri | DHP_A4_29 | gDNAContig | 4901 | 5100 |
| 122 | Amaranthus palmeri | DHP_A4_29 | gDNAContig | 4901 | 5100 |
| 123 | Amaranthus palmeri | DHP_A4_30 | gDNAContig | 5076 | 5275 |
| 124 | Amaranthus palmeri | DHP_A4_30 | gDNAContig | 5076 | 5275 |
| 125 | Amaranthus palmeri | DHP_A4_31 | gDNAContig | 5251 | 5450 |
| 126 | Amaranthus palmeri | DHP_A4_31 | gDNAContig | 5251 | 5450 |
| 127 | Amaranthus palmeri | DHP_A7_1 | cDNAContig | 1 | 200 |
| 128 | Amaranthus palmeri | DHP_A7_1 | cDNAContig | 1 | 200 |
| 129 | Amaranthus palmeri | DHP_A7_2 | cDNAContig | 176 | 375 |
| 130 | Amaranthus palmeri | DHP_A7_2 | cDNAContig | 176 | 375 |
| 131 | Amaranthus palmeri | DHP_A7_3 | cDNAContig | 351 | 550 |
| 132 | Amaranthus palmeri | DHP_A7_3 | cDNAContig | 351 | 550 |
| 133 | Amaranthus palmeri | DHP_A7_4 | cDNAContig | 526 | 725 |
| 134 | Amaranthus palmeri | DHP_A7_4 | cDNAContig | 526 | 725 |
| 135 | Amaranthus palmeri | DHP_A7_5 | cDNAContig | 701 | 900 |
| 136 | Amaranthus palmeri | DHP_A7_5 | cDNAContig | 701 | 900 |
| 137 | Amaranthus palmeri | DHP_A7_6 | cDNAContig | 876 | 1075 |
| 138 | Amaranthus palmeri | DHP_A7_6 | cDNAContig | 876 | 1075 |
| 139 | Amaranthus palmeri | DHP_A5_1 | cDNAContig | 1 | 200 |
| 140 | Amaranthus palmeri | DHP_A5_1 | cDNAContig | 1 | 200 |
| 141 | Amaranthus palmeri | DHP_A5_2 | cDNAContig | 176 | 375 |
| 142 | Amaranthus palmeri | DHP_A5_2 | cDNAContig | 176 | 375 |
| 143 | Amaranthus palmeri | DHP_A5_3 | cDNAContig | 351 | 550 |
| 144 | Amaranthus palmeri | DHP_A5_3 | cDNAContig | 351 | 550 |
| 145 | Amaranthus palmeri | DHP_A5_4 | cDNAContig | 526 | 725 |
| 146 | Amaranthus palmeri | DHP_A5_4 | cDNAContig | 526 | 725 |
| 147 | Amaranthus palmeri | DHP_A5_5 | cDNAContig | 701 | 900 |
| 148 | Amaranthus palmeri | DHP_A5_5 | cDNAContig | 701 | 900 |
| 149 | Amaranthus palmeri | DHP_A5_6 | cDNAContig | 876 | 1075 |
| 150 | Amaranthus palmeri | DHP_A5_6 | cDNAContig | 876 | 1075 |
| 151 | Amaranthus palmeri | DHP_A5_7 | cDNAContig | 1051 | 1250 |
| 152 | Amaranthus palmeri | DHP_A5_7 | cDNAContig | 1051 | 1250 |
| 153 | Amaranthus palmeri | DHP_A5_8 | cDNAContig | 1226 | 1425 |
| 154 | Amaranthus palmeri | DHP_A5_8 | cDNAContig | 1226 | 1425 |
| 155 | Amaranthus palmeri | DHP_A6_1 | cDNAContig | 1 | 200 |
| 156 | Amaranthus palmeri | DHP_A6_1 | cDNAContig | 1 | 200 |
| 157 | Amaranthus palmeri | DHP_A6_2 | cDNAContig | 176 | 375 |
| 158 | Amaranthus palmeri | DHP_A6_2 | cDNAContig | 176 | 375 |
| 159 | Amaranthus palmeri | DHP_A6_3 | cDNAContig | 351 | 550 |
| 160 | Amaranthus palmeri | DHP_A6_3 | cDNAContig | 351 | 550 |
| 161 | Amaranthus palmeri | DHP_A6_4 | cDNAContig | 526 | 725 |
| 162 | Amaranthus palmeri | DHP_A6_4 | cDNAContig | 526 | 725 |
| 163 | Amaranthus palmeri | DHP_A6_5 | cDNAContig | 701 | 900 |
| 164 | Amaranthus palmeri | DHP_A6_5 | cDNAContig | 701 | 900 |
| 165 | Amaranthus palmeri | DHP_A6_6 | cDNAContig | 876 | 1075 |
| 166 | Amaranthus palmeri | DHP_A6_6 | cDNAContig | 876 | 1075 |
| 167 | Amaranthus palmeri | DHP_A6_7 | cDNAContig | 1051 | 1250 |
| 168 | Amaranthus palmeri | DHP_A6_7 | cDNAContig | 1051 | 1250 |
| 169 | Amaranthus palmeri | DHP_A6_8 | cDNAContig | 1226 | 1425 |
| 170 | Amaranthus palmeri | DHP_A6_8 | cDNAContig | 1226 | 1425 |
| 171 | Amaranthus palmeri | DHP_A3_1 | gDNAContig | 1 | 200 |
| 172 | Amaranthus palmeri | DHP_A3_1 | gDNAContig | 1 | 200 |
| 173 | Amaranthus palmeri | DHP_A3_2 | gDNAContig | 176 | 375 |
| 174 | Amaranthus palmeri | DHP_A3_2 | gDNAContig | 176 | 375 |
| 175 | Amaranthus palmeri | DHP_A3_3 | gDNAContig | 351 | 550 |
| 176 | Amaranthus palmeri | DHP_A3_3 | gDNAContig | 351 | 550 |
| 177 | Amaranthus palmeri | DHP_A3_4 | gDNAContig | 526 | 725 |
| 178 | Amaranthus palmeri | DHP_A3_4 | gDNAContig | 526 | 725 |
| 179 | Amaranthus palmeri | DHP_A3_5 | gDNAContig | 701 | 900 |
| 180 | Amaranthus palmeri | DHP_A3_5 | gDNAContig | 701 | 900 |
| 181 | Amaranthus palmeri | DHP_A3_6 | gDNAContig | 876 | 1075 |
| 182 | Amaranthus palmeri | DHP_A3_6 | gDNAContig | 876 | 1075 |
| 183 | Amaranthus palmeri | DHP_A3_7 | gDNAContig | 1051 | 1250 |
| 184 | Amaranthus palmeri | DHP_A3_7 | gDNAContig | 1051 | 1250 |
| 185 | Amaranthus palmeri | DHP_A3_8 | gDNAContig | 1226 | 1425 |
| 186 | Amaranthus palmeri | DHP_A3_8 | gDNAContig | 1226 | 1425 |
| 187 | Amaranthus palmeri | DHP_A3_9 | gDNAContig | 1401 | 1600 |
| 188 | Amaranthus palmeri | DHP_A3_9 | gDNAContig | 1401 | 1600 |
| 189 | Amaranthus palmeri | DHP_A3_10 | gDNAContig | 1576 | 1775 |
| 190 | Amaranthus palmeri | DHP_A3_10 | gDNAContig | 1576 | 1775 |
| 191 | Amaranthus palmeri | DHP_A3_11 | gDNAContig | 1751 | 1950 |
| 192 | Amaranthus palmeri | DHP_A3_11 | gDNAContig | 1751 | 1950 |
| 193 | Amaranthus palmeri | DHP_A3_12 | gDNAContig | 1926 | 2125 |
| 194 | Amaranthus palmeri | DHP_A3_12 | gDNAContig | 1926 | 2125 |
| 195 | Amaranthus palmeri | DHP_A3_13 | gDNAContig | 2101 | 2300 |
| 196 | Amaranthus palmeri | DHP_A3_13 | gDNAContig | 2101 | 2300 |
| 197 | Amaranthus palmeri | DHP_A3_14 | gDNAContig | 2276 | 2475 |
| 198 | Amaranthus palmeri | DHP_A3_14 | gDNAContig | 2276 | 2475 |
| 199 | Amaranthus palmeri | DHP_A3_15 | gDNAContig | 2451 | 2650 |
| 200 | Amaranthus palmeri | DHP_A3_15 | gDNAContig | 2451 | 2650 |
| 201 | Amaranthus palmeri | DHP_A3_16 | gDNAContig | 2626 | 2825 |
| 202 | Amaranthus palmeri | DHP_A3_16 | gDNAContig | 2626 | 2825 |
| 203 | Amaranthus palmeri | DHP_A3_17 | gDNAContig | 2801 | 3000 |
| 204 | Amaranthus palmeri | DHP_A3_17 | gDNAContig | 2801 | 3000 |
| 205 | Amaranthus palmeri | DHP_A3_18 | gDNAContig | 2976 | 3175 |
| 206 | Amaranthus palmeri | DHP_A3_18 | gDNAContig | 2976 | 3175 |
| 207 | Amaranthus palmeri | DHP_A3_19 | gDNAContig | 3151 | 3350 |
| 208 | Amaranthus palmeri | DHP_A3_19 | gDNAContig | 3151 | 3350 |
| 209 | Amaranthus palmeri | DHP_A3_20 | gDNAContig | 3326 | 3525 |
| 210 | Amaranthus palmeri | DHP_A3_20 | gDNAContig | 3326 | 3525 |
| 211 | Amaranthus palmeri | DHP_A3_21 | gDNAContig | 3501 | 3700 |
| 212 | Amaranthus palmeri | DHP_A3_21 | gDNAContig | 3501 | 3700 |
| 213 | Amaranthus palmeri | DHP_A3_22 | gDNAContig | 3676 | 3875 |
| 214 | Amaranthus palmeri | DHP_A3_22 | gDNAContig | 3676 | 3875 |
| 215 | Amaranthus palmeri | DHP_A3_23 | gDNAContig | 3851 | 4050 |
| 216 | Amaranthus palmeri | DHP_A3_23 | gDNAContig | 3851 | 4050 |
| 217 | Amaranthus palmeri | DHP_A3_24 | gDNAContig | 4026 | 4225 |
| 218 | Amaranthus palmeri | DHP_A3_24 | gDNAContig | 4026 | 4225 |
| 219 | Amaranthus palmeri | DHP_A3_25 | gDNAContig | 4201 | 4400 |
| 220 | Amaranthus palmeri | DHP_A3_25 | gDNAContig | 4201 | 4400 |
| 221 | Amaranthus palmeri | DHP_A3_26 | gDNAContig | 4376 | 4575 |
| 222 | Amaranthus palmeri | DHP_A3_26 | gDNAContig | 4376 | 4575 |
| 223 | Amaranthus palmeri | DHP_A3_27 | gDNAContig | 4551 | 4750 |
| 224 | Amaranthus palmeri | DHP_A3_27 | gDNAContig | 4551 | 4750 |
| 225 | Amaranthus palmeri | DHP_A3_28 | gDNAContig | 4726 | 4925 |
| 226 | Amaranthus palmeri | DHP_A3_28 | gDNAContig | 4726 | 4925 |
| 227 | Amaranthus palmeri | DHP_A3_29 | gDNAContig | 4901 | 5100 |
| 228 | Amaranthus palmeri | DHP_A3_29 | gDNAContig | 4901 | 5100 |
| 229 | Amaranthus palmeri | DHP_A3_30 | gDNAContig | 5076 | 5275 |
| 230 | Amaranthus palmeri | DHP_A3_30 | gDNAContig | 5076 | 5275 |
| 231 | Amaranthus palmeri | DHP_A3_31 | gDNAContig | 5251 | 5450 |
| 232 | Amaranthus palmeri | DHP_A3_31 | gDNAContig | 5251 | 5450 |
| 233 | Amaranthus rudis | DHP_A10_1 | gDNAContig | 1 | 200 |
| 234 | Amaranthus rudis | DHP_A10_1 | gDNAContig | 1 | 200 |
| 235 | Amaranthus rudis | DHP_A10_2 | gDNAContig | 176 | 375 |
| 236 | Amaranthus rudis | DHP_A10_2 | gDNAContig | 176 | 375 |
| 237 | Amaranthus rudis | DHP_A10_3 | gDNAContig | 351 | 550 |
| 238 | Amaranthus rudis | DHP_A10_3 | gDNAContig | 351 | 550 |
| 239 | Amaranthus rudis | DHP_A10_4 | gDNAContig | 526 | 725 |
| 240 | Amaranthus rudis | DHP_A10_4 | gDNAContig | 526 | 725 |
| 241 | Amaranthus rudis | DHP_A13_1 | cDNAContig | 1 | 200 |
| 242 | Amaranthus rudis | DHP_A13_1 | cDNAContig | 1 | 200 |
| 243 | Amaranthus rudis | DHP_A13_2 | cDNAContig | 176 | 375 |
| 244 | Amaranthus rudis | DHP_A13_2 | cDNAContig | 176 | 375 |
| 245 | Amaranthus rudis | DHP_A13_3 | cDNAContig | 351 | 550 |
| 246 | Amaranthus rudis | DHP_A13_3 | cDNAContig | 351 | 550 |
| 247 | Amaranthus rudis | DHP_A14_1 | gDNAContig | 1 | 200 |
| 248 | Amaranthus rudis | DHP_A14_1 | gDNAContig | 1 | 200 |
| 249 | Amaranthus rudis | DHP_A14_2 | gDNAContig | 176 | 375 |
| 250 | Amaranthus rudis | DHP_A14_2 | gDNAContig | 176 | 375 |
| 251 | Amaranthus rudis | DHP_A14_3 | gDNAContig | 351 | 550 |
| 252 | Amaranthus rudis | DHP_A14_3 | gDNAContig | 351 | 550 |
| 253 | Amaranthus rudis | DHP_A17_1 | gDNAContig | 1 | 200 |
| 254 | Amaranthus rudis | DHP_A17_1 | gDNAContig | 1 | 200 |
| 255 | Amaranthus rudis | DHP_A16_1 | cDNAContig | 1 | 200 |
| 256 | Amaranthus rudis | DHP_A16_1 | cDNAContig | 1 | 200 |
| 257 | Amaranthus rudis | DHP_A16_2 | cDNAContig | 176 | 375 |
| 258 | Amaranthus rudis | DHP_A16_2 | cDNAContig | 176 | 375 |
| 259 | Amaranthus rudis | DHP_A8_1 | cDNAContig | 1 | 200 |
| 260 | Amaranthus rudis | DHP_A8_1 | cDNAContig | 1 | 200 |
| 261 | Amaranthus rudis | DHP_A8_2 | cDNAContig | 176 | 375 |
| 262 | Amaranthus rudis | DHP_A8_2 | cDNAContig | 176 | 375 |
| 263 | Amaranthus rudis | DHP_A8_3 | cDNAContig | 351 | 550 |
| 264 | Amaranthus rudis | DHP_A8_3 | cDNAContig | 351 | 550 |
| 265 | Amaranthus rudis | DHP_A8_4 | cDNAContig | 526 | 725 |
| 266 | Amaranthus rudis | DHP_A8_4 | cDNAContig | 526 | 725 |
| 267 | Amaranthus rudis | DHP_A8_5 | cDNAContig | 701 | 900 |
| 268 | Amaranthus rudis | DHP_A8_5 | cDNAContig | 701 | 900 |
| 269 | Amaranthus rudis | DHP_A8_6 | cDNAContig | 876 | 1075 |
| 270 | Amaranthus rudis | DHP_A8_6 | cDNAContig | 876 | 1075 |
| 271 | Amaranthus rudis | DHP_A8_7 | cDNAContig | 1051 | 1250 |
| 272 | Amaranthus rudis | DHP_A8_7 | cDNAContig | 1051 | 1250 |
| 273 | Amaranthus rudis | DHP_A8_8 | cDNAContig | 1226 | 1425 |
| 274 | Amaranthus rudis | DHP_A8_8 | cDNAContig | 1226 | 1425 |
| 275 | Amaranthus rudis | DHP_A12_1 | gDNAContig | 1 | 200 |
| 276 | Amaranthus rudis | DHP_A12_1 | gDNAContig | 1 | 200 |
| 277 | Amaranthus rudis | DHP_A12_2 | gDNAContig | 176 | 375 |
| 278 | Amaranthus rudis | DHP_A12_2 | gDNAContig | 176 | 375 |
| 279 | Amaranthus rudis | DHP_A12_3 | gDNAContig | 351 | 550 |
| 280 | Amaranthus rudis | DHP_A12_3 | gDNAContig | 351 | 550 |
| 281 | Amaranthus rudis | DHP_A12_4 | gDNAContig | 526 | 725 |
| 282 | Amaranthus rudis | DHP_A12_4 | gDNAContig | 526 | 725 |
| 283 | Amaranthus rudis | DHP_A15_1 | gDNAContig | 1 | 200 |
| 284 | Amaranthus rudis | DHP_A15_1 | gDNAContig | 1 | 200 |
| 285 | Amaranthus rudis | DHP_A15_2 | gDNAContig | 176 | 375 |
| 286 | Amaranthus rudis | DHP_A15_2 | gDNAContig | 176 | 375 |
| 287 | Amaranthus rudis | DHP_A15_3 | gDNAContig | 351 | 550 |
| 288 | Amaranthus rudis | DHP_A15_3 | gDNAContig | 351 | 550 |
| 289 | Amaranthus rudis | DHP_A11_1 | gDNAContig | 1 | 200 |
| 290 | Amaranthus rudis | DHP_A11_1 | gDNAContig | 1 | 200 |
| 291 | Amaranthus rudis | DHP_A11_2 | gDNAContig | 176 | 375 |
| 292 | Amaranthus rudis | DHP_A11_2 | gDNAContig | 176 | 375 |
| 293 | Amaranthus rudis | DHP_A11_3 | gDNAContig | 351 | 550 |
| 294 | Amaranthus rudis | DHP_A11_3 | gDNAContig | 351 | 550 |
| 295 | Amaranthus rudis | DHP_A11_4 | gDNAContig | 526 | 725 |
| 296 | Amaranthus rudis | DHP_A11_4 | gDNAContig | 526 | 725 |
| 297 | Amaranthus rudis | DHP_A9_1 | gDNAContig | 1 | 200 |
| 298 | Amaranthus rudis | DHP_A9_1 | gDNAContig | 1 | 200 |
| 299 | Amaranthus rudis | DHP_A9_2 | gDNAContig | 176 | 375 |
| 300 | Amaranthus rudis | DHP_A9_2 | gDNAContig | 176 | 375 |
| 301 | Amaranthus rudis | DHP_A9_3 | gDNAContig | 351 | 550 |
| 302 | Amaranthus rudis | DHP_A9_3 | gDNAContig | 351 | 550 |
| 303 | Amaranthus rudis | DHP_A9_4 | gDNAContig | 526 | 725 |
| 304 | Amaranthus rudis | DHP_A9_4 | gDNAContig | 526 | 725 |
| 305 | Amaranthus rudis | DHP_A9_5 | gDNAContig | 701 | 900 |
| 306 | Amaranthus rudis | DHP_A9_5 | gDNAContig | 701 | 900 |
| 307 | Amaranthus viridis | DHP_A19_1 | cDNAContig | 1 | 200 |
| 308 | Amaranthus viridis | DHP_A19_1 | cDNAContig | 1 | 200 |
| 309 | Amaranthus viridis | DHP_A19_2 | cDNAContig | 176 | 375 |
| 310 | Amaranthus viridis | DHP_A19_2 | cDNAContig | 176 | 375 |
| 311 | Amaranthus viridis | DHP_A19_3 | cDNAContig | 351 | 550 |
| 312 | Amaranthus viridis | DHP_A19_3 | cDNAContig | 351 | 550 |
| 313 | Amaranthus viridis | DHP_A18_1 | cDNAContig | 1 | 200 |
| 314 | Amaranthus viridis | DHP_A18_1 | cDNAContig | 1 | 200 |
| 315 | Amaranthus viridis | DHP_A18_2 | cDNAContig | 176 | 375 |
| 316 | Amaranthus viridis | DHP_A18_2 | cDNAContig | 176 | 375 |
| 317 | Amaranthus viridis | DHP_A18_3 | cDNAContig | 351 | 550 |
| 318 | Amaranthus viridis | DHP_A18_3 | cDNAContig | 351 | 550 |
| 319 | Amaranthus viridis | DHP_A18_4 | cDNAContig | 526 | 725 |
| 320 | Amaranthus viridis | DHP_A18_4 | cDNAContig | 526 | 725 |
| 321 | Amaranthus viridis | DHP_A18_5 | cDNAContig | 701 | 900 |
| 322 | Amaranthus viridis | DHP_A18_5 | cDNAContig | 701 | 900 |
| 323 | Amaranthus viridis | DHP_A18_6 | cDNAContig | 876 | 1075 |
| 324 | Amaranthus viridis | DHP_A18_6 | cDNAContig | 876 | 1075 |
| 325 | Amaranthus viridis | DHP_A18_7 | cDNAContig | 1051 | 1250 |
| 326 | Amaranthus viridis | DHP_A18_7 | cDNAContig | 1051 | 1250 |
| 327 | Amaranthus viridis | DHP_A18_8 | cDNAContig | 1226 | 1425 |
| 328 | Amaranthus viridis | DHP_A18_8 | cDNAContig | 1226 | 1425 |
| 329 | Ambrosia trifida | DHP_A21_1 | gDNAContig | 1 | 200 |
| 330 | Ambrosia trifida | DHP_A21_1 | gDNAContig | 1 | 200 |
| 331 | Ambrosia trifida | DHP_A21_2 | gDNAContig | 176 | 375 |
| 332 | Ambrosia trifida | DHP_A21_2 | gDNAContig | 176 | 375 |
| 333 | Ambrosia trifida | DHP_A21_3 | gDNAContig | 351 | 550 |
| 334 | Ambrosia trifida | DHP_A21_3 | gDNAContig | 351 | 550 |
| 335 | Ambrosia trifida | DHP_A21_4 | gDNAContig | 526 | 725 |
| 336 | Ambrosia trifida | DHP_A21_4 | gDNAContig | 526 | 725 |
| 337 | Ambrosia trifida | DHP_A21_5 | gDNAContig | 701 | 900 |
| 338 | Ambrosia trifida | DHP_A21_5 | gDNAContig | 701 | 900 |
| 339 | Ambrosia trifida | DHP_A21_6 | gDNAContig | 876 | 1075 |
| 340 | Ambrosia trifida | DHP_A21_6 | gDNAContig | 876 | 1075 |
| 341 | Ambrosia trifida | DHP_A21_7 | gDNAContig | 1051 | 1250 |
| 342 | Ambrosia trifida | DHP_A21_7 | gDNAContig | 1051 | 1250 |
| 343 | Ambrosia trifida | DHP_A28_1 | gDNAContig | 1 | 200 |
| 344 | Ambrosia trifida | DHP_A28_1 | gDNAContig | 1 | 200 |
| 345 | Ambrosia trifida | DHP_A26_1 | gDNAContig | 1 | 200 |
| 346 | Ambrosia trifida | DHP_A26_1 | gDNAContig | 1 | 200 |
| 347 | Ambrosia trifida | DHP_A24_1 | cDNAContig | 1 | 200 |
| 348 | Ambrosia trifida | DHP_A24_1 | cDNAContig | 1 | 200 |
| 349 | Ambrosia trifida | DHP_A24_2 | cDNAContig | 176 | 375 |
| 350 | Ambrosia trifida | DHP_A24_2 | cDNAContig | 176 | 375 |
| 351 | Ambrosia trifida | DHP_A27_1 | gDNAContig | 1 | 200 |
| 352 | Ambrosia trifida | DHP_A27_1 | gDNAContig | 1 | 200 |
| 353 | Ambrosia trifida | DHP_A23_1 | cDNAContig | 1 | 200 |
| 354 | Ambrosia trifida | DHP_A23_1 | cDNAContig | 1 | 200 |
| 355 | Ambrosia trifida | DHP_A23_2 | cDNAContig | 176 | 375 |
| 356 | Ambrosia trifida | DHP_A23_2 | cDNAContig | 176 | 375 |
| 357 | Ambrosia trifida | DHP_A29_1 | cDNAContig | 1 | 101 |
| 358 | Ambrosia trifida | DHP_A29_1 | cDNAContig | 1 | 101 |
| 359 | Ambrosia trifida | DHP_A20_1 | cDNAContig | 1 | 200 |
| 360 | Ambrosia trifida | DHP_A20_1 | cDNAContig | 1 | 200 |
| 361 | Ambrosia trifida | DHP_A20_2 | cDNAContig | 176 | 375 |
| 362 | Ambrosia trifida | DHP_A20_2 | cDNAContig | 176 | 375 |
| 363 | Ambrosia trifida | DHP_A20_3 | cDNAContig | 351 | 550 |
| 364 | Ambrosia trifida | DHP_A20_3 | cDNAContig | 351 | 550 |
| 365 | Ambrosia trifida | DHP_A20_4 | cDNAContig | 526 | 725 |
| 366 | Ambrosia trifida | DHP_A20_4 | cDNAContig | 526 | 725 |
| 367 | Ambrosia trifida | DHP_A20_5 | cDNAContig | 701 | 900 |
| 368 | Ambrosia trifida | DHP_A20_5 | cDNAContig | 701 | 900 |
| 369 | Ambrosia trifida | DHP_A20_6 | cDNAContig | 876 | 1075 |
| 370 | Ambrosia trifida | DHP_A20_6 | cDNAContig | 876 | 1075 |
| 371 | Ambrosia trifida | DHP_A20_5 | cDNAContig | 1051 | 1250 |
| 372 | Ambrosia trifida | DHP_A20_5 | cDNAContig | 1051 | 1250 |
| 373 | Ambrosia trifida | DHP_A25_1 | gDNAContig | 1 | 200 |
| 374 | Ambrosia trifida | DHP_A25_1 | gDNAContig | 1 | 200 |
| 375 | Ambrosia trifida | DHP_A25_2 | gDNAContig | 176 | 375 |
| 376 | Ambrosia trifida | DHP_A25_2 | gDNAContig | 176 | 375 |
| 377 | Conyza canadensis | DHP_A33_1 | cDNAContig | 1 | 200 |
| 378 | Conyza canadensis | DHP_A33_1 | cDNAContig | 1 | 200 |
| 379 | Conyza canadensis | DHP_A33_2 | cDNAContig | 176 | 375 |
| 380 | Conyza canadensis | DHP_A33_2 | cDNAContig | 176 | 375 |
| 381 | Conyza canadensis | DHP_A33_3 | cDNAContig | 351 | 550 |
| 382 | Conyza canadensis | DHP_A33_3 | cDNAContig | 351 | 550 |
| 383 | Conyza canadensis | DHP_A33_4 | cDNAContig | 526 | 725 |
| 384 | Conyza canadensis | DHP_A33_4 | cDNAContig | 526 | 725 |
| 385 | Conyza canadensis | DHP_A33_5 | cDNAContig | 701 | 900 |
| 386 | Conyza canadensis | DHP_A33_5 | cDNAContig | 701 | 900 |
| 387 | Conyza canadensis | DHP_A33_6 | cDNAContig | 876 | 1075 |
| 388 | Conyza canadensis | DHP_A33_6 | cDNAContig | 876 | 1075 |
| 389 | Conyza canadensis | DHP_A33_7 | cDNAContig | 1051 | 1250 |
| 390 | Conyza canadensis | DHP_A33_7 | cDNAContig | 1051 | 1250 |
| 391 | Conyza canadensis | DHP_A32_1 | cDNAContig | 1 | 200 |
| 392 | Conyza canadensis | DHP_A32_1 | cDNAContig | 1 | 200 |
| 393 | Conyza canadensis | DHP_A32_2 | cDNAContig | 176 | 375 |
| 394 | Conyza canadensis | DHP_A32_2 | cDNAContig | 176 | 375 |
| 395 | Conyza canadensis | DHP_A32_3 | cDNAContig | 351 | 550 |
| 396 | Conyza canadensis | DHP_A32_3 | cDNAContig | 351 | 550 |
| 397 | Conyza canadensis | DHP_A32_4 | cDNAContig | 526 | 725 |
| 398 | Conyza canadensis | DHP_A32_4 | cDNAContig | 526 | 725 |
| 399 | Conyza canadensis | DHP_A32_5 | cDNAContig | 701 | 900 |
| 400 | Conyza canadensis | DHP_A32_5 | cDNAContig | 701 | 900 |
| 401 | Conyza canadensis | DHP_A32_6 | cDNAContig | 876 | 1075 |
| 402 | Conyza canadensis | DHP_A32_6 | cDNAContig | 876 | 1075 |
| 403 | Conyza canadensis | DHP_A32_7 | cDNAContig | 1051 | 1250 |
| 404 | Conyza canadensis | DHP_A32_7 | cDNAContig | 1051 | 1250 |
| 405 | Conyza canadensis | DHP_A30_1 | gDNAContig | 1 | 200 |
| 406 | Conyza canadensis | DHP_A30_1 | gDNAContig | 1 | 200 |
| 407 | Conyza canadensis | DHP_A30_2 | gDNAContig | 176 | 375 |
| 408 | Conyza canadensis | DHP_A30_2 | gDNAContig | 176 | 375 |
| 409 | Conyza canadensis | DHP_A30_3 | gDNAContig | 351 | 550 |
| 410 | Conyza canadensis | DHP_A30_3 | gDNAContig | 351 | 550 |
| 411 | Conyza canadensis | DHP_A30_4 | gDNAContig | 526 | 725 |
| 412 | Conyza canadensis | DHP_A30_4 | gDNAContig | 526 | 725 |
| 413 | Conyza canadensis | DHP_A30_5 | gDNAContig | 701 | 900 |
| 414 | Conyza canadensis | DHP_A30_5 | gDNAContig | 701 | 900 |
| 415 | Conyza canadensis | DHP_A30_6 | gDNAContig | 876 | 1075 |
| 416 | Conyza canadensis | DHP_A30_6 | gDNAContig | 876 | 1075 |
| 417 | Conyza canadensis | DHP_A30_7 | gDNAContig | 1051 | 1250 |
| 418 | Conyza canadensis | DHP_A30_7 | gDNAContig | 1051 | 1250 |
| 419 | Conyza canadensis | DHP_A30_8 | gDNAContig | 1226 | 1425 |
| 420 | Conyza canadensis | DHP_A30_8 | gDNAContig | 1226 | 1425 |
| 421 | Conyza canadensis | DHP_A30_9 | gDNAContig | 1401 | 1600 |
| 422 | Conyza canadensis | DHP_A30_9 | gDNAContig | 1401 | 1600 |
| 423 | Conyza canadensis | DHP_A30_10 | gDNAContig | 1576 | 1775 |
| 424 | Conyza canadensis | DHP_A30_10 | gDNAContig | 1576 | 1775 |
| 425 | Conyza canadensis | DHP_A30_11 | gDNAContig | 1751 | 1950 |
| 426 | Conyza canadensis | DHP_A30_11 | gDNAContig | 1751 | 1950 |
| 427 | Conyza canadensis | DHP_A30_12 | gDNAContig | 1926 | 2125 |
| 428 | Conyza canadensis | DHP_A30_12 | gDNAContig | 1926 | 2125 |
| 429 | Conyza canadensis | DHP_A30_13 | gDNAContig | 2101 | 2300 |
| 430 | Conyza canadensis | DHP_A30_13 | gDNAContig | 2101 | 2300 |
| 431 | Conyza canadensis | DHP_A30_14 | gDNAContig | 2276 | 2475 |
| 432 | Conyza canadensis | DHP_A30_14 | gDNAContig | 2276 | 2475 |
| 433 | Conyza canadensis | DHP_A30_15 | gDNAContig | 2451 | 2650 |
| 434 | Conyza canadensis | DHP_A30_15 | gDNAContig | 2451 | 2650 |
| 435 | Conyza canadensis | DHP_A30_16 | gDNAContig | 2626 | 2825 |
| 436 | Conyza canadensis | DHP_A30_16 | gDNAContig | 2626 | 2825 |
| 437 | Conyza canadensis | DHP_A30_17 | gDNAContig | 2801 | 3000 |
| 438 | Conyza canadensis | DHP_A30_17 | gDNAContig | 2801 | 3000 |
| 439 | Conyza canadensis | DHP_A30_18 | gDNAContig | 2976 | 3175 |
| 440 | Conyza canadensis | DHP_A30_18 | gDNAContig | 2976 | 3175 |
| 441 | Conyza canadensis | DHP_A30_19 | gDNAContig | 3151 | 3350 |
| 442 | Conyza canadensis | DHP_A30_19 | gDNAContig | 3151 | 3350 |
| 443 | Conyza canadensis | DHP_A30_20 | gDNAContig | 3326 | 3525 |
| 444 | Conyza canadensis | DHP_A30_20 | gDNAContig | 3326 | 3525 |
| 445 | Conyza canadensis | DHP_A30_21 | gDNAContig | 3501 | 3700 |
| 446 | Conyza canadensis | DHP_A30_21 | gDNAContig | 3501 | 3700 |
| 447 | Conyza canadensis | DHP_A30_22 | gDNAContig | 3676 | 3875 |
| 448 | Conyza canadensis | DHP_A30_22 | gDNAContig | 3676 | 3875 |
| 449 | Conyza canadensis | DHP_A30_23 | gDNAContig | 3851 | 4050 |
| 450 | Conyza canadensis | DHP_A30_23 | gDNAContig | 3851 | 4050 |
| 451 | Conyza canadensis | DHP_A30_24 | gDNAContig | 4026 | 4225 |
| 452 | Conyza canadensis | DHP_A30_24 | gDNAContig | 4026 | 4225 |
| 453 | Conyza canadensis | DHP_A30_25 | gDNAContig | 4201 | 4400 |
| 454 | Conyza canadensis | DHP_A30_25 | gDNAContig | 4201 | 4400 |
| 455 | Conyza canadensis | DHP_A30_26 | gDNAContig | 4376 | 4575 |
| 456 | Conyza canadensis | DHP_A30_26 | gDNAContig | 4376 | 4575 |
| 457 | Conyza canadensis | DHP_A30_27 | gDNAContig | 4551 | 4750 |
| 458 | Conyza canadensis | DHP_A30_27 | gDNAContig | 4551 | 4750 |
| 459 | Conyza canadensis | DHP_A30_28 | gDNAContig | 4726 | 4925 |
| 460 | Conyza canadensis | DHP_A30_28 | gDNAContig | 4726 | 4925 |
| 461 | Conyza canadensis | DHP_A30_29 | gDNAContig | 4901 | 5100 |
| 462 | Conyza canadensis | DHP_A30_29 | gDNAContig | 4901 | 5100 |
| 463 | Conyza canadensis | DHP_A30_30 | gDNAContig | 5076 | 5275 |
| 464 | Conyza canadensis | DHP_A30_30 | gDNAContig | 5076 | 5275 |
| 465 | Conyza canadensis | DHP_A31_1 | gDNAContig | 1 | 200 |
| 466 | Conyza canadensis | DHP_A31_1 | gDNAContig | 1 | 200 |
| 467 | Conyza canadensis | DHP_A31_2 | gDNAContig | 176 | 375 |
| 468 | Conyza canadensis | DHP_A31_2 | gDNAContig | 176 | 375 |
| 469 | Conyza canadensis | DHP_A31_3 | gDNAContig | 351 | 550 |
| 470 | Conyza canadensis | DHP_A31_3 | gDNAContig | 351 | 550 |
| 471 | Conyza canadensis | DHP_A31_4 | gDNAContig | 526 | 725 |
| 472 | Conyza canadensis | DHP_A31_4 | gDNAContig | 526 | 725 |
| 473 | Conyza canadensis | DHP_A31_5 | gDNAContig | 701 | 900 |
| 474 | Conyza canadensis | DHP_A31_5 | gDNAContig | 701 | 900 |
| 475 | Conyza canadensis | DHP_A31_6 | gDNAContig | 876 | 1075 |
| 476 | Conyza canadensis | DHP_A31_6 | gDNAContig | 876 | 1075 |
| 477 | Conyza canadensis | DHP_A31_7 | gDNAContig | 1051 | 1250 |
| 478 | Conyza canadensis | DHP_A31_7 | gDNAContig | 1051 | 1250 |
| 479 | Conyza canadensis | DHP_A31_8 | gDNAContig | 1226 | 1425 |
| 480 | Conyza canadensis | DHP_A31_8 | gDNAContig | 1226 | 1425 |
| 481 | Conyza canadensis | DHP_A31_9 | gDNAContig | 1401 | 1600 |
| 482 | Conyza canadensis | DHP_A31_9 | gDNAContig | 1401 | 1600 |
| 483 | Conyza canadensis | DHP_A31_10 | gDNAContig | 1576 | 1775 |
| 484 | Conyza canadensis | DHP_A31_10 | gDNAContig | 1576 | 1775 |
| 485 | Conyza canadensis | DHP_A31_11 | gDNAContig | 1751 | 1950 |
| 486 | Conyza canadensis | DHP_A31_11 | gDNAContig | 1751 | 1950 |
| 487 | Conyza canadensis | DHP_A31_12 | gDNAContig | 1926 | 2125 |
| 488 | Conyza canadensis | DHP_A31_12 | gDNAContig | 1926 | 2125 |
| 489 | Conyza canadensis | DHP_A31_13 | gDNAContig | 2101 | 2300 |
| 490 | Conyza canadensis | DHP_A31_13 | gDNAContig | 2101 | 2300 |
| 491 | Conyza canadensis | DHP_A31_14 | gDNAContig | 2276 | 2475 |
| 492 | Conyza canadensis | DHP_A31_14 | gDNAContig | 2276 | 2475 |
| 493 | Conyza canadensis | DHP_A31_15 | gDNAContig | 2451 | 2650 |
| 494 | Conyza canadensis | DHP_A31_15 | gDNAContig | 2451 | 2650 |
| 495 | Conyza canadensis | DHP_A31_16 | gDNAContig | 2626 | 2825 |
| 496 | Conyza canadensis | DHP_A31_16 | gDNAContig | 2626 | 2825 |
| 497 | Conyza canadensis | DHP_A31_17 | gDNAContig | 2801 | 3000 |
| 498 | Conyza canadensis | DHP_A31_17 | gDNAContig | 2801 | 3000 |
| 499 | Conyza canadensis | DHP_A31_18 | gDNAContig | 2976 | 3175 |
| 500 | Conyza canadensis | DHP_A31_18 | gDNAContig | 2976 | 3175 |
| 501 | Conyza canadensis | DHP_A31_19 | gDNAContig | 3151 | 3350 |
| 502 | Conyza canadensis | DHP_A31_19 | gDNAContig | 3151 | 3350 |
| 503 | Conyza canadensis | DHP_A31_20 | gDNAContig | 3326 | 3525 |
| 504 | Conyza canadensis | DHP_A31_20 | gDNAContig | 3326 | 3525 |
| 505 | Conyza canadensis | DHP_A31_21 | gDNAContig | 3501 | 3700 |
| 506 | Conyza canadensis | DHP_A31_21 | gDNAContig | 3501 | 3700 |
| 507 | Conyza canadensis | DHP_A31_22 | gDNAContig | 3676 | 3875 |
| 508 | Conyza canadensis | DHP_A31_22 | gDNAContig | 3676 | 3875 |
| 509 | Conyza canadensis | DHP_A31_23 | gDNAContig | 3851 | 4050 |
| 510 | Conyza canadensis | DHP_A31_23 | gDNAContig | 3851 | 4050 |
| 511 | Conyza canadensis | DHP_A31_24 | gDNAContig | 4026 | 4225 |
| 512 | Conyza canadensis | DHP_A31_24 | gDNAContig | 4026 | 4225 |
| 513 | Conyza canadensis | DHP_A31_25 | gDNAContig | 4201 | 4400 |
| 514 | Conyza canadensis | DHP_A31_25 | gDNAContig | 4201 | 4400 |
| 515 | Conyza canadensis | DHP_A31_26 | gDNAContig | 4376 | 4575 |
| 516 | Conyza canadensis | DHP_A31_26 | gDNAContig | 4376 | 4575 |
| 517 | Conyza canadensis | DHP_A31_27 | gDNAContig | 4551 | 4750 |
| 518 | Conyza canadensis | DHP_A31_27 | gDNAContig | 4551 | 4750 |
| 519 | Conyza canadensis | DHP_A31_28 | gDNAContig | 4726 | 4925 |
| 520 | Conyza canadensis | DHP_A31_28 | gDNAContig | 4726 | 4925 |
| 521 | Conyza canadensis | DHP_A31_29 | gDNAContig | 4901 | 5100 |
| 522 | Conyza canadensis | DHP_A31_29 | gDNAContig | 4901 | 5100 |
| 523 | Conyza canadensis | DHP_A31_30 | gDNAContig | 5076 | 5275 |
| 524 | Conyza canadensis | DHP_A31_30 | gDNAContig | 5076 | 5275 |
| 525 | Digitaria sanguinalis | DHP_A41_1 | gDNAContig | 1 | 200 |
| 526 | Digitaria sanguinalis | DHP_A41_1 | gDNAContig | 1 | 200 |
| 527 | Digitaria sanguinalis | DHP_A41_2 | gDNAContig | 176 | 375 |
| 528 | Digitaria sanguinalis | DHP_A41_2 | gDNAContig | 176 | 375 |
| 529 | Digitaria sanguinalis | DHP_A41_3 | gDNAContig | 351 | 550 |
| 530 | Digitaria sanguinalis | DHP_A41_3 | gDNAContig | 351 | 550 |
| 531 | Digitaria sanguinalis | DHP_A41_4 | gDNAContig | 526 | 725 |
| 532 | Digitaria sanguinalis | DHP_A41_4 | gDNAContig | 526 | 725 |
| 533 | Digitaria sanguinalis | DHP_A41_5 | gDNAContig | 701 | 900 |
| 534 | Digitaria sanguinalis | DHP_A41_5 | gDNAContig | 701 | 900 |
| 535 | Digitaria sanguinalis | DHP_A41_6 | gDNAContig | 876 | 1075 |
| 536 | Digitaria sanguinalis | DHP_A41_6 | gDNAContig | 876 | 1075 |
| 537 | Digitaria sanguinalis | DHP_A41_7 | gDNAContig | 1051 | 1250 |
| 538 | Digitaria sanguinalis | DHP_A41_7 | gDNAContig | 1051 | 1250 |
| 539 | Digitaria sanguinalis | DHP_A41_8 | gDNAContig | 1226 | 1425 |
| 540 | Digitaria sanguinalis | DHP_A41_8 | gDNAContig | 1226 | 1425 |
| 541 | Digitaria sanguinalis | DHP_A41_9 | gDNAContig | 1401 | 1600 |
| 542 | Digitaria sanguinalis | DHP_A41_9 | gDNAContig | 1401 | 1600 |
| 543 | Digitaria sanguinalis | DHP_A41_10 | gDNAContig | 1576 | 1775 |
| 544 | Digitaria sanguinalis | DHP_A41_10 | gDNAContig | 1576 | 1775 |
| 545 | Digitaria sanguinalis | DHP_A41_11 | gDNAContig | 1751 | 1950 |
| 546 | Digitaria sanguinalis | DHP_A41_11 | gDNAContig | 1751 | 1950 |
| 547 | Digitaria sanguinalis | DHP_A41_12 | gDNAContig | 1926 | 2125 |
| 548 | Digitaria sanguinalis | DHP_A41_12 | gDNAContig | 1926 | 2125 |
| 549 | Digitaria sanguinalis | DHP_A41_13 | gDNAContig | 2101 | 2300 |
| 550 | Digitaria sanguinalis | DHP_A41_13 | gDNAContig | 2101 | 2300 |
| 551 | Digitaria sanguinalis | DHP_A41_14 | gDNAContig | 2276 | 2475 |
| 552 | Digitaria sanguinalis | DHP_A41_14 | gDNAContig | 2276 | 2475 |
| 553 | Digitaria sanguinalis | DHP_A41_15 | gDNAContig | 2451 | 2650 |
| 554 | Digitaria sanguinalis | DHP_A41_15 | gDNAContig | 2451 | 2650 |
| 555 | Digitaria sanguinalis | DHP_A41_16 | gDNAContig | 2626 | 2825 |
| 556 | Digitaria sanguinalis | DHP_A41_16 | gDNAContig | 2626 | 2825 |
| 557 | Digitaria sanguinalis | DHP_A41_17 | gDNAContig | 2801 | 3000 |
| 558 | Digitaria sanguinalis | DHP_A41_17 | gDNAContig | 2801 | 3000 |
| 559 | Digitaria sanguinalis | DHP_A41_18 | gDNAContig | 2976 | 3175 |
| 560 | Digitaria sanguinalis | DHP_A41_18 | gDNAContig | 2976 | 3175 |
| 561 | Digitaria sanguinalis | DHP_A41_19 | gDNAContig | 3151 | 3350 |
| 562 | Digitaria sanguinalis | DHP_A41_19 | gDNAContig | 3151 | 3350 |
| 563 | Digitaria sanguinalis | DHP_A41_20 | gDNAContig | 3326 | 3525 |
| 564 | Digitaria sanguinalis | DHP_A41_20 | gDNAContig | 3326 | 3525 |
| 565 | Digitaria sanguinalis | DHP_A41_21 | gDNAContig | 3501 | 3700 |
| 566 | Digitaria sanguinalis | DHP_A41_21 | gDNAContig | 3501 | 3700 |
| 567 | Digitaria sanguinalis | DHP_A41_22 | gDNAContig | 3676 | 3875 |
| 568 | Digitaria sanguinalis | DHP_A41_22 | gDNAContig | 3676 | 3875 |
| 569 | Digitaria sanguinalis | DHP_A42_1 | cDNAContig | 1 | 200 |
| 570 | Digitaria sanguinalis | DHP_A42_1 | cDNAContig | 1 | 200 |
| 571 | Digitaria sanguinalis | DHP_A42_2 | cDNAContig | 176 | 375 |
| 572 | Digitaria sanguinalis | DHP_A42_2 | cDNAContig | 176 | 375 |
| 573 | Digitaria sanguinalis | DHP_A42_3 | cDNAContig | 351 | 550 |
| 574 | Digitaria sanguinalis | DHP_A42_3 | cDNAContig | 351 | 550 |
| 575 | Digitaria sanguinalis | DHP_A42_4 | cDNAContig | 526 | 725 |
| 576 | Digitaria sanguinalis | DHP_A42_4 | cDNAContig | 526 | 725 |
| 577 | Digitaria sanguinalis | DHP_A42_5 | cDNAContig | 701 | 900 |
| 578 | Digitaria sanguinalis | DHP_A42_5 | cDNAContig | 701 | 900 |
| 579 | Digitaria sanguinalis | DHP_A42_6 | cDNAContig | 876 | 1075 |
| 580 | Digitaria sanguinalis | DHP_A42_6 | cDNAContig | 876 | 1075 |
| 581 | Digitaria sanguinalis | DHP_A42_7 | cDNAContig | 1051 | 1250 |
| 582 | Digitaria sanguinalis | DHP_A42_7 | cDNAContig | 1051 | 1250 |
| 583 | Digitaria sanguinalis | DHP_A42_8 | cDNAContig | 1226 | 1425 |
| 584 | Digitaria sanguinalis | DHP_A42_8 | cDNAContig | 1226 | 1425 |
| 585 | Euphorbia heterophylla | DHP_A35_1 | gDNAContig | 1 | 200 |
| 586 | Euphorbia heterophylla | DHP_A35_1 | gDNAContig | 1 | 200 |
| 587 | Euphorbia heterophylla | DHP_A35_2 | gDNAContig | 176 | 375 |
| 588 | Euphorbia heterophylla | DHP_A35_2 | gDNAContig | 176 | 375 |
| 589 | Euphorbia heterophylla | DHP_A35_3 | gDNAContig | 351 | 550 |
| 590 | Euphorbia heterophylla | DHP_A35_3 | gDNAContig | 351 | 550 |
| 591 | Euphorbia heterophylla | DHP_A35_4 | gDNAContig | 526 | 725 |
| 592 | Euphorbia heterophylla | DHP_A35_4 | gDNAContig | 526 | 725 |
| 593 | Euphorbia heterophylla | DHP_A35_5 | gDNAContig | 701 | 900 |
| 594 | Euphorbia heterophylla | DHP_A35_5 | gDNAContig | 701 | 900 |
| 595 | Euphorbia heterophylla | DHP_A35_6 | gDNAContig | 876 | 1075 |
| 596 | Euphorbia heterophylla | DHP_A35_6 | gDNAContig | 876 | 1075 |
| 597 | Euphorbia heterophylla | DHP_A35_7 | gDNAContig | 1051 | 1250 |
| 598 | Euphorbia heterophylla | DHP_A35_7 | gDNAContig | 1051 | 1250 |
| 599 | Euphorbia heterophylla | DHP_A35_8 | gDNAContig | 1226 | 1425 |
| 600 | Euphorbia heterophylla | DHP_A35_8 | gDNAContig | 1226 | 1425 |
| 601 | Euphorbia heterophylla | DHP_A35_9 | gDNAContig | 1401 | 1600 |
| 602 | Euphorbia heterophylla | DHP_A35_9 | gDNAContig | 1401 | 1600 |
| 603 | Euphorbia heterophylla | DHP_A35_10 | gDNAContig | 1576 | 1775 |
| 604 | Euphorbia heterophylla | DHP_A35_10 | gDNAContig | 1576 | 1775 |
| 605 | Euphorbia heterophylla | DHP_A35_11 | gDNAContig | 1751 | 1950 |
| 606 | Euphorbia heterophylla | DHP_A35_11 | gDNAContig | 1751 | 1950 |
| 607 | Euphorbia heterophylla | DHP_A35_12 | gDNAContig | 1926 | 2125 |
| 608 | Euphorbia heterophylla | DHP_A35_12 | gDNAContig | 1926 | 2125 |
| 609 | Euphorbia heterophylla | DHP_A35_13 | gDNAContig | 2101 | 2300 |
| 610 | Euphorbia heterophylla | DHP_A35_13 | gDNAContig | 2101 | 2300 |
| 611 | Euphorbia heterophylla | DHP_A35_14 | gDNAContig | 2276 | 2475 |
| 612 | Euphorbia heterophylla | DHP_A35_14 | gDNAContig | 2276 | 2475 |
| 613 | Euphorbia heterophylla | DHP_A35_15 | gDNAContig | 2451 | 2650 |
| 614 | Euphorbia heterophylla | DHP_A35_15 | gDNAContig | 2451 | 2650 |
| 615 | Euphorbia heterophylla | DHP_A35_16 | gDNAContig | 2626 | 2825 |
| 616 | Euphorbia heterophylla | DHP_A35_16 | gDNAContig | 2626 | 2825 |
| 617 | Euphorbia heterophylla | DHP_A35_17 | gDNAContig | 2801 | 3000 |
| 618 | Euphorbia heterophylla | DHP_A35_17 | gDNAContig | 2801 | 3000 |
| 619 | Euphorbia heterophylla | DHP_A35_18 | gDNAContig | 2976 | 3175 |
| 620 | Euphorbia heterophylla | DHP_A35_18 | gDNAContig | 2976 | 3175 |
| 621 | Euphorbia heterophylla | DHP_A35_19 | gDNAContig | 3151 | 3350 |
| 622 | Euphorbia heterophylla | DHP_A35_19 | gDNAContig | 3151 | 3350 |
| 623 | Euphorbia heterophylla | DHP_A35_20 | gDNAContig | 3326 | 3525 |
| 624 | Euphorbia heterophylla | DHP_A35_20 | gDNAContig | 3326 | 3525 |
| 625 | Euphorbia heterophylla | DHP_A35_21 | gDNAContig | 3501 | 3700 |
| 626 | Euphorbia heterophylla | DHP_A35_21 | gDNAContig | 3501 | 3700 |
| 627 | Euphorbia heterophylla | DHP_A35_22 | gDNAContig | 3676 | 3875 |
| 628 | Euphorbia heterophylla | DHP_A35_22 | gDNAContig | 3676 | 3875 |
| 629 | Euphorbia heterophylla | DHP_A35_23 | gDNAContig | 3851 | 4050 |
| 630 | Euphorbia heterophylla | DHP_A35_23 | gDNAContig | 3851 | 4050 |
| 631 | Euphorbia heterophylla | DHP_A35_24 | gDNAContig | 4026 | 4225 |
| 632 | Euphorbia heterophylla | DHP_A35_24 | gDNAContig | 4026 | 4225 |
| 633 | Euphorbia heterophylla | DHP_A35_25 | gDNAContig | 4201 | 4400 |
| 634 | Euphorbia heterophylla | DHP_A35_25 | gDNAContig | 4201 | 4400 |
| 635 | Euphorbia heterophylla | DHP_A37_1 | cDNAContig | 1 | 200 |
| 636 | Euphorbia heterophylla | DHP_A37_1 | cDNAContig | 1 | 200 |
| 637 | Euphorbia heterophylla | DHP_A37_2 | cDNAContig | 176 | 375 |
| 638 | Euphorbia heterophylla | DHP_A37_2 | cDNAContig | 176 | 375 |
| 639 | Euphorbia heterophylla | DHP_A37_3 | cDNAContig | 351 | 550 |
| 640 | Euphorbia heterophylla | DHP_A37_3 | cDNAContig | 351 | 550 |
| 641 | Euphorbia heterophylla | DHP_A37_4 | cDNAContig | 526 | 725 |
| 642 | Euphorbia heterophylla | DHP_A37_4 | cDNAContig | 526 | 725 |
| 643 | Euphorbia heterophylla | DHP_A37_5 | cDNAContig | 701 | 900 |
| 644 | Euphorbia heterophylla | DHP_A37_5 | cDNAContig | 701 | 900 |
| 645 | Euphorbia heterophylla | DHP_A37_6 | cDNAContig | 876 | 1075 |
| 646 | Euphorbia heterophylla | DHP_A37_6 | cDNAContig | 876 | 1075 |
| 647 | Euphorbia heterophylla | DHP_A37_7 | cDNAContig | 1051 | 1250 |
| 648 | Euphorbia heterophylla | DHP_A37_7 | cDNAContig | 1051 | 1250 |
| 649 | Euphorbia heterophylla | DHP_A37_8 | cDNAContig | 1226 | 1425 |
| 650 | Euphorbia heterophylla | DHP_A37_8 | cDNAContig | 1226 | 1425 |
| 651 | Euphorbia heterophylla | DHP_A36_1 | cDNAContig | 1 | 200 |
| 652 | Euphorbia heterophylla | DHP_A36_1 | cDNAContig | 1 | 200 |
| 653 | Euphorbia heterophylla | DHP_A36_2 | cDNAContig | 176 | 375 |
| 654 | Euphorbia heterophylla | DHP_A36_2 | cDNAContig | 176 | 375 |
| 655 | Euphorbia heterophylla | DHP_A36_3 | cDNAContig | 351 | 550 |
| 656 | Euphorbia heterophylla | DHP_A36_3 | cDNAContig | 351 | 550 |
| 657 | Euphorbia heterophylla | DHP_A36_4 | cDNAContig | 526 | 725 |
| 658 | Euphorbia heterophylla | DHP_A36_4 | cDNAContig | 526 | 725 |
| 659 | Euphorbia heterophylla | DHP_A36_5 | cDNAContig | 701 | 900 |
| 660 | Euphorbia heterophylla | DHP_A36_5 | cDNAContig | 701 | 900 |
| 661 | Euphorbia heterophylla | DHP_A36_6 | cDNAContig | 876 | 1075 |
| 662 | Euphorbia heterophylla | DHP_A36_6 | cDNAContig | 876 | 1075 |
| 663 | Euphorbia heterophylla | DHP_A36_7 | cDNAContig | 1051 | 1250 |
| 664 | Euphorbia heterophylla | DHP_A36_7 | cDNAContig | 1051 | 1250 |
| 665 | Euphorbia heterophylla | DHP_A36_8 | cDNAContig | 1226 | 1425 |
| 666 | Euphorbia heterophylla | DHP_A36_8 | cDNAContig | 1226 | 1425 |
| 667 | Euphorbia heterophylla | DHP_A40_1 | gDNAContig | 1 | 200 |
| 668 | Euphorbia heterophylla | DHP_A40_1 | gDNAContig | 1 | 200 |
| 669 | Euphorbia heterophylla | DHP_A40_2 | gDNAContig | 176 | 375 |
| 670 | Euphorbia heterophylla | DHP_A40_2 | gDNAContig | 176 | 375 |
| 671 | Euphorbia heterophylla | DHP_A39_1 | gDNAContig | 1 | 200 |
| 672 | Euphorbia heterophylla | DHP_A39_1 | gDNAContig | 1 | 200 |
| 673 | Euphorbia heterophylla | DHP_A39_2 | gDNAContig | 176 | 375 |
| 674 | Euphorbia heterophylla | DHP_A39_2 | gDNAContig | 176 | 375 |
| 675 | Euphorbia heterophylla | DHP_A38_1 | cDNAContig | 1 | 200 |
| 676 | Euphorbia heterophylla | DHP_A38_1 | cDNAContig | 1 | 200 |
| 677 | Euphorbia heterophylla | DHP_A38_2 | cDNAContig | 176 | 375 |
| 678 | Euphorbia heterophylla | DHP_A38_2 | cDNAContig | 176 | 375 |
| 679 | Euphorbia heterophylla | DHP_A38_3 | cDNAContig | 351 | 550 |
| 680 | Euphorbia heterophylla | DHP_A38_3 | cDNAContig | 351 | 550 |
| 681 | Euphorbia heterophylla | DHP_A34_1 | gDNAContig | 1 | 200 |
| 682 | Euphorbia heterophylla | DHP_A34_1 | gDNAContig | 1 | 200 |
| 683 | Euphorbia heterophylla | DHP_A34_2 | gDNAContig | 176 | 375 |
| 684 | Euphorbia heterophylla | DHP_A34_2 | gDNAContig | 176 | 375 |
| 685 | Euphorbia heterophylla | DHP_A34_3 | gDNAContig | 351 | 550 |
| 686 | Euphorbia heterophylla | DHP_A34_3 | gDNAContig | 351 | 550 |
| 687 | Euphorbia heterophylla | DHP_A34_4 | gDNAContig | 526 | 725 |
| 688 | Euphorbia heterophylla | DHP_A34_4 | gDNAContig | 526 | 725 |
| 689 | Euphorbia heterophylla | DHP_A34_5 | gDNAContig | 701 | 900 |
| 690 | Euphorbia heterophylla | DHP_A34_5 | gDNAContig | 701 | 900 |
| 691 | Euphorbia heterophylla | DHP_A34_6 | gDNAContig | 876 | 1075 |
| 692 | Euphorbia heterophylla | DHP_A34_6 | gDNAContig | 876 | 1075 |
| 693 | Euphorbia heterophylla | DHP_A34_7 | gDNAContig | 1051 | 1250 |
| 694 | Euphorbia heterophylla | DHP_A34_7 | gDNAContig | 1051 | 1250 |
| 695 | Euphorbia heterophylla | DHP_A34_8 | gDNAContig | 1226 | 1425 |
| 696 | Euphorbia heterophylla | DHP_A34_8 | gDNAContig | 1226 | 1425 |
| 697 | Euphorbia heterophylla | DHP_A34_9 | gDNAContig | 1401 | 1600 |
| 698 | Euphorbia heterophylla | DHP_A34_9 | gDNAContig | 1401 | 1600 |
| 699 | Euphorbia heterophylla | DHP_A34_10 | gDNAContig | 1576 | 1775 |
| 700 | Euphorbia heterophylla | DHP_A34_10 | gDNAContig | 1576 | 1775 |
| 701 | Euphorbia heterophylla | DHP_A34_11 | gDNAContig | 1751 | 1950 |
| 702 | Euphorbia heterophylla | DHP_A34_11 | gDNAContig | 1751 | 1950 |
| 703 | Euphorbia heterophylla | DHP_A34_12 | gDNAContig | 1926 | 2125 |
| 704 | Euphorbia heterophylla | DHP_A34_12 | gDNAContig | 1926 | 2125 |
| 705 | Euphorbia heterophylla | DHP_A34_13 | gDNAContig | 2101 | 2300 |
| 706 | Euphorbia heterophylla | DHP_A34_13 | gDNAContig | 2101 | 2300 |
| 707 | Euphorbia heterophylla | DHP_A34_14 | gDNAContig | 2276 | 2475 |
| 708 | Euphorbia heterophylla | DHP_A34_14 | gDNAContig | 2276 | 2475 |
| 709 | Euphorbia heterophylla | DHP_A34_15 | gDNAContig | 2451 | 2650 |
| 710 | Euphorbia heterophylla | DHP_A34_15 | gDNAContig | 2451 | 2650 |
| 711 | Euphorbia heterophylla | DHP_A34_16 | gDNAContig | 2626 | 2825 |
| 712 | Euphorbia heterophylla | DHP_A34_16 | gDNAContig | 2626 | 2825 |
| 713 | Euphorbia heterophylla | DHP_A34_17 | gDNAContig | 2801 | 3000 |
| 714 | Euphorbia heterophylla | DHP_A34_17 | gDNAContig | 2801 | 3000 |
| 715 | Euphorbia heterophylla | DHP_A34_18 | gDNAContig | 2976 | 3175 |
| 716 | Euphorbia heterophylla | DHP_A34_18 | gDNAContig | 2976 | 3175 |
| 717 | Euphorbia heterophylla | DHP_A34_19 | gDNAContig | 3151 | 3350 |
| 718 | Euphorbia heterophylla | DHP_A34_19 | gDNAContig | 3151 | 3350 |
| 719 | Euphorbia heterophylla | DHP_A34_20 | gDNAContig | 3326 | 3525 |
| 720 | Euphorbia heterophylla | DHP_A34_20 | gDNAContig | 3326 | 3525 |
| 721 | Euphorbia heterophylla | DHP_A34_21 | gDNAContig | 3501 | 3700 |
| 722 | Euphorbia heterophylla | DHP_A34_21 | gDNAContig | 3501 | 3700 |
| 723 | Euphorbia heterophylla | DHP_A34_22 | gDNAContig | 3676 | 3875 |
| 724 | Euphorbia heterophylla | DHP_A34_22 | gDNAContig | 3676 | 3875 |
| 725 | Euphorbia heterophylla | DHP_A34_23 | gDNAContig | 3851 | 4050 |
| 726 | Euphorbia heterophylla | DHP_A34_23 | gDNAContig | 3851 | 4050 |
| 727 | Euphorbia heterophylla | DHP_A34_24 | gDNAContig | 4026 | 4225 |
| 728 | Euphorbia heterophylla | DHP_A34_24 | gDNAContig | 4026 | 4225 |
| 729 | Euphorbia heterophylla | DHP_A34_25 | gDNAContig | 4201 | 4400 |
| 730 | Euphorbia heterophylla | DHP_A34_25 | gDNAContig | 4201 | 4400 |
| 731 | Euphorbia heterophylla | DHP_A34_26 | gDNAContig | 4376 | 4575 |
| 732 | Euphorbia heterophylla | DHP_A34_26 | gDNAContig | 4376 | 4575 |
| 733 | Kochia scoparia | DHP_A43_1 | gDNAContig | 1 | 200 |
| 734 | Kochia scoparia | DHP_A43_1 | gDNAContig | 1 | 200 |
| 735 | Kochia scoparia | DHP_A43_2 | gDNAContig | 176 | 375 |
| 736 | Kochia scoparia | DHP_A43_2 | gDNAContig | 176 | 375 |
| 737 | Kochia scoparia | DHP_A43_3 | gDNAContig | 351 | 550 |
| 738 | Kochia scoparia | DHP_A43_3 | gDNAContig | 351 | 550 |
| 739 | Kochia scoparia | DHP_A43_4 | gDNAContig | 526 | 725 |
| 740 | Kochia scoparia | DHP_A43_4 | gDNAContig | 526 | 725 |
| 741 | Kochia scoparia | DHP_A43_5 | gDNAContig | 701 | 900 |
| 742 | Kochia scoparia | DHP_A43_5 | gDNAContig | 701 | 900 |
| 743 | Kochia scoparia | DHP_A43_6 | gDNAContig | 876 | 1075 |
| 744 | Kochia scoparia | DHP_A43_6 | gDNAContig | 876 | 1075 |
| 745 | Kochia scoparia | DHP_A43_7 | gDNAContig | 1051 | 1250 |
| 746 | Kochia scoparia | DHP_A43_7 | gDNAContig | 1051 | 1250 |
| 747 | Kochia scoparia | DHP_A43_8 | gDNAContig | 1226 | 1425 |
| 748 | Kochia scoparia | DHP_A43_8 | gDNAContig | 1226 | 1425 |
| 749 | Kochia scoparia | DHP_A43_9 | gDNAContig | 1401 | 1600 |
| 750 | Kochia scoparia | DHP_A43_9 | gDNAContig | 1401 | 1600 |
| 751 | Kochia scoparia | DHP_A43_10 | gDNAContig | 1576 | 1775 |
| 752 | Kochia scoparia | DHP_A43_10 | gDNAContig | 1576 | 1775 |
| 753 | Kochia scoparia | DHP_A43_11 | gDNAContig | 1751 | 1950 |
| 754 | Kochia scoparia | DHP_A43_11 | gDNAContig | 1751 | 1950 |
| 755 | Kochia scoparia | DHP_A43_12 | gDNAContig | 1926 | 2125 |
| 756 | Kochia scoparia | DHP_A43_12 | gDNAContig | 1926 | 2125 |
| 757 | Kochia scoparia | DHP_A43_13 | gDNAContig | 2101 | 2300 |
| 758 | Kochia scoparia | DHP_A43_13 | gDNAContig | 2101 | 2300 |
| 759 | Kochia scoparia | DHP_A43_14 | gDNAContig | 2276 | 2475 |
| 760 | Kochia scoparia | DHP_A43_14 | gDNAContig | 2276 | 2475 |
| 761 | Kochia scoparia | DHP_A43_15 | gDNAContig | 2451 | 2650 |
| 762 | Kochia scoparia | DHP_A43_15 | gDNAContig | 2451 | 2650 |
| 763 | Kochia scoparia | DHP_A43_16 | gDNAContig | 2626 | 2825 |
| 764 | Kochia scoparia | DHP_A43_16 | gDNAContig | 2626 | 2825 |
| 765 | Kochia scoparia | DHP_A43_17 | gDNAContig | 2801 | 3000 |
| 766 | Kochia scoparia | DHP_A43_17 | gDNAContig | 2801 | 3000 |
| 767 | Kochia scoparia | DHP_A43_18 | gDNAContig | 2976 | 3175 |
| 768 | Kochia scoparia | DHP_A43_18 | gDNAContig | 2976 | 3175 |
| 769 | Kochia scoparia | DHP_A43_19 | gDNAContig | 3151 | 3350 |
| 770 | Kochia scoparia | DHP_A43_19 | gDNAContig | 3151 | 3350 |
| 771 | Kochia scoparia | DHP_A43_20 | gDNAContig | 3326 | 3525 |
| 772 | Kochia scoparia | DHP_A43_20 | gDNAContig | 3326 | 3525 |
| 773 | Kochia scoparia | DHP_A43_21 | gDNAContig | 3501 | 3700 |
| 774 | Kochia scoparia | DHP_A43_21 | gDNAContig | 3501 | 3700 |
| 775 | Kochia scoparia | DHP_A43_22 | gDNAContig | 3676 | 3875 |
| 776 | Kochia scoparia | DHP_A43_22 | gDNAContig | 3676 | 3875 |
| 777 | Kochia scoparia | DHP_A46_1 | cDNAContig | 1 | 200 |
| 778 | Kochia scoparia | DHP_A46_1 | cDNAContig | 1 | 200 |
| 779 | Kochia scoparia | DHP_A46_2 | cDNAContig | 176 | 375 |
| 780 | Kochia scoparia | DHP_A46_2 | cDNAContig | 176 | 375 |
| 781 | Kochia scoparia | DHP_A46_3 | cDNAContig | 351 | 550 |
| 782 | Kochia scoparia | DHP_A46_3 | cDNAContig | 351 | 550 |
| 783 | Kochia scoparia | DHP_A46_4 | cDNAContig | 526 | 725 |
| 784 | Kochia scoparia | DHP_A46_4 | cDNAContig | 526 | 725 |
| 785 | Kochia scoparia | DHP_A46_5 | cDNAContig | 701 | 900 |
| 786 | Kochia scoparia | DHP_A46_5 | cDNAContig | 701 | 900 |
| 787 | Kochia scoparia | DHP_A46_6 | cDNAContig | 876 | 1075 |
| 788 | Kochia scoparia | DHP_A46_6 | cDNAContig | 876 | 1075 |
| 789 | Kochia scoparia | DHP_A46_7 | cDNAContig | 1051 | 1250 |
| 790 | Kochia scoparia | DHP_A46_7 | cDNAContig | 1051 | 1250 |
| 791 | Kochia scoparia | DHP_A46_8 | cDNAContig | 1226 | 1425 |
| 792 | Kochia scoparia | DHP_A46_8 | cDNAContig | 1226 | 1425 |
| 793 | Kochia scoparia | DHP_A44_1 | gDNAContig | 1 | 200 |
| 794 | Kochia scoparia | DHP_A44_1 | gDNAContig | 1 | 200 |
| 795 | Kochia scoparia | DHP_A44_2 | gDNAContig | 176 | 375 |
| 796 | Kochia scoparia | DHP_A44_2 | gDNAContig | 176 | 375 |
| 797 | Kochia scoparia | DHP_A44_3 | gDNAContig | 351 | 550 |
| 798 | Kochia scoparia | DHP_A44_3 | gDNAContig | 351 | 550 |
| 799 | Kochia scoparia | DHP_A44_4 | gDNAContig | 526 | 725 |
| 800 | Kochia scoparia | DHP_A44_4 | gDNAContig | 526 | 725 |
| 801 | Kochia scoparia | DHP_A44_5 | gDNAContig | 701 | 900 |
| 802 | Kochia scoparia | DHP_A44_5 | gDNAContig | 701 | 900 |
| 803 | Kochia scoparia | DHP_A44_6 | gDNAContig | 876 | 1075 |
| 804 | Kochia scoparia | DHP_A44_6 | gDNAContig | 876 | 1075 |
| 805 | Kochia scoparia | DHP_A44_7 | gDNAContig | 1051 | 1250 |
| 806 | Kochia scoparia | DHP_A44_7 | gDNAContig | 1051 | 1250 |
| 807 | Kochia scoparia | DHP_A44_8 | gDNAContig | 1226 | 1425 |
| 808 | Kochia scoparia | DHP_A44_8 | gDNAContig | 1226 | 1425 |
| 809 | Kochia scoparia | DHP_A44_9 | gDNAContig | 1401 | 1600 |
| 810 | Kochia scoparia | DHP_A44_9 | gDNAContig | 1401 | 1600 |
| 811 | Kochia scoparia | DHP_A44_10 | gDNAContig | 1576 | 1775 |
| 812 | Kochia scoparia | DHP_A44_10 | gDNAContig | 1576 | 1775 |
| 813 | Kochia scoparia | DHP_A44_11 | gDNAContig | 1751 | 1950 |
| 814 | Kochia scoparia | DHP_A44_11 | gDNAContig | 1751 | 1950 |
| 815 | Kochia scoparia | DHP_A44_12 | gDNAContig | 1926 | 2125 |
| 816 | Kochia scoparia | DHP_A44_12 | gDNAContig | 1926 | 2125 |
| 817 | Kochia scoparia | DHP_A44_13 | gDNAContig | 2101 | 2300 |
| 818 | Kochia scoparia | DHP_A44_13 | gDNAContig | 2101 | 2300 |
| 819 | Kochia scoparia | DHP_A44_14 | gDNAContig | 2276 | 2475 |
| 820 | Kochia scoparia | DHP_A44_14 | gDNAContig | 2276 | 2475 |
| 821 | Kochia scoparia | DHP_A44_15 | gDNAContig | 2451 | 2650 |
| 822 | Kochia scoparia | DHP_A44_15 | gDNAContig | 2451 | 2650 |
| 823 | Kochia scoparia | DHP_A44_16 | gDNAContig | 2626 | 2825 |
| 824 | Kochia scoparia | DHP_A44_16 | gDNAContig | 2626 | 2825 |
| 825 | Kochia scoparia | DHP_A44_17 | gDNAContig | 2801 | 3000 |
| 826 | Kochia scoparia | DHP_A44_17 | gDNAContig | 2801 | 3000 |
| 827 | Kochia scoparia | DHP_A44_18 | gDNAContig | 2976 | 3175 |
| 828 | Kochia scoparia | DHP_A44_18 | gDNAContig | 2976 | 3175 |
| 829 | Kochia scoparia | DHP_A44_19 | gDNAContig | 3151 | 3350 |
| 830 | Kochia scoparia | DHP_A44_19 | gDNAContig | 3151 | 3350 |
| 831 | Lolium multiflorum | DHP_A47_1 | gDNAContig | 1 | 200 |
| 832 | Lolium multiflorum | DHP_A47_1 | gDNAContig | 1 | 200 |
| 833 | Lolium multiflorum | DHP_A47_2 | gDNAContig | 176 | 375 |
| 834 | Lolium multiflorum | DHP_A47_2 | gDNAContig | 176 | 375 |
| 835 | Lolium multiflorum | DHP_A47_3 | gDNAContig | 351 | 550 |
| 836 | Lolium multiflorum | DHP_A47_3 | gDNAContig | 351 | 550 |
| 837 | Lolium multiflorum | DHP_A47_4 | gDNAContig | 526 | 725 |
| 838 | Lolium multiflorum | DHP_A47_4 | gDNAContig | 526 | 725 |
| 839 | Lolium multiflorum | DHP_A47_5 | gDNAContig | 701 | 900 |
| 840 | Lolium multiflorum | DHP_A47_5 | gDNAContig | 701 | 900 |
| 841 | Lolium multiflorum | DHP_A47_6 | gDNAContig | 876 | 1075 |
| 842 | Lolium multiflorum | DHP_A47_6 | gDNAContig | 876 | 1075 |
| 843 | Lolium multiflorum | DHP_A47_7 | gDNAContig | 1051 | 1250 |
| 844 | Lolium multiflorum | DHP_A47_7 | gDNAContig | 1051 | 1250 |
| 845 | Lolium multiflorum | DHP_A47_8 | gDNAContig | 1226 | 1425 |
| 846 | Lolium multiflorum | DHP_A47_8 | gDNAContig | 1226 | 1425 |
| 847 | Lolium multiflorum | DHP_A47_9 | gDNAContig | 1401 | 1600 |
| 848 | Lolium multiflorum | DHP_A47_9 | gDNAContig | 1401 | 1600 |
| 849 | Lolium multiflorum | DHP_A47_10 | gDNAContig | 1576 | 1775 |
| 850 | Lolium multiflorum | DHP_A47_10 | gDNAContig | 1576 | 1775 |
| 851 | Lolium multiflorum | DHP_A47_11 | gDNAContig | 1751 | 1950 |
| 852 | Lolium multiflorum | DHP_A47_11 | gDNAContig | 1751 | 1950 |
| 853 | Lolium multiflorum | DHP_A47_12 | gDNAContig | 1926 | 2125 |
| 854 | Lolium multiflorum | DHP_A47_12 | gDNAContig | 1926 | 2125 |
| 855 | Lolium multiflorum | DHP_A47_13 | gDNAContig | 2101 | 2300 |
| 856 | Lolium multiflorum | DHP_A47_13 | gDNAContig | 2101 | 2300 |
| 857 | Lolium multiflorum | DHP_A47_14 | gDNAContig | 2276 | 2475 |
| 858 | Lolium multiflorum | DHP_A47_14 | gDNAContig | 2276 | 2475 |
| 859 | Lolium multiflorum | DHP_A47_15 | gDNAContig | 2451 | 2650 |
| 860 | Lolium multiflorum | DHP_A47_15 | gDNAContig | 2451 | 2650 |
| 861 | Lolium multiflorum | DHP_A47_16 | gDNAContig | 2626 | 2825 |
| 862 | Lolium multiflorum | DHP_A47_16 | gDNAContig | 2626 | 2825 |
| 863 | Lolium multiflorum | DHP_A47_17 | gDNAContig | 2801 | 3000 |
| 864 | Lolium multiflorum | DHP_A47_17 | gDNAContig | 2801 | 3000 |
| 865 | Lolium multiflorum | DHP_A47_18 | gDNAContig | 2976 | 3175 |
| 866 | Lolium multiflorum | DHP_A47_18 | gDNAContig | 2976 | 3175 |
| 867 | Lolium multiflorum | DHP_A47_19 | gDNAContig | 3151 | 3350 |
| 868 | Lolium multiflorum | DHP_A47_19 | gDNAContig | 3151 | 3350 |
| 869 | Lolium multiflorum | DHP_A47_20 | gDNAContig | 3326 | 3525 |
| 870 | Lolium multiflorum | DHP_A47_20 | gDNAContig | 3326 | 3525 |
| 871 | Lolium multiflorum | DHP_A47_21 | gDNAContig | 3501 | 3700 |
| 872 | Lolium multiflorum | DHP_A47_21 | gDNAContig | 3501 | 3700 |
| 873 | Lolium multiflorum | DHP_A47_22 | gDNAContig | 3676 | 3875 |
| 874 | Lolium multiflorum | DHP_A47_22 | gDNAContig | 3676 | 3875 |
| 875 | Lolium multiflorum | DHP_A48_1 | cDNAContig | 1 | 200 |
| 876 | Lolium multiflorum | DHP_A48_1 | cDNAContig | 1 | 200 |
| 877 | Lolium multiflorum | DHP_A48_2 | cDNAContig | 176 | 375 |
| 878 | Lolium multiflorum | DHP_A48_2 | cDNAContig | 176 | 375 |
| 879 | Lolium multiflorum | DHP_A48_3 | cDNAContig | 351 | 550 |
| 880 | Lolium multiflorum | DHP_A48_3 | cDNAContig | 351 | 550 |
| 881 | Lolium multiflorum | DHP_A48_4 | cDNAContig | 526 | 725 |
| 882 | Lolium multiflorum | DHP_A48_4 | cDNAContig | 526 | 725 |
| 883 | Lolium multiflorum | DHP_A48_5 | cDNAContig | 701 | 900 |
| 884 | Lolium multiflorum | DHP_A48_5 | cDNAContig | 701 | 900 |
| 885 | Lolium multiflorum | DHP_A48_6 | cDNAContig | 876 | 1075 |
| 886 | Lolium multiflorum | DHP_A48_6 | cDNAContig | 876 | 1075 |
| 887 | Lolium multiflorum | DHP_A48_7 | cDNAContig | 1051 | 1250 |
| 888 | Lolium multiflorum | DHP_A48_7 | cDNAContig | 1051 | 1250 |
| 889 | Lolium multiflorum | DHP_A48_8 | cDNAContig | 1226 | 1425 |
| 890 | Lolium multiflorum | DHP_A48_8 | cDNAContig | 1226 | 1425 |
| 891 | Lolium multiflorum | DHP_A51_1 | gDNAContig | 1 | 200 |
| 892 | Lolium multiflorum | DHP_A51_1 | gDNAContig | 1 | 200 |
| 893 | Lolium multiflorum | DHP_A50_1 | cDNAContig | 1 | 200 |
| 894 | Lolium multiflorum | DHP_A50_1 | cDNAContig | 1 | 200 |
| 895 | Lolium multiflorum | DHP_A50_2 | cDNAContig | 176 | 375 |
| 896 | Lolium multiflorum | DHP_A50_2 | cDNAContig | 176 | 375 |
| 897 | Lolium multiflorum | DHP_A50_3 | cDNAContig | 351 | 550 |
| 898 | Lolium multiflorum | DHP_A50_3 | cDNAContig | 351 | 550 |
| 899 | Lolium multiflorum | DHP_A50_4 | cDNAContig | 526 | 725 |
| 900 | Lolium multiflorum | DHP_A50_4 | cDNAContig | 526 | 725 |
| 901 | Lolium multiflorum | DHP_A53_1 | gDNAContig | 1 | 200 |
| 902 | Lolium multiflorum | DHP_A53_1 | gDNAContig | 1 | 200 |
| 903 | Lolium multiflorum | DHP_A52_1 | cDNAContig | 1 | 200 |
| 904 | Lolium multiflorum | DHP_A52_1 | cDNAContig | 1 | 200 |
| 905 | Lolium multiflorum | DHP_A54_1 | cDNAContig | 1 | 150 |
| 906 | Lolium multiflorum | DHP_A54_1 | cDNAContig | 1 | 150 |

**Table 3**

| SEQ ID NO | Gene | # Species | Species |
|---|---|---|---|
| 907 | DHP | 5 | Amaranthus hybridus, Amaranthus palmeri, Amaranthus rudis, Amaranthus viridis,Kochia scoparia |
| 908 | DHP | 5 | Amaranthus hybridus, Amaranthus palmeri, Amaranthus rudis, Amaranthus viridis,Kochia scoparia |
| 909 | DHP | 5 | Amaranthus hybridus, Amaranthus palmeri, Amaranthus rudis, Amaranthus viridis,Kochia scoparia |
| 910 | DHP | 5 | Amaranthus lividus, Amaranthus palmeri, Amaranthus rudis, Amaranthus viridis,Kochia scoparia |
| 911 | DHP | 5 | Amaranthus lividus, Amaranthus palmeri, Amaranthus rudis, Amaranthus viridis,Kochia scoparia |
| 912 | DHP | 5 | Amaranthus hybridus, Amaranthus palmeri, Amaranthus rudis, Amaranthus viridis,Kochia scoparia |
| 913 | DHP | 5 | Amaranthus hybridus, Amaranthus palmeri, Amaranthus rudis, Amaranthus viridis,Kochia scoparia |
| 914 | DHP | 5 | Amaranthus hybridus, Amaranthus palmeri, Amaranthus rudis, Amaranthus viridis,Kochia scoparia |
| 915 | DHP | 5 | Amaranthus hybridus, Amaranthus palmeri, Amaranthus rudis, Amaranthus viridis,Kochia scoparia |
| 916 | DHP | 5 | Amaranthus hybridus, Amaranthus palmeri, Amaranthus rudis, Amaranthus viridis,Kochia scoparia |
| 917 | DHP | 5 | Amaranthus lividus, Amaranthus palmeri, Amaranthus rudis, Amaranthus viridis,Kochia scoparia |
| 918 | DHP | 5 | Amaranthus hybridus, Amaranthus palmeri, Amaranthus rudis, Amaranthus viridis,Kochia scoparia |
| 919 | DHP | 5 | Amaranthus hybridus, Amaranthus palmeri, Amaranthus rudis, Amaranthus viridis,Kochia scoparia |
| 920 | DHP | 5 | Amaranthus hybridus, Amaranthus palmeri, Amaranthus rudis, Amaranthus viridis,Kochia scoparia |
| 921 | DHP | 5 | Amaranthus hybridus, Amaranthus palmeri, Amaranthus rudis, Amaranthus viridis,Kochia scoparia |
| 922 | DHP | 4 | Amaranthus lividus, Amaranthus palmeri, Amaranthus rudis, Amaranthus viridis |
| 923 | DHP | 4 | Amaranthus lividus, Amaranthus palmeri, Amaranthus rudis, Amaranthus viridis |
| 924 | DHP | 4 | Amaranthus lividus, Amaranthus palmeri, Amaranthus rudis, Amaranthus viridis |
| 925 | DHP | 4 | Amaranthus lividus, Amaranthus palmeri, Amaranthus rudis, Amaranthus viridis |
| 926 | DHP | 4 | Amaranthus hybridus, Amaranthus palmeri, Amaranthus rudis, Amaranthus viridis |
| 927 | DHP | 4 | Amaranthus hybridus, Amaranthus palmeri, Amaranthus rudis, Amaranthus viridis |
| 928 | DHP | 4 | Amaranthus lividus, Amaranthus palmeri, Amaranthus rudis, Amaranthus viridis |
| 929 | DHP | 4 | Amaranthus lividus, Amaranthus palmeri, Amaranthus rudis, Amaranthus viridis |
| 930 | DHP | 4 | Amaranthus lividus, Amaranthus palmeri, Amaranthus rudis, Amaranthus viridis |
| 931 | DHP | 4 | Amaranthus lividus, Amaranthus palmeri, Amaranthus rudis, Amaranthus viridis |
| 932 | DHP | 4 | Amaranthus lividus, Amaranthus palmeri, Amaranthus rudis, Amaranthus viridis |
| 933 | DHP | 4 | Amaranthus hybridus, Amaranthus palmeri, Amaranthus rudis, Amaranthus viridis |
| 934 | DHP | 4 | Amaranthus lividus, Amaranthus palmeri, Amaranthus rudis, Amaranthus viridis |
| 935 | DHP | 4 | Amaranthus lividus, Amaranthus palmeri, Amaranthus rudis, Amaranthus viridis |
| 936 | DHP | 4 | Amaranthus hybridus, Amaranthus palmeri, Amaranthus rudis, Amaranthus viridis |
| 937 | DHP | 4 | Amaranthus lividus, Amaranthus palmeri, Amaranthus rudis, Amaranthus viridis |
| 938 | DHP | 4 | Amaranthus lividus, Amaranthus palmeri, Amaranthus rudis, Amaranthus viridis |
| 939 | DHP | 4 | Amaranthus lividus, Amaranthus palmeri, Amaranthus rudis, Amaranthus viridis |
| 940 | DHP | 4 | Amaranthus lividus, Amaranthus palmeri, Amaranthus rudis, Amaranthus viridis |
| 941 | DHP | 4 | Amaranthus lividus, Amaranthus palmeri, Amaranthus rudis, Amaranthus viridis |
| 942 | DHP | 4 | Amaranthus hybridus, Amaranthus palmeri, Amaranthus rudis, Amaranthus viridis |
| 943 | DHP | 4 | Amaranthus lividus, Amaranthus palmeri, Amaranthus rudis, Amaranthus viridis |
| 944 | DHP | 4 | Amaranthus lividus, Amaranthus palmeri, Amaranthus rudis, Amaranthus viridis |
| 945 | DHP | 4 | Amaranthus lividus, Amaranthus palmeri, Amaranthus rudis, Amaranthus viridis |
| 946 | DHP | 4 | Amaranthus lividus, Amaranthus palmeri, Amaranthus rudis, Amaranthus viridis |
| 947 | DHP | 4 | Amaranthus lividus, Amaranthus palmeri, Amaranthus rudis, Amaranthus viridis |
| 948 | DHP | 4 | Amaranthus lividus, Amaranthus palmeri, Amaranthus rudis, Amaranthus viridis |
| 949 | DHP | 4 | Amaranthus lividus, Amaranthus palmeri, Amaranthus rudis, Amaranthus viridis |
| 950 | DHP | 4 | Amaranthus lividus, Amaranthus palmeri, Amaranthus rudis, Amaranthus viridis |
| 951 | DHP | 4 | Amaranthus lividus, Amaranthus palmeri, Amaranthus rudis, Amaranthus viridis |
| 952 | DHP | 4 | Amaranthus lividus, Amaranthus palmeri, Amaranthus rudis, Amaranthus viridis |
| 953 | DHP | 4 | Amaranthus lividus, Amaranthus palmeri, Amaranthus rudis, Amaranthus viridis |
| 954 | DHP | 4 | Amaranthus lividus, Amaranthus palmeri, Amaranthus rudis, Amaranthus viridis |
| 955 | DHP | 4 | Amaranthus lividus, Amaranthus palmeri, Amaranthus rudis, Amaranthus viridis |
| 956 | DHP | 4 | Amaranthus lividus, Amaranthus rudis, Amaranthus viridis,Kochia scoparia |
| 957 | DHP | 4 | Amaranthus lividus, Amaranthus palmeri, Amaranthus rudis, Amaranthus viridis |
| 958 | DHP | 4 | Amaranthus hybridus, Amaranthus palmeri, Amaranthus rudis, Amaranthus viridis |
| 959 | DHP | 4 | Amaranthus palmeri, Amaranthus rudis, Amaranthus viridis,Kochia scoparia |
| 960 | DHP | 4 | Amaranthus hybridus, Amaranthus palmeri, Amaranthus rudis, Amaranthus viridis |
| 961 | DHP | 4 | Amaranthus lividus, Amaranthus palmeri, Amaranthus rudis, Amaranthus viridis |
| 962 | DHP | 4 | Amaranthus lividus, Amaranthus palmeri, Amaranthus rudis, Amaranthus viridis |
| 963 | DHP | 4 | Amaranthus lividus, Amaranthus palmeri, Amaranthus rudis, Amaranthus viridis |
| 964 | DHP | 4 | Amaranthus hybridus, Amaranthus palmeri, Amaranthus rudis, Amaranthus viridis |
| 965 | DHP | 4 | Amaranthus hybridus, Amaranthus palmeri, Amaranthus rudis, Amaranthus viridis |
| 966 | DHP | 4 | Amaranthus hybridus, Amaranthus palmeri, Amaranthus rudis, Amaranthus viridis |
| 967 | DHP | 4 | Amaranthus lividus, Amaranthus palmeri, Amaranthus rudis, Amaranthus viridis |
| 968 | DHP | 4 | Amaranthus lividus, Amaranthus palmeri, Amaranthus rudis, Amaranthus viridis |
| 969 | DHP | 4 | Amaranthus lividus, Amaranthus palmeri, Amaranthus rudis, Amaranthus viridis |
| 970 | DHP | 4 | Amaranthus hybridus, Amaranthus palmeri, Amaranthus rudis, Amaranthus viridis |
| 971 | DHP | 4 | Amaranthus lividus, Amaranthus palmeri, Amaranthus rudis, Amaranthus viridis |
| 972 | DHP | 4 | Amaranthus lividus, Amaranthus palmeri, Amaranthus rudis, Amaranthus viridis |
| 973 | DHP | 4 | Amaranthus lividus, Amaranthus palmeri, Amaranthus rudis, Amaranthus viridis |
| 974 | DHP | 4 | Amaranthus lividus, Amaranthus palmeri, Amaranthus rudis, Amaranthus viridis |
| 975 | DHP | 4 | Amaranthus lividus, Amaranthus palmeri, Amaranthus rudis, Amaranthus viridis |
| 976 | DHP | 4 | Amaranthus lividus, Amaranthus palmeri, Amaranthus rudis, Amaranthus viridis |
| 977 | DHP | 4 | Amaranthus lividus, Amaranthus palmeri, Amaranthus rudis, Amaranthus viridis |
| 978 | DHP | 4 | Amaranthus lividus, Amaranthus palmeri, Amaranthus rudis, Amaranthus viridis |
| 979 | DHP | 4 | Amaranthus lividus, Amaranthus palmeri, Amaranthus rudis, Amaranthus viridis |
| 980 | DHP | 4 | Amaranthus lividus, Amaranthus palmeri, Amaranthus rudis, Amaranthus viridis |
| 981 | DHP | 4 | Amaranthus hybridus, Amaranthus palmeri, Amaranthus rudis, Amaranthus viridis |
| 982 | DHP | 4 | Amaranthus lividus, Amaranthus palmeri, Amaranthus rudis, Amaranthus viridis |
| 983 | DHP | 4 | Amaranthus lividus, Amaranthus palmeri, Amaranthus rudis, Amaranthus viridis |
| 984 | DHP | 4 | Amaranthus lividus, Amaranthus palmeri, Amaranthus rudis, Amaranthus viridis |
| 985 | DHP | 4 | Amaranthus hybridus, Amaranthus palmeri, Amaranthus rudis, Amaranthus viridis |
| 986 | DHP | 4 | Amaranthus lividus, Amaranthus palmeri, Amaranthus rudis, Amaranthus viridis |
| 987 | DHP | 4 | Amaranthus lividus, Amaranthus palmeri, Amaranthus rudis, Amaranthus viridis |
| 988 | DHP | 4 | Amaranthus lividus, Amaranthus palmeri, Amaranthus rudis, Amaranthus viridis |
| 989 | DHP | 4 | Amaranthus lividus, Amaranthus palmeri, Amaranthus rudis, Amaranthus viridis |
| 990 | DHP | 4 | Amaranthus lividus, Amaranthus palmeri, Amaranthus rudis, Amaranthus viridis |
| 991 | DHP | 4 | Amaranthus lividus, Amaranthus palmeri, Amaranthus rudis, Amaranthus viridis |
| 992 | DHP | 4 | Amaranthus lividus, Amaranthus palmeri, Amaranthus rudis, Amaranthus viridis |
| 993 | DHP | 4 | Amaranthus lividus, Amaranthus palmeri, Amaranthus rudis, Amaranthus viridis |
| 994 | DHP | 4 | Amaranthus lividus, Amaranthus palmeri, Amaranthus rudis, Amaranthus viridis |
| 995 | DHP | 4 | Amaranthus lividus, Amaranthus palmeri, Amaranthus rudis, Amaranthus viridis |
| 996 | DHP | 4 | Amaranthus lividus, Amaranthus palmeri, Amaranthus rudis, Amaranthus viridis |
| 997 | DHP | 4 | Amaranthus lividus, Amaranthus palmeri, Amaranthus rudis, Amaranthus viridis |
| 998 | DHP | 4 | Amaranthus lividus, Amaranthus palmeri, Amaranthus rudis, Amaranthus viridis |
| 999 | DHP | 4 | Amaranthus lividus, Amaranthus palmeri, Amaranthus rudis, Amaranthus viridis |
| 1000 | DHP | 4 | Amaranthus palmeri, Amaranthus rudis, Amaranthus viridis,Kochia scoparia |
| 1001 | DHP | 4 | Amaranthus lividus, Amaranthus palmeri, Amaranthus rudis, Amaranthus viridis |
| 1002 | DHP | 4 | Amaranthus lividus, Amaranthus palmeri, Amaranthus rudis, Amaranthus viridis |
| 1003 | DHP | 4 | Amaranthus lividus, Amaranthus palmeri, Amaranthus rudis, Amaranthus viridis |
| 1004 | DHP | 4 | Amaranthus lividus, Amaranthus palmeri, Amaranthus rudis, Amaranthus viridis |
| 1005 | DHP | 4 | Amaranthus lividus, Amaranthus palmeri, Amaranthus rudis, Amaranthus viridis |
| 1006 | DHP | 4 | Amaranthus hybridus, Amaranthus palmeri, Amaranthus rudis, Amaranthus viridis |
| 1007 | DHP | 4 | Amaranthus lividus, Amaranthus palmeri, Amaranthus rudis, Amaranthus viridis |
| 1008 | DHP | 4 | Amaranthus lividus, Amaranthus palmeri, Amaranthus rudis, Amaranthus viridis |
| 1009 | DHP | 4 | Amaranthus lividus, Amaranthus palmeri, Amaranthus rudis, Amaranthus viridis |
| 1010 | DHP | 4 | Amaranthus lividus, Amaranthus palmeri, Amaranthus rudis, Amaranthus viridis |
| 1011 | DHP | 4 | Amaranthus lividus, Amaranthus palmeri, Amaranthus rudis, Amaranthus viridis |
| 1012 | DHP | 4 | Amaranthus lividus, Amaranthus palmeri, Amaranthus rudis, Amaranthus viridis |
| 1013 | DHP | 4 | Amaranthus lividus, Amaranthus palmeri, Amaranthus rudis, Amaranthus viridis |
| 1014 | DHP | 4 | Amaranthus lividus, Amaranthus palmeri, Amaranthus rudis, Amaranthus viridis |
| 1015 | DHP | 4 | Amaranthus hybridus, Amaranthus palmeri, Amaranthus rudis, Amaranthus viridis |
| 1016 | DHP | 4 | Amaranthus hybridus, Amaranthus palmeri, Amaranthus rudis, Amaranthus viridis |
| 1017 | DHP | 4 | Amaranthus lividus, Amaranthus rudis, Amaranthus viridis,Kochia scoparia |
| 1018 | DHP | 4 | Amaranthus lividus, Amaranthus palmeri, Amaranthus rudis, Amaranthus viridis |
| 1019 | DHP | 4 | Amaranthus lividus, Amaranthus palmeri, Amaranthus rudis, Amaranthus viridis |
| 1020 | DHP | 4 | Amaranthus hybridus, Amaranthus palmeri, Amaranthus rudis, Amaranthus viridis |
| 1021 | DHP | 4 | Amaranthus lividus, Amaranthus palmeri, Amaranthus rudis, Amaranthus viridis |
| 1022 | DHP | 4 | Amaranthus lividus, Amaranthus palmeri, Amaranthus rudis, Amaranthus viridis |
| 1023 | DHP | 4 | Amaranthus hybridus, Amaranthus palmeri, Amaranthus rudis, Amaranthus viridis |
| 1024 | DHP | 4 | Amaranthus palmeri, Amaranthus rudis, Amaranthus viridis,Kochia scoparia |
| 1025 | DHP | 4 | Amaranthus lividus, Amaranthus palmeri, Amaranthus rudis, Amaranthus viridis |
| 1026 | DHP | 4 | Amaranthus lividus, Amaranthus palmeri, Amaranthus rudis, Amaranthus viridis |
| 1027 | DHP | 4 | Amaranthus lividus, Amaranthus palmeri, Amaranthus rudis, Amaranthus viridis |
| 1028 | DHP | 4 | Amaranthus lividus, Amaranthus palmeri, Amaranthus rudis, Amaranthus viridis |
| 1029 | DHP | 4 | Amaranthus lividus, Amaranthus palmeri, Amaranthus rudis, Amaranthus viridis |
| 1030 | DHP | 4 | Amaranthus lividus, Amaranthus palmeri, Amaranthus rudis, Amaranthus viridis |
| 1031 | DHP | 4 | Amaranthus lividus, Amaranthus palmeri, Amaranthus rudis, Amaranthus viridis |
| 1032 | DHP | 4 | Amaranthus lividus, Amaranthus palmeri, Amaranthus rudis, Amaranthus viridis |
| 1033 | DHP | 4 | Amaranthus lividus, Amaranthus palmeri, Amaranthus rudis, Amaranthus viridis |
| 1034 | DHP | 4 | Amaranthus lividus, Amaranthus palmeri, Amaranthus rudis, Amaranthus viridis |
| 1035 | DHP | 4 | Amaranthus lividus, Amaranthus palmeri, Amaranthus rudis, Amaranthus viridis |
| 1036 | DHP | 4 | Amaranthus hybridus, Amaranthus palmeri, Amaranthus rudis, Amaranthus viridis |
| 1037 | DHP | 4 | Amaranthus lividus, Amaranthus palmeri, Amaranthus rudis, Amaranthus viridis |
| 1038 | DHP | 4 | Amaranthus hybridus, Amaranthus palmeri, Amaranthus rudis, Amaranthus viridis |
| 1039 | DHP | 4 | Amaranthus lividus, Amaranthus palmeri, Amaranthus rudis, Amaranthus viridis |
| 1040 | DHP | 4 | Amaranthus lividus, Amaranthus palmeri, Amaranthus rudis, Amaranthus viridis |
| 1041 | DHP | 4 | Amaranthus lividus, Amaranthus palmeri, Amaranthus rudis, Amaranthus viridis |
| 1042 | DHP | 4 | Amaranthus lividus, Amaranthus palmeri, Amaranthus rudis, Amaranthus viridis |
| 1043 | DHP | 4 | Amaranthus hybridus, Amaranthus palmeri, Amaranthus rudis, Amaranthus viridis |
| 1044 | DHP | 4 | Amaranthus hybridus, Amaranthus palmeri, Amaranthus rudis, Amaranthus viridis |
| 1045 | DHP | 4 | Amaranthus lividus, Amaranthus palmeri, Amaranthus rudis, Amaranthus viridis |
| 1046 | DHP | 4 | Amaranthus lividus, Amaranthus palmeri, Amaranthus rudis, Amaranthus viridis |
| 1047 | DHP | 4 | Amaranthus hybridus, Amaranthus palmeri, Amaranthus rudis, Amaranthus viridis |
| 1048 | DHP | 4 | Amaranthus lividus, Amaranthus palmeri, Amaranthus rudis, Amaranthus viridis |
| 1049 | DHP | 4 | Amaranthus lividus, Amaranthus palmeri, Amaranthus rudis, Amaranthus viridis |
| 1050 | DHP | 4 | Amaranthus lividus, Amaranthus palmeri, Amaranthus rudis, Amaranthus viridis |
| 1051 | DHP | 4 | Amaranthus lividus, Amaranthus palmeri, Amaranthus rudis, Amaranthus viridis |
| 1052 | DHP | 4 | Amaranthus lividus, Amaranthus palmeri, Amaranthus rudis, Amaranthus viridis |
| 1053 | DHP | 4 | Amaranthus lividus, Amaranthus palmeri, Amaranthus rudis, Amaranthus viridis |
| 1054 | DHP | 4 | Amaranthus lividus, Amaranthus palmeri, Amaranthus rudis, Amaranthus viridis |
| 1055 | DHP | 4 | Amaranthus lividus, Amaranthus palmeri, Amaranthus rudis, Amaranthus viridis |
| 1056 | DHP | 4 | Amaranthus lividus, Amaranthus palmeri, Amaranthus rudis, Amaranthus viridis |
| 1057 | DHP | 4 | Amaranthus lividus, Amaranthus palmeri, Amaranthus rudis, Amaranthus viridis |
| 1058 | DHP | 4 | Amaranthus lividus, Amaranthus palmeri, Amaranthus rudis, Amaranthus viridis |
| 1059 | DHP | 4 | Amaranthus lividus, Amaranthus palmeri, Amaranthus rudis, Amaranthus viridis |
| 1060 | DHP | 4 | Amaranthus lividus, Amaranthus palmeri, Amaranthus rudis, Amaranthus viridis |
| 1061 | DHP | 4 | Amaranthus lividus, Amaranthus palmeri, Amaranthus rudis, Amaranthus viridis |
| 1062 | DHP | 4 | Amaranthus hybridus, Amaranthus palmeri, Amaranthus rudis, Amaranthus viridis |
| 1063 | DHP | 4 | Amaranthus lividus, Amaranthus palmeri, Amaranthus rudis, Amaranthus viridis |
| 1064 | DHP | 4 | Amaranthus lividus, Amaranthus palmeri, Amaranthus rudis, Amaranthus viridis |
| 1065 | DHP | 4 | Amaranthus lividus, Amaranthus palmeri, Amaranthus rudis, Amaranthus viridis |
| 1066 | DHP | 4 | Amaranthus lividus, Amaranthus palmeri, Amaranthus rudis, Amaranthus viridis |
| 1067 | DHP | 4 | Amaranthus lividus, Amaranthus palmeri, Amaranthus rudis, Amaranthus viridis |
| 1068 | DHP | 4 | Amaranthus lividus, Amaranthus palmeri, Amaranthus rudis, Amaranthus viridis |
| 1069 | DHP | 4 | Amaranthus lividus, Amaranthus palmeri, Amaranthus rudis, Amaranthus viridis |
| 1070 | DHP | 4 | Amaranthus lividus, Amaranthus palmeri, Amaranthus rudis, Amaranthus viridis |
| 1071 | DHP | 4 | Amaranthus lividus, Amaranthus palmeri, Amaranthus rudis, Amaranthus viridis |
| 1072 | DHP | 4 | Amaranthus lividus, Amaranthus palmeri, Amaranthus rudis, Amaranthus viridis |
| 1073 | DHP | 4 | Amaranthus lividus, Amaranthus palmeri, Amaranthus rudis, Amaranthus viridis |
| 1074 | DHP | 4 | Amaranthus lividus, Amaranthus palmeri, Amaranthus rudis, Amaranthus viridis |
| 1075 | DHP | 4 | Amaranthus lividus, Amaranthus palmeri, Amaranthus rudis, Amaranthus viridis |
| 1076 | DHP | 4 | Amaranthus lividus, Amaranthus palmeri, Amaranthus rudis, Amaranthus viridis |
| 1077 | DHP | 4 | Amaranthus lividus, Amaranthus palmeri, Amaranthus rudis, Amaranthus viridis |
| 1078 | DHP | 4 | Amaranthus lividus, Amaranthus palmeri, Amaranthus rudis, Amaranthus viridis |
| 1079 | DHP | 4 | Amaranthus lividus, Amaranthus palmeri, Amaranthus rudis, Amaranthus viridis |
| 1080 | DHP | 4 | Amaranthus lividus, Amaranthus palmeri, Amaranthus rudis, Amaranthus viridis |
| 1081 | DHP | 4 | Amaranthus lividus, Amaranthus palmeri, Amaranthus rudis, Amaranthus viridis |
| 1082 | DHP | 4 | Amaranthus lividus, Amaranthus palmeri, Amaranthus rudis, Amaranthus viridis |
| 1083 | DHP | 4 | Amaranthus lividus, Amaranthus palmeri, Amaranthus rudis, Amaranthus viridis |
| 1084 | DHP | 4 | Amaranthus lividus, Amaranthus palmeri, Amaranthus rudis, Amaranthus viridis |
| 1085 | DHP | 4 | Amaranthus lividus, Amaranthus palmeri, Amaranthus rudis, Amaranthus viridis |
| 1086 | DHP | 4 | Amaranthus lividus, Amaranthus palmeri, Amaranthus rudis, Amaranthus viridis |
| 1087 | DHP | 4 | Amaranthus lividus, Amaranthus palmeri, Amaranthus rudis, Amaranthus viridis |
| 1088 | DHP | 4 | Amaranthus lividus, Amaranthus palmeri, Amaranthus rudis, Amaranthus viridis |
| 1089 | DHP | 4 | Amaranthus lividus, Amaranthus palmeri, Amaranthus rudis, Amaranthus viridis |
| 1090 | DHP | 4 | Amaranthus lividus, Amaranthus palmeri, Amaranthus rudis, Amaranthus viridis |
| 1091 | DHP | 4 | Amaranthus palmeri, Amaranthus rudis, Amaranthus viridis,Kochia scoparia |
| 1092 | DHP | 4 | Amaranthus lividus, Amaranthus palmeri, Amaranthus rudis, Amaranthus viridis |
| 1093 | DHP | 4 | Amaranthus lividus, Amaranthus palmeri, Amaranthus rudis, Amaranthus viridis |
| 1094 | DHP | 4 | Amaranthus lividus, Amaranthus palmeri, Amaranthus rudis, Amaranthus viridis |
| 1095 | DHP | 4 | Amaranthus lividus, Amaranthus palmeri, Amaranthus rudis, Amaranthus viridis |
| 1096 | DHP | 4 | Amaranthus lividus, Amaranthus palmeri, Amaranthus rudis, Amaranthus viridis |
| 1097 | DHP | 4 | Amaranthus lividus, Amaranthus palmeri, Amaranthus rudis, Amaranthus viridis |
| 1098 | DHP | 4 | Amaranthus lividus, Amaranthus palmeri, Amaranthus rudis, Amaranthus viridis |
| 1099 | DHP | 4 | Amaranthus lividus, Amaranthus palmeri, Amaranthus rudis, Amaranthus viridis |
| 1100 | DHP | 4 | Amaranthus lividus, Amaranthus palmeri, Amaranthus rudis, Amaranthus viridis |
| 1101 | DHP | 4 | Amaranthus hybridus, Amaranthus palmeri, Amaranthus rudis, Amaranthus viridis |
| 1102 | DHP | 4 | Amaranthus lividus, Amaranthus palmeri, Amaranthus rudis, Amaranthus viridis |
| 1103 | DHP | 4 | Amaranthus lividus, Amaranthus palmeri, Amaranthus rudis, Amaranthus viridis |
| 1104 | DHP | 4 | Amaranthus lividus, Amaranthus palmeri, Amaranthus rudis, Amaranthus viridis |
| 1105 | DHP | 4 | Amaranthus lividus, Amaranthus rudis, Amaranthus viridis,Kochia scoparia |
| 1106 | DHP | 4 | Amaranthus lividus, Amaranthus palmeri, Amaranthus rudis, Amaranthus viridis |
| 1107 | DHP | 4 | Amaranthus lividus, Amaranthus palmeri, Amaranthus rudis, Amaranthus viridis |
| 1108 | DHP | 4 | Amaranthus lividus, Amaranthus palmeri, Amaranthus rudis, Amaranthus viridis |
| 1109 | DHP | 4 | Amaranthus lividus, Amaranthus palmeri, Amaranthus rudis, Amaranthus viridis |
| 1110 | DHP | 4 | Amaranthus lividus, Amaranthus palmeri, Amaranthus rudis, Amaranthus viridis |
| 1111 | DHP | 4 | Amaranthus lividus, Amaranthus palmeri, Amaranthus rudis, Amaranthus viridis |
| 1112 | DHP | 4 | Amaranthus lividus, Amaranthus palmeri, Amaranthus rudis, Amaranthus viridis |
| 1113 | DHP | 4 | Amaranthus lividus, Amaranthus palmeri, Amaranthus rudis, Amaranthus viridis |
| 1114 | DHP | 4 | Amaranthus lividus, Amaranthus palmeri, Amaranthus rudis, Amaranthus viridis |
| 1115 | DHP | 4 | Amaranthus lividus, Amaranthus palmeri, Amaranthus rudis, Amaranthus viridis |
| 1116 | DHP | 4 | Amaranthus lividus, Amaranthus palmeri, Amaranthus rudis, Amaranthus viridis |
| 1117 | DHP | 4 | Amaranthus lividus, Amaranthus palmeri, Amaranthus rudis, Amaranthus viridis |
| 1118 | DHP | 4 | Amaranthus lividus, Amaranthus palmeri, Amaranthus rudis, Amaranthus viridis |
| 1119 | DHP | 4 | Amaranthus hybridus, Amaranthus palmeri, Amaranthus rudis, Amaranthus viridis |
| 1120 | DHP | 4 | Amaranthus hybridus, Amaranthus palmeri, Amaranthus rudis, Amaranthus viridis |
| 1121 | DHP | 4 | Amaranthus lividus, Amaranthus palmeri, Amaranthus rudis, Amaranthus viridis |
| 1122 | DHP | 4 | Amaranthus palmeri, Amaranthus rudis, Amaranthus viridis,Kochia scoparia |
| 1123 | DHP | 4 | Amaranthus lividus, Amaranthus palmeri, Amaranthus rudis, Amaranthus viridis |
| 1124 | DHP | 4 | Amaranthus lividus, Amaranthus palmeri, Amaranthus rudis, Amaranthus viridis |
| 1125 | DHP | 4 | Amaranthus lividus, Amaranthus palmeri, Amaranthus rudis, Amaranthus viridis |
| 1126 | DHP | 4 | Amaranthus lividus, Amaranthus palmeri, Amaranthus rudis, Amaranthus viridis |
| 1127 | DHP | 4 | Amaranthus lividus, Amaranthus palmeri, Amaranthus rudis, Amaranthus viridis |
| 1128 | DHP | 4 | Amaranthus lividus, Amaranthus palmeri, Amaranthus rudis, Amaranthus viridis |
| 1129 | DHP | 4 | Amaranthus lividus, Amaranthus palmeri, Amaranthus rudis, Amaranthus viridis |
| 1130 | DHP | 4 | Amaranthus palmeri, Amaranthus rudis, Amaranthus viridis,Kochia scoparia |
| 1131 | DHP | 4 | Amaranthus lividus, Amaranthus palmeri, Amaranthus rudis, Amaranthus viridis |
| 1132 | DHP | 4 | Amaranthus hybridus, Amaranthus palmeri, Amaranthus rudis, Amaranthus viridis |
| 1133 | DHP | 4 | Amaranthus hybridus, Amaranthus palmeri, Amaranthus rudis, Amaranthus viridis |
| 1134 | DHP | 4 | Amaranthus lividus, Amaranthus palmeri, Amaranthus rudis, Amaranthus viridis |
| 1135 | DHP | 4 | Amaranthus lividus, Amaranthus palmeri, Amaranthus rudis, Amaranthus viridis |
| 1136 | DHP | 4 | Amaranthus hybridus, Amaranthus palmeri, Amaranthus rudis, Amaranthus viridis |
| 1137 | DHP | 4 | Amaranthus lividus, Amaranthus palmeri, Amaranthus rudis, Amaranthus viridis |
| 1138 | DHP | 4 | Amaranthus lividus, Amaranthus palmeri, Amaranthus rudis, Amaranthus viridis |
| 1139 | DHP | 4 | Amaranthus lividus, Amaranthus palmeri, Amaranthus rudis, Amaranthus viridis |
| 1140 | DHP | 4 | Amaranthus hybridus, Amaranthus palmeri, Amaranthus rudis, Amaranthus viridis |
| 1141 | DHP | 4 | Amaranthus hybridus, Amaranthus palmeri, Amaranthus rudis, Amaranthus viridis |
| 1142 | DHP | 4 | Amaranthus lividus, Amaranthus rudis, Amaranthus viridis,Kochia scoparia |
| 1143 | DHP | 4 | Amaranthus lividus, Amaranthus palmeri, Amaranthus rudis, Amaranthus viridis |
| 1144 | DHP | 4 | Amaranthus lividus, Amaranthus palmeri, Amaranthus rudis, Amaranthus viridis |
| 1145 | DHP | 4 | Amaranthus lividus, Amaranthus palmeri, Amaranthus rudis, Amaranthus viridis |
| 1146 | DHP | 4 | Amaranthus lividus, Amaranthus palmeri, Amaranthus rudis, Amaranthus viridis |
| 1147 | DHP | 4 | Amaranthus lividus, Amaranthus palmeri, Amaranthus rudis, Amaranthus viridis |
| 1148 | DHP | 4 | Amaranthus lividus, Amaranthus palmeri, Amaranthus rudis, Amaranthus viridis |
| 1149 | DHP | 4 | Amaranthus hybridus, Amaranthus palmeri, Amaranthus rudis, Amaranthus viridis |
| 1150 | DHP | 4 | Amaranthus lividus, Amaranthus palmeri, Amaranthus rudis, Amaranthus viridis |
| 1151 | DHP | 4 | Amaranthus lividus, Amaranthus palmeri, Amaranthus rudis, Amaranthus viridis |
| 1152 | DHP | 4 | Amaranthus lividus, Amaranthus palmeri, Amaranthus rudis, Amaranthus viridis |
| 1153 | DHP | 4 | Amaranthus lividus, Amaranthus palmeri, Amaranthus rudis, Amaranthus viridis |
| 1154 | DHP | 4 | Amaranthus lividus, Amaranthus palmeri, Amaranthus rudis, Amaranthus viridis |
| 1155 | DHP | 4 | Amaranthus lividus, Amaranthus palmeri, Amaranthus rudis, Amaranthus viridis |
| 1156 | DHP | 4 | Amaranthus lividus, Amaranthus palmeri, Amaranthus rudis, Amaranthus viridis |
| 1157 | DHP | 4 | Amaranthus hybridus, Amaranthus palmeri, Amaranthus rudis, Amaranthus viridis |
| 1158 | DHP | 4 | Amaranthus lividus, Amaranthus palmeri, Amaranthus rudis, Amaranthus viridis |
| 1159 | DHP | 4 | Amaranthus hybridus, Amaranthus palmeri, Amaranthus rudis, Amaranthus viridis |
| 1160 | DHP | 4 | Amaranthus lividus, Amaranthus palmeri, Amaranthus rudis, Amaranthus viridis |
| 1161 | DHP | 4 | Amaranthus hybridus, Amaranthus palmeri, Amaranthus rudis, Amaranthus viridis |
| 1162 | DHP | 4 | Amaranthus lividus, Amaranthus palmeri, Amaranthus rudis, Amaranthus viridis |
| 1163 | DHP | 4 | Amaranthus lividus, Amaranthus palmeri, Amaranthus rudis, Amaranthus viridis |
| 1164 | DHP | 4 | Amaranthus lividus, Amaranthus palmeri, Amaranthus rudis, Amaranthus viridis |
| 1165 | DHP | 4 | Amaranthus lividus, Amaranthus palmeri, Amaranthus rudis, Amaranthus viridis |
| 1166 | DHP | 4 | Amaranthus lividus, Amaranthus rudis, Amaranthus viridis,Kochia scoparia |
| 1167 | DHP | 4 | Amaranthus hybridus, Amaranthus palmeri, Amaranthus rudis, Amaranthus viridis |
| 1168 | DHP | 4 | Amaranthus lividus, Amaranthus palmeri, Amaranthus rudis, Amaranthus viridis |
| 1169 | DHP | 4 | Amaranthus lividus, Amaranthus palmeri, Amaranthus rudis, Amaranthus viridis |
| 1170 | DHP | 4 | Amaranthus lividus, Amaranthus palmeri, Amaranthus rudis, Amaranthus viridis |
| 1171 | DHP | 4 | Amaranthus lividus, Amaranthus palmeri, Amaranthus rudis, Amaranthus viridis |
| 1172 | DHP | 4 | Amaranthus lividus, Amaranthus palmeri, Amaranthus rudis, Amaranthus viridis |
| 1173 | DHP | 4 | Amaranthus lividus, Amaranthus palmeri, Amaranthus rudis, Amaranthus viridis |
| 1174 | DHP | 4 | Amaranthus lividus, Amaranthus rudis, Amaranthus viridis,Kochia scoparia |
| 1175 | DHP | 4 | Amaranthus lividus, Amaranthus palmeri, Amaranthus rudis, Amaranthus viridis |

## Claims

1. A method of plant control comprising: treating a plant with a composition comprising a double-stranded RNA (dsRNA) polynucleotide and a transfer agent, wherein said dsRNA polynucleotide is identical or complementary to an RNA sequence of a 7,8-dihydropteroate synthase (DHPS) gene sequence, wherein said DHPS gene sequence is selected from the group consisting of SEQ ID NOs:1, 2, 5, 9, 11, 18, 19, and a polynucleotide fragment thereof with at least 18 contiguous nucleotides from said DHPS gene sequence, wherein said transfer agent is an organosilicone composition or an organosilicone compound contained therein and conditions the surface of said plant for permeation by said dsRNA polynucleotide, and whereby said plant's growth, development, or reproductive ability is suppressed or delayed or said plant is more sensitive to a DHPS inhibitor herbicide or a mitosis inhibitor herbicide as a result of said dsRNA polynucleotide containing composition relative to an untreated plant.

2. The method as claimed in claim 1, wherein
(i) said plant is selected from the group consisting of *Amaranthus palmeri, Amaranthus rudis, Amaranthus hybridus, Amaranthus lividus, Amaranthus viridis, Ambrosia trifida, Conyza canadensis, and Euphorbia heterophylla, ;* or
(ii) said composition further comprises said DHPS inhibitor herbicide or said mitosis inhibitor herbicide and said treating comprises external application to said plant with said composition, preferably said composition further comprises one or more co-herbicides different from said DHPS inhibitor herbicide or said mitosis inhibitor herbicide, wherein said one or more co-herbicides are selected from the group consisting of amide herbicides, arsenical herbicides, benzothiazole herbicides, benzoylcyclohexanedione herbicides, benzofuranyl alkylsulfonate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, glycine herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.

3. The method as claimed in claim 1, wherein said composition comprises any combination of two or more of said dsRNA polynucleotides and said treating comprises external application to said plant with said composition.

4. A composition comprising a dsRNA polynucleotide and a transfer agent, wherein said dsRNA polynucleotide is identical or complementary to an RNA sequence of a DHPS gene sequence, wherein said DHPS gene sequence is selected from the group consisting of SEQ ID NOs:1, 2, 5, 9, 11, 18, 19, and a polynucleotide fragment thereof with at least 18 contiguous nucleotides from said DHPS gene sequence, wherein said transfer agent is an organosilicone composition or an organosilicone compound contained therein and conditions the surface of a plant for permeation by said dsRNA polynucleotide, and whereby said plant treated with said composition has its growth, development, or reproductive ability regulated, suppressed or delayed or said plant is more sensitive to a DHPS inhibitor herbicide or a mitosis inhibitor herbicide as a result of said dsRNA polynucleotide containing composition relative to an untreated plant.

5. The composition of claim 4, wherein
(i) said composition further comprises said DHPS inhibitor herbicide or said mitosis inhibitor herbicide, preferably said DHPS inhibitor herbicide is selected from the group consisting of carbamates and asulam, or said mitosis inhibitor herbicide is selected from the group consisting of dinitroaniline herbicides, or
(ii) said composition further comprises a co-herbicide, wherein said co-herbicide is selected from the group consisting of amide herbicides, arsenical herbicides, benzothiazole herbicides, benzoylcyclohexanedione herbicides, benzofuranyl alkylsulfonate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, glycine herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.

6. A method of reducing expression of a DHPS gene in a plant comprising: external application to said plant of a composition comprising a dsRNA polynucleotide and a transfer agent, wherein said dsRNA polynucleotide is identical or complementary to an RNA sequence of a DHPS gene sequence, wherein said DHPS gene sequence is selected from the group consisting of SEQ ID NOs:1, 2, 5, 9, 11, 18, 19, and a polynucleotide fragment thereof with at least 18 contiguous nucleotides from said DHPS gene sequence, wherein said transfer agent is an organosilicone composition or an organosilicone compound contained therein and conditions the surface of said plant for permeation by said dsRNA polynucleotide, and whereby said expression of said DHPS gene is reduced relative to an untreated plant.

7. A method of identifying dsRNA polynucleotides useful in modulating DHPS gene expression when externally treating a plant comprising: a) providing a plurality of dsRNA polynucleotides that comprise a region identical or complementary to a polynucleotide fragment of at least 18 contiguous nucleotides in length that is identical or complementary to a DHPS gene sequence selected from the group consisting of SEQ ID NOs:1, 2, 5, 9, 11, 18, and 19; b) externally treating said plant with a composition comprising one or more of said dsRNA polynucleotides and a transfer agent, wherein said transfer agent is an organosilicone composition or an organosilicone compound contained therein and conditions the surface of said plant for permeation by said one or more of said dsRNA polynucleotides; c) analyzing said plant, or extract for modulation of DHPS gene expression, and d) said plant treated with said composition has its growth, development or reproductive ability regulated, suppressed, or delayed or said plant is more sensitive to a DHPS inhibitor herbicide or a mitosis inhibitor herbicide as a result of said composition, relative to an untreated plant.

8. The method of claim 7, wherein
(i) said plant is selected from the group consisting of *Amaranthus palmeri, Amaranthus rudis, Amaranthus hybridus, Amaranthus lividus, Amaranthus viridis, Ambrosia trifida, Conyza canadensis, and Euphorbia heterophylla;*
(ii) said DHPS gene expression is reduced in said plant relative to a plant not treated with said composition.

9. An agricultural chemical composition comprising an admixture of a dsRNA polynucleotide, a transfer agent, a DHPS inhibitor herbicide or a mitosis inhibitor herbicide, and a co-herbicide, wherein said dsRNA polynucleotide is identical or complementary to a portion of an RNA sequence of a DHPS gene sequence, wherein said DHPS gene sequence is selected from the group consisting of SEQ ID NOs:1, 2, 5, 9, 11, 18, 19, and a polynucleotide fragment thereof with at least 18 contiguous nucleotides from said DHPS gene sequence, wherein said transfer agent is an organosilicone composition or an organosiliocone compound contained therein and conditions a surface of a plant for permeation by said dsRNA polynucleotide, and whereby said plant treated with said composition has its growth, development, or reproductive ability regulated, suppressed, or delayed or said plant is more sensitive to said DHPS inhibitor herbicide or said mitosis inhibitor herbicide as a result of said dsRNA polynucleotide containing composition, relative to an untreated plant.

10. The agricultural chemical composition of claim 9, wherein said co-herbicide is selected from the group consisting of amide herbicides, arsenical herbicides, benzothiazole herbicides, benzoylcyclohexanedione herbicides, benzofuranyl alkylsulfonate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, glycine herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.

11. An agricultural chemical composition comprising an admixture of a dsRNA polynucleotide, a transfer agent, a DHPS inhibitor herbicide, and a pesticide, wherein said dsRNA polynucleotide is identical or complementary to a portion of an RNA sequence of a DHPS gene sequence, wherein said DHPS gene sequence is selected from the group consisting of SEQ ID NOs:1, 2, 5, 9, 11, 18, 19, and a polynucleotide fragment thereof with at least 18 contiguous nucleotides from said DHPS gene sequence, wherein said transfer agent is an organosilicone composition or an organosilicone compound contained therein and conditions a surface of a plant for permeation by said dsRNA polynucleotide, whereby said plant treated with said composition has its growth, development, or reproductive ability regulated, suppressed, or delayed or said plant is more sensitive to said DHPS inhibitor herbicide or a mitosis inhibitor herbicide as a result of said dsRNA polynucleotide containing composition relative to an untreated plant.

12. The agricultural chemical composition of claim 11, wherein said pesticide is selected from the group consisting of insecticides, fungicides, nematicides, bactericides, acaricides, growth regulators, chemosterilants, semiochemicals, repellents, attractants, pheromones, feeding stimulants, and biopesticides.

13. A composition comprising a dsRNA polynucleotide and a transfer agent according to claim 4, wherein said dsRNA polynucleotide is identical or complementary to an RNA sequence of a DHPS gene sequence, wherein said DHPS gene sequence comprises SEQ ID NOs:1176, 1186, a complement thereof, and a polynucleotide fragment thereof with at least 18 contiguous nucleotides from said DHPS gene sequence.

## Patentansprüche

1. Verfahren zur Kontrolle von Pflanzen, Schritte umfassend, bei denen man: eine Pflanze mit einer Zusammensetzung behandelt, die ein doppelsträngiges RNA (dsRNA)-Polynukleotid und ein Übertragungsmittel umfasst, wobei das dsRNA-Polynukleotid identisch mit oder komplementär zu einer RNA-Sequenz einer 7,8-Dihydropteroatsynthase (DHPS)-Gensequenz ist, wobei die DHPS-Gensequenz aus der Gruppe ausgewählt wurde, die aus SEQ ID Nummern:1, 2, 5, 9, 11, 18, 19 und einem Polynukleotidfragment davon mit mindestens 18 aufeinanderfolgenden Nukleotiden der DHPS-Gensequenz besteht, wobei das Übertragungsmittel eine siliciumorganische Zusammensetzung oder eine darin enthaltene siliciumorganische Verbindung ist, welche die Oberfläche der Pflanze für das Eindringen des dsRNA-Polynukleotids konditioniert, und wobei das Wachstum, die Entwicklung oder die Fortpflanzungsfähigkeit der Pflanze unterdrückt oder verzögert wird oder die Pflanze im Vergleich zu einer unbehandelten Pflanze durch die Zusammensetzung, welche das dsRNA-Polynukleotid enthält, auf ein DHPS-Inhibitor-Herbizid oder ein Mitoseinhibitor-Herbizid empfindlicher reagiert.

2. Verfahren gemäß Anspruch 1, bei dem
(i) die Pflanze aus der Gruppe bestehend aus *Amaranthus palmeri, Amaranthus rudis, Amaranthus hybridus, Amaranthus lividus, Amaranthus viridis, Ambrosia trifida, Conyza canadensis* und *Euphorbia heterophylla* ausgewählt wurde; oder
(ii) die Zusammensetzung ferner das DHPS-Inhibitor-Herbizid oder das Mitoseinhibitor-Herbizid umfasst und die Behandlung eine äußere Verabreichung der Zusammensetzung auf die Pflanze umfasst, wobei die Zusammensetzung vorzugsweise ferner ein oder mehrere Co-Herbizide umfasst, die sich von dem DHPS-Inhibitor-Herbizid oder dem Mitoseinhibitor-Herbizid unterscheiden, und wobei das eine oder die mehreren Co-Herbizide aus der Gruppe bestehend aus Amidherbiziden, Arsenherbiziden, Benzothiazolherbiziden, Benzoylcyclohexandionherbiziden, Benzofuranylalkylsulfonat-Herbiziden, Cyclohexandion-Herbiziden, Cyclopropylisoxazol-Herbiziden, Dicarboximid-Herbiziden, Dinitroanilin-Herbiziden, Dinitrophenol-Herbiziden, Diphenyletherherbiziden, Dithiocarbamatherbiziden, Glycinherbiziden, halogenierten aliphatischen Herbiziden, Imidazolinonherbiziden, anorganischen Herbiziden, Nitrilherbiziden, phosphororganischen Herbiziden, Oxadiazolon-Herbiziden, Oxazol-Herbiziden, Phenoxy-Herbiziden, Phenylendiamin-Herbiziden, Pyrazol-Herbiziden, Pyridazin-Herbiziden, Pyridazinon-Herbiziden, Pyridin-Herbiziden, Pyrimidindiamin-Herbiziden, Pyrimidinyloxybenzylamin-Herbiziden, quaternären Ammonium-Herbiziden, Thiocarbamat-Herbiziden, Thiocarbonat-Herbiziden, Thioharnstoff-Herbiziden, Triazinon-Herbiziden, Triazol-Herbiziden, Triazolon-Herbiziden, Triazolopyrimidin-Herbiziden, Uracil-Herbiziden und Harnstoff-Herbiziden ausgewählt wurden.

3. Verfahren gemäß Anspruch 1, wobei die Zusammensetzung eine beliebige Kombination von zwei oder mehr der dsRNA-Polynukleotide umfasst und die Behandlung eine äußere Verabreichung der Zusammensetzung auf die Pflanze umfasst.

4. Zusammensetzung, die ein dsRNA-Polynukleotid und ein Übertragungsmittel umfasst, wobei das dsRNA-Polynukleotid identisch mit oder komplementär zu einer RNA-Sequenz einer DHPS-Gensequenz ist, wobei die DHPS-Gensequenz aus der Gruppe bestehend aus SEQ ID Nummern:1, 2, 5, 9, 11, 18, 19 und einem Polynukleotidfragment davon mit mindestens 18 aufeinanderfolgenden Nukleotiden der DHPS-Gensequenz ausgewählt wurde, wobei das Übertragungsmittel eine siliciumorganische Zusammensetzung oder eine darin enthaltene siliciumorganische Verbindung ist, welche die Oberfläche der Pflanze für das Eindringen des dsRNA-Polynukleotids konditioniert, und wobei das Wachstum, die Entwicklung oder die Fortpflanzungsfähigkeit der mit der Zusammensetzung behandelten Pflanze reguliert, unterdrückt oder verzögert wird oder die Pflanze im Vergleich zu einer unbehandelten Pflanze durch die Zusammensetzung, welche das dsRNA-Polynukleotid enthält, auf ein DHPS-Inhibitor-Herbizid oder ein Mitoseinhibitor-Herbizid empfindlicher reagiert.

5. Zusammensetzung gemäß Anspruch 4, in der
(i) die Zusammensetzung ferner das DHPS-Inhibitor-Herbizid oder das Mitoseinhibitor-Herbizid umfasst, wobei das DHPS-Inhibitor-Herbizid vorzugsweise aus der Gruppe bestehend aus Carbamaten und Asulam ausgewählt wurde oder das Mitoseinhibitor-Herbizid aus der Gruppe bestehend aus Dinitroanilin-Herbiziden ausgewählt wurde, oder
(ii) die Zusammensetzung ferner ein Co-Herbizid umfasst, wobei das Co-Herbizid aus der Gruppe bestehend aus Amidherbiziden, Arsenherbiziden, Benzothiazolherbiziden, Benzoylcyclohexandionherbiziden, Benzofuranylalkylsulfonat-Herbiziden, Cyclohexandion-Herbiziden, Cyclopropylisoxazol-Herbiziden, Dicarboximid-Herbiziden, Dinitroanilin-Herbiziden, Dinitrophenol-Herbiziden, Diphenyletherherbiziden, Dithiocarbamatherbiziden, Glycinherbiziden, halogenierten aliphatischen Herbiziden, Imidazolinonherbiziden, anorganischen Herbiziden, Nitrilherbiziden, phosphororganischen Herbiziden, Oxadiazolon-Herbiziden, Oxazol-Herbiziden, Phenoxy-Herbiziden, Phenylendiamin-Herbiziden, Pyrazol-Herbiziden, Pyridazin-Herbiziden, Pyridazinon-Herbiziden, Pyridin-Herbiziden, Pyrimidindiamin-Herbiziden, Pyrimidinyloxybenzylamin-Herbiziden, quaternären Ammonium-Herbiziden, Thiocarbamat-Herbiziden, Thiocarbonat-Herbiziden, Thioharnstoff-Herbiziden, Triazinon-Herbiziden, Triazol-Herbiziden, Triazolon-Herbiziden, Triazolopyrimidin-Herbiziden, Uracil-Herbiziden und Harnstoff-Herbiziden ausgewählt wurde.

6. Verfahren zur Verringerung der Expression eines DHPS-Gens in einer Pflanze, Schritte umfassend, bei denen man: eine Zusammensetzung, die ein dsRNA-Polynukleotid und ein Übertragungsmittel umfasst, äußerlich auf die Pflanze aufträgt, wobei das dsRNA-Polynukleotid identisch mit oder komplementär zu einer RNA-Sequenz einer DHPS-Gensequenz ist, wobei die DHPS-Gensequenz aus der Gruppe bestehend aus SEQ ID Nummern:1, 2, 5, 9, 11, 18, 19 und einem Polynukleotidfragment davon mit mindestens 18 aufeinanderfolgenden Nukleotiden der DHPS-Gensequenz ausgewählt wurde, wobei das Übertragungsmittel eine siliciumorganische Zusammensetzung oder eine darin enthaltene siliciumorganische Verbindung ist, welche die Oberfläche der Pflanze für das Eindringen das dsRNA-Polynukleotids konditioniert, und wobei die Expression des DHPS-Gens im Vergleich zu einer unbehandelten Pflanze reduziert wird.

7. Verfahren zur Identifizierung von dsRNA-Polynukleotiden, welche zur Veränderung der DHPS-Genexpression bei äußerlicher Auftragung auf eine Pflanze nützlich sind, Schritte umfassend, bei denen man: a) eine Vielzahl an dsRNA-Polynukleotiden bereitstellt, welche einen Bereich umfassen, der identisch mit oder komplementär zu einem Polynukleotidfragment mit einer Länge von mindestens 18 aufeinanderfolgenden Nukleotiden ist, welcher identisch mit oder komplementär zu einer DHPS-Gensequenz ist, die aus der Gruppe bestehend aus SEQ ID Nummern:1, 2, 5, 9, 11, 18 und 19 ausgewählt wurde; b) eine Zusammensetzung äußerlich auf die Pflanze aufträgt, wobei die Zusammensetzung eines oder mehrere der dsRNA-Polynukleotide und ein Übertragungsmittel umfasst, wobei das Übertragungsmittel eine siliciumorganische Zusammensetzung oder eine darin enthaltene siliciumorganische Verbindung ist, welche die Oberfläche der Pflanze für das Eindringen der einen oder mehreren dsRNA-Polynukleotide konditioniert; c) die Pflanze oder einen Extrakt davon auf die Veränderung der DHPS-Genexpression hin analysiert, und d) wobei das Wachstum, die Entwicklung oder die Fortpflanzungsfähigkeit der mit der Zusammensetzung behandelten Pflanze reguliert, unterdrückt oder verzögert wird oder die Pflanze im Vergleich zu einer unbehandelten Pflanze durch die Zusammensetzung, welche das dsRNA-Polynukleotid enthält, auf ein DHPS-Inhibitor-Herbizid oder ein Mitoseinhibitor-Herbizid empfindlicher reagiert.

8. Verfahren gemäß Anspruch 7, bei dem
(i) die Pflanze aus der Gruppe bestehend aus *Amaranthus palmeri, Amaranthus rudis, Amaranthus hybridus, Amaranthus lividus, Amaranthus viridis, Ambrosia trifida, Conyza canadensis* und *Euphorbia heterophylla* ausgewählt wurde;
(ii) die DHPS-Genexpression in der Pflanze im Vergleich zu einer nicht mit der Zusammensetzung behandelten Pflanze reduziert ist.

9. Landwirtschaftliche chemische Zusammensetzung, die eine Mischung aus einem dsRNA-Polynukleotid, einem Übertragungsmittel, einem DHPS-Inhibitor-Herbizid oder einem Mitoseinhibitor-Herbizid und einem Co-Herbizid umfasst, wobei das dsRNA-Polynukleotid identisch mit oder komplementär zu einem Teil einer RNA-Sequenz einer DHPS-Gensequenz ist, wobei die DHPS-Gensequenz aus der Gruppe bestehend aus SEQ ID Nummern:1, 2, 5, 9, 11, 18, 19 und einem Polynukleotidfragment davon mit mindestens 18 aufeinanderfolgenden Nukleotiden der DHPS-Gensequenz ausgewählt wurde, wobei das Übertragungsmittel eine siliciumorganische Zusammensetzung oder eine darin enthaltene siliciumorganische Verbindung ist, welche die Oberfläche der Pflanze für das Eindringen des dsRNA-Polynukleotids konditioniert, und wobei das Wachstum, die Entwicklung oder die Fortpflanzungsfähigkeit der mit der Zusammensetzung behandelten Pflanze reguliert, unterdrückt oder verzögert wird oder die Pflanze im Vergleich zu einer unbehandelten Pflanze durch die Zusammensetzung, welche das dsRNA-Polynukleotid enthält, auf ein DHPS-Inhibitor-Herbizid oder ein Mitoseinhibitor-Herbizid empfindlicher reagiert.

10. Landwirtschaftliche chemische Zusammensetzung gemäß Anspruch 9, wobei das Co-Herbizid aus der Gruppe bestehend aus Amidherbiziden, Arsenherbiziden, Benzothiazolherbiziden, Benzoylcyclohexandionherbiziden, Benzofuranylalkylsulfonat-Herbiziden, Cyclohexandion-Herbiziden, Cyclopropylisoxazol-Herbiziden, Dicarboximid-Herbiziden, Dinitroanilin-Herbiziden, Dinitrophenol-Herbiziden, Diphenyletherherbiziden, Dithiocarbamatherbiziden, Glycinherbiziden, halogenierten aliphatischen Herbiziden, Imidazolinonherbiziden, anorganischen Herbiziden, Nitrilherbiziden, phosphororganischen Herbiziden, Oxadiazolon-Herbiziden, Oxazol-Herbiziden, Phenoxy-Herbiziden, Phenylendiamin-Herbiziden, Pyrazol-Herbiziden, Pyridazin-Herbiziden, Pyridazinon-Herbiziden, Pyridin-Herbiziden, Pyrimidindiamin-Herbiziden, Pyrimidinyloxybenzylamin-Herbiziden, quaternären Ammonium-Herbiziden, Thiocarbamat-Herbiziden, Thiocarbonat-Herbiziden, Thioharnstoff-Herbiziden, Triazinon-Herbiziden, Triazol-Herbiziden, Triazolon-Herbiziden, Triazolopyrimidin-Herbiziden, Uracil-Herbiziden und Harnstoff-Herbiziden ausgewählt wurde.

11. Landwirtschaftliche chemische Zusammensetzung, die eine Mischung aus einem dsRNA-Polynukleotid, einem Übertragungsmittel, einem DHPS-Inhibitor-Herbizid und einem Pestizid umfasst, wobei das dsRNA-Polynukleotid identisch mit oder komplementär zu einem Teil einer RNA-Sequenz einer DHPS-Gensequenz ist, wobei die DHPS-Gensequenz aus der Gruppe bestehend aus SEQ ID Nummern:1, 2, 5, 9, 11, 18, 19 und einem Polynukleotidfragment davon mit mindestens 18 aufeinanderfolgenden Nukleotiden der DHPS-Gensequenz ausgewählt wurde, wobei das Übertragungsmittel eine siliciumorganische Zusammensetzung oder eine darin enthaltene siliciumorganische Verbindung ist, welche die Oberfläche der Pflanze für das Eindringen des dsRNA-Polynukleotids konditioniert, und wobei das Wachstum, die Entwicklung oder die Fortpflanzungsfähigkeit der mit der Zusammensetzung behandelten Pflanze reguliert, unterdrückt oder verzögert wird oder die Pflanze im Vergleich zu einer unbehandelten Pflanze durch die Zusammensetzung, welche das dsRNA-Polynukleotid enthält, auf ein DHPS-Inhibitor-Herbizid oder ein Mitoseinhibitor-Herbizid empfindlicher reagiert.

12. Landwirtschaftliche chemische Zusammensetzung gemäß Anspruch 11, bei der das Pestizid aus der Gruppe bestehend aus Insektiziden, Fungiziden, Nematiziden, Bakteriziden, Akariziden, Wachstumsregulatoren, chemische Sterilisationsmittel, Botenstoffe, Repellent, Lockstoffen, Pheromonen, Futterstimulanzien und Biopestiziden ausgewählt wurde.

13. Zusammensetzung, die ein dsRNA-Polynukleotid und ein Übertragungsmittel gemäß Anspruch 4 umfasst, wobei das dsRNA-Polynukleotid identisch mit oder komplementär zu einer RNA-Sequenz einer DHPS-Gensequenz ist, wobei die DHPS-Gensequenz die SEQ ID Nummern: 1176, 1186, ein Komplement davon und ein Polynukleotidfragment davon mit mindestens 18 aufeinanderfolgenden Nukleotiden der DHPS-Gensequenz umfasst.

## Revendications

1. Procédé de lutte contre des végétaux, qui comporte le traitement d'un végétal avec une composition comprenant un polynucléotide d'ARN double brin (ARNdb) et un agent de transfert, étant entendu que ledit polynucléotide d'ARNdb est identique ou complémentaire à une séquence d'ARN correspondant à une séquence de gène de 7,8-dihydroptéroate synthase (DHPS), ladite séquence de gène de DHPS étant choisie dans l'ensemble constitué par les séquences SEQ ID NO : 1, SEQ ID NO : 2, SEQ ID NO : 5, SEQ ID NO : 9, SEQ ID NO : 11, SEQ ID NO : 18 et SEQ ID NO : 19, et un fragment polynucléotidique de celles-ci comportant au moins 18 nucléotides contigus provenant de ladite séquence de gène de DHPS, et ledit agent de transfert est une composition à base d'organosilicone ou un composé de type organosilicone contenu en son sein, et prépare la surface dudit végétal à la perméation dudit polynucléotide d'ARNdb, moyennant quoi la croissance, le développement ou la capacité de reproduction dudit végétal est supprimé(e) ou retardé(e), ou bien ledit végétal, sous l'effet de ladite composition contenant ledit polynucléotide d'ARNdb, est plus sensible qu'un végétal non traité à un herbicide inhibiteur de DHPS ou un herbicide inhibiteur de mitose.

2. Procédé selon la revendication 1, dans lequel :
(i) ledit végétal est choisi dans le groupe constitué par les végétaux *Amaranthus palmeri, Amaranthus rudis, Amaranthus hybridus, Amaranthus lividus, Amaranthus viridis, Ambrosia trifida, Conyza canadensis* et *Euphorbia heterophylla,*
(ii) ou ladite composition contient également ledit herbicide inhibiteur de DHPS ou ledit herbicide inhibiteur de mitose et ledit traitement comporte l'application externe de ladite composition sur ledit végétal et, de préférence, ladite composition comprend en outre un ou plusieurs co-herbicide(s) différent(s) dudit herbicide inhibiteur de DHPS ou dudit herbicide inhibiteur de mitose, étant entendu que le(s)dit(s) co-herbicide(s) est ou sont choisi(s) dans l'ensemble constitué par les herbicides de type amide, herbicides arsenicaux, herbicides de type benzothiazole, herbicides de type benzoyl-cyclohexanedione, herbicides de type alkylsulfonate de benzofuranyle, herbicides de type cyclohexène-oxime, herbicides de type cyclopropyl-isoxazole, herbicides de type dicarboximide, herbicides de type dinitroaniline, herbicides de type dinitrophénol, herbicides de type éther diphénylique, herbicides de type dithiocarbamate, herbicides de type glycine, herbicides aliphatiques halogénés, herbicides de type imidazolinone, herbicides minéraux, herbicides de type nitrile, herbicides organophosphorés, herbicides de type oxadiazolone, herbicides de type oxazole, herbicides phénoxyliques, herbicides de type phénylène-diamine, herbicides de type pyrazole, herbicides de type pyridazine, herbicides de type pyridazinone, herbicides de type pyridine, herbicides de type pyrimidine-diamine, herbicides de type pyrimidinyloxy-benzylamine, herbicides de type ammonium quaternaire, herbicides de type thiocarbamate, herbicides de type thiocarbonate, herbicides de type thiourée, herbicides de type triazine, herbicides de type triazinone, herbicides de type triazole, herbicides de type triazolone, herbicides de type triazolo-pyrimidine, herbicides de type uracile et herbicides de type urée.

3. Procédé selon la revendication 1, dans lequel ladite composition comprend n'importe quelle combinaison d'au moins deux desdits polynucléotides d'ARNdb et ledit traitement comporte l'application externe de ladite composition sur ledit végétal.

4. Composition comprenant un polynucléotide d'ARNdb et un agent de transfert, dans laquelle ledit polynucléotide d'ARNdb est identique ou complémentaire à une séquence d'ARN correspondant à une séquence de gène de DHPS, ladite séquence de gène de DHPS étant choisie dans l'ensemble constitué par les séquences SEQ ID NO : 1, SEQ ID NO : 2, SEQ ID NO : 5, SEQ ID NO : 9, SEQ ID NO : 11, SEQ ID NO : 18 et SEQ ID NO : 19, et un fragment polynucléotidique de celles-ci comportant au moins 18 nucléotides contigus provenant de ladite séquence de gène de DHPS, et ledit agent de transfert est une composition à base d'organosilicone ou un composé de type organosilicone contenu en son sein, et prépare la surface d'un végétal à la perméation dudit polynucléotide d'ARNdb, moyennant quoi ledit végétal traité par ladite composition voit sa croissance, son développement ou sa capacité de reproduction régulé(e), supprimé(e) ou retardé(e), ou bien ledit végétal, sous l'effet de ladite composition contenant ledit polynucléotide d'ARNdb, est plus sensible qu'un végétal non traité à un herbicide inhibiteur de DHPS ou un herbicide inhibiteur de mitose.

5. Composition selon la revendication 4, dans laquelle :
(i) ladite composition contient également ledit herbicide inhibiteur de DHPS ou ledit herbicide inhibiteur de mitose et, de préférence, ledit herbicide inhibiteur de DHPS est choisi dans l'ensemble constitué par les carbamates et l'asulame, ou ledit herbicide inhibiteur de mitose est choisi parmi les herbicides de type dinitroaniline,
(ii) ou ladite composition comprend en outre un co-herbicide, ledit co-herbicide étant choisi dans l'ensemble constitué par les herbicides de type amide, herbicides arsenicaux, herbicides de type benzothiazole, herbicides de type benzoyl-cyclohexanedione, herbicides de type alkylsulfonate de benzofuranyle, herbicides de type cyclohexène-oxime, herbicides de type cyclopropyl-isoxazole, herbicides de type dicarboximide, herbicides de type dinitroaniline, herbicides de type dinitrophénol, herbicides de type éther diphénylique, herbicides de type dithiocarbamate, herbicides de type glycine, herbicides aliphatiques halogénés, herbicides de type imidazolinone, herbicides minéraux, herbicides de type nitrile, herbicides organophosphorés, herbicides de type oxadiazolone, herbicides de type oxazole, herbicides phénoxyliques, herbicides de type phénylène-diamine, herbicides de type pyrazole, herbicides de type pyridazine, herbicides de type pyridazinone, herbicides de type pyridine, herbicides de type pyrimidine-diamine, herbicides de type pyrimidinyloxy-benzylamine, herbicides de type ammonium quaternaire, herbicides de type thiocarbamate, herbicides de type thiocarbonate, herbicides de type thiourée, herbicides de type triazine, herbicides de type triazinone, herbicides de type triazole, herbicides de type triazolone, herbicides de type triazolo-pyrimidine, herbicides de type uracile et herbicides de type urée.

6. Procédé de réduction de l'expression d'un gène de DHPS dans un végétal, qui comporte l'application externe sur ledit végétal d'une composition comprenant un polynucléotide d'ARNdb et un agent de transfert, étant entendu que ledit polynucléotide d'ARNdb est identique ou complémentaire à une séquence d'ARN correspondant à une séquence de gène de DHPS, ladite séquence de gène de DHPS étant choisie dans l'ensemble constitué par les séquences SEQ ID NO : 1, SEQ ID NO : 2, SEQ ID NO : 5, SEQ ID NO : 9, SEQ ID NO : 11, SEQ ID NO : 18 et SEQ ID NO : 19, et un fragment polynucléotidique de celles-ci comportant au moins 18 nucléotides contigus provenant de ladite séquence de gène de DHPS, et ledit agent de transfert est une composition à base d'organosilicone ou un composé de type organosilicone contenu en son sein, et prépare la surface dudit végétal à la perméation dudit polynucléotide d'ARNdb, moyennant quoi ladite expression dudit gène de DHPS est moins importante que dans un végétal non traité.

7. Procédé d'identification de polynucléotides d'ARNdb utilisables pour moduler l'expression du gène de DHPS en traitant un végétal par voie externe, qui comprend le fait de :
a) se procurer plusieurs polynucléotides d'ARNdb qui comportent une région identique ou complémentaire à un fragment polynucléotidique d'une longueur d'au moins 18 nucléotides contigus, qui est lui-même identique ou complémentaire à une séquence de gène de DHPS choisie dans l'ensemble constitué par les séquences SEQ ID NO : 1, SEQ ID NO : 2, SEQ ID NO : 5, SEQ ID NO : 9, SEQ ID NO : 11, SEQ ID NO : 18 et SEQ ID NO : 19,
b) traiter par voie externe ledit végétal avec une composition comprenant un ou plusieurs desdits polynucléotides d'ARNdb et un agent de transfert, ledit agent de transfert étant une composition à base d'organosilicone ou un composé de type organosilicone contenu en son sein, et préparant la surface dudit végétal à la perméation dudit ou desdits polynucléotide(s) d'ARNdb,
c) analyser la modulation de l'expression du gène de DHPS dans ledit végétal ou extrait végétal,
d) et vérifier si ledit végétal traité par ladite composition voit sa croissance, son développement ou sa capacité de reproduction régulé(e), supprimé(e) ou retardé(e), ou bien si ledit végétal, sous l'effet de ladite composition, est plus sensible qu'un végétal non traité à un herbicide inhibiteur de DHPS ou un herbicide inhibiteur de mitose.

8. Procédé selon la revendication 7, dans lequel :
(i) ledit végétal est choisi dans le groupe constitué par les végétaux *Amaranthus palmeri, Amaranthus rudis, Amaranthus hybridus, Amaranthus lividus, Amaranthus viridis, Ambrosia trifida, Conyza canadensis* et *Euphorbia heterophylla,*
(ii) ladite expression du gène de DHPS dans ledit végétal est moins importante que dans un végétal non traité.

9. Composition agrochimique comprenant un mélange constitué par un polynucléotide d'ARNdb, un agent de transfert, un herbicide inhibiteur de DHPS ou un herbicide inhibiteur de mitose, et un co-herbicide, dans laquelle ledit polynucléotide d'ARNdb est identique ou complémentaire à une portion d'une séquence d'ARN correspondant à une séquence de gène de DHPS, ladite séquence de gène de DHPS étant choisie dans l'ensemble constitué par les séquences SEQ ID NO : 1, SEQ ID NO : 2, SEQ ID NO : 5, SEQ ID NO : 9, SEQ ID NO : 11, SEQ ID NO : 18 et SEQ ID NO : 19, et un fragment polynucléotidique de celles-ci comportant au moins 18 nucléotides contigus provenant de ladite séquence de gène de DHPS, et ledit agent de transfert est une composition à base d'organosilicone ou un composé de type organosilicone contenu en son sein, et prépare la surface d'un végétal à la perméation dudit polynucléotide d'ARNdb, moyennant quoi ledit végétal traité par ladite composition voit sa croissance, son développement ou sa capacité de reproduction régulé(e), supprimé(e) ou retardé(e), ou bien ledit végétal, sous l'effet de ladite composition contenant ledit polynucléotide d'ARNdb, est plus sensible qu'un végétal non traité audit herbicide inhibiteur de DHPS ou audit herbicide inhibiteur de mitose.

10. Composition agrochimique selon la revendication 9, dans laquelle ledit co-herbicide est choisi dans l'ensemble constitué par les herbicides de type amide, herbicides arsenicaux, herbicides de type benzothiazole, herbicides de type benzoyl-cyclohexanedione, herbicides de type alkylsulfonate de benzofuranyle, herbicides de type cyclohexène-oxime, herbicides de type cyclopropyl-isoxazole, herbicides de type dicarboximide, herbicides de type dinitroaniline, herbicides de type dinitrophénol, herbicides de type éther diphénylique, herbicides de type dithiocarbamate, herbicides de type glycine, herbicides aliphatiques halogénés, herbicides de type imidazolinone, herbicides minéraux, herbicides de type nitrile, herbicides organophosphorés, herbicides de type oxadiazolone, herbicides de type oxazole, herbicides phénoxyliques, herbicides de type phénylène-diamine, herbicides de type pyrazole, herbicides de type pyridazine, herbicides de type pyridazinone, herbicides de type pyridine, herbicides de type pyrimidine-diamine, herbicides de type pyrimidinyloxy-benzylamine, herbicides de type ammonium quaternaire, herbicides de type thiocarbamate, herbicides de type thiocarbonate, herbicides de type thiourée, herbicides de type triazine, herbicides de type triazinone, herbicides de type triazole, herbicides de type triazolone, herbicides de type triazolo-pyrimidine, herbicides de type uracile et herbicides de type urée.

11. Composition agrochimique comprenant un mélange constitué par un polynucléotide d'ARNdb, un agent de transfert, un herbicide inhibiteur de DHPS et un pesticide, dans laquelle ledit polynucléotide d'ARNdb est identique ou complémentaire à une portion d'une séquence d'ARN correspondant à une séquence de gène de DHPS, ladite séquence de gène de DHPS étant choisie dans l'ensemble constitué par les séquences SEQ ID NO : 1, SEQ ID NO : 2, SEQ ID NO : 5, SEQ ID NO : 9, SEQ ID NO : 11, SEQ ID NO : 18 et SEQ ID NO : 19, et un fragment polynucléotidique de celles-ci comportant au moins 18 nucléotides contigus provenant de ladite séquence de gène de DHPS, et ledit agent de transfert est une composition à base d'organosilicone ou un composé de type organosilicone contenu en son sein, et prépare la surface d'un végétal à la perméation dudit polynucléotide d'ARNdb, moyennant quoi ledit végétal traité par ladite composition voit sa croissance, son développement ou sa capacité de reproduction régulé(e), supprimé(e) ou retardé(e), ou bien ledit végétal, sous l'effet de ladite composition contenant ledit polynucléotide d'ARNdb, est plus sensible qu'un végétal non traité audit herbicide inhibiteur de DHPS ou à un herbicide inhibiteur de mitose.

12. Composition agrochimique selon la revendication 11, dans laquelle ledit pesticide est choisi dans le groupe constitué par les insecticides, fongicides, nématicides, bactéricides, acaricides, régulateurs de croissance, chimiostérilisants, substances sémiochimiques, répulsifs, attractifs, phéromones, phagostimulants et biopesticides.

13. Composition comprenant un polynucléotide d'ARNdb et un agent de transfert selon la revendication 4, dans laquelle ledit polynucléotide d'ARNdb est identique ou complémentaire à une séquence d'ARN correspondant à une séquence de gène de DHPS, ladite séquence de gène de DHPS comprenant les séquences SEQ ID NO : 1176 et SEQ ID NO : 1186, un complément de celles-ci et un fragment polynucléotidique de celles-ci comportant au moins 18 nucléotides contigus provenant de ladite séquence de gène de DHPS.
